# EUROPÄISCHE PATENTSCHRIFT

(11) **EP 3 030 562 B1**
(45) Veröffentlichungstag und Bekanntmachung des Hinweises auf die Patenterteilung: **26.07.2017**
(21) Anmeldenummer: 14749778.8
(22) Anmeldetag: 05.08.2014
(51) Int. Cl.: C07D 471/04, A61K 31/437

(54) **SUBSTITUIERTE PYRAZOLO[1,5-A]PYRIDIN-3-CARBOXAMIDE UND IHRE VERWENDUNG**
SUBSTITUTED PYRAZOLO[1,5-A]-PYRIDINE-3-CARBOXAMIDES AND USE THEREOF
PYRAZOLO[1,5-A]PYRIDINE-3-CARBOXAMIDES SUBSTITUÉS ET LEUR UTILISATION

(30) Priorität: 08.08.2013 EP 13179782; 02.05.2014 EP 14166893
(43) Veröffentlichungstag der Anmeldung: 15.06.2016
(73) Patentinhaber: Bayer Pharma Aktiengesellschaft, 13353 Berlin (DE)
(72) Erfinder: VAKALOPOULOS, Alexandros, 40721 Hilden (DE); FOLLMANN, Markus, 50859 Köln (DE); BUCHGRABER, Philipp, 10435 Berlin (DE); GROMOV, Alexey, 40699 Erkrath (DE); HARTUNG, Ingo, 13158 Berlin (DE); LINDNER, Niels, 42115 Wuppertal (DE); WUNDER, Frank, 42117 Wuppertal (DE); STASCH, Johannes-Peter, 81679 München (DE); MARQUARDT, Tobias, 42115 Wuppertal (DE); REDLICH, Gorden, 44799 Bochum (DE); DIETZ, Lisa, 42113 Wuppertal (DE); LI, Volkhart Min-Jian, 42553 Velbert (DE)
(74) Vertreter: BIP Patents
(86) Internationale Anmeldenummer: PCT/EP2014/066780
(87) Internationale Veröffentlichungsnummer: WO 2015/018814

(56) Entgegenhaltungen:
- WO-A1-2012/072512
- KOJIMA AKIHIKO ET AL: "Phosphodiesterase inhibitors. Part 6: Design, synthesis, and structure-activity relationships of PDE4-inhibitory pyrazolo[1,5-a]pyridines with anti-inflammatory acti", BIOORGANIC & MEDICINAL CHEMISTRY LETTERS, Bd. 23, Nr. 19, 8. August 2013 (2013-08-08), Seiten 5311-5316, XP028707942, ISSN: 0960-894X, DOI: 10.1016/J.BMCL.2013.07.069

## Beschreibung

Die vorliegende Anmeldung betrifft neue substituierte Pyrazolo[1,5-a]pyridin-3-carboxamide, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Eines der wichtigsten zellulären Übertragungssysteme in Säugerzellen ist das cyclische Guanosin monophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorphosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Unter anderem in WO 2012/072512-A1 und WO 2010/117787 sind verschiedene Pyrazolo[1,5-a]pyridin-Derivate beschrieben, die zur Behandlung von Erkrankungen verwendet werden können.

Aufgabe der vorliegenden Erfindung war die Bereitstellung neuer Substanzen, die als Stimulatoren der löslichen Guanylatcyclase wirken, und als solche zur Behandlung und/oder Prophylaxe von Krankheiten geeignet sind.

Gegenstand der vorliegenden Erfindung sind Verbindungen der allgemeinen Formel (I) 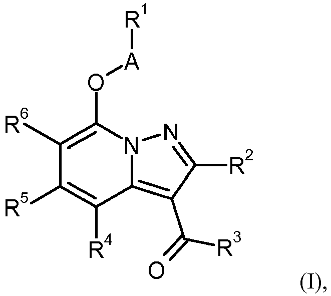 in welcher
- A: für CH₂, CD₂ oder CH(CH₃) steht,
- R¹: für (C₄-C₆)-Alkyl, (C₃-C₇)-Cycloalkyl, Pyridyl oder Phenyl steht,
wobei (C₄-C₆)-Alkyl bis zu sechsfach mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 oder 2 Substituenten unabhängig voneinander ausgewählt aus der Gruppe Halogen, Cyano und (C₁-C₄)-Alkyl substituiert ist,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₂-C₃)-Alkinyl, (C₁-C₄)-Alkoxy, (C₃-C₅)-Cycloalkyl, Difluormethoxy und Trifluormethoxy substituiert sein kann oder an zwei benachbarten Kohlenstoffatomen des Phenyls mit einer Difluormethylendioxy-Brücke substituiert sein kann,
- R²: für Wasserstoff, (C₁-C₄)-Alkyl, (C₁-C₄)-Alkoxymethyl, Cyclopropyl, Monofluormethyl, Difluormethyl oder Trifluormethyl steht,
- R³: für eine Gruppe der Formel 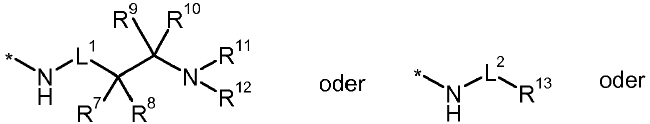 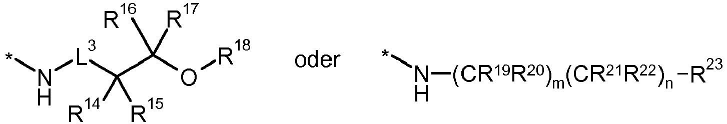
steht, wobei
- *: für die Anknüpfstelle an die Carbonylgruppe steht,
- L¹: für eine Bindung, Methandiyl oder 1,2-Ethandiyl steht,
worin Methandiyl und 1,2-Ethandiyl mit 1 oder 2 Substituenten unabhängig voneinander ausgewählt aus der Gruppe Fluor, Trifluormethyl, (C₁-C₄)-Alkyl, (C₃-C₅)-Cycloalkyl, Hydroxy und (C₁-C₄)-Alkoxy substituiert sein können,
- L²: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₅)-Cycloalkyl, Hydroxy und (C₁-C₄)-Alkoxy substituiert sein können,
- L³: für eine Bindung, Methandiyl oder 1,2-Ethandiyl steht,
worin Methandiyl oder 1,2-Ethandiyl mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein können,
- R⁷: für Wasserstoff, (C₁-C₆)-Alkyl, (C₂-C₆)-Alkenyl, (C₂-C₆)-Alkinyl, (C₃-C₇)-Cycloalkyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy substituiert sein kann,
worin Phenyl, Phenoxy und Benzyloxy mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Nitro, Trifluormethyl, (C₁-C₄)-Alkyl, (C₁-C₄)-Alkoxy, Difluormethoxy, Trifluormethoxy und (C₁-C₄)-Alkylsulfonyl substituiert sein können,
- R⁸: für Wasserstoff oder (C₁-C₆)-Alkyl steht,
oder
- R⁷ und R⁸: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₃-C₇)-Cycloalkyl, (C₂-C₆)-Alkenyl, (C₂-C₆)-Alkinyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Hydroxy, (C₁-C₄)-Alkoxy, Benzyloxy, Phenoxy und Phenyl substituiert sein kann,
worin Benzyloxy, Phenoxy und Phenyl mit 1 bis 3 Substituenten Halogen substituiert sein können,
worin (C₃-C₇)-Cycloalkyl mit 1 oder 2 Substituenten Fluor oder (C₁-C₄)-Alkyl substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁰: für Wasserstoff oder (C₁-C₆)-Alkyl steht,
oder
- R⁹ und R¹⁰: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R⁷ und R⁹: zusammen mit den Kohlenstoffatomen, an die sie gebunden sind, einen 3-bis 7-gliedrigen Carbocyclus oder einen 4- bis 7-gliedrigen Heterocyclus bilden,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
mit der Maßgabe, dass die Reste R⁷ und R⁹ nicht beide gleichzeitig für Phenyl oder 5- oder 6-gliedriges Heteroaryl stehen,
- R¹¹: für Wasserstoff oder (C₁-C₄)-Alkyl steht,
worin (C₁-C₄)-Alkyl mit 1 bis 3 Substituenten unabhängig voneinander ausgewählt aus der Gruppe Fluor, Trifluormethyl, Hydroxy und (C₁-C₄)-Alkoxy substituiert sein kann,
- R¹²: für Wasserstoff, (C₁-C₆)-Alkyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Stickstoffatom, an das sie gebunden sind, einen 4- bis 7-gliedrigen Aza-Heterocyclus bilden,
- R¹³: für 5- bis 9-gliedriges, über ein Ring-Kohlenstoffatom gebundenes AzaHeterocyclyl steht,
worin 5- bis 9-gliedriges Aza-Heterocyclyl mit 1 bis 5 Substituenten unabhängig voneinander ausgewählt aus der Gruppe Fluor, Trifluormethyl, (C₁-C₄)-Alkyl, (C₃-C₇)-Cycloalkyl und Benzyl substituiert sein kann,
und
worin 5- bis 9-gliedriges Aza-Heterocyclyl mit einem Phenyl-Ring anneliert sein kein, der seinerseits mit 1 oder 2 Substituenten ausgewählt aus Halogen, (C₁-C₄)-Alkyl und Trifluormethyl substituiert sein kann,
- R¹⁴: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, -(C=O)NR²⁴R²⁵, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
worin R²⁴ für Wasserstoff, (C₁-C₄)-Alkyl, (C₃-C₇)-Cycloalkyl, Aryl oder Naphthyl steht,
worin R²⁵ für Wasserstoff oder Methyl steht,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁵: für Wasserstoff oder (C₁-C₆)-Alkyl steht,
worin (C₁-C₄)-Alkyl mit Hydroxy substituiert sein kann,
oder
- R¹⁴ und R¹⁵: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R¹⁶: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁷: für Wasserstoff oder (C₁-C₆)-Alkyl steht,
worin (C₁-C₄)-Alkyl mit Hydroxy substituiert sein kann,
oder
- R¹⁶ und R¹⁷: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass die Reste R¹⁴ und R¹⁶ nicht beide gleichzeitig für Phenyl oder 5-oder 6-gliedriges Heteroaryl stehen,
oder
- R¹⁴ und R¹⁶: zusammen mit den Kohlenstoffatomen, an die sie gebunden sind, einen 3-bis 7-gliedrigen Carbocyclus oder einen 4- bis 7-gliedrigen Heterocyclus bilden,
mit der Maßgabe, dass gleichzeitig nicht mehr als eines der Reste-Paare R¹⁴ und R¹⁵, R¹⁶ und R¹⁷ bzw. R¹⁴ und R¹⁶ einen Carbo- oder Heterocyclus bildet,
- R¹⁸: für Wasserstoff oder (C₁-C₄)-Alkyl steht,
worin (C₁-C₄)-Alkyl mit 1 bis 5 Substituenten Fluor substituiert sein kann,
- m: für 0, 1 oder 2 steht,
- n: für 0 oder 1 steht,
- R¹⁹: für Wasserstoff, Cyano oder (C₁-C₄)-Alkyl steht,
worin (C₁-C₄)-Alkyl mit 1 bis 5 Substituenten Fluor substituiert sein kann,
- R²⁰: für Wasserstoff oder (C₁-C₄)-Alkyl steht,
worin (C₁-C₄)-Alkyl mit 1 bis 5 Substituenten Fluor substituiert sein kann,
- R²¹: für Wasserstoff oder (C₁-C₄)-Alkyl steht,
worin (C₁-C₄)-Alkyl mit 1 bis 5 Substituenten Fluor substituiert sein kann,
- R²²: für Wasserstoff oder (C₁-C₄)-Alkyl steht,
worin (C₁-C₄)-Alkyl mit 1 bis 3 Substituenten Fluor substituiert sein kann,
oder
- R¹⁹ und R²⁰: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R²¹ und R²²: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R¹⁹ und R²¹: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass gleichzeitig nicht mehr als eines der Reste-Paare R¹⁹ und R²⁰, R²¹ und R²² bzw. R¹⁹ und R²¹ einen Carbo- oder Heterocyclus bildet,
- R²³: für (C₁-C₆)-Alkyl, 5- bis 9-gliedriges, über ein Ring-Kohlenstoffatom gebundenes Heterocyclyl, 5- bis 9-gliedriges Carbocyclyl, Phenyl, Indanyl oder 5- bis 10-gliedriges Heteroaryl steht,
wobei (C₁-C₆)-Alkyl mit 1 bis 3 Substituenten unabhängig voneinander ausgewählt aus der Gruppe Fluor, Trifluormethyl und Cyano substituiert sein kann,
wobei Phenyl mit 1 bis 3 Substituenten unabhängig voneinander ausgewählt aus der Gruppe Halogen, Cyano, Trifluormethyl, Difluormethyl, (C₁-C₆)-Alkyl, (C₁-C₄)-Alkylcarbonyl, (C₁-C₄)-Alkoxycarbonyl, Hydroxycarbonyl, -(C=O)NR²⁶R²⁷, (C₁-C₄)-Alkylsulfonyl, (C₃-C₆)-Cycloalkylsulfonyl, (C₁-C₄)-Alkylthio, (C₁-C₄)-Alkoxy, Trifluormethoxy, Difluormethoxy, Phenoxy, Hydroxy, 5- bis 10-gliedriges Heteroaryl und (C₃-C₇)-Cycloalkyl substituiert sein kann,
worin (C₁-C₆)-Alkyl mit 1 oder 2 Substituenten unabhängig voneinander ausgewählt aus der Gruppe Fluor, Trifluormethoxy, (C₁-C₄)-Alkylcarbonyl, -(C=O)NR²⁶R²⁷, (C₁-C₄)-Alkoxy, (C₃-C₆)-Cycloalkyl, Morpholinyl, Piperidinyl, Pyrrolidinyl, Piperazinyl, Phenyl, Hydroxy und Amino substituiert sein kann,
worin Phenyl mit 1 bis 3 Substituenten Halogen substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl, (C₁-C₄)-Alkylsulfonyl und Methoxy-(C₁-C₄)-alkyl substituiert sein kann,
worin (C₃-C₆)-Cycloalkyl mit Amino oder Hydroxy substituiert sein kann,
und worin
R²⁶ und R²⁷ jeweils unabhängig voneinander für Wasserstoff, (C₁-C₄)-Alkyl oder (C₃-C₇)-Cycloalkyl stehen, wobei Indanyl mit 1 oder 2 Substituenten unabhängig voneinander ausgewählt aus der Gruppe Fluor, Trifluormethyl und Hydroxy substituiert sein kann,
wobei 5- bis 10-gliedriges Heteroaryl mit 1 bis 3 Substituenten unabhängig voneinander ausgewählt aus der Gruppe Fluor, Chlor, Cyano, (C₁-C₆)-Alkyl, Trifluormethyl, (C₁-C₄)-Alkoxy, Amino, (C₁-C₄)-Alkoxycarbonyl, Hydroxy-carbonyl, -(C=O)NR²⁵R²⁶, Phenyl, Pyridyl, Pyrimidyl, 1,3-Thiazol-5-yl und (C₃-C₇)-Cycloalkyl substituiert sein kann,
worin (C₁-C₆)-Alkyl mit 1 bis 3 Substituenten unabhängig voneinander ausgewählt aus der Gruppe Halogen, Cyano, Hydroxy, Amino, Trifluormethyl, Difluormethyl, (C₁-C₄)-Alkylsulfonyl, (C₁-C₄)-Alkyl-carbonyl, (C₁-C₄)-Alkoxycarbonyl, Hydroxycarbonyl, (C₁-C₄)-Alkylthio, (C₁-C₄)-Alkoxy, Trifluormethoxy, Difluormethoxy, Phenoxy, Phenyl, Pyridyl, Pyrimidyl, 5-gliedriges Heteroaryl, Tetrahydrothiophenyl-1,1-dioxid, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
worin 5-gliedriges Heteroaryl mit 1 bis 3 Substituenten unabhängig voneinander ausgewählt aus der Gruppe Halogen, (C₁-C₄)-Alkyl und (C₁-C₄)-Alkoxy substituiert sein kann,
worin Piperidinyl mit 1 bis 4 Substituenten Fluor substituiert sein kann,
worin Phenyl mit 1 bis 3 Substituenten unabhängig voneinander ausgewählt aus der Gruppe Halogen, (C₁-C₄)-Alkyl und (C₁-C₄)-Alkoxy substituiert sein kann,
worin Azetidin mit Hydroxy substituiert sein kann,
worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
und worin
R²⁶ und R²⁷ jeweils unabhängig voneinander für Wasserstoff, (C₁-C₄)-Alkyl oder (C₃-C₇)-Cycloalkyl stehen, wobei 5- bis 9-gliedriges, über ein Ring-Kohlenstoffatom gebundenes, Heterocyclyl mit 1 oder 2 Substituenten unabhängig voneinander ausgewählt aus der Gruppe Oxo, Fluor, Hydroxy und (C₁-C₄)-Alkyl substituiert sein kann,
und
wobei 5- bis 9-gliedriges Carbocyclyl mit 1 oder 2 Substituenten unabhängig voneinander ausgewählt aus der Gruppe Trifluormethyl, Fluor, Hydroxy, Hydroxycarbonyl, (C₁-C₄)-Alkoxycarbonyl und (C₁-C₄)-Alkyl substituiert sein kann,
- R⁴: für Wasserstoff steht,
- R⁵: für Wasserstoff, Halogen, Cyano, Difluormethyl, Trifluormethyl, (C₁-C₄)-Alkyl, (C₃-C₇)-Cycloalkyl, (C₂-C₄)-Alkinyl, (C₁-C₄)-Alkylamino, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Gegenstand der vorliegenden Erfindung sind Verbindungen der allgemeinen Formel (I) 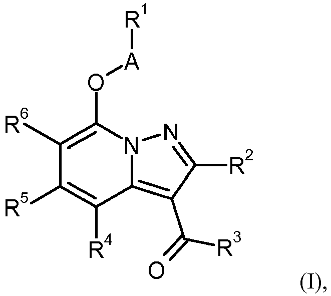 in welcher
- A: für CH₂, CD₂ oder CH(CH₃) steht,
- R¹: für (C₄-C₆)-Alkyl, (C₃-C₇)-Cycloalkyl, Pyridyl oder Phenyl steht,
wobei (C₄-C₆)-Alkyl bis zu sechsfach mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 oder 2 Substituenten unabhängig voneinander ausgewählt aus der Gruppe Halogen, Cyano und (C₁-C₄)-Alkyl substituiert ist,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₂-C₃)-Alkinyl, (C₁-C₄)-Alkoxy, (C₃-C₅)-Cycloalkyl, Difluormethoxy und Trifluormethoxy substituiert sein kann oder an zwei benachbarten Kohlenstoffatomen des Phenyls mit einer Difluormethylendioxy-Brücke substituiert sein kann,
- R²: für Wasserstoff, (C₁-C₄)-Alkyl, (C₁-C₄)-Alkoxymethyl, Cyclopropyl, Cyclobutyl, Monofluormethyl, Difluormethyl oder Trifluormethyl steht,
- R³: für eine Gruppe der Formel 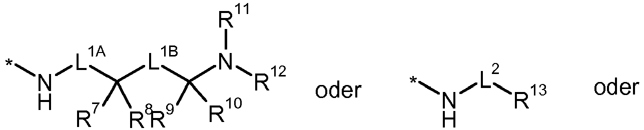 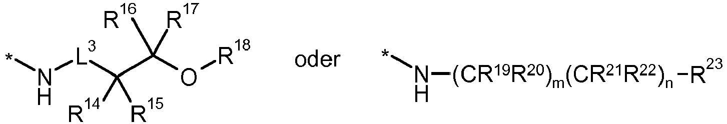
steht, wobei
- *: für die Anknüpfstelle an die Carbonylgruppe steht,
- L^{1A}: für eine Bindung, Methandiyl, 1,2-Ethandiyl oder 1,3-Propandiyl steht,
worin Methandiyl, 1,2-Ethandiyl oder 1,3-Propandiyl mit 1 oder 2 Substituenten unabhängig voneinander ausgewählt aus der Gruppe Fluor, Trifluormethyl, (C₁-C₄)-Alkyl, (C₃-C₅)-Cycloalkyl, Hydroxy und (C₁-C₄)-Alkoxy substituiert sein können,
- L^{1B}: für eine Bindung, Methandiyl oder 1,2-Ethandiyl steht,
- L²: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₅)-Cycloalkyl, Hydroxy und (C₁-C₄)-Alkoxy substituiert sein können,
- L³: für eine Bindung, Methandiyl oder 1,2-Ethandiyl steht,
worin Methandiyl oder 1,2-Ethandiyl mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein können,
- R⁷: für Wasserstoff, (C₁-C₆)-Alkyl, (C₃-C₇)-Cycloalkyl, (C₂-C₆)-Alkenyl, (C₂-C₆)-Alkinyl, Cyano, 5- bis 10-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl, sowie bis zu sechsfach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Halogen und (C₁-C₄)-Alkoxy substituiert sein können,
worin (C₃-C₇)-Cycloalkyl mit 1 oder 2 Substituenten Fluor, Trifluormethyl, (C₁-C₄)-Alkyl oder (C₁-C₄)-Alkoxy substituiert sein kann,
worin Phenyl und 5- bis 10-gliedriges Heteroaryl mit 1 bis 4 Substituenten unabhängig voneinander ausgewählt aus der Gruppe Halogen, Cyano, Nitro, Trifluormethyl, (C₁-C₄)-Alkyl, (C₂-C₄)-Alkenyl, (C₁-C₄)-Alkoxy, Difluormethoxy, Trifluormethoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin Phenyl an zwei benachbarten Kohlenstoffatomen des Phenyls mit einer Methylendioxy-Brücke oder Ethylendioxy-Brücke substituiert sein kann,
oder
für Fluor stehen kann, wenn L^{1A} nicht für eine Bindung steht,
- R⁸: für Wasserstoff oder (C₁-C₆)-Alkyl steht,
oder
für Fluor stehen kann, wenn L^{1A} nicht für eine Bindung steht,
oder
- R⁷ und R⁸: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₃-C₇)-Cycloalkyl, (C₂-C₆)-Alkenyl, (C₂-C₆)-Alkinyl, Cyano, 5- bis 10-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl sowie bis zu sechsfach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Halogen und (C₁-C₄)-Alkoxy substituiert sein können,
worin (C₃-C₇)-Cycloalkyl mit 1 oder 2 Substituenten Fluor, Trifluormethyl, (C₁-C₄)-Alkyl oder (C₁-C₄)-Alkoxy substituiert sein kann,
worin Phenyl und 5- bis 10-gliedriges Heteroaryl mit 1 bis 4 Substituenten unabhängig voneinander ausgewählt aus der Gruppe Halogen, Cyano, Nitro, Trifluormethyl, (C₁-C₄)-Alkyl, (C₂-C₄)-Alkenyl, (C₁-C₄)-Alkoxy, Difluormethoxy, Trifluormethoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin Phenyl an zwei benachbarten Kohlenstoffatomen des Phenyls mit einer Methylendioxy-Brücke oder Ethylendioxy-Brücke substituiert sein kann,
- R¹⁰: für Wasserstoff oder (C₁-C₆)-Alkyl steht,
oder
- R⁹ und R¹⁰: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R⁷ und R⁹: zusammen mit den Kohlenstoffatomen, an die sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus mit 1 oder 2 Substituenten (C₁-C₄)-Alkyl substituiert sein können,
und
worin der 3- bis 7-gliedrige Carbocyclus und der 4- bis 7-gliedrige Heterocyclus mit einem Phenyl-Ring oder einem Pyridyl-Ring anneliert sein können, die ihrerseits mit 1 oder 2 Substituenten ausgewählt aus Halogen, (C₁-C₄)-Alkyl und Trifluormethyl substituiert sein können,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
mit der Maßgabe, dass die Reste R⁷ und R⁹ nicht beide gleichzeitig für Phenyl oder 5- oder 6-gliedriges Heteroaryl stehen,
- R¹¹: für Wasserstoff oder (C₁-C₄)-Alkyl steht,
worin (C₁-C₄)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
- R¹²: für Wasserstoff, (C₁-C₆)-Alkyl, (C₁-C₄)-Alkylcarbonyl, (C₁-C₄)-Alkoxycarbonyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit (C₁-C₄)-Alkyl substituiert sein kann,
- R¹³: für 5- bis 10-gliedriges, über ein Ring-Kohlenstoffatom gebundenes Aza-Heterocyclyl steht,
worin 5- bis 10-gliedriges, über ein Ring-Kohlenstoffatom gebundenes Aza-Heterocyclyl mit 1 bis 2 Substituenten Trifluormethyl, (C₃-C₇)-Cycloalkyl, Oxo und Benzyl sowie bis zu vierfach mit (C₁-C₄)-Alkyl und bis zu zweifach mit Fluor substituiert sein kann,
worin 5- bis 10-gliedriges Aza-Heterocyclyl mit einem Phenyl-Ring oder einem Pyridyl-Ring anneliert sein kann, die ihrerseits mit 1 oder 2 Substituenten ausgewählt aus Halogen, (C₁-C₄)-Alkyl, (C₁-C₄)-Alkoxy und Trifluormethyl substituiert sein können,
oder
für Amino stehen kann, wenn L² für eine Bindung steht,
worin Amino mit (C₁-C₁₀)-Alkyl, (C₁-C₄)-Alkylcarbonyl, (C₃-C₆)-Carbocyclyl, 4-bis 7-gliedriges Heterocyclyl, Phenyl oder 5- oder 6-gliedriges Heteroaryl substituiert sein kann,
worin (C₁-C₄)-Alkylcarbonyl mit Mono-alkylamino oder Di-alkylamino substituiert sein kann,
worin (C₃-C₆)-Carbocyclyl und 4- bis 7-gliedriges Heterocyclyl mit Hydroxy substituiert sein können,
und
worin Phenyl und 5- oder 6-gliedriges Heteroaryl mit 1 bis 3 Substituenten unabhängig voneinander ausgewählt aus der Gruppe Halogen, (C₁-C₄)-Alkyl und Trifluormethyl substituiert sein können,
- R¹⁴: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, -(C=O)NR²⁴R²⁵, 5- oder 6-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy, sowie bis zu sechsfach mit Fluor substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
worin R²⁴ für Wasserstoff, (C₁-C₄)-Alkyl, (C₃-C₇)-Cycloalkyl, Aryl oder Naphthyl steht,
worin R²⁵ für Wasserstoff oder Methyl steht,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁵: für Wasserstoff oder (C₁-C₆)-Alkyl steht,
worin (C₁-C₄)-Alkyl mit Hydroxy substituiert sein kann,
oder
- R¹⁴ und R¹⁵: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R¹⁶: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy, sowie bis zu sechsfach mit Fluor substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁷: für Wasserstoff oder (C₁-C₆)-Alkyl steht,
worin (C₁-C₄)-Alkyl mit Hydroxy substituiert sein kann,
oder
- R¹⁶ und R¹⁷: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass die Reste R¹⁴ und R¹⁶ nicht beide gleichzeitig für Phenyl oder 5-oder 6-gliedriges Heteroaryl stehen,
oder
- R¹⁴ und R¹⁶: zusammen mit den Kohlenstoffatomen, an die sie gebunden sind, einen 3-bis 7-gliedrigen Carbocyclus oder einen 4- bis 7-gliedrigen Heterocyclus bilden,
mit der Maßgabe, dass gleichzeitig nicht mehr als eines der Reste-Paare R¹⁴ und R¹⁵, R¹⁶ und R¹⁷ bzw. R¹⁴ und R¹⁶ einen Carbo- oder Heterocyclus bildet,
- R¹⁸: für Wasserstoff oder (C₁-C₄)-Alkyl steht, worin (C₁-C₄)-Alkyl mit 1 bis 5 Substituenten Fluor substituiert sein kann,
- m: für 0, 1 oder 2 steht,
- n: für 0 oder 1 steht,
- R¹⁹: für Wasserstoff, Cyano oder (C₁-C₆)-Alkyl steht, worin (C₁-C₆)-Alkyl mit 1 bis 5 Substituenten Fluor substituiert sein kann,
- R²⁰: für Wasserstoff oder (C₁-C₄)-Alkyl steht, worin (C₁-C₄)-Alkyl mit 1 bis 5 Substituenten Fluor substituiert sein kann,
- R²¹: für Wasserstoff oder (C₁-C₆)-Alkyl steht, worin (C₁-C₆)-Alkyl mit 1 bis 5 Substituenten Fluor substituiert sein kann,
- R²²: für Wasserstoff oder (C₁-C₄)-Alkyl steht, worin (C₁-C₄)-Alkyl mit 1 bis 5 Substituenten Fluor substituiert sein kann,
oder
- R¹⁹ und R²⁰: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R²¹ und R²²: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R¹⁹ und R²¹: zusammen mit dem Kohlenstoffatom, an das sie gebunden sind, einen 3-bis 7-gliedrigen Carbocyclus oder einen 4- bis 7-gliedriger Heterocyclus bilden, worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass gleichzeitig nicht mehr als eines der Reste-Paare R¹⁹ und R²⁰, R²¹ und R²² bzw. R¹⁹ und R²¹ einen Carbo- oder Heterocyclus bildet,
- R²³: für (C₁-C₆)-Alkyl, (C₁-C₆)-Alkoxy, (C₁-C₄)-Alkoxycarbonyl, Hydroxycarbonyl, Aminocarbonyl, Aminosulfonyl, 5- bis 10-gliedriges, über ein Ring-Kohlenstoffatom gebundenes Heterocyclyl, 5- bis 10-gliedriges Carbocyclyl, Phenyl oder 5- bis 10-gliedriges Heteroaryl steht,
worin (C₁-C₆)-Alkyl mit Cyano, sowie bis zu sechsfach mit Fluor substituiert sein kann,
worin (C₁-C₆)-Alkoxy mit Hydroxy, Amino, Mono-alkylamino, Di-alkylamino, Cyclopropyl, Phenyl oder (C₂-C₄)-Alkenyl substituiert sein kann,
worin Aminocarbonyl mit (C₁-C₆)-Alkyl oder (C₃-C₆)-Cycloalkyl substituiert sein kann,
worin Aminosulfonyl mit (C₁-C₆)-Alkyl oder (C₃-C₆)-Cycloalkyl substituiert sein kann,
worin Phenyl mit 1 bis 3 Substituenten unabhängig voneinander ausgewählt aus der Gruppe Halogen, Cyano, Trifluormethyl, Difluormethyl, (C₁-C₆)-Alkyl, (C₁-C₄)-Alkylcarbonyl, (C₁-C₄)-Alkoxycarbonyl, Hydroxycarbonyl, -(C=O)NR²⁶R²⁷, (C₁-C₄)-Alkylsulfonyl, (C₃-C₆)-Cycloalkylsulfonyl, (C₁-C₄)-Alkylthio, (C₁-C₄)-Alkoxy, Trifluormethoxy, Difluormethoxy, Phenoxy, Hydroxy, 5- bis 10-gliedriges Heteroaryl, 4- bis 7-gliedriges Heterocyclyl und (C₃-C₇)-Cycloalkyl substituiert sein kann,
worin (C₁-C₆)-Alkyl mit 1 oder 2 Substituenten unabhängig voneinander ausgewählt aus der Gruppe Fluor, Trifluormethoxy, (C₁-C₄)-Alkylcarbonyl, -(C=O)NR²⁶R²⁷, (C₁-C₄)-Alkoxy, (C₃-C₆)-Cycloalkyl, Morpholinyl, Piperidinyl, Pyrrolidinyl, Piperazinyl, Phenyl, Hydroxy und Amino substituiert sein kann,
worin Phenyl mit 1 bis 3 Substituenten Halogen substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl, (C₁-C₄)-Alkylsulfonyl und Methoxy-(C₁-C₄)-alkyl substituiert sein kann,
worin (C₃-C₆)-Cycloalkyl mit Amino oder Hydroxy substituiert sein kann,
und worin
R²⁶ und R²⁷ jeweils unabhängig voneinander für Wasserstoff, (C₁-C₄)-Alkyl oder (C₃-C₇)-Cycloalkyl stehen, worin 5- bis 10-gliedriges Heteroaryl mit 1 bis 3 Substituenten unabhängig voneinander ausgewählt aus der Gruppe Fluor, Chlor, Cyano, (C₁-C₆)-Alkyl, Trifluormethyl, (C₁-C₄)-Alkoxy, Amino, (C₁-C₄)-Alkoxycarbonyl, Hydroxy-carbonyl, -(C=O)NR²⁵R²⁶, Phenyl, Pyridyl, Pyrimidyl, 1,3-thiazol-5-yl und (C₃-C₇)-Cycloalkyl substituiert sein kann,
worin (C₁-C₆)-Alkyl mit 1 bis 3 Substituenten unabhängig voneinander ausgewählt aus der Gruppe Halogen, Cyano, Hydroxy, Amino, Trifluormethyl, Difluormethyl, (C₁-C₄)-Alkylsulfonyl, (C₁-C₄)-Alkyl-carbonyl, (C₁-C₄)-Alkoxycarbonyl, Hydroxycarbonyl, (C₁-C₄)-Alkylthio, (C₁-C₄)-Alkoxy, Trifluormethoxy, Difluormethoxy, Phenoxy, Phenyl, Pyridyl, Pyrimidyl, 5-gliedriges Heteroaryl, Tetrahydrothiophenyl-1,1-dioxid, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
worin 5-gliedriges Heteroaryl mit 1 bis 3 Substituenten unabhängig voneinander ausgewählt aus der Gruppe Halogen, (C₁-C₄)-Alkyl und (C₁-C₄)-Alkoxy substituiert sein kann,
worin Piperidinyl mit 1 bis 4 Substituenten Fluor substituiert sein kann,
worin Phenyl mit 1 bis 3 Substituenten unabhängig voneinander ausgewählt aus der Gruppe Halogen, (C₁-C₄)-Alkyl und (C₁-C₄)-Alkoxy substituiert sein kann,
worin Azetidin mit Hydroxy substituiert sein kann,
worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
und worin
R²⁶ und R²⁷ jeweils unabhängig voneinander für Wasserstoff, (C₁-C₄)-Alkyl oder (C₃-C₇)-Cycloalkyl stehen, worin 5- bis 10-gliedriges, über ein Ring-Kohlenstoffatom gebundenes, Heterocyclyl mit 1 bis 3 Substituenten unabhängig voneinander ausgewählt aus der Gruppe Oxo, Fluor,Trifluormethyl, Hydroxy und (C₁-C₄)-Alkyl substituiert sein kann,
worin 5- bis 10-gliedriges über ein Ring-Kohlenstoffatom gebundenes, Heterocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl und Trifluormethyl substituiert sein können,
und
worin 5- bis 10-gliedriges Carbocyclyl mit 1 bis 3 Substituenten unabhängig voneinander ausgewählt aus der Gruppe Trifluormethyl, Fluor, Cyano, Hydroxy, Hydroxycarbonyl, (C₁-C₄)-Alkoxycarbonyl, Amino und (C₁-C₄)-Alkyl substituiert sein kann,
worin (C₁-C₄)-Alkyl mit Hydroxy oder Hydroxycarbonyl substituiert sein kann,
worin 5- bis 10-gliedriges Carbocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
- R⁴: für Wasserstoff steht,
- R⁵: für Wasserstoff, Halogen, Cyano, Difluormethyl, Trifluormethyl, (C₁-C₄)-Alkyl, (C₃-C₇)-Cycloalkyl, (C₂-C₄)-Alkinyl, (C₁-C₄)-Alkylamino, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Gegenstand der vorliegenden Erfindung sind Verbindungen der allgemeinen Formel (I) 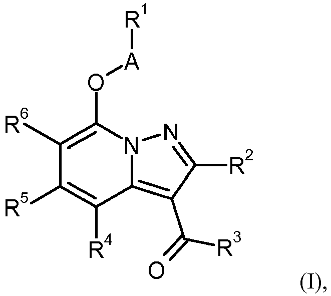 in welcher
- A: für CH₂, CD₂ oder CH(CH₃) steht,
- R¹: für (C₄-C₆)-Alkyl, (C₃-C₇)-Cycloalkyl, Pyridyl oder Phenyl steht,
wobei (C₄-C₆)-Alkyl bis zu sechsfach mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 oder 2 Substituenten unabhängig voneinander ausgewählt aus der Gruppe Halogen, Cyano und (C₁-C₄)-Alkyl substituiert ist,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₂-C₃)-Alkinyl, (C₁-C₄)-Alkoxy, (C₃-C₅)-Cycloalkyl, Difluormethoxy und Trifluormethoxy substituiert sein kann oder an zwei benachbarten Kohlenstoffatomen des Phenyls mit einer Difluormethylendioxy-Brücke substituiert sein kann,
- R²: für Wasserstoff, (C₁-C₄)-Alkyl, (C₁-C₄)-Alkoxymethyl, Cyclopropyl, Cyclobutyl, Monofluormethyl, Difluormethyl oder Trifluormethyl steht,
- R³: für eine Gruppe der Formel 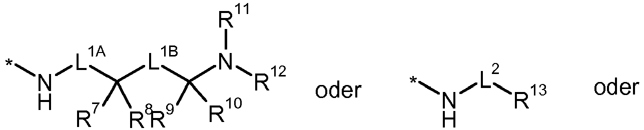 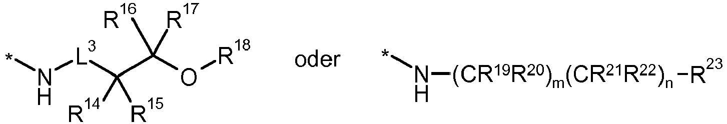
steht, wobei
- *: für die Anknüpfstelle an die Carbonylgruppe steht,
- L^{1A}: für eine Bindung, Methandiyl, 1,2-Ethandiyl oder 1,3-Propandiyl steht,
worin Methandiyl, 1,2-Ethandiyl oder 1,3-Propandiyl mit 1 oder 2 Substituenten unabhängig voneinander ausgewählt aus der Gruppe Fluor, Trifluormethyl, (C₁-C₄)-Alkyl, (C₃-C₅)-Cycloalkyl, Hydroxy und (C₁-C₄)-Alkoxy substituiert sein können,
- L^{1B}: für eine Bindung, Methandiyl oder 1,2-Ethandiyl steht,
- L²: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₅)-Cycloalkyl, Hydroxy und (C₁-C₄)-Alkoxy substituiert sein können,
- L³: für eine Bindung, Methandiyl oder 1,2-Ethandiyl steht,
worin Methandiyl oder 1,2-Ethandiyl mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein können,
- R⁷: für Wasserstoff, (C₁-C₆)-Alkyl, (C₃-C₇)-Cycloalkyl, (C₂-C₆)-Alkenyl, (C₂-C₆)-Alkinyl, Cyano, 5- bis 10-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl, sowie bis zu sechsfach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Halogen und (C₁-C₄)-Alkoxy substituiert sein können,
worin (C₃-C₇)-Cycloalkyl mit 1 oder 2 Substituenten Fluor, Trifluormethyl, (C₁-C₄)-Alkyl oder (C₁-C₄)-Alkoxy substituiert sein kann,
worin Phenyl und 5- bis 10-gliedriges Heteroaryl mit 1 bis 4 Substituenten unabhängig voneinander ausgewählt aus der Gruppe Halogen, Cyano, Nitro, Trifluormethyl, Difluormethyl, (C₁-C₄)-Alkyl, (C₂-C₄)-Alkenyl, (C₁-C₄)-Alkoxy, Difluormethoxy, Trifluormethoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
worin (C₁-C₄)-Alkyl mit Amino oder Hydroxy substitiuiert sein kann, und
worin Phenyl an zwei benachbarten Kohlenstoffatomen des Phenyls mit einer Methylendioxy-Brücke oder Ethylendioxy-Brücke substituiert sein kann,
oder
für Fluor stehen kann, wenn L^{1A} nicht für eine Bindung steht,
- R⁸: für Wasserstoff oder (C₁-C₆)-Alkyl steht,
oder
für Fluor stehen kann, wenn L^{1A} nicht für eine Bindung steht,
oder
- R⁷ und R⁸: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₃-C₇)-Cycloalkyl, (C₂-C₆)-Alkenyl, (C₂-C₆)-Alkinyl, Cyano, 5- bis 10-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl sowie bis zu sechsfach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Halogen und (C₁-C₄)-Alkoxy substituiert sein können,
worin (C₃-C₇)-Cycloalkyl mit 1 oder 2 Substituenten Fluor, Trifluormethyl, (C₁-C₄)-Alkyl oder (C₁-C₄)-Alkoxy substituiert sein kann,
worin Phenyl und 5- bis 10-gliedriges Heteroaryl mit 1 bis 4 Substituenten unabhängig voneinander ausgewählt aus der Gruppe Halogen, Cyano, Nitro, Trifluormethyl, (C₁-C₄)-Alkyl, (C₂-C₄)-Alkenyl, (C₁-C₄)-Alkoxy, Difluormethoxy, Trifluormethoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin Phenyl an zwei benachbarten Kohlenstoffatomen des Phenyls mit einer Methylendioxy-Brücke oder Ethylendioxy-Brücke substituiert sein kann,
- R¹⁰: für Wasserstoff oder (C₁-C₆)-Alkyl steht,
oder
- R⁹ und R¹⁰: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R⁷ und R⁹: zusammen mit den Kohlenstoffatomen, an die sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus mit 1 oder 2 Substituenten (C₁-C₄)-Alkyl substituiert sein können,
und
worin der 3- bis 7-gliedrige Carbocyclus und der 4- bis 7-gliedrige Heterocyclus mit einem Phenyl-Ring oder einem Pyridyl-Ring anneliert sein können, die ihrerseits mit 1 oder 2 Substituenten ausgewählt aus Halogen, (C₁-C₄)-Alkyl und Trifluormethyl substituiert sein können,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
mit der Maßgabe, dass die Reste R⁷ und R⁹ nicht beide gleichzeitig für Phenyl oder 5- oder 6-gliedriges Heteroaryl stehen,
- R¹¹: für Wasserstoff oder (C₁-C₄)-Alkyl steht,
worin (C₁-C₄)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
- R¹²: für Wasserstoff, (C₁-C₆)-Alkyl, (C₁-C₄)-Alkylcarbonyl, (C₁-C₄)-Alkoxycarbonyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit (C₁-C₄)-Alkyl substituiert sein kann,
- R¹³: für 5- bis 10-gliedriges, über ein Ring-Kohlenstoffatom gebundenes Aza-Heterocyclyl oder über ein Ring-Stickstoffatom gebundenes 5- oder 6-gliedriges Heterocyclyl steht,
worin 5- bis 10-gliedriges, über ein Ring-Kohlenstoffatom gebundenes Aza-Heterocyclyl mit 1 bis 2 Substituenten Trifluormethyl, (C₃-C₇)-Cycloalkyl, Oxo und Benzyl sowie bis zu vierfach mit (C₁-C₄)-Alkyl und bis zu zweifach mit Fluor substituiert sein kann,
worin 5- bis 10-gliedriges Aza-Heterocyclyl mit einem Phenyl-Ring oder einem Pyridyl-Ring anneliert sein kann, die ihrerseits mit 1 oder 2 Substituenten ausgewählt aus Halogen, (C₁-C₄)-Alkyl, (C₁-C₄)-Alkoxy und Trifluormethyl substituiert sein können,
worin über ein Ring-Stickstoffatom gebundenes 5- oder 6-gliedriges Heterocyclyl mit (C₁-C₄)-Alkyl oder (C₁-C₄)-Cycloalkyl substituiert sein kann,
worin (C₁-C₄)-Alkyl mit Difluormethyl oder Trifluormethyl substituiert sein kann,
oder
für Amino stehen kann, wenn L² für eine Bindung steht,
worin Amino mit (C₁-C₁₀)-Alkyl, (C₁-C₄)-Alkylcarbonyl, (C₃-C₆)-Carbocyclyl, 4-bis 7-gliedriges Heterocyclyl, Phenyl oder 5- oder 6-gliedriges Heteroaryl substituiert sein kann,
worin (C₁-C₄)-Alkylcarbonyl mit Mono-alkylamino, Di-alkylamino oder über ein Ring-Stickstoffatom gebundenes 5- oder 6-gliedriges Heterocyclyl substituiert sein kann,
worin über ein Ring-Stickstoffatom gebundenes 5- oder 6-gliedriges Heterocyclyl mit Oxo substituiert sein kann,
worin (C₃-C₆)-Carbocyclyl und 4- bis 7-gliedriges Heterocyclyl mit Hydroxy substituiert sein können,
und
worin Phenyl und 5- oder 6-gliedriges Heteroaryl mit 1 bis 3 Substituenten unabhängig voneinander ausgewählt aus der Gruppe Halogen, (C₁-C₄)-Alkyl und Trifluormethyl substituiert sein können,
- R¹⁴: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, -(C=O)NR²⁴R²⁵, 5- oder 6-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy, sowie bis zu sechsfach mit Fluor substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
worin R²⁴ für Wasserstoff, (C₁-C₄)-Alkyl, (C₃-C₇)-Cycloalkyl, Aryl oder Naphthyl steht,
worin R²⁵ für Wasserstoff oder Methyl steht,
und
worin Phenyl und 5- oder 6-gliedriges Heteroaryl mit 1 bis 3 Substituenten unabhängig voneinander ausgewählt aus der Gruppe Halogen, Cyano, Trifluormethyl, Difluormethyl, (C₁-C₄)-Alkyl, (C₁-C₄)-Alkoxy und (C₁-C₄)-Alkylsulfonyl substituiert sein können,
worin (C₁-C₄)-Alkyl mit Amino oder Hydroxy substitiuiert sein kann,
- R¹⁵: für Wasserstoff oder (C₁-C₆)-Alkyl steht,
worin (C₁-C₄)-Alkyl mit Hydroxy substituiert sein kann,
oder
- R¹⁴ und R¹⁵: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R¹⁶: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy, sowie bis zu sechsfach mit Fluor substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁷: für Wasserstoff oder (C₁-C₆)-Alkyl steht,
worin (C₁-C₄)-Alkyl mit Hydroxy substituiert sein kann,
oder
- R¹⁶ und R¹⁷: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass die Reste R¹⁴ und R¹⁶ nicht beide gleichzeitig für Phenyl oder 5-oder 6-gliedriges Heteroaryl stehen,
oder
- R¹⁴ und R¹⁶: zusammen mit den Kohlenstoffatomen, an die sie gebunden sind, einen 3-bis 7-gliedrigen Carbocyclus oder einen 4- bis 7-gliedrigen Heterocyclus bilden,
mit der Maßgabe, dass gleichzeitig nicht mehr als eines der Reste-Paare R¹⁴ und R¹⁵, R¹⁶ und R¹⁷ bzw. R¹⁴ und R¹⁶ einen Carbo- oder Heterocyclus bildet,
- R¹⁸: für Wasserstoff oder (C₁-C₄)-Alkyl steht,
worin (C₁-C₄)-Alkyl mit 1 bis 5 Substituenten Fluor substituiert sein kann,
- m: für 0, 1 oder 2 steht,
- n: für 0 oder 1 steht,
- R¹⁹: für Wasserstoff, Cyano oder (C₁-C₆)-Alkyl steht,
worin (C₁-C₆)-Alkyl mit 1 bis 5 Substituenten Fluor substituiert sein kann,
- R²⁰: für Wasserstoff oder (C₁-C₄)-Alkyl steht,
worin (C₁-C₄)-Alkyl mit 1 bis 5 Substituenten Fluor substituiert sein kann,
- R²¹: für Wasserstoff oder (C₁-C₆)-Alkyl steht,
worin (C₁-C₆)-Alkyl mit 1 bis 5 Substituenten Fluor substituiert sein kann,
- R²²: für Wasserstoff oder (C₁-C₄)-Alkyl steht,
worin (C₁-C₄)-Alkyl mit 1 bis 5 Substituenten Fluor substituiert sein kann,
oder
- R¹⁹ und R²⁰: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R²¹ und R²²: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R¹⁹ und R²¹: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass gleichzeitig nicht mehr als eines der Reste-Paare R¹⁹ und R²⁰, R²¹ und R²² bzw. R¹⁹ und R²¹ einen Carbo- oder Heterocyclus bildet,
- R²³: für (C₁-C₆)-Alkyl, (C₁-C₆)-Alkoxy, (C₁-C₄)-Alkoxycarbonyl, Hydroxycarbonyl, Aminocarbonyl, Aminosulfonyl, 5- bis 10-gliedriges, über ein Ring-Kohlenstoffatom gebundenes Heterocyclyl, 5- bis 10-gliedriges Carbocyclyl, Phenyl oder 5- bis 10-gliedriges Heteroaryl steht,
worin (C₁-C₆)-Alkyl mit Cyano, sowie bis zu sechsfach mit Fluor substituiert sein kann,
worin (C₁-C₆)-Alkoxy mit Hydroxy, Amino, Mono-alkylamino, Di-alkylamino, Cyclopropyl, Phenyl oder (C₂-C₄)-Alkenyl substituiert sein kann,
worin Aminocarbonyl mit (C₁-C₆)-Alkyl oder (C₃-C₆)-Cycloalkyl substituiert sein kann,
worin Aminosulfonyl mit (C₁-C₆)-Alkyl oder (C₃-C₆)-Cycloalkyl substituiert sein kann,
worin Phenyl mit 1 bis 3 Substituenten unabhängig voneinander ausgewählt aus der Gruppe Halogen, Cyano, Trifluormethyl, Difluormethyl, (C₁-C₆)-Alkyl, (C₁-C₄)-Alkylcarbonyl, (C₁-C₄)-Alkoxycarbonyl, Hydroxycarbonyl, -(C=O)NR²⁶R²⁷, (C₁-C₄)-Alkylsulfonyl, (C₃-C₆)-Cycloalkylsulfonyl, (C₁-C₄)-Alkylthio, (C₁-C₄)-Alkoxy, Trifluormethoxy, Difluormethoxy, Phenoxy, Hydroxy, 5- bis 10-gliedriges Heteroaryl, 4- bis 7-gliedriges Heterocyclyl und (C₃-C₇)-Cycloalkyl substituiert sein kann,
worin (C₁-C₆)-Alkyl mit 1 oder 2 Substituenten unabhängig voneinander ausgewählt aus der Gruppe Fluor, Trifluormethoxy, (C₁-C₄)-Alkylcarbonyl, -(C=O)NR²⁶R²⁷, (C₁-C₄)-Alkoxy, (C₃-C₆)-Cycloalkyl, Morpholinyl, Piperidinyl, Pyrrolidinyl, Piperazinyl, Phenyl, Hydroxy und Amino substituiert sein kann,
worin Phenyl mit 1 bis 3 Substituenten Halogen substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl, (C₁-C₄)-Alkylsulfonyl und Methoxy-(C₁-C₄)-alkyl substituiert sein kann,
worin (C₃-C₆)-Cycloalkyl mit Amino oder Hydroxy substituiert sein kann,
und worin
R²⁶ und R²⁷ jeweils unabhängig voneinander für Wasserstoff, (C₁-C₄)-Alkyl oder (C₃-C₇)-Cycloalkyl stehen, worin 5- bis 10-gliedriges Heteroaryl mit 1 bis 3 Substituenten unabhängig voneinander ausgewählt aus der Gruppe Fluor, Chlor, Cyano, (C₁-C₆)-Alkyl, Trifluormethyl, (C₁-C₄)-Alkoxy, Amino, (C₁-C₄)-Alkoxycarbonyl, Hydroxy-carbonyl, -(C=O)NR²⁵R²⁶, Phenyl, Pyridyl, Pyrimidyl, 1,3-thiazol-5- und (C₃-C₇)-Cycloalkyl substituiert sein kann,
worin (C₁-C₆)-Alkyl mit 1 bis 3 Substituenten unabhängig voneinander ausgewählt aus der Gruppe Halogen, Cyano, Hydroxy, Amino, Trifluormethyl, Difluormethyl, (C₁-C₄)-Alkylsulfonyl, (C₁-C₄)-Alkyl-carbonyl, (C₁-C₄)-Alkoxycarbonyl, Hydroxycarbonyl, (C₁-C₄)-Alkylthio, (C₁-C₄)-Alkoxy, Trifluormethoxy, Difluormethoxy, Phenoxy, Phenyl, Pyridyl, Pyrimidyl, 5-gliedriges Heteroaryl, Tetrahydrothiophenyl-1,1-dioxid, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
worin 5-gliedriges Heteroaryl mit 1 bis 3 Substituenten unabhängig voneinander ausgewählt aus der Gruppe Halogen, (C₁-C₄)-Alkyl und (C₁-C₄)-Alkoxy substituiert sein kann,
worin Piperidinyl mit 1 bis 4 Substituenten Fluor substituiert sein kann,
worin Phenyl mit 1 bis 3 Substituenten unabhängig voneinander ausgewählt aus der Gruppe Halogen, (C₁-C₄)-Alkyl und (C₁-C₄)-Alkoxy substituiert sein kann,
worin Azetidin mit Hydroxy substituiert sein kann,
worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
und worin
R²⁶ und R²⁷ jeweils unabhängig voneinander für Wasserstoff, (C₁-C₄)-Alkyl oder (C₃-C₇)-Cycloalkyl stehen, worin 5- bis 10-gliedriges, über ein Ring-Kohlenstoffatom gebundenes, Heterocyclyl mit 1 bis 3 Substituenten unabhängig voneinander ausgewählt aus der Gruppe Oxo, Fluor,Trifluormethyl, Hydroxy und (C₁-C₄)-Alkyl substituiert sein kann,
worin 5- bis 10-gliedriges über ein Ring-Kohlenstoffatom gebundenes, Heterocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl und Trifluormethyl substituiert sein können,
und
worin 5- bis 10-gliedriges Carbocyclyl mit 1 bis 3 Substituenten unabhängig voneinander ausgewählt aus der Gruppe Trifluormethyl, Fluor, Cyano, Hydroxy, Hydroxycarbonyl, (C₁-C₄)-Alkoxycarbonyl, Amino und (C₁-C₄)-Alkyl substituiert sein kann,
worin (C₁-C₄)-Alkyl mit Hydroxy oder Hydroxycarbonyl substituiert sein kann,
worin 5- bis 10-gliedriges Carbocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
- R⁴: für Wasserstoff steht,
- R⁵: für Wasserstoff, Halogen, Cyano, Difluormethyl, Trifluormethyl, (C₁-C₄)-Alkyl, (C₃-C₇)-Cycloalkyl, (C₂-C₄)-Alkinyl, (C₁-C₄)-Alkylamino, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl.

Cycloalkyl bzw. Carbocyclus bzw. Carbocyclyl steht in Rahmen der Erfindung für einen monocyclischen, bicyclischen oder tricyclischen gesättigten Alkylrest mit der jeweils angegebenen Anzahl an Ring-Kohlenstoffatomen. Beispielhaft und vorzugsweise seien genannt: Cyclopropyl, Cyclobutyl, Cyclopentyl, Cyclohexyl, Cycloheptyl und Adamantyl.

Alkenyl steht im Rahmen der Erfindung für einen linearen oder verzweigten Alkenylrest mit 2 bis 6 Kohlenstoffatomen und einer oder zwei Doppelbindungen. Bevorzugt ist ein linearer oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.

Alkinyl steht im Rahmen der Erfindung für einen linearen oder verzweigten Alkinylrest mit 2 bis 6 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.

Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl und Butan-2,3-diyl.

Mono-alkylamino steht im Rahmen der Erfindung für eine Amino-Gruppe mit einem linearen oder verzweigten Alkylsubstituenten, der 1 bis 4 Kohlenstoffatome aufweist. Beispielhaft und vorzugsweise seien genannt: Methylamino, Ethylamino, n-Propylamino, Isopropylamino und tert.-Butylamino.

Di-alkylamino steht im Rahmen der Erfindung für eine Amino-Gruppe mit zwei gleichen oder verschiedenen linearen oder verzweigten Alkylsubstituenten, die jeweils 1 bis 4 Kohlenstoffatome aufweisen. Beispielhaft und vorzugsweise seien genannt: *N*,*N*-Dimethylamino, *N*,*N*-Diethylamino, *N*-Ethyl-*N*-methylamino, *N*-Methyl-*N*-n-propylamino, *N*-Isopropyl-*N*-n-propylamino und *N*-tert.-Butyl-*N*-methylamino.

Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.

Alkoxycarbonyl stehen im Rahmen der Erfindung für einen linearen oder verzweigten Alkoxyrest mit 1 bis 4 Kohlenstoffatomen und einer am Sauerstoffatom angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.

Ein 4- bis 7-gliedrige bzw. 5- bis 10-gliedriger Heterocyclus steht im Rahmen der Erfindung für einen monocyclischen, gesättigten Heterocyclus mit insgesamt 4 bis 7 Ringatomen bzw. 5 bis 10 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.

Ein 4- bis 7-gliedriger Aza-Heterocyclus steht im Rahmen der Erfindung in R¹¹ und R¹² für einen monocyclischen, gesättigten Heterocyclus mit insgesamt 4 bis 7 Ringatomen, der ein Stickstoffatom enthält und darüberhinaus ein weiteres Ring-Heteroatom aus der Reihe N, O, S, SO oder SO₂ enthalten kann und über ein Ring-Stickstoffatom verknüpft ist. Beispielhaft seien genannt: Azetidinyl, Pyrrolidinyl, Pyrazolidinyl, Piperidinyl, Piperazinyl, Morpholinyl, Thiomorpholinyl, 1,1-Dioxothiomorpholinyl, Hexahydroazepinyl und Hexahydro-1,4-diazepinyl.

5- bis 10-gliedriges Aza-Heterocyclyl steht im Rahmen der Erfindung in R¹³ für einen monocyclischen oder bicyclischen, gesättigten oder teilweise ungesättigten Heterocyclus mit insgesamt 5 bis 10 Ringatomen, der ein Stickstoffatom enthält und darüberhinaus ein oder zwei weitere Ring-Heteroatome aus der Reihe N, O, S, SO und/oder SO₂ enthalten kann und über ein Ring-Kohlenstoffatom verknüpft ist. Beispielhaft seien genannt: Pyrrolidinyl, Pyrazolidinyl, Piperidinyl, Piperazinyl, Morpholinyl, Thiomorpholinyl, 1,1-Dioxothiomorpholinyl, Hexahydroazepinyl, Hexahydro-1,4-diazepinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Azabicyclo[4.1.0]heptanyl und Quinuclidinyl.

Heteroaryl steht im Rahmen der Erfindung für einen monocyclischen oder bicyclischen aromatischen Heterocyclus (Heteroaromaten) der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Chinolinyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl und Triazinyl.

Halogen schließt im Rahmen der Erfindung Fluor, Chlor, Brom und Iod ein. Bevorzugt sind Chlor oder Fluor.

In der Formel der Gruppe, für die R³ bzw. R¹ stehen kann, steht der Endpunkt der Linie, an dem das Zeichen * und # steht, nicht für ein Kohlenstoffatom beziehungsweise eine CH₂-Gruppe, sondern ist Bestandteil der Bindung zu dem jeweils bezeichneten Atom, an das R³ bzw. R¹ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt sind im Rahmen der vorliegenden Erfindung Verbindungen der Formel (I), in welcher
- A: für CH₂ oder CD₂ steht,
- R¹: für (C₃-C₆)-Cycloalkyl, Pyridyl oder Phenyl steht,
wobei (C₃-C₆)-Cycloalkyl mit 1 bis 2 Substituenten unabhängig voneinander ausgewählt aus der Gruppe Fluor, Trifluormethyl, Methyl und Ethyl substituiert sein kann,
wobei Pyridyl mit 1 oder 2 Substituenten F substituiert ist,
und
wobei Phenyl mit 1 bis 4 Substituenten unabhängig voneinander ausgewählt aus der Gruppe Halogen, Cyano, Difluormethyl, Trifluormethyl, (C₁-C₄)-Alkyl, (C₁-C₄)-Alkoxy und (C₃-C₅)-Cycloalkyl substituiert sein kann,
- R²: für Wasserstoff, (C₁-C₄)-Alkyl, Cyclopropyl, Difluormethyl oder Trifluormethyl steht,
- R³: für eine Gruppe der Formel 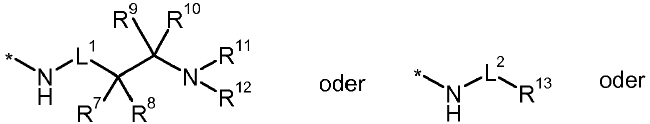 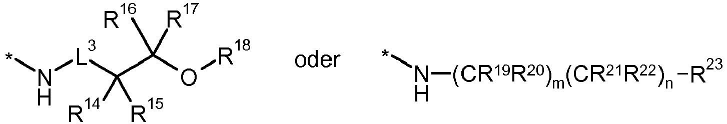
steht, wobei
- *: für die Anknüpfstelle an die Carbonylgruppe steht,
- L¹: für eine Bindung, Methandiyl oder 1,2-Ethandiyl steht,
- L²: für eine Bindung, Methandiyl oder 1,2-Ethandiyl steht,
- L³: für eine Bindung, Methandiyl oder 1,2-Ethandiyl steht,
- R⁷: für Wasserstoff, (C₁-C₆)-Alkyl, (C₃-C₇)-Cycloalkyl, Phenyl oder 5- oder 6-gliedriges Heteroaryl steht,
worin (C₁-C₆)-Alkyl mit 1 bis 5 Substituenten Fluor substituiert sein kann,
und
worin Phenyl und 5- oder 6-gliedriges Heteroaryl mit 1 bis 3 Substituenten unabhängig voneinander ausgewählt aus der Gruppe Fluor, Chlor, Brom, Cyano, Trifluormethyl, Methyl, Ethyl, Methoxy oder Ethoxy substituiert sein können,
- R⁸: für Wasserstoff oder (C₁-C₄)-Alkyl steht,
oder
- R⁷ und R⁸: zusammen mit dem Kohlenstoffatom, an das sie gebunden sind, einen 3-bis 5-gliedrigen Carbocyclus bilden,
- R⁹: für Wasserstoff, (C₁-C₆)-Alkyl, (C₃-C₅)-Cycloalkyl, 5- oder 6-gliedriges Heteroaryl oder Phenyl steht,
worin (C₁-C₆)-Alkyl mit 1 bis 5 Substituenten Fluor substituiert sein kann,
worin (C₁-C₆)-Alkyl mit (C₁-C₄)-Alkoxy, Benzyloxy oder Phenoxy substituiert sein kann,
worin Benzyloxy und Phenoxy mit 1 bis 3 Substituenten unabhängig voneineander ausgewählt aus der Gruppe Fluor, Chlor und Brom substituiert sein können,
worin (C₃-C₅)-Cycloalkyl mit 1 oder 2 Substituenten Fluor oder (C₁-C₄)-Alkyl substituiert sein kann,
und
worin Phenyl und 5- oder 6-gliedriges Heteroaryl mit 1 bis 3 Substituenten unabhängig voneinander ausgewählt aus der Gruppe Fluor, Chlor, Brom, Cyano, Trifluormethyl, Methyl, Ethyl, Methoxy oder Ethoxy substituiert sein können,
- R¹⁰: für Wasserstoff oder (C₁-C₄)-Alkyl steht,
oder
- R⁹ und R¹⁰: zusammen mit dem Kohlenstoffatom, an das sie gebunden sind, einen 3-bis 5-gliedrigen Carbocyclus bilden,
oder
- R⁷ und R⁹: zusammen mit dem Kohlenstoffatom, an das sie gebunden sind, einen 3-bis 6-gliedrigen Carbocyclus oder einen 4- bis 7-gliedrigen Heterocyclus bilden,
worin der 3- bis 6-gliedrige Carbocyclus und der 4- bis 7-gliedrige Heterocyclus mit 1 oder 2 Substituenten Fluor oder (C₁-C₄)-Alkyl substituiert sein können,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
und
mit der Maßgabe, dass die Reste R⁷ und R⁹ nicht beide gleichzeitig für Phenyl oder 5- oder 6-gliedriges Heteroaryl stehen,
- R¹¹: für Wasserstoff oder (C₁-C₄)-Alkyl steht,
worin (C₁-C₄)-Alkyl mit 1 bis 5 Substituenten Fluor substituiert sein kann,
- R¹²: für Wasserstoff, (C₁-C₆)-Alkyl oder (C₃-C₇)-Cycloalkyl steht,
worin (C₁-C₆)-Alkyl mit 1 bis 5 Substituenten Fluor substituiert sein kann,
oder
- R¹¹ und R¹²: zusammen mit dem Sticktoffatom, an das sie gebunden sind, einen 4- bis 7-gliedrigen Aza-Heterocyclus bilden,
- R¹³: für 5- bis 9-gliedriges, über ein Ring-Kohlenstoffatom gebundenes Aza-Heterocyclyl steht,
worin 5- bis 9-gliedriges Aza-Heterocyclyl mit 1 bis 5 Substituenten unabhängig voneinander ausgewählt aus der Gruppe Fluor, Methyl und Ethyl substituiert sein kann,
- R¹⁴: für Wasserstoff, (C₁-C₆)-Alkyl, (C₃-C₅)-Cycloalkyl, -(C=O)NR²⁴R²⁵, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy und (C₁-C₄)-Alkoxy substituiert sein kann,
worin R²⁴ für Wasserstoff, (C₁-C₄)-Alkyl, Aryl oder Naphthyl steht,
worin R²⁵ für Wasserstoff steht,
und
worin Phenyl und 5- oder 6-gliedriges Heteroaryl mit 1 bis 3 Substituenten unabhängig voneinander ausgewählt aus der Gruppe Fluor, Chlor, Brom, Trifluormethyl, Methyl und Ethyl substituiert sein können,
- R¹⁵: für Wasserstoff oder (C₁-C₄)-Alkyl steht,
oder
- R¹⁴ und R¹⁵: zusammen mit dem Kohlenstoffatom, an das sie gebunden sind, einen 3-bis 5-gliedrigen Carbocyclus bilden,
- R¹⁶: für Wasserstoff, (C₁-C₆)-Alkyl oder (C₃-C₅)-Cycloalkyl steht,
worin (C₁-C₆)-Alkyl mit 1 bis 5 Substituenten Fluor substituiert sein kann,
und
worin (C₃-C₅)-Cycloalkyl mit 1 oder 2 Substituenten unabhängig voneinander ausgewählt aus der Gruppe Fluor, Trifluormethyl, Hydroxy und (C₁-C₄)-Alkyl substituiert sein kann,
- R¹⁷: für Wasserstoff oder (C₁-C₄)-Alkyl steht,
oder
- R¹⁶ und R¹⁷: zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten Fluor oder (C₁-C₄)-Alkyl substituiert sein kann,
oder
- R¹⁴ und R¹⁶: zusammen mit dem Kohlenstoffatom, an das sie gebunden sind, einen 3-bis 6-gliedrigen Carbocyclus oder einen 4- bis 7-gliedrigen Heterocyclus bilden,
worin der 3- bis 6-gliedrige Carbocyclus und der 4- bis 7-gliedrige Heterocyclus mit 1 oder 2 Substituenten Fluor oder (C₁-C₄)-Alkyl substituiert sein können,
mit der Maßgabe, dass die Reste R¹⁴ und R¹⁶ nicht beide gleichzeitig für Phenyl stehen,
und
mit der Maßgabe, dass gleichzeitig nicht mehr als eines der Reste-Paare R¹⁴ und R¹⁵, R¹⁶ und R¹⁷ bzw. R¹⁴ und R¹⁶ einen Carbo- oder Heterocyclus bildet,
- R¹⁸: für Wasserstoff oder (C₁-C₄)-Alkyl steht,
worin (C₁-C₄)-Alkyl mit 1 bis 5 Substituenten Fluor substituiert sein kann,
- m: für 0 oder 1 steht,
- n: für 0 oder 1 steht,
- R¹⁹: für Wasserstoff, Cyano oder (C₁-C₄)-Alkyl steht,
worin (C₁-C₄)-Alkyl mit 1 bis 5 Substituenten Fluor substituiert sein kann,
- R²⁰: für Wasserstoff oder (C₁-C₄)-Alkyl steht, worin (C₁-C₄)-Alkyl mit 1 bis 5 Substituenten Fluor substituiert sein kann,
- R²¹: für Wasserstoff oder (C₁-C₄)-Alkyl steht, worin (C₁-C₄)-Alkyl mit 1 bis 5 Substituenten Fluor substituiert sein kann,
- R²²: für Wasserstoff oder (C₁-C₄)-Alkyl steht, worin (C₁-C₄)-Alkyl mit 1 bis 5 Substituenten Fluor substituiert sein kann,
oder
- R¹⁹ und R²⁰: zusammen mit dem Kohlenstoffatom, an das sie gebunden sind, einen 3-bis 5-gliedrigen Carbocyclus bilden,
worin der 3- bis 5-gliedrige Carbocyclus mit 1 oder 2 Substituenten unabhängig voneinander ausgewählt aus der Gruppe Fluor, Methyl und Ethyl substituiert sein kann,
oder
- R²¹ und R²²: zusammen mit dem Kohlenstoffatom, an das sie gebunden sind, einen 3-bis 5-gliedrigen Carbocyclus bilden,
worin der 3- bis 5-gliedrige Carbocyclus mit 1 oder 2 Substituenten unabhängig voneinander ausgewählt aus der Gruppe Fluor, Methyl und Ethyl substituiert sein kann,
oder
- R¹⁹ und R²¹: zusammen mit dem Kohlenstoffatom, an das sie gebunden sind, einen 3-bis 5-gliedrigen Carbocyclus bilden,
worin der 3- bis 5-gliedrige Carbocyclus mit 1 oder 2 Substituenten unabhängig voneinander ausgewählt aus der Gruppe Fluor, Methyl und Ethyl substituiert sein kann,
mit der Maßgabe, dass gleichzeitig nicht mehr als eines der Reste-Paare R¹⁹ und R²⁰, R²¹ und R²² bzw. R¹⁹ und R²¹ einen Carbo- oder Heterocyclus bildet,
- R²³: für (C₁-C₆)-Alkyl, 5- bis 9-gliedriges, über ein Ring-Kohlenstoffatom gebundenes Heterocyclyl, 5- bis 9-gliedriges Carbocyclyl, Phenyl, Indanyl oder 5- bis 10-gliedriges Heteroaryl steht,
wobei (C₁-C₆)-Alkyl mit Cyano oder bis zu dreifach mit Fluor substituiert sein kann,
wobei Phenyl mit 1 bis 3 Substituenten unabhängig voneinander ausgewählt aus der Gruppe Halogen, Cyano, Trifluormethyl, Difluormethyl, (C₁-C₄)-Alkyl, (C₁-C₄)-Alkoxy und 5- bis 10-gliedriges Heteroaryl substituiert sein kann,
worin (C₁-C₄)-Alkyl mit 1 oder 2 Substituenten unabhängig voneinander ausgewählt aus der Gruppe Fluor, Trifluormethoxy, (C₁-C₄)-Alkoxy, (C₃-C₆)-Cycloalkyl, Hydroxy und Amino substituiert sein kann,
wobei Indanyl mit 1 oder 2 Substituenten unabhängig voneinander ausgewählt aus der Gruppe Fluor, Trifluormethyl und Hydroxy substituiert sein kann,
wobei 5- bis 10-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, (C₁-C₄)-Alkoxy, Amino und Hydroxy substituiert sein kann,
worin (C₁-C₄)-Alkyl mit 1 bis 3 Substituenten unabhängig voneinander ausgewählt aus der Gruppe Halogen, Cyano, Hydroxy, Amino, Trifluormethyl, Difluormethyl, (C₁-C₄)-Alkoxy, Trifluormethoxy, Difluormethoxy und Phenyl substituiert sein kann,
worin Phenyl mit 1 bis 3 Substituenten Halogen substituiert sein kann,
wobei 5- bis 9-gliedriges, über ein Ring-Kohlenstoffatom gebundenes, Heterocyclyl mit 1 oder 2 Substituenten unabhängig voneinander ausgewählt aus der Gruppe Oxo, Fluor, Hydroxy und (C₁-C₄)-Alkyl substituiert sein kann, und
wobei 5- bis 9-gliedriges Carbocyclyl mit 1 oder 2 Substituenten unabhängig voneinander ausgewählt aus der Gruppe Trifluormethyl, Fluor, Hydroxy, Hydroxycarbonyl, (C₁-C₄)-Alkoxycarbonyl und (C₁-C₄)-Alkyl substituiert sein kann,
- R⁴: für Wasserstoff steht,
- R⁵: für Wasserstoff, Fluor, Chlor, Brom, Cyano, Difluormethyl, Trifluormethyl, (C₁-C₄)-Alkyl, (C₂-C₄)-Alkinyl oder (C₃-C₅)-Cycloalkyl steht,
- R⁶: für Wasserstoff oder Fluor steht,
sowie ihre *N-*Oxide, Salze, Solvate, Salze der *N-*Oxide und Solvate der *N*-Oxide und Salze.

Bevorzugt sind im Rahmen der vorliegenden Erfindung Verbindungen der Formel (I), in welcher
- A: für CH₂ oder CD₂ steht,
- R¹: für (C₃-C₆)-Cycloalkyl, Pyridyl oder Phenyl steht,
wobei (C₃-C₆)-Cycloalkyl mit 1 bis 2 Substituenten unabhängig voneinander ausgewählt aus der Gruppe Fluor, Trifluormethyl, Methyl und Ethyl substituiert sein kann,
wobei Pyridyl mit 1 oder 2 Substituenten Fluor substituiert ist,
und
wobei Phenyl mit 1 bis 4 Substituenten unabhängig voneinander ausgewählt aus der Gruppe Halogen, Cyano, Difluormethyl, Trifluormethyl, (C₁-C₄)-Alkyl, (C₁-C₄)-Alkoxy und (C₃-C₅)-Cycloalkyl substituiert sein kann,
- R²: für Wasserstoff, (C₁-C₄)-Alkyl, Cyclopropyl, Cyclobutyl, Difluormethyl oder Trifluormethyl steht,
- R³: für eine Gruppe der Formel 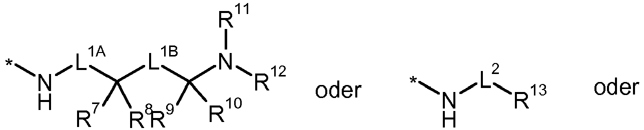 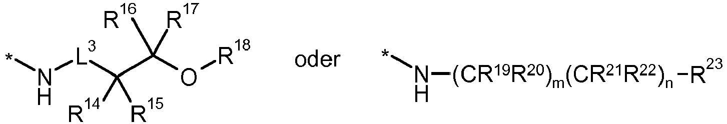
steht, wobei
- *: für die Anknüpfstelle an die Carbonylgruppe steht,
- L^{1A}: für eine Bindung, Methandiyl, 1,2-Ethandiyl oder 1,3-Propandiyl steht,
- L^{1B}: für eine Bindung, Methandiyl oder 1,2-Ethandiyl steht,
- L²: für eine Bindung, Methandiyl oder 1,2-Ethandiyl steht,
- L³: für eine Bindung, Methandiyl oder 1,2-Ethandiyl steht,
- R⁷: für Wasserstoff, (C₁-C₆)-Alkyl, (C₃-C₇)-Cycloalkyl, 5- oder 6-gliedriges Heteroaryl oder Phenyl steht,
worin (C₁-C₆)-Alkyl bis zu fünffach mit Fluor substituiert sein kann,
worin Phenyl und 5- oder 6-gliedriges Heteroaryl mit 1 bis 3 Substituenten unabhängig voneinander ausgewählt aus der Gruppe Fluor, Chlor, Brom, Cyano, Nitro, Trifluormethyl, Methyl, Ethyl, Methoxy, Ethoxy, Difluormethoxy, Trifluormethoxy, Methoxycarbonyl und Ethoxycarbonyl substituiert sein können,
und
worin Phenyl an zwei benachbarten Kohlenstoffatomen des Phenyls mit einer Methylendioxy-Brücke oder Ethylendioxy-Brücke substituiert sein kann,
oder
für Fluor stehen kann, wenn L^{1A} nicht für eine Bindung steht,
- R⁸: für Wasserstoff oder (C₁-C₄)-Alkyl steht,
oder
für Fluor stehen kann, wenn L^{1A} nicht für eine Bindung steht,
oder
- R⁷ und R⁸: zusammen mit dem Kohlenstoffatom, an das sie gebunden sind, einen 3-bis 7-gliedrigen Carbocyclus bilden,
- R⁹: für Wasserstoff, (C₁-C₆)-Alkyl, (C₃-C₅)-Cycloalkyl, Cyano, 5- bis 10-gliedriges Heteroaryl oder Phenyl steht,
worin (C₁-C₆)-Alkyl mit (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl, sowie bis zu fünffach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Fluor, Chlor, Brom, Methoxy und Ethoxy substituiert sein können,
worin (C₃-C₅)-Cycloalkyl mit 1 oder 2 Substituenten Fluor, Trifluormethyl, Methyl, Ethyl, Methoxy oder Ethoxy substituiert sein kann,
worin Phenyl und 5- bis 10-gliedriges Heteroaryl mit 1 bis 3 Substituenten unabhängig voneinander ausgewählt aus der Gruppe Fluor, Chlor, Brom, Cyano, Nitro, Trifluormethyl, Methyl, Ethyl, Ethenyl, Propenyl, Methoxy, Ethoxy, Difluormethoxy, Trifluormethoxy, Methoxycarbonyl und Ethoxycarbonyl substituiert sein können,
worin Methoxy und Ethoxy mit Hydroxy substituiert sein können, und
worin Phenyl an zwei benachbarten Kohlenstoffatomen des Phenyls mit einer Methylendioxy-Brücke oder Ethylendioxy-Brücke substituiert sein kann,
- R¹⁰: für Wasserstoff oder (C₁-C₄)-Alkyl steht,
oder
- R⁹ und R¹⁰: zusammen mit dem Kohlenstoffatom, an das sie gebunden sind, einen 3-bis 7-gliedrigen Carbocyclus oder einen 4- bis 7-gliedrigen Heterocyclus bilden,
oder
- R⁷ und R⁹: zusammen mit den Kohlenstoffatomen, an die sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus mit 1 oder 2 Substituenten (C₁-C₄)-Alkyl substituiert sein können,
und
worin der 3- bis 7-gliedrige Carbocyclus und der 4- bis 7-gliedrige Heterocyclus mit einem Phenyl-Ring oder einem Pyridyl-Ring anneliert sein können, die ihrerseits mit 1 oder 2 Substituenten ausgewählt aus Fluor, Chlor, Brom, Methyl, Ethyl und Trifluormethyl substituiert sein können,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
mit der Maßgabe, dass die Reste R⁷ und R⁹ nicht beide gleichzeitig für Phenyl oder 5- oder 6-gliedriges Heteroaryl stehen,
- R¹¹: für Wasserstoff oder (C₁-C₄)-Alkyl steht,
worin (C₁-C₄)-Alkyl bis zu fünffach mit Fluor substituiert sein kann,
- R¹²: für Wasserstoff, (C₁-C₆)-Alkyl, (C₃-C₅)-Cycloalkyl oder (C₁-C₄)-Alkylcarbonyl steht,
worin (C₁-C₆)-Alkyl bis zu fünffach mit Fluor substituiert sein kann,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit Methyl oder Ethyl substituiert sein kann,
- R¹³: für 5- bis 10-gliedriges, über ein Ring-Kohlenstoffatom gebundenes Aza-Heterocyclyl steht,
worin 5- bis 10-gliedriges, über ein Ring-Kohlenstoffatom gebundenes Aza-Heterocyclyl mit 1 oder 2 Substituenten Trifluormethyl, (C₃-C₇)-Cycloalkyl, Oxo und Benzyl sowie bis zu vierfach mit (C₁-C₄)-Alkyl und bis zu zweifach mit Fluor substituiert sein kann,
worin 5- bis 10-gliedriges Aza-Heterocyclyl mit einem Phenyl-Ring oder einem Pyridyl-Ring anneliert sein kann, die ihrerseits mit 1 oder 2 Substituenten ausgewählt aus Fluor, Chlor, Methyl, (C₁-C₄)-Alkoxy und Trifluormethyl substituiert sein können,
oder
für Amino stehen kann, wenn L² für eine Bindung steht,
worin Amino mit (C₁-C₄)-Alkyl, (C₁-C₄)-Alkylcarbonyl, (C₃-C₆)-Carbocyclyl, 4-bis 7-gliedriges Heterocyclyl, Phenyl oder 5- oder 6-gliedriges Heteroaryl substituiert sein kann,
worin (C₁-C₄)-Alkylcarbonyl mit Mono-alkylamino oder Di-alkylamino substituiert sein kann,
worin (C₃-C₆)-Carbocyclyl und 4- bis 7-gliedriges Heterocyclyl mit Hydroxy substituiert sein können,
und
worin Phenyl und 5- oder 6-gliedriges Heteroaryl mit 1 bis 3 Substituenten unabhängig voneinander ausgewählt aus der Gruppe Fluor, Chlor, Methyl und Trifluormethyl substituiert sein können,
- R¹⁴: für Wasserstoff, (C₁-C₆)-Alkyl, (C₃-C₇)-Cycloalkyl, -(C=O)NR²⁴R²⁵, 5- oder 6-gliedriges Heteroaryl oder Phenyl steht,
worin (C₁-C₆)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
worin R²⁴ für Wasserstoff, (C₁-C₄)-Alkyl, Aryl oder Naphthyl steht,
worin R²⁵ für Wasserstoff steht,
und
worin Phenyl und 5- oder 6-gliedriges Heteroaryl mit 1 bis 3 Substituenten unabhängig voneinander ausgewählt aus der Gruppe Fluor, Chlor, Brom, Trifluormethyl, Methyl und Ethyl substituiert sein können,
- R¹⁵: für Wasserstoff oder (C₁-C₆)-Alkyl steht,
worin (C₁-C₄)-Alkyl mit Hydroxy substituiert sein kann,
oder
- R¹⁴ und R¹⁵: zusammen mit dem Kohlenstoffatom, an das sie gebunden sind, einen 3-bis 7-gliedrigen Carbocyclus bilden,
- R¹⁶: für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
- R¹⁷: für Wasserstoff oder (C₁-C₆)-Alkyl steht,
oder
- R¹⁶ und R¹⁷: zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor, Methyl und Ethyl substituiert sein kann,
oder
- R¹⁴ und R¹⁶: zusammen mit den Kohlenstoffatomen, an die sie gebunden sind, einen 3-bis 7-gliedrigen Carbocyclus oder einen 4- bis 7-gliedrigen Heterocyclus bilden,
mit der Maßgabe, dass gleichzeitig nicht mehr als eines der Reste-Paare R¹⁴ und R¹⁵, R¹⁶ und R¹⁷ bzw. R¹⁴ und R¹⁶ einen Carbo- oder Heterocyclus bildet,
- R¹⁸: für Wasserstoff oder (C₁-C₄)-Alkyl steht, worin (C₁-C₄)-Alkyl mit 1 bis 5 Substituenten Fluor substituiert sein kann,
- m: für 0 oder 1 steht,
- n: für 0 oder 1 steht,
- R¹⁹: für Wasserstoff, Cyano oder (C₁-C₄)-Alkyl steht,
worin (C₁-C₄)-Alkyl mit 1 bis 5 Substituenten Fluor substituiert sein kann,
- R²⁰: für Wasserstoff oder (C₁-C₄)-Alkyl steht,
worin (C₁-C₄)-Alkyl mit 1 bis 5 Substituenten Fluor substituiert sein kann,
- R²¹: für Wasserstoff oder (C₁-C₄)-Alkyl steht,
worin (C₁-C₄)-Alkyl mit 1 bis 5 Substituenten Fluor substituiert sein kann,
- R²²: für Wasserstoff oder (C₁-C₄)-Alkyl steht,
worin (C₁-C₄)-Alkyl mit 1 bis 5 Substituenten Fluor substituiert sein kann,
oder
- R¹⁹ und R²⁰: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R²¹ und R²²: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R¹⁹ und R²¹: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass gleichzeitig nicht mehr als eines der Reste-Paare R¹⁹ und R²⁰, R²¹ und R²² bzw. R¹⁹ und R²¹ einen Carbo- oder Heterocyclus bildet,
- R²³: für (C₁-C₆)-Alkyl, (C₁-C₆)-Alkoxy, (C₁-C₄)-Alkoxycarbonyl, Hydroxycarbonyl, Aminocarbonyl, Aminosulfonyl, 5- bis 10-gliedriges, über ein Ring-Kohlenstoffatom gebundenes Heterocyclyl, 5- bis 10-gliedriges Carbocyclyl, Phenyl oder 5- bis 10-gliedriges Heteroaryl steht,
worin (C₁-C₆)-Alkyl mit Cyano sowie bis zu sechsfach mit Fluor substituiert sein kann,
worin (C₁-C₆)-Alkoxy mit Hydroxy oder (C₂-C₄)-Alkenyl substituiert sein kann,
worin Aminocarbonyl mit (C₁-C₆)-Alkyl oder (C₃-C₆)-Cycloalkyl substituiert sein kann,
worin Aminosulfonyl mit (C₁-C₆)-Alkyl oder (C₃-C₆)-Cycloalkyl substituiert sein kann,
worin Phenyl mit 1 bis 3 Substituenten unabhängig voneinander ausgewählt aus der Gruppe Halogen, Cyano, Trifluormethyl, Difluormethyl, (C₁-C₄)-Alkyl, (C₁-C₄)-Alkoxy, 5- bis 10-gliedriges Heteroaryl und 4- bis 7-gliedriges Heterocyclyl substituiert sein kann,
worin (C₁-C₆)-Alkyl mit 1 oder 2 Substituenten unabhängig voneinander ausgewählt aus der Gruppe Fluor, Trifluormethoxy, (C₁-C₄)-Alkoxy, (C₃-C₆)-Cycloalkyl, Hydroxy und Amino substituiert sein kann,
worin 5- bis 10-gliedriges Heteroaryl mit 1 bis 3 Substituenten unabhängig voneinander ausgewählt aus der Gruppe Fluor, Chlor, Cyano, (C₁-C₄)-Alkyl, Trifluormethyl, (C₁-C₄)-Alkoxy und Amino, substituiert sein kann,
worin (C₁-C₄)-Alkyl mit 1 bis 3 Substituenten unabhängig voneinander ausgewählt aus der Gruppe Halogen, Cyano, Hydroxy, Amino, Trifluormethyl, Difluormethyl, (C₁-C₄)-Alkoxy, Trifluormethoxy, Difluormethoxy und Phenyl substituiert sein kann,
worin Phenyl mit 1 bis 3 Substituenten Halogen substituiert sein kann,
worin 5- bis 10-gliedriges, über ein Ring-Kohlenstoffatom gebundenes, Heterocyclyl mit 1 oder 2 Substituenten unabhängig voneinander ausgewählt aus der Gruppe Oxo, Fluor,Trifluormethyl, Hydroxy und (C₁-C₄)-Alkyl substituiert sein kann,
worin 5- bis 10-gliedriges über ein Ring-Kohlenstoffatom gebundenes, Heterocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
und
worin 5- bis 10-gliedriges Carbocyclyl mit 1 oder 2 Substituenten unabhängig voneinander ausgewählt aus der Gruppe Trifluormethyl, Fluor, Cyano, Hydroxy, Hydroxycarbonyl, (C₁-C₄)-Alkoxycarbonyl, Amino und (C₁-C₄)-Alkyl substituiert sein kann,
worin 5- bis 10-gliedriges Carbocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
- R⁴: für Wasserstoff steht,
- R⁵: für Wasserstoff, Fluor, Chlor, Brom, Cyano, Difluormethyl, Trifluormethyl, (C₁-C₄)-Alkyl, (C₃-C₅)-Cycloalkyl oder (C₂-C₄)-Alkinyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ oder CD₂ steht,
- R¹: für (C₃-C₆)-Cycloalkyl, Pyridyl oder Phenyl steht,
wobei (C₃-C₆)-Cycloalkyl mit 1 bis 2 Substituenten unabhängig voneinander ausgewählt aus der Gruppe Fluor, Trifluormethyl, Methyl und Ethyl substituiert sein kann,
wobei Pyridyl mit 1 oder 2 Substituenten Fluor substituiert ist,
und
wobei Phenyl mit 1 bis 4 Substituenten unabhängig voneinander ausgewählt aus der Gruppe Halogen, Cyano, Difluormethyl, Trifluormethyl, (C₁-C₄)-Alkyl, (C₁-C₄)-Alkoxy und (C₃-C₅)-Cycloalkyl substituiert sein kann,
- R²: für Wasserstoff, (C₁-C₄)-Alkyl, Cyclopropyl, Cyclobutyl, Difluormethyl oder Trifluor-methyl steht,
- R³: für eine Gruppe der Formel 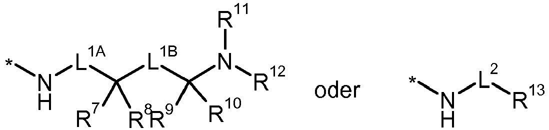
oder 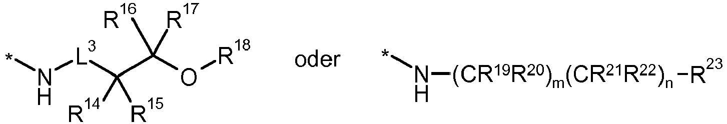 steht, wobei
- *: für die Anknüpfstelle an die Carbonylgruppe steht,
- L^{1A}: für eine Bindung, Methandiyl, 1,2-Ethandiyl oder 1,3-Propandiyl steht,
- L^{1B}: für eine Bindung, Methandiyl oder 1,2-Ethandiyl steht,
- L²: für eine Bindung, Methandiyl oder 1,2-Ethandiyl steht,
- L³: für eine Bindung, Methandiyl oder 1,2-Ethandiyl steht,
- R⁷: für Wasserstoff, (C₁-C₆)-Alkyl, (C₃-C₇)-Cycloalkyl, 5- oder 6-gliedriges Heteroaryl oder Phenyl steht,
worin (C₁-C₆)-Alkyl bis zu fünffach mit Fluor substituiert sein kann,
worin Phenyl und 5- oder 6-gliedriges Heteroaryl mit 1 bis 3 Substituenten unabhängig voneinander ausgewählt aus der Gruppe Fluor, Chlor, Brom, Cyano, Nitro, Trifluormethyl, Difluormethyl, Methyl, Ethyl, Methoxy, Ethoxy, Difluormethoxy, Trifluormethoxy, Methoxycarbonyl und Ethoxycarbonyl substituiert sein können,
und
worin Phenyl an zwei benachbarten Kohlenstoffatomen des Phenyls mit einer Methylendioxy-Brücke oder Ethylendioxy-Brücke substituiert sein kann,
oder
für Fluor stehen kann, wenn L^{1A} nicht für eine Bindung steht,
- R⁸: für Wasserstoff oder (C₁-C₄)-Alkyl steht,
oder
für Fluor stehen kann, wenn L^{1A} nicht für eine Bindung steht,
oder
- R⁷ und R⁸: zusammen mit dem Kohlenstoffatom, an das sie gebunden sind, einen 3-bis 7-gliedrigen Carbocyclus bilden,
- R⁹: für Wasserstoff, (C₁-C₆)-Alkyl, (C₃-C₅)-Cycloalkyl, Cyano, 5- bis 10-gliedriges Heteroaryl oder Phenyl steht,
worin (C₁-C₆)-Alkyl mit (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl, sowie bis zu fünffach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Fluor, Chlor, Brom, Methoxy und Ethoxy substituiert sein können,
worin (C₃-C₅)-Cycloalkyl mit 1 oder 2 Substituenten Fluor, Trifluormethyl, Methyl, Ethyl, Methoxy oder Ethoxy substituiert sein kann,
worin Phenyl und 5- bis 10-gliedriges Heteroaryl mit 1 bis 3 Substituenten unabhängig voneinander ausgewählt aus der Gruppe Fluor, Chlor, Brom, Cyano, Nitro, Trifluormethyl, Methyl, Ethyl, Ethenyl, Propenyl, Methoxy, Ethoxy, Difluormethoxy, Trifluormethoxy, Methoxycarbonyl und Ethoxycarbonyl substituiert sein können,
worin Methoxy und Ethoxy mit Hydroxy substituiert sein können,
und
worin Phenyl an zwei benachbarten Kohlenstoffatomen des Phenyls mit einer Methylendioxy-Brücke oder Ethylendioxy-Brücke substituiert sein kann,
- R¹⁰: für Wasserstoff oder (C₁-C₄)-Alkyl steht,
oder
- R⁹ und R¹⁰: zusammen mit dem Kohlenstoffatom, an das sie gebunden sind, einen 3-bis 7-gliedrigen Carbocyclus oder einen 4- bis 7-gliedrigen Heterocyclus bilden,
oder
- R⁷ und R⁹: zusammen mit den Kohlenstoffatomen, an die sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus mit 1 oder 2 Substituenten (C₁-C₄)-Alkyl substituiert sein können,
und
worin der 3- bis 7-gliedrige Carbocyclus und der 4- bis 7-gliedrige Heterocyclus mit einem Phenyl-Ring oder einem Pyridyl-Ring anneliert sein können, die ihrerseits mit 1 oder 2 Substituenten ausgewählt aus Fluor, Chlor, Brom, Methyl, Ethyl und Trifluormethyl substituiert sein können,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
mit der Maßgabe, dass die Reste R⁷ und R⁹ nicht beide gleichzeitig für Phenyl oder 5- oder 6-gliedriges Heteroaryl stehen,
- R¹¹: für Wasserstoff oder (C₁-C₄)-Alkyl steht,
worin (C₁-C₄)-Alkyl bis zu fünffach mit Fluor substituiert sein kann,
- R¹²: für Wasserstoff, (C₁-C₆)-Alkyl, (C₃-C₅)-Cycloalkyl oder (C₁-C₄)-Alkylcarbonyl steht,
worin (C₁-C₆)-Alkyl bis zu fünffach mit Fluor substituiert sein kann,
oder
- R¹¹ und R¹²: zusammen mit dem Sticktoffatom, an das sie gebunden sind, einen 4- bis 7-gliedrigen Aza-Heterocyclus bilden,
worin der 4- bis 7-gliedrige Aza-Heterocyclus mit Methyl oder Ethyl substituiert sein kann,
- R¹³: für 5- bis 10-gliedriges, über ein Ring-Kohlenstoffatom gebundenes Aza-Heterocyclyl oder über ein Ring-Stickstoffatom gebundenes 5- oder 6-gliedriges Heterocyclyl steht,
worin 5- bis 10-gliedriges, über ein Ring-Kohlenstoffatom gebundenes Aza-Heterocyclyl mit 1 oder 2 Substituenten Trifluormethyl, (C₃-C₇)-Cycloalkyl, Oxo und Benzyl sowie bis zu vierfach mit (C₁-C₄)-Alkyl und bis zu zweifach mit Fluor substituiert sein kann,
worin 5- bis 10-gliedriges Aza-Heterocyclyl mit einem Phenyl-Ring oder einem Pyridyl-Ring anneliert sein kann, die ihrerseits mit 1 oder 2 Substituenten ausgewählt aus Fluor, Chlor, Methyl, (C₁-C₄)-Alkoxy und Trifluormethyl substituiert sein können,
worin über ein Ring-Stickstoffatom gebundenes 5- oder 6-gliedriges Heterocyclyl mit (C₁-C₄)-Alkyl oder (C₁-C₄)-Cycloalkyl substituiert sein kann,
oder
für Amino stehen kann, wenn L² für eine Bindung steht,
worin Amino mit (C₁-C₄)-Alkyl, (C₁-C₄)-Alkylcarbonyl, (C₃-C₆)-Carbocyclyl, 4-bis 7-gliedriges Heterocyclyl, Phenyl oder 5- oder 6-gliedriges Heteroaryl substituiert sein kann,
worin (C₁-C₄)-Alkylcarbonyl mit Mono-alkylamino, Di-alkylamino oder über ein Ring-Stickstoffatom gebundenes 5- oder 6-gliedriges Heterocyclyl substituiert sein kann,
worin über ein Ring-Stickstoffatom gebundenes 5- oder 6-gliedriges Heterocyclyl mit Oxo substituiert sein kann,
worin (C₃-C₆)-Carbocyclyl und 4- bis 7-gliedriges Heterocyclyl mit Hydroxy substituiert sein können,
und
worin Phenyl und 5- oder 6-gliedriges Heteroaryl mit 1 bis 3 Substituenten unabhängig voneinander ausgewählt aus der Gruppe Fluor, Chlor, Methyl und Trifluormethyl substituiert sein können,
- R¹⁴: für Wasserstoff, (C₁-C₆)-Alkyl, (C₃-C₇)-Cycloalkyl, -(C=O)NR²⁴R²⁵, 5- oder 6-gliedriges Heteroaryl oder Phenyl steht,
worin (C₁-C₆)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
worin R²⁴ für Wasserstoff, (C₁-C₄)-Alkyl, Aryl oder Naphthyl steht,
worin R²⁵ für Wasserstoff steht,
und
worin Phenyl und 5- oder 6-gliedriges Heteroaryl mit 1 bis 3 Substituenten unabhängig voneinander ausgewählt aus der Gruppe Fluor, Chlor, Brom, Trifluormethyl, Difluormethyl, Methyl, Ethyl und 1-Amino-2-methylpropyl substituiert sein können,
- R¹⁵: für Wasserstoff oder (C₁-C₆)-Alkyl steht,
worin (C₁-C₄)-Alkyl mit Hydroxy substituiert sein kann,
oder
- R¹⁴ und R¹⁵: zusammen mit dem Kohlenstoffatom, an das sie gebunden sind, einen 3-bis 7-gliedrigen Carbocyclus bilden,
- R¹⁶: für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
- R¹⁷: für Wasserstoff oder (C₁-C₆)-Alkyl steht,
oder
- R¹⁶ und R¹⁷: zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor, Methyl und Ethyl substituiert sein kann,
oder
- R¹⁴ und R¹⁶: zusammen mit den Kohlenstoffatomen, an die sie gebunden sind, einen 3-bis 7-gliedrigen Carbocyclus oder einen 4- bis 7-gliedrigen Heterocyclus bilden,
mit der Maßgabe, dass gleichzeitig nicht mehr als eines der Reste-Paare R¹⁴ und R¹⁵, R¹⁶ und R¹⁷ bzw. R¹⁴ und R¹⁶ einen Carbo- oder Heterocyclus bildet,
- R¹⁸: für Wasserstoff oder (C₁-C₄)-Alkyl steht,
worin (C₁-C₄)-Alkyl mit 1 bis 5 Substituenten Fluor substituiert sein kann,
- m: für 0 oder 1 steht,
- n: für 0 oder 1 steht,
- R¹⁹: für Wasserstoff, Cyano oder (C₁-C₄)-Alkyl steht,
worin (C₁-C₄)-Alkyl mit 1 bis 5 Substituenten Fluor substituiert sein kann,
- R²⁰: für Wasserstoff oder (C₁-C₄)-Alkyl steht,
worin (C₁-C₄)-Alkyl mit 1 bis 5 Substituenten Fluor substituiert sein kann,
- R²¹: für Wasserstoff oder (C₁-C₄)-Alkyl steht,
worin (C₁-C₄)-Alkyl mit 1 bis 5 Substituenten Fluor substituiert sein kann,
- R²²: für Wasserstoff oder (C₁-C₄)-Alkyl steht,
worin (C₁-C₄)-Alkyl mit 1 bis 5 Substituenten Fluor substituiert sein kann,
oder
- R¹⁹ und R²⁰: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R²¹ und R²²: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
- R¹⁹ und R²¹: zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass gleichzeitig nicht mehr als eines der Reste-Paare R¹⁹ und R²⁰, R²¹ und R²² bzw. R¹⁹ und R²¹ einen Carbo- oder Heterocyclus bildet,
- R²³: für (C₁-C₆)-Alkyl, (C₁-C₆)-Alkoxy, (C₁-C₄)-Alkoxycarbonyl, Hydroxycarbonyl, Aminocarbonyl, Aminosulfonyl, 5- bis 10-gliedriges, über ein Ring-Kohlenstoffatom gebundenes Heterocyclyl, 5- bis 10-gliedriges Carbocyclyl, Phenyl oder 5- bis 10-gliedriges Heteroaryl steht,
worin (C₁-C₆)-Alkyl mit Cyano sowie bis zu sechsfach mit Fluor substituiert sein kann,
worin (C₁-C₆)-Alkoxy mit Hydroxy oder (C₂-C₄)-Alkenyl substituiert sein kann,
worin Aminocarbonyl mit (C₁-C₆)-Alkyl oder (C₃-C₆)-Cycloalkyl substituiert sein kann,
worin Aminosulfonyl mit (C₁-C₆)-Alkyl oder (C₃-C₆)-Cycloalkyl substituiert sein kann,
worin Phenyl mit 1 bis 3 Substituenten unabhängig voneinander ausgewählt aus der Gruppe Halogen, Cyano, Trifluormethyl, Difluormethyl, (C₁-C₄)-Alkyl, (C₁-C₄)-Alkoxy, 5- bis 10-gliedriges Heteroaryl und 4- bis 7-gliedriges Heterocyclyl substituiert sein kann,
worin (C₁-C₆)-Alkyl mit 1 oder 2 Substituenten unabhängig voneinander ausgewählt aus der Gruppe Fluor, Trifluormethoxy, (C₁-C₄)-Alkoxy, (C₃-C₆)-Cycloalkyl, Hydroxy und Amino substituiert sein kann,
worin 5- bis 10-gliedriges Heteroaryl mit 1 bis 3 Substituenten unabhängig voneinander ausgewählt aus der Gruppe Fluor, Chlor, Cyano, (C₁-C₄)-Alkyl, Trifluormethyl, (C₁-C₄)-Alkoxy und Amino, substituiert sein kann,
worin (C₁-C₄)-Alkyl mit 1 bis 3 Substituenten unabhängig voneinander ausgewählt aus der Gruppe Halogen, Cyano, Hydroxy, Amino, Trifluormethyl, Difluormethyl, (C₁-C₄)-Alkoxy, Trifluormethoxy, Difluormethoxy und Phenyl substituiert sein kann,
worin Phenyl mit 1 bis 3 Substituenten Halogen substituiert sein kann,
worin 5- bis 10-gliedriges, über ein Ring-Kohlenstoffatom gebundenes, Heterocyclyl mit 1 oder 2 Substituenten unabhängig voneinander ausgewählt aus der Gruppe Oxo, Fluor,Trifluormethyl, Hydroxy und (C₁-C₄)-Alkyl substituiert sein kann,
worin 5- bis 10-gliedriges über ein Ring-Kohlenstoffatom gebundenes, Heterocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
und
worin 5- bis 10-gliedriges Carbocyclyl mit 1 oder 2 Substituenten unabhängig voneinander ausgewählt aus der Gruppe Trifluormethyl, Fluor, Cyano, Hydroxy, Hydroxycarbonyl, (C₁-C₄)-Alkoxycarbonyl, Amino und (C₁-C₄)-Alkyl substituiert sein kann,
worin 5- bis 10-gliedriges Carbocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
- R⁴: für Wasserstoff steht,
- R⁵: für Wasserstoff, Fluor, Chlor, Brom, Cyano, Difluormethyl, Trifluormethyl, (C₁-C₄)-Alkyl, Methoxy, (C₃-C₅)-Cycloalkyl oder (C₂-C₄)-Alkinyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für Cyclohexyl, Pyridyl oder Phenyl steht,
wobei Cyclohexyl mit 1 bis 2 Substituenten unabhängig voneinander ausgewählt aus der Gruppe Fluor und Methyl substituiert sein kann,
wobei Pyridyl mit 1 oder 2 Substituenten F substituiert ist,
und
wobei Phenyl mit 1 bis 4 Substituenten unabhängig voneinander ausgewählt aus der Gruppe Fluor, Chlor, Methyl, Methoxy und Cyclopropyl substituiert sein kann,
- R²: für Methyl, Cyclopropyl oder Trifluormethyl steht,
- R³: für eine Gruppe der Formel 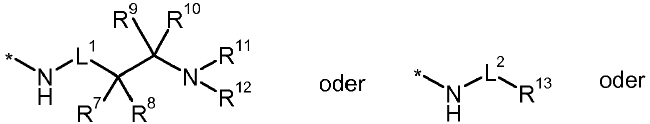 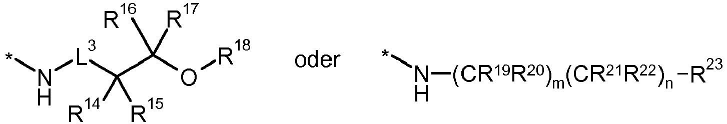
steht, wobei
- *: für die Anknüpfstelle an die Carbonylgruppe steht,
- L¹: für eine Bindung steht,
- L²: für eine Bindung steht,
- L³: für eine Bindung steht,
- R⁷: für Wasserstoff, (C₁-C₆)-Alkyl oder Phenyl steht,
worin (C₁-C₆)-Alkyl mit 1 bis 5 Substituenten Fluor substituiert sein kann,
und
worin Phenyl mit 1 bis 3 Substituenten unabhängig voneinander ausgewählt aus der Gruppe Fluor, Chlor, Trifluormethyl, Methyl oder Methoxy substituiert sein kann,
- R⁸: für Wasserstoff, Methyl oder Ethyl steht,
- R⁹: für Wasserstoff, (C₁-C₆)-Alkyl, Cyclopropyl oder Cyclobutyl steht,
worin (C₁-C₆)-Alkyl mit 1 bis 5 Substituenten Fluor substituiert sein kann,
- R¹⁰: für Wasserstoff oder (C₁-C₄)-Alkyl steht,
oder
- R⁹ und R¹⁰: zusammen mit dem Kohlenstoffatom, an das sie gebunden sind, einen 3-bis 6-gliedrigen Carbocyclus bilden,
- R¹¹: für Wasserstoff, Methyl oder Ethyl steht, worin Ethyl mit 1 bis 3 Substituenten Fluor substituiert sein kann,
- R¹²: für Wasserstoff, (C₁**-**C₄)-Alkyl oder (C₃-C₅)-Cycloalkyl steht,
oder
- R¹¹ und R¹²: zusammen mit dem Sticktoffatom, an das sie gebunden sind, einen Morpholinyl-Ring oder Piperidinyl-Ring bilden,
- R¹³: für 9-Azabicyclo[3.3.1]nonan-3-yl oder Piperidin-4-yl steht,
worin 9-Azabicyclo[3.3.1]nonan-3-yl mit Methyl substituiert ist,
worin Piperidin-4-yl mit 1 bis 5 Substituenten Methyl substituiert ist,
- R¹⁴: für Wasserstoff, (C₁-C₆)-Alkyl, -(C=O)NR²⁴R²⁵ oder Phenyl steht,
worin (C₁-C₆)-Alkyl mit einem Rest Hydroxy oder Methoxy oder bis zu fünffach mit Fluor substituiert sein kann,
worin R²⁴ für Aryl oder Naphthyl steht,
worin R²⁵ für Wasserstoff steht
und
worin Phenyl mit 1 bis 3 Substituenten unabhängig voneinander ausgewählt aus der Gruppe Fluor, Chlor, Trifluormethyl und Methyl substituiert sein kann,
- R¹⁵: für Wasserstoff oder (C₁-C₄)-Alkyl steht,
- R¹⁶: für Wasserstoff, (C₁-C₆)-Alkyl, Cyclopropyl oder Cyclobutyl steht, worin (C₁-C₆)-Alkyl mit 1 bis 5 Substituenten Fluor substituiert sein kann,
worin Cyclopropyl und Cyclobutyl mit 1 oder 2 Substituenten unabhängig voneinander ausgewählt aus der Gruppe Fluor oder Methyl substituiert sein können,
- R¹⁷: für Wasserstoff oder (C₁-C₄)-Alkyl steht,
oder
- R¹⁶ und R¹⁷: zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten Fluor oder Methyl substituiert sein kann,
- R¹⁸: für Wasserstoff oder (C₁-C₄)-Alkyl steht,
worin (C₁-C₃)-Alkyl mit 1 bis 5 Substituenten Fluor substituiert sein kann,
- m: für 0 oder 1 steht,
- n: für 0 oder 1 steht,
- R¹⁹: für Wasserstoff, Cyano oder Methyl steht,
worin Methyl mit 1 bis 3 Substituenten Fluor substituiert sein kann,
- R²⁰: für Wasserstoff oder Methyl steht,
worin Methyl mit 1 bis 3 Substituenten Fluor substituiert sein kann,
- R²¹: für Wasserstoff oder Methyl steht,
worin Methyl mit 1 bis 3 Substituenten Fluor substituiert sein kann,
- R²²: für Wasserstoff oder Methyl steht,
worin Methyl mit 1 bis 3 Substituenten Fluor substituiert sein kann,
oder
- R¹⁹ und R²⁰: zusammen mit dem Kohlenstoffatom, an das sie gebunden sind, einen 3-bis 5-gliedrigen Carbocyclus bilden,
oder
- R¹⁹ und R²¹: zusammen mit dem Kohlenstoffatom, an das sie gebunden sind, einen Cyclopropyl-Ring bilden,
mit der Maßgabe, dass gleichzeitig nicht mehr als eines der Reste-Paare R¹⁹ und R²⁰ bzw. R¹⁹ und R²¹ einen Carbocyclus bildet,
- R²³: für (C₁-C₆)-Alkyl, 2-Oxopyrrolidin-3-yl, 2-Oxotetrahydrofuran-3-yl, Cyclopentyl, Cyclohexyl, Phenyl, Indanyl, 1,2,4-Oxadiazol-5-yl, 1H-Imidazol-2-yl, 1H-Pyrazol-4-yl, Pyridin-3-yl, Pyrimidin-5-yl, Chinolin-4-yl oder Pyrazolo[1,5-a]pyridin-3-yl, steht,
wobei (C₁-C₆)-Alkyl mit Cyano oder bis zu dreifach mit Fluor substituiert sein kann,
wobei Phenyl mit 1 bis 3 Substituenten unabhängig voneinander ausgewählt aus der Gruppe Fluor, Chlor, Cyano, Trifluormethyl, Methyl, Ethyl, Methoxy und Pyridyl substituiert sein kann,
wobei Indanyl mit Hydroxy substituiert ist,
wobei 1,2,4-Oxadiazol-5-yl, 1H-Imidazol-2-yl, 1H-Pyrazol-4-yl, Pyridin-3-yl, Pyrimidin-5-yl, Chinolin-4-yl oder Pyrazolo[1,5-a]pyridin-3-yl mit 1 bis 3 Substituenten unabhängig voneinander ausgewählt aus der Gruppe Fluor, Chlor, Trifluormethyl, (C₁-C₃)-Alkyl, Amino und Hydroxy substituiert sein können,
worin (C₁-C₃)-Alkyl mit Fluor, Hydroxy, Amino oder Trifluormethyl, substituiert sein kann,
wobei Cyclopentyl und Cyclohexyl mit Methoxycarbonyl oder Ethoxycarbonyl substituiert sind,
- R⁴: für Wasserstoff steht,
- R⁵: für Wasserstoff, Fluor, Chlor, Methyl oder Ethyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für Cyclohexyl, Pyridyl oder Phenyl steht,
wobei Pyridyl mit 1 oder 2 Substituenten F substituiert ist,
und
wobei Phenyl mit 1 bis 4 Substituenten unabhängig voneinander ausgewählt aus der Gruppe Fluor, Chlor, Cyano, Methyl, Ethyl, Methoxy, Ethoxy und Cyclopropyl substituiert sein kann,
- R²: für Methyl, Cyclopropyl, Difluormethyl oder Trifluormethyl steht,
- R³: für eine Gruppe der Formel 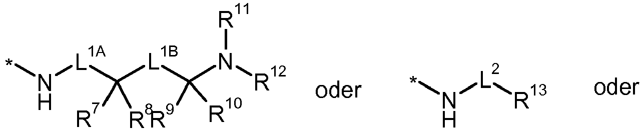 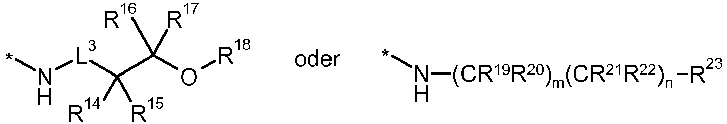 steht, wobei
* für die Anknüpfstelle an die Carbonylgruppe steht,
L^{1A} für eine Bindung oder Methandiyl steht,
L^{1B} für eine Bindung steht,
L² für eine Bindung steht,
L³ für eine Bindung steht,
R⁷ für Wasserstoff, (C₁-C₆)-Alkyl oder Phenyl steht,
worin (C₁-C₆)-Alkyl bis zu fünffach mit Fluor substituiert sein kann,
worin Phenyl mit 1 bis 3 Substituenten unabhängig voneinander ausgewählt aus der Gruppe Fluor, Chlor, Brom, Trifluormethyl, Methyl, Methoxy, Difluormethoxy und Trifluormethoxy substituiert sein können,
und
worin Phenyl an zwei benachbarten Kohlenstoffatomen des Phenyls mit einer Methylendioxy-Brücke oder Ethylendioxy-Brücke substituiert sein kann,
oder
für Fluor stehen kann, wenn L^{1A} nicht für eine Bindung steht,
R⁸ für Wasserstoff, Methyl oder Ethyl steht,
oder
für Fluor stehen kann, wenn L^{1A} nicht für eine Bindung steht,
R⁹ für Wasserstoff, (C₁-C₆)-Alkyl, Cyclopropyl oder Cyclobutyl steht,
worin (C₁-C₆)-Alkyl bis zu fünffach mit Fluor substituiert sein kann,
worin Cyclopropyl und Cyclobutyl mit Trifluormethyl substituiert sein können,
R¹⁰ für Wasserstoff oder (C₁-C₄)-Alkyl steht,
oder
- R⁹ und R¹⁰: zusammen mit dem Kohlenstoffatom, an das sie gebunden sind, einen 3-bis 7-gliedrigen Carbocyclus bilden,
- R¹¹: für Wasserstoff, Methyl oder Ethyl steht,
worin Ethyl bis zu dreifach mit Fluor substituiert sein kann,
- R¹²: für Wasserstoff, (C₁-C₄)-Alkyl, Cyclopropyl oder Cyclobutyl steht,
worin (C₁-C₆)-Alkyl bis zu fünffach mit Fluor substituiert sein kann,
oder
- R¹¹ und R¹²: zusammen mit dem Sticktoffatom, an das sie gebunden sind, einen Morpholinyl-Ring bilden,
- R¹³: für 9-Azabicyclo[3.3.1]nonan-3-yl, Pyrrolidinyl, Piperidin-4-yl, Azepanyl oder 1,2,3,4-Tetrahydrochinolinyl steht,
worin Piperidin-4-yl mit 1 bis 5 Substituenten Methyl substituiert sein kann sowie bis zu zweifach mit Fluor substituiert sein kann,
worin1,2,3,4-Tetrahydrochinolinyl mit 1 bis 2 Substituenten unabhängig voneinander ausgewählt aus der Gruppe Fluor, Oxo, Methyl, Ethyl, Methoxy, Ethoxy und Trifluormethyl substituiert sein kann, worin 9-Azabicyclo[3.3.1]nonan-3-yl mit Methyl substituiert sein kann,
worin Pyrrolidinyl mit 1 bis 2 Substituenten unabhängig voneinander ausgewählt aus der Gruppe Fluor, Oxo, Methyl und Ethyl substituiert sein kann,
- R¹⁴: für Wasserstoff, (C₁-C₆)-Alkyl, -(C=O)NR²⁴R²⁵ oder Phenyl steht,
worin (C₁-C₆)-Alkyl mit Hydroxy oder Methoxy sowie bis zu fünffach mit Fluor substituiert sein kann,
worin R²⁴ für Aryl oder Naphthyl steht,
worin R²⁵ für Wasserstoff steht,
und
worin Phenyl mit 1 oder 2 Substituenten unabhängig voneinander ausgewählt aus der Gruppe Fluor, Chlor, Trifluormethyl und Methyl substituiert sein kann,
- R¹⁵: für Wasserstoff oder (C₁-C₆)-Alkyl steht,
- R¹⁶: für Wasserstoff, (C₁-C₆)-Alkyl, Cyclopropyl oder Cyclobutyl steht,
worin (C₁-C₆)-Alkyl bis zu sechsfach mit Fluor substituiert sein kann,
- R¹⁷: für Wasserstoff oder (C₁-C₆)-Alkyl steht,
oder
- R¹⁶ und R¹⁷: zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann,
- R¹⁸: für Wasserstoff oder (C₁-C₄)-Alkyl steht,
worin (C₁-C₄)-Alkyl mit 1 bis 5 Substituenten Fluor substituiert sein kann,
- m: für 0 oder 1 steht,
- n: für 0 oder 1 steht,
- R¹⁹: für Wasserstoff, Cyano oder Methyl steht,
worin Methyl mit 1 bis 3 Substituenten Fluor substituiert sein kann,
- R²⁰: für Wasserstoff oder Methyl steht,
worin Methyl mit 1 bis 3 Substituenten Fluor substituiert sein kann,
- R²¹: für Wasserstoff oder Methyl steht,
worin Methyl mit 1 bis 3 Substituenten Fluor substituiert sein kann,
- R²²: für Wasserstoff oder Methyl steht,
worin Methyl mit 1 bis 3 Substituenten Fluor substituiert sein kann,
oder
- R¹⁹ und R²⁰: zusammen mit dem Kohlenstoffatom, an das sie gebunden sind, einen 3-bis 5-gliedrigen Carbocyclus bilden,
oder
- R¹⁹ und R²¹: zusammen mit dem Kohlenstoffatom, an das sie gebunden sind, einen Cyclopropyl-Ring bilden,
mit der Maßgabe, dass gleichzeitig nicht mehr als eines der Reste-Paare R¹⁹ und R²⁰ bzw. R¹⁹ und R²¹ einen Carbocyclus bildet,
- R²³: für (C₁-C₆)-Alkyl, 2-Oxopyrrolidin-3-yl, 2-Oxotetrahydrofuran-3-yl, Cyclopentyl, Cyclohexyl, Indanyl, 1,2,4-Oxadiazol-5-yl, 1H-Imidazol-2-yl, 1H-Pyrazol-4-yl, Pyridin-3-yl, Pyrimidin-5-yl, Chinolin-4-yl, Pyrazolo[1,5-a]pyridin-3-yl, 3,4-Dihydro-2H-pyranyl, 1,2,3,4-Tetrahydronaphthalinyl, Bicyclo[2.2.2]octanyl, Chroman-4-yl, 2,3-Dihydro-1-benzofuran-3-yl, 2,3-Dihydro-1H-indenyl, 3,4-Dihydrochinolinyl, 3,4-Dihydro-2H-pyrano[2,3-b]pyridinyl oder Phenyl steht,
worin (C₁-C₆)-Alkyl mit Cyano sowie bis zu dreifach mit Fluor substituiert sein kann,
worin Phenyl mit 1 bis 3 Substituenten unabhängig voneinander ausgewählt aus der Gruppe Fluor, Chlor, Cyano, Trifluormethyl, Methyl, Ethyl, Methoxy, 1H-Imidazol-1-yl und Pyridyl substituiert sein kann,
worin 1,2,4-Oxadiazol-5-yl, 1H-Imidazol-2-yl, 1H-Pyrazol-4-yl, Pyridin-3-yl, Pyrimidin-5-yl, Chinolin-4-yl oder Pyrazolo[1,5-a]pyridin-3-yl mit 1 bis 3 Substituenten unabhängig voneinander ausgewählt aus der Gruppe Fluor, Chlor, Trifluormethyl, (C₁-C₃)-Alkyl, Amino und Hydroxy substituiert sein können,
worin (C₁-C₃)-Alkyl mit Fluor, Hydroxy, Amino oder Trifluormethyl, substituiert sein kann,
worin Cyclopentyl, Cyclohexyl und Bicyclo[2.2.2]octanyl mit Methoxycarbonyl, Ethoxycarbonyl, Hydroxycarbonyl und Cyano substiuiert sein können,
worin Chroman-4-yl, 2,3-Dihydro-1-benzofuran-3-yl, 2,3-Dihydro-1H-indenyl, 3,4-Dihydrochinolinyl und 3,4-Dihydro-2H-pyrano[2,3-b]pyridinyl mit 1 oder 2 Substituenten ausgewählt aus der Gruppe Fluor, Chlor, Methyl, Ethyl, Methoxy, Ethoxy und Trifluormethyl substituiert sein können,
und
worin 2,3-Dihydro-1H-indenyl und Indanyl mit 1 oder 2 Substituenten ausgewählt aus der Gruppe Fluor, Chlor, Methyl, Ethyl, Methoxy, Ethoxy, Hydroxy und Trifluormethyl substituiert sein können,
worin Methyl und Ethyl mit Hydroxy substituiert sein können,
- R⁴: für Wasserstoff steht,
- R⁵: für Wasserstoff, Fluor, Chlor, Methyl oder Ethyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für eine Phenyl-Gruppe der Formel 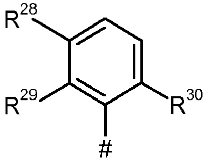
steht, wobei # für die Anknüpfstelle an A steht,
und
R²⁸ für Wasserstoff oder Fluor steht,
R²⁹ für Fluor steht,
R³⁰ für Fluor steht,
- R²: für Methyl steht,
- R³: für eine Gruppe der Formel 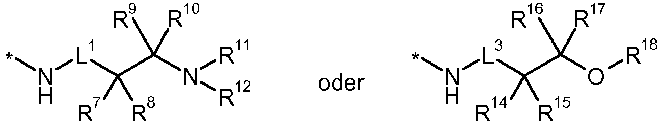 steht, wobei
* für die Anknüpfstelle an die Carbonylgruppe steht,
L¹ für eine Bindung steht,
L³ für eine Bindung steht,
R⁷ für Wasserstoff, (C₁-C₆)-Alkyl oder Phenyl steht,
worin (C₁-C₆)-Alkyl mit 1 bis 5 Substituenten Fluor substituiert sein kann,
und
worin Phenyl mit 1 bis 2 Substituenten Chlor oder Fluor substituiert sein kann,
R⁸ für Wasserstoff, Methyl oder Ethyl steht,
R⁹ für Wasserstoff, (C₁-C₆)-Alkyl, Trifluormethyl oder Cyclopropyl steht,
worin (C₁-C₆)-Alkyl mit 1 bis 3 Substituenten Fluor substituiert sein kann,
R¹⁰ für Wasserstoff, Methyl oder Ethyl steht,
R¹¹ für Wasserstoff steht,
R¹² für Wasserstoff steht,
R¹⁴ für Wasserstoff, (C₁-C₆)-Alkyl oder Phenyl steht,
worin (C₁-C₆)-Alkyl mit einem Rest Hydroxy oder bis zu fünffach mit Fluor substituiert sein kann,
und
worin Phenyl mit 1 oder 2 Substituenten Fluor substituiert sein kann,
R¹⁵ für Wasserstoff, Methyl oder Ethyl steht,
R¹⁶ für Wasserstoff oder (C₁-C₆)-Alkyl steht,
R¹⁷ für Wasserstoff, Methyl oder Ethyl steht,
oder
R ¹⁶ und R ¹⁷ zusammen mit dem Kohlenstoffatom, an das sie gebunden sind, einen 3-bis 6-gliedrigen Carbocyclus bilden,
R¹⁸ für Wasserstoff steht,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für eine Phenyl-Gruppe der Formel 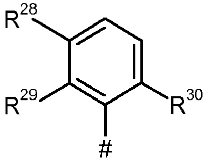 steht, wobei
# für die Anknüpfstelle an A steht,
und
R²⁸ für Wasserstoff oder Fluor steht,
R²⁹ für Fluor steht,
R³⁰ für Fluor steht,
- R²: für Methyl oder Cyclopropyl steht,
- R³: für eine Gruppe der Formel 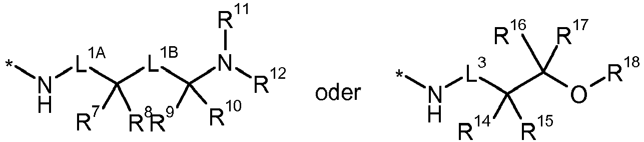 steht, wobei
* für die Anknüpfstelle an die Carbonylgruppe steht,
L^{1A} für eine Bindung steht,
L^{1B} für eine Bindung steht,
R⁷ für Wasserstoff, (C₁-C₆)-Alkyl oder Phenyl steht,
worin (C₁-C₆)-Alkyl bis zu fünffach mit Fluor substituiert sein kann,
worin Phenyl mit 1 bis 2 Substituenten Chlor oder Fluor substituiert sein kann,
R⁸ für Wasserstoff, Methyl oder Ethyl steht,
R⁹ für Wasserstoff, (C₁-C₆)-Alkyl oder Cyclopropyl steht,
worin (C₁-C₆)-Alkyl bis zu fünffach mit Fluor substituiert sein kann,
R¹⁰ für Wasserstoff, Methyl oder Ethyl steht,
R¹¹ für Wasserstoff steht,
R¹² für Wasserstoff steht,
R¹⁴ für Wasserstoff, (C₁-C₆)-Alkyl oder Phenyl steht,
worin (C₁-C₆)-Alkyl mit Hydroxy sowie bis zu fünffach mit Fluor substituiert sein kann,
und
worin Phenyl mit 1 oder 2 Substituenten Fluor substituiert sein kann,
R¹⁵ für Wasserstoff, Methyl oder Ethyl steht,
R¹⁶ für Wasserstoff oder (C₁-C₆)-Alkyl steht,
R¹⁷ für Wasserstoff, Methyl oder Ethyl steht,
oder
R¹⁶ und R¹⁷ zusammen mit dem Kohlenstoffatom, an das sie gebunden sind, einen 3-bis 6-gliedrigen Carbocyclus bilden,
R¹⁸ für Wasserstoff steht,
- R⁴: für Wasserstoff steht,
- R⁵: für Wasserstoff oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: für eine Phenyl-Gruppe der Formel 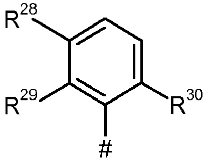 steht, wobei
# für die Anknüpfstelle an A steht,
und
R²⁸ für Wasserstoff oder Fluor steht,
R²⁹ für Fluor steht,
R³⁰ für Fluor steht, sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R²: für Methyl oder Cyclopropyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R²: für Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R²: für Cyclopropyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 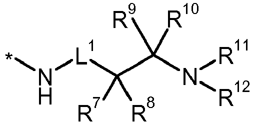
steht, wobei
- *: für die Anknüpfstelle an die Carbonylgruppe steht,
- L¹: für eine Bindung steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 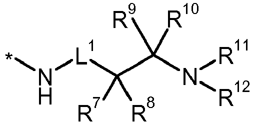 steht, wobei
* für die Anknüpfstelle an die Carbonylgruppe steht,
R⁷ für Wasserstoff, (C₁-C₆)-Alkyl oder Phenyl steht,
worin (C₁-C₆)-Alkyl bis zu fünffach mit Fluor substituiert sein kann,
worin Phenyl mit 1 bis 2 Substituenten Chlor oder Fluor substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 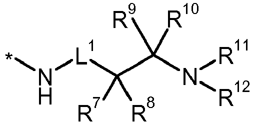 steht, wobei
* für die Anknüpfstelle an die Carbonylgruppe steht,
R⁸ für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 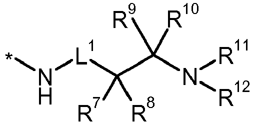 steht, wobei
* für die Anknüpfstelle an die Carbonylgruppe steht,
R⁹ für Wasserstoff, (C₁-C₆)-Alkyl oder Cyclopropyl steht, worin (C₁-C₆)-Alkyl bis zu fünffach mit Fluor substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 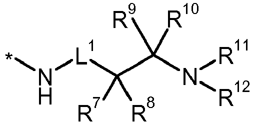 steht, wobei
* für die Anknüpfstelle an die Carbonylgruppe steht,
und
R¹⁰ für Wasserstoff oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 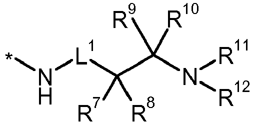 steht, wobei
* für die Anknüpfstelle an die Carbonylgruppe steht,
und
R¹¹ für Wasserstoff steht,
R¹² für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 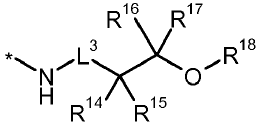
steht, wobei
- *: für die Anknüpfstelle an die Carbonylgruppe steht,
- L³: für eine Bindung steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 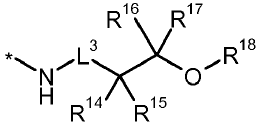 steht, wobei
* für die Anknüpfstelle an die Carbonylgruppe steht,
R¹⁴ für Wasserstoff, (C₁-C₆)-Alkyl oder Phenyl steht,
worin mit sowie bis fünffach mit Fluor substituiert sein (C₁-C₆)-Alkyl Hydroxy zu kann,
und
worin Phenyl mit 1 oder 2 Substituenten Fluor substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 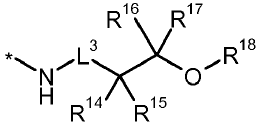 steht, wobei
* für die Anknüpfstelle an die Carbonylgruppe steht,
R¹⁵ für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 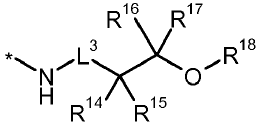 steht, wobei
* für die Anknüpfstelle an die Carbonylgruppe steht,
und
R¹⁷ für Wasserstoff oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 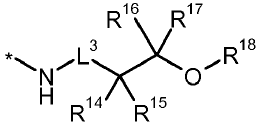 steht, wobei
* für die Anknüpfstelle an die Carbonylgruppe steht,
und
R¹⁸ für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁵: für Wasserstoff oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁵: für Methyl steht,
sowie ihre *N*-Oxide, Salze, Solvate, Salze der *N*-Oxide und Solvate der *N*-Oxide und Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
eine Verbindung der Formel (II) 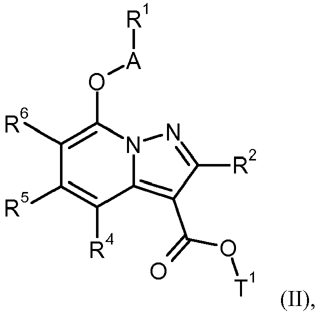 in welcher A, R¹, R², R⁴, R⁵ und R⁶ jeweils die oben angegebenen Bedeutungen haben und
- T¹: für (C₁-C₄)-Alkyl oder Benzyl steht,
in einem inerten Lösungsmittel in Gegenwart einer geeigneten Base oder Säure zu einer Carbonsäure der Formel (III) 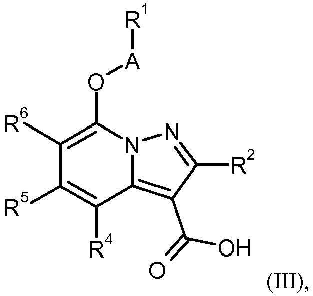 in welcher A, R¹, R², R⁴, R⁵ und R⁶ jeweils die oben angegebenen Bedeutungen haben, umsetzt und diese in der Folge in einem inerten Lösungsmittel unter Amidkupplungsbedingungen mit einem Amin der Formel (IV-A), (IV-B), (IV-C) oder (IV-D) 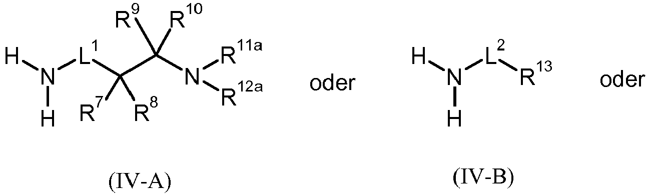 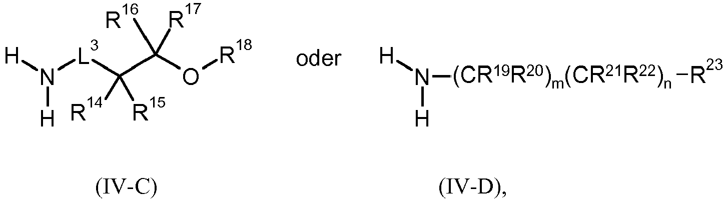 in welchem L¹, L², L³, R⁷, R⁸, R⁹, R¹⁰, R¹³, R¹⁴, R¹⁵, R¹⁶, R¹⁷, R¹⁸, R¹⁹, R²⁰, R²¹, R²² und R²³ jeweils die oben angegebenen Bedeutungen haben
und
R^{11A} und R^{12A} die oben für R¹¹ und R¹² angegebenen Bedeutungen haben oder für eine AminoSchutzgruppe, wie beispielsweise tert.-Butoxycarbonyl, Benzyloxycarbonyl oder Benzyl stehen, umsetzt,
anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Das beschriebene Herstellverfahren kann durch das folgende Syntheseschema (Schema 1) beispielhaft verdeutlicht werden:

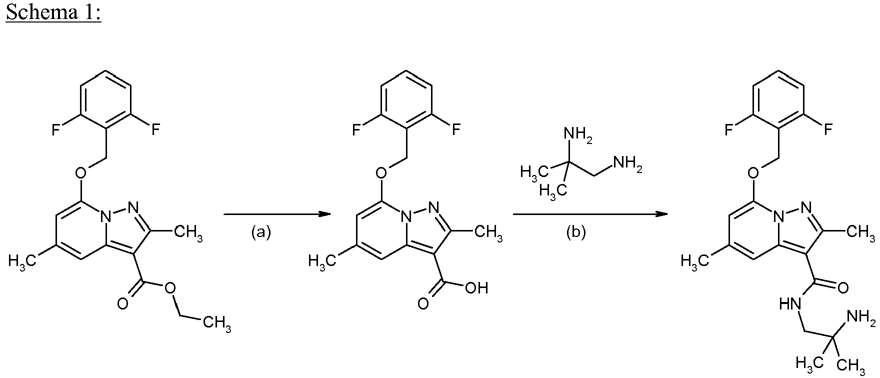

### [(a) Natriumhydroxid, 1,4-Dioxan, 90°C; (b) HATU, N,N-Diisopropylethylamin, DMF, Raumtemperatur].

Die Verbindungen der Formeln (IV-A), (IV-B), (IV-C) und (IV-D) sind kommerziell erhältlich, literaturbekannt oder können in Analogie zu literaturbekannten Verfahren hergestellt werden.

Inerte Lösungsmittel für die Verfahrensschritte (III) + (IV) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N*-Dimethylformamid, *N,N-*Dimethylacetamid, *N,N'-*Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung in den Verfahrensschritte (III) + (IV) → (I) eignen sich beispielsweise Carbodiimide wie *N,N'*-Diethyl-, *N,N'*-Dipropyl-, *N,N*'-Diisopropyl-, *N,N'-*Dicyclohexylcarbodiimid (DCC) oder *N-*(3-Dimethylaminopropyl)-*N'*-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-tert.-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid (T3P), 1-Chlor-*N,N*,2-trimethylprop-1-en-1-amin, Cyanophosphonsäurediethylester, Bis-(2-oxo-3-oxazolidinyl)-phosphorylchlorid, Benzotriazol-1-yloxy-tris(dimethylamino)phosphonium-hexafluorphosphat, Benzotriazol-1-yloxy-tris-(pyrrolidino)phosphonium-hexafluorphosphat (PyBOP), *O*-(Benzotriazol-1-yl)-*N*,*N,N',N'-*tetramethyluronium-tetrafluorborat (TBTU), *O*-(Benzotriazol-1-yl)-*N,N,N',N'*-tetramethyluronium-hexafluorphosphat (HBTU), 2-(2-Oxo-1-(2*H*)-pyridyl)-1,1,3,3-tetramethyluronium-tetrafluorborat (TPTU), *O*-(7-Azabenzotriazol-1-yl)-*N,N,N',N'*-tetramethyluronium-hexafluorphosphat (HATU) oder *O*-(1*H*-6-Chlorbenzotriazol-1-yl)-1,1,3,3-tetramethyluronium-tetrafluorborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N*-Diisopropylethylamin. Bevorzugt wird TBTU in Verbindung mit N-Methylmorpholin, HATU in Verbindung mit *N,N-*Diisopropylethylamin oder 1-Chlor-*N*,*N*,2-trimethylprop-1-en-1-amin verwendet.

Die Kondensation (III) + (IV) → (I) wird im Allgemeinen in einem Temperaturbereich von -20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Alternativ kann die Carbonsäure der Formel (III) auch zunächst in das entsprechende Carbonsäurechlorid überführt werden und dieses dann direkt oder in einer separaten Umsetzung mit einem Amin der Formel (IV) zu den erfindungsgemäßen Verbindungen umgesetzt werden. Die Bildung von Carbonsäurechloriden aus Carbonsäuren erfolgt nach den dem Fachmann bekannten Methoden, beispielsweise durch Behandlung mit Thionylchlorid, Sulfurylchlorid oder Oxalylchlorid in Gegenwart einer geeigneten Base, beispielsweise in Gegenwart von Pyridin, sowie optional unter Zusatz von Dimethylformamid, optional in einem geeigneten inerten Lösemittel.

Die Hydrolyse der Ester-Gruppe T¹ der Verbindungen der Formel (II) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren. Im Falle der Benzylester erfolgt die Esterspaltung bevorzugt hydrogenolytisch mit Palladium auf Aktivkohle oder Raney-Nickel. Als inerte Lösungsmittel eignen sich für diese Reaktion Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt.

Als Basen für die Ester-Hydrolyse sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Als Amino- Schutzgruppe wird bevorzugt *tert*.-Butoxycarbonyl (Boc) oder Benzyloxycarbonyl (Z) verwendet. Als Schutzgruppe für eine Hydroxy- oder Carboxyl-Funktion wird vorzugsweise *tert.-*Butyl oder Benzyl eingesetzt. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Diethylether, Dichlormethan oder Essigsäure durchgeführt; gegebenenfalls kann die Abspaltung auch ohne ein zusätzliches inertes Lösungsmittel erfolgen. Im Falle von Benzyl und Benzyloxycarbonyl als Schutzgruppe können diese auch durch Hydrogenolyse in Gegenwart eines Palladium-Katalysators entfernt werden. Die Abspaltung der genannten Schutzgruppen kann gegebenenfalls simultan in einer Eintopf-Reaktion oder in separaten Reaktionschritten vorgenommen werden.

Die Verbindungen der Formel (II) sind literaturbekannt oder können hergestellt werden, indem

### [A] eine Verbindung der Formel (V)

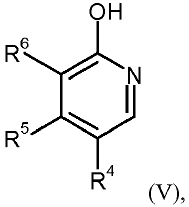

in welcher R⁴, R⁵ und R⁶ die oben angegebene Bedeutung haben, in einem inerten Lösungsmittel in Gegenwart einer geeigneten Base mit einer Verbindung der Formel (VI) 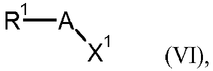 in welcher A und R¹ die oben angegebene Bedeutung haben und
- X¹: für eine geeignete Abgangsgruppe, insbesondere Chlor, Brom, Iod, Mesylat, Triflat oder Tosylat, steht,
zu einer Verbindung der Formel (VII) 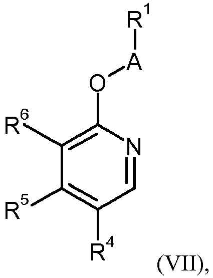 in welcher A, R¹, R⁴, R⁵ und R⁶ jeweils die oben angegebenen Bedeutungen haben,
umgesetzt wird,
diese dann mit *O*-(2-Mesitylenesulfonyl)hydroxylamin (MSH) in eine Verbindung (VIII) 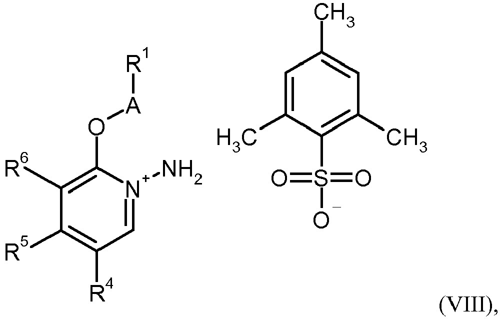 überführt wird,
und diese anschliessend in einem inerten Lösungsmittel mit einer Verbindung der Formel (IX) 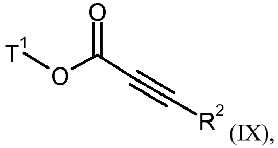 in welcher R² und T¹ jeweils die oben angegebenen Bedeutungen haben,
umgesetzt wird,
oder

### [B] eine Verbindung der Formel (X)

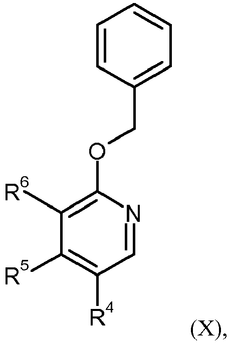

in welcher R⁴, R⁵ und R⁶ die oben angegebene Bedeutung haben,
mit *O*-(2-Mesitylenesulfonyl)hydroxylamin (MSH) in eine Verbindung (XI) 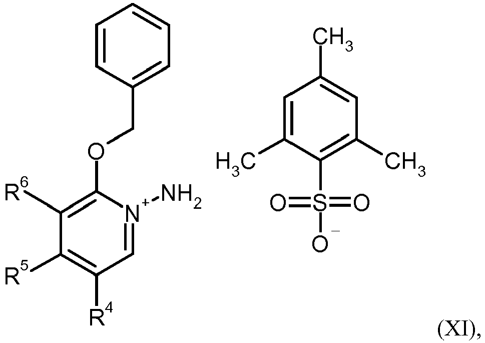 überführt wird,
diese anschliessend in einem inerten Lösungsmittel mit einer Verbindung der Formel (IX) 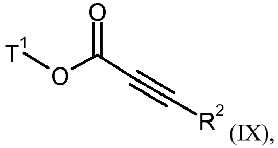 in welcher R² und T¹ jeweils die oben angegebenen Bedeutungen haben, zu einer Verbindung (XII) umgesetzt wird, 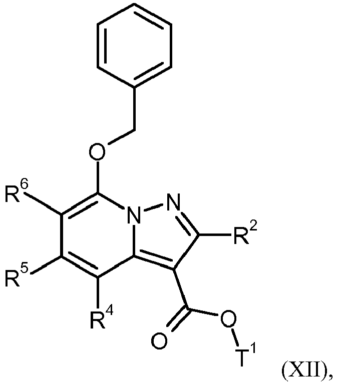 in welcher R², R⁴, R⁵ und R⁶ jeweils die oben angegebenen Bedeutungen haben und
- T¹: für (C₁-C₄)-Alkyl oder Benzyl steht,
von dieser im Folgenden nach den dem Fachmann bekannten Methoden die Benzylgruppe abspaltet und die resultierende Verbindung (XIII) 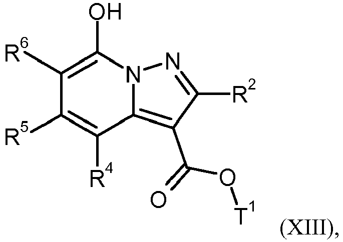 in welcher R², R⁴, R⁵ und R⁶ jeweils die oben angegebenen Bedeutungen haben und
- T¹: für (C₁-C₄)-Alkyl oder Benzyl steht,
in einem inerten Lösungsmittel unter Mitsunobu-Bedingungen mit einer Verbindung der Formel (XIV) 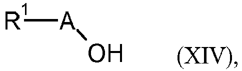 in welcher A und R¹ die oben angegebene Bedeutung haben,
umgesetzt wird.

Die beschriebenen Verfahren werden durch die nachfolgenden Schemata (Schema 2 und Schema 3) beispielhaft verdeutlicht:

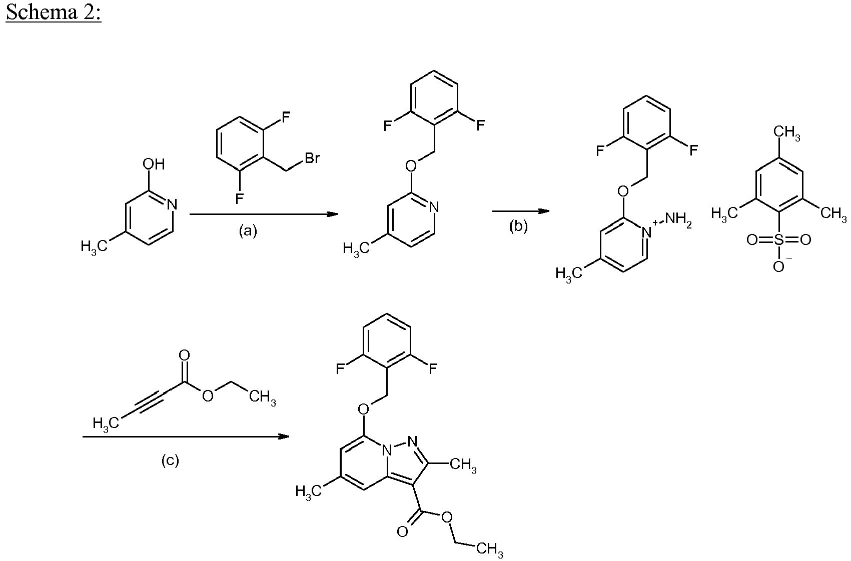

### [(a) Ag₂CO₃, THF, Rückfluss; (b) O-(2-mesitylenesulfonyl)hydroxylamin (MSH), Dichlormethan, Raumtemperatur; (c) K₂CO₃, DMF, Raumtemperatur].

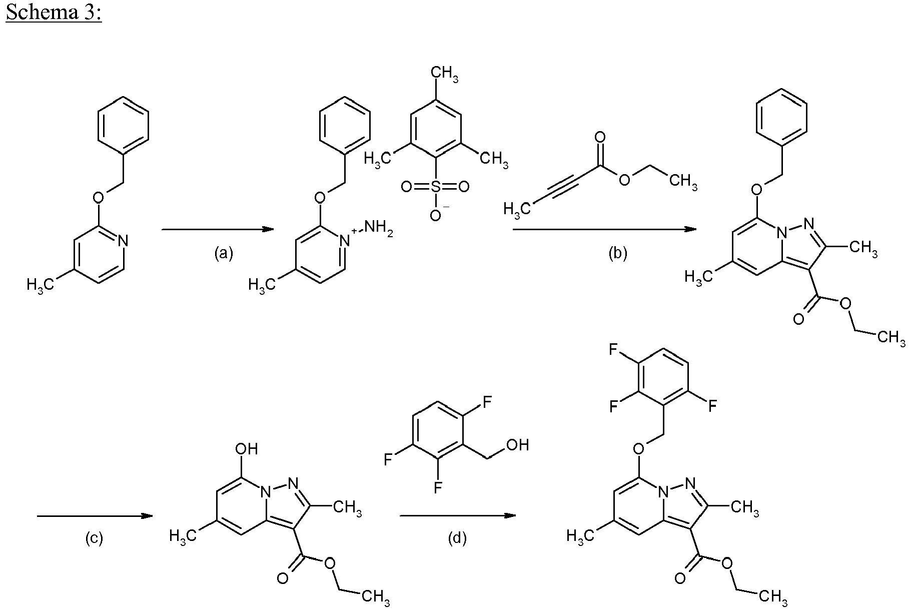

### [(a) O-(2-mesitylenesulfonyl)hydroxylamin (MSH), Dichlormethan, Raumtemperatur; (b) K₂CO₃, DMF, Raumtemperatur; (c) Cyclohexen, Pd/C, Ethanol, Rückfluss; (d) Triphenylphosphin, Diisopropyl-(E)-diazen-1,2-dicarboxylat (DIAD), THF, Raumtemperatur].

Alternativ zu der in Schema 2 gezeigten Einführung von R¹ durch Umsetzung der Verbindungen (V) mit Verbindungen der Formel (VI), ist es ebenso möglich - wie in Schema 4 gezeigt - Verbindungen (XV) mit Alkoholen der Formel (XIV) zu Verbindungen (XVI) umzusetzen.

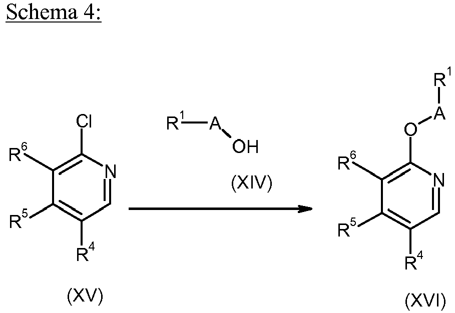

Typische Reaktionsbedingungen für derartige Umsetzungen finden sich in der Fachliteratur, z.B. Poon, K.W.C. *Synlet* **2005**, *6*, 841. Typischerweise wird in Anwesenheit einer Base wie Kaliumhydroxid oder Natriumhydrid, gegebenenfalls unter Zusatz eines 18-Krone-6-ethers, in einem inerten Lösemittel, z.B. THF oder Toluol, bei einer Temperatur zwischen 0 °C und dem Siedepunkt des verwendeten Lösemittels umgesetzt.

Inerte Lösungsmittel für den Verfahrensschritt (V) + (VI) → (VII) sind beispielsweise Ether wie Diethylether, Dioxan, Tetrahydrofuran, Dimethoxymethan, Glykoldimethylether oder Diethylenglykoldimethylether oder andere Lösungsmittel wie Aceton, Methylethylketon, Essigsäureethylester, Acetonitril, *N*,*N*-Dimethylformamid, *N*,*N*-Dimethylacetamid, Dimethylsulfoxid, *N,N'*-Dimethylpropylenharnstoff (DMPU), *N*-Methylpyrrolidon (NMP). Ebenso ist es möglich, Gemische der genannten Lösungsmittel einzusetzen. Bevorzugt wird Dimethoxyethan verwendet.

Als Basen für den Verfahrensschritt (V) + (VI) → (VII) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)-amid oder Lithiumdiisopropylamid, oder organische Amine wie Triethylamin, *N*-Methylmorpholin, *N*-Methylpiperidin, *N,N*-Diisopropylethylamin, Pyridin, 4-(*N,N*-Dimethylamino)-pyridin (DMAP), 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Natrium- oder Kalium-tert.-butylat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für den Verfahrensschritt (VII) → (VIII) sind beispielsweise Dichlormethan, 1,2-Dichlorethan oder andere Lösungsmittel wie Aceton, Methylethylketon, Essigsäureethylester, Acetonitril, *N,N*-Dimethylformamid, *N,N*-Dimethylacetamid, Dimethylsulfoxid, *N,N'-*Dimethylpropylenharnstoff (DMPU), *N*-Methylpyrrolidon (NMP). Ebenso ist es möglich, Gemische der genannten Lösungsmittel einzusetzen. Bevorzugt wird Dichlormethan verwendet.

Die Reaktion erfolgt im Allgemeinen in einem Temperaturbereich von 0°C bis +120°C, bevorzugt bei +20°C bis +80°C. Die Umsetzung kann bei normalem, erhöhtem oder bei erniedrigtem Druck durchgeführt werden (z.B. von 0.5 bis 5 bar).

Inerte Lösungsmittel für den Ringschluss zum Imidazo[1,2-a]pyrazin-Grundgerüst (VIII) + (IX) → (II) sind die üblichen organischen Lösungsmittel. Hierzu gehören bevorzugt Alkohole wie Methanol, Ethanol, n-Propanol, Isopropanol, n-Butanol, n-Pentanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, 1,2-Dichlorethan, Acetonitril, Dimethylformamid oder Dimethylsulfoxid. Ebenso ist es möglich, Gemische der genannten Lösungsmittel einzusetzen. Bevorzugt wird Ethanol verwendet.

Der Ringschluss erfolgt im Allgemeinen in einem Temperaturbereich von +50°C bis +150°C, bevorzugt bei +50°C bis +100°C, gegebenenfalls in einer Mikrowelle.

Der Ringschluss (VIII) + (IX) → (II) erfolgt optional in Gegenwart wasserziehender Reaktionszusätze, beispielsweise in Gegenwart von Molekularsieb (3Å oder 4Å Porengröße) oder mittels Wasserabscheider. Die Umsetzung (VIII) + (IX) → (II) erfolgt unter Verwendung eines Überschusses des Reagenzes der Formel (VIII), beispielsweise mit 1 bis 20 Äquivalenten des Reagenzes (VIII), gegebenenfalls unter Zusatz von Basen (wie z.B. Natriumhydrogencarbonat) wobei die Zugabe dieses Reagenzes einmalig oder in mehreren Portionen erfolgen kann.

Die Abspaltung der Benzylgruppe im Reaktionsschritt (XII) → (XIII) erfolgt hierbei nach üblichen, aus der Schutzgruppenchemie bekannten Methoden, vorzugsweise durch Hydrogenolyse in Gegenwart von eines Palladiumkatalysators, wie beispielsweise Palladium auf Aktivkohle, in einem inerten Lösungsmittel, wie beispielsweise Ethanol oder Essigsäureethylester [siehe auch z.B. T.W. Greene und P.G.M. Wuts, *Protective Groups in Organic Synthesis*, Wiley, New York, 1999]. Die Mitsunobu-Kondensation (XIII) + (XIV) → (II) erfolgt in Gegenwart eines Aktivierungsreagenzes, z.B. Diethyl-(E)-diazen-1,2-dicarboxylat (DEAD) oder Diisopropyl-(E)-diazen-1,2-dicarboxylat (DIAD), sowie eines Phosphinreagenzes, z.B. Triphenylphosphin oder Tributylphosphin, in einem inerten Lösemittel, z.B. THF, Dichlormethan, Toluol oder DMF, bei einer Temperatur zwischen 0 °C und dem Siedepunkt des verwendeten Lösemittels.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die erfindungsgemäßen Verbindungen besitzen wertvolle pharmakologische Eigenschaften und können zur Herstellung eines Medikaments zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Desweiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

**Abkürzungen und Akronyme:**

| | |
|---|---|
| abs. | absolutiert (= getrocknet) |
| aq. | wässrige Lösung |
| Boc | *tert*-Butyloxycarbonyl |
| br. | Verbreitertes Signal (NMR Kupplungsmuster) |
| CAS-Nr. | Chemical Abstracts Service Nummer |
| Cbz | Benzyloxycarbonyl |
| δ | Verschiebung im NMR Spektrum (Angabe in ppm) |
| d | Dublett (NMR-Kupplungsmuster) |
| DC | Dünnschichtchromatographie |
| DCI | direkte chemische Ionisation (bei MS) |
| DMAP | 4-*N,N*-Dimethylaminopyridin |
| DMF | Dimethylformamid |
| DMSO | Dimethylsulfoxid |
| d. Th. | der Theorie (bei Ausbeute) |
| eq. | Äquivalent(e) |
| ESI | Elektrospray-Ionisation (bei MS) |
| Et | Ethyl |
| h | Stunde(n) |
| HATU | N-[(Dimethylamino)(3H-[1,2,3]triazolo[4,5-b]-pyridin-3-yloxy)methylen]-N-methylmethanaminiumhexafluorophosphat |
| HPLC | Hochdruck-, Hochleistungsflüssigchromatographie |
| HRMS | hochaufgelöste Massenspektrometrie |
| konz. | konzentriert |
| J | Kopplungskonstante (NMR) |
| LC-MS | Flüssigchromatographie-gekoppelte Massenspektrometrie |
| LiHMDS | Lithiumhexamethyldisilazid |
| m | Multiplett (NMR Kopplungsmuster) |
| Me | Methyl |
| min | Minute(n) |
| MS | Massenspektrometrie |
| NMR | Kernresonanzspektrometrie |
| Ph | Phenyl |
| q | Quartett (NMR Kopplungsmuster) |
| quint. | Quintett (NMR Kopplungsmuster) |
| rel | relative Stereochemie |
| R_{F} | Retentionsfaktor (bei Dünnschichtchromatographie) |
| RT | Raumtemperatur |
| Rₜ | Retentionszeit (bei HPLC) |
| S | Singulett (NMR Kopplungsmuster) |
| t | Triplett (NMR Kopplungsmuster) |
| THF | Tetrahydrofuran |
| TBTU | (Benzotriazol-1-yloxy)bisdimethylaminomethyliumfluorborat |
| UV | Ultraviolett-Spektrometrie |
| v/v | Volumen zu Volumen-Verhältnis (einer Lösung) |

### LC-MS- und HPLC-Methoden:

### Methode 1 (LC-MS):

Instrument: Micromass Quattro Premier mit Waters UPLC Acquity; Säule: Thermo Hypersil GOLD 1.9 µ 50 x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm

### Methode 2 (LC-MS):

Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 210 - 400 nm.

### Methode 3 (LC-MS):

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 1 1 Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 4 (präparative HPLC):

Säule: Chromatorex C18 10 µ 250 x 20mm Gradient: A= Wasser + 0.5% Ameisensäure, B= Acetonitril, 0 min = 5% B, 3 min = 5% B vorspülen ohne Substanz, dann Injektion, 5 min = 5% B, 25 min = 30% B, 38 min = 30% B, 38.1 min = 95% B, 43 min= 95% B, 43.01 min= 5% B, 48.0 min= 5% B Flussrate 20 ml/min, Wellenlänge 210 nm.

### Methode 5 (präparative HPLC):

Säule: Chromatorex C18 10 µ 250x20 mm Gradient: A= Wasser + 0,5% Ameisensäure, B= ACetonitril, 0 min = 5% B, 3 min = 5% B vorspülen ohne Substanz, dann Injektion, 5 min = 5% B, 25 min = 50% B, 38 min = 50% B, 38.1 min = 95% B, 43 min= 95% B, 43.01 min= 5% B, 48.0 min= 5% B Flussrate 20 ml/min, Wellenlänge 210 nm.

### Methode 6 (präparative HPLC):

Säule: XBridge Prep. C18 5µ 50 x 19 mm Gradient: A = Wasser + 0.5% Ammoniumhydroxid, B= Acetonitril, 0 min = 5% B, 3 min = 5% B vorspülen ohne Substanz, dann Injektion, 5 min = 5% B, 25 min = 50% B, 38 min = 50% B, 38.1 min = 95% B, 43 min= 95% B, 43.01min= 5% B, 48.0 min= 5% B Flussrate 15 ml/min, Wellenlänge 210 nm.

### Methode 7 (LC-MS):

Instrument MS: Waters (Micromass) QM; Instrument HPLC: Agilent 1100 Serie; Säule: Agilent ZORBAX Extend-C18 3.0 x 50mm 3.5-Micron; Eluent A: 1 1 Wasser + 0.01 mol Ammoniumcarbonat, Eluent B: 1 1 Acetonitril; Gradient: 0.0 min 98% A → 0.2 min 98% A → 3.0 min 5% A → 4.5 min 5% A ; Ofen: 40°C; Fluss: 1.75 ml/min; UV-Detektion: 210 nm

### Methode 8 (LC-MS):

Instrument: Waters ACQUITY SQD UPLC System; Säule: Waters Acquity UPLC HSS T3 1.8 µ 30 x 2 mm; Eluent A: 1 1 Wasser + 0.25 ml 99%ige Ameisensäure, Eluent B: 1 1 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 9 (präparative HPLC):

Instrument MS: Waters; Instrument HPLC: Waters; Säule Waters X-Bridge C18, 18 mm x 50 mm, 5 µm, Eluent A: Wasser + 0.05% Triethylamin, Eluent B: Acetonitril (ULC) + 0.05% Triethylamin, mit Gradient; Fluss: 40 ml/min; UV-Detektion: DAD; 210 - 400 nm.

### bzw.:

Instrument MS: Waters; Instrument HPLC: Waters; Säule Phenomenex Luna 5µ C18 100A, AXIA Tech. 50 x 21.2 mm, Eluent A: Wasser + 0.05% Ameisensäure, Eluent B: Acetonitril (ULC) + 0.05% Ameisensäure, mit Gradient; Fluss: 40 ml/min; UV-Detektion: DAD; 210 - 400 nm.

### bzw.:

Instrument MS: Waters, Instrument HPLC: Waters Säule Waters X-Bridge C18, 19 mm x 50 mm, 5 µm, Eluent A: Wasser + 0.05% Ammoniak, Eluent B: Acetonitril (ULC) mit Gradient; Fluss: 40 ml/min; UV-Detektion: DAD; 210 - 400 nm.

### Methode 10 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98% A - 0.9 min 25% A - 1.0 min 5% A - 1.4 min 5% A - 1.41 min 98% A - 1.5 min 98% A; Ofen: 40°C; Fluss: 0.600 ml/min; UV-Detektion: DAD; 210 nm.

### Methode 11 (MS):

Instrument: Waters ZQ 2000; Elektrospray-Ionisierung; Eluent A: 1 1 Wasser + 0.25 ml 99%ige Ameisensäure, Eluent B: 1 1 Acetonitril + 0.25 ml 99%ige Ameisensäure; 25% A, 75% B; Fluss: 0.25 ml/min.

### Methode 12 (DCI-MS):

Instrument: Thermo Fisher-Scientific DSQ; chemische Ionisierung; Reaktantgas NH₃; Quellentemperatur: 200°C; Ionisierungsenergie 70eV.

### Methode 13 (LC-MS):

Instrument MS: Waters (Micromass) Quattro Micro; Instrument HPLC: Agilent 1100 Serie; Säule: YMC-Triart C18 3 µ 50 x 3 mm; Eluent A: 1 1 Wasser + 0.01 mol Ammoniumcarbonat, Eluent B: 1 1 Acetonitril; Gradient: 0.0 min 100% A → 2.75 min 5% A → 4.5 min 5% A; Ofen: 40°C; Fluss: 1.25 ml/min; UV-Detektion: 210 nm.

### Methode 14 (GC-MS):

Instrument: Thermo Scientific DSQII, Thermo Scientific Trace GC Ultra; Säule: Restek RTX-35MS, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Methode 15 (LC-MS):

Instrument: Acquity UPLC gekoppelt mit Quattro Micro Massenspektrometer; Säule: Acquity UPLC BEH C18 (50 mm x 2,1 mm ID, 1.7 µm Packungsdiameter); mobile Phase A: 10 mM wässrige Ammoniumhydrogencarbonatlösung (mit Ammoniak auf einen pH-Wert von 10 eingestellt), mobile Phase B: Acetonitril; Gradient: 0,0 min 97 % A, 3 % B, Fließgeschwindigkeit 1 ml/min; 1,5 min 100 % B, Fließgeschwindigkeit 1 ml/min; 1,9 min 100 % B, Fließgeschwindigkeit 1 ml/min; 2,0 min 97 % A, 3 % B, Fließgeschwindigkeit 0,05 ml/min; Säulentemperatur: 40°C; UV-Detektion: von 210 nm bis 350 nm; MS-Bedingungen: Ionisierungs-Modus: alternierende Scans Positives und Negatives Elektrospray (ES+/ES-); Scan-Bereich: 100 bis 1000 AMU.

### Methode 16 (LC-MS):

Instrument: Acquity UPLC gekoppelt mit Quattro Micro Massenspektrometer; Säule: Acquity UPLC BEH C18 (50 mm x 2,1 mm ID, 1.7 µm Packungsdiameter); mobile Phase A: 0,1% Ameisensäure in Wasser, mobile Phase B: 0,1% Ameisensäure in Acetonitril; Gradient: 0,0 min 97 % A, 3 % B, Fließgeschwindigkeit 1 ml/min; 1,5 min 100 % B, Fließgeschwindigkeit 1 ml/min; 1,9 min 100 % B, Fließgeschwindigkeit 1 ml/min; 2,0 min 97 % A, 3 % B, Fließgeschwindigkeit 0,05 ml/min; Säulentemperatur: 40°C; UV-Detektion: von 210 nm bis 350 nm; MS-Bedingungen: Ionisierungs-Modus: alternierende Scans Positives und Negatives Elektrospray (ES+/ES-); Scan-Bereich: 100 bis 1000 AMU.

### Methode 17 (LC-MS):

Instrument: Waters 2690, PDA-Detektor Waters 2996 gekoppelt mit Quattro Micro Massen-MS-Detektor; Säule: Waters SunFire C18 3,5 µm, 2,1x50 mm; mobile Phase A: 10 mM wässrige Ammoniumhydrogencarbonatlösung (mit Ammoniak auf einen pH-Wert von 10 eingestellt), mobile Phase B: Acetonitril; Gradient: 0,0 min 95 % A, 5 % B, Fließgeschwindigkeit 0,5 ml/min; 3,0 min 95 % A, 5 % B, Fließgeschwindigkeit 0,5 ml/min; 17,50 min 5 % A, 95 % B, Fließgeschwindigkeit 0,5 ml/min; 19,00 min 5 % A, 95 % B, Fließgeschwindigkeit 0,5 ml/min; 19,50 min 95 % A, 5 % B, Fließgeschwindigkeit 0,5 ml/min; 20,00 min 95 % A, 5 % B, Fließgeschwindigkeit 0,5 ml/min; Säulentemperatur: 30 °C; UV-Detektion: von 210 nm bis 400 nm; MS-Bedingungen: Ionisierungs-Modus: Scans Positives und Negatives Elektrospray (ES+/ES-); Scan-Bereich:130 bis 1100 AMU.

### Methode 18 (LC-MS):

Instrument: Waters 2690, PDA-Detektor Waters 2996 gekoppelt mit Quattro Micro Massen-MS-Detektor; Säule: Waters SunFire C18 3,5 µm, 2,1x50 mm; mobile Phase A: 0,1% Ameisensäure in Wasser, mobile Phase B: 0,1% Ameisensäure in Acetonitril; Gradient: 0,0 min 95 % A, 5 % B, Fließgeschwindigkeit 0,5 ml/min; 3,0 min 95 % A, 5 % B, Fließgeschwindigkeit 0,5 ml/min; 17,50 min 5 % A, 95 % B, Fließgeschwindigkeit 0,5 ml/min; 19,00 min 5 % A, 95 % B, Fließgeschwindigkeit 0,5 ml/min; 19,50 min 95 % A, 5 % B, Fließgeschwindigkeit 0,5 ml/min; 20,00 min 95 % A, 5 % B, Fließgeschwindigkeit 0,5 ml/min; Säulentemperatur: 30 °C; UV-Detektion: von 210 nm bis 400 nm; MS-Bedingungen: Ionisierungs-Modus: Scans Positives und Negatives Elektrospray (ES+/ES-); Scan-Bereich:130 bis 1100 AMU.

### Methode 19 (LC-MS):

MS-Instrumententyp: Waters ZMD; HPLC-Instrumententyp: Waters 1525; Säule: Phenomenex Luna 3 µm C18(2) 30 mm x 4,6 mm; mobile Phase A: Wasser 0,1% Ameisensäure, mobile Phase B: Acetonitril 0,1% Ameisensäure; Gradient: 0,0 min 95% A -> 0,5 min 95% A -> 4,5 min 5% A - > 5,5 min 5% A; Fließgeschwindigkeit: 2 ml/min; UV-Detektion: DAD.

### Methode 20 (LC-MS):

MS-Instrumententyp: Waters Micromass ZQ2000; HPLC-Instrumententyp: Waters Acquity UPLC-System; Säule: Acquity UPLC BEH C18 1,7 µm 100 mm x 2,1 mm; mobile Phase A: Wasser 0,1% Ameisensäure, mobile Phase B: Acetonitril 0,1% Ameisensäure; Gradient: 0,0 min 95% A -> 0,4 min 95% A -> 6,0 min 5% A -> 6,8 min 5% A; Fließgeschwindigkeit: 0,4 ml/min; UV-Detektion: PDA.

### Methode 21 (LC-MS):

Instrument: Waters 2690, PDA-Detektor Waters 2996 gekoppelt mit Quattro Micro Massen-MS-Detektor; Säule: XBridge Prep. MS C18 OBD (150 mm x 30mm ID 5 4 µm Korngröße) bei Raumtemperatur; mobile Phase A: 10 mM NH₄HCO₃, mit Ammoniak auf einen pH-Wert von 10 eingestellt, mobile Phase B: Acetonitril; Gradient: 0,0 min 97 % A, 3 % B; 1,0 min 97 % A, 3 % B; 30 min 0 % A, 100 % B; 35 min 0 % A, 100 % B, Fließgeschwindigkeit 50 ml/min; Säulentemperatur: 30 °C; UV-Detektion: von 210 nm bis 400 nm; MS-Bedingungen: Ionisierungs-Modus: Scans Positives und Negatives Elektrospray (ES+/ES-); Scan-Bereich:100 bis 1000 AMU.

### Methode 22 (LC-MS, analytisch):

Instrument: Agilent MS Quad 6150; HPLC: Agilent 1290; Säule: Waters Acquity UPLC HSS T3 1.8 µ 50 x 2.1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1.20 ml/min; UV-Detektion: 205 - 305 nm.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische bzw. saure Funktionalität enthalten. Ein solches Salz kann durch verschiedene dem Fachmann bekannte Methoden in die entsprechende freie Base bzw. Säure überführt werden.

Salze können unter- oder überstöchiometrisch vorliegen, insbesondere bei Vorliegen eines Amins oder einer Carbonsäure. Zusätzlich können bei den vorliegenden Pyrazolopyridinen unter sauren Bedingungen stets Salze, auch unterstöchiometrisch, vorliegen, ohne dass diese im ¹H-NMR erkenntlich sind und ohne besondere Angabe und Kennzeichnung dieser in den jeweiligen IUPAC-Namen und Strukturformeln.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung.

### Ausgangsverbindungen und Intermediate:

### Beispiel 1A

### 2-[(2,6-Difluorbenzyl)oxy]-4-methylpyridin

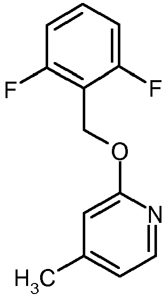

Eine Mischung von 5.00 g (24.2 mmol, 1.0 eq.) 2,6-Difluorbenzylbromid [CAS-Nr: 85118-00-9] und 3.16 g (29.0 mmol, 1.2 eq.) 2-Hydroxy-4-methylpyridin [CAS-Nr: 13466-41-6] wurde in 50 ml THF gelöst. Die Lösung wurde mit 7.99 g (29.0 mmol, 1.2 eq.) Silbercarbonat versetzt und unter Lichtausschluss über Nacht auf Rückfluss erhitzt. Anschließend wurde die Reaktionsmischung über Kieselgur filtriert, mit Essigsäureethylester eluiert und das Filtrat wurde eingeengt. Das Rohprodukt wurde mittels Biotage Isolera (100 g Kieselgelkartusche, Cyclohexan/Essigsäureethylester-Gradient, 0 % nach 10 % Essigsäureethylester) gereinigt. Es wurden 3.51 g der Titelverbindung (61 % d. Th.) erhalten.
DC (Kieselgel, Cyclohexan/Essigsäureethylester 10:1): R_{F} = 0.50
LC-MS (Methode 2): Rₜ = 1.17 min
MS (ESpos): m/z = 236 (M+H)⁺
¹H-NMR (400 MHz, DMSO-d₆): δ [ppm] = 2.26 (s, 3H), 5.35 (s, 2H), 6.66 (s, 1H), 6.86 (d, 1H), 7.12 - 7.21 (m, 2H), 7.47 - 7.56 (m, 1H), 8.06 (d, 1H).

### Beispiel 2A

### 1-Amino-2-[(2,6-difluorbenzyl)oxy]-4-methylpyridinium-2,4,6-trimethylbenzolsulfonat

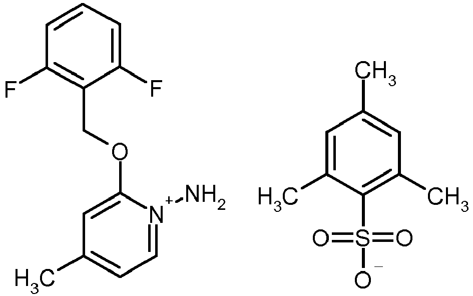

Eine Mischung von 3.4 ml (43.9 mmol, 10 eq.) Trifluoressigsäure und 0.33 ml Wasser wurden auf -5°C gekühlt. Bei dieser Temperatur wurden 1.88 g (6.59 mmol, 1.5 eq.) Ethyl-(1E)-N-[(mesitylsulfonyl)oxy-]ethanimidoat [CAS-Nr: 38202-27-6] portionsweise zugegeben. Nach 1.5 h wurden 30 ml Eiswasser hinzugefügt, kurz nachgerührt und das ausgefallene *O*-(2-Mesitylenesulfonyl)hydroxylamin (MSH) wurde durch eine vorher gekühlte Fritte abfiltriert und mit 30 ml Eiswasser gewaschen. Das wasserfeuchte *O*-(2-Mesitylenesulfonyl)hydroxylamin wurde in 12 ml Dichlormethan gelöst, mit Magnesiumsulfat getrocknet, filtriert und und das Filtrat wurde direkt zu einer Lösung von 1.03 g (4.39 mmol, 1.0 eq.) 2-[(2,6-Difluorbenzyl)oxy]-4-methylpyridin aus Beispiel 1A in 2 ml Dichlormethan getropft. Die Mischung wurde über Nacht bei RT gerührt. Anschließend wurde Diethylether zugetropft, der anfallende Niederschlag abfiltriert, mit Diethylether gewaschen und getrocknet. Es wurden 1.3 g der Titelverbindung isoliert (59% d. Th., Reinheit 90%).
¹H-NMR (400 MHz, DMSO-d₆): δ [ppm] = 2.17 (s, 3H), 2.46 - 2.57 (s, 3H und s, 6H überlagert mit den Lösungsmittelsignal), 5.64 (s, 2H), 6.74 (s, 2H), 7.23 - 7.48 (m, 4H), 7.60 - 7.70 (m, 1H), 7.86 (br s, 1H), 8.44 (d, 1H).

### Beispiel 3A

### Ethyl-7-[(2,6-difluorbenzyl)oxy]-2,5-dimethylpyrazolo[1,5-a]pyridin-3-carboxylat

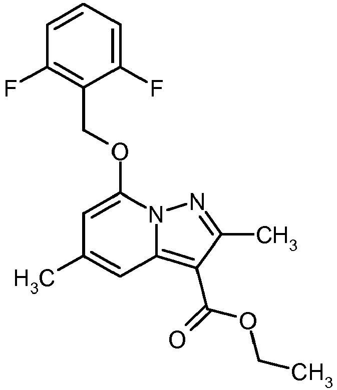

1.62 g (3.60 mmol, 1.0 eq.) 1-Amino-2-[(2,6-difluorbenzyl)oxy]-4-methylpyridinium-2,4,6-trimethylbenzolsulfonat aus Beispiel 2A wurden in 36 ml DMF gelöst und mit 0.84 ml (7.19 mmol, 2.0 eq.) Ethylbut-2-inoat [CAS-Nr: 4341-76-8] versetzt. Es wurden 0.994 g (7.19 mmol, 2.0 eq.) Kaliumcarbonat zugegeben und die Mischung wurde 1.5 h bei RT gerührt. Anschließend wurde das Gemisch auf 150 ml Wasser gegossen, kurz ausgerührt, der ausgefallene Feststoff abfiltriert, mit Wasser gewaschen und getrocknet. Es wurden 440 mg der Titelverbindung (45% d. Th., 87%) erhalten.
LC-MS (Methode 2): Rₜ = 1.22 min
MS (ESpos): m/z = 361 (M+H)⁺

### Beispiel 4A

### 7-[(2,6-Difluorbenzyl)oxy]-2,5-dimethylpyrazolo[1,5-a]pyridin-3-carbonsäure

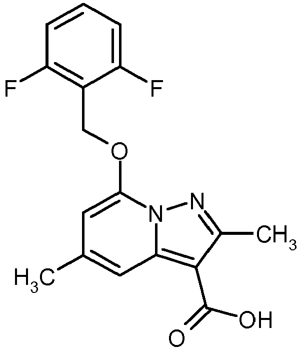

Eine Lösung von 0.440 g(1.22 mmol, 1 eq.) Ethyl-7-[(2,6-difluorbenzyl)oxy]-2,5-dimethyl-pyrazolo[1,5-a]pyridin-3-carboxylat aus Beispiel 3A in 12.7 ml Dioxan wurde mit 4.9 ml (4.88 mmol, 4.0 eq.) 1 N wässriger Natronlauge versetzt und 36 h bei 90°C gerührt. Anschließend wurde das Reaktionsgemisch eingeengt und der ausgefallene Feststoff abfiltriert. Das Filtrat wurde mit 6 N wässriger Salzsäure angesäuert, kurz ausgerührt, der ausgefallene Feststoff wurde abfiltriert, mit Wasser gewaschen und getrocknet. Es wurden 248 mg der Titelverbindung (61% d. Th., Reinheit 60%) erhalten, die ohne weitere Reinigung weiter umgesetzt wurde.
LC-MS (Methode 2): Rₜ = 0.96 min
MS (ESpos): m/z = 333 (M+H)⁺

### Beispiel 5A

### 2-(Benzyloxy)-4-methylpyridin

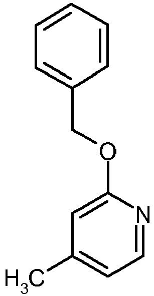

Eine Mischung von 13.6 ml (114 mmol, 1.0 eq.) Benzylbromid und 15.0 g (137 mmol, 1.2 eq.) 2-Hydroxy-4-methylpyridin [CAS-Nr: 13466-41-6] wurde in 470 ml THF gelöst. Die Lösung wurde mit 37.9 g (137 mmol, 1.2 eq.) Silbercarbonat versetzt und unter Lichtausschluss über Nacht auf Rückfluss erhitzt. Anschließend wurde die Reaktionsmischung über Kieselgur filtriert, mit Essigsäureethylester eluiert und das Filtrat wurde eingeengt. Das Rohprodukt wurde durch Kieselgelchromatographie (700 g Kieselgel, Cyclohexan/Essigsäureethylester, 95:5) gereinigt. Es wurden 21.4 g der Titelverbindung (94 % d. Th.) erhalten.
DC (Kieselgel, Cyclohexan/Essigsäureethylester 9:1): R_{F} = 0.41
LC-MS (Methode 2): Rₜ = 1.12 min
MS (ESpos): m/z = 200 (M+H)⁺
¹H-NMR (400 MHz, DMSO-d₆): δ [ppm] = 2.27 (s, 3H), 5.33 (s, 2H), 6.70 (s, 1H), 6.83 (d, 1H), 7.27 - 7.45 (m, 5H), 8.02 (d, 1H).

### Beispiel 6A

### 1-Amino-2-(benzyloxy)-4-methylpyridinium-2,4,6-trimethylbenzolsulfonat

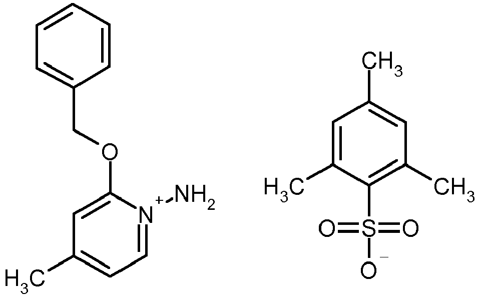

Eine Mischung von 18.0 ml Trifluoressigsäure (233 mmol, 10 eq.) und 2.66 ml Wasser wurden auf -5°C gekühlt. Bei dieser Temperatur wurden 9.99 g (35.0 mmol, 1.5 eq.) Ethyl-(1E)-N-[(mesitylsulfonyl)oxy-]ethanimidoat [CAS-Nr: 38202-27-6] portionsweise zugegeben. Nach 1.5 h wurden 150 ml Eiswasser zugegeben, kurz nachgerührt und mit 100 ml Dichlormethan extrahiert. Die organische Phase wurde mit Magnesiumsulfat getrocknet, filtriert und die erhaltene Lösung von *O*-(2-mesitylenesulfonyl)hydroxylamin (MSH) wurde direkt zu einer auf 0°C gekühlten Lösung von 4.65 g (23.3 mmol, 1.0 eq.) 2-(Benzyloxy)-4-methylpyridin aus Beispiel 5A in 50 ml Dichlormethan getropft. Es wurde für 2 h bei RT gerührt. Anschließend wurde 1 l Diethylether zugetropft, der ausgefallene Feststoff abfiltriert, mit 250 mL Diethylether gewaschen und an der Luft getrocknet. Es wurden 4.6 g der Titelverbindung isoliert (48% d. Th.).
LC-MS (Methode 2): Rₜ = 0.45 min;
MS (ESpos): m/z = 215 (C₁₃H₁₅N₂O) (M)⁺; Rₜ = 0.57 min;
MS (ESneg): m/z = 199 (C₉H₁₁O₃S)⁻;

### Beispiel 7A

### Ethyl-7-(benzyloxy)-2,5-dimethylpyrazolo[1,5-a]pyridin-3-carboxylat

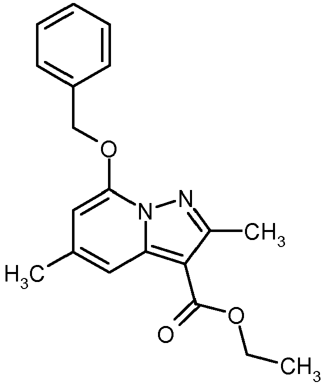

11.7 g (28.2 mmol, 1.0 eq.) 1-Amino-2-(benzyloxy)-4-methylpyridinium-2,4,6-trimethylbenzolsulfonat aus Beispiel 6A wurden in 280 ml DMF gelöst und mit 6.6 ml (56 mmol, 2.0 eq.) Ethylbut-2-inoat [CAS-Nr: 4341-76-8] versetzt. Es wurden 7.8 g (56 mmol, 2.0 eq.) Kaliumcarbonat zugegeben und 1 h bei RT gerührt. Anschließend wurden 3.9 g (28 mmol, 1 eq.) Kaliumcarbonat zugegeben und die Mischung wurde für weitere 16 h bei RT gerührt. Dann wurde das Gemisch auf 540 ml Wasser gegossen, kurz ausgerührt, der ausgefallene Feststoff abfiltriert, mit 220 ml Wasser gewaschen und getrocknet. Es wurden 3.1 g der Titelverbindung (34% d. Th., Reinheit 87%) erhalten.
LC-MS (Methode 2): Rₜ = 1.20 min
MS (ESpos): m/z = 325 (M+H)⁺
¹H-NMR (400 MHz, DMSO-d₆): δ [ppm] = 1.34 (t, 3H), 2.43 (s, 3H), 4.27 (q, 2H), 5.43 (s, 2H), 6.60 (d, 1H), 7.37 - 7.49 (m, 4H), 7.52 - 7.59 (m, 2H), [s, 3H unter Lösungsmittelsignal].

### Beispiel 8A

### Ethyl-7-hydroxy-2,5-dimethylpyrazolo[1,5-a]pyridin-3-carboxylat

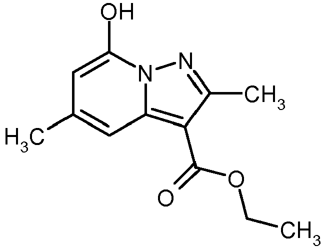

2 g (5.98 mmol) Ethyl-7-(benzyloxy)-2,5-dimethylpyrazolo[1,5-a]pyridin-3-carboxylat aus Beispiel 7A wurden unter Argon in 80 ml Ethanol vorgelegt, mit 636 mg (0.59 mmol, 10%ig) Palladium auf Aktivkohle und 18 ml (179.42 mmol) Cyclohexen versetzt. Das Reaktionsgemisch wurde 2.5 Stunden unter Rückfluss gerührt. Dann wurde über Kieselgur abfiltriert, mit Ethanol gewaschen und das Filtrat eingeengt. Der Rückstand wurde in DMSO und Acetonitril aufgenommen und mittels präparativer HPLC gereinigt (RP18 Säule, Laufmittel: Acetonitril/Wasser-Gradient unter Zusatz von 0.1% TFA). Die Produktfraktionen wurden vereinigt, eingeengt und lyophilisiert. Es wurden 1.2 g der Zielverbindung (86% d. Th.) erhalten.
LC-MS (Methode 7): Rₜ = 1.60 min
MS (ESpos): m/z = 235 (M+H)⁺
¹H-NMR (500 MHz, DMSO-d₆): δ = 1.33 (t, 3H), 2.35 (s, 3H), 2.54 (s, 3H; verdeckt unter Lösungsmittelpeak), 4.26 (q, 2H), 6.17 (d, 1H), 7.26 (s, 1H).

### Beispiel 9A

### Ethyl-2,5-dimethyl-7-[(2,3,6-trifluorbenzyl)oxy]pyrazolo[1,5-a]pyridin-3-carboxylat

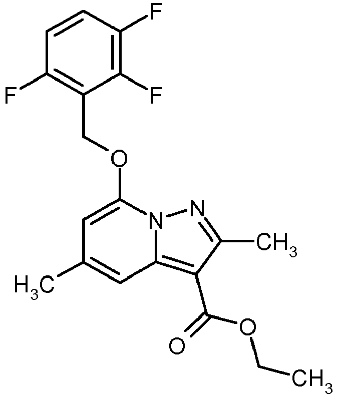

1.2 g (5.25 mmol) Ethyl-7-hydroxy-2,5-dimethylpyrazolo[1,5-a]pyridin-3-carboxylat aus Beispiel 8A wurden in 48 ml THF gelöst. 1.7 g (10.50 mmol) 2,3,6-Trifluorbenzylalkohol und 2.9 g (11.03 mmol) Triphenylphosphin wurden hinzugegeben. Anschließend wurde die Lösung mit 2.2 ml (11.03 mmol) Diisopropyl-(E)-diazen-1,2-dicarboxylat versetzt und 1 h bei RT gerührt. 120 ml tert-Butylmethylether wurden hinzugegeben, dann kurz ausgerührt, der enstandene Feststoff abfiltriert und im Hochvakuum getrocknet. Es wurden 1.2 g der Zielverbindung (62% d. Th.) erhalten.
LC-MS (Methode 2): Rₜ = 1.22 min
MS (ESpos): m/z = 379 (M+H)⁺
¹H-NMR (500 MHz, DMSO-d₆): δ = 1.34 (t, 3H), 2.46 (s, 3H), 2.51 (s, 3H; verdeckt unter Lösungsmittelpeak), 4.28 (d, 2H), 5.51 (s, 2H), 6.70 (s, 1H), 7.29 - 7.37 (m, 1H), 7.48 (s, 1H), 7.66 - 7.76 (m, 1H).

### Beispiel 10A

### 2,5-Dimethyl-7-[(2,3,6-trifluorbenzyl)oxy]pyrazolo[1,5-a]pyridin-3-carbonsäure

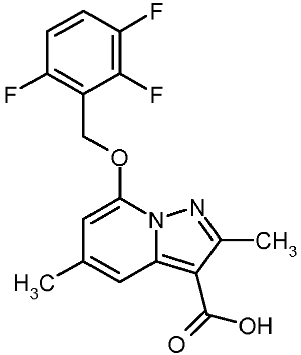

700 mg (1.81 mmol) Ethyl-2,5-dimethyl-7-[(2,3,6-trifluorbenzyl)oxy]pyrazolo[1,5-a]pyridin-3-carboxylat aus Beispiel 9A wurden in 18 ml Dioxan vorgelegt und auf 90°C erhitzt. Es wurden 4.5 ml Dioxan und 7.25 ml (14.50 mmol) 2 N wässrige Natronlauge hinzugegeben und die Reaktionsmischung wurde zwei Tage bei 90°C gerührt. Es wurden nochmals 3.63 ml (7.26 mmol) 2 N wässrige Natronlauge hinzugegeben und es wurde weitere 2 Stunden bei 90°C gerührt. Die Reaktionslösung wurde mit 15 ml IN wässriger Salzsäure versetzt und 30 min. gerührt. Dabei fiel Feststoff aus. Diese Suspension wurde filtriert, der abfiltrierte Feststoff wurde mit wenig Wasser gewaschen und im Hochvakuum getrocknet. Es wurden 358 mg der Zielverbindung (54% d. Th.) erhalten.
LC-MS (Methode 2): Rₜ = 0.97 min
MS (ESpos): m/z = 351 (M+H)⁺

### Beispiel 11A

### rac-Benzyl-(2-cyanbutan-2-yl)carbamat

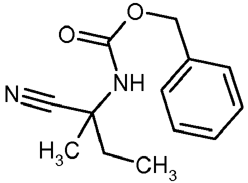

5.00 g (50.94 mmol) 2-Amino-2-methylbutanonitril [Synthese beschrieben in: Lonza AG, US 5698704 (1997); Deng, S. L. et al. Synthesis 2001, 2445; Hjorringgaard, C. U. et al. J. Org. Chem. 2009, 74, 1329; Ogrel, A. et al. Eur. J. Org. Chem. 2000, 857] wurden in 50 ml THF und 6.5 ml Wasser vorgelegt, mit 21.83 g (157.92 mmol) Kaliumcarbonat versetzt und bei 0°C langsam mit 7.9 ml (56.04 mmol) Benzylchlorocarbonat (Chlorameisensäurebenzylester) versetzt. Nach Zugabe von 8 ml THF und 3 ml Wasser wurde das Reaktionsgemisch langsam auf RT kommend über Nacht gerührt. Dann wurde mit Wasser versetzt und dreimal mit Essigsäureethylester extrahiert. Die vereinten organischen Phasen wurden über Natriumsulfat getrocknet und eingeengt. Der Rückstand wurde in Diethylether gelöst und mit Petrolether ausgefällt. Das Produkt wurde abfiltriert, der Feststoff mit etwas Petrolether gewaschen und im Hochvakuum getrocknet. Es wurden 11.35 g der Zielverbindung (93% d. Th.) erhalten.
LC-MS (Methode 2): Rₜ = 0.97 min
MS (ESpos): m/z = 233 (M+H)⁺
¹H-NMR (400 MHz, DMSO-d₆): δ = 0.95 (t, 3H), 1.51 (s, 3H), 1.75 - 1.95 (m, 2H), 5.07 (s, 2H), 7.30 - 7.43 (m, 4H), 7.88 - 8.03 (m, 1H).

### Beispiel 12A

### ent-Benzyl-(2-cyanbutan-2-yl)carbamat (Enantiomer A)

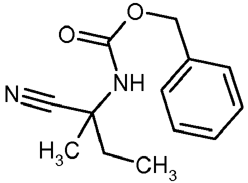

8 g *rac-*Benzyl-(2-cyanbutan-2-yl)carbamat aus Beispiel 11A wurden durch präparative Trennung an chiraler Phase in die Enantiomere getrennt [Säule: Daicel Chiralcel OJ-H, 5 µm, 250 x 20 mm, Eluent: 50% Iso-Hexan, 50% Isopropanol, Fluß: 20 ml/min; 40°C, Detektion: 220 nm].
Enantiomer A: Ausbeute: 3.23 g (>99% ee)
Rₜ = 6.69 min [Daicel Chiralcel OJ-H, 5 µm, 250 x 4.6 mm; Eluent: 50% Iso-Hexan, 50% Isopropanol; Fluss 1.0 ml/min; 30°C; Detektion: 220 nm].

### Beispiel 13A

### ent-Benzyl-(2-cyanbutan-2-yl)carbamat (Enantiomer B)

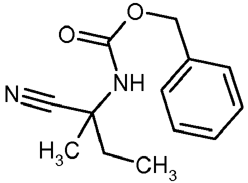

8 g *rac*-Benzyl-(2-cyanbutan-2-yl)carbamat aus Beispiel 11A wurden durch präparative Trennung an chiraler Phase in die Enantiomere getrennt [Säule: Daicel Chiralcel OJ-H, 5 µm, 250 x 20 mm, Eluent: 50% Iso-Hexan, 50% Isopropanol, Fluß: 20 ml/min; 40°C, Detektion: 220 nm].
Enantiomer B: Ausbeute: 3.18 g (>99% ee)
Rₜ = 8.29 min [Daicel Chiralcel OJ-H, 5 µm, 250 x 4.6 mm; Eluent: 50% Iso-Hexan, 50% Isopropanol; Fluss 1.0 ml/min; 30°C; Detektion: 220 nm].

### Beispiel 14A

### ent-Benzyl-(1-amino-2-methylbutan-2-yl)carbamat (Enantiomer A)

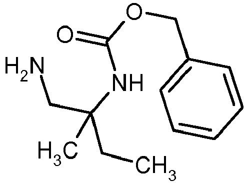

4.00 g (17.22 mmol) *ent*-Benzyl-(2-cyanbutan-2-yl)carbamat aus Beipiel 12A wurden in 50 ml einer 7 N Lösung von Ammoniak in Methanol gelöst, mit 5.33 g Raney-Nickel versetzt und 24 h bei ca. 25 bar bei RT hydriert. Es wurde über Celite abfiltriert, mit Methanol gewaschen und eingeengt. Das Rohprodukt wurde mittels Kieselgelchromatographie gereinigt (Laufmittel: Dichlormethan/2N Ammoniak in Methanol = 10/0.5). Es wurden 2.20 g der Zielverbindung (54% d. Th.) erhalten.
LC-MS (Methode 2): Rₜ = 0.56 min
MS (ESpos): m/z = 237 (M+H)⁺

### Beispiel 15A

### ent-Benzyl-(1-amino-2-methylbutan-2-yl)carbamat (Enantiomer B)

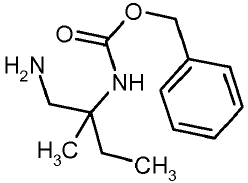

4.00 g (17.22 mmol) *ent*-Benzyl-(2-cyanbutan-2-yl)carbamat aus Beispiel 13A wurden in 50 ml 7 N ammoniakalische Methanol-Lösung gelöst, mit 5.33 g Raney-Nickel versetzt und 24 h bei ca. 25 bar bei RT hydriert. Das Reaktionsgemisch wurde über Celtite abfiltriert, gut mit Methanol gespült und eingeengt. Das Rohprodukt wurde mittels Kieselgelchromatographie gereinigt (Laufmittel: Dichlormethan/2N Ammoniak in Methanol = 10/0.5). Es wurden 3.56 g der Zielverbindung (87% d. Th.) erhalten.
LC-MS (Methode 3): Rₜ = 1.40 min
MS (ESpos): m/z = 237 (M+H)⁺

### Beispiel 16A

### rac-Benzyl-(2-cyanpentan-2-yl)carbamat

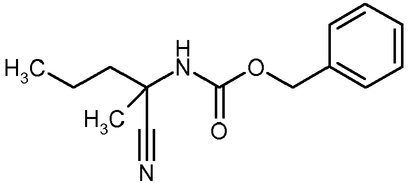

20 g (178.3 mmol) *rac*-2-Amino-2-methylpentanonitril (beschrieben in: Deng, S L. et al., Synthesis 2001, 2445-2449; Freifelder, M. et al., J. Am. Chem. Soc. 1960, 696-698) wurden in 2.63 1 THF/Wasser (8/1) vorgelegt und mit 76.4 g (552.7 mmol) Kaliumcarbonat versetzt. Dann wurden bei 0°C 27.6 ml (196.1 mmol) Chlorameisensäurebenzylester langsam zugetropft und über Nacht bei RT gerührt. Die Reaktionsmischung wurde eingeengt, der Rückstand mit Wasser versetzt und zweimal mit Essigsäureethylester extrahiert. Die vereinigten organischen Phasen wurden über Natriumsulfat getrocknet, filtriert und eingeengt. Der Rückstand wurde mittels Kieselgel-Chromatographie (Laufmittel: Cyclohexan/Essigsäureethylester = 4/1) gereinigt. Es wurden 43.84 g der Zielverbindung (76% d. Th., Reinheit 76%) erhalten.
LC-MS (Methode 2): Rₜ = 1.02 min
MS (ESpos): m/z = 247 (M+H)⁺
¹H-NMR (400 MHz, DMSO-d₆): δ = 0.90 (t, 3H), 1.31 - 1.48 (m, 2H), 1.52 (s, 3H), 1.70 - 1.88 (m, 2H), 5.07 (s, 2H), 7.30 - 7.42 (m, 5H), 8.00 (br. s, 1H).

### Beispiel 17A

### ent-Benzyl-(2-cyanpentan-2-yl)carbamat (Enantiomer A)

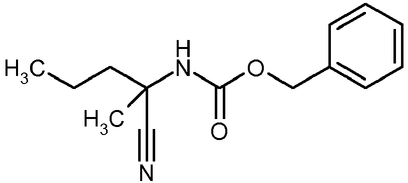

43.8 g (135.3 mmol) *rac*-Benzyl-(2-cyanpentan-2-yl)carbamat aus Beispiel 16A wurden durch präparative Trennung an chiraler Phase in die Enantiomere getrennt [Säule: SFC Chiralpak AZ-H, 5 µm, 250 x 50 mm, Eluent: 85% CO₂, 15% Methanol, Fluß: 250 ml/min; Temperatur: 28°C, backpressure: 100 bar, Detektion: 220 nm].
Enantiomer A: Ausbeute: 13.13 g (>99% ee)
Rₜ = 2.76 min [SFC Chiralpak AZ-H, 5 µm, 250 x 4.6 mm; Eluent: 90% CO₂, 10% Methanol; Fluß: 3 ml/min; Detektion: 220 nm].

### Beispiel 18A

### ent-Benzyl-(2-cyanpentan-2-yl)carbamat (Enantiomer B)

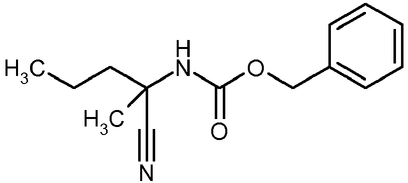

43.8 g (135.3 mmol) *rac*-Benzyl-(2-cyanpentan-2-yl)carbamat aus Beispiel 16A wurden durch präparative Trennung an chiraler Phase in die Enantiomere getrennt [Säule: SFC Chiralpak AZ-H, 5 µm, 250 x 50 mm, Eluent: 85% CO₂, 15% Methanol, Fluß: 250 ml/min; Temperatur: 28°C, backpressure: 100 bar, Detektion: 220 nm].
Enantiomer B: Ausbeute: 13.48 g (ca. 90.4% ee)
Rt= 3.93 min [SFC Chiralpak AZ-H, 5 µm, 250 x 4.6 mm; Eluent: 90% CO₂, 10% Methanol; Fluß: 3 ml/min; Detektion: 220 nm].

### Beispiel 19A

### ent-Benzyl-(1-amino-2-methylpentan-2-yl)carbamat (Enantiomer A)

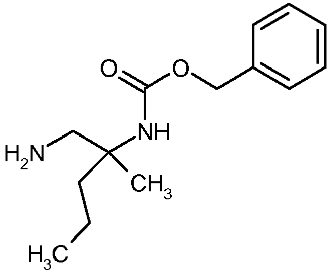

13.1 g (53.31 mmol) *ent*-Benzyl-(2-cyanpentan-2-yl)carbamat (Enantiomer A) aus Beispiel 17A wurden in 155 ml 7 N Ammoniak-Lösung in Methanol gelöst und unter Argon mit 16.5 g Raney-Nickel (50%ige wässrige Aufschlämmung) versetzt. Das Reaktionsgemisch wurde über Nacht im Autoklaven bei 20-30 bar hydriert. Die Reaktionsmischung wurde über Celite abfiltriert, mit Methanol, Dichlormethan/2 N Ammoniak in Methanol (20/1) gespült und eingeengt. Der Rückstand wurde mittels Kieselgel-Chromatographie (Laufmittel: Dichlormethan/Methanol 40/1nach 20/1) gereinigt. Es wurden 9.85 g der Zielverbindung (63% d. Th., Reinheit 86%) erhalten.
LC-MS (Methode 2): Rₜ = 0.58 min
MS (ESpos): m/z = 251 (M+H)⁺
¹H-NMR (400 MHz, DMSO-d₆): δ = 0.83 (t, 3H), 1.11 (s, 3H), 1.15 - 1.24 (m, 2H), 1.37 (br. s, 2H), 1.42 - 1.51 (m, 1H), 1.53 - 1.63 (m, 1H), 2.46 (d, 1H), 2.66 (d, 1H), 4.97 (s, 2H), 6.69 (br. s, 1H), 7.26 - 7.40 (m, 5H).

### Beispiel 20A

### ent-Benzyl-(1-amino-2-methylpentan-2-yl)carbamat (Enantiomer B)

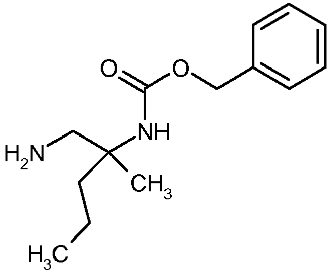

13.5 g (54.73 mmol) *ent*-Benzyl-(2-cyanpentan-2-yl)carbamat (Enantiomer B) aus Beispiel 18A wurden in 159 ml 7 N Ammoniak-Lösung in Methanol gelöst und unter Argon mit 16.95 g Raney-Nickel (50%ige wässrige Aufschlämmung) versetzt. Das Reaktionsgemisch wurde über Nacht im Autoklaven bei 20-30 bar hydriert. Die Reaktionsmischung wurde über Celite abfiltriert, mit Methanol, Dichlormethan/2 N Ammoniak in Methanol (10/1) gespült und eingeengt. Der Rückstand wurde mittels Kieselgel-Chromatographie (Laufmittel: Dichlormethan/Methanol 40/1nach 20/1) gereinigt. Es wurden 9.46 g der Zielverbindung (61% d. Th., Reinheit 88%) erhalten.
LC-MS (Methode 2): Rₜ = 0.58 min
MS (ESpos): m/z = 251 (M+H)⁺
¹H-NMR (400 MHz, DMSO-d₆): δ = 0.83 (t, 3H), 1.11 (s, 3H), 1.15 - 1.24 (m, 2H), 1.37 (br. s, 2H), 1.42 - 1.51 (m, 1H), 1.53 - 1.63 (m, 1H), 2.46 (d, 1H), 2.66 (d, 1H), 4.97 (s, 2H), 6.69 (br. s., 1H), 7.26 - 7.40 (m, 5H).

### Beispiel 21A

### ent-Benzyl-{1-[({7-[(2,6-difluorbenzyl)oxy]-2,5-dimethylpyrazolo[1,5-a]pyridin-3-yl}carbonyl)amino]-2-methylbutan-2-yl}carbamat (Enantiomer A)

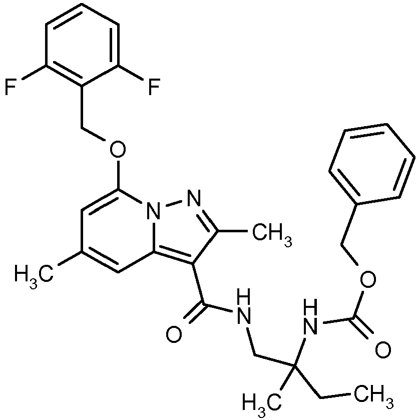

Eine Mischung von 74.7 mg (0.225 mmol, 1.0 eq.) 7-[(2,6-Difluorbenzyl)oxy]-2,5-dimethyl-pyrazolo[1,5-a]pyridin-3-carbonsäure aus Beispiel 4A, 106 mg (0.450 mmol, 2.0 eq.) *ent*-Benzyl-(1-amino-2-methylbutan-2-yl)carbamat aus Beispiel 14A (Enantiomer A) und 0.196 ml (1.12 mmol, 5.0 eq.) N,N-Diisopropylethylamin in 2.3 ml DMF wurden mit 111 mg (0.292 mmol, 1.3 eq.) HATU versetzt und 60 h bei RT gerührt. Dann wurde Wasser zugegeben und dreimal mit Essigsäureethylester extrahiert. Die vereinten organischen Phasen wurden mit gesättigter wässriger Natriumchloridlösung gewaschen, mit Magesiumsulfat getrocknet, filtriert und eingeengt. Das Rohprodukt wurde mittels Biotage Isolera (10 g Kieselgelkartusche, Cyclohexan /Essigsäureethylester-Gradient: 0 % nach 100 % Essigsäureethylester) gereinigt. Es wurden 81.7 mg der Titelverbindung (60 % d. Th., Reinheit 91%) erhalten.
DC (Kieselgel, Cyclohexan/Essigsäureethylester 10:1): R_{F} = 0.33
LC-MS (Methode 2): Rₜ = 1.24 min
MS (ESpos): m/z = 551 (M+H)⁺

### Beispiel 22A

### ent-Benzyl-{1-[({2,5-dimethyl-7-[(2,3,6-trifluorbenzyl)oxy]pyrazolo[1,5-a]pyridin-3-yl}carbonyl)amino]-2-methylbutan-2-yl}carbamat Trifluoracetat (Enantiomer A)

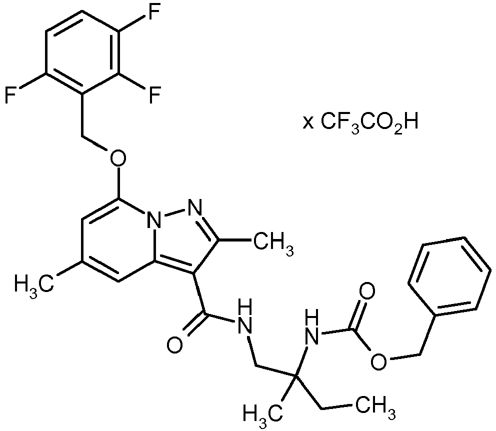

37.8 mg (0.11 mmol) 2,5-Dimethyl-7-[(2,3,6-trifluorbenzyl)oxy]pyrazolo[1,5-a]pyridin-3-carbonsäure aus Beispiel 10A wurden mit 49 mg (0.13 mmol) HATU und 0.1 ml (0.54 mmol) N,N-Diisopropylethylamin in 0.3 ml DMF vorgelegt und 10 min bei Raumtemperatur gerührt. Anschließend wurden 0.5 ml (0.16 mmol) *ent*-Benzyl-(1-amino-2-methylbutan-2-yl)carbamat aus Beispiel 14A (Enantiomer A) zur Reaktionslösung gegeben und 2 Stunden bei RT gerührt. Es wurden nochmals 24.5 mg (0.06 mmol) HATU zur Reakionslösung gegeben und über Nacht bei RT gerührt. Die Reaktionslösung wurde mit TFA versetzt und mittels präparativer HPLC gereinigt (RP18 Säule, Laufmittel: Acetonitril/Wasser-Gradient unter Zusatz von 0.1% TFA). Es wurden 36 mg der Zielverbindung (41% d. Th., Reinheit 83%) erhalten.
LC-MS (Methode 2): Rₜ = 1.26 min
MS (ESpos): m/z = 569 (M-TFA+H)⁺

### Beispiel 23A

### ent-Benzyl-{1-[({2,5-dimethyl-7-[(2,3,6-trifluorbenzyl)oxy]pyrazolo[1,5-a]pyridin-3-yl}carbonyl)amino]-2-methylpentan-2-yl}carbamat Trifluoracetat (Enantiomer B)

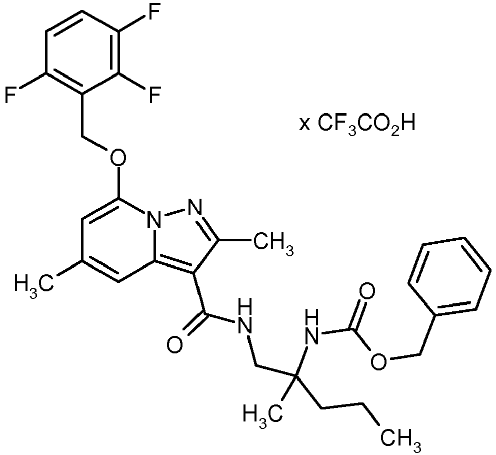

250 mg (0.68 mmol) 2,5-Dimethyl-7-[(2,3,6-trifluorbenzyl)oxy]pyrazolo[1,5-a]pyridin-3-carbonsäure aus Beispiel 10A wurden mit 309 mg (0.81 mmol) HATU und 0.59 ml (3.39 mmol) N,N-Diisopropylethylamin in 0.9 ml DMF vorgelegt und 10 min bei Raumtemperatur gerührt. Anschließend wurden 255 mg (1.02 mmol) *ent*-Benzyl-(1-amino-2-methylpentan-2-yl)carbamat aus Beispiel 20A (Enantiomer B) zur Reaktionslösung gegeben und über Nacht bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, eingeengt und lyophilisiert. Es wurden 353 mg der Zielverbindung (72% d. Th.) erhalten.
LC-MS (Methode 2): Rₜ = 1.31 min
MS (ESpos): m/z = 583 (M-TFA+H)⁺
¹H-NMR (400 MHz, DMSO-d₆): δ = 0.85 (t, 3H), 1.20 (s, 3H), 1.23 - 1.38 (m, 3H), 1.48 - 1.62 (m, 1H), 1.65 - 1.79 (m, 1H), 2.39 (s, 3H), 2.51 (s, 3H; unter Lösungsmittelpeak), 4.99 (s, 2H), 5.49 (s, 2H), 6.56 (s, 1H), 7.11 - 7.19 (m, 1H), 7.26 - 7.44 (m, 8H), 7.66 - 7.76 (m, 1H).

### Beispiel 24A

### 3,3,4,4,4-Pentafluorbutyltrifluormethansulfonat

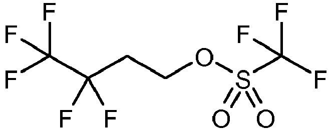

Unter Argon wurden 198.49 g (703.51 mmol) Trifluormethansulfonsäureanhydrid vorgelegt. Der Reaktionskolben wurde in ein 70°C heißes Ölbad getaucht und auf 56°C Innentemperatur erhitzt. 88.2 ml (738.68 mmol) 3,3,4,4,4-Pentafluorobutanol wurden innerhalb von 35 min zum Reaktionsgemisch getropft und das Gemisch wurde zwei Stunden bei 70-73°C Badtemperatur und 69°C Innentemperatur gerührt. Das Reaktionsgemisch wurde am Rotationsverdampfer aufkonzentriert und der Rückstand in 1500 ml Dichlormethan aufgenommen. Es wurde einmal mit 300 ml kaltem Wasser, einmal mit 300 ml kalter, wässriger gesättigter Natriumhydrogencarbonat-Lösung und einmal mit 300 ml kaltem Wasser gewaschen. Die organische Phase wurde mit Magnesiumsulfat getrocknet, abfiltriert und eingeengt. Man erhielt 192.86 g (92.6% d. Th.) der Zielverbindung.
¹H-NMR (400 MHz, DMSO-d₆): δ = 2.71 - 2.89 (m, 2H), 4.58 (t, 2H).

### Beispiel 25A

### rac-Methyl-5,5,6,6,6-pentafluornorleucinathydrochlorid (Racemat)

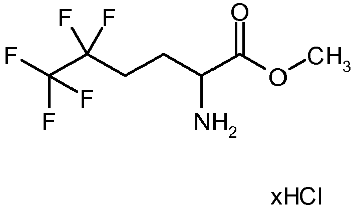

Unter Argon wurden 132 g (521.0 mmol) Methyl-N-(diphenylmethylen)glycinat [beschrieben in: WO2010/123792 A1, 2010; p. 11-13] in 1000 ml THF (wasserfrei) vorgelegt und auf -40°C abgekühlt. 625.2 ml (625.20 mmol) Bis-(trimethylsilyl)-lithiumamid (1 M in THF) wurden innerhalb von 30 min zugetropft. Nach 10 min bei -40°C wurde innerhalb von 35 min die Innentemperatur auf 0°C ansteigen gelassen. 192.86 g (651.25 mmol) 3,3,4,4,4-Pentafluorbutyltrifluormethansulfonat aus Beispiel 24A, gelöst in 400 ml THF wurden bei 0°C in die Reaktionslösung getropft. Nach 10 min wurde das Kältebad entfernt und das Gemisch wurde 3 Tage bei RT gerührt. Anschließend wurde die Reaktionsmischung auf 0°C abgekühlt und mit 410 ml (1.33 mol) 3 N wässriger Salzsäure tropfenweise versetzt. Das Kältebad wurde entfernt und die Reaktionslösung wurde zwei Stunden bei RT gerührt. Das Gemisch wurde anschließend eingeengt. Man erhielt 141.5 g der Zielverbindung als Rohgemisch (Reinheit unbekannt), welche ohne weitere Reinigung in die Folgestufe eingesetzt wurde.

### Beispiel 26A

### rac-Methyl-N-[(benzyloxy)carbonyl]-5,5,6,6,6-pentafluornorleucinat (Racemat)

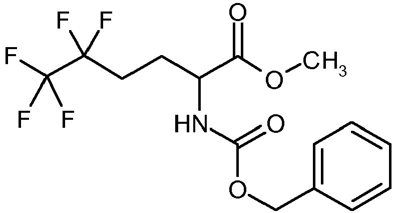

141.5 g (520.99 mmol) *rac*-Methyl-5,5,6,6,6-pentafluornorleucinathydrochlorid aus Beispiel 25A wurden unter Argon in 850 ml THF und 850 ml Wasser aufgenommen und vorsichtig mit 223.2 g (1.62 mol) Kaliumcarbonat bei RT versetzt. Anschließend wurden 82 ml (573.09 mmol) Chlorameisensäurebenzylester zugetropft und die Suspension über Nacht bei RT gerührt. Das Reaktionsgemisch wurde zweimal mit 500 ml Essigsäurethylester extrahiert, die organische Phase wurde mit Magnesiumsulfat getrocknet, abfiltriert und eingeengt. Der Rückstand wurde in 50 ml Dichlormethan verdünnt und mittels Kieselgel-Chromatographie (Laufmittel: Cyclohexan/Essigsäurethylester 9/1 nach 4/1) gereinigt. Die isolierten Produktfraktionen wurden nochmals mittels präparativer HPLC gereinigt [Säule: Daiso C18 10µm Bio 300 x 100mm, neutral; Eluent: Acetonitril/Wasser-Gradient; Fluss: 250 ml/min; Temperatur: RT; Wellenlänge: 210 nm). Man erhielt 27.4 g (14% d. Th.) der Zielverbindung.
LC-MS (Methode 2): Rₜ = 1.09 min.
MS (ESIpos): m/z = 370 (M+H)⁺.
¹H-NMR (400 MHz, DMSO-d₆): δ = 1.78 - 1.91 (m, 1H), 1.93 - 2.05 (m, 1H), 2.10 - 2.30 (m, 1H), 2.30 - 2.46 (m, 1H), 3.66 (s, 3H), 4.18 - 4.26 (m, 1H), 5.05 (s, 2H), 7.27 - 7.40 (m, 5H), 7.89 (d, 1H).

### Beispiel 27A

### rac-Benzyl-(6,6,7,7,7-pentafluor-2-hydroxy-2-methylheptan-3-yl)carbamat (Racemat)

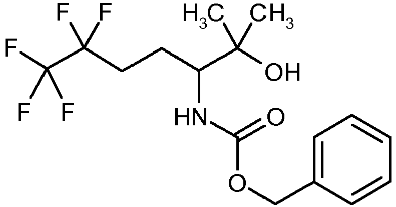

1.7 g (3.68 mmol, 80% rein) *rac*-Methyl-N-[(benzyloxy)carbonyl]-5,5,6,6,6-pentafluornorleucinat (Racemat) aus Beispiel 26A wurden unter Argon in THF vorgelegt und das Reaktionsgemisch wurde auf 0°C abgekühlt. 4.3 ml (12.89 mmol) 3M Methylmagnesiumbromid in Diethylether wurden zugetropft und es wurde 15 min bei 0°C nachgerührt. Dann ließ man das Gemisch langsam auf RT erwärmen und rührte über Nacht bei Raumtemperatur. Das Reaktionsgemisch wurde vorsichtig mit gesättigter wässriger Ammoniumchlorid-Lösung versetzt und dann die Reaktionslösung zur Hälfte eingeengt. Der Rückstand wurde zwischen Dichlormethan und Wasser verteilt, die organische Phase zweimal mit Wasser gewaschen, über Natriumsulfat getrocknet, filtriert und eingeengt. Der Rückstand wurde mittels Kieselgel-Chromatographie (Cyclohexan/Essigsäurethylester 10:1 nach 7:3) gereinigt. Man erhielt 1.31 g (96% d. Th.) der Zielverbindung.
LC-MS (Methode 2): Rₜ = 1.03 min.
MS (ESIpos): m/z = 370 (M+H)⁺.
¹H-NMR (400 MHz, DMSO-d₆): δ = 1.01 (s, 3H), 1.08 (s, 3H), 1.43 - 1.56 (m, 1H), 1.92 - 2.01 (m, 1H), 2.01 - 2.19 (m, 2H), 3.36 - 3.44 (m, 1H), 4.48 (s, 1H), 4.99 - 5.12 (m, 2H), 7.11 (d, 1H), 7.27 - 7.38 (m, 5H).

### Beispiel 28A

### ent-Benzyl-(6,6,7,7,7-pentafluor-2-hydroxy-2-methylheptan-3-yl)carbamat (Enantiomer A)

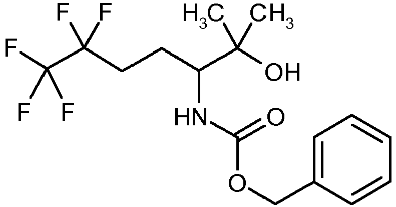

1.31 g von Beispiel 27A wurden durch präparative Trennung an chiraler Phase in die Enantiomere getrennt [Säule: Daicel Chiralpak AY-H, 5 µm, 250 x 20 mm, Eluent: 90% iso-Hexan, 10% Ethanol, Fluß: 15 ml/min; 35°C; Detektion: 220 nm].
Enantiomer A:
Ausbeute: 459 mg (99% ee)
Rₜ= 4.31 min [Daicel Chiralpak AY-H, 5µm, 250 x 4.6 mm; Eluent: 90% iso-Hexan, 10% Ethanol; Fluss: 1.0 ml/min; 30°C; Detektion: 220 nm].

### Beispiel 29A

### ent-3-Amino-6,6,7,7,7-pentafluor-2-methylheptan-2-olhydrochlorid (Enantiomer A)

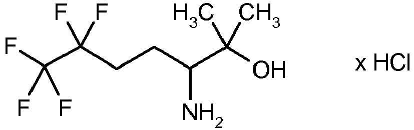

455 mg (1.23 mmol) *ent*-Benzyl-(6,6,7,7,7-pentafluor-2-hydroxy-2-methylheptan-3-yl)carbamat (Enantiomer A) aus Beispiel 28A wurden in 8.6 ml Ethanol vorgelegt, mit 131 mg Palladium auf Kohle (10%ig) und 3.74 ml (36.96 mmol) Cyclohexen versetzt und 3 h unter Rückfluss gerührt. Das Reaktionsgemisch wurde über einen Millipore-Filter filtriert und mit Ethanol gewaschen. Das Filtrat wurde mit 1.23 ml Chlorwasserstoff (2 N in Diethylether) versetzt, eingeengt und im Hochvakuum getrocknet. Es wurden 335 mg (98% d. Th.) der Zielverbindung erhalten.
MS (Methode 11): m/z = 236 (M-HCl+H)⁺
¹H-NMR (400 MHz, DMSO-d₆): δ = 1.11 (s, 3H), 1.22 (s, 3H), 1.58 - 1.72 (m, 1H), 1.80 - 1.92 (m, 1H), 2.27 - 2.46 (m, 2H, teilweise verdeckt durch DMSO-Peak), 2.94 - 3.04 (m, 1H), 5.35 (s, 1H), 7.80 - 8.01 (m, 3H).

### Beispiel 30A

### ent-N-[2-Methyl-6-(trifluormethyl)-1,2,3,4-tetrahydro-1,5-naphthyridin-4-yl]acetamid (Enantiomer A)

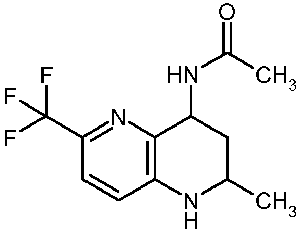

6.00 g (21.96 mmol) *rac*-N-[2-Methyl-6-(trifluormethyl)-1,2,3,4-tetrahydro-1,5-naphthyridin-4-yl]acetamid (beschrieben in: M.-C. Fernandez et al. Bioorg. Med. Chem. Lett. 2012, 22, 3056-3062) wurden durch präparative Trennung an der chiralen Phase in die Enantiomere getrennt [Säule: SFC Chiralpak AY-H, 20 µm, 360 x 50 mm, Eluent: 90% Kohlendioxid, 10% Methanol, Fluß: 400 ml/min; Temperatur: 38°C; backpressure: 80 bar; Detektion: 220 nm].
Enantiomer A: Ausbeute: 2.41 g (>99% ee)
Rₜ = 2.66 min [Daicel Chiralpak AY-H, 5 µm, 250 x 4.6 mm; Eluent: 90% Kohlendioxid, 10% Isopropanol; Fluss: 3 ml/min; Detektion: 210 nm].

### Beispiel 31A

### ent-2-Methyl-6-(trifluormethyl)-1,2,3,4-tetrahydro-1,5-naphthyridin-4-amin Hydrochlorid

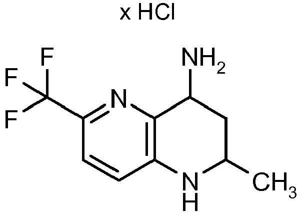

152 mg (0.56 mmol) *ent*-N-[2-Methyl-6-(trifluormethyl)-1,2,3,4-tetrahydro-1,5-naphthyridin-4-yl]acetamid (Enantiomer A) aus Beispiel 30A (beschrieben in: M.-C. Fernandez et al. Bioorg. Med. Chem. Lett. 2012, 22, 3056-3062) wurde mit 2.8 ml gesättigter Chlorwasserstofflösung in Methanol versetzt und in der Mikrowelle für 1 Stunde bei 80°C gerührt. Das Reaktionsgemisch wurde eingeengt und lyophilisiert. Es wurden 147 mg der Zielverbindung (97% d. Th., Reinheit ca. 98%) erhalten.
LC-MS (Methode 2): Rₜ = 0.40 min
MS (ESpos): m/z = 232 (M-HCl+H)⁺
¹H-NMR (400 MHz, DMSO-d₆): δ = 1.23 (d, 3H), 1.59 (q, 1H), 2.25 - 2.35 (m, 1H), 3.58 - 3.69 (m, 1H), 4.50 - 4.61 (m, 1H), 6.97 - 7.05 (m, 2H), 7.52 (d, 1H), 8.40 (br. s, 3H).

### Beispiel 32A

### rac-2-Amino-4-(benzyloxy)-2-methylbutanonitril

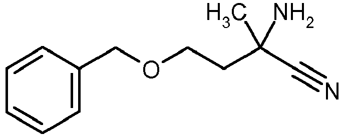

5.31 g (108.3 mmol) Natriumcyanid in 10 ml Wasser wurden mit 6.37 g (119.1 mmol) Ammoniumchlorid (gelöst in 15 ml warmem Wasser) und 9 ml (216.6 mmol) konz. Ammoniak in Wasser versetzt. Anschließend wurden 19.3 g (108.3 mmol) 4-(Benzyloxy)butan-2-on, gelöst in 3 ml Ethanol, zugegeben. Es wurde 15 min bei RT und 2 h bei 60°C gerührt. Erneut wurden 4 g (81.6 mmol) Natriumcyanid, 4.8g (89.7 mmol) Ammoniumchlorid und 6.5 ml (156.4 mmol) konz. Ammoniak in Wasser zugegeben und weitere 2 h bei 60°C gerührt. Dann wurde abgekühlt und die Reaktionslösung mit je 300 ml Methylenchlorid und Wasser versetzt. Nach Phasentrennung wurde die wässrige Phase mit 300 ml Methylenchlorid extrahiert. Die vereinigten organischen Phasen wurden getrocknet und eingeengt. Das Rohprodukt wurde über Kieselgel gereinigt (Cyclohexan/Essigsäureethylester-Gradient 6/4 - 1/1). Es wurden 19.9 g der Zielverbindung (77% Reinheit, 69% d. Th.) erhalten.
LC-MS (Methode 13): Rₜ = 2.31 min
MS (ESpos): m/z = 205 (M+H)⁺
¹H-NMR (400 MHz, DMSO-d₆): δ = 1.37 (s, 3H), 1.81 - 1.94 (m, 2H), 2.57 (br. s, 2H), 3.58 - 3.69 (m, 2H), 4.48 (s, 2H), 7.25 - 7.38 (m, 5H).

### Beispiel 33A

### rac-4-(Benzyloxy)-2-methylbutan-1,2-diamin

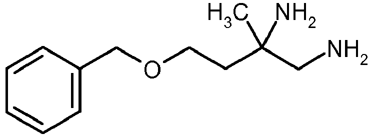

0.5 g (2.45 mmol) *rac*-2-Amino-4-(benzyloxy)-2-methylbutanonitril aus Beispiel 32A in 25 ml trockenem THF wurden unter Argon bei 0°C mit 1.59 ml (1.59 mmol) Lithiumaluminiumhydrid (1 N Lösung in Diethylether) versetzt. Die Reaktionslösung wurde erst 30 min bei 0°C gerührt und anschließend langsam auf Raumtemperatur kommend 1 h nachgerührt. Dann wurde vorsichtig mit 245 µl Wasser, 245 µl 2 N wässriger Natronlauge und 490 µl Wasser versetzt. Der Niederschlag wurde abfiltriert, mit THF und Methanol gewaschen, das Filtrat eingeengt und der Rückstand mittels Kieselgel-Chromatographie gereinigt (Laufmittel: Dichlormethan/2 N Ammoniak in Methanol = 20/1, isokratisch). Es wurden 0.30 g der Zielverbindung (96% Reinheit, 57% d. Th.) erhalten.
LC-MS (Methode 13): Rₜ = 1.94 min
MS (ESpos): m/z = 209 (M+H)⁺
¹H-NMR (400 MHz, DMSO-d₆): δ = 0.90 (s, 3H), 1.56 (t, 2H), 2.27 - 2.38 (m, 2H), 3.45 - 3.60 (m, 2H), 4.42 (s, 2H), 7.22 - 7.36 (m, 5H).

### Beispiel 34A

### rac-3-(3,4-Difluorphenoxy)-2-methylpropan-1,2-diamin

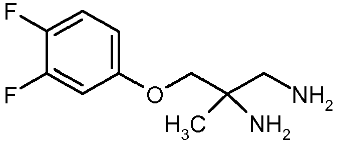

300 mg (1.41 mmol) *rac*-2-Amino-3-(3,4-difluorphenoxy)-2-methylpropanonitril wurden in 14.4 ml abs. THF vorgelegt und unter Argon bei 0°C mit 0.92 ml (0.92 mmol) einer 1 N Lithiumaluminiumhydrid-Lösung in Diethylether versetzt. Die Reaktionslösung wurde 30 min bei 0°C gerührt, dann ließ man langsam auf Raumtemperatur erwärmen und rührte über Nacht. Das Reaktionsgemisch wurde vorsichtig mit 140 µl Wasser, 140 µl 2 N wässriger Natriumhydroxidlösung und 280 µl Wasser versetzt, der Niederschlag wurde abfiltriert, mit THF und Methanol gewaschen, das Filtrat eingeengt und der Rückstand mittels Kieselgel-Chromatographie gereinigt (Laufmittel: Dichlormethan/2N Ammoniak in Methanol = 20/1). Es wurden 87 mg der Zielverbindung (24% d. Th.; Reinheit ca. 84%) erhalten.
LC-MS (Methode 7): Rₜ = 1.73 min
MS (ESpos): m/z = 217 (M+H)⁺

### Beispiel 35A

### 3-(Aminomethyl)oxetan-3-amin Dihydrochlorid

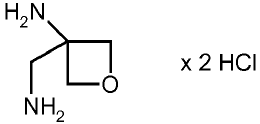

585 mg (2.07 mmol) 3-(Aminomethyl)-*N,N*-dibenzyloxetan-3-amin [Synthese beschrieben in: US2008/103183 A1, 2008; p. 48] wurden in Ethanol (29.2 ml) vorgelegt, mit 441 mg (0.41 mmol) 10%igem Palladium auf Aktivkohle und 6.3 ml (62.2 mmol) Cyclohexen versetzt. Die Reaktionsmischung wurde 8 h unter Rückfluss gerührt. Anschließend wurde über einen Millipore^{®}-Filter filtriert, mit Methanol gewaschen, das Filtrat mit 2.6 ml (5.2 mmol) 2 M Chlorwasserstoff in Diethylether versetzt, eingeengt und im Hochvakuum getrocknet. Man erhielt 423 mg (87% d. Th., Reinheit 75%) der Zielverbindung.
DCI-MS (Methode 12): m/z = 103 (M-2HCl+H)⁺

### Beispiel 36A

### 2-(Trifluormethyl)piperidin-4-amin-Hydrochlorid

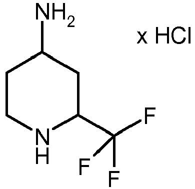

115 mg (0.43 mmol) *tert*-Butyl-[2-(trifluormethyl)piperidin-4-yl]carbamat wurde in 2.2 ml Diethylether vorgelegt, mit 2.14 ml (4.28 mmol) 2 N Salzsäure in Diethylether versetzt und über Nacht bei RT gerührt. Das Reaktionsgemisch wurde eingeengt und der Rückstand im Hochvakuum getrocknet. Es wurden 89 mg der Zielverbindung (101% d. Th.) erhalten.

### Beispiel 37A

### Methyl-3-(aminomethyl)adamantan-1-carboxylat

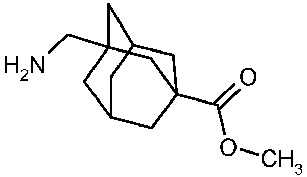

73 mg (0.30 mmol) 3-(Aminomethyl)adamantan-1-carbonsäure wurden in 1.48 ml Methanol vorgelegt und mit 1.48 ml 4 N Chlorwasserstoff-Lösung in Dioxan versetzt. Das Gemisch wurde 5 h unter Rückfluss gerührt. Das Gemisch wurde eingedampft und der Rückstand wurde mit gesättigter Natriumhydrogencarbonat-Lösung versetzt. Es wurde dreimal mit Ethylacetat extrahiert und die vereinten organischen Phasen wurden über Natriumsulfat getrocknet und anschließend eingedampft und getrocknet. Es wurden 55 mg der Zielverbindung (82% d. Th.) erhalten, welche ohne weitere Reinigung in die Folgestufe eingesetzt wurden.

### Beispiel 38A

### rac-3-(3,4-Difluorphenyl)-3-(1,3-dioxo-1,3-dihydro-2H-isoindol-2-yl)propansäure

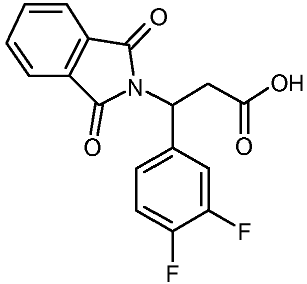

### 1.Stufe:

697 g 3,4-Difluorbenzaldehyd (4.76 mol, 1 Äquivalent) wurden mit 495 g Malonsäure (4.76 mol, 1 Äquivalent) und 733 g Ammoniumacetat (9.52 mol, 2 Äquivalente) in 2788 ml Ethanol 20 h bei Rückfluss unter Argon gerührt. Dann wurde auf RT abgekühlt und über Nacht bei RT gerührt. Die ausgefallenen Kristalle wurden abgesaut, mit Ethanol und Diethylether gewaschen und im Vakuum getrocknet. Es wurden 590 g (62% d. Th.) *rac*-3-Amino-3-(3,4-difluorphenyl)propansäure erhalten.
*rac*-3-Amino-3-(3,4-difluorphenyl)propansäure:
LC-MS (Methode 1): Rₜ = 0.27 min
MS (ESpos): m/z = 202.0 (M+H)⁺

### 2. Stufe:

0.20 g (0.99 mmol) *rac*-3-Amino-3-(3,4-difluorphenyl)propansäure und 0.15 g (0.99 mmol) Phthalsäureanhydrid wurden in 0.8 ml DMF gelöst und bei 135°C über Nacht zum Rückfluss erhitzt. Die Reaktionslösung wurde auf ca. 9 ml Wasser gegeben. Die resultierende Suspension wurde zweimal mit Essigsäureethylester extrahiert. Die vereinigten organischen Phasen wurden mit Wasser gewaschen, über Natriumsulfat getrocknet, filtriert und eingeengt. Das Rohprodukt wurde mittels präparativer HPLC gereinigt (RP18 Säule, Laufmittel: Acetonitril/Wasser-Gradient unter Zusatz von 0.1% TFA). Es wurden 0.2 g der Titelverbindung (61% d. Th.) erhalten.
LC-MS (Methode 2): Rₜ = 0.97 min
MS (ESpos): m/z = 332 (M+H)⁺
¹H-NMR (400 MHz, DMSO-d₆): δ = 3.24-3.3.33 (m, 1H), 3.44-3.52 (m, 1H), 5.63-5.70 (m, 1H), 7.23-7.28 (m, 1H), 7.36-7.47 (m, 1H), 7.49-7.57 (m, 1H), 7.82-7.90 (m, 4H), 12.51 (br s, 1H).

### Beispiel 39A

### rac-tert.-Butyl-[2-(3,4-difluorphenyl)-2-(1,3-dioxo-1,3-dihydro-2H-isoindol-2-yl)ethyl]carbamat

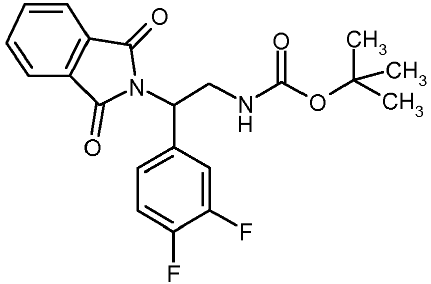

Unter Argon wurde eine Lösung aus 5.0 g *rac*-3-(3,4-Difluorphenyl)-3-(1,3-dioxo-1,3-dihydro-2H-isoindol-2-yl)propansäure (Beispiel 38A, 15.09 mmol) und 3.06 g Triethylamin (30.19 mmol) in 65 ml Toluol vorgelegt, mit 4.36 g Diphenylphosphorazidat (15.85 mmol) versetzt und 3.5 h bei RT gerührt. Anschließend wurden 65 ml tert.-Butanol zugegeben und über Nacht unter Rückfluss gerührt. Nach dem Abkühlen wurde die Reaktionslösung eingeengt und mittels Flashchromatographie gereinigt (Laufmittel: Petrolether/Essigsäureethylester 2:1, isokratisch). Es wurden 3.1 g der Titelverbindung (45% d. Th.) erhalten.
LC-MS (Methode 2): Rₜ = 1.19 min
MS (ESpos): m/z = 403 (M+H)⁺
¹H-NMR (400 MHz, DMSO-d₆): δ = 1.26 (s, 9H), 3.73-3.90 (m, 2H), 5.32-5.39 (m, 1H), 7.20-7.27 (m, 2H), 7.36-7.46 (m, 1H), 7.48-7.56 (m, 1H), 7.81-7.91 (m, 4H).

### Beispiel 40A

### rac-tert.-Butyl-[2-amino-2-(3,4-difluorphenyl)ethyl]carbamat

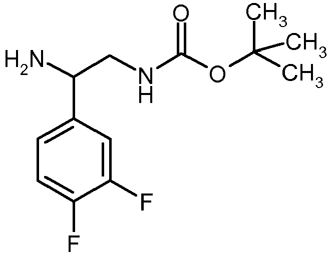

6.13 g *rac*-tert.-Butyl-[2-(3,4-difluorphenyl)-2-(1,3-dioxo-1,3-dihydro-2H-isoindol-2-yl)ethyl]carbamat (Beispiel 39A, Reinheit ca. 60%, ca. 9.14 mmol) wurde in 13.1 ml 40%iger, wässriger Methylamin-Lösung vorgelegt und über Nacht bei 60°C in einem verschlossenen Gefäß gerührt. Das Reaktionsgemisch wurde eingeengt und der Rückstand mittels Kieselgel-Chromatographie gereinigt (Laufmittel: Dichlormethan:Methanol:Diethylamin 30:1:0.1; 20:1:0.1). Es wurden 1.83 g der Titelverbindung (74% d. Th.) erhalten.
LC-MS (Methode 1): Rₜ = 0.65 min
MS (ESpos): m/z = 273 (M+H)⁺
¹H-NMR (400 MHz, DMSO-d₆): δ = 1.33 (s, 9H), 1.96 (br s, 2H), 2.92-3.10 (m, 2H), 3.81-3.88 (m, 1H), 6.76-6.82 (m, 1H), 7.11-7.17 (m, 1H), 7.27-7.40 (m, 2H).

### Beispiel 41A

### ent- tert.-Butyl-[2-amino-2-(3,4-difluorphenyl)ethyl]carbamat (Enantiomer A)

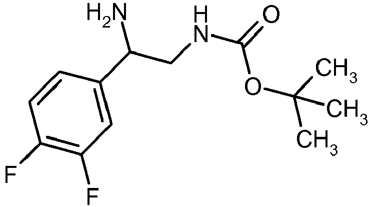

100 mg *rac*-tert.-Butyl-[2-amino-2-(3,4-difluorphenyl)ethyl]carbamat (Beispiel 40A) wurden an chiraler Phase in die Enantiomere getrennt [Säule: Daicel Chiralpak AY-H, 5 µm, 250 x 20 mm, Eluent: 80% iso-Hexan, 20% Ethanol + 0.2% Diethylamin, Fluß 15 ml/min; 30°C, Detektion: 220 nm].
Ausbeute: 43 mg Enantiomer A (99% Reinheit, >99% ee)
Rt= 4.58 min [Daicel Chiralpak AY-H, 5µm, 250 x 4.6 mm; Eluent: 80% iso-Hexan, 20% Ethanol + 0.2% Diethylamin; Fluss 1.0 ml/min; 30°C; Detektion: 220 nm].

### Beispiel 42A

### rac-2-Amino-4,4,4-trifluorbutan-1-ol

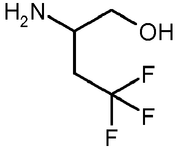

12.9 ml 2 M Lithiumborhydrid-Lösung in THF (25.8 mmol, 2.5 Äquivalente) wurden in 20 ml THF vorgelegt, mit 6.5 ml Chlortrimethylsilan (51.1 mmol, 5 Äquivalente) versetzt und es wurde 5 min bei RT gerührt. Dann wurden portionsweise 2.0 g *rac*-2-Amino-4,4,4-trifluorbutansäurehydrochlorid (10.3 mmol, 1 Äquivalent) zugegeben und es wurde über Nacht bei RT gerührt. Dann wurde tropfenweise mit 20.4 ml Methanol versetzt und nach beendeter Zugabe eingeengt. Der Rückstand wurde mit 12 ml einer 20%-igen wässrigen Kaliumhydroxid-Lösung versetzt und dreimal mit Dichlormethan extrahiert. Die vereinigten organischen Lösungen wurden über Natriumsulfat getrocknet, filtriert und eingeengt. Es wurden 1.58 g (96% d. Th.; Reinheit 90%) der Titelverbindung erhalten.
MS (Methode 11): m/z = 144.0 (M+H)⁺
¹H-NMR (400 MHz, DMSO-d₆): δ = 1.54 (br. s, 2 H), 1.97 - 2.15 (m, 1 H), 2.28 - 2.45 (m, 1 H), 2.84 - 2.98 (m, 1 H), 3.24 (d, 2 H), 4.78 (br. s, 1 H).

### Beispiel 43A

### rac-Benzyl-(4,4,4-trifluor-1-hydroxybutan-2-yl)carbamat

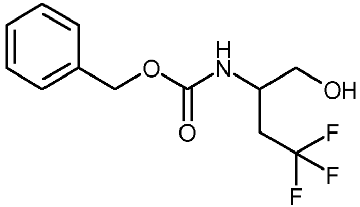

400 mg *rac*-2-Amino-4,4,4-trifluorbutan-1-ol (Beispiel 42A, 2.5 mmol, Reinheit ca. 90%, 1 Äquivalent) in 36 ml 1,4-Dioxan wurden bei RT mit 0.38 ml 50%-iger wässriger Kaliumcarbonat-Lösung (1.7 mmol, 0.68 Äquivalente) und 0.54 ml Chlorameisensäurebenzylester (3.8 mmol, 1.5 Äquivalente) versetzt und es wurde 2 h bei RT gerührt. Dann wurden 0.11 ml Chlorameisensäurebenzylester (0.76 mmol, 0.3 Äquivalente) und 0.08 ml 50%-iger wässriger Kaliumcarbonat-Lösung (0.35 mmol, 0.14 Äquivalente) nachgegeben und weitere 30 min bei RT gerührt. Nach Einengen im Vakuum wurde mittels präparative HPLC (RP18 Säule, Laufmittel: Acetonitril/Wasser-Gradient unter Zusatz von 0.1% TFA) gereinigt. Das erhaltene Rohprodukt wurde in Essigsäureethylester aufgenommen und zweimal mit gesättigter, wässriger Natriumhydrogencarbonat-Lösung gewaschen. Die wässrige Phase wurde mit Essigsäureethylester extrahiert und die vereinigten organischen Phasen wurden über Natriumsulfat getrocknet, filtriert und eingeengt. Es wurden 490 mg (70% d. Th.) der Titelverbindung erhalten.
LC-MS (Methode 2): Rₜ = 0.81 min
MS (ESpos): m/z = 278.1 (M+H)⁺
¹H-NMR (400 MHz, DMSO-d₆): δ = 2.20 - 2.38 (m, 1 H), 3.19 - 3.27 (m, 1 H), 3.28 - 3.42 (m, 2 H), 3.72 - 3.83 (m, 1 H), 4.96 - 5.08 (m, 3 H), 7.26 - 7.39 (m, 5 H).

### Beispiel 44A

### rac-Benzyl-[1-(1,3-dioxo-1,3-dihydro-2H-isoindol-2-yl)-4,4,4-trifluorbutan-2-yl]carbamat

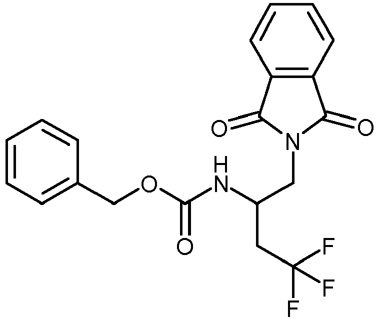

Unter Argon wurden 1.58 g *rac*-Benzyl-(4,4,4-trifluor-1-hydroxybutan-2-yl)carbamat (Beispiel 43A, 5.70 mmol, 1 Äquivalent), 0.84 g 1H-Isoindol-1,3(2H)-dion (Phthalimid, 5.70 mmol, 1 Äquivalente) und 2.24 g Triphenylphosphin (8.54 mmol, 1.5 Äquivalente) in 28 ml THF gelöst und bei RT mit 1.84 g Di-tert.-butyl-(*E*)-diazen-1,2-dicarboxylat (DIAD, 8.54 mmol, 1.5 Äquivalente) versetzt. Es wurde 30 min bei RT gerührt und dann mit einem Wasser/Acetonitril Gemisch versetzt. Reinigung mittels präparativer HPLC (RP18 Säule, Laufmittel: Acetonitril/Wasser-Gradient unter Zusatz von 0.1 % TFA) lieferte 1.92 g (79% d. Th.) der Titelverbindung.
LC-MS (Methode 2): Rₜ = 1.08 min
MS (ESpos): m/z = 407.2 (M+H)⁺

### Beispiel 45A

### rac-Benzyl-(1-amino-4,4,4-trifluorbutan-2-yl)carbamat Trifluoracetat

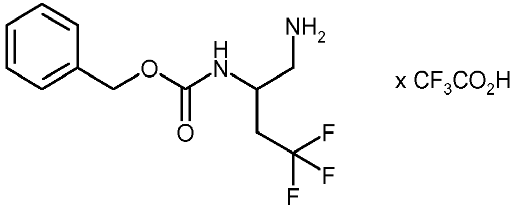

1.86 g *rac*-Benzyl-[1-(1,3-dioxo-1,3-dihydro-2H-isoindol-2-yl)-4,4,4-trifluorbutan-2-yl]carbamat (Beispiel 44A, 4.35 mmol, 1 Äquivalent) wurden in 15.0 ml 40%iger wässriger MethylaminLösung (174 mmol, 40 Äquivalente) gelöst und 30 min bei 60°C in einem geschlossenen Kolben gerührt. Die Reaktionsmischung wurde eingeengt und der Rückstand wurde anschließend über die präparative HPLC (RP18 Säule, Laufmittel: Acetonitril/Wasser-Gradient unter Zusatz von 0.1% TFA) gereinigt. Es wurden 1.25 g (74% d. Th.) der Titelverbindung erhalten.
LC-MS (Methode 2): Rₜ = 0.64 min
MS (ESpos): m/z = 277.2 (M+H)⁺
¹H-NMR (400 MHz, DMSO-d₆): δ = 2.57 - 2.71 (m, 1 H), 2.76 - 2.89 (m, 1 H), 2.93 - 3.04 (m, 1 H), 4.00 - 4.14 (m, 1 H), 5.02 (d, 1 H), 5.09 (d, 1 H), 7.27 - 7.42 (m, 5 H), 7.47 - 7.53 (m, 1 H), 7.88 - 8.08 (br. s, 3 H), [weiteres Signal unter DMSO Peak verborgen].

### Beispiel 46A

### rac-4,4,4-trifluorobutane-1,2-diamine Dihydrochlorid

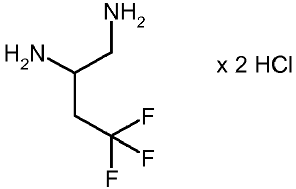

1.99 g (5.10 mmol) *rac*-Benzyl-(1-amino-4,4,4-trifluorbutan-2-yl)carbamat Trifluoracetat (Beispiel 45A) wurden in 130 ml Methanol gelöst. Es wurden 543 mg Palladium auf Kohle (10%ig) bei RT hinzugegeben und das Gemisch wurde über Nacht unter Normaldruck hydriert. Die Reaktionsmischung wurde über Celite filtriert und es wurde mit Methanol gewaschen. Das Filtrat wurde mit 5.1 ml Chlorwasserstoff-Lösung (2 N in Diethylether) versetzt, anschließend am Rotationsverdampfer eingedampft und im Vakuum getrocknet. Es wurden 1.10 g (100% d. Th.) der Titelverbindung erhalten.
MS (Methode 11): m/z = 143.2 (M-2HCl+H)⁺
¹H-NMR (500 MHz, DMSO-d₆): δ = 2.75 - 2.88 (m, 1 H), 2.90 - 3.02 (m, 1 H), 3.11 - 3.19 (m, 1 H), 3.21 - 3.29 (m, 1 H), 3.78 - 3.86 (M, 1 H), 8.69 (br. s, 6 H).

### Beispiel 47A

### 2-Methyl-4-phenyl-2-(trifluormethyl)-1,3-oxazolidin (Diastereomere)

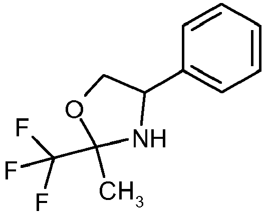

55.13 g (492.0 mmol) 1,1,1-Trifluoraceton in Toluol (1.35 1) wurden mit 45 g (328.0 mmol) *rac*-2-Amino-2-phenylethanol und 8.24 g (32.8 mmol) Pyridinium-p-toluolsulfonat versetzt. Die Reaktionsmischung wurde 16 h unter Rückfluss am Wasserabscheider gekocht. Das Gemisch wurde auf 0°C abgekühlt, der entstandene Feststoff abfiltriert und im Hochvakuum getrocknet. Man erhielt 68.6 g (77% d. Th., Reinheit 85%) der Zielverbindung.
LC-MS (Methode 2): Rₜ = 0.99 min
MS (ESIpos): m/z = 232 (M+H)⁺
¹H-NMR (400 MHz, DMSO-d₆): δ = 1.54 (s, 3H), 3.56 (t, 1H), 3.81 (d, 1H), 4.28 (t, 1H), 4.35 - 4.43 (m, 1H), 7.29 - 7.47 (m, 5H).

### Beispiel 48A

### 3,3,3-Trifluor-2-[(2-hydroxy-1-phenylethyl)amino]-2-methylpropanonitril (Diastereomere)

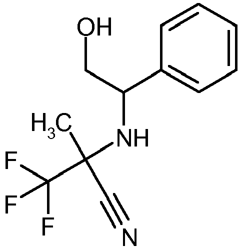

52.8 g (228.3 mmol) 2-Methyl-4-phenyl-2-(trifluormethyl)-1,3-oxazolidin (Diastereomere) aus Beispiel 47A wurden unter Argon in Dichlormethan (2 1) vorgelegt und auf 0°C abgekühlt. 42.85 ml (342.5 mmol) Trimethylsilylcyanid und 42.1 ml (342.5 mmol) Bortrifluorid-diethylether-Komplex wurden langsam zugegeben und es wurde 16 h bei RT gerührt. Anschließend wurde die Reaktionslösung in 1.5 1 gesättigte Natriumhydrogencarbonat-Lösung gegossen. Anschließend wurde noch mit 400 g Natriumhydrogencarbonat versetzt und die Lösung mit konz. Natronlauge auf pH 10 gestellt. Die wässrige Lösung wurde dreimal mit 500 ml Dichlormethan extrahiert, die vereinigten organischen Phasen über Natriumsulfat getrocknet, abfiltriert und eingeengt. Man erhielt 56.8 g (96% d. Th., 2 Diastereomere) der Zielverbindung.
LC-MS (Methode 2): Rₜ = 0.89 min und 0.93 min.
MS (ESIneg): m/z = 303 (M-H+HCOOH)⁻

### Beispiel 49A

### 2-[(3-Amino-1,1,1-trifluor-2-methylpropan-2-yl)amino]-2-phenylethanol (Diastereomere)

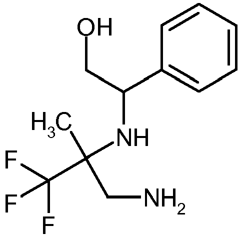

31 g (120.0 mmol) 3,3,3-Trifluor-2-[(2-hydroxy-1-phenylethyl)amino]-2-methylpropanonitril aus Beispiel 48A wurde in tert.-Butylmethylether (3.1 1) vorgelegt, auf 0°C abgekühlt, mit 18.25 g (480.2 mmol) Lithiumaluminiumhydrid versetzt und die Reaktionsmischung 16 h bei RT gerührt. Anschließend wurde auf 0°C abgekühlt, erst mit 24 ml Wasser gequencht, dann mit 24 ml 15%iger wässriger Kaliumhydroxid-Lösung und 48 ml Wasser versetzt. Die entstandene Mischung wurde über Kieselgel filtriert und mit tert.-Butylmethylether gewaschen. Die organische Phase wurde abgetrennt, über Natriumsulfat getrocknet, filtriert und eingeengt. Man erhielt 29.2 g (83% d. Th., Reinheit 89%) der Zielverbindung.
LC-MS (Methode 2): Rₜ = 0.52 min
MS (ESIpos): m/z = 263 (M+H)⁺

### Beispiel 50A

### tert.-Butyl-{3,3,3-trifluor-2-[(2-hydroxy-1-phenylethyl)amino]-2-methylpropyl}carbamat (Diastereomere)

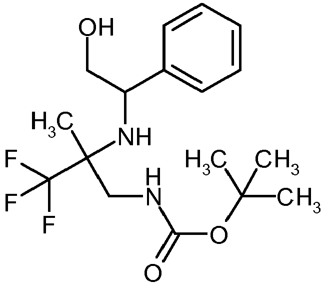

26.2 g (99.9 mmol) 2-[(3-Amino-1,1,1-trifluor-2-methylpropan-2-yl)amino]-2-phenylethanol (Diastereomere) aus Beispiel 49A in THF (500 ml) wurden mit 29.1 ml (209.8 mmol) Triethylamin und 23.98 g (109.9 mmol) Di-tert.-butyldicarbonat (gelöst in 286 ml THF) versetzt. Die Reaktionsmischung wurde 16 h bei RT gerührt. Anschließend wurde eingeengt und in jeweils 500 ml gesättigter, wässriger Natriumhydrogencarbonat-Lösung und Essigsäureethylester aufgenommen. Die Phasen wurden getrennt, die organische Phase über Natriumsulfat getrocknet, abfiltriert und eingeengt. Man erhielt 39.80 g (110% d. Th.) der Zielverbindung, welche ohne weitere Reinigung in die nächste Stufe eingesetzt wurde.
FIA-MS (Methode 11, ESpos): m/z = 363 (M+H)⁺

### Beispiel 51A

### rac-tert.-Butyl-(2-amino-3,3,3-trifluor-2-methylpropyl)carbamat

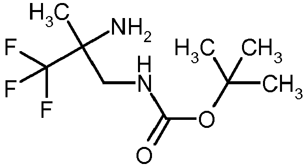

39 g (107.6 mmol) tert.-Butyl-{3,3,3-trifluor-2-[(2-hydroxy-1-phenylethyl)amino]-2-methylpropyl}carbamat aus Beispiel 50A wurden unter Argon in Ethanol (700 ml) vorgelegt und mit 5.44 g (53.8 mmol) Palladium(II)hydroxid (20% auf Aktivkohle, wasserfeucht ca. 60%ig) versetzt. Die Reaktionsmischung wurde 16 h bei Normaldruck hydriert. Dann wurde über Kieselgel filtriert und eingeengt. Der Rückstand wurde mittels Kieselgelchromatographie (Cyclohexan/Essigsäureethylester-Gradient: 9/1 nach 6/4) gereinigt. Man erhielt 15.8 g (61% d. Th.) der Zielverbindung.
FIA-MS (Methode 11, ESpos): m/z = 243 (M+H)⁺
¹H-NMR (400 MHz, CDCl₃): δ = 1.22 (s, 3H), 1.45 (s, 9H), 3.13 - 3.23 (m, 1H), 3.37 - 3.48 (m, 1H), 4.89 (br. s, 1H).

### Beispiel 52A

### rac-3,3,3-Trifluor-2-methylpropan-1,2-diamindihydrochlorid

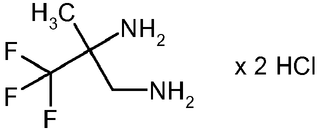

15 g (61.9 mmol) *rac*-tert.-Butyl-(2-amino-3,3,3-trifluor-2-methylpropyl)carbamat aus Beispiel 51A in Dioxan (188 ml) wurden mit 188 ml 4 M Chlorwasserstoff in Dioxan versetzt. Die Reaktionsmischung wurde 16 h bei RT gerührt, dann eingeengt und unter Argon aufbewahrt. Man erhielt 14.4 g (108% d. Th.) der Zielverbindung, die nicht weiter gereinigt wurde.
FIA-MS (Methode 11, ESpos): m/z = 143 (M-2HCl+H)⁺
¹H-NMR (400 MHz, D₂O) δ = 1.40 (s, 3H), 3.21 - 3.31 (m, 2H).

### Beispiel 53A

### rac-2-Amino-3-fluor-2-methylpropanonitril

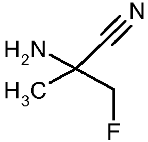

Die Titelverbindung ist literaturbekannt:
1) McConathy, J. et al., Journal of Medicinal Chemistry 2002, 45, 2240-2249.
2) Bergmann, E.D. et al., Journal of the Chemical Society 1963, 3462-3463.

### Weitere Methode:

1.0 g (0.94 ml; 13.15 mmol) Fluoraceton wurden in 11 ml 2 N Ammoniak in Methanol vorgelegt. Bei RT wurden nacheinander 721 mg (14.72 mmol) Natriumcyanid und 788 mg (14.72 mmol) Ammoniumchlorid zugeben und das Gemisch wurde 2 Stunden bei Rückfluss gerührt. Die Reaktionslösung wurde abgekühlt, filtriert und mit Methylenchlorid gewaschen. Aus der Mutterlauge fiel ein Feststoff aus, der abfiltiert wurde. Aus der Mutterlauge wurden Methylenchlorid und Methanol bei Normaldruck abdestilliert. Es wurden 1.32 g der Zielverbindung (89% d. Th., Reinheit ca. 90%) erhalten. Das Produkt wurde ohne weitere Reinigung in die nächste Reaktion eingesetzt.
GC-MS (Methode 14): Rₜ = 1.64 min
MS (EIpos): m/z = 87 (M-CH₃)⁺

### Beispiel 54A

### rac-Benzyl-(2-cyan-1-fluorpropan-2-yl)carbamat

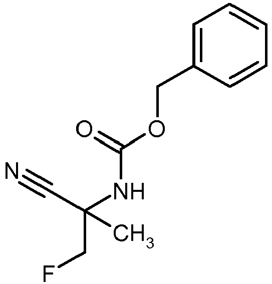

1.34 g (11.83 mmol, ca. 90%ig) *rac*-2-Amino-3-fluor-2-methylpropanonitril aus Beispiel 53A wurden in 29 ml THF/Wasser (9/1) mit 5.07 g (36.67 mmol) Kaliumcarbonat versetzt. Bei 0°C wurden langsam 1.69 ml (11.83 mmol) Chlorameisensäurebenzylester zugetropft und die Reaktionsmischung wurde über Nacht bei RT gerührt. Das Lösungsmittel wurde abdekantiert und die wässrige Phase zweimal mit THF ausgeschüttelt und das THF anschließend abdekantiert. Die vereinigten organischen Phasen wurden mit Natriumsulfat getrocknet, abfiltriert und eingeengt. Der Rückstand wurde mittels Kieselgelchromatographie getrennt (Laufmittel: Cyclohexan/Essigsäureethylester-Gradient) und die Produktfraktionen wurden am Rotationsverdampfer eingedampft. Es wurden 1.89 g der Zielverbindung (66% d. Th.; Reinheit 97%) erhalten.
LC-MS (Methode 2): Rₜ = 0.89 min
MS (ESpos): m/z = 237 (M+H)⁺
¹H-NMR (400 MHz, DMSO-d₆): δ = 1.58 (d, 3H), 4.47 - 4.78 (m, 2H), 5.10 (s, 2H), 7.30 - 7.43 (m, 5H), 8.34 (br. s, 1H).

### Beispiel 55A

### ent-Benzyl-(2-cyan-1-fluorpropan-2-yl)carbamat (Enantiomer A)

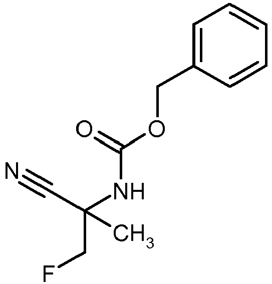

3.0 g (12.69 mmol) *rac*-Benzyl-(2-cyan-1-fluorpropan-2-yl)carbamat aus Beispiel 54A wurden durch präparative Trennung an chiraler Phase in die Enantiomere getrennt [Säule: Daicel Chiralpak AY-H, 5 µm, 250 x 20 mm, Eluent: 80% Iso-Hexan, 20% Isopropanol, Fluß: 15 ml/min; 40°C, Detektion: 220 nm].
Enantiomer A: Ausbeute: 1.18 g (>99% ee)
Rₜ = 5.37 min [Daicel Chiralcel AY-H, 5 µm, 250 x 4.6 mm; Eluent: 70% iso-Hexan, 30% 2-Propanol; Fluss 1.0 ml/min; 40°C; Detektion: 220 nm].

### Beispiel 56A

### ent-Benzyl-(2-cyan-1-fluorpropan-2-yl)carbamat (Enantiomer B)

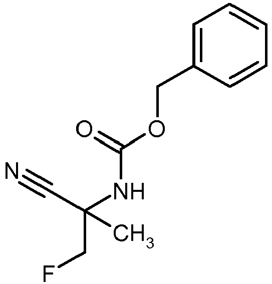

3.0 g (12.69 mmol) *rac*-Benzyl-(2-cyan-1-fluorpropan-2-yl)carbamat aus Beispiel 54A wurden durch präparative Trennung an chiraler Phase in die Enantiomere getrennt [Säule: Daicel Chiralpak AY-H, 5 µm, 250 x 20 mm, Eluent: 80% Iso-Hexan, 20% Isopropanol, Fluß: 15 ml/min; 40°C, Detektion: 220 nm].
Enantiomer B: Ausbeute: 1.18 g (>99% ee)
Rₜ = 6.25 min [Daicel Chiralcel AY-H, 5 µm, 250 x 4.6 mm; Eluent: 70% iso-Hexan, 30% 2-Propanol; Fluss 1.0 ml/min; 40°C; Detektion: 220 nm].

### Beispiel 57A

### rac-Benzyl-(1-amino-3-fluor-2-methylpropan-2-yl)carbamat

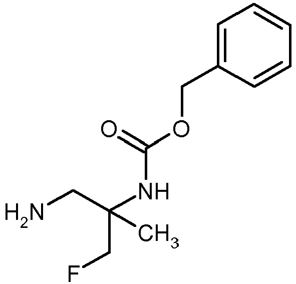

Unter Argon wurden 1.2 g (5.08 mmol) *rac*-Benzyl-(2-cyan-1-fluorpropan-2-yl)carbamat aus Beispiel 54A in 14.9 ml 7 N Ammoniak in Methanol mit 1.55 g Raney-Nickel (wässrige Aufschlämmung) versetzt und 24 Stunden bei ca. 25 bar Wasserstoff-Druck und RT hydriert. Das Reaktionsgemisch wurde über Kieselgur filtriert, mit Methanol gewaschen und eingeengt. Es wurden 1.2 g der Zielverbindung (98% d. Th.) erhalten.
LC-MS (Methode 2): Rₜ = 0.49 min
MS (ESpos): m/z = 241 (M+H)⁺

### Beispiel 58A

### ent-Benzyl-(1-amino-3-fluor-2-methylpropan-2-yl)carbamat (Enantiomer A)

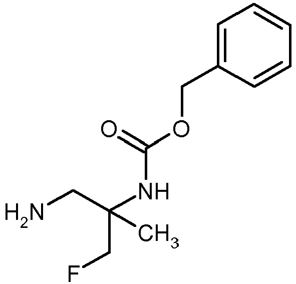

Unter Argon wurden 1.2 g (5.08 mmol) *ent*-Benzyl-(2-cyan-1-fluorpropan-2-yl)carbamat (Enantiomer A) aus Beispiel 55A in 14.9 ml 7 N Ammoniak in Methanol mit 1.55 g Raney-Nickel (wässrige Aufschlämmung) versetzt und 24 Stunden bei ca. 25 bar Wasserstoff-Druck und RT hydriert. Das Reaktionsgemisch wurde über Kieselgur filtriert, mit Methanol gewaschen und eingeengt. Es wurden 700 mg der Zielverbindung (57% d. Th.; Reinheit ca. 85%) erhalten.
LC-MS (Methode 2): Rₜ = 0.52 min
MS (ESpos): m/z = 241 (M+H)⁺

### Beispiel 59A

### ent-Benzyl-(1-amino-3-fluor-2-methylpropan-2-yl)carbamat (Enantiomer B)

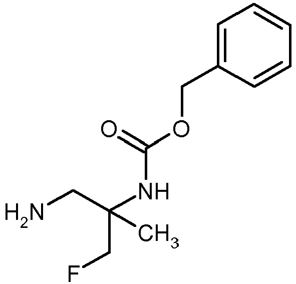

Unter Argon wurden 1.2 g (5.08 mmol) *ent*-Benzyl-(2-cyan-1-fluorpropan-2-yl)carbamat (Enantiomer B) aus Beispiel 56A in 14.9 ml 7 N Ammoniak in Methanol mit 1.55 g Raney-Nickel (wässrige Aufschlämmung) versetzt und 24 Stunden bei ca. 25 bar Wasserstoff-Druck und RT hydriert. Das Reaktionsgemisch wurde über Kieselgur filtriert, mit Methanol gewaschen und eingeengt. Es wurden 1.2 g der Zielverbindung (98% d. Th.; Reinheit ca. 85%) erhalten.
LC-MS (Methode 2): Rₜ = 0.50 min
MS (ESpos): m/z = 241 (M+H)⁺

### Beispiel 60A

### rac-2-Amino-5,5,5-trifluor-2-methylpentanonitril

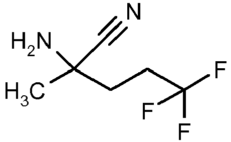

8.0 g (57.1 mmol) 5,5,5-Trifluorpentan-2-on [CAS Registry Nummer: 1341078-97-4; käuflich erhältlich oder das Methylketon kann nach literaturbekannten Methoden, welche dem Fachmann bekannt sind z. B. über a) zwei Stufen aus 4,4,4-Trifluorobutanal nach Y. Bai et al. Angewandte Chemie 2012, 51, 4112-4116; K. Hiroi et al. Synlett 2001, 263-265; K. Mikami et al. 1982 Chemistry Letters, 1349-1352; b) oder aus 4,4,4-Trifluorbutansäure nach A. A. Wube et al. Bioorganic and Medicinal Chemistry 2011, 19, 567-579; G. M. Rubottom et al. Journal of Organic Chemistry 1983, 48, 1550-1552; T. Chen et al. Journal of Organic Chemistry 1996, 61, 4716-4719, hergestellt werden. Die Isolierung des Produktes kann durch Destillation oder Chromatographie erfolgen] wurden in 47.8 ml 2 N Ammoniak in Methanol vorgelegt und bei Raumtemperatur mit 3.69 g (75.4 mmol) Natriumcyanid sowie 4.03 g (75.4 mmol) Ammoniumchlorid versetzt und 4 Stunden unter Rückfluss gerührt. Das Reaktionsgemisch wurde abgekühlt, mit Diethylether versetzt und der enthaltene Feststoff wurde abfiltriert. Das Lösungsmittel aus dem Filtrat wurde unter Normaldruck abdestilliert. Es wurden 8.7 g der Titelverbindung (92 % d. Th.) als Rückstand erhalten, der ohne weitere Reinigung in die Folgestufe eingesetzt wurde.
GC-MS (Methode 14): Rₜ = 1.90 min
MS (ESpos): m/z = 151 (M-CH₃)⁺

### Beispiel 61A

### rac-Benzyl-(2-cyan-5,5,5-trifluorpentan-2-yl)carbamat

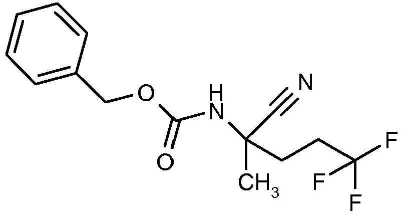

8.7 g (52.36 mmol) *rac*-2-Amino-5,5,5-trifluor-2-methylpentanonitril aus Beispiel 60A wurden in 128 ml Tetrahydrofuran/Wasser = 9/1 vorgelegt und mit 22.43 g (162.3 mmol) Kaliumcarbonat versetzt. Bei 0°C wurden langsam 8.93 g (52.36 mmol) Chlorameisensäurebenzylester zugetropft. Dann ließ man unter Rühren langsam auf Raumtemperatur erwärmen und rührte über Nacht bei Raumtemperatur. Das überstehende Lösungsmittel wurde abdekantiert, der Rückstand zweimal mit je 100 ml Tetrahydrofuran verrührt wonach das überstehende Lösungsmittel jeweils abdekantiert wurde. Die vereinigten organischen Phasen wurde eingeengt und das Rohprodukt wurde mittels Kieselgelchromatographie gereinigt (Laufmittel: Cyclohexan/Essigsäureethylester-Gradient 9/1 bis 4/1). Es wurden 11.14 g der Titelverbindung (68 % d. Th.) erhalten.
LC-MS (Methode 2): Rₜ = 1.01 min
MS (ESpos): m/z = 301 (M+H)⁺
¹H-NMR (400 MHz, DMSO-d₆): δ [ppm] = 1.58 (s, 3H), 2.08 - 2.21 (m, 2H), 2.24 - 2.52 (m, 2H), 5.09 (s, 2H), 7.29 - 7.41 (m, 5H), 8.17 (br. s, 1H).

### Beispiel 62A

### ent-Benzyl-(2-cyan-5,5,5-trifluorpentan-2-yl)carbamat (Enantiomer A)

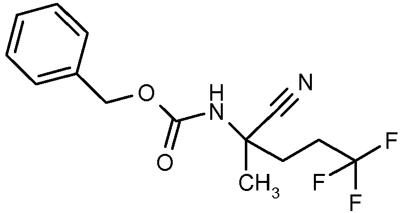

11.14 g *rac*-Benzyl-(2-cyan-5,5,5-trifluorpentan-2-yl)carbamat aus Beispiel 61A wurden durch präparative Trennung an der chiralen Phase in die Enantiomere getrennt [Säule: Daicel Chiralpak AZ-H, 5 µm, SFC, 250 x 50 mm, Eluent: 94% Kohlendioxid, 6% Methanol, Fluss: 200 ml/min, Temperatur: 38°C, Druck: 135 bar; Detektion: 210 nm].
Enantiomer A: 4.12 g (ca. 79% ee)
Rₜ = 1.60 min [SFC, Daicel Chiralpak AZ-H, 250 x 4.6 mm, 5 µm, Eluent: 90% Kohlendioxid, 10% Methanol, Fluss: 3 ml/min, Temperatur: 30°C, Detektion: 220 nm].
LC-MS (Methode 2): Rₜ = 1.01 min
MS (ESpos): m/z = 301 (M+H)⁺

### Beispiel 63A

### ent-Benzyl-(2-cyan-5,5,5-trifluorpentan-2-yl)carbamat (Enantiomer B)

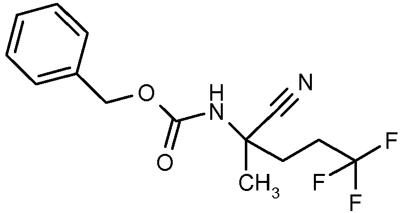

11.14 g *rac*-Benzyl-(2-cyan-5,5,5-trifluorpentan-2-yl)carbamat aus Beispiel 61A wurden durch präparative Trennung an der chiralen Phase in die Enantiomere getrennt [Säule: Daicel Chiralpak AZ-H, 5 µm, SFC, 250 x 50 mm, Eluent: 94% Kohlendioxid, 6% Methanol, Fluss: 200 ml/min, Temperatur: 38°C, Druck: 135 bar; Detektion: 210 nm].
Enantiomer B: 4.54 g (ca. 70% ee; Reinheit ca. 89%)
Rₜ = 1.91 min [SFC, Daicel Chiralpak AZ-H, 250 x 4.6 mm, 5 µm, Eluent: 90% Kohlendioxid, 10% Methanol, Fluss: 3 ml/min, Temperatur: 30°C, Detektion: 220 nm].
LC-MS (Methode 2): Rₜ = 1.01 min
MS (ESpos): m/z = 301 (M+H)⁺

### Beispiel 64A

### ent-Benzyl-(1-amino-5,5,5-trifluor-2-methylpentan-2-yl)carbamat (Enantiomer A)

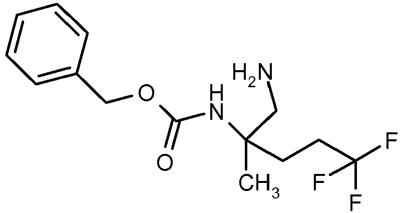

4.12 g (13.17 mmol) *ent*-Benzyl-(2-cyan-5,5,5-trifluorpentan-2-yl)carbamat (Enantiomer A) aus Beispiel 62A wurden in 39 ml 7 N Ammoniak-Lösung in Methanol gelöst und unter Argon mit 4 g Raney-Nickel (50%ige wässrige Aufschlämmung) versetzt. Das Reaktionsgemisch wurde über Nacht im Autoklaven bei 20-30 bar hydriert. Es wurden nochmals 1 g Raney-Nickel (50%ige wässrige Aufschlämmung) hinzugegeben und das Reaktionsgemisch wurde 5 h im Autoklaven bei 20-30 bar hydriert. Die Reaktionsmischung wurde über Kieselgur abfiltriert, mit Methanol gespült und eingeengt. Es wurden 3.35 g (56% d. Th.; Reinheit ca. 67%) der Zielverbindung erhalten, welche ohne weitere Reinigung in die Folgestufe eingesetzt wurde.
LC-MS (Methode 8): Rₜ = 1.68 min
MS (ESpos): m/z = 305 (M+H)⁺
¹H-NMR (400 MHz, DMSO-d₆): δ [ppm] = 1.13 (s, 3H), 1.40 (br. s, 2H), 1.70 - 1.80 (m, 1H), 1.83 - 1.95 (m, 1H), 2.08 - 2.2 (m, 2H), 4.98 (s, 2H), 6.85 (br. s, 1H), 7.28 - 7.41 (m, 5H).

### Beispiel 65A

### ent-Benzyl-(1-amino-5,5,5-trifluor-2-methylpentan-2-yl)carbamat (Enantiomer B)

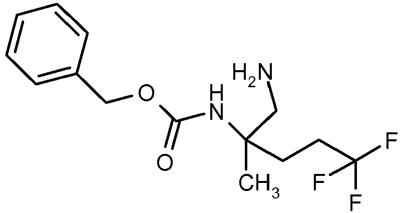

4.54 g (13.45 mmol; Reinheit ca. 89%) *ent*-Benzyl-(2-cyan-5,5,5-trifluorpentan-2-yl)carbamat (Enantiomer B) aus Beispiel 63A wurden in 39 ml 7 N Ammoniak-Lösung in Methanol gelöst und unter Argon mit 5 g Raney-Nickel (50%ige wässrige Aufschlämmung) versetzt. Das Reaktionsgemisch wurde 3 h im Autoklaven bei 20-30 bar hydriert. Die Reaktionsmischung wurde über Kieselgur ab filtriert, mit Methanol gespült und eingeengt. Es wurden 4.20 g (97% d. Th.; Reinheit ca. 95%) der Zielverbindung erhalten, welche ohne weitere Reinigung in die Folgestufe eingesetzt wurde.
LC-MS (Methode 7): Rₜ = 2.19 min
MS (ESpos): m/z = 305 (M+H)⁺
¹H-NMR (400 MHz, DMSO-d₆): δ [ppm] = 1.13 (s, 3H), 1.40 (br. s, 2H), 1.69 - 1.80 (m, 1H), 1.83 - 1.96 (m, 1H), 2.07 - 2.22 (m, 2H), 4.98 (s, 2H), 6.85 (br. s, 1H), 7.27 - 7.40 (m, 5H).

### Beispiel 66A

### rac-2-[(Diphenylmethylen)amino]-4-fluorbutanonitril

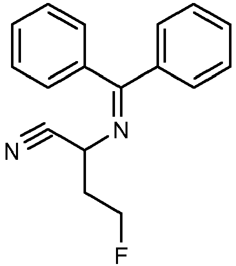

16.5 g (74.91 mmol) [(Diphenylmethylen)amino]acetonitril wurden in 495 ml abs. THF vorgelegt und bei -78°C unter Argon mit 35.96 ml (89.89 mmol) *n*-Butyllithium (2.5 N in Hexan) versetzt und 15 min bei -78°C gerührt. Anschließend wurde die Reaktionslösung auf 0°C erwärmt. Es wurden 13.03 g (74.91 mmol) 1-Iodo-2-fluoroethan zu der Reaktionslösung getropft und 15 min bei 0°C nachgerührt. Die Reaktionslösung wurde bei 0°C mit Wasser gequenscht, mit Ethylacetat versetzt und mit gesättigter, wässriger Natriumchloridlösung gewaschen. Die wässrige Phase wurde zweimal mit Ethylacetat reextrahiert. Die vereinigten organischen Phasen wurden über Natriumsulfat getrocknet, filtriert und einrotiert. Der Rückstand wurde mittels Kieselgel-Chromatographie (Laufmittel: Dichlormethan/Cyclohexan = 1/1 bis 2/1) gereinigt. Es wurden 18.7 g der Zielverbindung (80% d. Th., Reinheit 85%) erhalten.
LC-MS (Methode 3): Rₜ = 2.42 min
MS (ESpos): m/z = 267 (M+H)⁺
¹H-NMR (400 MHz, DMSO-d₆): δ = 2.13 - 2.41 (m, 2H), 4.40 (t, 1H), 4.43 - 4.71 (m, 2H), 7.25 - 7.30 (m, 2H), 7.33 - 7.63 (m, 8H).

### Beispiel 67A

### rac-2-[(Diphenylmethylen)amino]-4,4-difluorbutanonitril

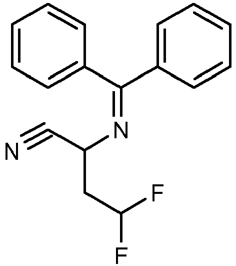

18 g (81.72 mmol) [(Diphenylmethylen)amino]acetonitril wurden in 500 ml abs. THF vorgelegt und bei -78°C unter Argon mit 39.22 ml (98.06 mmol) *n*-Butyllithium (2.5 N in Hexan) versetzt und 15 min bei -78°C nachgerührt. Anschließend wurde die Reaktionslösung auf 0°C erwärmt. Es wurden 17.25 g (89.89 mmol) 1,1-Difluor-2-iodethan zu der Reaktionslösung getropft und 15 min bei 0°C nachgerührt. Die Reaktionslösung wurde bei 0°C mit Wasser gequenscht, mit Ethylacetat versetzt und dreimal mit halbgesättigter, wässriger Natriumchloridlösung gewaschen. Die vereinigten wässrigen Phasen wurden zweimal mit Ethylacetat reextrahiert. Die vereinigten organischen Phasen wurden über Natriumsulfat getrocknet, filtriert und einrotiert. Der Rückstand wurde mittels Kieselgel-Chromatographie (Laufmittel: Dichlormethan/Cyclohexan = 1/1) gereinigt. Es wurden 13.57 g der Zielverbindung (49% d. Th., Reinheit 84%) erhalten.
LC-MS (Methode 3): Rₜ = 2.48 min
MS (ESpos): m/z = 285 (M+H)⁺
¹H-NMR (400 MHz, DMSO-d₆): δ = 2.53 - 2.61 (m, 2H; z.T. überlagert mit Lösungsmittelpeak), 4.50 (t, 1H), 6.08 - 6.41 (m, 1H), 7.23 - 7.33 (m, 2H), 7.38 - 7.47 (m, 2H), 7.49 - 7.67 (m, 6H).

### Beispiel 68A

### rac-2-[(Diphenylmethylen)amino]-5-fluorpentanonitril

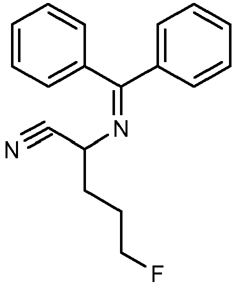

18 g (81.72 mmol) [(Diphenylmethylen)amino]acetonitril wurden in 500 ml abs. THF vorgelegt und bei -78°C unter Argon mit 39.22 ml (98.06 mmol) *n*-Butyllithium (2.5 N in Hexan) versetzt und 15 min bei -78°C nachgerührt. Anschließend wurde die Reaktionslösung auf 0°C erwärmt, 16.9 g (89.89 mmol) 1-Fluor-3-iodpropan zu der Reaktionslösung getropft und 15 min bei 0°C nachgerührt. Die Reaktionslösung wurde bei 0°C mit Wasser gequenscht, mit Ethylacetat versetzt und mit gesättigter, wässriger Natriumchloridlösung gewaschen. Die wässrige Phase wurde zweimal mit Ethylacetat reextrahiert. Die vereinigten organischen Phasen wurden über Natriumsulfat getrocknet, filtriert und einrotiert. Der Rückstand wurde mittels Kieselgel-Chromatographie (Laufmittel: Toluol 100%, Nachreinigung mit Dichlormethan/Cyclohexan = 1/1 bis 2/1) gereinigt. Es wurden in Summe 16.73 g der Zielverbindung (73% d. Th.) erhalten.
LC-MS (Methode 3): Rₜ = 2.50 min
MS (ESpos): m/z = 281 (M+H)⁺
¹H-NMR (400 MHz, DMSO-d₆): δ = 1.66 - 1.85 (m, 2H), 1.87 - 2.00 (m, 2H), 4.26 - 4.41 (m, 2H), 4.43 - 4.55 (m, 1H), 7.20 - 7.33 (m, 2H), 7.38 - 7.48 (m, 2H), 7.48 - 7.63 (m, 6H).

### Beispiel 69A

### rac-2-[(Diphenylmethylen)amino]-4-fluor-2-methylbutanonitril

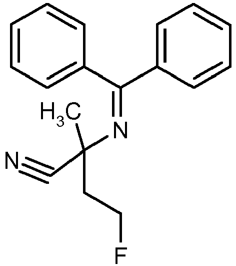

19.94 g (63.64 mmol, Reinheit 85%) *rac*-2-[(Diphenylmethylen)amino]-4-fluorbutanonitril aus Beispiel 66A wurden in 421 ml abs. THF vorgelegt und bei -78°C unter Argon mit 25.71 ml (64.28 mmol) *n*-Butyllithium (2.5 N in Hexan) versetzt und 10 min bei -78°C nachgerührt. Anschließend wurde die Reaktionslösung bei -78°C mit 36.1 g (254.57 mmol) Iodmethan versetzt. Das Reaktionsgemisch wurde langsam über 4.5 h auf 0°C gebracht. Nach vollständiger Abreaktion des Startmaterials wurde die Reaktionslösung bei 0°C mit Wasser gequenscht, mit Ethylacetat versetzt und zweimal mit gesättigter, wässriger Natriumchloridlösung gewaschen. Die organische Phase wurde über Natriumsulfat getrocknet, filtriert und einrotiert. Der Rückstand wurde mittels Kieselgel-Chromatographie (Laufmittel: Cyclohexan/Ethylacetat = 15/1) gereinigt. Es wurden 17.2 g der Zielverbindung (78% d. Th., Reinheit 81%) erhalten.
LC-MS (Methode 3): Rₜ = 2.46 min
MS (ESpos): m/z = 281 (M+H)⁺
¹H-NMR (400 MHz, DMSO-d₆): δ = 1.65 - 1.67 (s, 3H), 2.30 - 2.47 (m, 2H), 4.55 - 4.84 (m, 2H), 7.27 - 7.32 (m, 2H), 7.37 - 7.42 (m, 2H), 7.43 - 7.52 (m, 6H).

### Beispiel 70A

### rac-2-[(Diphenylmethylen)amino]-4,4-difluor-2-methylbutanonitril

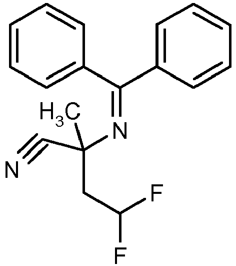

13.07 g (38.62 mmol) *rac*-2-[(Diphenylmethylen)amino]-4,4-difluorbutanonitril aus Beispiel 67A wurden in 255 ml abs. THF vorgelegt und bei -78°C unter Argon mit 15.6 ml (39.0 mmol) *n-*Butyllithium (2.5 N in Hexan) versetzt und 10 min bei -78°C nachgerührt. Anschließend wurde die Reaktionslösung bei -78°C mit 22.6 g (154.46 mmol) Iodmethan versetzt. Das Reaktionsgemisch wurde langsam über 3.5 h auf 0°C gebracht. Nach vollständiger Abreaktion des Startmaterials wurde die Reaktionslösung bei 0°C mit Wasser gequenscht, mit Ethylacetat versetzt und zweimal mit gesättigter, wässriger Natriumchloridlösung gewaschen. Die organische Phase wurde über Natriumsulfat getrocknet, filtriert und einrotiert. Der Rückstand wurde mittels Kieselgel-Chromatographie (Laufmittel: Cyclohexan/Ethylacetat = 15/1) gereinigt. Es wurden 11.4 g der Zielverbindung (91% d. Th., Reinheit 92%) erhalten.
LC-MS (Methode 3): Rₜ = 2.52 min
MS (ESpos): m/z = 299 (M+H)⁺
¹H-NMR (400 MHz, DMSO-d₆): δ = 1.67 (s, 3H), 2.55 - 2.77 (m, 2H), 6.14 - 6.48 (m, 1H), 7.28 - 7.34 (m, 2H), 7.36 - 7.44 (m, 2H), 7.44 - 7.54 (m, 6H).

### Beispiel 71A

### rac-2-[(Diphenylmethylen)amino]-5-fluor-2-methylpentanonitril

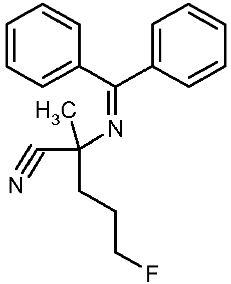

16.73 g (59.68 mmol) *rac*-2-[(Diphenylmethylen)amino]-5-fluorpentanonitril aus Beispiel 68A wurden in 394 ml abs. THF vorgelegt und bei -78°C unter Argon mit 24.11 ml (60.27 mmol) *n-*Butyllithium (2.5 N in Hexan) versetzt und 10 min bei -78°C nachgerührt. Anschließend wurde die Reaktionslösung bei -78°C mit 34.93 g (238.70 mmol) Iodmethan versetzt. Das Reaktionsgemisch wurde langsam über 4.5 h auf 0°C gebracht. Die Reaktionslösung wurde bei 0°C mit Wasser gequenscht, mit Ethylacetat versetzt und zweimal mit gesättigter, wässriger Natriumchloridlösung gewaschen. Die organische Phase wurde über Natriumsulfat getrocknet, filtriert und einrotiert. Der Rückstand wurde mittels Kieselgel-Chromatographie (Laufmittel: Cyclohexan/Ethylacetat = 15/1) gereinigt. Es wurden 18.94 g der Zielverbindung (95% d. Th., Reinheit 88%) erhalten.
LC-MS (Methode 3): Rₜ = 2.55 min
MS (ESpos): m/z = 295 (M+H)⁺
¹H-NMR (400 MHz, DMSO-d₆): δ = 1.62 (s, 3H), 1.73 - 1.90 (m, 2H), 1.94 - 2.03 (m, 1H), 2.04 - 2.18 (m, 1H), 4.47 (t, 1H), 4.58 (t, 1H), 7.23 - 7.33 (m, 2H), 7.35 - 7.43 (m, 2H), 7.44 - 7.56 (m, 6H).

### Beispiel 72A

### rac-2-Amino-4-fluor-2-methylbutanonitrilhydrochlorid

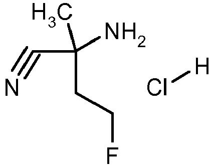

17.45 g (50.45 mmol; Reinheit 81%) *rac*-2-[(Diphenylmethylen)amino]-4-fluor-2-methylbutanonitril aus Beispiel 69A wurden in 235.6 ml Tetrahydrofuran und 9.1 ml Wasser gelöst, mit 111 ml (55.46 mmol) Chlorwasserstoff-Lösung (0.5 N in Diethylether) versetzt und über Nacht bei Raumtemperatur gerührt. Die leicht trübe Reaktionslösung wurde mit 25.21 ml (50.42 mmol) Chlorwasserstoff-Lösung (2 N in Diethylether) versetzt und einrotiert. Das isolierte Rohprodukt wurde direkt ohne weitere Reinigung weiter umgesetzt.
LC-MS (Methode 3): Rₜ = 0.22 min
MS (ESpos): m/z = 117 (M-HCl+H)⁺

### Beispiel 73A

### rac-Benzyl-(2-cyan-4-fluorbutan-2-yl)carbamat

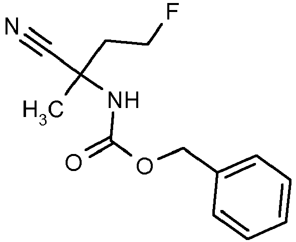

Das Rohprodukt *rac*-2-Amino-4-fluor-2-methylbutanonitrilhydrochlorid aus Beispiel 72A wurde in 165 ml Tetrahydrofuran/Wasser (1:1) vorgelegt, mit 28.57 g (206.71 mmol) Kaliumcarbonat und 9.46 g (55.46 mmol) Chlorameisensäurebenzylester versetzt. Das Reaktionsgemisch (Zwei-Phasen-Gemisch) wurde über Nacht bei Raumtemperatur gerührt. Es wurden erneut 1.72 g (10.1 mmol) Chlorameisensäurebenzylester zu der Reaktion gegeben und weitere 2 h bei Raumtemperatur gerührt. Anschließend wurde das Zweiphasensystem voneinander getrennt und die wässrige Phase zweimal mit Ethylacetat extrahiert. Die vereinigten organischen Phasen wurden einmal mit gesättigter, wässriger Natriumchloridlösung gewaschen und anschließend über Natriumsulfat getrocknet, filtriert und einrotiert. Der Rückstand wurde mittels Kieselgel-Chromatographie gereinigt (Laufmittel: Cyclohexan/Ethylacetat-Gradient 20/1 bis 5/1). Es wurden 5.04 g der Zielverbindung (38 % d. Th. über zwei Stufen; Reinheit 96%) erhalten.
LC-MS (Methode 3): Rₜ = 1.95 min
MS (ESpos): m/z = 251 (M+H)⁺
¹H-NMR (400 MHz, DMSO-d₆): δ [ppm] = 1.59 (s, 3H), 2.20 - 2.43 (m, 2H), 4.55 (t, 1H), 4.67 (t, 1H), 5.08 (s, 2H), 7.28 - 7.45 (m, 5H), 8.12 (br. s, 1H).

### Beispiel 74A

### rac-2-Amino-4,4-difluor-2-methylbutanonitrilhydrochlorid

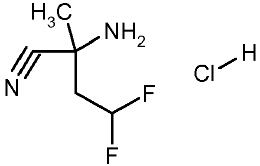

10.84 g (33.43 mmol; Reinheit 92%) *rac*-2-[(Diphenylmethylen)amino]-4,4-difluor-2-methylbutanonitril aus Beispiel 70A wurden in 156 ml Tetrahydrofuran und 6 ml Wasser gelöst, mit 73.5 ml (36.77 mmol) Chlorwasserstoff-Lösung (0.5 N in Diethylether) versetzt und über Nacht bei Raumtemperatur gerührt. Die Reaktionslösung wurde mit 16.71 ml (33.43 mmol) Chlorwasserstoff-Lösung (2 N in Diethylether) versetzt und einrotiert. Das isolierte Rohprodukt wurde direkt ohne weitere Reinigung weiter umgesetzt.
LC-MS (Methode 3): Rₜ = 0.32 min
MS (ESpos): m/z = 135 (M-HCl+H)⁺

### Beispiel 75A

### rac-Benzyl-(2-cyan-4,4-difluorbutan-2-yl)carbamat

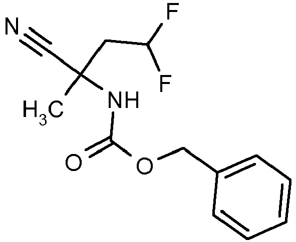

Das Rohprodukt *rac*-2-Amino-4,4-difluor-2-methylbutanonitrilhydrochlorid aus Beispiel 74A wurde in 109 ml Tetrahydrofuran/Wasser (1:1) vorgelegt, mit 18.94 g (137.06 mmol) Kaliumcarbonat und 6.27 g (36.77 mmol) Chlorameisensäurebenzylester versetzt. Das Reaktionsgemisch (Zwei-Phasen-Gemisch) wurde über Nacht bei Raumtemperatur gerührt. Es wurden erneut 1.14 g (6.69 mmol) Chlorameisensäurebenzylester zu der Reaktion gegeben und weitere 2 h bei Raumtemperatur gerührt. Anschließend wurde das Zweiphasensystem voneinander getrennt und die wässrige Phase zweimal mit Ethylacetat extrahiert. Die vereinigten organischen Phasen wurden einmal mit gesättigter, wässriger Natriumchloridlösung gewaschen und anschließend über Natriumsulfat getrocknet, filtriert und einrotiert. Der Rückstand wurde mittels Kieselgel-Chromatographie gereinigt (Laufmittel: Cyclohexan/Ethylacetat-Gradient 20/1 bis 5/1). Es wurden 7.68 g der Zielverbindung (61 % d. Th. über zwei Stufen; Reinheit 71%) erhalten.
LC-MS (Methode 3): Rₜ = 2.04 min
MS (ESpos): m/z = 269 (M+H)⁺
¹H-NMR (400 MHz, DMSO-d₆): δ [ppm] = 1.65 (s, 3H), 2.51 - 2.65 (m, 2H), 5.10 (s, 2H), 6.08 - 6.41 (m, 1H), 7.27 - 7.44 (m, 5H), 8.24 (br. s, 1H).

### Beispiel 76A

### rac-2-Amino-5-fluor-2-methylpentanonitrilhydrochlorid

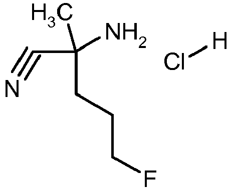

18.94 g (56.62 mmol; Reinheit 88%) rac-2-[(Diphenylmethylen)amino]-5-fluor-2-methylpentanonitril aus Beispiel 71A wurden in 264.6 ml Tetrahydrofuran und 10.2 ml Wasser gelöst, mit 124.6 ml (62.28 mmol) Chlorwasserstoff-Lösung (0.5 N in Diethylether) versetzt und über Nacht bei Raumtemperatur gerührt. Die Reaktionslösung wurde mit 28.3 ml (56.62 mmol) Chlorwasserstoff-Lösung (2 N in Diethylether) versetzt und einrotiert. Das isolierte Rohprodukt wurde direkt ohne weitere Reinigung weiter umgesetzt.
LC-MS (Methode 3): Rₜ = 0.25 min
MS (ESpos): m/z = 131 (M-HCl+H)⁺

### Beispiel 77A

### rac-Benzyl-(2-cyan-5-fluorpentan-2-yl)carbamat

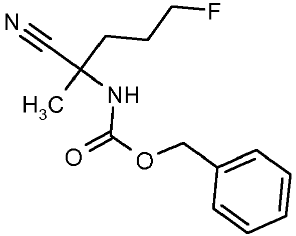

Das Rohprodukt *rac*-2-Amino-5-fluor-2-methylpentanonitrilhydrochlorid aus Beispiel 76A wurde in 185 ml Tetrahydrofuran/Wasser (1/1) vorgelegt, mit 32.09 g (232.18 mmol) Kaliumcarbonat und 10.63 g (62.29 mmol) Chlorameisensäurebenzylester versetzt. Das Reaktionsgemisch (Zwei-Phasen-Gemisch) wurde über Nacht bei Raumtemperatur gerührt. Es wurden erneut 1.93 g (11.33 mmol) Chlorameisensäurebenzylester zu der Reaktion gegeben und weitere 2 h bei Raumtemperatur gerührt. Anschließend wurde das Zweiphasensystem voneinander getrennt und die wässrige Phase zweimal mit Ethylacetat extrahiert. Die vereinigten organischen Phasen wurden einmal mit gesättigter, wässriger Natriumchloridlösung gewaschen und anschließend über Natriumsulfat getrocknet, filtriert und einrotiert. Der Rückstand wurde mittels Kieselgel-Chromatographie gereinigt (Laufmittel: Cyclohexan/Ethylacetat-Gradient 20/1 bis 5/1). Es wurden 11.77 g der Zielverbindung (72 % d. Th. über zwei Stufen, Reinheit 92%) erhalten.
LC-MS (Methode 3): Rₜ = 2.03 min
MS (ESpos): m/z = 265 (M+H)⁺
¹H-NMR (400 MHz, DMSO-d₆): δ [ppm] = 1.55 (s, 3H), 1.66 - 1.85 (m, 2H), 1.86 - 2.04 (m, 2H), 4.40 (t, 1H), 4.52 (t, 1H), 5.08 (s, 2H), 7.28 - 7.44 (m, 5H), 8.05 (br. s, 1H).

### Beispiel 78A

### ent-Benzyl-(2-cyan-4,4-difluorbutan-2-yl)carbamat (Enantiomer A)

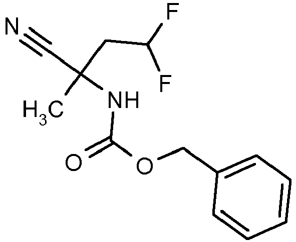

7.68 g (20.33 mmol, Reinheit 71%) *rac*-Benzyl-(2-cyan-4,4-difluorbutan-2-yl)carbamat aus Beispiel 75A wurden durch präparative Trennung an chiraler Phase in die Enantiomere getrennt [Säule: Daicel Chiralpak AY-H, 5 µm, 250 x 20 mm, Eluent: 80% iso-Hexan, 20% Isopropanol, Fluss: 25 ml/min; Temperatur: 22°C, Detektion: 210 nm].
Enantiomer A: Ausbeute: 2.64 g (>99% ee)
Rₜ = 6.67 min [Chiralpak AY-H, 5 µm, 250 x 4.6 mm; Eluent: 80% iso-Hexan, 20% Isopropanol; Fluss: 3 ml/min; Detektion: 220 nm].

### Beispiel 79A

### ent-Benzyl-(2-cyan-4,4-difluorbutan-2-yl)carbamat (Enantiomer B)

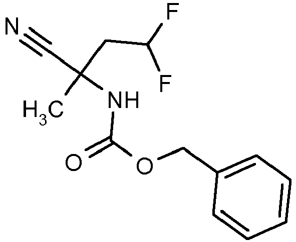

7.68 g (20.33 mmol, Reinheit 71%) *rac*-Benzyl-(2-cyan-4,4-difluorbutan-2-yl)carbamat aus Beispiel 75A wurden durch präparative Trennung an chiraler Phase in die Enantiomere getrennt [Säule: Daicel Chiralpak AY-H, 5 µm, 250 x 20 mm, Eluent: 80% iso-Hexan, 20% Isopropanol, Fluss: 25 ml/min; Temperatur: 22°C, Detektion: 210 nm].
Enantiomer B: Ausbeute: 2.76 g (93% ee)
Rₜ = 7.66 min [Chiralpak AY-H, 5 µm, 250 x 4.6 mm; Eluent: 80% iso-Hexan, 20% Isopropanol; Fluss: 3 ml/min; Detektion: 220 nm].

### Beispiel 80A

### ent-Benzyl-(2-cyan-5-fluorpentan-2-yl)carbamat (Enantiomer A)

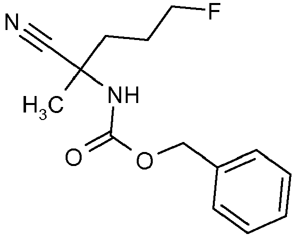

11.77 g (40.97 mmol, Reinheit 92%) *rac*-Benzyl-(2-cyan-5-fluorpentan-2-yl)carbamat aus Beispiel 77A wurden durch präparative Trennung an chiraler Phase in die Enantiomere getrennt [Säule: SFC Daicel Chiralpak AZ-H, 5 µm, 250 x 30 mm, Eluent: 90% CO₂ 10% Methanol, Fluss: 100 ml/min; Temperatur: 40°C, Detektion: 210 nm].
Enantiomer A: Ausbeute: 5.7 g (>99% ee)
Rt= 1.76 min [SFC Chiralpak AZ-3, 3 µm, 50 x 4.6 mm; Eluent: CO₂/Methanol-Gradient (5% bis 60% Methanol); Fluss: 3 ml/min; Detektion: 220 nm].

### Beispiel 81A

### ent-Benzyl-(2-cyan-5-fluorpentan-2-yl)carbamat (Enantiomer B)

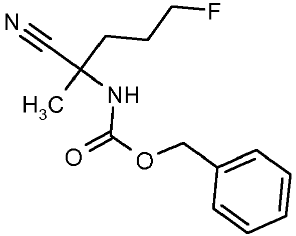

11.77 g (40.97 mmol, Reinheit 92%) *rac*-Benzyl-(2-cyan-5-fluorpentan-2-yl)carbamat aus Beispiel 77A wurden durch präparative Trennung an chiraler Phase in die Enantiomere getrennt [Säule: SFC Daicel Chiralpak AZ-H, 5 µm, 250 x 30 mm, Eluent: 90% CO₂ 10% Methanol, Fluss: 100 ml/min; Temperatur: 40°C, Detektion: 210 nm].
Enantiomer B: Ausbeute: 5.0 g (>99% ee)
Rt= 1.97 min [SFC Chiralpak AZ-3, 3 µm, 50 x 4.6 mm; Eluent: CO₂/Methanol-Gradient (5% bis 60% Methanol); Fluss: 3 ml/min; Detektion: 220 nm].

### Beispiel 82A

### ent-Benzyl-(1-amino-4,4-difluor-2-methylbutan-2-yl)carbamat (Enantiomer A)

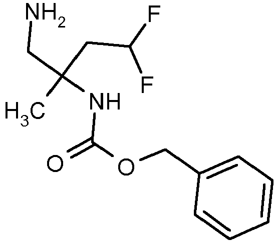

2.3 g (8.57 mmol) *ent*-Benzyl-(2-cyan-4,4-difluorbutan-2-yl)carbamat (Enantiomer A) aus Beispiel 78A wurden in 75 ml 7 N Ammoniak-Lösung in Methanol gelöst und unter Argon mit 2.66 g Raney-Nickel (50%ige wässrige Aufschlämmung) versetzt. Das Reaktionsgemisch wurde für 1.5 h im Autoklaven bei 20-30 bar hydriert. Die Reaktionsmischung wurde über Celite abfiltriert, mit Methanol und 2 N Ammoniak in Methanol gespült und eingeengt. Es wurden 2.23 g der Zielverbindung (94% d. Th., Reinheit 98%) erhalten.
LC-MS (Methode 3): Rₜ = 1.48 min
MS (ESpos): m/z = 273 (M+H)⁺
¹H-NMR (400 MHz, DMSO-d₆): δ [ppm] = 1.19 (s, 3H), 1.48 (br. s, 2H), 2.08 - 2.40 (m, 2H), 2.53 - 2.72 (m, 2H; z.T. überlagert mit Lösungsmittelpeak), 5.00 (s, 2H), 5.90 - 6.23 (m, 1H), 6.95 (br. s, 1H), 7.25 - 7.41 (m, 5H).

### Beispiel 83A

### ent-Benzyl-(1-amino-4,4-difluor-2-methylbutan-2-yl)carbamat (Enantiomer B)

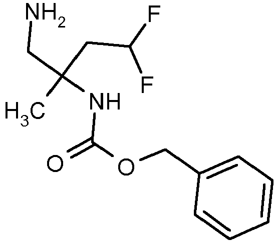

2.76 g (10.29 mmol) *ent*-Benzyl-(2-cyan-4,4-difluorbutan-2-yl)carbamat (Enantiomer B) aus Beispiel 79A wurden in 90 ml 7 N Ammoniak-Lösung in Methanol gelöst und unter Argon mit 3.19 g Raney-Nickel (50%ige wässrige Aufschlämmung) versetzt. Das Reaktionsgemisch wurde für 1.5 h im Autoklaven bei 20-30 bar hydriert. Die Reaktionsmischung wurde über Celite ab filtriert, mit Methanol und 2 N Ammoniak in Methanol gespült und eingeengt. Es wurden 2.64 g der Zielverbindung (88% d. Th., Reinheit 93%) erhalten.
LC-MS (Methode 3): Rₜ = 1.49 min
MS (ESpos): m/z = 273 (M+H)⁺
¹H-NMR (400 MHz, DMSO-d₆): δ [ppm] = 1.19 (s, 3H), 1.48 (br. s, 2H), 2.08 - 2.40 (m, 2H), 2.53 - 2.73 (m, 2H; z.T. überlagert mit Lösungsmittelpeak), 5.00 (s, 2H), 5.90 - 6.24 (m, 1H), 6.95 (br. s, 1H), 7.25 - 7.41 (m, 5H).

### Beispiel 84A

### ent-Benzyl-(1-amino-5-fluor-2-methylpentan-2-yl)carbamat (Enantiomer A)

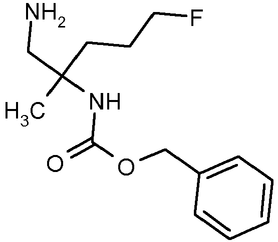

5.7 g (21.57 mmol) *ent*-Benzyl-(2-cyan-5-fluorpentan-2-yl)carbamat (Enantiomer A) aus Beispiel 80A wurden in 125 ml 7 N Ammoniak-Lösung in Methanol gelöst und unter Argon mit 6.68 g Raney-Nickel (50%ige wässrige Aufschlämmung) versetzt. Das Reaktionsgemisch wurde für 4.5 h im Autoklaven bei 20-30 bar hydriert. Die Reaktionsmischung wurde über Celite abfiltriert, mit Methanol und 2 N Ammoniak in Methanol gespült und eingeengt. Es wurden 5.22 g der Zielverbindung (77% d. Th., Reinheit 85%) erhalten.
LC-MS (Methode 3): Rₜ = 1.51 min
MS (ESpos): m/z = 269 (M+H)⁺

### Beispiel 85A

### ent-Benzyl-(1-amino-5-fluor-2-methylpentan-2-yl)carbamat (Enantiomer B)

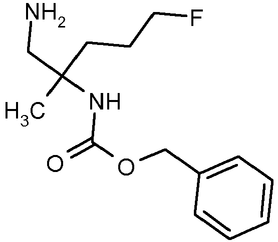

5.0 g (18.92 mmol) *ent*-Benzyl-(2-cyan-5-fluorpentan-2-yl)carbamat (Enantiomer B) aus Beispiel 81A wurden in 110 ml 7 N Ammoniak-Lösung in Methanol gelöst und unter Argon mit 5.86 g Raney-Nickel (50%ige wässrige Aufschlämmung) versetzt. Das Reaktionsgemisch wurde für 4.5 h im Autoklaven bei 20-30 bar hydriert. Die Reaktionsmischung wurde über Celite abfiltriert, mit Methanol und 2 N Ammoniak in Methanol gespült und eingeengt. Es wurden 4.6 g der Zielverbindung (84% d. Th., Reinheit 93%) erhalten.
LC-MS (Methode 3): Rₜ = 1.47 min
MS (ESpos): m/z = 269 (M+H)⁺

### Beispiel 86A

### rac-4-Fluor-2-methylbutan-1,2-diamin Dihydrochlorid

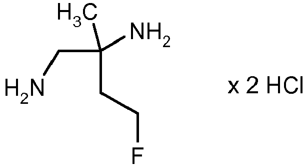

1.00 g (4.00 mmol) *rac*-Benzyl-(2-cyan-4-fluorbutan-2-yl)carbamat aus Beispiel 73A wurden in 114 ml Ethanol/Eisessig (1/1) gelöst und mit 0.85 g Palladium auf A-Kohle (10%ig) versetzt. Das Reaktionsgemisch wurde für 3 h im Autoklaven bei 30-50 bar hydriert. Die Reaktionsmischung wurde einen Faltenfilter abfiltriert, mit Ethanol gespült und anschließend nochmal über einen Millipore-Filter filtriert. Das Filtrat wurde mit 10 ml Chlorwasserstoff-Lösung (2 N in Diethylether) versetzt und anschließend eingedampft. Es wurden 1.04 g der Zielverbindung erhalten, welche ohne weitere Reinigung in die Folgestufe eingesetzt wurde.
LC-MS (Methode 3): Rₜ = 0.19 min
MS (ESpos): m/z = 121 (M-2HCl+H)⁺

### Beispiel 87A

### ent-Benzyl-{1-[({7-[(2,6-difluorbenzyl)oxy]-2,5-dimethylpyrazolo[1,5-a]pyridin-3-yl}carbonyl)amino]-2-methylpentan-2-yl}carbamat Trifluoracetat (Enantiomer B)

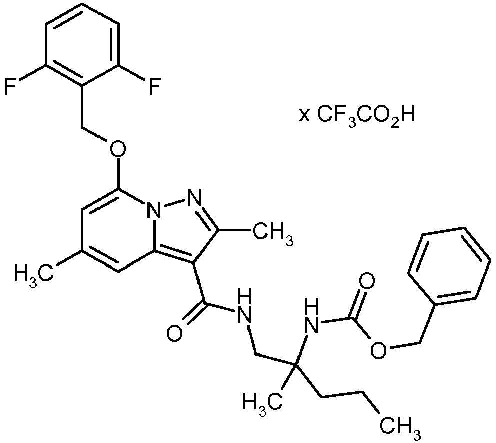

210 mg (0.62 mmol) 7-[(2,6-Difluorbenzyl)oxy]-2,5-dimethylpyrazolo[1,5-a]pyridin-3-carbonsäure aus Beispiel 4A wurden mit 259 mg (0.68 mmol) HATU und 0.324 ml (1.86 mmol) N,N-Diisopropylethylamin in 1.8 ml DMF vorgelegt und 10 min bei Raumtemperatur gerührt. Anschließend wurden 211 mg (0.74 mmol) *ent*-Benzyl-(1-amino-2-methylpentan-2-yl)carbamat (Enantiomer B) aus Beispiel 20A zur Reaktionslösung gegeben und über Nacht bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, und eingeengt. Es wurden 190 mg der Zielverbindung (42% d. Th.; Reinheit 92%) erhalten.
LC-MS (Methode 2): Rₜ = 1.24 min
MS (ESpos): m/z = 565 (M-TFA+H)⁺

### Beispiel 88A

### ent-Benzyl-{1-[({7-[(2,6-difluorbenzyl)oxy]-2,5-dimethylpyrazolo[1,5-a]pyridin-3-yl}carbonyl)amino]-3-fluor-2-methylpropan-2-yl}carbamat Trifluoroacetat (Enantiomer A)

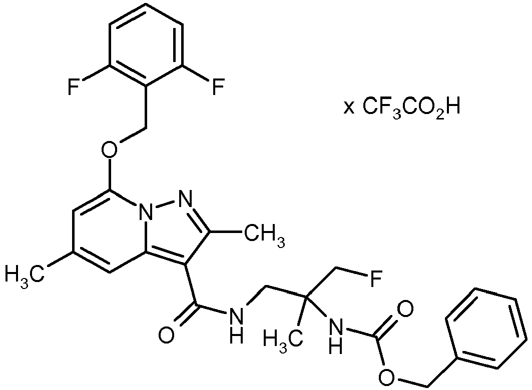

250 mg (0.56 mmol; ca. 75%ige Reinheit) 7-[(2,6-Difluorbenzyl)oxy]-2,5-dimethylpyrazolo[1,5-a]pyridin-3-carbonsäure aus Beispiel 4A wurden mit 236 mg (0.62 mmol) HATU und 0.30 ml (1.69 mmol) N,N-Diisopropylethylamin in 1.68 ml DMF vorgelegt, 20 min vorgerührt, dann mit 192 mg (0.68 mmol, 85 %ig) *ent*-Benzyl-(1-amino-3-fluor-2-methylpropan-2-yl)carbamat (Enantiomer A) aus Beispiel 58A versetzt und 2 h bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 231 mg der Zielverbindung (59% d. Th.; Reinheit 95%) erhalten.
LC-MS (Methode 2): Rₜ = 1.20 min
MS (ESpos): m/z = 555 (M-TFA+H)⁺

### Beispiel 89A

### ent-Benzyl-{1-[({7-[(2,6-difluorbenzyl)oxy]-2,5-dimethylpyrazolo[1,5-a]pyridin-3-yl}carbonyl)amino]-3-fluor-2-methylpropan-2-yl}carbamat Trifluoroacetat (Enantiomer B)

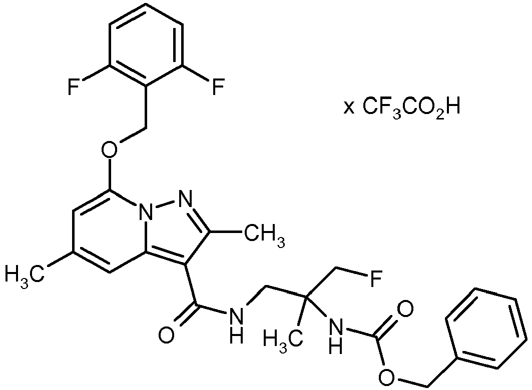

250 mg (0.56 mmol; ca. 75%ige Reinheit) 7-[(2,6-Difluorbenzyl)oxy]-2,5-dimethylpyrazolo[1,5-a]pyridin-3-carbonsäure aus Beispiel 4A wurden mit 236 mg (0.62 mmol) HATU und 0.30 ml (1.69 mmol) N,N-Diisopropylethylamin in 1.68 ml DMF vorgelegt, 20 min vorgerührt, dann mit 192 mg (0.68 mmol, 85 %ig) *ent*-Benzyl-(1-amino-3-fluor-2-methylpropan-2-yl)carbamat (Enantiomer B) aus Beispiel 59A versetzt und 2 h bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 240 mg der Zielverbindung (47% d. Th.; Reinheit 74%) erhalten.
LC-MS (Methode 2): Rₜ = 1.20 min
MS (ESpos): m/z = 555 (M-TFA+H)⁺

### Beispiel 90A

### ent-Benzyl-{1-[({7-[(2,6-difluorbenzyl)oxy]-2,5-dimethylpyrazolo[1,5-a]pyridin-3-yl}carbonyl)amino]-5,5,5-trifluor-2-methylpentan-2-yl}carbamat Trifluoroacetat (Enantiomer A)

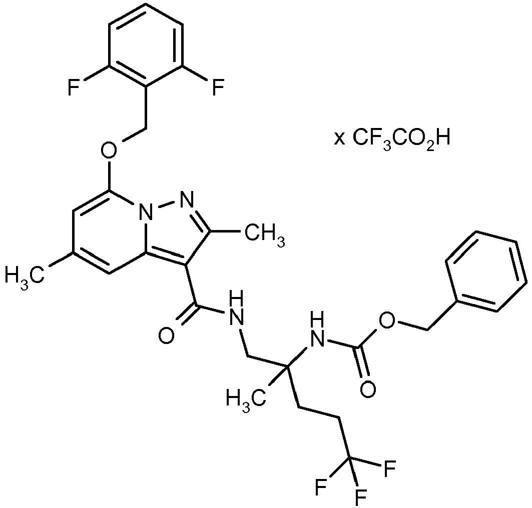

50 mg (0.11 mmol; ca. 75%ige Reinheit) 7-[(2,6-Difluorbenzyl)oxy]-2,5-dimethylpyrazolo[1,5-a]pyridin-3-carbonsäure aus Beispiel 4A, 47 mg (0.12 mmol) HATU und 44 mg (0.34 mmol) N,N-Diisopropylethylamin wurden in 0.33 ml DMF vorgelegt und 10 min bei RT gerührt. Anschließend wurden 62 mg (0.14 mmol; Reinheit ca. 67%) *ent*-Benzyl-(1-amino-5,5,5-trifluor-2-methylpentan-2-yl)carbamat (Enantiomer A) aus Beispiel 64A hinzugegeben und das Gemisch wurde 2 h bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 52 mg (62% d. Th.) der Titelverbindung erhalten.
LC-MS (Methode 2): Rₜ = 1.25 min
MS (ESpos): m/z = 619 (M-TFA+H)⁺

### Beispiel 91A

### ent-Benzyl-{1-[({7-[(2,6-difluorbenzyl)oxy]-2,5-dimethylpyrazolo[1,5-a]pyridin-3-yl}carbonyl)amino]-5,5,5-trifluor-2-methylpentan-2-yl}carbamat Trifluoroacetat (Enantiomer B)

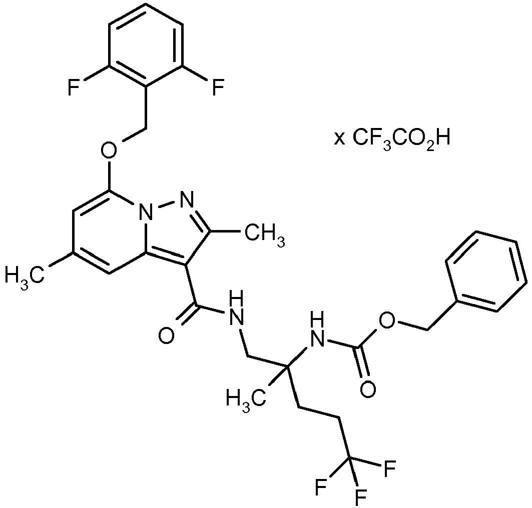

325 mg (0.73 mmol; ca. 75%ige Reinheit) 7-[(2,6-Difluorbenzyl)oxy]-2,5-dimethylpyrazolo[1,5-a]pyridin-3-carbonsäure aus Beispiel 4A, 307 mg (0.81 mmol) HATU und 284 mg (2.20 mmol) N,N-Diisopropylethylamin wurden in 2.2 ml DMF vorgelegt und 10 min bei RT gerührt. Anschließend wurden 282 mg (0.88 mmol; Reinheit ca. 95%) *ent*-Benzyl-(1-amino-5,5,5-trifluor-2-methylpentan-2-yl)carbamat (Enantiomer B) aus Beispiel 65A hinzugegeben und das Gemisch wurde 2 h bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 311 mg (57% d. Th.) der Titelverbindung erhalten.
LC-MS (Methode 2): Rₜ = 1.29 min
MS (ESpos): m/z = 619 (M-TFA+H)⁺

### Beispiel 92A

### ent-Benzyl-{1-[({7-[(2,6-difluorbenzyl)oxy]-2,5-dimethylpyrazolo[1,5-a]pyridin-3-yl}carbonyl)amino]-5-fluor-2-methylpentan-2-yl}carbamat Trifluoracetat (Enantiomer A)

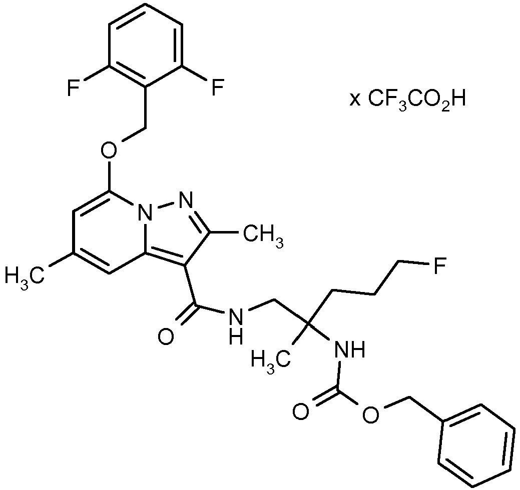

250 mg (0.75 mmol) 7-[(2,6-Difluorbenzyl)oxy]-2,5-dimethylpyrazolo[1,5-a]pyridin-3-carbonsäure aus Beispiel 4A wurden in 2.5 ml DMF gelöst, mit 372 mg (0.98 mmol) HATU und 0.66 ml (3.76 mmol) N,N-Diisopropylethylamin versetzt und 20 min bei Raumtemperatur gerührt. Anschließend wurden 309 mg (0.98 mmol, Reinheit 85%) *ent*-Benzyl-(1-amino-5-fluor-2-methylpentan-2-yl)carbamat (Enantiomer A) aus Beispiel 84A versetzt. Nach vollständiger Abreaktion des Startmaterials (ca. 30 min) wurde mit Wasser versetzt, der entstandene Feststoff ab filtriert und mit Wasser gewaschen. Der Rückstand wurde mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Es wurden 168 mg der Titelverbindung (32% d. Th.) erhalten.
LC-MS (Methode 2): Rₜ = 1.17 min
MS (ESpos): m/z = 583 (M-TFA+H)⁺

### Beispiel 93A

### ent-Benzyl-{1-[({7-[(2,6-difluorbenzyl)oxy]-2,5-dimethylpyrazolo[1,5-a]pyridin-3-yl}carbonyl)amino]-5-fluor-2-methylpentan-2-yl}carbamat (Enantiomer B)

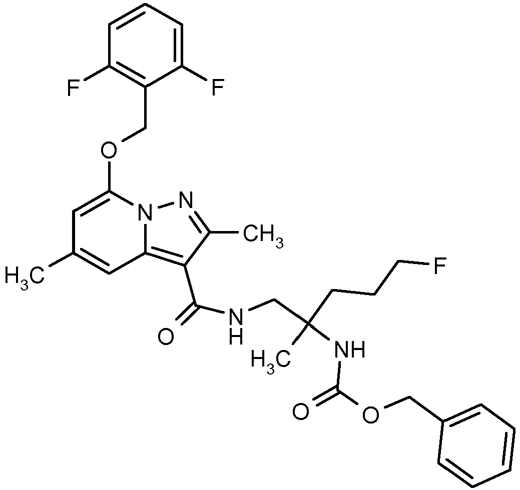

250 mg (0.75 mmol) 7-[(2,6-Difluorbenzyl)oxy]-2,5-dimethylpyrazolo[1,5-a]pyridin-3-carbonsäure aus Beispiel 4A wurden in 2.5 ml DMF gelöst, mit 372 mg (0.98 mmol) HATU und 0.66 ml (3.76 mmol) N,N-Diisopropylethylamin versetzt und 20 min bei Raumtemperatur gerührt. Anschließend wurden 283 mg (0.98 mmol, Reinheit 93%) *ent*-Benzyl-(1-amino-5-fluor-2-methylpentan-2-yl)carbamat (Enantiomer B) aus Beispiel 85A versetzt. Nach vollständiger Abreaktion des Startmaterials (ca. 30 min) wurde mit Wasser versetzt, der entstandene Feststoff ab filtriert und mit Wasser gewaschen. Der Rückstand wurde mittels präparativer HPLC gereinigt (RP18 Säule, Laufmittel: Acetonitril/Wasser-Gradient unter Zusatz von 0.1% TFA). Die Produktfraktionen wurden eingedampft und der Rückstand wurde mit 8 ml 2 M Ammoniak-Lösung versetzt, 10 Min verrührt und anschließend einrotiert. Der daraus entstandene Rückstand wurde in Dichlormethan und wenig Methanol aufgenommen und mittels Kieselgelchromatographie gereinigt (Laufmittel: Dichlormethan/Ethylacetat: 50/1 nach Dichlormethan/Methanol/Ethylacetat: 50/1/1). Die Produktfraktionen wurden vereinigt und eingedampft. Es wurden 96 mg der Titelverbindung (21% d. Th., Reinheit 96%) erhalten.
LC-MS (Methode 7): Rₜ = 2.79 min
MS (ESpos): m/z = 583 (M+H)⁺

### Beispiel 94A

### ent-Benzyl-{1-[({7-[(2,6-difluorbenzyl)oxy]-2,5-dimethylpyrazolo[1,5-a]pyridin-3-yl}carbonyl)amino]-4,4-difluor-2-methylbutan-2-yl}carbamat (Enantiomer A)

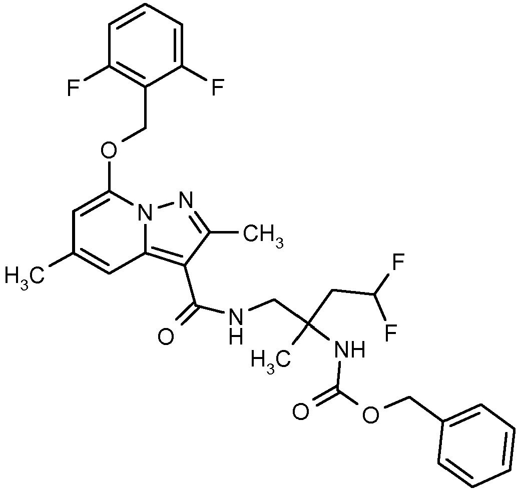

350 mg (1.05 mmol) 7-[(2,6-Difluorbenzyl)oxy]-2,5-dimethylpyrazolo[1,5-a]pyridin-3-carbonsäure aus Beispiel 4A wurden in 3.5 ml DMF gelöst, mit 521 mg (1.37 mmol) HATU und 0.92 ml (5.27 mmol) N,N-Diisopropylethylamin versetzt und 20 min bei Raumtemperatur gerührt. Anschließend wurden 380 mg (1.37 mmol, Reinheit 98%) *ent*-Benzyl-(1-amino-4,4-difluor-2-methylbutan-2-yl)carbamat (Enantiomer A) aus Beispiel 82A versetzt. Nach vollständiger Abreaktion des Startmaterials (ca. 60 min) wurde mit Wasser versetzt, der entstandene Feststoff abfiltriert und mit Wasser gewaschen. Der Rückstand wurde mittels präparativer HPLC gereinigt (RP18 Säule, Laufmittel: Acetonitril/Wasser-Gradient unter Zusatz von 0.1% TFA). Die Produktfraktionen wurden vereinigt und eingedampft und nochmals mittels Kieselgelchromatographie nachgereinigt (Laufmittel: Cyclohexan/Ethylacetat-Gradient 2/1 bis 1/1). Es wurden 157 mg der Titelverbindung (18% d. Th., Reinheit 72%) erhalten.
LC-MS (Methode 2): Rₜ = 1.18 min
MS (ESpos): m/z = 587 (M+H)⁺

### Beispiel 95A

### ent-Benzyl-{1-[({7-[(2,6-difluorbenzyl)oxy]-2,5-dimethylpyrazolo[1,5-a]pyridin-3-yl}carbonyl)amino]-4,4-difluor-2-methylbutan-2-yl}carbamat Trifluoracetat (Enantiomer B)

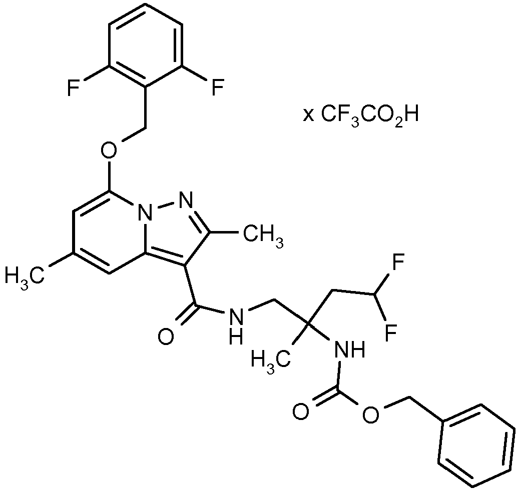

350 mg (1.05 mmol) 7-[(2,6-Difluorbenzyl)oxy]-2,5-dimethylpyrazolo[1,5-a]pyridin-3-carbonsäure aus Beispiel 4A wurden in 3.5 ml DMF gelöst, mit 481 mg (1.26 mmol) HATU und 0.92 ml (5.27 mmol) N,N-Diisopropylethylamin versetzt und 20 min bei Raumtemperatur gerührt. Anschließend wurden 366 mg (1.26 mmol, Reinheit 93%) *ent*-Benzyl-(1-amino-4,4-difluor-2-methylbutan-2-yl)carbamat (Enantiomer B) aus Beispiel 83A versetzt. Nach vollständiger Abreaktion (ca. 30 min) des Startmaterials wurde mit Wasser versetzt, der entstandene Feststoff abfiltriert und mit Wasser gewaschen. Der Rückstand wurde mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Es wurden 281 mg der Titelverbindung (38% d. Th., Reinheit 99%) erhalten.
LC-MS (Methode 2): Rₜ = 1.22 min
MS (ESpos): m/z = 587 (M-TFA+H)⁺

### Beispiel 96A

### Methyl-7-[(2,6-difluorbenzyl)oxy]-5-methyl-2-propylpyrazolo[1,5-a]pyridin-3-carboxylat

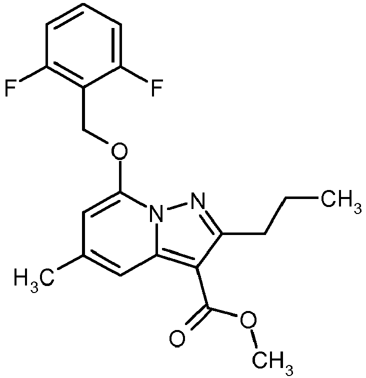

0.50 g (1.01 mmol) 1-Amino-2-[(2,6-difluorbenzyl)oxy]-4-methylpyridinium-2,4,6-trimethylbenzolsulfonat aus Beispiel 2A wurden in 3.3 ml DMF gelöst und mit 229 mg (1.82 mmol) Methylhex-2-inoat versetzt. Es wurden 251 mg (1.82 mmol) Kaliumcarbonat zugegeben und die Mischung wurde für 3 h bei RT gerührt. Anschließend wurde das Gemisch auf 24 ml Wasser gegossen, kurz ausgerührt, der ausgefallene Feststoff abfiltriert, mit Wasser gewaschen und getrocknet. Es wurden 169 mg der Titelverbindung (42% d. Th.; Reinheit 95%) erhalten.
LC-MS (Methode 2): Rₜ = 1.25 min
MS (ESpos): m/z = 375 (M+H)⁺

In Analogie zu Beispiel 96A wurden die in Tabelle 1A gezeigten Beispielverbindungen hergestellt, indem 1-Amino-2-[(2,6-difluorbenzyl)oxy]-4-methylpyridinium-2,4,6-trimethylbenzolsulfonat aus Beispiel 2A mit den entsprechenden, kommerziell erhältlichen oder literaturbekannten Alkinen unter den beschriebenen Bedingungen umgesetzt wurden:

**Tabelle 1A:**

| **Beispiel** | **IUPAC-Name/Struktur (Ausbeute)** | **Analytische Daten** |
|---|---|---|
| **97A** | Methyl-7-[(2,6-difluorbenzyl)oxy]-5-methylpyrazolo[1,5-a]pyridin-3-carboxylat | LC-MS (Methode 2): Rₜ = 1.05 min MS (ESpos): m/z = 333 (M+H)⁺ |
| | 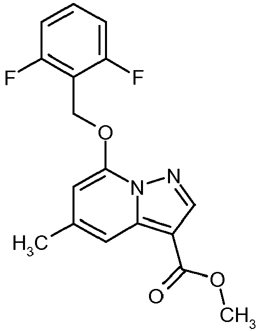 | |
| | (71% d. Th.; Reinheit 85%) | |
| **98A** | Ethyl-7-[(2,6-difluorbenzyl)oxy]-5-methyl-2-(trifluormethyl)pyrazolo[1,5-a]pyridin-3-carboxylat | LC-MS (Methode 2): Rₜ = 1.26 min MS (ESpos): m/z = 415 (M+H)⁺ |
| | 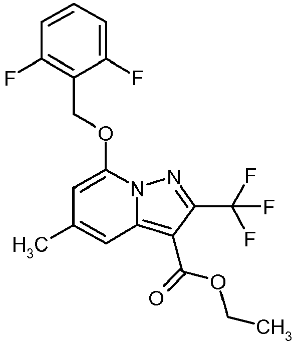 | |
| | (39% d. Th.; Reinheit 86%) | |

### Beispiel 99A

### Methyl-2-cyclopropyl-7-[(2,6-difluorbenzyl)oxy]-5-methylpyrazolo[1,5-a]pyridin-3-carboxylat

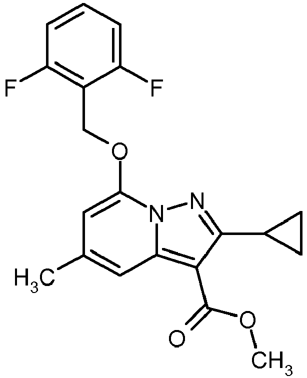

1.0 g (2.22 mmol) 1-Amino-2-[(2,6-difluorbenzyl)oxy]-4-methylpyridinium-2,4,6-trimethylbenzolsulfonat aus Beispiel 2A wurden in 7.2 ml DMF gelöst und mit 496 mg (4.00 mmol) Methyl-3-cyclopropylprop-2-inoat versetzt. Es wurden 552 mg (4.00 mmol) Kaliumcarbonat zugegeben und die Mischung wurde für 3 h bei RT gerührt. Anschließend wurde das Gemisch auf 50 ml Wasser gegossen, kurz ausgerührt, der ausgefallene Feststoff abfiltriert, mit Wasser gewaschen und getrocknet. Es wurden 384 mg der Titelverbindung (46% d. Th.) erhalten.
LC-MS (Methode 2): Rₜ = 1.20 min
MS (ESpos): m/z = 373 (M+H)⁺
¹H-NMR (500 MHz, DMSO-d₆): δ [ppm] = 0.88 - 0.94 (m, 2H), 0.95 - 1.00 (m, 2H), 2.43 (s, 3H), 2.70 - 2.77 (m, 1H), 3.83 (s, 3H), 5.46 (s, 2H), 6.66 (s, 1H), 7.21 - 7.27 (m, 2H), 7.48 (s, 1H), 7.57 - 7.64 (m, 1H).

### Beispiel 100A

### 7-[(2,6-Difluorbenzyl)oxy]-5-methyl-2-propylpyrazolo[1,5-a]pyridin-3-carbonsäure

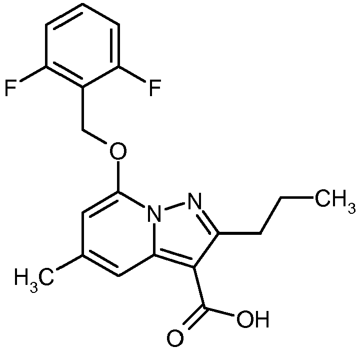

Eine Lösung von 169 mg (0.43 mmol; Reinheit ca. 95%) Methyl-7-[(2,6-difluorbenzyl)oxy]-5-methyl-2-propylpyrazolo[1,5-a]pyridin-3-carboxylat aus Beispiel 96A in 4.5 ml Dioxan wurde mit 2.6 ml (2.6 mmol) 1 N Natronlauge versetzt und 24 h bei 90°C zur Reaktion gebracht. Anschließend wurden nochmals 0.5 ml (0.5 mmol) 1 N Natronlauge hinzugegeben und 1 h bei 90°C gerührt. Die Reaktionslösung wurde abgekühlt und mit 1 N Salzsäure auf pH 2 gestellt. Der dabei ausgefallene Feststoff wurde abfiltriert und im Hochvakuum getrocknet. Es wurden 180 mg der Titelverbindung (72%ig laut LC-MS, 84% d. Th.) erhalten und ohne weitere Reinigung umgesetzt.
LC-MS (Methode 2): Rₜ = 1.04 min
MS (ESpos): m/z = 361 (M+H)⁺

In Analogie zu Beispiel 100A wurden die in Tabelle 2A gezeigten Beispielverbindungen hergestellt, indem die korrespondierenden Carbonsäurester mit Natronlauge (6 - 10 Äquivalente) unter den beschriebenen Bedingungen umgesetzt wurden. Die Reaktionstemperatur betrug 90 bis 100°C, die Reaktionsdauer lag zwischen 10 h und 24 h:

**Tabelle 2A:**

| **Beispiel** | **IUPAC-Name/Struktur (Ausbeute)** | **Analytische Daten** |
|---|---|---|
| **101A** | 7-[(2,6-Difluorbenzyl)oxy]-5-methylpyrazolo[1,5-a]pyridin-3-carbonsäure ¹⁾ | LC-MS (Methode 2): Rₜ = 0.89 min MS (ESpos): m/z = 319 (M+H)⁺ |
| | 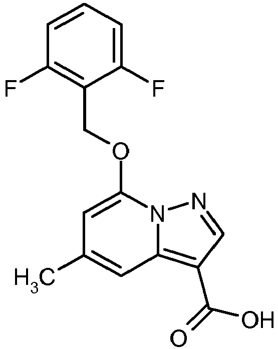 | |
| | (42% d. Th.) | |
| **102A** | 7-[(2,6-Difluorbenzyl)oxy]-5-methyl-2-(trifluormethyl)pyrazolo[1,5-a]pyridin-3-carbonsäure | LC-MS (Methode 2): Rₜ = 1.02 min MS (ESpos): m/z = 387 (M+H)⁺ |
| | 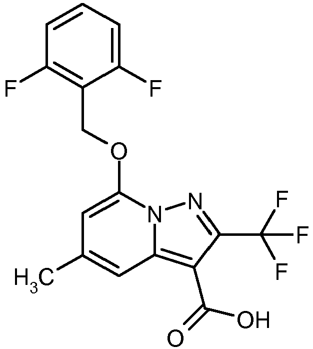 | |
| | (65% d. Th.; Reinheit ca. 90%) | |

| | | |
|---|---|---|
| ¹⁾ Alternative Aufarbeitung: Die Reaktionslösung wurde abgekühlt und mit 1 N Salzsäure auf pH = 2 gestellt. Der dabei ausgefallene Feststoff wurde abfiltriert und getrocknet. Der Rückstand wurde zweimal mit Dichlormethan/Cyclohexan (6/1) ausgerührt. Die Suspension wurde jeweils ab filtriert. Der vereinigten Feststoffe wurden im Hochvakuum getrocknet. | | |

### Beispiel 103A

### 2-Cyclopropyl-7-[(2,6-difluorbenzyl)oxy]-5-methylpyrazolo[1,5-a]pyridin-3-carbonsäure

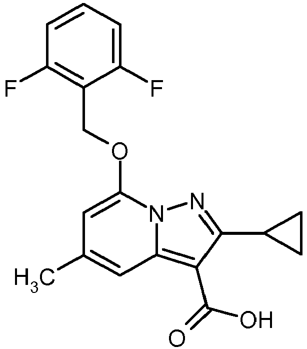

Eine Lösung von 384 mg (1.02 mmol) Methyl-2-cyclopropyl-7-[(2,6-difluorbenzyl)oxy]-5-methylpyrazolo[1,5-a]pyridin-3-carboxylat aus Beispiel 99A in 10.6 ml Dioxan wurde mit 10.2 ml (10.2 mmol) 1 N Natronlauge versetzt und das Gemisch wurde 7 h bei 100°C gerührt. Die Reaktionslösung wurde abgekühlt und mit 1 N Salzsäure auf pH 2 gestellt. Der dabei ausgefallene Feststoff wurde abfiltriert und im Hochvakuum getrocknet. Das Filtrat wurde nochmals mit 1 N Salzsäure versetzt. Der dabei ausgefallene Feststoff wurde abfiltriert und und im Hochvakuum zusammen mit dem zuvor isolierten Feststoff getrocknet. Es wurden insgesamt 361 mg der Titelverbindung (74%ig laut LC-MS, 73% d. Th.) erhalten und ohne weitere Reinigung umgesetzt.
LC-MS (Methode 2): Rₜ = 1.00 min
MS (ESpos): m/z = 359 (M+H)⁺
¹H-NMR (400 MHz, DMSO-d₆): δ [ppm] = 0.87 - 0.99 (m, 4H), 2.42 (s, 3H), 2.73 - 2.82 (m, 1H), 5.45 (s, 2H), 6.61 (s, 1H), 7.20 - 7.28 (m, 2H), 7.48 (s, 1H), 7.55 - 7.65 (m, 1H), 12.29 (br. s, 1H).

### Beispiel 104A

### ent-Benzyl-{1-[({7-[(2,6-difluorbenzyl)oxy]-5-methyl-2-propylpyrazolo[1,5-a]pyridin-3-yl}carbonyl)amino]-2-methylpentan-2-yl}carbamat Trifluoracetat (Enantiomer B)

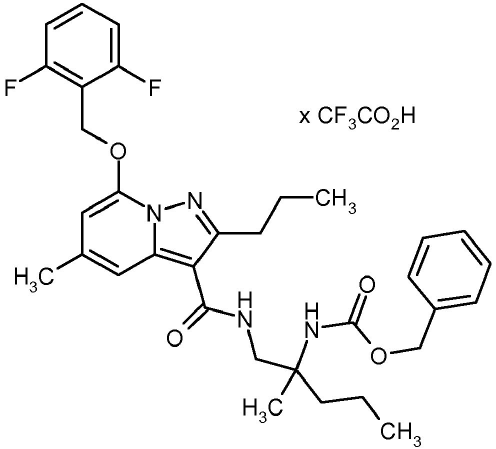

90 mg (0.19 mmol; Reinheit ca. 75%) 7-[(2,6-Difluorbenzyl)oxy]-5-methyl-2-propylpyrazolo[1,5-a]pyridin-3-carbonsäure aus Beispiel 100A wurden mit 78 mg (0.21 mmol) HATU und 0.1 ml (0.56 mmol) N,N-Diisopropylethylamin in 0.6 ml DMF vorgelegt und 10 min bei Raumtemperatur gerührt. Anschließend wurden 64 mg (0.23 mmol) *ent*-Benzyl-(1-amino-2-methylpentan-2-yl)carbamat (Enantiomer B) aus Beispiel 20A zur Reaktionslösung gegeben und über Nacht bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, und eingeengt. Es wurden 54 mg der Zielverbindung (41 % d. Th.) erhalten.
LC-MS (Methode 2): Rₜ = 1.34 min
MS (ESpos): m/z = 593.6 (M-TFA+H)⁺

In Analogie zu Beispiel 104A wurden die in Tabelle 3A gezeigten Beispielverbindungen hergestellt, indem die entsprechenden Carbonsäuren mit *ent*-Benzyl-(1-amino-2-methylpentan-2-yl)carbamat (Enantiomer B) aus Beispiel 20A (1.1 - 1.5 Äquivalente), HATU (1.1 - 3 Äquivalente) und *N,N-*Diisopropylethylamin (3 - 5 Äquivalente) unter den beschriebenen Reaktionsbedingungen (Reaktionszeit: 0.5 - 24 h; Temperatur: RT) umgesetzt wurden.

Beispielhafte Aufarbeitung der Reaktionsmischung:
Das Reaktionsgemisch wurde mit Wasser, TFA oder Ameisensäure verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Dickschichtchromatographie oder Kieselgelchromatographie gereinigt (Laufmittel: Dichlormethan/Methanol oder Dichlormethan/2 M Ammoniak in Methanol). Die produkthaltigen Fraktionen wurden eingedampft.

Die produkthaltigen Fraktionen der präparativen HPLC wurden ggf. eingedampft, der Rückstand in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

**Tabelle 3A:**

| **Beispiel** | **IUPAC-Name / Struktur (Ausbeute)** | **Analytische Daten** |
|---|---|---|
| **105A** | *ent*-Benzyl-{1-[({7-[(2,6-difluorbenzyl)oxy]-5-methyl-2-(trifluormethyl)pyrazolo[1,5-a]pyridin-3-yl}carbonyl)amino]-2-methylpentan-2-yl}carbamat Trifluoracetat (Enantiomer B) | LC-MS (Methode 2): Rₜ = 1.30 min MS (ESpos): m/z = 619 (M-TFA+H)⁺ |
| | 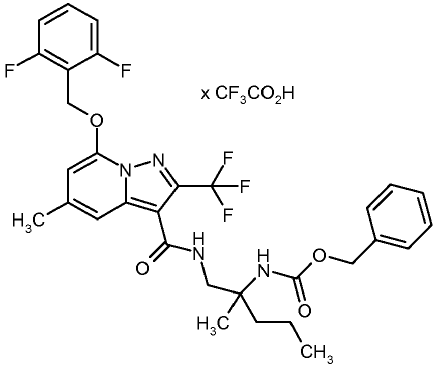 | |
| | (53% d. Th.) | |
| **106A** | *ent*-Benzyl-{1-[({7-[(2,6-difluorbenzyl)oxy]-5-methylpyrazolo[1,5-a]pyridin-3-yl}carbonyl)amino]-2-methylpentan-2-yl}carbamat Trifluoracetat (Enantiomer B) | LC-MS (Methode 2): Rₜ = 1.29 min MS (ESpos): m/z = 551 (M-TFA+H)⁺ |
| | 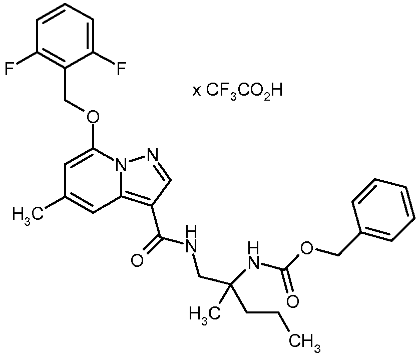 | |
| | (63% d. Th.) | |

### Beispiel 107A

### ent-Benzyl-{1-[({2-cyclopropyl-7-[(2,6-difluorbenzyl)oxy]-5-methylpyrazolo[1,5-a]pyridin-3-yl}carbonyl)amino]-2-methylbutan-2-yl}carbamat Trifluoracetat (Enantiomer A)

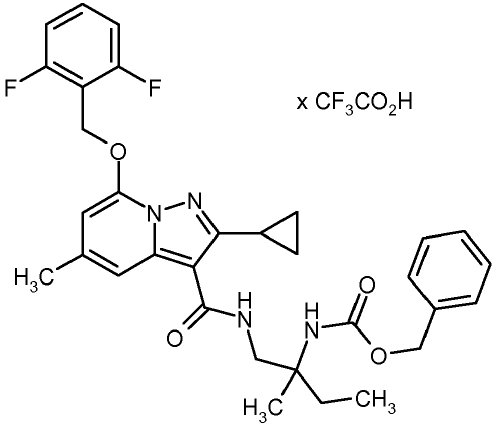

70 mg (0.15 mmol; Reinheit ca. 74%) 2-Cyclopropyl-7-[(2,6-difluorbenzyl)oxy]-5-methylpyrazolo[1,5-a]pyridin-3-carbonsäure aus Beispiel 103A wurden mit 71 mg (0.19 mmol) HATU und 0.13 ml (0.72 mmol) N,N-Diisopropylethylamin in 0.5 ml DMF vorgelegt und 20 min bei Raumtemperatur gerührt. Anschließend wurden 44 mg (0.19 mmol) *ent*-Benzyl-(1-amino-2-methylbutan-2-yl)carbamat (Enantiomer A) aus Beispiel 14A zur Reaktionslösung gegeben und 1 h bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, und eingeengt. Es wurden 57 mg der Zielverbindung (51% d. Th.; Reinheit 90%) erhalten.
LC-MS (Methode 2): Rₜ = 1.26 min
MS (ESpos): m/z = 577 (M-TFA+H)⁺

### Beispiel 108A

### ent-Benzyl-{1-[({2-cyclopropyl-7-[(2,6-difluorbenzyl)oxy]-5-methylpyrazolo[1,5-a]pyridin-3-yl}carbonyl)amino]-2-methylpentan-2-yl}carbamat Trifluoracetat (Enantiomer B)

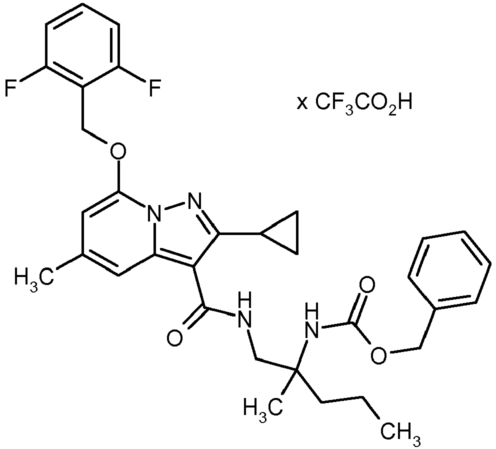

107 mg (0.22 mmol; Reinheit ca. 74%) 2-Cyclopropyl-7-[(2,6-difluorbenzyl)oxy]-5-methylpyrazolo[1,5-a]pyridin-3-carbonsäure aus Beispiel 103A wurden mit 125 mg (0.33 mmol) HATU und 0.16 ml (0.90 mmol) N,N-Diisopropylethylamin in 1.0 ml DMF vorgelegt und 10 min bei Raumtemperatur gerührt. Anschließend wurden 82 mg (0.29 mmol: Reinheit 88%) *ent*-Benzyl-(1-amino-2-methylpentan-2-yl)carbamat (Enantiomer B) aus Beispiel 20A zur Reaktionslösung gegeben und 1 h bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, und eingeengt. Es wurden 88 mg der Zielverbindung (57% d. Th.) erhalten.
LC-MS (Methode 2): Rₜ = 1.31 min
MS (ESpos): m/z = 591 (M-TFA+H)⁺

### Beispiel 109A

### ent-Benzyl-{1-[({2-cyclopropyl-7-[(2,6-difluorbenzyl)oxy]-5-methylpyrazolo[1,5-a]pyridin-3-yl}carbonyl)amino]-5,5,5-trifluor-2-methylpentan-2-yl}carbamat Trifluoracetat (Enantiomer B)

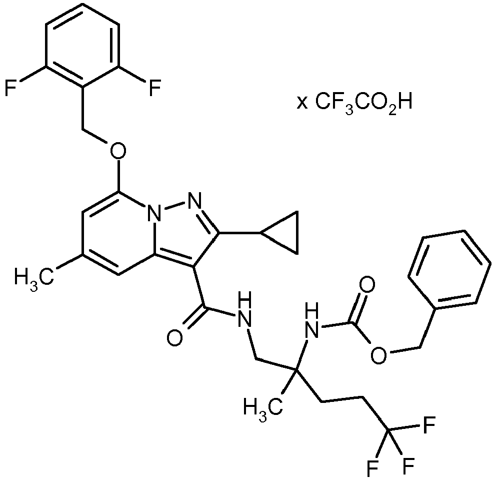

100 mg (0.21 mmol; Reinheit ca. 74%) 2-Cyclopropyl-7-[(2,6-difluorbenzyl)oxy]-5-methylpyrazolo[1,5-a]pyridin-3-carbonsäure aus Beispiel 103A wurden mit 102 mg (0.27 mmol) HATU und 0.18 ml (1.03 mmol) N,N-Diisopropylethylamin in 0.7 ml DMF vorgelegt und 10 min bei Raumtemperatur gerührt. Anschließend wurden 86 mg (0.27 mmol: Reinheit 95%) *ent*-Benzyl-(1-amino-5,5,5-trifluor-2-methylpentan-2-yl)carbamat (Enantiomer B) aus Beispiel 65A zur Reaktionslösung gegeben und 0.5 h bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Es wurden 97 mg der Zielverbindung (61% d. Th.) erhalten.
LC-MS (Methode 2): Rₜ = 1.32 min
MS (ESpos): m/z = 645 (M-TFA+H)⁺

### Beispiel 110A

### ent-Benzyl-{1-[({2-cyclopropyl-7-[(2,6-difluorbenzyl)oxy]-5-methylpyrazolo[1,5-a]pyridin-3-yl}carbonyl)amino]-4,4-difluor-2-methylbutan-2-yl}carbamat Trifluoracetat (Enantiomer A)

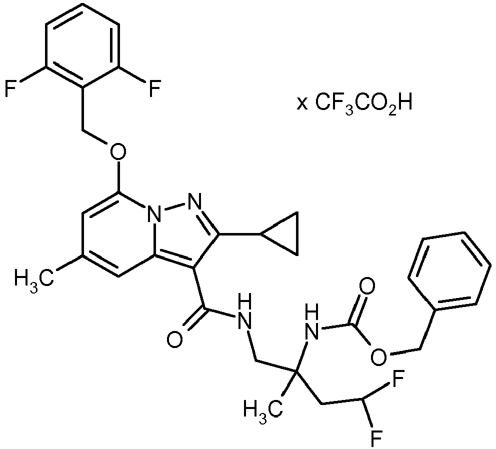

60 mg (0.12 mmol; Reinheit ca. 74%) 2-Cyclopropyl-7-[(2,6-difluorbenzyl)oxy]-5-methylpyrazolo[1,5-a]pyridin-3-carbonsäure aus Beispiel 103A wurden mit 61 mg (0.16 mmol) HATU und 0.11 ml (0.62 mmol) N,N-Diisopropylethylamin in 0.4 ml DMF vorgelegt und 20 min bei Raumtemperatur gerührt. Anschließend wurden 58 mg (0.21 mmol: Reinheit 98%) *ent*-Benzyl-(1-amino-4,4-difluor-2-methylbutan-2-yl)carbamat (Enantiomer A) aus Beispiel 82A zur Reaktionslösung gegeben und 1 h bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Es wurden 55 mg der Zielverbindung (58% d. Th.; Reinheit 95%) erhalten.
LC-MS (Methode 2): Rₜ = 1.26 min
MS (ESpos): m/z = 613 (M-TFA+H)⁺

### Beispiel 111A

### ent-Benzyl-{1-[({2-cyclopropyl-7-[(2,6-difluorbenzyl)oxy]-5-methylpyrazolo[1,5-a]pyridin-3-yl}carbonyl)amino]-4,4-difluor-2-methylbutan-2-yl}carbamat Trifluoracetat (Enantiomer B)

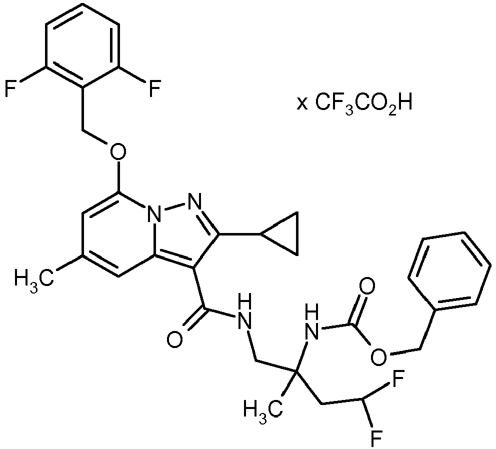

60 mg (0.12 mmol; Reinheit ca. 74%) 2-Cyclopropyl-7-[(2,6-difluorbenzyl)oxy]-5-methylpyrazolo[1,5-a]pyridin-3-carbonsäure aus Beispiel 103A wurden mit 61 mg (0.16 mmol) HATU und 0.11 ml (0.62 mmol) N,N-Diisopropylethylamin in 0.4 ml DMF vorgelegt und 20 min bei Raumtemperatur gerührt. Anschließend wurden 50 mg (0.16 mmol: Reinheit 93%) *ent*-Benzyl-(1-amino-4,4-difluor-2-methylbutan-2-yl)carbamat (Enantiomer B) aus Beispiel 83A zur Reaktionslösung gegeben und 0.5 h bei RT gerührt. Das Gemisch wurde mit Wasser versetzt und 0.5 h bei RT verrührt. Der erhaltene Feststoff wurde abfiltriert und mit Wasser gewaschen. Der Feststoff wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Es wurden 54 mg der Zielverbindung (49% d. Th.; Reinheit 82%) erhalten.
LC-MS (Methode 2): Rₜ = 1.26 min
MS (ESpos): m/z = 613 (M-TFA+H)⁺

### Beispiel 112A

### tert-Butyl-{3-[({7-[(2,6-difluorbenzyl)oxy]-2,5-dimethylpyrazolo[1,5-a]pyridin-3-yl}carbonyl)amino]-2,2-difluorpropyl}carbamat Trifluoracetat

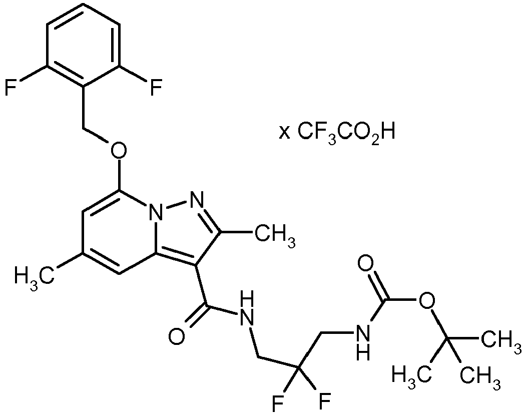

80 mg (0.18 mmol; Reinheit ca. 75%) 7-[(2,6-Difluorbenzyl)oxy]-2,5-dimethylpyrazolo[1,5-a]pyridin-3-carbonsäure aus Beispiel 4A wurden mit 89 mg (0.24 mmol) HATU und 94 µl (0.54 mmol) N,N-Diisopropylethylamin in 0.6 ml DMF vorgelegt und 20 min bei Raumtemperatur gerührt. Anschließend wurden 49 mg (0.24 mmol) tert-Butyl-(3-amino-2,2-difluorpropyl)carbamat zur Reaktionslösung gegeben und 30 min bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1 % TFA). Die Produktfraktionen wurden vereinigt, und eingedampft. Es wurden 77 mg der Zielverbindung (66% d. Th.) erhalten.
LC-MS (Methode 2): Rₜ = 1.21 min
MS (ESpos): m/z = 525 (M-TFA+H)⁺

In Analogie zu Beispiel 112A wurden die in Tabelle 4A gezeigten Beispielverbindungen hergestellt, indem 7-[(2,6-Difluorbenzyl)oxy]-2,5-dimethylpyrazolo[1,5-a]pyridin-3-carbonsäure aus Beispiel 4A mit den entsprechenden, kommerziell erhältlichen oder literaturbekannten Aminen (1.1 - 1.5 Äquivalente), HATU (1.1 - 3 Äquivalente) und *N,N-*Diisopropylethylamin (3 - 5 Äquivalente) unter den beschriebenen Reaktionsbedingungen (Reaktionszeit: 0.5 - 24 h; Temperatur: RT oder 60°C) umgesetzt wurden.

Beispielhafte Aufarbeitung der Reaktionsmischung:
Das Reaktionsgemisch wurde mit Wasser, TFA oder Ameisensäure verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Dickschichtchromatographie oder Kieselgelchromatographie gereinigt (Laufmittel: Dichlormethan/Methanol oder Dichlormethan/2 M Ammoniak in Methanol). Die produkthaltigen Fraktionen wurden eingedampft.

Die produkthaltigen Fraktionen der präparativen HPLC wurden ggf. eingedampft, der Rückstand in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

Alternativ dazu wurde die Reaktionslösung mit Wasser versetzt und der ausgefallene Feststoff ca. 30 min bei Raumtemperatur verrührt. Anschließend wurde der Feststoff abfiltriert, gut mit Wasser gewaschen und im Hochvakuum getrocknet.

**Tabelle 4A:**

| **Beispiel** | **IUPAC-Name / Struktur (Ausbeute)** | **Analytische Daten** |
|---|---|---|
| **113A** | *rac*-tert-Butyl-2-{[({7-[(2,6-difluorbenzyl)oxy]-2,5-dimethylpyrazolo[1,5-a]pyridin-3-yl}carbonyl)amino]methyl}-3,4-dihydrochinolin-1(2H)-carboxylat Trifluoracetat | LC-MS (Methode 2): Rₜ = 1.25 min MS (ESpos): m/z = 577 (M-TFA+H)⁺ |
| | 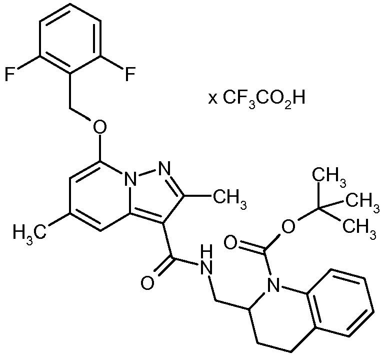 | |
| | (44% d. Th.) | |
| **114A** | *rac*-Ethyl-6-{2-[(tert-butoxycarbonyl)amino]-1-[({7-[(2,6-difluorbenzyl)oxy]-2,5-dimethylpyrazolo[1,5-a]pyridin-3-yl}carbonyl)amino]ethyl}pyridin-2-carboxylat Trifluoracetat | LC-MS (Methode 7): Rₜ = 2.77 min MS (ESpos): m/z = 624 (M-TFA+H)⁺ |
| | 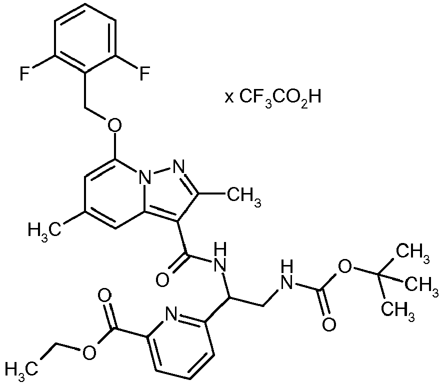 | |
| | (8% d. Th.) | |
| **115A** | *rac*-tert-Butyl-{2-(4-cyanphenyl)-2-[({7-[(2,6-difluorbenzyl)oxy]-2,5-dimethylpyrazolo[1,5-a]pyridin-3-yl}carbonyl)amino]ethyl}carbamat Trifluoracetat | LC-MS (Methode 2): Rₜ = 1.14 min MS (ESpos): m/z = 576 (M-TFA+H)⁺ |
| | 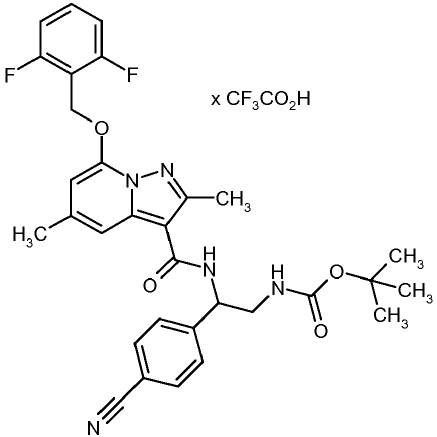 | |
| | (56% d. Th.) | |

### Beispiel 116A

### 7-[(2,3-Difluorbenzyl)oxy]-2,5-dimethylpyrazolo[1,5-a]pyridin-3-carbonsäureethylester

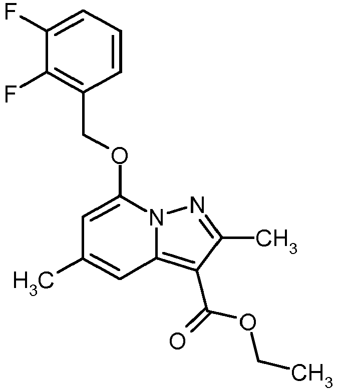

Eine Lösung von 180 mg (0.768 mmol) 7-Hydroxy-2,5-dimethylpyrazolo[1,5-a]pyridin-3-carbonsäureethylester aus Beispiel 8A in 7 ml trockenem Tetrahydrofuran wurde mit 221.5 mg (1.537 mmol) (2,3-Difluorphenyl)methanol (CAS 75853-18-8), 423.2 mg (1.614 mmol) Triphenylphosphin und 0.32 ml (1.614 mmol) Azodicarbonsäurediethylester versetzt. Die erhaltene Mischung wurde 1 h bei Raumtemperatur gerührt, ein Teil des Lösungsmittels wurde im Vakuum abgezogen und der Rückstand wurde mit 5 ml tert.-Butylmethylether versetzt. Der erhaltene Niederschlag wurde ab filtriert und im Vakuum getrocknet, wodurch man 190 mg (67% d. Th., 97% rein) der Zielverbindung erhielt.
LC-MS (Methode 16): Rₜ = 1.29 min
MS (ESpos): m/z = 361.24 (M+H)⁺
¹H-NMR (300 MHz, DMSO-d₆): δ [ppm] = 1.32 (t, 3 H), 2.43 (s, 3 H), 2.49 (s, 3 H), 4.20 - 3.32 (m, 2 H), 5.52 (s, 2 H), 6.65 (s, 1 H), 7.26 - 7.38 (m, 1 H), 7.41 - 7.65 (m, 3 H).

### Beispiel 117A

### 7-[(2,3-Difluorbenzyl)oxy]-2,5-dimethylpyrazolo[1,5-a]pyridin-3-carbonsäure

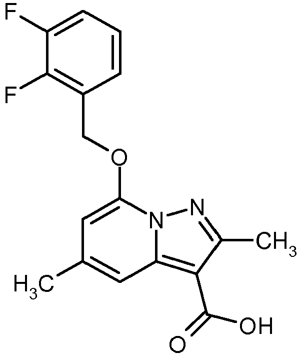

Eine Lösung von 190 mg (0.527 mmol) 7-[(2,3-Difluorbenzyl)oxy]-2,5-dimethylpyrazolo[1,5-a]pyridin-3-carbonsäureethylester aus Beispiel 116A in 5 ml Dioxan wurde mit 2.1 ml 2 N Natronlauge versetzt. Die erhaltene Mischung wurde 15 h bei 90°C gerührt. Aufgrund unvollständiger Umsetzung wurde mit 1 ml Dimethylsulfoxid versetzt, gefolgt von weiterer 2 N Natronlauge (1 ml). Die erhaltene Mischung wurde weitere 2 h gerührt. Nach dem Ende der Umsetzung wurde das Lösungsmittel im Vakuum entfernt. Der Rückstand wurde mit Acetonitril (5 ml) versetzt, gefolgt von der tropfenweisen Zugabe von Trifluoressigsäure (2 ml). Der erhaltene Niederschlag wurde abfiltriert und im Vakuum getrocknet, wodurch man 130 mg (71% d. Th., 93% rein) der Zielverbindung erhielt.
LC-MS (Methode 16): Rₜ = 1.01 min
MS (ESpos): m/z = 333.2 (M+H)⁺
¹H-NMR (300 MHz, DMSO-d₆): δ [ppm] = 2.40 (s, 3 H), 2.49 (s, 3 H), 5.50 (s, 2 H), 6.65 (s, 1 H), 7.25 - 7.35 (m, 1 H), 7.42 - 7.57 (m, 3 H).

### Beispiel 118A

### rac-{1-[({7-[(2,3-Difluorbenzyl)oxy]-2,5-dimethylpyrazolo[1,5-a]pyridin-3-yl}carbonyl)amino]-2-methylpentan-2-yl}carbamidsäure-tert.-butylester

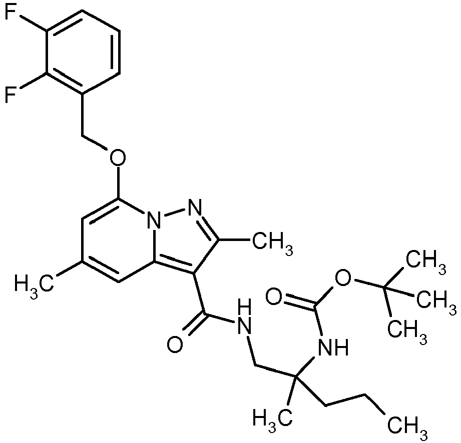

Eine Lösung von 120 mg (0.336 mmol) 7-[(2,3-Difluorbenzyl)oxy]-2,5-dimethylpyrazolo[1,5-a]pyridin-3-carbonsäure aus Beispiel 117A in 2 ml DMF wurde mit 70.8 mg (0.437 mmol) 1,1'-Carbonyldiimidazol und 36 mg (0.235 mmol) 1-Hydroxybenzotriazol versetzt. Nach 15 min Rühren bei Raumtemperatur wurden 94.4 mg (0.437 mmol) *rac*-(1-Amino-2-methylpentan-2-yl)carbamidsäure-tert.-butylester aus Beispiel 152A gefolgt von 0.117 ml (0.672 mmol) N,N-Diisopropylethylamin zugegeben. Das Röhrchen wurde verschlossen und in einen Biotage Initiator unter 20-minütiger Mikrowellenbestrahlung bei 100°C gegeben. Die Reaktionsmischung wurde zwischen Essigsäureethylester (10 ml) und Wasser (10 ml) verteilt, die Phasen wurden getrennt und die wässrige Phase wurde mit 2 N wässriger Salzsäure auf pH = 5 angesäuert und mit Dichlormethan (2 x 15 ml) extrahiert. Die vereinigten organischen Phasen wurden im Vakuum eingeengt und der Rückstand wurde durch Flash-Chromatographie unter Verwendung einer vorgepackten Kieselgelkartusche (Laufmittel: Cyclohexan-Essigsäureethylester 10:1 bis 1:1) aufgereinigt, wodurch man 35 mg (15% d. Th., 78% rein) der Zielverbindung erhielt.
LC-MS (Methode 16): Rₜ = 1.37 min
MS (ESpos): m/z = 531.5 (M+H)⁺

### Beispiel 119A

### 7-(Cyclohexylmethoxy)-2,5-dimethylpyrazolo[1,5-a]pyridin-3-carbonsäureethylester

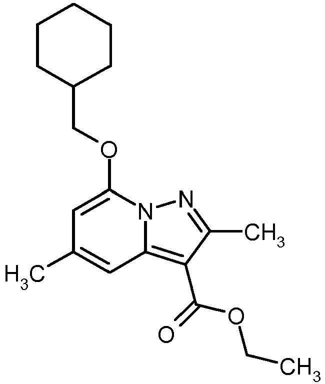

Eine Lösung von 180 mg (0.768 mmol) 7-Hydroxy-2,5-dimethylpyrazolo[1,5-a]pyridin-3-carbonsäureethylester aus Beispiel 8A in 7 ml trockenem Tetrahydrofuran wurde mit 0.189 ml (537 mmol) Cyclohexylmethanol, 423.2 mg (1.614 mmol) Triphenylphosphin und 0.32 ml (1.614 mmol) Azodicarbonsäurediethylester versetzt. Die erhaltene Mischung wurde 1 h bei Raumtemperatur gerührt. Ein Teil des Lösungsmittels wurde im Vakuum abgezogen und der Rückstand wurde mit 5 ml tert.-Butylmethylether versetzt. Der erhaltene Niederschlag wurde ab filtriert und die Mutterlauge wurde zwischen Dichlormethan (15 ml) und Wasser (10 ml) verteilt. Die organische Phase wurde abgetrennt und im Vakuum eingeengt. Der Rückstand wurde durch Flash-Chromatographie unter Verwendung einer vorgepackten Kieselgelkartusche (Laufmittel: Cyclohexan-Essigsäureethylester 10:1 bis 1:1) aufgereinigt, wodurch man 220 mg (60% d. Th., 71% rein) der Zielverbindung erhielt.
LC-MS (Methode 23): Rₜ = 1.46 min
MS (ESpos): m/z = 331.31 (M+H)⁺

### Beispiel 120A

### 7-(Cyclohexylmethoxy)-2,5-dimethylpyrazolo[1,5-a]pyridin-3-carbonsäure

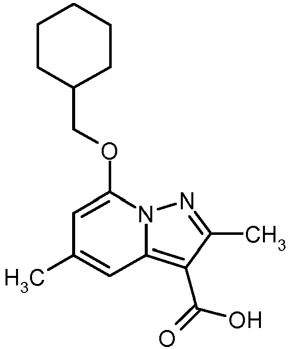

Eine Lösung von 220 mg (0.469 mmol, 71% rein) 7-(Cyclohexylmethoxy)-2,5-dimethylpyrazolo[1,5-a]pyridin-3-carbonsäureethylester aus Beispiel 119A in 4 ml Dioxan wurde mit 2 ml 2 N Natronlauge versetzt. Die erhaltene Mischung wurde 15 h bei 100°C gerührt. Das Lösungsmittel wurde im Vakuum abgezogen und der Rückstand wurde mit Acetonitril (5 ml) und dann tropfenweise mit Trifluoressigsäure (2 ml) versetzt. Der erhaltene Niederschlag wurde abfiltriert und im Vakuum getrocknet, wodurch man 110 mg (74% d. Th., 95% rein) der Zielverbindung erhielt.
LC-MS (Methode 16): Rₜ = 1.17 min
MS (ESpos): m/z = 303.22 (M+H)⁺

### Beispiel 121A

### rac-[1-({[7-(Cyclohexylmethoxy)-2,5-dimethylpyrazolo[1,5-a]pyridin-3-yl]carbonyl}amino)-2-methylpentan-2-yl]carbamidsäure-tert.-butylester

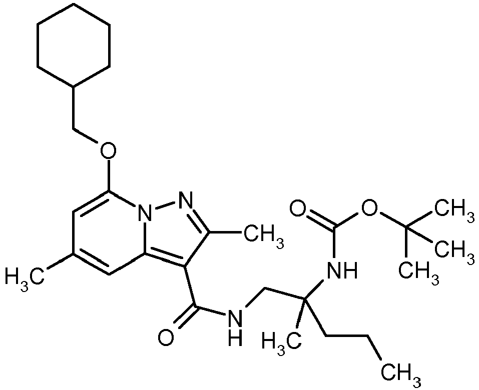

Eine Lösung von 110 mg (0.364 mmol) 7-(Cyclohexylmethoxy)-2,5-dimethylpyrazolo[1,5-a]pyridin-3-carbonsäure aus Beispiel 120A in 1 ml Thionylchlorid wurde 1 h bei 100°C gerührt. Nach dem Ende der Umsetzung wurde das Lösungsmittel im Vakuum abgezogen und der Rückstand wurde in 3 ml Dichlormethan suspendiert und dann zu einer Lösung von 102.3 mg (0,473 mmol) *rac*-(1-Amino-2-methylpentan-2-yl)carbamidsäure-tert.-butylester aus Beispiel 152A in 2 ml trockenem Dichlormethan gegeben. Die erhaltene Lösung wurde über Nacht bei Raumtemperatur gerührt. Das Lösungsmittel wurde im Vakuum abgezogen und der Rückstand wurde durch Flash-Chromatographie unter Verwendung einer vorgepackten Kieselgelkartusche (Laufmittel: Cyclohexan-Essigsäureethylester 10:1 bis 1:1) aufgereinigt, wodurch man 40 mg (13% d. Th.; 59% rein) der Zielverbindung erhielt.
LC-MS (Methode 16): Rₜ = 1.51 min
MS (ESpos): m/z = 501.55 (M+H)⁺

### Beispiel 122A

### 2,5-Dimethyl-7-(3-methylbutoxy)pyrazolo[1,5-a]pyridin-3-carbonsäureethylester

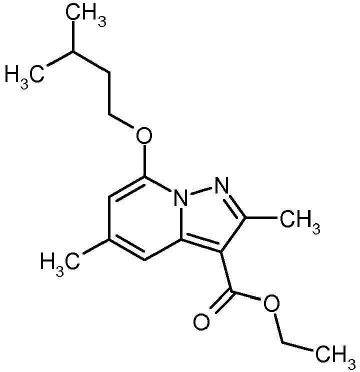

Eine Lösung von 180 mg (0.768 mmol) 7-Hydroxy-2,5-dimethylpyrazolo[1,5-a]pyridin-3-carbonsäureethylester aus Beispiel 8A in 7 ml trockenem Tetrahydrofuran wurde mit 0.167 ml (1.537 mmol) 3-Methylbutan-1-ol (CAS 123-51-3), 423.2 mg (1.614 mmol) Triphenylphosphin und 0.32 ml (1.614 mmol) Azodicarbonsäurediethylester versetzt. Die erhaltene Mischung wurde 1 h bei Raumtemperatur gerührt. Ein Teil des Lösungsmittels wurde im Vakuum abgezogen und der Rückstand wurde mit 5 ml tert.-Butylmethylether versetzt. Der erhaltene Niederschlag wurde abfiltriert und die Mutterlauge wurde zwischen Dichlormethan (15 ml) und Wasser (10 ml) verteilt. Die organische Phase wurde abgetrennt und im Vakuum eingeengt. Der Rückstand wurde durch Flash-Chromatographie unter Verwendung einer vorgepackten Kieselgelkartusche (Laufmittel: Cyclohexan-Essigsäureethylester 10:1 bis 1:1) aufgereinigt, wodurch man 225 mg (69% d. Th., 72% rein) der Zielverbindung erhielt.
LC-MS (Methode 16): Rₜ = 1.35 min
MS (ESpos): m/z = 305.28 (M+H)⁺

### Beispiel 123A

### 2,5-Dimethyl-7-(3-methylbutoxy)pyrazolo[1,5-a]pyridin-3-carbonsäure

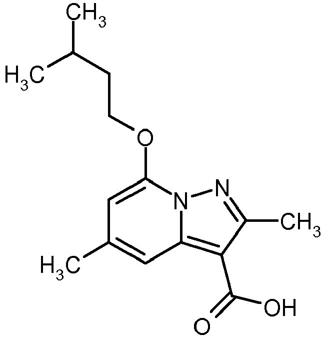

Eine Lösung von 220 mg (0.469 mmol, 72% rein) 2,5-Dimethyl-7-(3-methylbutoxy)pyrazolo[1,5-a]pyridin-3-carbonsäureethylester aus Beispiel 122A in 4 ml Dioxan wurde mit 2 ml 2 N Natronlauge versetzt. Die erhaltene Mischung wurde 15 h bei 100°C gerührt. Das Lösungsmittel wurde im Vakuum entfernt. Der Rückstand wurde mit Acetonitril (5 ml) versetzt, gefolgt von der tropfenweisen Zugabe von Trifluoressigsäure (2 ml). Der erhaltene Niederschlag wurde abfiltriert und im Vakuum getrocknet, wodurch man 45 mg (28% d. Th., 90% rein) der Zielverbindung erhielt.
LC-MS (Methode 16): Rₜ = 1.06 min
MS (ESpos): m/z = 277.26 (M+H)⁺
¹H-NMR (300 MHz, DMSO-d₆): δ [ppm] = 0.95 (d, 6H), 1.68 - 1.89 (m, 1 H), 2.39 (s, 3 H), 2.48 (s, 3 H), 4.31 (s, 2 H), 6.44 (s, 1 H), 7.39 (s, 1 H).

### Beispiel 124A

### rac-[1-({[2,5-Dimethyl-7-(3-methylbutoxy)pyrazolo[1,5-a]pyridin-3-yl]carbonyl}amino)-2-methylpentan-2-yl]carbamidsäure-tert.-butylester

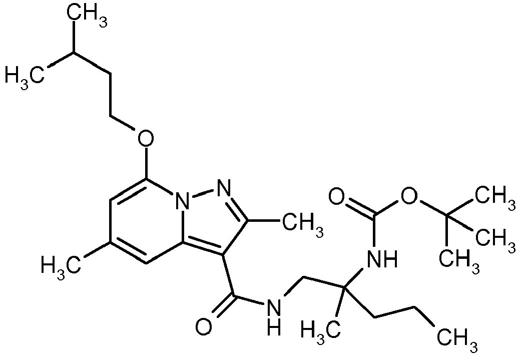

Eine Lösung von 45 mg (0.163 mmol) 2,5-Dimethyl-7-(3-methylbutoxy)pyrazolo[1,5-a]pyridin-3-carbonsäure aus Beispiel 8A in 3 ml Tetrahydrofuran wurde mit 30 mg (0.177 mmol) 1-Hydroxybenzotriazol und 30 mg (0.177 mmol) 1-(3-Dimethylaminopropyl)-3-ethylcarbodiimid versetzt. Die erhaltene Lösung wurde 15 min bei Raumtemperatur gerührt und dann mit 45.7 mg (0.212 mmol) *rac*-(1-Amino-2-methylpentan-2-yl)carbamidsäure-tert.-butylester aus Beispiel 152A gefolgt von 0.85 ml (0.212 mmol) N,N-Diisopropylethylamin versetzt. Es wurde weitere 18 h bei Raumtemperatur gerührt. Das Lösungsmittel wurde im Vakuum abgezogen und der Rückstand wurde zwischen Essigsäureethylester (10 ml) und Wasser (10 ml) verteilt. Die organische Phase wurde abgetrennt und mit Wasser und Wässrige Natriumchlorid-Lösung gewaschen, mit einer Phasentrennkartusche getrocknet und weiter im Vakuum getrocknet, wodurch man 80 mg (61% d. Th., 54% rein) der Zielverbindung erhielt.
LC-MS (Methode 16): Rₜ = 1.43 min
MS (ESpos): m/z = 475.45 (M+H)⁺

### Beispiel 125A

### rac-{1-[({7-[(2,6-Difluor-3-methoxybenzyl)oxy]-2,5-dimethylpyrazolo[1,5-a]pyridin-3-yl}carbonyl)amino]-2-methylpentan-2-yl}carbamidsäure-tert.-butylester

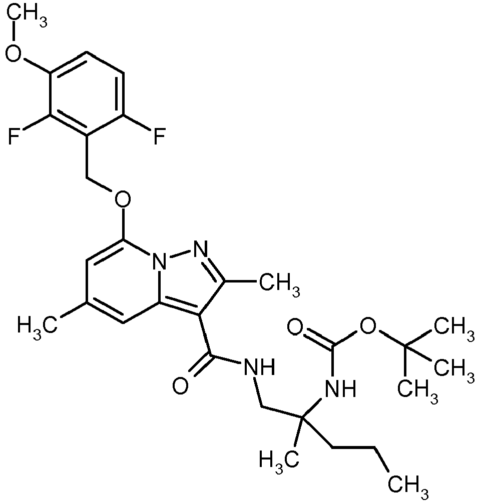

Eine Lösung von 80 mg (0.20 mmol) *rac*-(1-{[(7-Hydroxy-2,5-dimethylpyrazolo[1,5-a]pyridin-3-yl)carbonyl]amino}-2-methylpentan-2-yl)carbamidsäure-tert.-butylester aus Beispiel 141A und 38 mg (0.20 mmol) 2-(Chlormethyl)-1,3-difluor-4-methoxybenzol aus Beispiel 133A in 2 ml DMF wurde mit 129 mg (0.396 mmol) Cäsiumcarbonat versetzt. Die erhaltene Suspension wurde 72 h bei Raumtemperatur gerührt. Die Reaktionsmischung wurde mit Dichlormethan (15 ml) verdünnt und mit gesättigter wässriger Natriumhydrogencarbonatlösung (10 ml) extrahiert. Die organische Phase wurde im Vakuum eingedampft, wodurch man ein Rohmaterial erhielt, das die Zielverbindung in einer Mehrkomponentenmischung enthielt (30% rein). Das Rohmaterial wurde ohne weitere Aufreinigung in den nächsten Schritt eingesetzt.
LC-MS (Methode 16): Rₜ = 1.36 min
MS (ESpos): m/z = 561.42 (M+H)⁺

### Beispiel 126A

### rac-{{1-[({7-[(3-Cyano-2,6-difluorbenzyl)oxy]-2,5-dimethylpyrazolo[1,5-a]pyridin-3-yl}carbonyl)amino]-2-methylpentan-2-yl}carbamidsäure-tert.-butylester

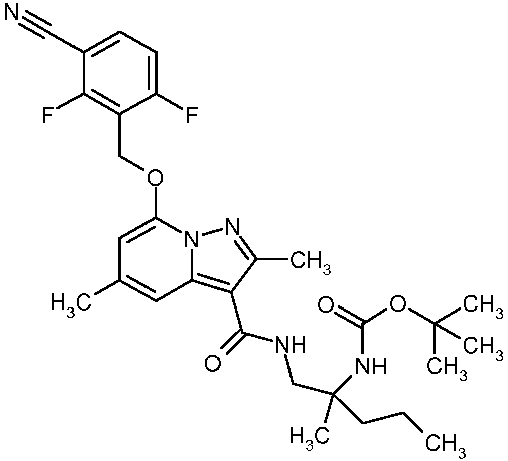

Eine Lösung von 80 mg (0.20 mmol) *rac*-(1-{[(7-Hydroxy-2,5-dimethylpyrazolo[1,5-a]pyridin-3-yl)carbonyl]amino}-2-methylpentan-2-yl)carbamidsäure-tert.-butylester aus Beispiel 141A und 56 mg (0.27 mmol) 3-(Chlormethyl)-2,4-difluorbenzonitril Beispiel 135A in 3 ml DMF wurde mit 129 mg (0.396 mmol) Cäsiumcarbonat versetzt. Die erhaltene Suspension wurde 72 h bei Raumtemperatur gerührt und dann mit Dichlormethan (15 ml) verdünnt und mit gesättigter wässriger Natriumhydrogencarbonatlösung (10 ml) extrahiert. Die organische Phase wurde im Vakuum eingedampft, wodurch man ein Rohprodukt erhielt, das die Zielverbindung in einer Mehrkomponentenmischung enthielt (20% rein). Dieses Rohmaterial wurde ohne weitere Aufreinigung in den nächsten Schritt eingesetzt.
LC-MS (Methode 16): Rₜ = 1.33 min
MS (ESpos): m/z = 556.39 (M+H)⁺

### Beispiel 127A

### 2-[(2,6-Difluorbenzyl)oxy]pyridin

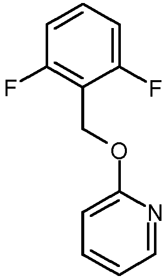

Eine Lösung von 2 ml (18.0 mmol) (2,6-Difluorphenyl)methanol in 15 ml bei 0°C gerührtem trockenem Tetrahydrofuran wurde mit 865.9 mg (21.6 mmol) Natriumhydrid (60% in Mineralöl) versetzt, gefolgt von 1.72 ml (18.0 mmol) 2-Brompyridin. Die erhaltene Lösung wurde 15 h bei Raumtemperatur gerührt. Die Reaktionsmischung wurde mit Wasser (15 ml) verdünnt und mit Dichlormethan (2 x 20 ml) extrahiert. Die organische Phase wurde abgetrennt, mit einer Phasentrennkartusche getrocknet und im Vakuum eingeengt. Der Rückstand wurde durch Flash-Chromatographie unter Verwendung einer vorgepackten Kieselgelkartusche (Laufmittel: Cyclohexan-Essigsäureethylester 10:1 bis 1:1) aufgereinigt, wodurch man 800 mg (15% d. Th., 77% rein) der Zielverbindung erhielt.
LC-MS (Methode 16): Rₜ = 1.17 min
MS (ESpos): m/z = 222.13 (M+H)⁺

### Beispiel 128A

### 1-Amino-2-[(2,6-difluorbenzyl)oxy]pyridinium 2,4,6-trimethylbenzolsulfonat

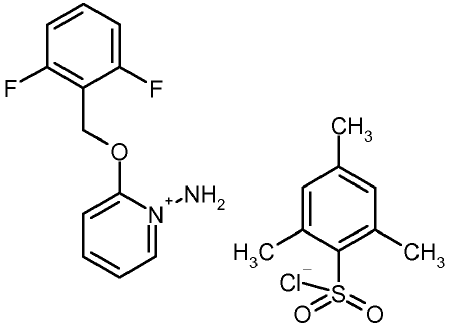

1.1 g (4.01 mmol) O-(2-Mesitylensulfonyl)acetohydroxamsäureethylester (CAS 38202-27-6) wurden portionsweise zu einer auf -5°C vorgekühlten Mischung von Trifluoressigsäure (3 ml) und Wasser (0.5 ml) gegeben. Die erhaltene Mischung wurde 1.5 Stunden lang bei -5°C gerührt und mit 10 ml Eis/Wasser verdünnt. Die Mischung wurde mit Dichlormethan (10 ml) extrahiert. Die organische Phase wurde über Natriumsulfat getrocknet, filtriert und zu einer auf 0°C vorgekühlten Lösung von 800 mg (2.67 mmol, 77% rein) 2-[(2,6-Difluorbenzyl)oxy]pyridin aus Beispiel 127A in Dichlormethan (2 ml) gegeben. Die Mischung wurde 18 Stunden lang bei Raumtemperatur gerührt. Die Mischung wurde unter Rühren im Verlauf von 40 Minuten tropfenweise zu Diethylether (500 ml) gegeben. Es wurde 1 weitere Stunde lang bei Raumtemperatur gerührt, was einen Niederschlag lieferte, der abfiltriert und mit Diethylether (50 ml) gewaschen wurde, wodurch man 540 mg (36% d. Th., 81% rein) der Zielverbindung erhielt.
LC-MS (Methode 16): Rₜ = 0.56 min
MS (ESpos): m/z = 237.16 (M+H)⁺

### Beispiel 129A

### 7-[(2,6-Difluorbenzyl)oxy]-2-methylpyrazolo[1,5-a]pyridin-3-carbonsäureethylester

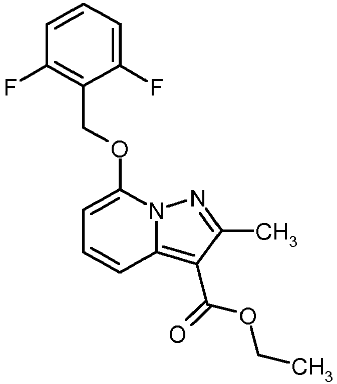

Eine bei Raumtemperatur gerührte Lösung von 540 mg (1.00 mmol, 81% rein) 1-Amino-2-[(2,6-difluorbenzyl)oxy]pyridinium-2,4,6-trimethylbenzolsulfonat aus Beispiel 128A in DMF (10 ml) wurde mit 0.35 ml (3.06 mmol) 2-Butinsäureethylester und 623 mg (4.51 mmol) Kaliumcarbonat versetzt. Die Reaktionsmischung wurde über Nacht bei Raumtemperatur gerührt und dann unter Rühren tropfenweise zu 20 ml Wasser gegeben. Die erhaltene Suspension wurde mit Essigsäureethylester (2 x 20 ml) extrahiert. Die vereinigten organischen Phasen wurden mit einer Phasentrennkartusche getrocknet und im Vakuum eingeengt. Der Rückstand wurde durch Flash-Chromatographie unter Verwendung einer vorgepackten Kieselgelkartusche (Laufmittel: Cyclohexan-Essigsäureethylester 10:1 bis 1:1) aufgereinigt, wodurch man 200 mg (25% d. Th., 43% rein) der Zielverbindung erhielt.
LC-MS (Methode 16): Rₜ = 1.23 min
MS (ESpos): m/z = 347.21 (M+H)⁺

### Beispiel 130A

### 7-[(2,6-Difluorbenzyl)oxy]-2-methylpyrazolo[1,5-a]pyridin-3-carbonsäure

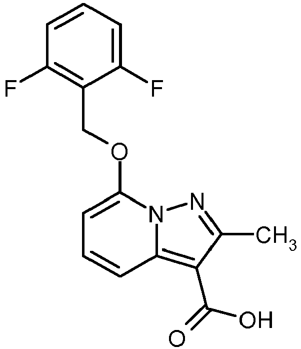

Eine Lösung von 200 mg (0.228 mmol, 43% rein) 7-[(2,6-Difluorbenzyl)oxy]-2-methylpyrazolo[1,5-a]pyridin-3-carbonsäureethylester aus Beispiel 129A in 3 ml Dioxan wurde mit 1 ml 2 N Natronlauge versetzt. Die erhaltene Mischung wurde 72 h bei Raumtemperatur gerührt. Das Lösungsmittel wurde im Vakuum abgezogen und der Rückstand wurde durch Flash-Chromatographie unter Verwendung einer vorgepackten Kieselgelkartusche (Laufmittel: Dichlormethan-Methanol 100:1 bis 10:1) aufgereinigt, wodurch man 100 mg (77% d. Th., 61% rein) der Zielverbindung erhielt.
LC-MS (Methode 16): Rₜ = 0.94 min
MS (ESpos): m/z = 319.15 (M+H)⁺

### Beispiel 131A

### rac-{1-[({7-[(2,6-Difluorbenzyl)oxy]-2-methylpyrazolo[1,5-a]pyridin-3-yl}carbonyl)amino]-2-methylpentan-2-yl}carbamidsäure-tert.-butylester

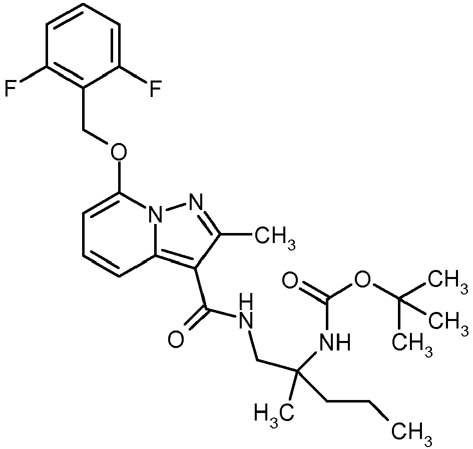

Eine Lösung von 100 mg (0.19 mmol, 61% rein) 7-[(2,6-Difluorbenzyl)oxy]-2-methylpyrazolo[1,5-a]pyridin-3-carbonsäure aus Beispiel 130A in 4 ml Tetrahydrofuran wurde mit 39.1 mg (0.287 mmol) 1-Hydroxybenzotriazol und 74.3 mg (0.287 mmol) 1-(3-Dimethylaminopropyl)-3-ethylcarbodiimidhydrochlorid versetzt. Die erhaltene Lösung wurde 15 min bei Raumtemperatur gerührt und dann mit 62.2 mg (0.287 mmol) *rac*-(1-Amino-2-methylpentan-2-yl)carbamidsäure-tert.-butylester aus Beispiel 152A gefolgt von 0.1 ml (0.575 mmol) N,N-Diisopropylethylamin versetzt. Nach der Zugabe wurde weitere 15 h bei Raumtemperatur gerührt. Das Lösungsmittel wurde im Vakuum abgezogen und der Rückstand wurde zwischen Dichlormethan (10 ml) und Wasser (10 ml) verteilt. Die organische Phase wurde abgetrennt und mit Wasser und gesättigter wässriger Natriumchloridlösung gewaschen, mit einer Phasentrennkartusche getrocknet und im Vakuum eingedampft, wodurch man 45 mg (24% d. Th., 53% rein) der Zielverbindung erhielt.
LC-MS (Methode 16): Rₜ = 1.32 min
MS (ESpos): m/z = 517.37 (M+H)⁺

### Beispiel 132A

### (2,6-Difluor-3-methoxyphenyl)methanol

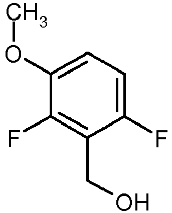

Eine bei 0°C gerührte Lösung von 600 mg (3.48 mmol) 2,6-Difluor-3-methoxybenzaldehyd (CAS 149949-30-4) in 8 ml Ethanol wurde portionsweise mit 65.9 mg (1.74 mmol) Natriumborhydrid versetzt. Nach 30 min Rühren bei 0°C wurde die Reaktionsmischung mit Wasser gequencht und dreimal mit Methyl-tert.-butylether extrahiert. Die vereinigten organischen Extrakte wurde mit Wasser und gesättigter wässriger Natriumchloridlösung gewaschen und mit einer Phasetrennkartusche getrocknet. Das Lösungsmittel wurde im Vakuum zur Trockne abgezogen, wodurch man 550 mg (89% d. Th.) der Zielverbindung erhielt.
¹H-NMR (300 MHz, DMSO-d₆): δ [ppm] = 3.80 (s, 3 H), 4.48 (d, 2 H), 5.23 (t, 1 H), 6.91 - 7.02 (m, 1 H), 7.03 - 7.14 (m, 1 H).

### Beispiel 133A

### 2-(Chlormethyl)-1,3-difluor-4-methoxybenzol

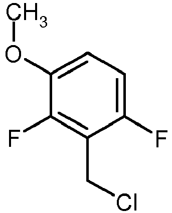

Eine Lösung von 120 mg (0.689 mmol) 2,6-Difluor-3-methoxyphenyl)methanol aus Beispiel 132A in 3 ml Dichlormethan und 1 ml Thionylchlorid wurde über Nacht bei Raumtemperatur gerührt. Das Lösungsmittel wurde im Vakuum abgedampft und der Rückstand wurde durch Flash-Chromatographie unter Verwendung einer vorgepackten Kieselgelkartusche (Laufmittel: Cyclohexan-Essigsäureethylester 10:1 bis 1:1) aufgereinigt, wodurch man 50 mg (38% d. Th.) der Zielverbindung erhielt.
¹H-NMR (300 MHz, DMSO-d₆): δ [ppm] = 3.82 (s, 3 H), 4.75 (s, 2 H), 7.06 - 7.13 (m, 1 H), 7.16 - 7.25 (m, 1 H).

### Beispiel 134A

### 2,4-Difluor-3-(hydroxymethyl)benzonitril

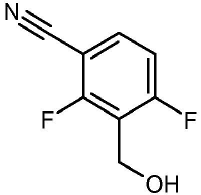

Eine bei 0°C gerührte Lösung von 300 mg (3.48 mmol) 2,4-Difluor-3-formylbenzonitril (CAS 149489-14-5) in 10 ml Ethanol wurde portionsweise mit 34 mg (0.89 mmol) Natriumborhydrid versetzt. Nach 1 h Rühren bei 0°C wurde die Reaktionsmischung mit Wasser gemischt und dreimal mit Methyl-tert.-butylether extrahiert. Die vereinigten organischen Extrakte wurden mit Wasser und gesättigter wässriger Natriumchloridlösung gewaschen und mit einer Phasetrennkartusche getrocknet. Das Lösungsmittel wurde im Vakuum zur Trockne abgezogen, wodurch man 280 mg (82% d. Th.) der Zielverbindung erhielt.
¹H-NMR (300 MHz, DMSO-d₆): δ [ppm] = 4.51 (d, 2 H), 5.45 (t, 1H), 7.34 (t, 1 H), 7.98 (dd, 1 H).

### Beispiel 135A

### 3-Chlormethyl-2,4-difluorbenzonitril

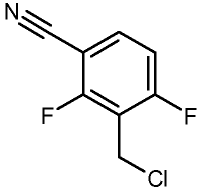

Eine Lösung von 120 mg (0.710 mmol) 2,4-Difluor-3-(hydroxymethyl)benzonitril aus Beispiel 134A in 3 ml Dichlormethan und 1 ml Thionylchlorid wurde 3 h bei Raumtemperatur gerührt. Das Lösungsmittel wurde im Vakuum abgedampft und der Rückstand wurde durch Flash-Chromatographie unter Verwendung einer vorgepackten Kieselgelkartusche (Laufmittel: Cyclohexan-Essigsäureethylester 10:1 bis 1:1) aufgereinigt, wodurch man 60 mg (45% d. Th.) der Zielverbindung erhielt.
¹H-NMR (300 MHz, DMSO-d₆): δ [ppm] = 4.82 (s, 2 H), 7.45 (t, 1 H), 8.10 (dd, 1 H).

### Beispiel 136A

### 7-[(2-Chlor-6-fluorbenzyl)oxy]-2,5-dimethylpyrazolo[1,5-a]pyridin-3-carbonsäureethylester

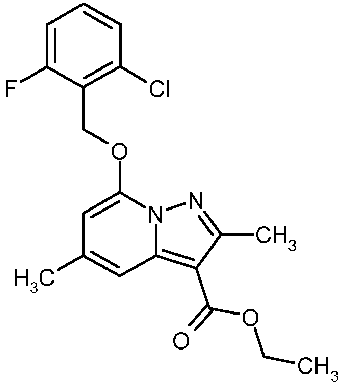

Eine Lösung von 180 mg (0.76 mmol) 7-Hydroxy-2,5-dimethylpyrazolo[1,5-a]pyridin-3-carbonsäureethylester (Beispiel 8A) in 7 ml Tetrahydrofuran wurde mit 247 mg (1.52 mmol) 2-Chlor-6-fluorbenzylalkohol, 419 mg (1.60 mmol) Triphenylphosphin und 0.32 ml (1.60 mmol) Azodicarbonsäurediisopropylester versetzt. Die Reaktionsmischung wurde 1 h bei Raumtemperatur gerührt und im Vakuum eingeengt. Der Rückstand wurde durch Flash-Chromatographie unter Verwendung einer vorgepackten Kieselgelkartusche (mobile Phase: Cyclohexan-Essigsäureethylester, Gradient 10% bis 30%) aufgereinigt, wodurch man 280 mg (84% Ausbeute, 84% rein) der Zielverbindung erhielt.
LC-MS (Methode 16): Rₜ = 1.34 min; m/z = 377.22 (M+H)⁺
¹H-NMR (500 MHz, CDCl₃) δ [ppm] = 1.42 (m, 3H), 2.46 (s, 3H), 2.67 (s, 3H), 4.38 (m, 2H), 5.52 (d, 2H), 6.26 (s, 1H), 7.08 (m, 1H), 7.29 (s, 1H), 7.35 (m, 1H), 7.57 (s, 1H).

### Beispiel 137A

### 7-(2-Chlor-6-fluorbenzyloxy)-2,5-dimethylpyrazolo[1,5-a]pyridin-3-carbonsäure

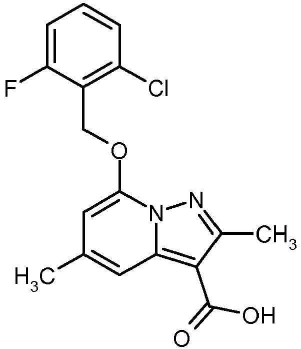

Eine Lösung von 198 mg (0.62 mmol) 7-(2-Chlor-6-fluorbenzyloxy)-2,5-dimethylpyrazolo[1,5-a]pyridin-3-carbonsäureethylester (Beispiel 136A) in 6.7 ml 1,4-Dioxan wurde mit 2 M Natronlauge (2.5 ml) versetzt. Es wurde weitere 18 Stunden lang bei 90°C gerührt. Die Reaktionsmischung wurde mit 0.8 ml Dimethylsulfoxid versetzt und weitere 6 Stunden lang bei 90°C gerührt. Die Reaktionsmischung wurde im Vakuum eingeengt. Der Rückstand wurde mit Acetonitril (5 ml) und Trifluoressigsäure (0.8 ml) und anschließend mit Wasser und Dichlormethan versetzt. Die organische Phase wurde abgetrennt und im Vakuum eingeengt. Der Rückstand wurde durch Flash-Chromatographie unter Verwendung einer vorgepackten Kieselgelkartusche (mobile Phase: Dichlormethan/Methanol, Gradient 80% bis 100%) aufgereinigt, wodurch man 105 mg (27% Ausbeute, 56% rein) der Zielverbindung erhielt, die ohne weitere Aufreinigung in den nächsten Schritt eingesetzt wurde.
LC-MS (Methode 16): Rₜ = 1.05 min; m/z = 349.15 (M+H)⁺

### Beispiel 138A

### rac-{1-[({7-[(2-chloro-6-fluorobenzyl)oxy]-2,5-dimethylpyrazolo[1,5-a]pyridin-3-yl}carbonyl)amino]-2-methylpentan-2-yl}carbamidsäure-tert.-butylester

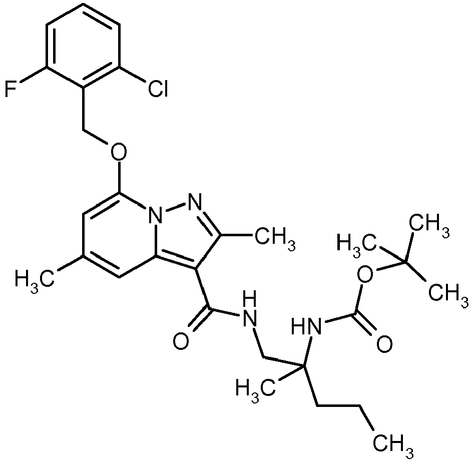

Eine Lösung von 26 mg (0.074 mmol) 7-(2-Chlor-6-fluorbenzyloxy)-2,5-dimethylpyrazolo[1,5-a]pyridin-3-carbonsäure (Beispiel 137A) in 3 ml Tetrahydrofuran wurde mit 17 mg (0.09 mmol) 1-(3-Dimethylaminopropyl)-3-ethylcarbodiimidhydrochlorid, 14 mg (0.09 mmol) 1-Hydroxybenzotriazol, 21 mg (0.096 mmol) *rac*-(1-Aminomethyl-1-methylbutyl)carbamidsäure-tert.-butylester aus Beispiel 152A und 0.039 ml (0.222 mmol) N,N-Diisopropylethylamin versetzt. Es wurde weitere 18 Stunden lang bei Raumtemperatur gerührt. Die Reaktionsmischung wurde dann 3 Stunden lang auf 60°C erhitzt. Nach dem Eindampfen im Vakuum wurde der Rückstand zwischen Dichlormethan und Wasser verteilt. Die organische Phase wurde abgetrennt und im Vakuum eingedampft, wodurch man 40 mg (99% Ausbeute) der Zielverbindung erhielt.
LC-MS (Methode 16): Rₜ = 1.42 min; m/z = 547.35 (M+H)⁺
¹H-NMR (500 MHz, CDCl₃): δ [ppm] = 0.94 (t, 3 H), 1.25 (s, 3 H), 1.33 - 1.41 (m, 2 H), 1.42 - 1.44 (m, 9H), 1.53 - 1.61 (m, 1 H), 1.68 - 1.78 (m, 1 H), 2.43 (s, 3 H), 2.69 (s, 3 H), 3.73 (qd, 2 H), 4.64 (br. s, 1 H), 5.51 (s, 2 H), 6.21 (s, 1 H), 6.90 (br. s., 1 H), 7.07 (t, 1 H), 7.27 - 7.29 (m, 1 H), 7.35 (td, 1 H), 7.65 (s, 1 H).
¹³C-NMR (126 MHz, CDCl₃): δ [ppm] = 14.5, 14.8, 16.8, 21.9, 23.8, 28.4, 40.4, 46.8, 56.0, 62.8, 79.5, 94.3, 103.9, 110.0, 114.4, 120.3, 125.7, 131.5, 136.9, 137.9, 143.4, 149.0, 151.1, 162.3, 165.1.

### Beispiel 139A

### 7-Benzyloxy-2,5-dimethylpyrazolo[1,5-a]pyridin-3-carbonsäure

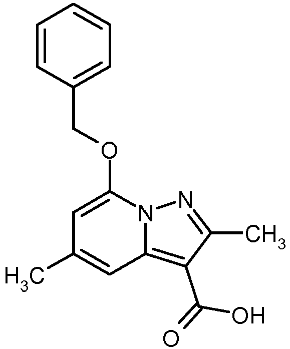

Eine Lösung von 390 mg (1.13 mmol) 7-Benzyloxy-2,5-dimethylpyrazolo[1,5-a]pyridin-3-carbonsäureethylester (Beispiel 7A) in 6 ml 1,4-Dioxan wurde mit 4 ml 2 M Natronlauge versetzt. Es wurde weitere 18 Stunden lang bei 100 °C gerührt. Die Reaktionsmischung wurde im Vakuum abgekühlt und dann im Vakuum eingeengt. Der Rückstand wurde mit Acetonitril (10 ml) und Wasser (2 ml) versetzt, gefolgt von 2 ml Trifluoressigsäure, die tropfenweise zugesetzt wurden. Der erhaltene Niederschlag wurde abfiltriert und im Vakuum getrocknet, wodurch man 262 mg (72% Ausbeute, 92% rein) der Zielverbindung erhielt.
LC-MS (Methode 15): Rₜ = 0.65 min; m/z = 297.21 (M+H)⁺
¹H NMR (300 MHz, DMSO-d₆) δ [ppm] = 2.40 (s, 3H), 2.50 (s, 3H), 5.41 (s, 2H), 6.56 (d, 1H), 7.37 - 7.56 (m, 6H), 12.21 (br.s, 1H).

### Beispiel 140A

### rac-[1-({[7-(Benzyloxy)-2,5-dimethylpyrazolo[1,5-a]pyridin-3-yl]carbonyl}amino)-2-methylpentan-2-yl]carbamidsäure-tert.-butylester

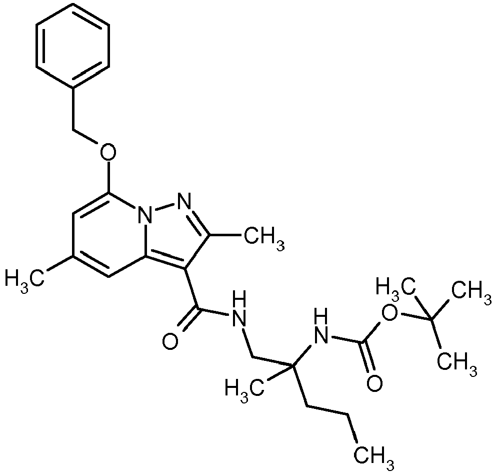

Eine Lösung von 240 mg (0.81 mmol) 7-Benzyloxy-2,5-dimethylpyrazolo[1,5-a]pyridin-3-carbonsäure (Beispiel 139A) in 12 ml Tetrahydrofuran wurde mit 132 mg (0.97 mmol) 1-Hydroxy-7-azabenzotriazol und 186 mg (0.97 mmol) 1-(3-Dimethylaminopropyl)-3-ethylcarbodiimidhydrochlorid versetzt. Die Reaktionsmischung wurde 10 Minuten lang bei Raumtemperatur gerührt. Die erhaltene Mischung wurde mit 0.422 ml (2.43 mmol) N,N-Diisopropylethylamin und 210 mg (0.97 mmol) *rac*-(1-Aminomethyl-1-methylbutyl)carbamidsäure-tert.-butylester aus Beispiel 152A versetzt. Es wurde weitere 18 Stunden lang bei Raumtemperatur gerührt, und der Inhalt wurde dann im Vakuum eingeengt. Der Rückstand wurde zwischen Dichlormethan und Wasser verteilt. Die organische Phase wurde abgetrennt und mit Wasser und wässrige Natriumchlorid-Lösung gewaschen, über Natriumsulfat getrocknet, filtriert und im Vakuum eingedampft. Der Rückstand wurde durch Flash-Chromatographie unter Verwendung einer vorgepackten Kieselgelkartusche (mobile Phase: Cyclohexan/Essigsäureethylester, Gradient 0% bis 50%) aufgereinigt, wodurch man 370 mg (65% Ausbeute, 71% rein) der Zielverbindung erhielt, die ohne weitere Aufreinigung in den nächsten Schritt eingesetzt wurde.
LC-MS (Methode 15): Rₜ = 1.36 min; m/z = 495.39 (M+H)⁺

### Beispiel 141A

### rac-(1-{[(7-hydroxy-2,5-dimethylpyrazolo[1,5-a]pyridin-3-yl)carbonyl]amino}-2-methylpentan-2-yl)carbamidsäure-tert.-butylester

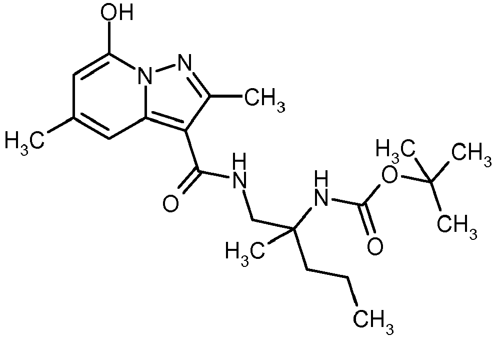

Eine Lösung von 207 mg (0.418 mmol) *rac*-[1-{[7-Benzyloxy-2,5-dimethylpyrazolo[1,5-a]pyridin-3-carbonyl]amino}methyl}-1-methylbutyl)carbamidsäure-tert.-butylester (Beispiel 140A) in 6 ml absolutem Ethanol wurde mit 47 mg Palladium auf Kohle (10 Gew.-% Beladung aktiviert) und 1.3 ml Cyclohexen versetzt. Die Reaktionsmischung wurde unter einer Argonatmosphäre 2.5 Stunden lang auf Rückfluss erhitzt. Die erhaltene Mischung wurde auf Raumtemperatur abgekühlt, über eine Schicht Celite filtriert und im Vakuum eingeengt, wodurch man 206 mg (99% Ausbeute, 81 % rein) der Zielverbindung erhielt.
LC-MS (Methode 15): Rₜ = 0.84 min; m/z = 405.35 (M+H)⁺

### Beispiel 142A

### rac-[1-[({2,5-Dimethyl-7-[4,4,4-trifluor-3-(trifluormethyl)butoxy]pyrazolo[1,5-a]pyridin-3-yl}carbonyl)amino]-2-methylpentan-2-yl}carbamidsäure-tert.-butylester

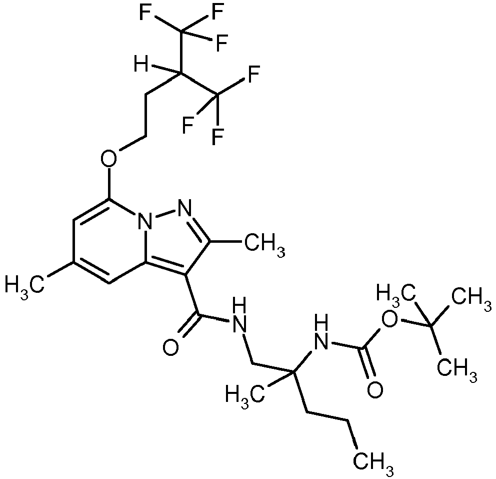

Eine Lösung von 40 mg (0.10 mmol) *rac*-[1-{[7-Hydroxy-2,5-dimethylpyrazolo[1,5-a]pyridin-3-carbonyl)amino]}-1-methylbutyl)carbamidsäure-tert.-butylester (Beispiel 141A) in 1 ml DMF wurde mit 65 mg (0.2 mmol) Cäsiumcarbonat und 78 mg (0.3 mmol) 4-Brom-1,1,1-trifluor-2-(trifluormethyl)butan (CAS: 203303-02-0) versetzt. Die Reaktionsmischung wurde 18 Stunden lang bei Raumtemperatur gerührt. Das Lösungsmittel wurde im Vakuum abgedampft. Der Rückstand wurde zwischen Dichlormethan und Wasser verteilt, die Phasen wurden getrennt und die organische Phase wurde im vakuum eingedampft, wodurch man 55 mg (51% Ausbeute, 54% rein) der Zielverbindung erhielt, die ohne weitere Aufreinigung in den nächsten Schritt eingesetzt wurde.
LC-MS (Methode 15): Rₜ = 1.40 min; m/z = 583.37 (M+H)⁺

### Beispiel 143A

### 2-Brommethyl-3-fluorpyridin

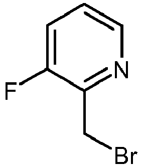

Eine Lösung von 285 mg (2.242 mmol) (3-Fluorpyridin-2-yl)methanol (CAS: 31181-79-0) in trockenem Dichlormethan (9 ml) wurde unter einer Argonatmosphäre in einem Eiswasserbad mit 882 mg (3.36 mmol) Triphenylphosphin und 1.12 g (3.36 mmol) Tetrabrommethan versetzt. Die Reaktionsmischung wurde 22 Stunden lang bei Raumtemperatur gerührt. Das Lösungsmittel wurde abgedampft und der Rückstand wurde durch Flash-Chromatographie unter Verwendung einer vorgepackten Kieselgelkartusche (mobile Phase: Cyclohexan-Essigsäureethylester, Gradient 15% bis 30%) aufgereinigt, wodurch man 156 mg (11% Ausbeute, 98% rein) der Zielverbindung erhielt.
LC-MS (Methode 16): Rₜ = 0.79 min; m/z = 192.01 (M+H)⁺

### Beispiel 144A

### rac-{1-[({7-[(3-Fluorpyridin-2-yl)methoxy]-2,5-dimethylpyrazolo[1,5-a]pyridin-3-yl}carbonyl)amino]-2-methylpentan-2-yl}carbamidsäure-tert.-butylester

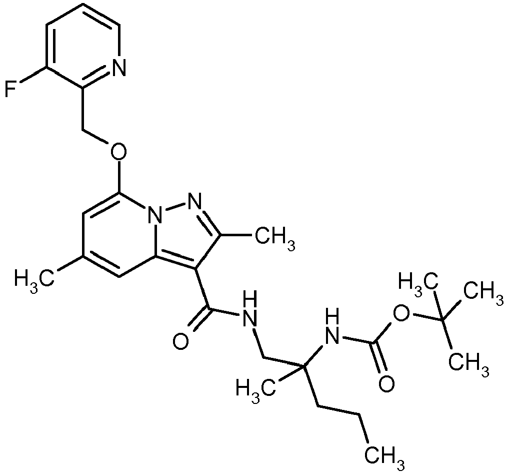

Eine Lösung von 49 mg (0.12 mmol) *rac*-[1-{[7-Hydroxy-2,5-dimethylpyrazolo[1,5-a]pyridin-3-carbonyl)amino]}-1-methylbutyl)carbamidsäure-tert.-butylester (Beispiel 141A) in 2.1 ml DMF wurde mit 78 mg (0.24 mmol) Cäsiumcarbonat und 69 mg (0.360 mmol) 2-Brommethyl-3-fluorpyridin (Beispiel 143A) versetzt. Die Reaktionsmischung wurde 18 Stunden lang bei Raumtemperatur gerührt. Das Lösungsmittel wurde im Vakuum abgedampft und der Rückstand wurde zwischen Dichlormethan und Wasser verteilt. Die organische Phase wurde abgetrennt und im Vakuum eingedampft. Der Rückstand wurde durch präparative HPLC-Chromatographie (Methode 21) aufgereinigt, wodurch man 21 mg (33%Ausbeute, 96% rein) der Zielverbindung erhielt.
LC-MS (Methode 15): Rₜ = 1.22 min; m/z = 514.34 (M+H)⁺

### Beispiel 145A

### 4-Brom-2-[(2,6-difluorbenzyl)oxy]pyridin

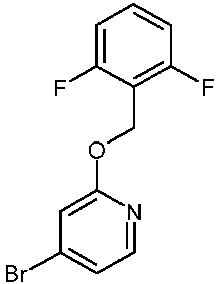

15.5 ml (15.5 mmol) einer 1 M Lösung von Kalium-tert.-butanolat in Tetrahydrofuran wurde bei 0°C tropfenweise zu einer Lösung von 1.72 ml (15.5 mmol) (2,6-Difluorphenyl)methanol und 1.6 ml (15.5 mmol) 4-Brom-2-fluorpyridin in 50 ml wasserfreiem Tetrahydrofuran gegeben. Die Reaktionsmischung wurde 18 h bei Raumtemperatur gerührt. Es wurde zwischen Essigsäureethylester und Wasser verteilt. Die organische Phase wurde abgetrennt und mit Wasser und gesättigter wässriger Natriumchloridlösung gewaschen, über Natriumsulfat getrocknet, filtriert und eingeengt. Der Rückstand wurde durch Chromatographie an Kieselgel (40 g Kieselgelkartusche, mobile Phase: Cyclohexan/Essigsäureethylester, Gradient 0% bis 100%) aufgereinigt. Hierdurch erhielt man 4.12 g der Zielverbindung (88% d. Th.).
LC-MS (Methode 19): Rₜ = 4.17 min; m/z = 300 und 302 (M+H)⁺

### Beispiel 146A

### 4-Cyclopropyl-2-[(2,6-difluorbenzyl)oxy]pyridin

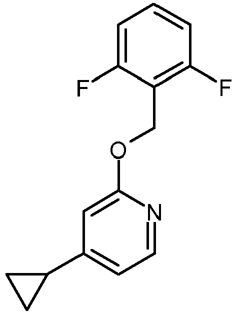

Eine Mischung von 500 mg (1.66 mmol) 4-Brom-2-[(2,6-difluorbenzyl)oxy]pyridin (Beispiel 145A), 365 µl (2.0 mmol) 2-Cyclopropyl-4,4,5,5-tetramethyl-1,3,2-dioxaborolan, 139 mg (0.17 mmol) 1,1'-Bis(diphenylphosphino)ferrocenpalladium(II)-dichlorid-Dichlormethan-Komplex und 1.63 g (5.0 mmol) Caesiumcarbonat in 0.5 ml Wasser und 4 ml Dioxan wurde 5 min mit Argon entgast und in einem verschlossenen Röhrchen 2 h bei 100°C gerührt. Die Reaktionsmischung wurde auf Raumtemperatur abgekühlt und der Rückstand wurde zwischen Essigsäureethylester und Wasser verteilt. Die organische Phase wurde abgetrennt und mit gesättigter wässriger Natriumchloridlösung gewaschen, über Natriumsulfat getrocknet, filtriert und eingeengt. Der Rückstand wurde durch Chromatographie an Kieselgel (40 g Kieselgelkartusche, mobile Phase: Methanol/Essigsäureethylester, Gradient 0% bis 25%) aufgereinigt. Hierdurch erhielt man 350 mg der Zielverbindung (80% d. Th.).
LC-MS (Methode 19): Rₜ = 3.93 min; m/z = 262 (M+H)⁺

### Beispiel 147A

### 1-Amino-4-cyclopropyl-2-[(2,6-difluorbenzyl)oxy]pyridinium-2,4,6-trimethylbenzolsulfonat

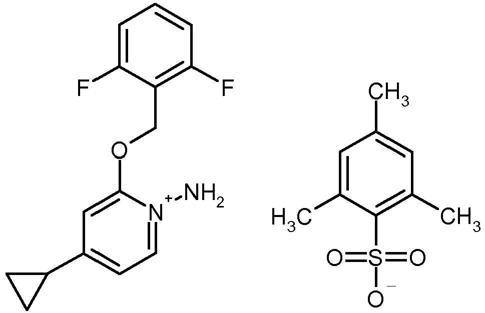

767 mg (2.7 mmol) O-(2-Mesitylensulfonyl)acethydroxamsäureethylester wurden portionsweise zu einer auf -5°C vorgekühlten Mischung von 1.4 ml Trifluoressigsäure und 0.2 ml Wasser gegeben. Die erhaltene Mischung wurde 1.5 h bei -5°C gerührt und mit 10 ml Eis/Wasser verdünnt. Die Mischung wurde mit 4 ml Dichlormethan extrahiert. Die organische Phase wurde über gesättigter wässriger Kaliumcarbonatlösung getrocknet, filtriert und zu einer auf 5°C vorgekühlten Lösung von 470 mg (1.8 mmol) 4-Cyclopropyl-2-[(2,6-difluorbenzyl)oxy]pyridin (Beispiel 146A) in 4 ml Dichlormethan gegeben. Die Mischung wurde 18 h bei Raumtemperatur gerührt und eingeengt. Der Rückstand wurde in 100 ml Diethylether gegossen, abfiltriert, mit Diethylether gewaschen und über Nacht getrocknet. Hierdurch erhielt man 543 mg der Zielverbindung (63% d. Th.).
LC-MS (Methode 19): Rₜ = 0.31, 1.85 und 2.00 min; m/z = 277 (M+H)⁺

### Beispiel 148A

### 5-Cyclopropyl-7-[(2,6-difluorbenzyl)oxy]-2-methylpyrazolo[1,5-a]pyridin-3-carbonsäureethylester

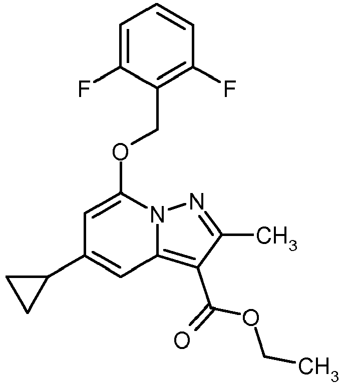

468 mg (3.4 mmol) Kaliumcarbonat wurden zu einer Mischung von 264 µl (2.3 mmol) But-2-insäureethylester und 540 mg (1.1 mmol) 1-Amino-4-cyclopropyl-2-[(2,6-difluorbenzyl)oxy]pyridinium-2,4,6-trimethylbenzolsulfonat (Beispiel 147A) in 10 ml DMF gegeben. Die Reaktionsmischung wurde 2 h bei Raumtemperatur gerührt und dann in 25 ml auf 5°C vorgekühltes Wasser gegossen. Der Niederschlag wurde abfiltriert, mit Wasser gewaschen und getrocknet.

Hierdurch erhielt man 138 mg der Zielverbindung (31% d. Th.).
LC-MS (Methode 19): Rt = 3.87 min; m/z = 387 (M+H)⁺

Das Filtrat wurde mit Essigsäureethylester extrahiert (x 2). Die vereinigten organischen Extrakte wurden mit Wasser und gesättigter wässriger Natriumchloridlösung gewaschen, über Natriumsulfat getrocknet, filtriert und eingeengt. Der Rückstand wurde durch Chromatographie an Kieselgel (40 g Kieselgelkartusche, mobile Phase: Methanol/Essigsäureethylester, Gradient 0% bis 25%) aufgereinigt. Hierdurch erhielt man weitere 48 mg der Zielverbindung (11% d. Th.).
LC-MS (Methode 19): Rₜ = 3.87 min; m/z = 387 (M+H)⁺

### Beispiel 149A

### 5-Cyclopropyl-7-[(2,6-difluorbenzyl)oxy]-2-methylpyrazolo[1,5-a]pyridin-3-carbonsäure

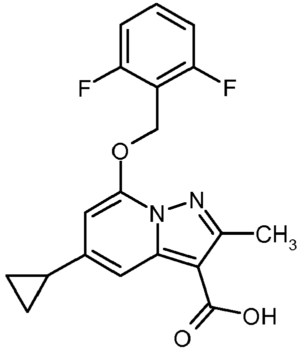

1.8 ml (1.8 mmol) einer 1 M wässrigen Lösung von Natriumhydroxid wurden zu einer Lösung von 180 mg (0.47 mmol) 5-Cyclopropyl-7-(2,6-difluorbenzyloxy)-2-methylpyrazolo[1,5-a]pyridin-3-carbonsäureethylester (Beispiel 148A) in 18 ml Methanol gegeben. Die Reaktion wurde 18 h bei Rückfluss gerührt und im Vakuum eingeengt. Der Rückstand wurde in 4 ml Tetrahydrofuran aufgenommen und mit 70 mg (0.55 mmol) Kaliumtrimethylsilanolat versetzt. Die Reaktionsmischung wurde 18 h bei 65°C gerührt und im Vakuum eingeengt, wodurch man die Zielverbindung erhielt, die direkt in den nächsten Schritt eingesetzt wurde.
LC-MS (Methode 19): Rₜ = 3.33 min; m/z = 359 (M+H)⁺

### Beispiel 150A

### rac-{1-[({5-Cyclopropyl-7-[(2,6-difluorbenzyl)oxy]-2-methylpyrazolo[1,5-a]pyridin-3-yl}carbonyl)amino]-2-methylpentan-2-yl}carbamidsäure-tert.-butylester

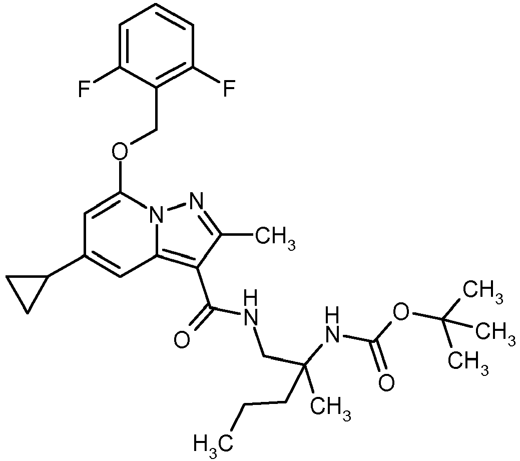

76 mg (0.6 mmol) 1-Hydroxy-7-azabenzotriazol und 107 mg (0.6 mmol) 1-(3-Dimethylaminopropyl)-3-ethylcarbodiimidhydrochlorid wurden zu einer Lösung von 167 mg (0.5 mmol) 5-Cyclopropyl-7-[(2,6-difluorbenzyl)oxy]-2-methylpyrazolo[1,5-a]pyridin-3-carbonsäure (Beispiel 149A), 244 µl (1.4 mmol) N,N-Diisopropylethylamin und 121 mg (0.6 mmol) *rac*-(1-Amino-2-methylpentan-2-yl)carbamidsäure-tert.-butylester aus Beispiel 152A in 5 ml Tetrahydrofuran gegeben. Die Mischung wurde 18 h bei Raumtemperatur gerührt und im Vakuum eingeengt. Der Rückstand wurde zwischen Essigsäureethylester und Wasser verteilt. Die organische Phase wurde abgetrennt und mit Wasser und gesättigter wässriger Natriumchloridlösung gewaschen, über Natriumsulfat getrocknet, filtriert und eingeengt. Der Rückstand wurde durch Chromatographie an Kieselgel (40 g Kieselgelkartusche, mobile Phase: Cyclohexan/Essigsäureethylester, Gradient 0% bis 100%) aufgereinigt. Hierdurch erhielt man 125 mg der Zielverbindung (48 % d. Th.).
LC-MS (Methode 26): Rₜ = 4.31 min; m/z = 557 (M+H)⁺
¹H-NMR (400 MHz, DMSO-d₆): δ [ppm] = 0.80 - 0.86 (m, 2H), 0.94 (dd, 3H), 1.04 (ddd, 2H), 1.25 (s, 3H), 1.30 - 1.40 (m, 2H), 1.42 (s, 9H), 1.56 (ddd, 1H), 1.73 (ddd, 1H), 1.91 - 1.99 (m, 1H), 2.68 (s, 3H), 3.65 - 3.79 (m, 2H), 4.63 (s, 1H), 5.46 (s, 2H), 6.06 (d, 1H), 6.95 (dd, 2H), 7.37 (tdd, 1H), 7.56 (d, 1H).

### Beispiel 151A

### rac-tert-Butyl-(2-cyanpentan-2-yl)carbamat-2-methylpropan-2-ol

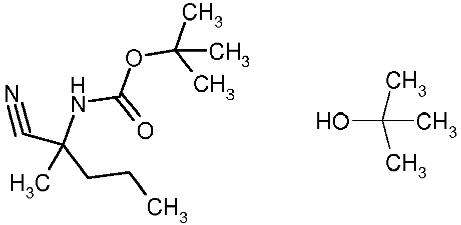

64.21 g (294.18 mmol) Di-tert-butyldicarbonat wurden in einem Rundkolben vorgelegt und bei RT sehr langssam mit 30 g (267.44 mmol) *rac*-2-Amino-2-methylpentannitril versetzt (Innenthermometer, Temperatur maximal bei 30°C) und das Gemisch wurde über Nacht bei Raumtemperatur nachgerührt. Die Reaktionsmischung wurde mit Dichlormethan versetzt und zweimal mit 1 N Natronlauge gewaschen. Die organische Phase wurde über Natriumsulfat getrocknet, filtriert und eingedampft (bei 30°C Badtemperatur). Es wurden 76.33 g (quantitative Ausbeute) der Zielverbindung erhalten.

### Beispiel 152A

### rac-tert-Butyl-(1-amino-2-methylpentan-2-yl)carbamat

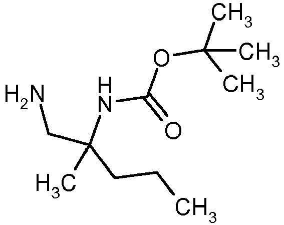

61.0 g (212.98 mmol) *rac*-tert-Butyl-(2-cyanpentan-2-yl)carbamat-2-methylpropan-2-ol aus Beispiel 151A wurden in 618 ml 7 N ammoniakalisches Methanol gelöst und unter Argon mit 66 g Raney-Nickel (50%ige wässrige Aufschlämmung) versetzt. Anschließend wurde das Reaktionsgemisch über Nacht im Autoklaven bei 20 - 30 bar hydriert. Das Reaktionsgemisch wurde über Celtite ab filtriert, gut mit Methanol gespült und eingedampft. Es wurden 43.70 g (95% d. Th.) der Zielverbindung erhalten.
LC-MS (Methode 7): Rₜ = 1.95 min
MS (ESpos): m/z = 217 (M+H)⁺

### Beispiel 153A

### ent-Benzyl-{1-[({2,5-dimethyl-7-[(2,3,6-trifluorbenzyl)oxy]pyrazolo[1,5-a]pyridin-3-yl}carbonyl)amino]-5,5,5-trifluor-2-methylpentan-2-yl}carbamat Trifluoroacetat (Enantiomer B)

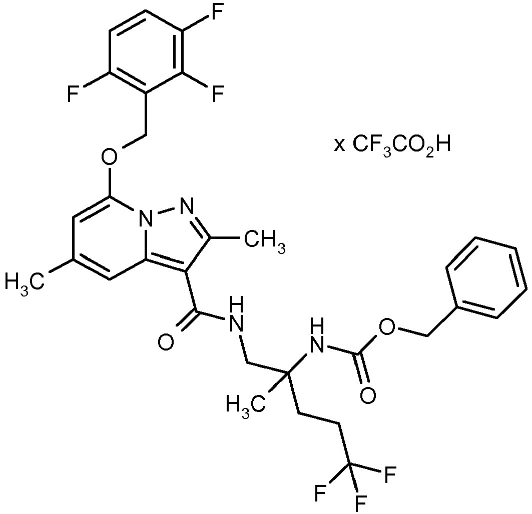

100 mg (0.25 mmol; ca. 87%ige Reinheit) 2,5-Dimethyl-7-[(2,3,6-trifluorbenzyl)oxy]pyrazolo[1,5-a]pyridin-3-carbonsäure aus Beispiel 10A, 103 mg (0.32 mmol; Reinheit ca. 95%) *ent*-Benzyl-(1-amino-5,5,5-trifluor-2-methylpentan-2-yl)carbamat (Enantiomer B) aus Beispiel 65A und 123 mg (0.32 mmol) HATU wurden in 0.8 ml DMF vorgelegt und 10 min bei RT gerührt. Anschließend wurden 0.22 ml (0.11 mmol) N,N-Diisopropylethylamin hinzugegeben und das Gemisch wurde 1 h bei RT gerührt. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 61 mg (25% d. Th.; Reinheit 75%) der Titelverbindung erhalten.
LC-MS (Methode 2): Rₜ = 1.23 min
MS (ESpos): m/z = 637 (M-TFA+H)⁺

### Beispiel 154A

### 4-Methyl-2-[(2,3,6-trifluorbenzyl)oxy]pyridin

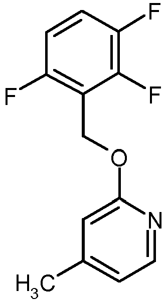

Eine Mischung von 8.32 g (36.96 mmol) 2-(Brommethyl)-1,3,4-trifluorbenzol und 4.84 g (44.35 mmol) 2-Hydroxy-4-methylpyridin [CAS-Nr: 13466-41-6] wurde in 227 ml THF gelöst. Die Lösung wurde mit 12.23 g (44.35 mmol) Silbercarbonat versetzt und unter Lichtausschluss über Nacht auf Rückfluss erhitzt. Anschließend wurde die Reaktionsmischung über Kieselgur filtriert, mit Essigsäureethylester gewaschen und das Filtrat wurde eingeengt. Das Rohprodukt wurde mittels Kieselgelchromatographie gereinigt (Laufmittel: Cyclohexan/Dichlormethan-Gradient: 10/0 nach 1/1). Es wurden 1.45 g der Titelverbindung (15% d. Th.) erhalten.
LC-MS (Methode 22): Rₜ = 1.44 min
MS (ESpos): m/z = 254 (M+H)⁺
¹H-NMR (400 MHz, DMSO-d₆): δ [ppm] = 2.27 (s, 3H), 5.38 (s, 2H), 6.67 (s, 1H), 6.87 (d, 1H), 7.16 - 7.25 (m, 1H), 7.51 - 7.62 (m, 1H), 8.05 (d, 1H).

### Beispiel 155A

### 1-Amino-4-methyl-2-[(2,3,6-trifluorbenzyl)oxy]pyridinium-2,4,6-trimethylbenzolsulfonat

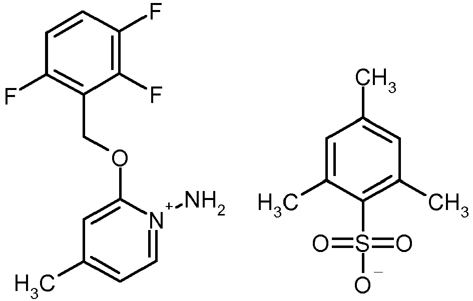

1) Eine Mischung von 5.8 ml (75.82 mmol) Trifluoressigsäure und 0.59 ml Wasser wurden auf - 5°C gekühlt. Bei dieser Temperatur wurden 3.25 g (11.37 mmol) Ethyl-(1E)-N-[(mesitylsulfonyl)oxy-]ethanimidoat [CAS-Nr: 38202-27-6] portionsweise zugegeben. Nach 1.5 h wurde das Gemisch auf 51 ml Eiswasser gegeben und mit 41 ml Dichlormethan extrahiert. Die organische Phase wurde über Natriumsulfat getrocknet und über eine Fritte wurde das Natriumsulfat abfiltriert. Die resultierende Lösung von *O*-(2-Mesitylenesulfonyl)hydroxylamin (MSH) wurde direkt zu einer Lösung von 1.92 g (7.58 mmol) 4-Methyl-2-[(2,3,6-trifluorbenzyl)oxy]pyridin aus Beispiel 154A in 5.1 ml Dichlormethan bei 0°C zugetropft. Die Mischung wurde 2 h bei RT gerührt.
2) Es wurde nochmals eine Mischung von 5.8 ml (75.82 mmol) Trifluoressigsäure und 0.59 ml Wasser auf -5°C abgekühlt. Bei dieser Temperatur wurden 3.25 g (11.37 mmol) Ethyl-(1E)-N-[(mesitylsulfonyl)oxy-]ethanimidoat [CAS-Nr: 38202-27-6] portionsweise zugegeben. Nach 1.5 h wurde das Gemisch auf 51 ml Eiswasser gegeben und mit 41 ml Dichlormethan extrahiert. Die organische Phase wurde über Natriumsulfat getrocknet und über eine Fritte wurde das Natriumsulfat abfiltriert. Die resultierende Lösung von *O*-(2-Mesitylenesulfonyl)hydroxylamin (MSH) wurde direkt zu der Reaktionslösung (beschrieben unter 1)) zugetropft.

Das Reaktionsgemisch wurde über Nacht bei RT weitergerührt. Anschließend wurden 250 ml Diethylether zugetropft, der anfallende Niederschlag abfiltriert, mit Diethylether gewaschen und getrocknet. Es wurden 2.5 g der Titelverbindung isoliert (69% d. Th., Reinheit 98%).
LC-MS (Methode 3): Rₜ = 1.28 und 1.38 min (Doppelpeak)
MS (ESpos): m/z (peak bei 1.28 min) = 269 (M)⁺ des Kations
¹H-NMR (400 MHz, DMSO-d₆): δ [ppm] = 2.17 (s, 3H), 2.46 - 2.57 (s, 3H und s, 6H überlagert mit den Lösungsmittelsignal), 5.68 (s, 2H), 6.74 (s, 2H), 7.29 - 7.36 (m, 1H), 7.37 - 7.44 (m, 3H), 7.67 - 7.78 (m, 1H), 7.85 (s, 1H), 8.44 (d, 1H).

### Beispiel 156A

### Methyl-2-cyclopropyl-5-methyl-7-[(2,3,6-trifluorbenzyl)oxy]pyrazolo[1,5-a]pyridin-3-carboxylat

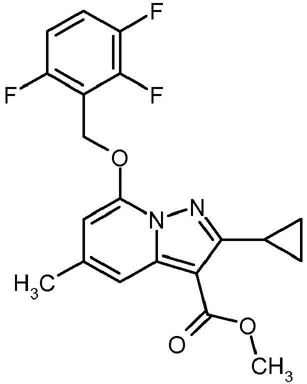

1.45 g (3.00 mmol, Reinheit 98%) 1-Amino-4-methyl-2-[(2,3,6-trifluorbenzyl)oxy]pyridinium-2,4,6-trimethylbenzolsulfonat aus Beispiel 155A wurden in 9.7 ml DMF gelöst und mit 670 mg (5.40 mmol) Methyl-3-cyclopropylprop-2-inoat versetzt. Es wurden 747 mg (5.40 mmol) Kaliumcarbonat zugegeben und die Mischung wurde für 3 h bei RT gerührt. Anschließend wurde das Gemisch auf 73 ml Wasser gegossen, kurz ausgerührt, der ausgefallene Feststoff abfiltriert, mit Wasser gewaschen und getrocknet. Es wurden 462 mg der Titelverbindung (33% d. Th.; Reinheit 85%) erhalten.
LC-MS (Methode 7): Rₜ = 2.90 min
MS (ESpos): m/z = 391 (M+H)⁺

### Beispiel 157A

### 2-Cyclopropyl-5-methyl-7-[(2,3,6-trifluorbenzyl)oxy]pyrazolo[1,5-a]pyridin-3-carbonsäure

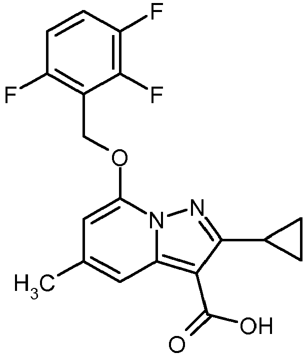

Eine Lösung von 462 mg (1.01 mmol; Reinheit 85%) Methyl-2-cyclopropyl-5-methyl-7-[(2,3,6-trifluorbenzyl)oxy]pyrazolo[1,5-a]pyridin-3-carboxylat aus Beispiel 156A in 10.5 ml Dioxan und 3 ml DMSO wurde mit 5.55 ml (11.10 mmol) 2 N Natronlauge versetzt und das Gemisch wurde 6.5 h bei 100°C gerührt. Es wurde nochmals 1 ml (2 mmol) 2 N Natronlauge hinzugegeben und das Gemisch wurde für weitere 2.5 h bei 100°C gerührt. Die Reaktionslösung wurde auf 0°C abgekühlt und mit 1 N Salzsäure auf pH 2 gestellt. Der dabei ausgefallene Feststoff wurde 30 min gerührt, abfiltriert und im Hochvakuum getrocknet. Es wurden 300 mg der Titelverbindung (74% d. Th.; Reinheit 93%) erhalten und ohne weitere Reinigung umgesetzt.
LC-MS (Methode 2): Rₜ = 1.00 min
MS (ESpos): m/z = 377 (M+H)⁺
¹H-NMR (400 MHz, DMSO-d₆): δ [ppm] = 0.87 - 0.99 (m, 4H), 2.42 (s, 3H), 2.74 - 2.82 (m, 1H), 5.50 (s, 2H), 6.62 (s, 1H), 7.25 - 7.33 (m, 1H), 7.50 (s, 1H), 7.62 - 7.73 (m, 1H), 12.29 (br. s, 1H).

### Beispiel 158A

### ent-Benzyl-{1-[({2-cyclopropyl-5-methyl-7-[(2,3,6-trifluorbenzyl)oxy]pyrazolo[1,5-a]pyridin-3-yl}carbonyl)amino]-2-methylpentan-2-yl}carbamat Trifluoroacetat (Enantiomer B)

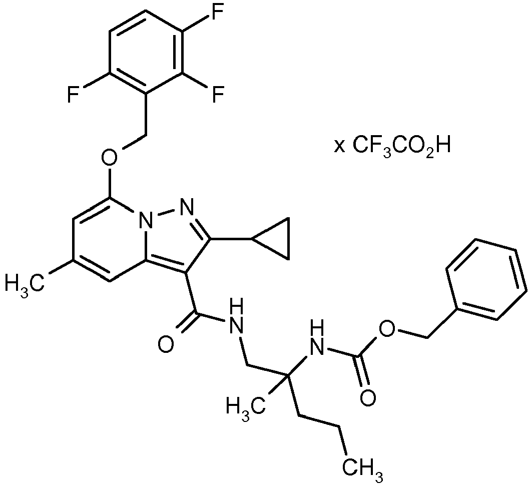

90 mg (0.22 mmol; Reinheit 93%) 2-Cyclopropyl-5-methyl-7-[(2,3,6-trifluorbenzyl)oxy]pyrazolo[1,5-a]pyridin-3-carbonsäure aus Beispiel 157A, 110 mg (0.29 mmol) HATU und 0.19 ml (1.11 mmol) N,N-Diisopropylethylamin wurden in 0.74 ml DMF vorgelegt und 10 min bei RT gerührt. Anschließend wurden 82 mg (0.29 mmol; Reinheit ca. 88%) *ent*-Benzyl-(1-amino-2-methylpentan-2-yl)carbamat (Enantiomer B) aus Beispiel 20A hinzugegeben und das Gemisch wurde 1 h bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 82 mg (51% d. Th.) der Titelverbindung erhalten.
LC-MS (Methode 2): Rₜ = 1.30 min
MS (ESpos): m/z = 609 (M-TFA+H)⁺

### Beispiel 159A

### ent-Benzyl-{1-[({2-cyclopropyl-5-methyl-7-[(2,3,6-trifluorbenzyl)oxy]pyrazolo[1,5-a]pyridin-3-yl}carbonyl)amino]-5,5,5-trifluor-2-methylpentan-2-yl}carbamat Trifluoroacetat (Enantiomer B)

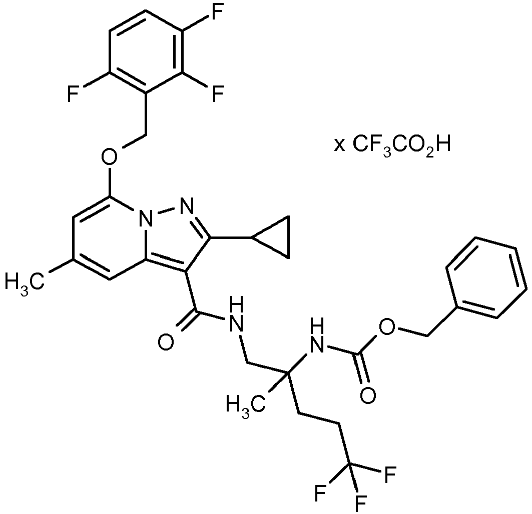

90 mg (0.22 mmol; Reinheit 93%) 2-Cyclopropyl-5-methyl-7-[(2,3,6-trifluorbenzyl)oxy]pyrazolo[1,5-a]pyridin-3-carbonsäure aus Beispiel 157A, 110 mg (0.29 mmol) HATU und 0.19 ml (1.11 mmol) N,N-Diisopropylethylamin wurden in 0.74 ml DMF vorgelegt und 10 min bei RT gerührt. Anschließend wurden 93 mg (0.29 mmol; Reinheit ca. 95%) *ent*-Benzyl-(1-amino-5,5,5-trifluor-2-methylpentan-2-yl)carbamat (Enantiomer B) aus Beispiel 65A hinzugegeben und das Gemisch wurde 1 h bei RT gerührt. Die Reaktionslösung wurde mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden am Rotationsverdampfer eingedampft. Es wurden 79 mg (45% d. Th.) der Titelverbindung erhalten.
LC-MS (Methode 2): Rₜ = 1.29 min
MS (ESpos): m/z = 663 (M-TFA+H)⁺

### Beispiel 160A

### 2-[(2,6-Difluorbenzyl)oxy]-4-methoxypyridin

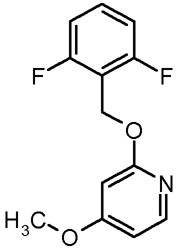

Eine zuvor auf 0°C abgekühlte Lösung von 3.5 g (27.53 mmol) 2-Fluor-4-methoxypyridin (CAS: 175965-83-0) und 3.97 g (27.53 mmol) 2,6-Difluorbenzylalkohol in 97 ml trockenem Tetrahydrofuran wurde tropfenweise mit 27.5 ml (27.53 mmol) einer 1M Lösung von Kalium-tert.-butanolat in Tetrahydrofuran versetzt. Die erhaltene Mischung wurde 18 Stunden lang bei Raumtemperatur gerührt. Das Lösungsmittel wurde abgedampft und der Rückstand wurde zwischen Wasser und Essigsäureethylester verteilt. Die organische Phase wurde abgetrennt, mit Wasser und wässriger Natriumchlorid-Lösung gewaschen, über Natriumsulfat getrocknet, filtriert und im Vakuum eingedampft, wodurch man 5.77 g (Ausbeute 66%, Reinheit 79%) des Produkts erhielt.
LC-MS (Methode 15): Rₜ = 1.17 min; m/z = 252.14 (M+H)⁺
¹H-NMR (300 MHz, DMSO-d₆) δ [ppm] = 3.78 (s, 3H), 5.35 (s, 2H), 6.38 (d, J = 2.17 Hz, 1H), 6.61 - 6.64 (m, 1H), 7.16 (m, 2H), 7.46 - 7.54 (m, 1H), 7.99 (d, J = 5.96 Hz, 1H).

### Beispiel 161A

### 1-Amino-2-[(2,6-difluorbenzyl)oxy]-4-methoxypyridinium-2,4,6-trimethylbenzolsulfonat

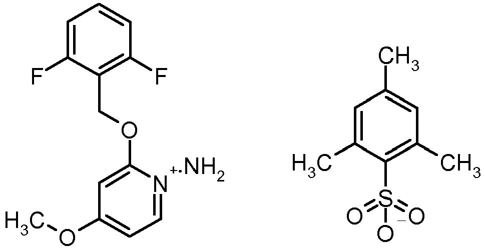

7.76 g (27.2 mmol) O-(2-Mesitylensulfonyl)acetohydroxamsäureethylester (CAS: 38202-27-6) wurden portionsweise zu einer auf -5°C vorgekühlten Mischung von 14.0 ml Trifluoressigsäure und 2.5 ml Wasser gegeben. Die erhaltene Mischung wurde 1.5 Stunden lang bei -5°C gerührt und mit 170 ml Eis/Wasser verdünnt. Es wurde mit 110 ml Dichlormethan extrahiert. Die organische Phase wurde über Natriumsulfat getrocknet, filtriert und zu einer auf 0°C vorgekühlten Lösung von 5.77 g (18.13 mmol, Reinheit 80%) 2-[(2,6-Difluorbenzyl)oxy]-4-methoxypyridin (Beispiel 160A) in 60 ml Dichlormethan gegeben. Die Mischung wurde 18 Stunden lang bei Raumtemperatur gerührt und tropfenweise zu einer gerührten Lösung von Diethylether (500 ml) gegeben. Es wurde eine weitere Stunde lang gerührt, wobei man das Lösungsmittel abdampfen ließ. Der erhaltene Niederschlag wurde filtriert, mit 100 ml Diethylether gewaschen und im Vakuum getrocknet, wodurch man 3.99 g (Ausbeute 34%, Reinheit 72%) an Produkt erhielt.
LC-MS (Methode 15): Rₜ = 0.60 min; m/z = 267.18 (M+H)⁺

### Beispiel 162A

### 7-[(2,6-Difluorbenzyl)oxy]-5-methoxy-2-methylpyrazolo[1,5-a]pyridin-3-carbonsäureethylester

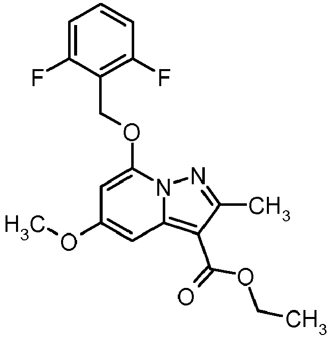

Eine Lösung von 3.99 g (6.15 mmol, Reinheit 72%) 1-Amino-2-[(2,6-difluorbenzyl)oxy]-4-methoxypyridinium-2,4,6-trimethylbenzolsulfonat (Beispiel 161A) in 70 ml N,N-Dimethylformamid wurde mit 1.38 g (12.31 mmol) 2-butinsäureethylester (CAS: 4341-76-8) und 2.55 g (18.46 mmol) Kaliumcarbonat versetzt. Die Reaktionsmischung wurde 18 Stunden lang bei Raumtemperatur gerührt und unter Rühren tropfenweise zu 150 ml Wasser gegeben. Der erhaltene Niederschlag wurde ab filtriert und im Vakuum getrocknet, wodurch man 477 mg (Ausbeute 12%, Reinheit 57%) an Produkt erhielt.
LC-MS (Methode 15): Rₜ = 1.24 min; m/z = 377.54 (M+H)⁺

### Beispiel 163A

### 7-[(2,6-Difluorbenzyl)oxy]-5-methoxy-2-methylpyrazolo[1,5-a]pyridin-3-carbonsäure

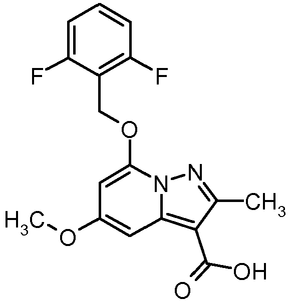

Eine Lösung von 375 mg (0.57 mmol, Reinheit 57%) 7-[(2,6-Difluorbenzyl)oxy]-5-methoxy-2-methylpyrazolo[1,5-a]pyridin-3-carbonsäureethylester (Beispiel 162A) in 8.0 ml 1,4-Dioxan wurde mit 6.0 ml wässriger 2M Natronlauge versetzt. Es wurde 18 Stunden lang bei 100°C nachgerührt. Die Reaktionsmischung wurde im Vakuum eingeengt. Der Rückstand wurde mit 4 ml Acetonitril und 2 ml Wasser gefolgt von 0.8 ml Trifluoressigsäure versetzt. Der erhaltene Niederschlag wurde abfiltriert und im Vakuum getrocknet, wodurch man 172 mg (Ausbeute 65%, Reinheit 75%) an Produkt erhielt.
LC-MS (Methode 15): Rₜ = 0.64 min; m/z = 349.11 (M+H)⁺
¹H-NMR (300 MHz, DMSO-d₆) δ [ppm] = 2.45 (s, 3H), 3.90 (s, 3H), 5.46 (s, 2H), 6.47 (d, J = 2.43 Hz, 1H), 7.03 (d, J = 2.39 Hz, 1H), 7.23 - 7.30 (m, 2H), 7.57 - 7.65 (m, 1H), 12.18 (br. s., 1H).

### Beispiel 164A

### rac-{1-[({7-[(2,6-Difluorbenzyl)oxy]-5-methoxy-2-methylpyrazolo[1,5-a]pyridin-3-yl} carbonyl)-amino]-2-methylpentan-2-yl}carbamidsäure-tert.-butylester

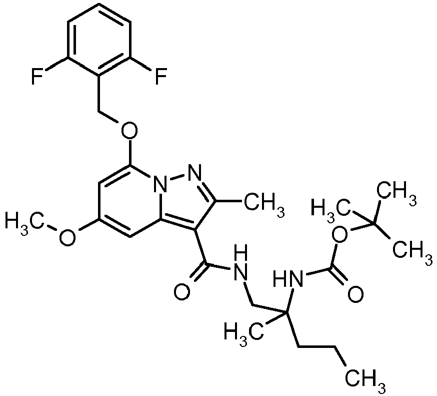

Eine Lösung von 170 mg (0.366 mmol, Reinheit 75%) an 7-[(2,6-Difluorbenzyl)oxy]-5-methoxy-2-methylpyrazolo[1,5-a]pyridin-3-carbonsäure (Beispiel 163A) in 8 ml Tetrahydrofuran wurde mit 84 mg (0.44 mmol) 1-(3-Dimethylaminopropyl)-3-ethylcarbodiimidhydrochlorid (CAS: 25952-53-8) und 60 mg (0.44 mmol) 1-Hydroxy-7-azabenzotriazol (CAS: 39968-33-7) versetzt. Die Lösung wurde 20 Minuten bei Raumtemperatur gerührt. Die Reaktionsmischung wurde mit 0.191 ml (1.01 mmol) N,N-Diisopropylethylamin und 158 mg (0.732 mmol) *rac*-(1-Amino-2-methylpentan-2-yl)carbamidsäure-tert.-butylester (Beispiel 152A) versetzt. Es wurde 18 Stunden lang bei Raumtemperatur nachgerührt, und die Reaktionsmischung wurde dann im Vakuum eingeengt. Der Rückstand wurde zwischen Dichlormethan und Wasser verteilt. Die organische Phase wurde abgetrennt, mit einer gesättigten wässrigen Natriumhydrogencarbonatlösung, Wasser sowie wässriger Natriumchlorid-Lösung extrahiert, über Natriumsulfat getrocknet, filtriert und im Vakuum eingedampft. Der verbliebene Rückstand wurde durch Flash-Chromatographie unter Verwendung einer vorgepackten Kieselgelkartusche (Laufmittel: Dichlormethan/Methanol, Gradient 0% bis 10%) aufgereinigt, wodurch man 120 mg (Ausbeute 44%, Reinheit 73%) an Produkt erhielt.
LC-MS (Methode 15): Rₜ = 1.33 min; m/z = 547.36 (M+H)⁺

### Beispiel 165A

### 2-[(2,6-Difluorbenzyl)oxy]-4-ethylpyridin

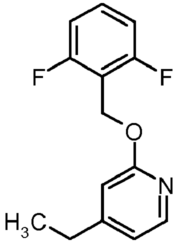

Eine Lösung von 3.8 g (9.24 mmol, Reinheit 73%) 4-Brom-2-[(2,6-difluorbenzyl)oxy]pyridin (Beispiel 145A) in 54 ml 1,4-Dioxan und 13 ml Wasser wurde 10 Minuten lang unter Argon entgast. Die Lösung wurde mit 9.04 g (27.73 mmol) Cäsiumcarbonat, 0.82 g (11.09 mmol) Ethylboronsäure (CAS: 4433-63-0) und 0.75 g (0.924 mmol) (1,1)-Bis(diphenylphosphino)ferrocendichlorpalladium(II) -Komplex mit Dichlormethan (CAS: 95464-05-4), versetzt. Das Reaktionsgefäß wurde verschlossen und der Inhalt wurde 18 Stunden lang bei 100°C gerührt. Das Lösungsmittel wurde abgedampft und der Rückstand wurde zwischen Wasser und Essigsäureethylester verteilt. Die organische Phase wurde abgetrennt und im Vakuum eingedampft. Der Rückstand wurde durch Flash-Chromatographie unter Verwendung einer vorgepackten Kieselgelkartusche (Laufmittel: Cyclohexan/Essigsäureethylester, Gradient 0% bis 5%) aufgereinigt, wodurch man 1.38 g (Ausbeute 41%, Reinheit 69%) an Produkt erhielt.
LC-MS (Methode 15): Rₜ= 1.31 min; m/z = 250.19 (M+H)⁺
¹H-NMR (300 MHz, DMSO-d₆) δ [ppm] = 1.19 (t, J = 7.58 Hz, 3H), 2.58 (q, J = 7.63 Hz, 2H), 5.41 (s, 2H), 6.57 - 6.58 (m, 1H), 6.72 - 6.75 (m, 1H), 6.91 (t, J = 7.45 Hz, 2H), 7.26 - 7.32 (m, 1H), 8.05 (d, J = 5.20 Hz, 1H).

### Beispiel 166A

### 1-Amino-2-[(2,6-difluorbenzyl)oxy]-4-ethylpyridinium 2,4,6-trimethylbenzolsulfonat

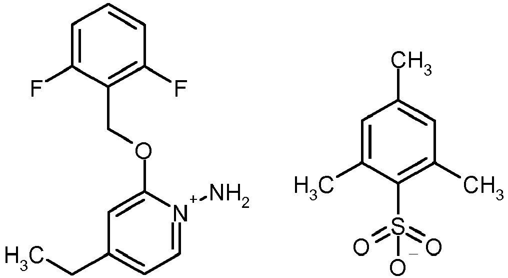

1.74 g (6.08 mmol) O-(2-Mesitylensulfonyl)acetohydroxamsäureethylester (CAS: 38202-27-6) wurden portionsweise zu einer auf -5°C vorgekühlten Mischung von 3.1 ml Trifluoressigsäure und 0.6 ml Wasser gegeben. Die erhaltene Mischung wurde 1.5 Stunden lang bei -5°C gerührt und mit 40 ml Eis/Wasser verdünnt. Die Mischung wurde mit 20 ml Dichlormethan extrahiert. Die organische Phase wurde über Natriumsulfat getrocknet, filtriert und zu einer auf 0°C vorgekühlten Lösung von 1.46 g (4.05 mmol, Reinheit 69%) 2-[(2,6-Difluorbenzyl)oxy]-4-ethylpyridin (Beispiel 165A) in 12 ml Dichlormethan gegeben. Die Mischung wurde 18 Stunden lang bei Raumtemperatur gerührt und tropfenweise zu einer gerührten Lösung von Diethylether (150 ml) gegeben. Die Lösung wurde im Vakuum eingeengt, wodurch man 1.88 g (Ausbeute 38%, Reinheit 38%) an Produkt erhielt, welches ohne weitere Aufreinigung in den nächsten Schritt eingesetzt wurde.
LC-MS (Methode 15): Rₜ = 0.73 min; m/z = 265.19 (M+H)⁺

### Beispiel 167A

### 7-[(2,6-Difluorbenzyl)oxy]-5-ethyl-2-methylpyrazolo[1,5-a]pyridin-3-carbonsäureethylester

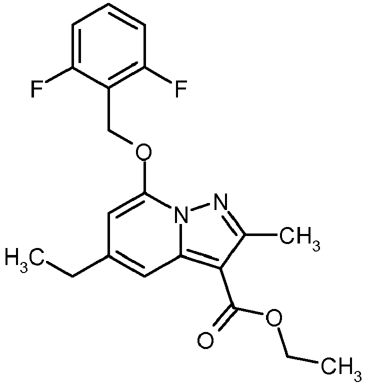

Eine Lösung von 1.88 g (1.54 mmol, Reinheit 38%) 1-Amino-2-[(2,6-difluorbenzyl)oxy]-4-ethylpyridinium-2,4,6-trimethylbenzolsulfonat (Beispiel 166A) in 12 ml N,N-Dimethylformamid wurde mit 0.35 g (3.08 mmol) 2-Butinsäureethylester (CAS: 4341-76-8) und 0.64 g (4.61 mmol) Kaliumcarbonat versetzt. Die Reaktionsmischung wurde 3 Stunden lang bei Raumtemperatur gerührt. Das Lösungsmittel wurde eingeengt und der Rückstand wurde zwischen Wasser und Essigsäureethylester verteilt. Die organische Phase wurde abgetrennt, mit wässriger Natriumchlorid-Lösung gewaschen und im Vakuum eingedampft. Der Rückstand wurde durch Flash-Chromatographie unter Verwendung einer vorgepackten Kieselgelkartusche (Laufmittel: Cyclohexan/Essigsäureethylester, Gradient 0% bis 10%) aufgereinigt, wodurch man 0.29 g (Ausbeute 35%, Reinheit 70%) an Produkt erhielt.
LC-MS (Methode 15): Rₜ = 1.35 min; m/z = 375.26 (M+H)⁺
¹H-NMR (300 MHz, CDCl₃) δ [ppm] = 1.33 (t, J = 7.60 Hz, 3H), 1.39 - 1.45 (m, 3H), 2.66 (s, 3H), 2.65 - 2.77 (m, 2H), 4.33 - 4.40 (m, 2H), 5.47 (br. s., 2H), 6.27 (d, J = 1.60 Hz, 1H), 6.91 - 6.98 (m, 2H), 7.32 - 7.40 (m, 1H), 7.57 - 7.58 (m, 1H).

### Beispiel 168A

### 7-[(2,6-Difluorbenzyl)oxy]-5-ethyl-2-methylpyrazolo[1,5-a]pyridin-3-carbonsäure

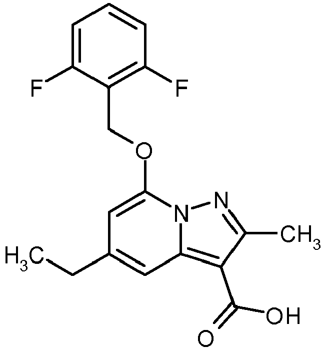

Eine Lösung von 287 mg (0.54 mmol, Reinheit 70%) 7-[(2,6-Difluorbenzyl)oxy]-5-ethyl-2-methylpyrazolo[1,5-a]pyridin-3-carbonsäureethylester (Beispiel 167A) in 5.5 ml 1,4-Dioxan wurde mit 2.7 ml wässriger 2M Natronlauge versetzt. Es wurde 18 Stunden lang bei 90°C nachgerührt. Die Reaktionsmischung wurde im Vakuum eingeengt. Der Rückstand wurde mit Wasser versetzt und der pH-Wert wurde auf 7 eingestellt. Der erhaltene Niederschlag wurde abfiltriert und im Vakuum getrocknet, wodurch man 280 mg (Ausbeute 35%, Reinheit 23%) an Produkt erhielt, welches ohne Aufreinigung in den nächsten Schritt eingesetzt wurde.
LC-MS (Methode 15): Rₜ= 0.70 min; m/z = 347.21 (M+H)⁺

### Beispiel 169A

### rac-{1-[({7-[(2,6-Difluorbenzyl)oxy]-5-ethyl-2-methylpyrazolo[1,5-a]pyridin-3-yl}carbonyl)amino]-2-methylpentan-2-yl}carbamidsäure-tert.-butylester

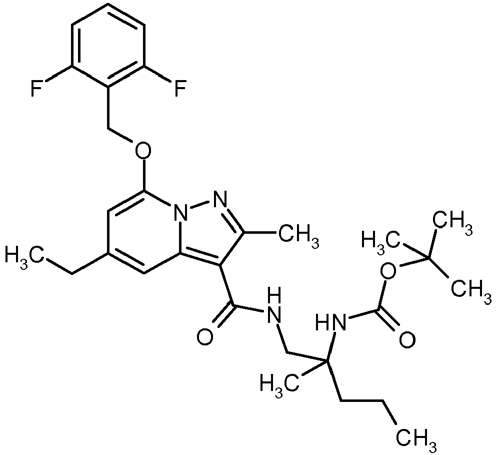

Eine Lösung von 280 mg (0.19 mmol, Reinheit 23%) 7-[(2,6-Difluorbenzyl)oxy]-5-ethyl-2-methylpyrazolo[1,5-a]pyridin-3-carbonsäure (Beispiel 168A) in 4 ml Tetrahydrofuran wurde mit 43 mg (0.22 mmol) 1-(3-Dimethylaminopropyl)-3-ethylcarbodiimidhydrochlorid (CAS: 25952-53-8) und 30 mg (0.22 mmol) 1-Hydroxy-7-azabenzotriazol (CAS: 39968-33-7) versetzt. Die Lösung wurde 20 Minuten lang bei Raumtemperatur gerührt. Die Reaktionsmischung wurde mit 0.097 ml (0.56 mmol) N,N-Diisopropylethylamin und 48 mg (0.22 mmol) *rac*-(1-Amino-2-methylpentan-2-yl)carbamidsäure-tert.-butylester (Beispiel 152A) versetzt. Es wurde 24 Stunden lang bei Raumtemperatur nachgerührt, und die Reaktionsmischung wurde dann im Vakuum eingeengt. Der Rückstand wurde zwischen Dichlormethan und Wasser verteilt. Die organische Phase wurde abgetrennt und im Vakuum eingedampft. Der Rückstand wurde durch präparative HPLC-Chromatographie (Methode 21) aufgereinigt, wodurch man 19 mg (Ausbeute 19%, Reinheit 95%) an Produkt erhielt.
LC-MS (Methode 15): Rₜ = 1.41 min; m/z = 545.44 (M+H)⁺

### Beispiel 170A

### (2S)-2-Amino-2-(2-thienyl)ethanol

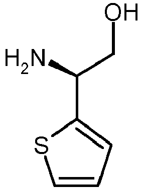

1.59 ml (3.18 mmol) Lithiumborhydrid (2 M in THF) wurden in 2.5 ml trockenem THF vorgelegt, mit 0.80 ml (6.30 mmol) Chlortrimethylsilan bei RT versetzt und 5 min bei RT gerührt. Anschließend wurden 200 mg (1.27 mmol) (2S)-Amino(2-thienyl)essigsäure portionsweise zugegeben und das Reaktionsgemisch wurde über Nacht bei RT gerührt. Zum Reaktionsgemisch wurden tropfenweise 2.5 ml Methanol gegeben und das Gemisch wurde eingeengt. Der Rückstand wurde mit 1.5 ml einer 20%igen wässrigen Kaliumhydroxid-Lösung versetzt und dreimal mit Dichlormethan extrahiert. Die vereinigten organischen Phasen wurden über Natriumsulfat getrocknet, filtriert und eingeengt. Es wurden 173 mg der Zielverbindung (90% d. Th.) erhalten.
LC-MS (Methode 7): Rₜ = 1.13 min; m/z = 127 (M-NH₃+H)⁺
¹H-NMR (400 MHz, DMSO-d₆): δ = 2.00 (br. s, 2H), 3.28 - 3.36 (m, 1H; überlagert mit Lösungsmittelsignal), 3.46 - 3.56 (m, 1H), 4.08 - 4.14 (m, 1H), 4.89 (t, 1H), 6.91 - 6.96 (m, 2H), 7.29 - 7.34 (m, 1H).

In Analogie zu Beispiel 170A wurden die in Tabelle 5A gezeigten Beispielverbindungen hergestellt, indem Lithiumborhydrid (1.7-2.5 Äquivalente) und Chlortrimethylsilan (3.4-5 Äquivalente) mit den entsprechenden, kommerziell erhältlichen oder literaturbekannten Aminosäuren unter den beschriebenen Reaktionsbedingungen umgesetzt wurden:

**Tabelle 5A:**

| **Beispiel** | **IUPAC-Name / Struktur (Ausbeute)** | **Analytische Daten** |
|---|---|---|
| **171A** | (2*S*)-2-Amino-2-(5-methyl-2-furyl)ethanol | ¹H-NMR (400 MHz, DMSO-d₆): δ = 1.65 - 1.78 (m, 2H), 2.21 (s, 3H), 3.30 - 3.39 (m, 1H; überlagert mit Lösungsmittelsignal), 3.51 - 3.61 (m, 1H), 3.71 - 3.79 (m, 1H), 4.71 (br. s, 1H), 5.92 (d, 1H), 6.06 (d, 1H). LC-MS (Methode 7): Rₜ = 1.23 min; m/z = 125 (M-NH₃+H)⁺ |
| | 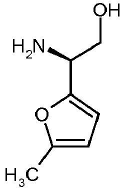 | |
| | (88% d. Th.) | |
| **172A** | (2*R*)-2-Amino-2-(5-methyl-2-furyl)ethanol | ¹H-NMR (400 MHz, DMSO-d₆): δ = 1.65 - 1.77 (m, 2H), 2.21 (s, 3H), 3.30 - 3.39 (m, 1H; überlagert mit Lösungsmittelsignal), 3.51 - 3.61 (m, 1H), 3.71 - 3.80 (m, 1H), 4.71 (br. s, 1H), 5.92 (d, 1H), 6.06 (d, 1H). LC-MS (Methode 7): Rₜ = 1.24 min; m/z = 125 (M-NH₃+H)⁺ |
| | 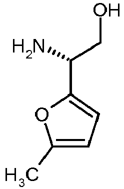 | |
| | (79% d. Th.) | |

### Beispiel 173A

### tert-Butyl-[(1S)-1-{5-[(1S)-1-amino-2-hydroxyethyl]-1,3,4-oxadiazol-2-yl}-2-methylpropyl]carbamat Acetat

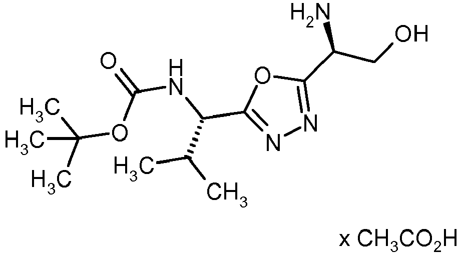

Die Zielverbindung kann in zwei Stufen analog E. Ko et al. Journal of the American Chemical Society 2011, 133, 3, 462-477 hergestellt werden.

Stufe 1: Reaktion von tert-Butyl-[(2*S*)-1-hydrazino-3-methyl-1-oxobutan-2-yl]carbamat [CAS: 72039-28-2], O-Benzyl-N-[(benzyloxy)carbonyl]-L-serin [CAS: 20806-43-3] mit 1-Ethyl-3-(3-dimethylaminopropyl)carbodiimid Hydrochlorid (EDC, CAS: 25952-53-8) und anschließend mit Iod, Triethylamin und Triphenylphosphin liefert Benzyl-[(1S)-2-(benzyloxy)-1-(5-{(1S)-1-[(tert-butoxycarbonyl)amino]-2-methylpropyl}-1,3,4-oxadiazol-2-yl)ethyl]carbamat.

Stufe 2: Reaktion von Benzyl-[(1S)-2-(benzyloxy)-1-(5-{(1S)-1-[(tert-butoxycarbonyl)amino]-2-methylpropyl}-1,3,4-oxadiazol-2-yl)ethyl]carbamat mit Wasserstoff, Palladium/Aktivkohle in Essigsäure oder Essigsäure/Ethanol oder Essigsäure/Methanol bei RT, 40°C, 60°C oder 78°C.
LC-MS (Methode 7): Rₜ = 1.73 min
MS (ESpos): m/z = 301 (M-HOAc+H)⁺

### Beispiel 174A

### tert-Butyl-[(1S)-1-(5-{(1S)-1-[({7-[(2,6-difluorbenzyl)oxy]-2,5-dimethylpyrazolo[1,5-a]pyridin-3-yl}carbonyl)amino]-2-hydroxyethyl}-1,3,4-oxadiazol-2-yl)-2-methylpropyl]carbamat Trifluoracetat

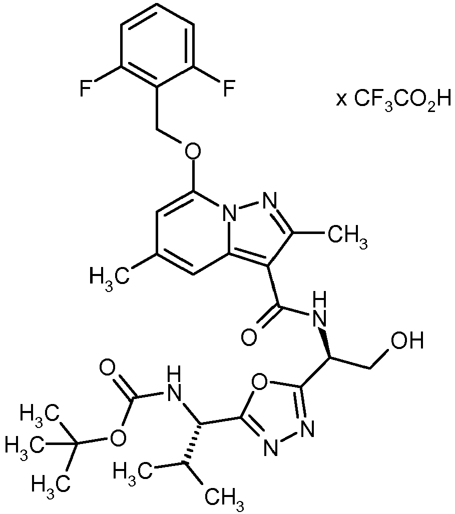

124 mg (0.37 mmol) 7-[(2,6-Difluorbenzyl)oxy]-2,5-dimethylpyrazolo[1,5-a]pyridin-3-carbonsäure aus Beispiel 4A wurden mit 184 mg (0.49 mmol) HATU und 0.52 ml (3.00 mmol) N,N-Diisopropylethylamin in 1.24 ml DMF vorgelegt und 10 min bei Raumtemperatur gerührt. Anschließend wurden 175 mg (0.49 mmol) tert-Butyl-[(1*S*)-1-{5-[(1*S*)-1-amino-2-hydroxyethyl]-1,3,4-oxadiazol-2-yl}-2-methylpropyl]carbamat Acetat aus Beispiel 173A zur Reaktionslösung gegeben und das Gemisch wurde 1 h bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Es wurden 211 mg der Zielverbindung (73% d. Th.; Reinheit 94%) erhalten.
LC-MS (Methode 2): Rₜ = 1.05 min
MS (ESpos): m/z = 615 (M-TFA+H)⁺

### Ausführungsbeispiele

### Beispiel 1

### N-(2-Amino-2-methylpropyl)-7-[(2,6-difluorbenzyl)oxy]-2,5-dimethylpyrazolo[1,5-a]pyridin-3-carboxamid Formiat

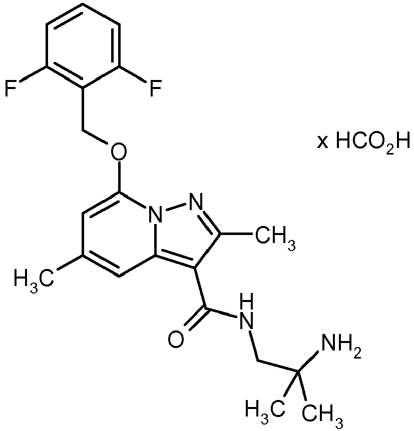

Eine Mischung von 78.0 mg (0.235 mmol, 1.0 eq.) 7-[(2,6-Difluorbenzyl)oxy]-2,5-dimethylpyrazolo[1,5-a]pyridin-3-carbonsäure aus Beispiel 4A, 49 µl (0.47 mmol, 2.0 eq.) 1,2-Diamino-2-methylpropan [CAS-Nr.: 811-93-8] und 0.20 ml (1.2 mmol, 5.0 eq.) N,N-Diisopropylethylamin in 2.4 ml DMF wurde mit 116 mg HATU (0.305 mmol, 1.3 eq.) versetzt und für 6 h bei RT gerührt. Anschließend wurde Wasser zugegeben und dreimal mit Essigsäureethylester extrahiert. Die vereinten organischen Phasen wurden mit gesättigter wässriger Natriumchloridlösung gewaschen, über Magnesiumsulfat getrocknet, filtriert und eingeengt. Der Rückstand wurde mit Wasser ausgerührt, der ausgefallene Feststoff abfiltriert und mittels präparativer HPLC (Methode 4) gereinigt. Es wurden 22.3 mg der Titelverbindung (24 % d. Th.) erhalten.
LC-MS (Methode 2): Rₜ = 0.75 min
MS (ESpos): m/z = 403 (M+H)⁺
¹H NMR (400 MHz, DMSO-d₆) δ [ppm] = 1.21 (s, 6H) 2.42 (s, 3H), 2.53 (s, 3H), 3.37 (d, 2H) 5.45 (s, 2H) 6.57 (s, 1H) 7.21 - 7.32 (m, 2H) 7.41 (s, 1H) 7.58 - 7.68 (m, 1H), 7.68 - 7.75 (m, 1H), 8.36 (br. s, 1H).

### Beispiel 2

### ent-N-(2-Amino-2-methylbutyl)-7-[(2,6-difluorbenzyl)oxy]-2,5-dimethylpyrazolo[1,5-a]pyridin-3-carboxamid Formiat (Enantiomer A)

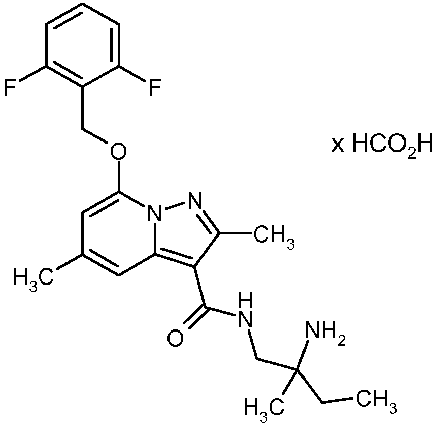

Eine Mischung von 81.0 mg *ent*-Benzyl-{1-[({7-[(2,6-difluorbenzyl)oxy]-2,5-dimethylpyrazolo[1,5-a]pyridin-3-yl}carbonyl)amino]-2-methylbutan-2-yl}carbamat (0.147 mmol, 1.0 eq.) aus Beispiel 21A (Enantiomer A) und 10 mg (0.015 mmol, 0.1 eq) 20 %igem Palladiumhydroxid auf Aktivkohle in 5.0 ml Ethanol wurde für 4.5 h bei RT und Normaldruck hydriert. Anschließend wurde über Kieselgur filtriert, mit Methanol und Dichlormethan gewaschen und das Filtrat eingeengt. Das Rohprodukt wurde mittels präparativer HPLC (Methode 5) gereinigt, wobei 30 mg der Titelverbindung (49 % d. Th.) erhalten wurden.
LC-MS (Methode 2): Rₜ = 0.76 min
MS (ESpos): m/z = 417 (M+H)⁺
¹H-NMR (400 MHz, DMSO-d₆): δ [ppm] = 0.91 (t, 3H), 1.11 (s, 3H), 1.38 - 1.61 (m, 2H), 2.41 (s, 3H), 2.52 (s, 3H), 3.27 - 3.41 (m, 2H) 5.45 (s, 2H) 6.57 (s, 1H) 7.22 - 7.31 (m, 2H), 7.41 (s, 1H) 7.53 - 7.68 (m, 2H), 8.34 (br. s, 1H).

### Beispiel 3

### ent-N-(2-Amino-2-methylbutyl)-2,5-dimethyl-7-[(2,3,6-trifluorbenzyl)oxy]pyrazolo[1,5-a]pyridin-3-carboxamid (Enantiomer A)

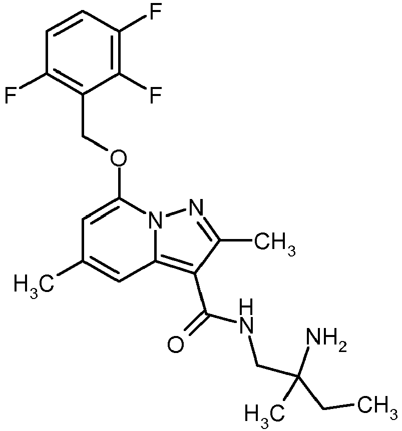

36 mg (0.04 mmol, Reinheit 83%) *ent*-Benzyl-{1-[({2,5-dimethyl-7-[(2,3,6-trifluorbenzyl)-oxy]pyrazolo[1,5-a]pyridin-3-yl}carbonyl)amino]-2-methylbutan-2-yl}carbamat Trifluoracetat aus Beispiel 22A (Enantiomer A) wurden unter Argon in 2 ml Ethanol gelöst, mit 3 mg (0.004 mmol) 20%igem Palladium(II)hydroxid auf Aktivkohle versetzt und für 5 Stunden bei Normaldruck hydriert. Die Reaktionslösung wurde über einen Millipore Filter filtriert, mit Ethanol nachgewaschen und im Vakuum eingeengt. Der Rückstand wurde mittels präparativer HPLC gereinigt (RP18 Säule, Laufmittel: Acetonitril/Wasser-Gradient unter Zusatz von 0.05% TFA). Die Produktfraktionen wurden vereinigt und eingeengt. Anschließend wurde der Rückstand in Dichlormethan aufgenommen und mit 0.5 ml gesättigter, wässriger Natriumhydrogencarbonatlösung gewaschen. Die wässrige Phase wurde zweimal mit Dichlormethan re-extrahiert. Die vereinigten organischen Phasen wurden über Natriumsulfat getrocknet, filtriert, eingeengt und lyophilisiert. Es wurden 12 mg der Zielverbindung (62% d. Th.) erhalten.
LC-MS (Methode 2): Rₜ = 0.74 min
MS (ESpos): m/z = 435 (M+H)⁺
¹H-NMR (500 MHz, DMSO-d₆): δ = 0.86 (t, 3H), 0.97 (s, 3H), 1.28 - 1.41 (m, 2H), 1.48 (br. s, 2H), 2.40 (s, 3H), 2.51 (s, 3H), 3.09 - 3.24 (m, 2H), 5.49 (s, 2H), 6.55 (s, 1H), 7.20 (t, 1H), 7.28 - 7.35 (m, 1H), 7.41 (s, 1H), 7.65 - 7.73 (m, 1H).

### Beispiel 4

### ent-N-(2-Amino-2-methylpentyl)-2,5-dimethyl-7-[(2,3,6-trifluorbenzyl)oxy]pyrazolo[1,5-a]pyridin-3-carboxamid (Enantiomer B)

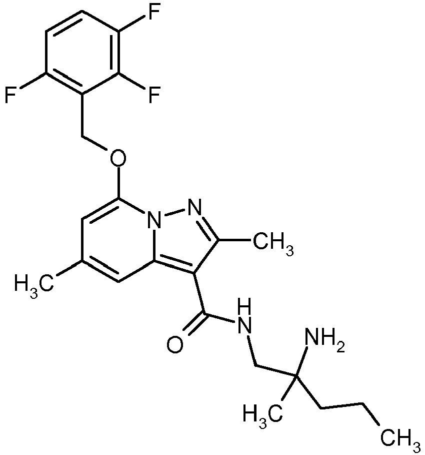

59 mg (0.08 mmol, Reinheit 93%) ent-Benzyl-{1-[({2,5-dimethyl-7-[(2,3,6-trifluorbenzyl)-oxy]pyrazolo[1,5-a]pyridin-3-yl}carbonyl)amino]-2-methylpentan-2-yl}carbamat Trifluoracetat aus Beispiel 23A (Enantiomer B) wurden in 3.7 ml Ethanol gelöst, mit 5.5 mg (0.01 mmol) 20%igem Palladium(II)hydroxid auf Aktivkohle versetzt und insgesamt 5 Stunden bei Normaldruck hydriert. Die Reaktionslösung wurde mittels Milliporfilter filtriert und das Filtrat einrotiert. Der Rückstand wurde mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Die Produktmischfraktionen wurden eingedampft und nochmals mittels präparativer HPLC gereinigt (RP18 Säule, Laufmittel: Acetonitril/Wasser-Gradient unter Zusatz von 0.1% TFA). Alle Produktfraktionen wurden vereinigt und eingeengt. Anschließend wurde der Rückstand in Dichlormethan und wenig Methanol aufgenommen und mit 0.5 ml gesättigter, wässriger Natriumhydrogencarbonatlösung gewaschen. Die wässrige Phase wurde zweimal mit Dichlormethan re-extrahiert. Die vereinigten organischen Phasen wurden über Natriumsulfat getrocknet, filtriert, eingeengt und lyophilisiert. Es wurden 15 mg der Zielverbindung (42% d. Th.) erhalten.
LC-MS (Methode 2): Rₜ = 0.83 min
MS (ESpos): m/z = 449 (M+H)⁺
¹H-NMR (400 MHz, DMSO-d₆): δ = 0.87 (t, 3H), 0.98 (s, 3H), 1.25 - 1.42 (m, 4H), 1.72 (br. s, 2H), 2.42 (s, 3H), 2.52 (s, 3H), 3.09 - 3.24 (m, 2H), 5.49 (s, 2H), 6.56 (s, 1H), 7.24 (t, 1H), 7.28 - 7.35 (m, 1H), 7.40 (s, 1H), 7.66 - 7.74 (m, 1H).

### Beispiel 5

### N-[2-(1-Hydroxycyclopentyl)ethyl]-2,5-dimethyl-7-[(2,3,6-trifluorbenzyl)oxy]pyrazolo[1,5-a]pyridin-3-carboxamid

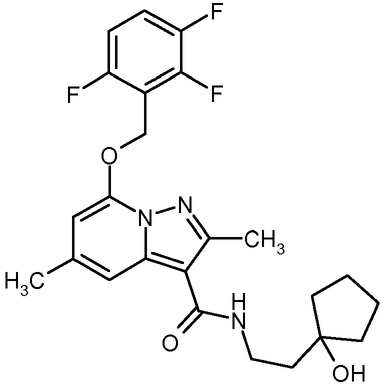

12.9 mg (0.1 mmol) 1-(2-Aminoethyl)cyclopentanol wurden in einer 96er deep well Multititerplatte vorgelegt. Eine Lösung von 35 mg (0.1 mmol) 2,5-Dimethyl-7-[(2,3,6-trifluorbenzyl)oxy]pyrazolo[1,5-a]pyridin-3-carbonsäure aus Beispiel 10A in 0.2 ml DMF sowie eine Lösung von 45.6 mg (0.12 mol) HATU in 0.2 ml DMF wurden nacheinander hinzugegeben. Nach Zugabe von 20.2 mg (0.2 mmol) 4-Methylmorpholin und 0.2 ml Dichlormethan wurde das Gemisch bei RT über Nacht geschüttelt. Dann wurde das Dichlormethan abgedampft, filtriert und aus dem Filtrat die Zielverbindung per präparativer LC-MS (Methode 9) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 5 mg (10% d. Th.) erhalten.
LC-MS (Methode 10): Rₜ = 1.08 min
MS (ESpos): m/z = 462 (M+H)⁺

In Analogie zu Beispiel 5 wurden die in Tabelle 1 gezeigten Beispielverbindungen hergestellt, indem 2,5-Dimethyl-7-[(2,3,6-trifluorbenzyl)oxy]pyrazolo[1,5-a]pyridin-3-carbonsäure aus Beispiel 10A mit den entsprechenden, kommerziell erhältlichen oder zuvor beschriebenen Aminen unter den beschriebenen Bedingungen umgesetzt wurden:

**Tabelle 1:**

| **Beispiel** | **IUPAC-Name/Struktur (Ausbeute)** | **Analytische Daten** |
|---|---|---|
| **6** | *N*-(2-Hydroxy-2,3-dihydro-1H-inden-1-yl)-2,5-dimethyl-7-[(2,3,6-trifluorbenzyl)oxy]pyrazolo[1,5-a]pyridin-3-carboxamid | LC-MS (Methode 10): Rₜ = 1.10 min MS (ESpos): m/z = 482 (M+H)⁺ |
| | 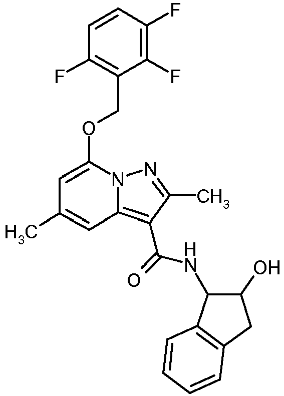 | |
| | (17% d. Th.) | |
| **7** | *N*-[(2S)-3-Hydroxy-1-(2-naphthylamino)-1-oxopropan-2-yl]-2,5-dimethyl-7-[(2,3,6-trifluorbenzyl)oxy]pyrazolo[1,5-a]pyridin-3-carboxamid | LC-MS (Methode 10): Rₜ = 1.12 min MS (ESpos): m/z = 563 (M+H)⁺ |
| | 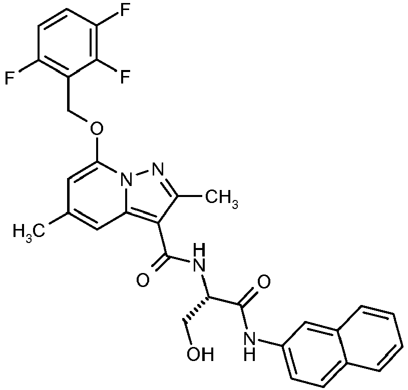 | |
| | (12% d. Th.) | |
| **8** | *N*-(2-Amino-2-ethylbutyl)-2,5-dimethyl-7-[(2,3,6-trifluorbenzyl)oxy]pyrazolo[1,5-a]pyridin-3-carboxamid | LC-MS (Methode 10): Rₜ = 0.78 min MS (ESpos): m/z = 449 (M+H)⁺ |
| | 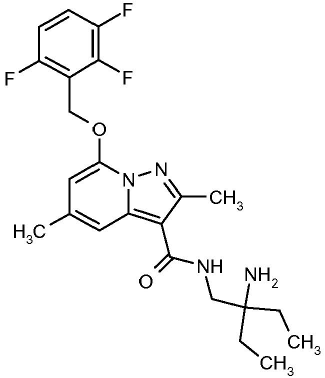 | |
| | (31% d. Th.) | |
| **9** | 2,5-Dimethyl-N-(2,2,6,6-tetramethylpiperidin-4-yl)-7-[(2,3,6-trifluorbenzyl)oxy]pyrazolo[1,5-a]pyridin-3-carboxamid | LC-MS (Methode 10): Rₜ = 0.77 min MS (ESpos): m/z = 489 (M+H)⁺ |
| | 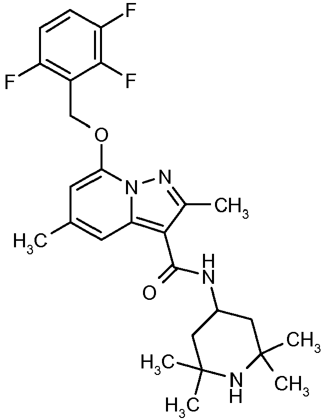 | |
| | (54% d. Th.) | |
| **10** | *rac*-2,5-Dimethyl-N-(2-phenylcyclopropyl)-7-[(2,3,6-trifluorbenzyl)oxy]pyrazolo[1,5-a]pyridin-3-carboxamid | LC-MS (Methode 10): Rₜ = 1.19 min MS (ESpos): m/z = 466 (M+H)⁺ |
| | 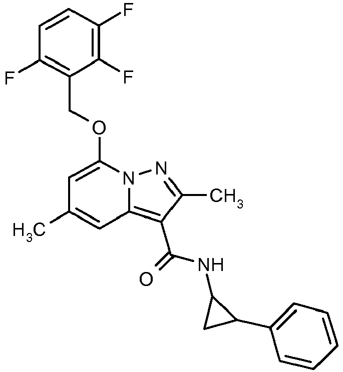 | |
| | (24% d. Th.) | |
| **11** | *N*-[1-(2-Hydroxyethyl)-3,5-dimethyl-1H-pyrazol-4-yl]-2,5-dimethyl-7-[(2,3,6-trifluorbenzyl)oxy]pyrazolo[1,5-a]pyridin-3-carboxamid | LC-MS (Methode 10): Rₜ = 0.95 min MS (ESpos): m/z = 488 (M+H)⁺ |
| | 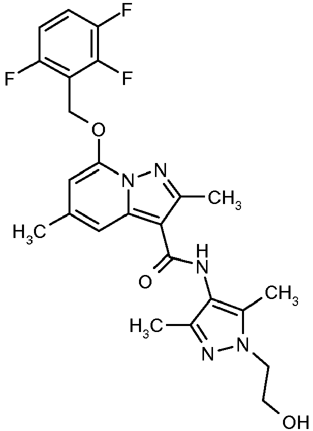 | |
| | (14% d. Th.) | |
| **12** | *N*-[2-(Dimethylamino)ethyl]-2,5-dimethyl-7-[(2,3,6-trifluorbenzyl)oxy]pyrazolo[1,5-a]pyridin-3-carboxamid | LC-MS (Methode 10): Rₜ = 0.73 min MS (ESpos): m/z = 421 (M+H)⁺ |
| | 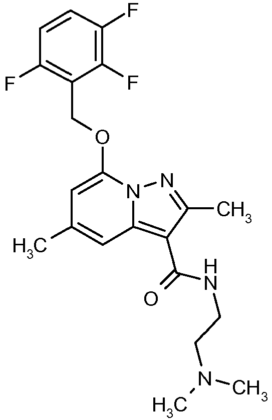 | |
| | (7% d. Th.) | |
| **13** | *rac*-N-(2-Amino-2-cyclopropylpropyl)-2,5-dimethyl-7-[(2,3,6-trifluorbenzyl)oxy]pyrazolo[1,5-a]pyridin-3-carboxamid | LC-MS (Methode 10): Rₜ = 0.76 min MS (ESpos): m/z = 447 (M+H)⁺ |
| | 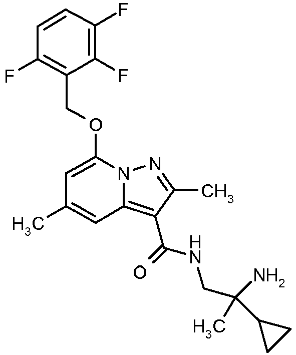 | |
| | (28% d. Th.) | |
| **14** | 2,5-Dimethyl-*N-*[2-(pyridin-3-yl)benzyl]-7-[(2,3,6-trifluorbenzyl)oxy]pyrazolo[1,5-a]pyridin-3-carboxamid | LC-MS (Methode 10): Rₜ = 1.07 min MS (ESpos): m/z = 517 (M+H)⁺ |
| | 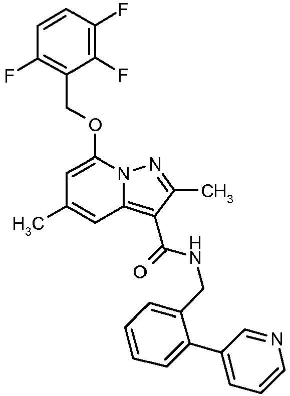 | |
| | (28% d. Th.) | |
| **15** | 2,5-Dimethyl-*N*-[2-(morpholin-4-yl)ethyl]-7-[(2,3,6-trifluorbenzyl)oxy]pyrazolo[1,5-a]pyridin-3-carboxamid | LC-MS (Methode 10): Rₜ = 0.73 min MS (ESpos): m/z = 463 (M+H)⁺ |
| | 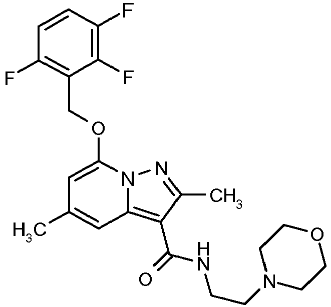 | |
| | (61% d. Th.) | |
| **16** | *N*-[1-(4-Fluorbenzyl)-3,5-dimethyl-1H-pyrazol-4-yl]-2,5-dimethyl-7-[(2,3,6-trifluorbenzyl)oxy]pyrazolo[1,5-a]pyridin-3-carboxamid | LC-MS (Methode 10): Rₜ = 1.14 min MS (ESpos): m/z = 552 (M+H)⁺ |
| | 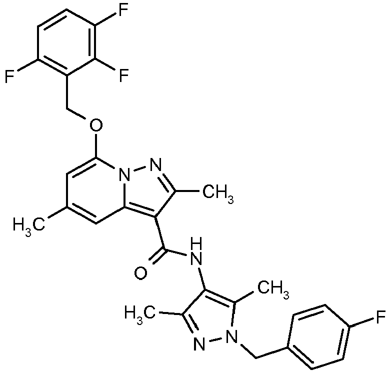 | |
| | (12% d. Th.) | |
| **17** | 2,5-Dimethyl-7-[(2,3,6-trifluorbenzyl)oxy]-*N*-[1-(2,2,2-trifluorethyl)-1H-pyrazol-4-yl]pyrazolo[1,5-a]pyridin-3-carboxamid | LC-MS (Methode 10): Rₜ = 1.10 min MS (ESpos): m/z = 498 (M+H)⁺ |
| | 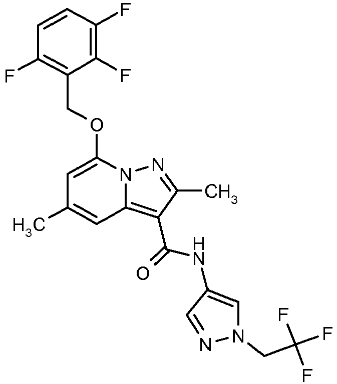 | |
| | (27% d. Th.) | |
| **18** | *N*-(1H-Imidazol-2-ylmethyl)-2,5-dimethyl-7-[(2,3,6-trifluorbenzyl)oxy]pyrazolo[1,5-a]pyridin-3-carboxamid | LC-MS (Methode 10): Rₜ = 0.72 min MS (ESpos): m/z = 430 (M+H)⁺ |
| | 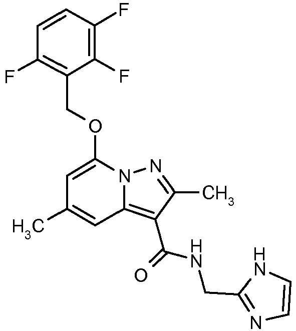 | |
| | (44% d. Th.) | |
| **19** | *rac*-2,5-Dimethyl-7-[(2,3,6-trifluorbenzyl)oxy]*-N-*{1-[3-(trifluormethyl)-1,2,4-oxadiazol-5-yl]ethyl}pyrazolo[1,5-a]pyridin-3-carboxamid | LC-MS (Methode 10): Rₜ = 1.19 min MS (ESpos): m/z = 514 (M+H)⁺ |
| | 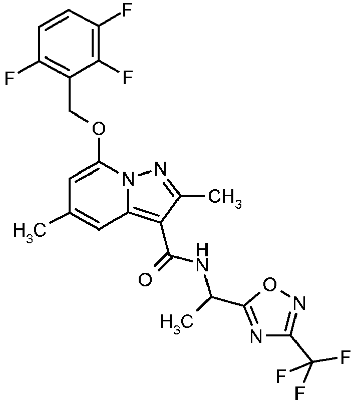 | |
| | (31% d. Th.) | |
| **20** | 2,5-Dimethyl-*N*-[5-methyl-3-(trifluormethyl)-1H-pyrazol-4-yl]-7-[(2,3,6-trifluorbenzyl)oxy]pyrazolo[1,5-a]pyridin-3-carboxamid | LC-MS (Methode 10): Rₜ = 1.05 min MS (ESpos): m/z = 498 (M+H)⁺ |
| | 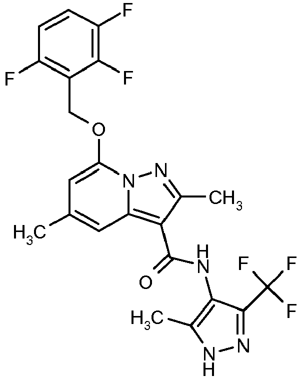 | |
| | (7% d. Th.) | |
| **21** | *N*-[(6-Chlorpyridin-3-yl)methyl]-2,5-dimethyl-7-[(2,3,6-trifluorbenzyl)oxy]pyrazolo[1,5-a]pyridin-3-carboxamid | LC-MS (Methode 10): Rₜ = 1.10 min MS (ESpos): m/z = 475 (M+H)⁺ |
| | 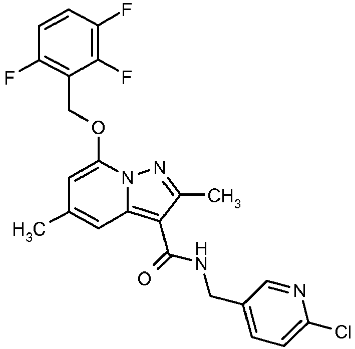 | |
| | (2% d. Th.) | |
| **22** | *ent*-2,5-Dimethyl-*N*-(6,6,7,7,7-pentafluor-2-hydroxy-2-methylheptan-3-yl)-7-[(2,3,6-trifluorbenzyl)oxy]pyrazolo[1,5-a]pyridin-3-carboxamid | LC-MS (Methode 10): Rₜ = 1.16 min MS (ESpos): m/z = 568 (M+H)⁺ |
| | 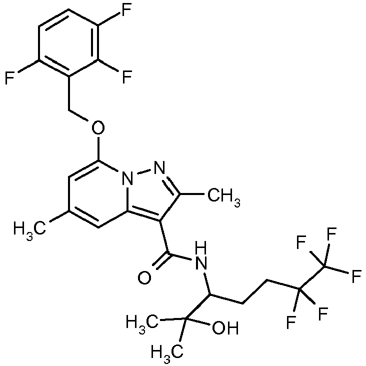 | |
| | (18% d. Th.) | |
| **23** | 2,5-Dimethyl-*N*-(pyrazolo[1,5-a]pyridin-3-yl)-7-[(2,3,6-trifluorbenzyl)oxy]pyrazolo[1,5-a]pyridin-3-carboxamid | LC-MS (Methode 10): Rₜ = 1.10 min MS (ESpos): m/z = 466 (M+H)⁺ |
| | 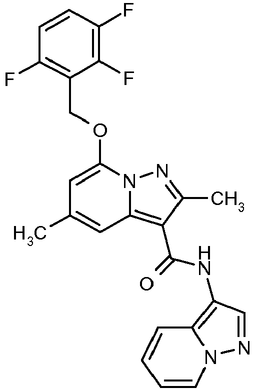 | |
| | (14% d. Th.) | |
| **24** | *rac*-2,5-Dimethyl-7-[(2,3,6-trifluorbenzyl)oxy]-*N-*[1-(2,2,2-trifluorethoxy)propan-2-yl]pyrazolo[1,5-a]pyridin-3-carboxamid | LC-MS (Methode 10): Rₜ = 1.15 min MS (ESpos): m/z = 490 (M+H)⁺ |
| | 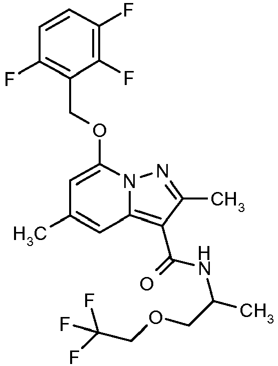 | |
| | (13% d. Th.) | |
| **25** | *N*-[(1-Hydroxycyclopropyl)methyl]-2,5-dimethyl-7-[(2,3,6-trifluorbenzyl)oxy]pyrazolo[1,5-a]pyridin-3-carboxamid | LC-MS (Methode 10): Rₜ = 0.98 min MS (ESpos): m/z = 420 (M+H)⁺ |
| | 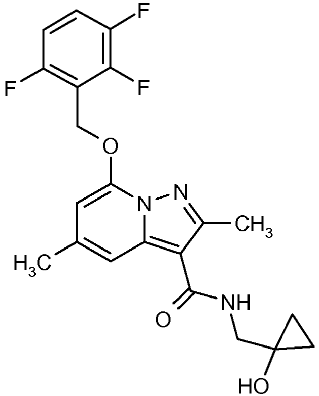 | |
| | (40% d. Th.) | |
| **26** | *N*-{2-[Cyclopropyl(2,2-difluorethyl)amino]ethyl}-2,5-dimethyl-7-[(2,3,6-trifluorbenzyl)oxy]pyrazolo[1,5-a]pyridin-3-carboxamid | LC-MS (Methode 10): Rₜ = 1.15 min MS (ESpos): m/z = 497 (M+H)⁺ |
| | 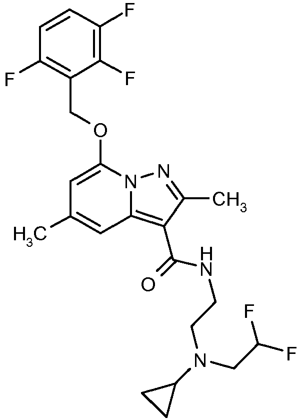 | |
| | (26% d. Th.) | |
| **27** | 2,5-Dimethyl-*N*- [(3S)-2-oxotetrahydrofuran-3-yl]-7-[(2,3,6-trifluorbenzyl)oxy]pyrazolo[1,5-a]pyridin-3-carboxamid | LC-MS (Methode 10): Rₜ = 1.00 min MS (ESpos): m/z = 434 (M+H)⁺ |
| | 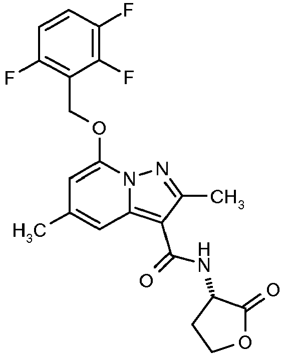 | |
| | (40% d. Th.) | |
| **28** | *N*-[1-Hydroxy-2-(hydroxymethyl)butan-2-yl]-2,5-dimethyl-7-[(2,3,6-trifluorbenzyl)oxy]pyrazolo[1,5-a]pyridin-3-carboxamid | LC-MS (Methode 10): Rₜ = 1.00 min MS (ESpos): m/z = 452 (M+H)⁺ |
| | 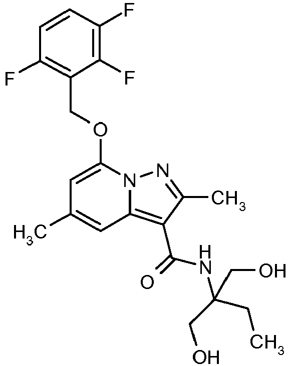 | |
| | (38% d. Th.) | |
| **29** | 2,5-Dimethyl-*N*-(9-methyl-9-azabicyclo[3.3.1]non-3-yl)-7-[(2,3,6-trifluorbenzyl)oxy]pyrazolo[1,5-a]pyridin-3-carboxamid | LC-MS (Methode 10): Rₜ = 0.77 min MS (ESpos): m/z = 487 (M+H)⁺ |
| | 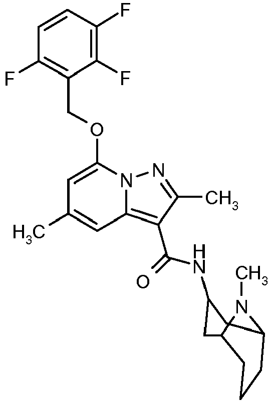 | |
| | (66% d. Th.) | |
| **30** | *rac*-N-[1-(4-Fluorphenyl)-2-hydroxyethyl]-2,5-dimethyl-7-[(2,3,6-trifluorbenzyl)oxy]pyrazolo[1,5-a]pyridin-3-carboxamid | LC-MS (Methode 10): Rₜ = 1.07 min MS (ESpos): m/z = 488 (M+H)⁺ |
| | 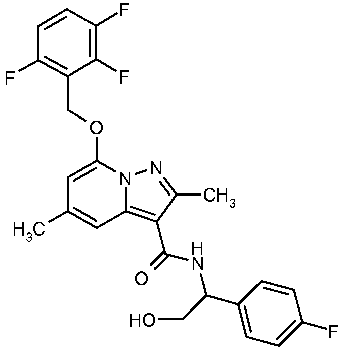 | |
| | (40% d. Th.) | |
| **31** | N-[1-(4-Chlorphenyl)cyclopentyl]-2,5-dimethyl-7-[(2,3,6-trifluorbenzyl)oxy]pyrazolo[1,5-a]pyridin-3-carboxamid | LC-MS (Methode 10): Rₜ = 1.26 min MS (ESpos): m/z = 528 (M+H)⁺ |
| | 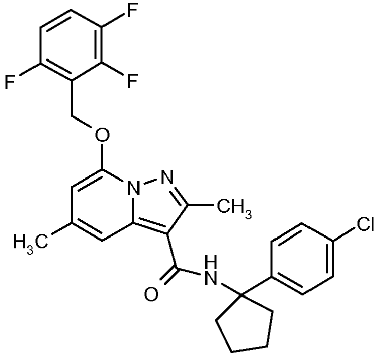 | |
| | (15% d. Th.) | |
| **32** | *N*-[2-(1H-Imidazol-1-yl)benzyl]-2,5-dimethyl-7-[(2,3,6-trifluorbenzyl)oxy]pyrazolo[1,5-a]pyridin-3-carboxamid | LC-MS (Methode 10): Rₜ = 0.82 min MS (ESpos): m/z = 506 (M+H)⁺ |
| | 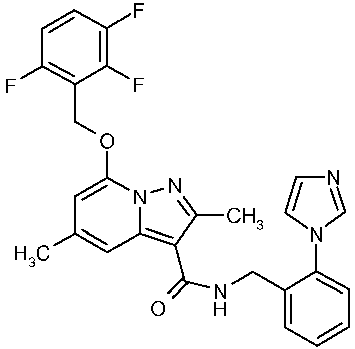 | |
| | (56% d. Th.) | |
| **33** | *N*-[1-(2-Hydroxyethyl)-1H-pyrazol-4-yl]-2,5-dimethyl-7-[(2,3,6-trifluorbenzyl)oxy]pyrazolo[1,5-a]pyridin-3-carboxamid | LC-MS (Methode 10): Rₜ = 0.94 min MS (ESpos): m/z = 460 (M+H)⁺ |
| | 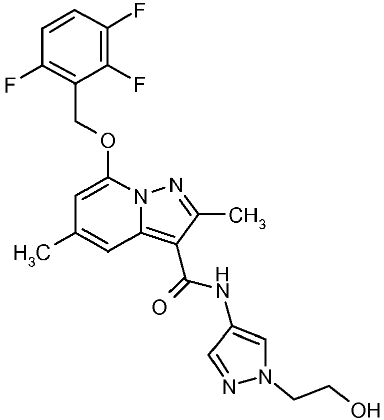 | |
| | (24% d. Th.) | |
| **34** | *N*-[(4-Chlorphenyl)(cyan)methyl]-2,5-dimethyl-7-[(2,3,6-trifluorbenzyl)oxy]pyrazolo[1,5-a]pyridin-3-carboxamid | LC-MS (Methode 10): Rₜ = 1.20 min MS (ESpos): m/z = 499 (M+H)⁺ |
| | 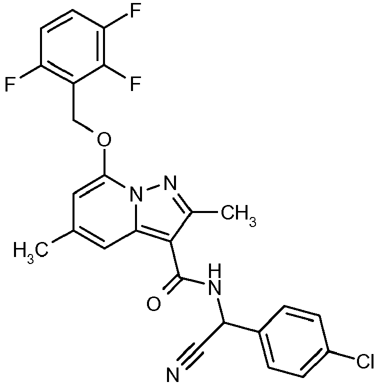 | |
| | (9% d. Th.) | |

### Beispiel 35

### Methyl-trans-4-{[({7-[(2,6-difluorbenzyl)oxy]-2,5-dimethylpyrazolo[1,5-a]pyridin-3-yl}carbonyl)amino]methyl}cyclohexancarboxylat

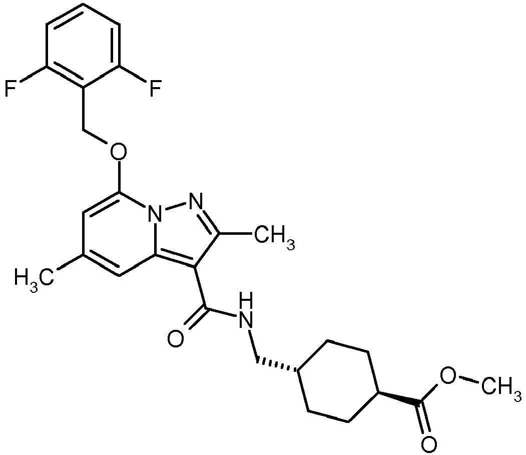

Eine Mischung von 100 mg (0.30 mmol, 1.0 eq.) 7-[(2,6-Difluorbenzyl)oxy]-2,5-dimethylpyrazolo[1,5-a]pyridin-3-carbonsäure aus Beispiel 4A, 116 mg TBTU (0.36 mmol, 1.2 eq.) und 0.132 ml (1.2 mmol, 4.0 eq.) 4-Methylmorpholin in 2.2 ml DMF wurde mit 103 mg (0.60 mmol, 2.0 eq.) Methyl-*trans*-4-(aminomethyl)cyclohexancarboxylat versetzt und das Gemisch wurde über Nacht bei RT gerührt. Das Reaktionsgemisch wurde mit Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonat-lösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 67 mg der Zielverbindung (46% d. Th.) erhalten.
LC-MS (Methode 2): Rₜ = 0.93 min
MS (ESpos): m/z = 486 (M+H)⁺

### Beispiel 36

### trans-4-{[({7-[(2,6-Difluorbenzyl)oxy]-2,5-dimethylpyrazolo[1,5-a]pyridin-3-yl}carbonyl)amino]methyl}cyclohexancarbonsäure

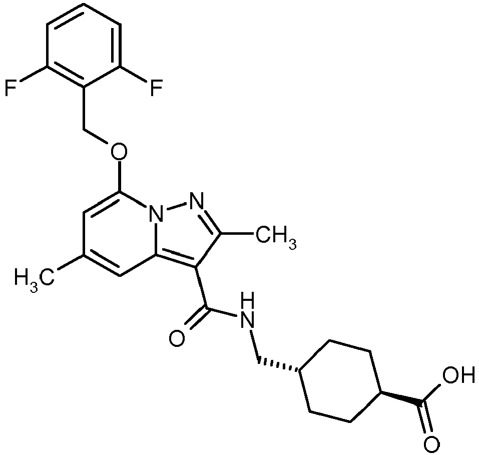

90 mg (0.19 mmol, 1.0 eq.) Methyl-*trans*-4-{[({7-[(2,6-difluorbenzyl)oxy]-2,5-dimethylpyrazolo[1,5-a]pyridin-3-yl}carbonyl)amino]methyl}cyclohexancarboxylat aus Beispiel 35 wurden in 5.5 ml THF/Methanol (5/1) gelöst und mit 1.3 ml 1 N wässrige Lithiumhydroxid-Lösung versetzt. Das Gemisch wurde 3 Tage bei RT gerührt. Anschließend wurden nochmals 2 ml 1 N Lithiumhydroxid-Lösung hinzugegeben und das Gemisch wurde 7 Tage bei 60°C gerührt.

Nach Abkühlen wurde das Gemisch mit 1 N Salzsäure auf pH = 3 angesäuert und am Rotationsverdampfer von den organischen Lösemitteln befreit. Die erhaltene wässrige Phase wurde dreimal mit Dichlormethan extrahiert und die vereinigten organischen Phasen wurden mit Wasser gewaschen, anschließend mit Natriumsulfat getrocknet, filtriert und eingedampft. Der Rückstand wurde mittels Dickschichtchromatographie gereinigt (Laufmittel: Dichlormethan/Methanol = 20/1). Es wurden 46 mg der Zielverbindung (53% d. Th.) erhalten.
LC-MS (Methode 2): Rₜ = 0.94 min
MS (ESpos): m/z = 472 (M+H)⁺
¹H NMR (500 MHz, DMSO-d₆) δ [ppm] = 0.92 -1.01 (m, 2H) 1.21 - 1.34 (m, 2H), 1.46 - 1.57 (m, 1H), 1.75 - 1.84 (m, 2H), 1.86 - 1.96 (m, 2H), 2.09 - 2.18 (m, 1H), 2.40 (s, 3H), 2.47 (s, 3H), 3.12 (t, 2H), 5.44 (s, 2H) 6.53 (s, 1H) 7.22 - 7.30 (m, 2H), 7.34 (s, 1H), 7.39 - 7.44 (m, 1H), 7.58 - 7.66 (m, 1H), 11.97 (br.s, 1H).

### Beispiel 37

### tert-Butyl-(3R)-3-[({7-[(2,6-difluorbenzyl)oxy]-2,5-dimethylpyrazolo[1,5-a]pyridin-3-yl}carbonyl)amino]-5-methylhexanoat

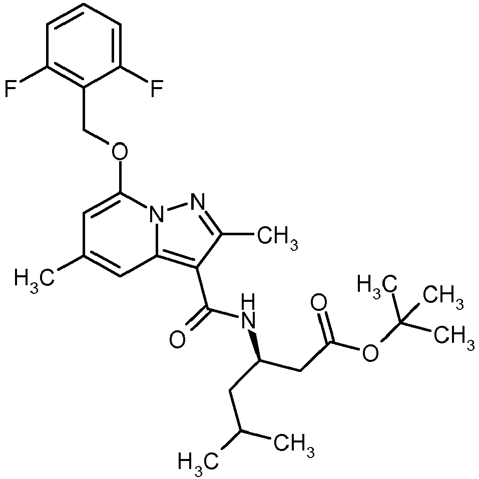

Eine Mischung von 100 mg (0.30 mmol, 1.0 eq.) 7-[(2,6-Difluorbenzyl)oxy]-2,5-dimethylpyrazolo[1,5-a]pyridin-3-carbonsäure aus Beispiel 4A, 116 mg TBTU (0.36 mmol, 1.2 eq.) und 0.132 ml (1.2 mmol, 4.0 eq.) 4-Methylmorpholin in 2.2 ml DMF wurden mit 121 mg (0.60 mmol, 2.0 eq.) tert-Butyl-(3R)-3-amino-5-methylhexanoat versetzt und das Gemisch wurde über Nacht bei RT gerührt. Das Reaktionsgemisch wurde mit Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonat-lösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 88 mg der Zielverbindung (57% d. Th.) erhalten.
LC-MS (Methode 2): Rₜ = 1.30 min
MS (ESpos): m/z = 516 (M+H)⁺

### Beispiel 38

### (3R)-3-[({7-[(2,6-Difluorbenzyl)oxy]-2,5-dimethylpyrazolo[1,5-a]pyridin-3-yl}carbonyl)amino]-5-methylhexansäure

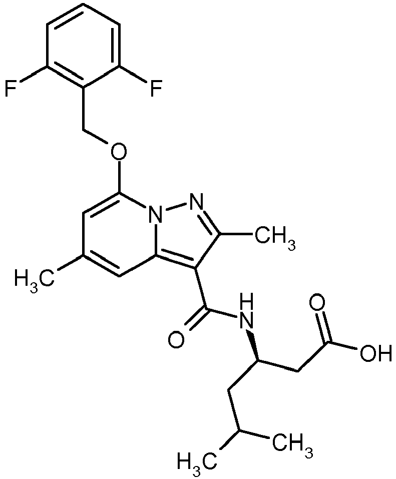

88 mg (0.17 mmol) tert-Butyl-(3R)-3-[({7-[(2,6-difluorbenzyl)oxy]-2,5-dimethylpyrazolo[1,5-a]pyridin-3-yl}carbonyl)amino]-5-methylhexanoat aus Beispiel 37 wurden in 2 ml Diethylether suspendiert und mit 2.56 ml 2 N Chlorwasserstoff-Lösung in Diethylether versetzt und das Gemisch wurde über Nacht bei RT gerührt. Anschließend wurden nochmals 2 ml 2 N Chlorwasserstoff-Lösung in Diethylether hinzugegeben und das Gemisch wurde 4 h bei RT gerührt. Das Reaktionsgemisch wurde eingedampft, mit Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, eingeengt und lyophilisiert. Es wurden 57 mg der Zielverbindung (73% d. Th.) erhalten.
LC-MS (Methode 2): Rₜ = 1.04 min
MS (ESpos): m/z = 460 (M+H)⁺
¹H NMR (500 MHz, DMSO-d₆) δ [ppm] = 0.90 (dd, 6H) 1.25 - 1.33 (m, 1H), 1.52 - 1.60 (m, 1H), 1.61 - 1.70 (m, 1H), 2.35 - 2.49 (m, 8H), 4.34 - 4.44 (m, 1H), 5.44 (s, 2H) 6.52 (s, 1H) 7.21 - 7.33 (m, 4H), 7.58 - 7.67 (m, 1H), 12.18 (br. s, 1H).

### Beispiel 39

### ent-N-(2-Amino-2-methylpentyl)-7-[(2,6-difluorbenzyl)oxy]-2,5-dimethylpyrazolo[1,5-a]pyridin-3-carboxamid (Enantiomer B)

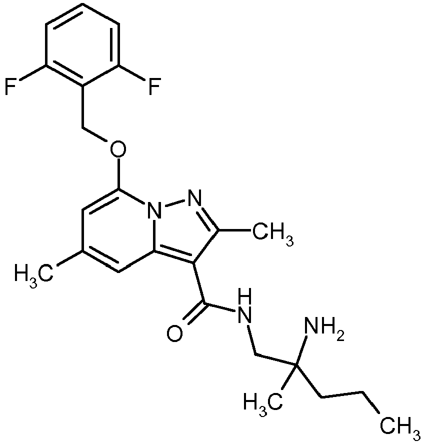

190 mg (0.26 mmol, Reinheit 92%) *ent*-Benzyl-{1-[({7-[(2,6-difluorbenzyl)oxy]-2,5-dimethylpyrazolo[1,5-a]pyridin-3-yl}carbonyl)amino]-2-methylpentan-2-yl}carbamat Trifluoracetat aus Beispiel 87A (Enantiomer B) wurden in 6.9 ml Ethanol gelöst, mit 8.6 mg (0.01 mmol) 10%igem Palladium auf Aktivkohle versetzt und 1.5 Stunden bei Normaldruck hydriert. Die Reaktionslösung wurde mittels Milliporfilter filtriert und das Filtrat einrotiert. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 79 mg der Zielverbindung (70% d. Th.) erhalten.
LC-MS (Methode 2): Rₜ = 0.74 min
MS (ESpos): m/z = 431 (M+H)⁺
¹H-NMR (400 MHz, DMSO-d₆): δ = 0.87 (t, 3H), 0.98 (s, 3H), 1.25 - 1.41 (m, 4H), 1.49 (br. s, 2H), 2.40 (s, 3H), 2.50 (s, 3H; unter Lösungsmittelpeak), 3.09 - 3.23 (m, 2H), 5.44 (s, 2H), 6.54 (s, 1H), 7.18 - 7.30 (m, 3H), 7.38 (s, 1H), 7.57 - 7.67 (m, 1H).

### Beispiel 40

### ent-N-(2-Amino-3-fluor-2-methylpropyl)-7-[(2,6-difluorbenzyl)oxy]-2,5-dimethylpyrazolo[1,5-a]pyridin-3-carboxamid (Enantiomer A)

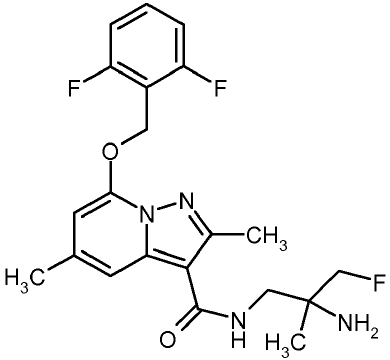

Unter Argon wurden 231 mg (0.33 mmol, 95%ig) *ent*-Benzyl-{1-[({7-[(2,6-difluorbenzyl)oxy]-2,5-dimethylpyrazolo[1,5-a]pyridin-3-yl}carbonyl)amino]-3-fluor-2-methylpropan-2-yl}carbamat Trifluoroacetat (Enantiomer A) aus Beispiel 88A in 8.5 ml Ethanol gelöst, mit 70 mg (0.07 mmol) Palladium auf Aktivkohle (10%ig) versetzt und die Reaktionsmischung wurde 2.5 Stunden bei Normaldruck hydriert. Das Reaktionsgemisch wurde über Celite abfiltriert, gut mit Ethanol gewaschen und das Filtrat anschließend eingeengt. Der Rückstand wurde mittels präparativer HPLC gereinigt (RP18 Säule, Laufmittel: Acetonitril/Wasser-Gradient unter Zusatz von 0.1% TFA). Die gesammelten Produktfraktionen wurden in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan reextrahiert. Die vereinigten organischen Phasen wurden über Natriumsulfat getrocknet, abfiltriert und eingeengt. Es wurden 90 mg der Zielverbindung (65% d. Th., Reinheit 99%) erhalten.
LC-MS (Methode 2): Rₜ = 0.67 min
MS (ESpos): m/z = 421 (M+H)⁺
¹H-NMR (500 MHz, DMSO-d₆): δ = 1.03 (s, 3H), 1.63 (br. s, 2H), 2.41 (s, 3H), 2.51 (s, 3H; überlagert mit Lösungsmittel-Peak), 3.20 - 3.36 (m, 2H; überlagert mit Wasser-Peak), 4.08 - 4.15 (m, 1H), 4.18 - 4.25 (m, 1H), 5.45 (s, 2H), 6.55 (s, 1H), 7.22 - 7.31 (m, 3H), 7.39 (s, 1H), 7.58 - 7.66 (m, 1H).

### Beispiel 41

### ent-N-(2-Amino-3-fluor-2-methylpropyl)-7-[(2,6-difluorbenzyl)oxy]-2,5-dimethylpyrazolo [1,5-a]pyridin-3-carboxamid (Enantiomer B)

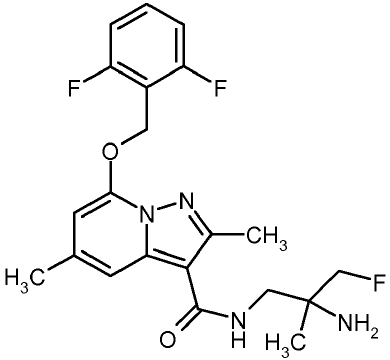

Unter Argon wurden 240 mg (0.27 mmol, 74%ig) *ent*-Benzyl-{1-[({7-[(2,6-difluorbenzyl)oxy]-2,5-dimethylpyrazolo[1,5-a]pyridin-3-yl}carbonyl)amino]-3-fluor-2-methylpropan-2-yl}carbamat Trifluoroacetat (Enantiomer B) aus Beispiel 89A in 6.8 ml Ethanol gelöst, mit 56 mg (0.05 mmol) Palladium auf Aktivkohle (10%ig) versetzt und die Reaktionsmischung wurde 8 Stunden bei Normaldruck hydriert. Das Reaktionsgemisch wurde über Celite abfiltriert, gut mit Ethanol gewaschen und das Filtrat anschließend eingeengt. Der Rückstand wurde in Acetonitril/Wasser aufgenommen, mit TFA versetzt und mittels präparativer HPLC gereinigt (RP18 Säule, Laufmittel: Acetonitril/Wasser-Gradient unter Zusatz von 0.1% TFA). Die gesammelten Produktfraktionen wurden eingeengt. Der Rückstand wurde in Dichlormethan und wenig Methanol aufgenommen und zweimal mit gesättigter, wässriger Natriumhydrogencarbonatlösung gewaschen. Die vereinigten wässrigen Phasen wurden zweimal mit Dichlormethan reextrahiert. Die vereinigten organischen Phasen wurden über Natriumsulfat getrocknet, abfiltriert und eingeengt. Es wurden 66 mg der Zielverbindung (59% d. Th., Reinheit 99%) erhalten.
LC-MS (Methode 2): Rₜ = 0.67 min
MS (ESpos): m/z = 421 (M+H)⁺
¹H-NMR (500 MHz, DMSO-d₆): δ = 1.03 (s, 3H), 1.68 (br. s, 2H), 2.41 (s, 3H), 2.51 (s, 3H; überlagert mit Lösungsmittel-Peak), 3.20 - 3.36 (m, 2H; überlagert mit Wasser-Peak), 4.08 - 4.15 (m, 1H), 4.18 - 4.25 (m, 1H), 5.44 (s, 2H), 6.55 (s, 1H), 7.22 - 7.31 (m, 3H), 7.39 (s, 1H), 7.58-7.67 (m, 1H).

### Beispiel 42

### ent-N-(2-Amino-5,5,5-trifluor-2-methylpentyl)-7-[(2,6-difluorbenzyl)oxy]-2,5-dimethylpyrazolo[1,5-a]pyridin-3-carboxamid (Enantiomer A)

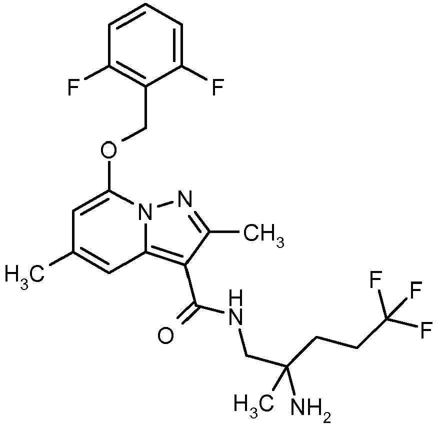

Eine Mischung von 52 mg (0.07 mmol) *ent*-Benzyl-{1-[({7-[(2,6-difluorbenzyl)oxy]-2,5-dimethylpyrazolo[1,5-a]pyridin-3-yl}carbonyl)amino]-5,5,5-trifluor-2-methylpentan-2-yl}carbamat Trifluoroacetat (Enantiomer A) aus Beispiel 90A und 15 mg Palladium auf Aktivkohle (10%ig) in 1.8 ml Ethanol wurde für 2 h bei Raumtemperatur und Normaldruck hydriert. Anschließend wurde über Celite abfiltriert, mit Ethanol gewaschen und das Filtrat eingeengt. Das Rohprodukt wurde mit Acetonitril/Wasser und TFA versetzt und mittels präparativer HPLC gereinigt (RP-C18, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 4 mg der Titelverbindung (12% d. Th.; Reinheit 99%) erhalten.
LC-MS (Methode 2): Rₜ = 0.77 min
MS (ESpos): m/z = 485 (M+H)⁺
¹H-NMR (500 MHz, DMSO-d₆): δ [ppm] = 1.02 (s, 3H), 1.46 - 1.58 (m, 2H), 1.67 (br. s, 2H), 2.24 - 2.47 (m, 5H), 2.50 (s, 3H; überlagert vom Lösungsmittelpeak), 3.14 - 3.25 (m, 2H), 5.44 (s, 2H), 6.54 (s, 1H), 7.21 - 7.30 (m, 2H), 7.34 (s, 1H), 7.40 (t, 1H), 7.58 - 7.66 (m, 1H).

### Beispiel 43

### ent-N-(2-Amino-5,5,5-trifluor-2-methylpentyl)-7-[(2,6-difluorbenzyl)oxy]-2,5-dimethylpyrazolo[1,5-a]pyridin-3-carboxamid (Enantiomer B)

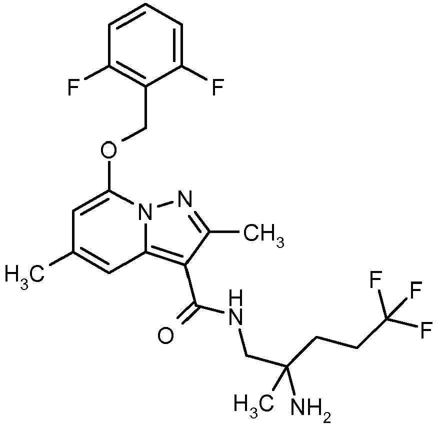

Eine Mischung von 311 mg (0.42 mmol) *ent*-Benzyl-{1-[({7-[(2,6-difluorbenzyl)oxy]-2,5-dimethylpyrazolo[1,5-a]pyridin-3-yl}carbonyl)amino]-5,5,5-trifluor-2-methylpentan-2-yl}carbamat Trifluoroacetat (Enantiomer B) aus Beispiel 91A und 13 mg Palladium auf Aktivkohle (10%ig) in 10.7 ml Ethanol wurde für 2.5 h bei Raumtemperatur und Normaldruck hydriert. Anschließend wurde über einen Millipore-Filter abfiltriert, mit Ethanol gewaschen und das Filtrat eingeengt. Das Rohprodukt wurde mit Acetonitril/Wasser und TFA versetzt und mittels präparativer HPLC gereinigt (RP-C18, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 159 mg der Titelverbindung (77% d. Th.; Reinheit 98%) erhalten.
LC-MS (Methode 2): Rₜ = 0.77 min
MS (ESpos): m/z = 485 (M+H)⁺
¹H-NMR (400 MHz, DMSO-d₆): δ [ppm] = 1.02 (s, 3H), 1.46 - 1.67 (m, 4H), 2.24 - 2.47 (m, 5H), 2.50 (s, 3H; überlagert vom Lösungsmittelpeak), 3.14 - 3.25 (m, 2H), 5.44 (s, 2H), 6.54 (s, 1H), 7.21 - 7.30 (m, 2H), 7.34 (s, 1H), 7.39 (t, 1H), 7.57 - 7.67 (m, 1H).
Enantiomer B: ca. 70% ee
Rt= 5.31 min [Daicel Chiralpak AZ-H, 5 µm, 250 x 4.6 mm; Eluent: 50% iso-Hexan, 50% Ethanol + 0.2% Diethylamin; Fluss 1.0 ml/min; Temperatur: 35°C; Detektion: 220 nm].

### Beispiel 44

### ent-N-(2-Amino-5-fluor-2-methylpentyl)-7-[(2,6-difluorbenzyl)oxy]-2,5-dimethylpyrazolo[1,5-a]pyridin-3-carboxamid (Enantiomer A)

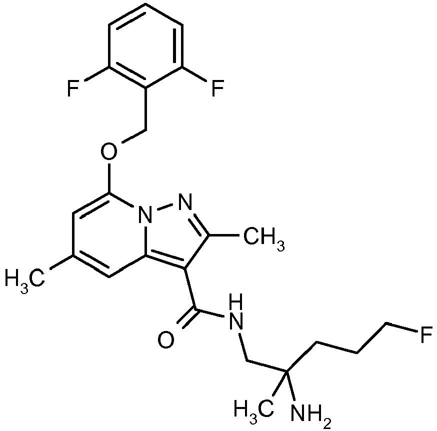

Eine Mischung von 82 mg (0.12 mmol) *ent*-Benzyl-{1-[({7-[(2,6-difluorbenzyl)oxy]-2,5-dimethylpyrazolo[1,5-a]pyridin-3-yl}carbonyl)amino]-5-fluor-2-methylpentan-2-yl}carbamat Trifluoracetat (Enantiomer A) aus Beispiel 92A und 3.7 mg Palladium auf Aktivkohle (10%ig) in 3 ml Ethanol wurde für 2 h bei Raumtemperatur und Normaldruck hydriert. Anschließend wurde über einen Millipore-Filter abfiltriert, mit Ethanol gewaschen und das Filtrat eingeengt. Das Rohprodukt wurde mit Acetonitril/Wasser und TFA versetzt und mittels präparativer HPLC gereinigt (RP-C18, Laufmittel: Acetonitril/Wasser-Gradient unter Zusatz von 0.1% TFA). Die Produktfraktionen wurden in Dichlormethan und wenig Methanol aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 39 mg der Titelverbindung (73% d. Th.; Reinheit 98%) erhalten.
LC-MS (Methode 2): Rₜ = 0.73 min
MS (ESpos): m/z = 449 (M+H)⁺
¹H-NMR (400 MHz, DMSO-d₆): δ [ppm] = 1.00 (s, 3H), 1.31 - 1.45 (m, 2H), 1.47 - 1.85 (m, 4H), 2.40 (s, 3H), 2.50 (s, 3H; überlagert vom Lösungsmittelpeak), 3.12 - 3.25 (m, 2H), 4.32 - 4.39 (m, 1H), 4.44 - 4.51 (m, 1H), 5.44 (s, 2H), 6.56 (s, 1H), 7.21 - 7.33 (m, 3H), 7.38 (s, 1H), 7.57 - 7.67 (m, 1H).

### Beispiel 45

### ent-N-(2-Amino-5-fluor-2-methylpentyl)-7-[(2,6-difluorbenzyl)oxy]-2,5-dimethylpyrazolo[1,5-a]pyridin-3-carboxamid (Enantiomer B)

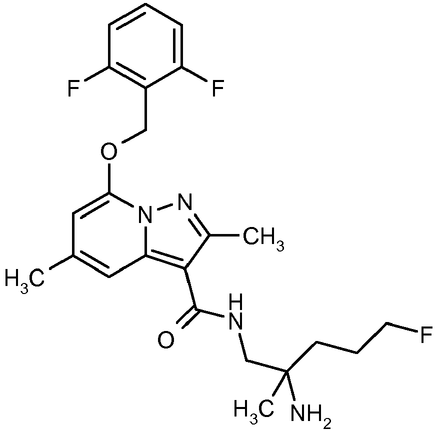

Eine Mischung von 109 mg (0.15 mmol; Reinheit 96%) *ent*-Benzyl-{1-[({7-[(2,6-difluorbenzyl)oxy]-2,5-dimethylpyrazolo[1,5-a]pyridin-3-yl}carbonyl)amino]-5-fluor-2-methylpentan-2-yl}carbamat (Enantiomer B) aus Beispiel 93A, 5 mg Palladium auf Aktivkohle (10%ig) in 4 ml Ethanol wurde für 6 h bei Raumtemperatur und Normaldruck hydriert. Anschließend wurde über einen Millipore-Filter abfiltriert, mit Ethanol gewaschen und das Filtrat eingeengt. Das Rohprodukt wurde mit Acetonitril/Wasser und TFA versetzt und mittels präparativer HPLC gereinigt (RP-C18, Laufmittel: Acetonitril/Wasser-Gradient unter Zusatz von 0.1% TFA). Die Produktfraktionen wurden in Dichlormethan und wenig Methanol aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 40 mg der Titelverbindung (58% d. Th.; Reinheit 97%) erhalten.
LC-MS (Methode 2): Rₜ = 0.73 min
MS (ESpos): m/z = 449 (M+H)⁺
¹H-NMR (500 MHz, DMSO-d₆): δ [ppm] = 1.00 (s, 3H), 1.32 - 1.44 (m, 2H), 1.47 - 1.84 (m, 4H), 2.40 (s, 3H), 2.50 (s, 3H; überlagert vom Lösungsmittelpeak), 3.12 - 3.24 (m, 2H), 4.34 - 4.39 (m, 1H), 4.44 - 4.49 (m, 1H), 5.44 (s, 2H), 6.56 (s, 1H), 7.22 - 7.32 (m, 3H), 7.38 (s, 1H), 7.58 - 7.66 (m, 1H).

### Beispiel 46

### ent-N-(2-Amino-4,4-difluor-2-methylbutyl)-7-[(2,6-difluorbenzyl)oxy]-2,5-dimethylpyrazolo[1,5-a]pyridin-3-carboxamid (Enantiomer A)

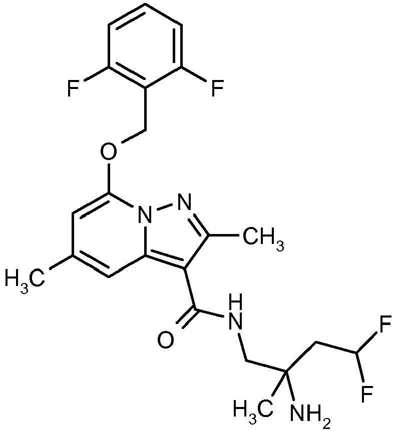

Eine Mischung von 157 mg (0.19 mmol; Reinheit 72%) *ent*-Benzyl-{1-[({7-[(2,6-difluorbenzyl)oxy]-2,5-dimethylpyrazolo[1,5-a]pyridin-3-yl}carbonyl)amino]-4,4-difluor-2-methylbutan-2-yl}carbamat (Enantiomer A) aus Beispiel 94A, 6.2 mg Palladium auf Aktivkohle (10%ig) und 22 µl TFA in 5 ml Ethanol wurde für 4.5 h bei Raumtemperatur und Normaldruck hydriert. Anschließend wurde über einen Millipore-Filter abfiltriert, mit Ethanol gewaschen und das Filtrat eingeengt. Das Rohprodukt wurde mit Acetonitril/Wasser und TFA versetzt und mittels präparativer HPLC gereinigt (RP-C18, Laufmittel: Acetonitril/Wasser-Gradient unter Zusatz von 0.1% TFA). Die Produktfraktionen wurden in Dichlormethan und wenig Methanol aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 58 mg der Titelverbindung (65% d. Th.; Reinheit 98%) erhalten.
LC-MS (Methode 2): Rₜ = 0.73 min
MS (ESpos): m/z = 453 (M+H)⁺
¹H-NMR (500 MHz, DMSO-d₆): δ [ppm] = 1.07 (s, 3H), 1.69 (br. s, 2H), 1.84 - 1.96 (m, 2H), 2.40 (s, 3H), 2.50 (s, 3H; überlagert vom Lösungsmittelpeak), 3.18 - 3.29 (m, 2H), 5.44 (s, 2H), 6.10 - 6.38 (m, 1H), 6.56 (s, 1H), 7.22 - 7.30 (m, 2H), 7.34 - 7.40 (m, 2H), 7.57 - 7.67 (m, 1H).

### Beispiel 47

### ent-N-(2-Amino-4,4-difluor-2-methylbutyl)-7-[(2,6-difluorbenzyl)oxy]-2,5-dimethylpyrazolo[1,5-a]pyridin-3-carboxamid (Enantiomer B)

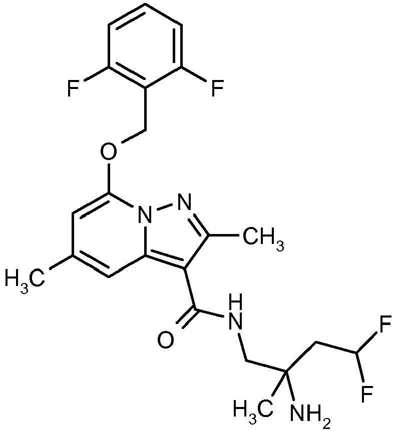

Eine Mischung von 281 mg (0.40 mmol; Reinheit 99%) *ent*-Benzyl-{1-[({7-[(2,6-difluorbenzyl)oxy]-2,5-dimethylpyrazolo[1,5-a]pyridin-3-yl}carbonyl)amino]-4,4-difluor-2-methylbutan-2-yl}carbamat Trifluoracetat (Enantiomer B) aus Beispiel 95A und 13 mg Palladium auf Aktivkohle (10%ig) in 10.2 ml Ethanol wurde für 3.5 h bei Raumtemperatur und Normaldruck hydriert. Anschließend wurde über einen Millipore-Filter abfiltriert, mit Ethanol gewaschen und das Filtrat eingeengt. Das Rohprodukt wurde mit Acetonitril/Wasser und TFA versetzt und mittels präparativer HPLC gereinigt (RP-C18, Laufmittel: Acetonitril/Wasser-Gradient unter Zusatz von 0.1% TFA). Die Produktfraktionen wurden in Dichlormethan und wenig Methanol aufgenommen und zweimal mit gesättigter, wässriger Natriumhydrogencarbonatlösung gewaschen. Die vereinten wässrigen Phasen wurden zweimal mit Dichlormethan extrahiert. Die vereinten organischen Phasen wurden über Natriumsulfat getrocknet, filtriert und eingedampft. Es wurden 145 mg der Titelverbindung (80% d. Th.; Reinheit 99%) erhalten.
LC-MS (Methode 2): Rₜ = 0.67 min
MS (ESpos): m/z = 453 (M+H)⁺
¹H-NMR (500 MHz, DMSO-d₆): δ [ppm] = 1.07 (s, 3H), 1.69 (br. s, 2H), 1.84 - 1.96 (m, 2H), 2.40 (s, 3H), 2.50 (s, 3H; überlagert vom Lösungsmittelpeak), 3.18 - 3.29 (m, 2H), 5.45 (s, 2H), 6.10 - 6.38 (m, 1H), 6.56 (s, 1H), 7.22 - 7.29 (m, 2H), 7.34 - 7.40 (m, 2H), 7.57 - 7.67 (m, 1H).

### Beispiel 48

### rac-N-(2-Amino-4-fluor-2-methylbutyl)-7-[(2,6-difluorbenzyl)oxy]-2,5-dimethylpyrazolo[1,5-a]pyridin-3-carboxamid

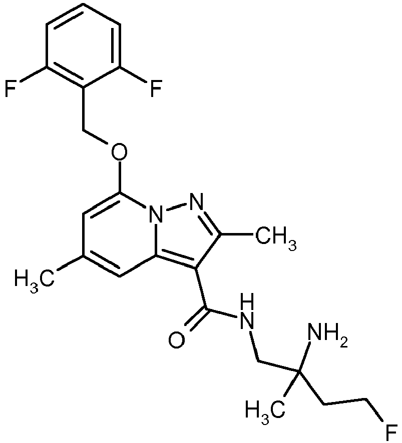

200 mg (0.45 mmol; Reinheit ca. 75%) 7-[(2,6-Difluorbenzyl)oxy]-5-methyl-2-propylpyrazolo[1,5-a]pyridin-3-carbonsäure aus Beispiel 4A wurden mit 274 mg (0.72 mmol) HATU und 0.42 ml (2.41 mmol) N,N-Diisopropylethylamin in 2.8 ml DMF vorgelegt und 20 min gerührt. Eine Lösung von 330 mg (1.27 mmol bei Annahme einer Reinheit von ca. 75%) *rac*-4-Fluor-2-methylbutan-1,2-diamin Dihydrochlorid aus Beispiel 86A in 1.4 ml DMF und und 0.63 ml (3.61 mmol) N,N-Diisopropylethylamin wurde zum ersten Reaktionsgemisch hinzugegeben und das Gemisch wurde 1 h bei RT gerührt. Die Reaktionslösung wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 75 mg der Zielverbindung (37% d. Th.; Reinheit 98%) erhalten.
LC-MS (Methode 2): Rₜ = 0.71 min
MS (ESpos): m/z = 435 (M+H)⁺
¹H-NMR (400 MHz, DMSO-d₆): δ = 1.03 (s, 3H), 1.48 - 1.83 (m, 4H), 2.40 (s, 3H), 2.50 (s, 3H; überlagert vom Lösungsmittelpeak), 3.14 - 3.28 (m, 2H), 4.55 - 4.62 (m, 1H), 4.66 - 4.73 (m, 1H), 5.45 (s, 2H), 6.55 (s, 1H), 7.20 - 7.32 (m, 3H), 7.38 (s, 1H), 7.58 - 7.67 (m, 1H).

### Beispiel 49

### ent-N-(2-Amino-4-fluor-2-methylbutyl)-7-[(2,6-difluorbenzyl)oxy]-2,5-dimethylpyrazolo[1,5-a]pyridin-3-carboxamid (Enantiomer A)

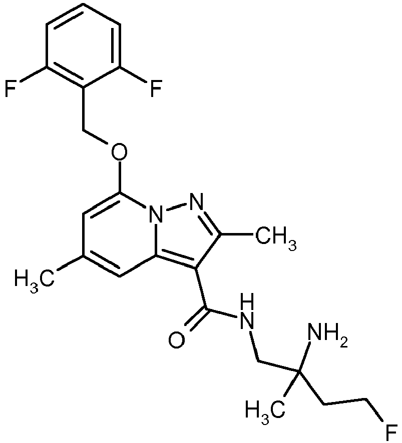

70 mg (0.16 mmol, Reinheit 98%) *rac*-N-(2-Amino-4-fluor-2-methylbutyl)-7-[(2,6-difluorbenzyl)oxy]-2,5-dimethylpyrazolo[1,5-a]pyridin-3-carboxamid aus Beispiel 48 wurden durch präparative Trennung an chiraler Phase in die Enantiomere getrennt [Säule: Daicel Chiralpak IF, 5 µm, 250 x 20 mm, Eluent: 100% Ethanol + 0.2% Diethylamin, Fluss: 15 ml/min; Temperatur: 45°C, Detektion: 220 nm].

Die Produktfraktionen wurden auf Trockeneis aufgefangen und eingedampft (Badtemperatur: 30°C) und lyophilisiert.
Enantiomer A: Ausbeute: 28 mg (>99% ee)
Rt= 6.76 min [Chiralpak AZ-H, 5 µm, 250 x 4.6 mm; Eluent: 100% Ethanol + 0.2% Diethylamin; Fluss: 1 ml/min; Detektion: 270 nm].

### Beispiel 50

### ent-N-(2-Amino-4-fluor-2-methylbutyl)-7-[(2,6-difluorbenzyl)oxy]-2,5-dimethylpyrazolo[1,5-a]pyridin-3-carboxamid (Enantiomer B)

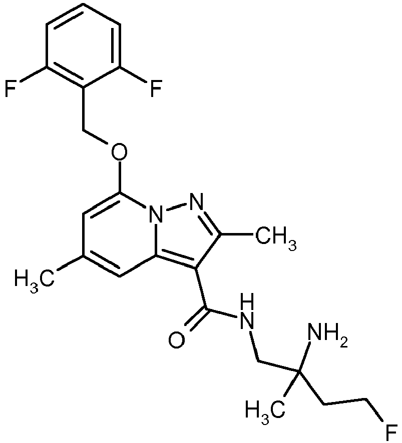

70 mg (0.16 mmol, Reinheit 98%) rac-N-(2-Amino-4-fluor-2-methylbutyl)-7-[(2,6-difluorbenzyl)oxy]-2,5-dimethylpyrazolo[1,5-a]pyridin-3-carboxamid aus Beispiel 48 wurden durch präparative Trennung an chiraler Phase in die Enantiomere getrennt [Säule: Daicel Chiralpak IF, 5 µm, 250 x 20 mm, Eluent: 100% Ethanol + 0.2% Diethylamin, Fluss: 15 ml/min; Temperatur: 45°C, Detektion: 220 nm].

Die Produktfraktionen wurden auf Trockeneis aufgefangen und eingedampft (Badtemperatur: 30°C) und lyophilisiert.
Enantiomer B: Ausbeute: 33 mg (94% ee)
Rt= 7.84 min [Chiralpak AZ-H, 5 µm, 250 x 4.6 mm; Eluent: 100% Ethanol + 0.2% Diethylamin; Fluss: 1 ml/min; Detektion: 270 nm].

### Beispiel 51

### ent-N-(2-Amino-2-methylpentyl)-7-[(2,6-difluorbenzyl)oxy]-5-methyl-2-propylpyrazolo[1,5-a]pyridin-3-carboxamid (Enantiomer B)

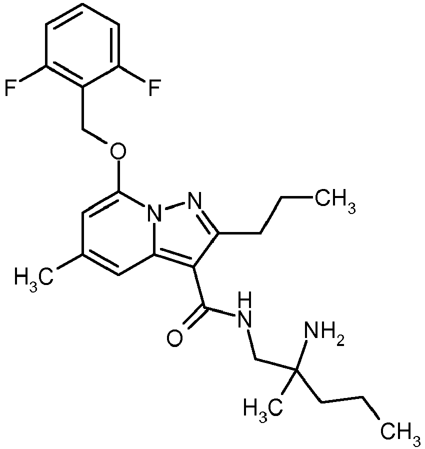

54 mg (0.08 mmol) *ent*-Benzyl-{1-[({7-[(2,6-difluorbenzyl)oxy]-5-methyl-2-propylpyrazolo[1,5-a]pyridin-3-yl}carbonyl)amino]-2-methylpentan-2-yl}carbamat Trifluoracetat (Enantiomer B) aus Beispiel 104A wurden in 2 ml Ethanol gelöst, mit 4 mg 10%igem Palladium auf Aktivkohle versetzt und 0.5 Stunden bei Normaldruck hydriert. Die Reaktionsmischung wurde mittels Milliporefilter filtriert und das Filtrat einrotiert. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 20 mg der Zielverbindung (56% d. Th.) erhalten.
LC-MS (Methode 2): Rₜ = 0.79 min
MS (ESpos): m/z = 459 (M+H)⁺

In Analogie zu Beispiel 51 wurden die in Tabelle 2 gezeigten Beispielverbindungen hergestellt, indem die geschützten Cbz-Amine mit 10%igem Palladium auf Aktivkohle (0.03 - 0.1 Äquivalente) unter den beschriebenen Reaktionsbedingungen (Reaktionszeit: 1 - 6 h; Temperatur: RT; Normaldruck) hydriert wurden.

Beispielhafte Aufarbeitung der Reaktionsmischung:
Das Reaktionsgemisch wurde mit Wasser, TFA oder Ameisensäure verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Dickschichtchromatographie oder Kieselgelchromatographie gereinigt (Laufmittel: Dichlormethan/Methanol oder Dichlormethan/2 M Ammoniak in Methanol). Die produkthaltigen Fraktionen wurden eingedampft.

Der Rückstand wurde ggf. in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

**Tabelle 2:**

| **Beispiel** | **IUPAC-Name / Struktur (Ausbeute)** | **Analytische Daten** |
|---|---|---|
| **52** | *ent*-N-(2-Amino-2-methylpentyl)-7-[(2,6-difluorbenzyl)oxy]-5-methyl-2-(trifluormethyl)pyrazolo[1,5-a]pyridin-3-carboxamid Trifluoracetat (Enantiomer B) | LC-MS (Methode 2): Rₜ = 0.83 min MS (ESpos): m/z = 485 (M+H)⁺ |
| | 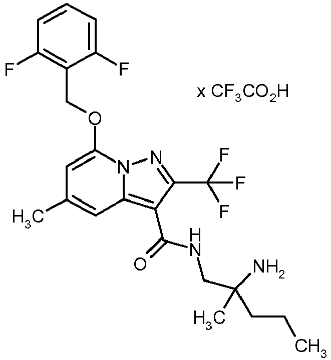 | |
| | (28% d. Th.) | |
| **53** | *ent*-N-(2-Amino-2-methylpentyl)-7-[(2,6-difluorbenzyl)oxy]-5-methylpyrazolo[1,5-a]pyridin-3-carboxamid (Enantiomer B) | LC-MS (Methode 2): Rₜ = 0.74 min MS (ESpos): m/z = 417 (M+H)⁺ |
| | 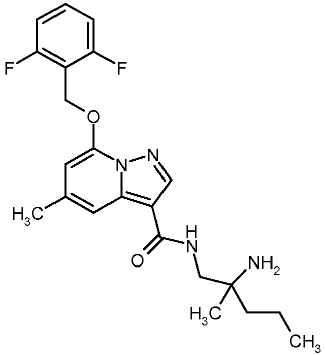 | |
| | (60% d. Th.) | |

### Beispiel 54

### ent-N-(2-Amino-2-methylbutyl)-2-cyclopropyl-7-[(2,6-difluorbenzyl)oxy]-5-methylpyrazolo[1,5-a]pyridin-3-carboxamid (Enantiomer A)

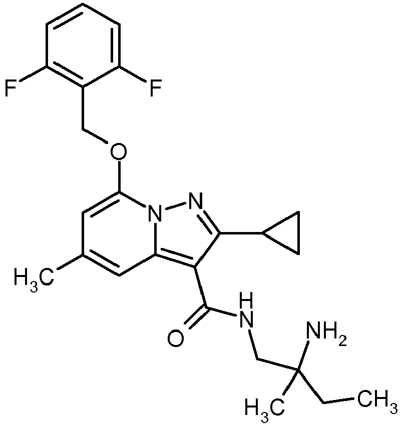

57 mg (0.07 mmol; Reinheit 90%) *ent*-Benzyl-{1-[({2-cyclopropyl-7-[(2,6-difluorbenzyl)oxy]-5-methylpyrazolo[1,5-a]pyridin-3-yl}carbonyl)amino]-2-methylbutan-2-yl}carbamat Trifluoracetat (Enantiomer A) aus Beispiel 107A wurden in 2.1 ml Ethanol gelöst, mit 3 mg 10%igem Palladium auf Aktivkohle versetzt und 3.5 Stunden bei Normaldruck hydriert. Die Reaktionsmischung wurde mittels Milliporefilter filtriert und das Filtrat einrotiert. Der Rückstand wurde in 2.1 ml Ethanol gelöst, mit 13 µl TFA und 3 mg 10%igem Palladium auf Aktivkohle versetzt und 1 Stunde bei Normaldruck hydriert. Die Reaktionsmischung wurde mittels Milliporefilter filtriert und das Filtrat einrotiert. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 26 mg der Zielverbindung (79% d. Th.) erhalten.
LC-MS (Methode 2): Rₜ = 0.75 min
MS (ESpos): m/z = 443 (M+H)⁺
¹H-NMR (500 MHz, DMSO-d₆): δ = 0.83 - 0.93 (m, 5H), 0.95 - 1.02 (m, 5H), 1.29 - 1.43 (m, 2H), 1.50 (br. s, 2H), 2.31 - 2.42 (m, 4H), 3.13 - 3.25 (m, 2H), 5.44 (s, 2H), 6.53 (s, 1H), 7.21 - 7.28 (m, 2H), 7.36 (t, 1H), 7.47 (s, 1H), 7.57 - 7.64 (m, 1H).

### Beispiel 55

### ent-N-(2-Amino-2-methylpentyl)-2-cyclopropyl-7-[(2,6-difluorbenzyl)oxy]-5-methylpyrazolo[1,5-a]pyridin-3-carboxamid (Enantiomer B)

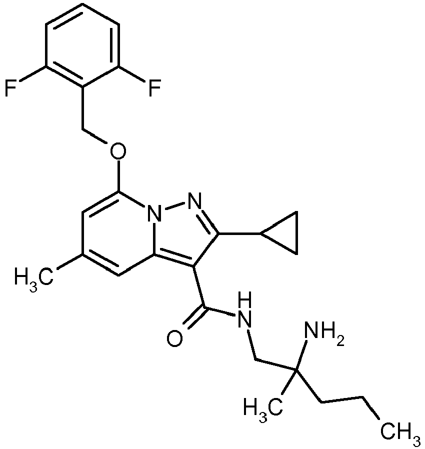

88 mg (0.12 mmol) *ent*-Benzyl-{1-[({2-cyclopropyl-7-[(2,6-difluorbenzyl)oxy]-5-methylpyrazolo[1,5-a]pyridin-3-yl}carbonyl)amino]-2-methylpentan-2-yl}carbamat Trifluoracetat (Enantiomer B) aus Beispiel 108A wurden in 3.2 ml Ethanol gelöst, mit 4 mg 10%igem Palladium auf Aktivkohle versetzt und 2 Stunden bei Normaldruck hydriert. Die Reaktionsmischung wurde mittels Milliporefilter filtriert und das Filtrat einrotiert. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 41 mg der Zielverbindung (72% d. Th.) erhalten.
LC-MS (Methode 2): Rₜ = 0.78 min
MS (ESpos): m/z = 457 (M+H)⁺
¹H-NMR (500 MHz, DMSO-d₆): δ = 0.83 - 0.93 (m, 5H), 0.96 - 1.02 (m, 5H), 1.25 - 1.40 (m, 4H), 1.70 (br. s, 2H), 2.32 - 2.42 (m, 4H), 3.12 - 3.25 (m, 2H), 5.44 (s, 2H), 6.53 (s, 1H), 7.21 - 7.29 (m, 2H), 7.39 (t, 1H), 7.45 (s, 1H), 7.57 - 7.65 (m, 1H).

### Beispiel 56

### ent-N-(2-Amino-4,4-difluor-2-methylbutyl)-2-cyclopropyl-7-[(2,6-difluorbenzyl)oxy]-5-methylpyrazolo[1,5-a]pyridin-3-carboxamid (Enantiomer A)

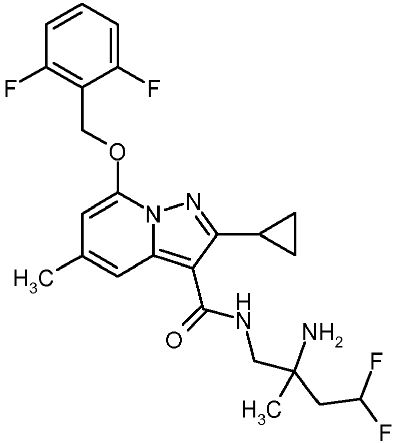

55 mg (0.07 mmol; Reinheit 95%) *ent*-Benzyl-{1-[({2-cyclopropyl-7-[(2,6-difluorbenzyl)oxy]-5-methylpyrazolo[1,5-a]pyridin-3-yl}carbonyl)amino]-4,4-difluor-2-methylbutan-2-yl}carbamat Trifluoracetat (Enantiomer A) aus Beispiel 110A wurden in 1.8 ml Ethanol gelöst, mit 2.3 mg 10%igem Palladium auf Aktivkohle versetzt und 3 Stunden bei Normaldruck hydriert. Die Reaktionsmischung wurde mittels Milliporefilter filtriert. Es wurden mit 20 µl (0.22 mmol) TFA und 23 mg 10%igem Palladium auf Aktivkohle versetzt und 1.5 Stunden bei Normaldruck hydriert. Die Reaktionsmischung wurde mittels Milliporefilter filtriert und das Filtrat eingedampft. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingedampft und lyophilisiert. Es wurden 30 mg der Zielverbindung (85% d. Th.) erhalten.
LC-MS (Methode 2): Rₜ = 0.79 min
MS (ESpos): m/z = 479 (M+H)⁺
¹H-NMR (400 MHz, DMSO-d₆): δ [ppm] = 0.86 - 0.93 (m, 2H), 0.94 - 1.01 (m, 2H), 1.08 (s, 3H), 1.70 (br. s, 2H), 1.85 - 1.99 (m, 2H), 2.34 - 2.43 (m, 4H), 3.20 - 3.29 (m, 2H; z. T. überlagert mit Lösungsmittelpeak), 5.45 (s, 2H), 6.08 - 6.42 (m, 1H), 6.53 (s, 1H), 7.19 - 7.29 (m, 2H), 7.42 (s, 1H), 7.49 (t, 1H), 7.55 - 7.65 (m, 1H).

### Beispiel 57

### ent-N-(2-Amino-4,4-difluor-2-methylbutyl)-2-cyclopropyl-7-[(2,6-difluorbenzyl)oxy]-5-methylpyrazolo[1,5-a]pyridin-3-carboxamid (Enantiomer B)

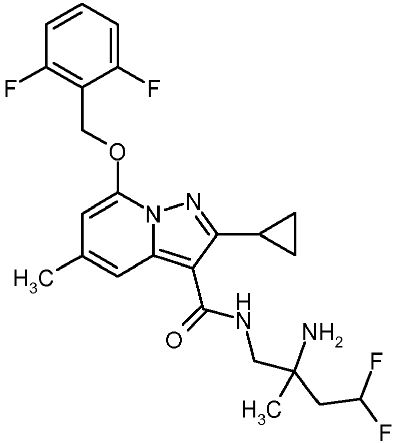

54 mg (0.06 mmol; Reinheit 82%) *ent*-Benzyl-{1-[({2-cyclopropyl-7-[(2,6-difluorbenzyl)oxy]-5-methylpyrazolo[1,5-a]pyridin-3-yl}carbonyl)amino]-4,4-difluor-2-methylbutan-2-yl}carbamat Trifluoracetat (Enantiomer B) aus Beispiel 111A wurden in 1.6 ml Ethanol gelöst, mit 2 mg 10%igem Palladium auf Aktivkohle versetzt und 4.5 Stunden bei Normaldruck hydriert. Die Reaktionsmischung wurde mittels Milliporefilter filtriert. Es wurden mit 5 µl (0.06 mmol) TFA und 2 mg 10%igem Palladium auf Aktivkohle versetzt und 1 Stunden bei Normaldruck hydriert. Die Reaktionsmischung wurde mittels Milliporefilter filtriert und das Filtrat eingedampft. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingedampft und lyophilisiert. Es wurden 17 mg der Zielverbindung (58% d. Th.) erhalten.
LC-MS (Methode 2): Rₜ = 0.79 min
MS (ESpos): m/z = 479 (M+H)⁺
¹H-NMR (400 MHz, DMSO-d₆): δ [ppm] = 0.87 - 0.93 (m, 2H), 0.94 - 1.01 (m, 2H), 1.08 (s, 3H), 1.72 (br. s, 2H), 1.86 - 1.99 (m, 2H), 2.34 - 2.43 (m, 4H), 3.20 - 3.29 (m, 2H; z. T. überlagert mit Lösungsmittelpeak), 5.45 (s, 2H), 6.08 - 6.42 (m, 1H), 6.53 (s, 1H), 7.20 - 7.29 (m, 2H), 7.42 (s, 1H), 7.49 (t, 1H), 7.55 - 7.65 (m, 1H).

### Beispiel 58

### ent-N-(2-Amino-5,5,5-trifluor-2-methylpentyl)-2-cyclopropyl-7-[(2,6-difluorbenzyl)oxy]-5-methylpyrazolo[1,5-a]pyridin-3-carboxamid (Enantiomer B)

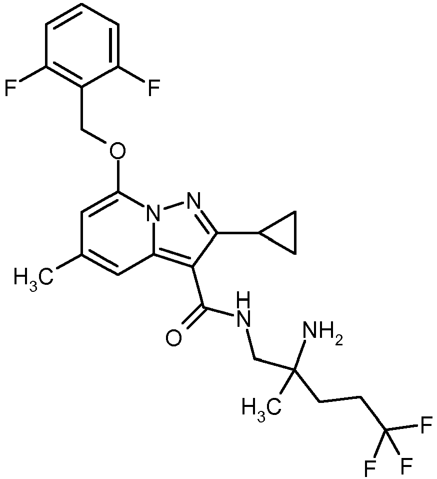

97 mg (0.13 mmol; Reinheit 82%) *ent*-Benzyl-{1-[({2-cyclopropyl-7-[(2,6-difluorbenzyl)oxy]-5-methylpyrazolo[1,5-a]pyridin-3-yl}carbonyl)amino]-5,5,5-trifluor-2-methylpentan-2-yl}carbamat Trifluoracetat (Enantiomer B) aus Beispiel 109A wurden in 3.2 ml Ethanol gelöst, mit 4 mg 10%igem Palladium auf Aktivkohle versetzt und 4.5 Stunden bei Normaldruck hydriert. Die Reaktionsmischung wurde mittels Milliporefilter filtriert und das Filtrat eingedampft. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingedampft und lyophilisiert. Es wurden 51 mg der Zielverbindung (78% d. Th.) erhalten.
LC-MS (Methode 2): Rₜ = 0.80 min
MS (ESpos): m/z = 511 (M+H)⁺
¹H-NMR (400 MHz, DMSO-d₆): δ [ppm] = 0.87 - 0.92 (m, 2H), 0.93 - 0.99 (m, 2H), 1.02 (s, 3H), 1.48 - 1.64 (m, 4H), 2.24 - 2.45 (m, 6H), 3.17 - 3.28 (m, 2H), 5.44 (s, 2H), 6.08 - 6.42 (m, 1H), 6.52 (s, 1H), 7.20 - 7.29 (m, 2H), 7.39 (s, 1H), 7.50 (t, 1H), 7.56 - 7.65 (m, 1H).

### Beispiel 59

### rac-N-(2-Amino-2-cyanethyl)-7-[(2,6-difluorbenzyl)oxy]-2,5-dimethylpyrazolo[1,5-a]pyridin-3-carboxamid

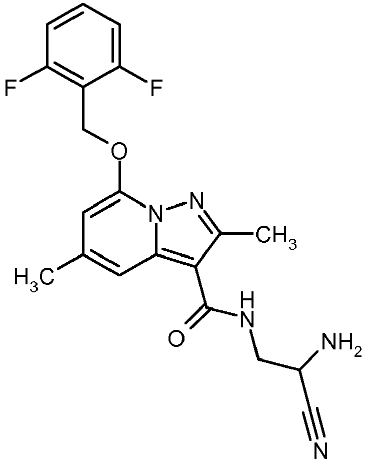

80 mg (0.18 mmol; ca. 75%ige Reinheit) 7-[(2,6-Difluorbenzyl)oxy]-2,5-dimethylpyrazolo[1,5-a]pyridin-3-carbonsäure aus Beispiel 4A wurden mit 76 mg (0.20 mmol) HATU und 0.21 ml (1.17 mmol) N,N-Diisopropylethylamin in 0.74 ml DMF vorgelegt und 10 min bei Raumtemperatur gerührt. Anschließend wurden 33 mg (0.21 mmol) *rac*-2,3-Diaminopropanonitril-Dihydrochlorid zur Reaktionslösung bei 0°Cgegeben und das Gemisch wurde über Nacht bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, und eingedampft. Es wurden 55 mg der Zielverbindung (75% d. Th.) erhalten.
LC-MS (Methode 2): Rₜ = 0.76 min
MS (ESpos): m/z = 400 (M+H)⁺
¹H-NMR (400 MHz, DMSO-d₆): δ = 2.40 (s, 3H), 2.42 - 2.54 (m, 5H; überlagert mit Lösungsmittelpeak), 3.39 - 3.55 (m, 2H), 3.91 - 4.04 (m, 1H), 5.44 (s, 2H), 6.57 (s, 1H), 7.19 - 7.30 (m, 2H), 7.38 (s, 1H), 7.57 - 7.67 (m, 1H), 7.72 (t, 1H).

In Analogie zu Beispiel 59 wurden die in Tabelle 3 gezeigten Beispielverbindungen hergestellt, indem 7-[(2,6-Difluorbenzyl)oxy]-2,5-dimethylpyrazolo[1,5-a]pyridin-3-carbonsäure aus Beispiel 4A mit den entsprechenden, kommerziell erhältlichen oder zuvor beschriebenen Aminen [Hydrazine, Hydrazide, Hydroxylamine] (1.1 - 5 Äquivalente), HATU (1.1 - 4.5 Äquivalente) und *N,N-*Diisopropylethylamin (3 - 12 Äquivalente) in DMF oder in DMF/Dichlormethan (1/1) unter den beschriebenen Reaktionsbedingungen (Reaktionszeit: 1 - 48 h; Temperatur: 0°C - RT, -20°C, RT oder 60°C) umgesetzt wurden.

Beispielhafte Aufarbeitung der Reaktionsmischung:
Die Reaktionslösung wurde mit Wasser versetzt und der ausgefallene Feststoff ca. 30 min bei Raumtemperatur verrührt. Anschließend wurde der Feststoff abfiltriert, gut mit Wasser gewaschen und im Hochvakuum getrocknet.

Alternativ dazu wurde das Reaktionsgemisch mit Wasser, TFA oder Ameisensäure verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Dickschichtchromatographie oder Kieselgelchromatographie gereinigt (Laufmittel: Dichlormethan/Methanol oder Dichlormethan/2 M Ammoniak in Methanol). Die produkthaltigen Fraktionen wurden eingedampft.

Die Produktfraktionen der Reinigungen wurden ggf. in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

**Tabelle 3:**

| **Beispiel** | **IUPAC-Name / Struktur (Ausbeute)** | **Analytische Daten** |
|---|---|---|
| **60** | N-[(3-Aminooxetan-3-yl)methyl]-7-[(2,6-difluorbenzyl)oxy]-2,5-dimethylpyrazolo[1,5-a]pyridin-3-carboxamid | LC-MS (Methode 2): Rₜ = 0.65 min MS (ESpos): m/z = 417 (M+H)⁺ |
| | 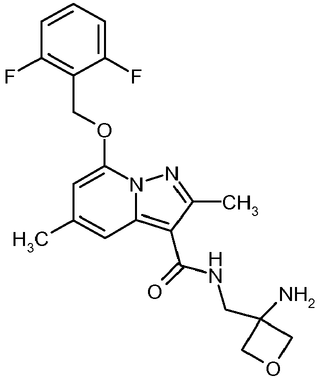 | |
| | (23% d. Th.) | |
| **61** | 7-[(2,6-Difluorbenzyl)oxy]-2,5-dimethyl-N-(2-sulfamoylethyl)pyrazolo[1,5-a]pyridin-3-carboxamid | LC-MS (Methode 2): Rₜ = 0.83 min MS (ESpos): m/z = 439 (M+H)⁺ |
| | 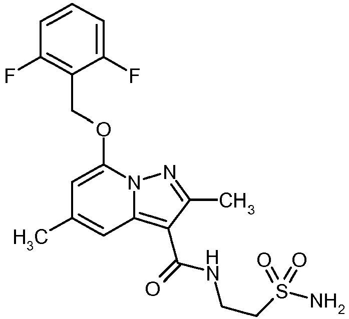 | |
| | (39% d. Th.) | |
| **62** | N-[(4-Cyancyclohexyl)methyl]-7-[(2,6-difluorbenzyl)oxy]-2,5-dimethylpyrazolo[1,5-a]pyridin-3-carboxamid (cis-/trans-Gemisch) | LC-MS (Methode 2): Rₜ = 1.02 min MS (ESpos): m/z = 453 (M+H)⁺ |
| | 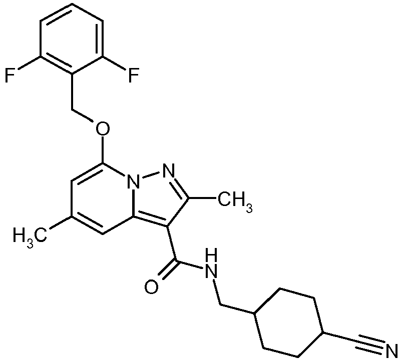 | |
| | (50% d. Th.) | |
| **63** | N-(5-Cyanpentyl)-7-[(2,6-difluorbenzyl)oxy]-2,5-dimethylpyrazolo[1,5-a]pyridin-3-carboxamid | LC-MS (Methode 2): Rₜ = 0.97 min MS (ESpos): m/z = 427 (M+H)⁺ |
| | 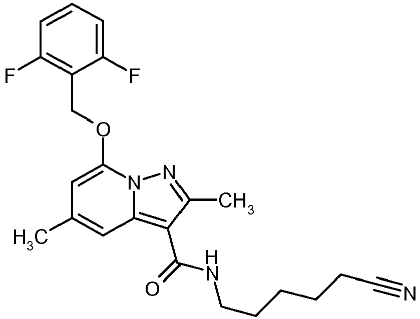 | |
| | (85% d. Th.) | |
| **64** | 7-[(2,6-Difluorbenzyl)oxy]-N-(2-hydroxyethoxy)-2,5-dimethylpyrazolo[1,5-a]pyridin-3-carboxamid Trifluoracetat | LC-MS (Methode 2): Rₜ = 0.80 min MS (ESpos): m/z = 392 (M-TFA+H)⁺ |
| | 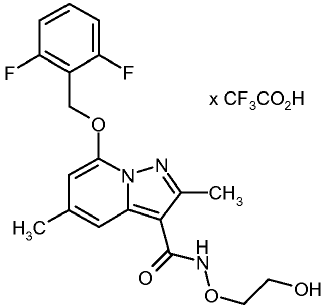 (13% d. Th.) | |
| **65** | N-(Allyloxy)-7-[(2,6-difluorbenzyl)oxy]-2,5-dimethylpyrazolo[1,5-a]pyridin-3-carboxamid Trifluoracetat | LC-MS (Methode 2): Rₜ = 0.95 min MS (ESpos): m/z = 388 (M-TFA+H)⁺ |
| | 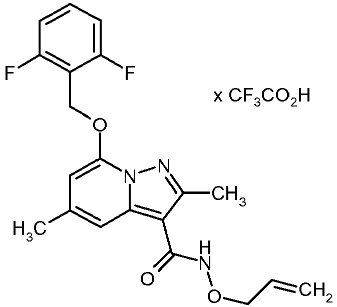 | |
| | (25% d. Th.; Reinheit 94%) | |
| **66** | 7-[(2,6-Difluorbenzyl)oxy]-N'-(4-fluorphenyl)-2,5-dimethylpyrazolo[1,5-a]pyridin-3-carbohydrazid Trifluoracetat | LC-MS (Methode 7): Rₜ = 2.54 min MS (ESpos): m/z = 441 (M-TFA+H)⁺ |
| | 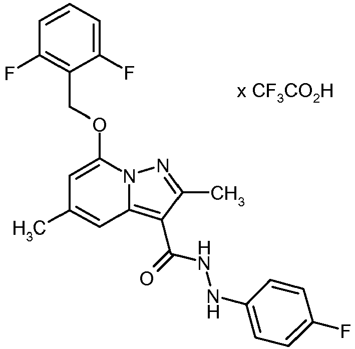 | |
| | (3% d. Th.; Reinheit 90%) ¹⁾ | |
| **67** | 7-[(2,6-Difluorbenzyl)oxy]-N'-[(dimethylamino)acetyl]-2,5-dimethylpyrazolo[1,5-a]pyridin-3-carbohydrazid | LC-MS (Methode 2): Rₜ = 0.62 min MS (ESpos): m/z = 432 (M+H)^{+ 1}H NMR (500 MHz, DMSO-d₆) δ = 2.28 (s, 6H), 2.41 (s, 3H), 2.51 (s, 3H; überlagert vom Lösungsmittelpeak), 3.01 (s, 2H), 5.47 (s, 2H), 6.58 (s, 1H), 7.21 - 7.30 (m, 2H), 7.38 (s, 1H), 7.57 - 7.67 (m, 1H), 9.33 (s, 1H), 9.63 (s, 1H). |
| | 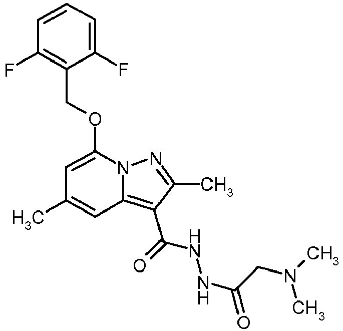 | |
| | (49% d. Th.) | |
| **68** | 7-[(2,6-Difluorbenzyl)oxy]-N'-(2-hydroxycyclopentyl)-2,5-dimethylpyrazolo[1,5-a]pyridin-3-carbohydrazid Trifluoracetat (Mischung von Stereoisomeren) | LC-MS (Methode 2): Rₜ = 0.83 min MS (ESpos): m/z = 431 (M-TFA+H)⁺ |
| | 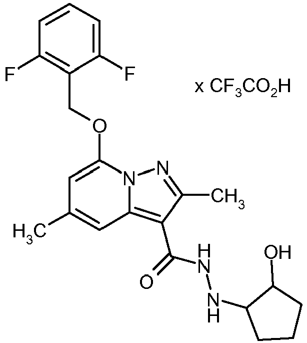 | |
| | (8% d. Th.) | |
| **69** | *rel*-N-[(3R,4S)-4-Aminotetrahydrofuran-3-yl]-7-[(2,6-difluorbenzyl)oxy]-2,5-dimethylpyrazolo[1,5-a]pyridin-3-carboxamid (*cis*-Stereoisomere) | LC-MS (Methode 2): Rₜ = 0.67 min MS (ESpos): m/z = 417 (M+H)⁺ |
| | 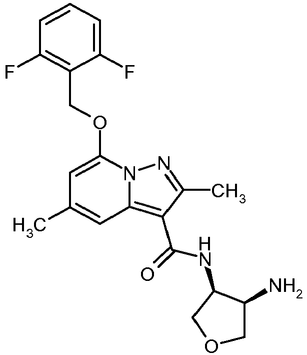 | |
| | (40% d. Th.) | |
| **70** | N-(1-Amino-2,3-dihydro-1H-inden-2-yl)-7-[(2,6-difluorbenzyl)oxy]-2,5-dimethylpyrazolo[1,5-a]pyridin-3-carboxamid (Mischung von Stereoisomeren) | LC-MS (Methode 7): Rₜ = 2.41 und 2.45 min MS (ESpos): m/z = 463 (M+H)⁺ |
| | 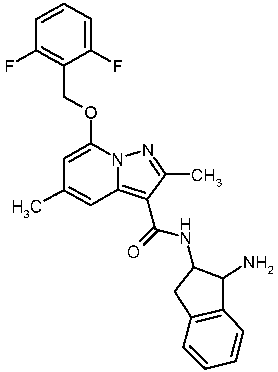 | |
| | (73% d. Th.) | |
| **71** | 7-[(2,6-Difluorbenzyl)oxy]-N-[(1S,2R)-2-hydroxy-2,3-dihydro-1H-inden-1-yl]-2,5-dimethylpyrazolo[1,5-a]pyridin-3-carboxamid Trifluoracetat | LC-MS (Methode 2): Rₜ = 1.03 min MS (ESpos): m/z = 464 (M-TFA+H)⁺ |
| | 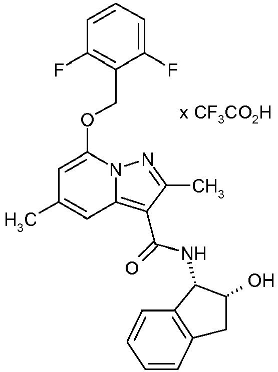 | |
| | (41% d. Th.) | |
| **72** | rac-N- {2-Amino-2-[3-(2-hydroxyethoxy)phenyl]ethyl}-7-[(2,6-difluorbenzyl)oxy]-2,5-dimethylpyrazolo[1,5-a]pyridin-3-carboxamid Trifluoracetat | LC-MS (Methode 2): Rₜ = 0.71 min MS (ESpos): m/z = 511 (M-TFA+H)⁺ |
| | 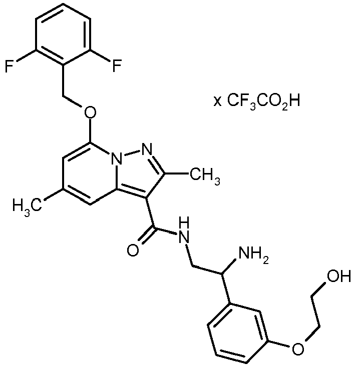 | |
| | (23% d. Th.; Reinheit 92%) | |
| **73** | *rac*-N-[2-Amino-2-(2-methyl-1,3-thiazol-4-yl)ethyl]-7-[(2,6-difluorbenzyl)oxy]-2,5-dimethylpyrazolo[1,5-a]pyridin-3-carboxamid Trifluoracetat | LC-MS (Methode 2): Rₜ = 0.72 min MS (ESpos): m/z = 472 (M-TFA+H)⁺ |
| | 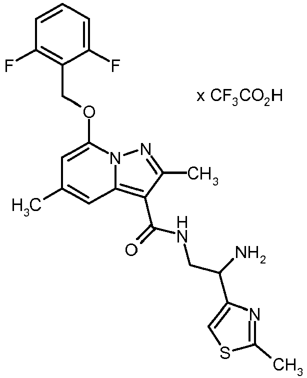 | |
| | (24% d. Th.) | |
| **74** | *rac*-7-[(2,6-Difluorbenzyl)oxy]-N-(3,4-dihydro-2H-pyrano[2,3-b]pyridin-4-yl)-2,5-dimethylpyrazolo[1,5-a]pyridin-3-carboxamid Trifluoracetat | LC-MS (Methode 2): Rₜ = 0.92 min MS (ESpos): m/z = 465 (M-TFA+H)⁺ |
| | 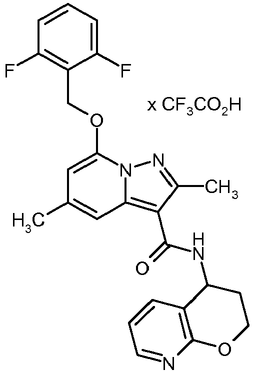 (62% d. Th.) | |
| **75** | *rac*-7-[(2,6-Difluorbenzyl)oxy]-2,5-dimethyl-N-(1-methyl-1,2,3,4-tetrahydrochinolin-4-yl)pyrazolo[1,5-a]pyridin-3-carboxamid Trifluoracetat | LC-MS (Methode 2): Rₜ = 1.16 min MS (ESpos): m/z = 477 (M-TFA+H)⁺ |
| | 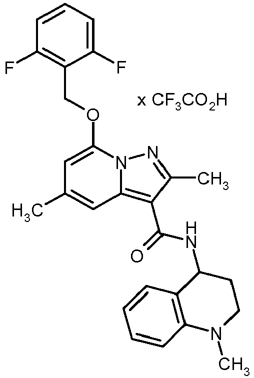 | |
| | (58% d. Th.; Reinheit 96%) | |
| **76** | *rac*-7-[(2,6-Difluorbenzyl)oxy]-2,5-dimethyl-N-(2-oxo-1,2,3,4-tetrahydrochinolin-4-yl)pyrazolo[1,5-a]pyridin-3-carboxamid Trifluoracetat | LC-MS (Methode 2): Rₜ = 0.90 min MS (ESpos): m/z = 477 (M-TFA+H)⁺ |
| | 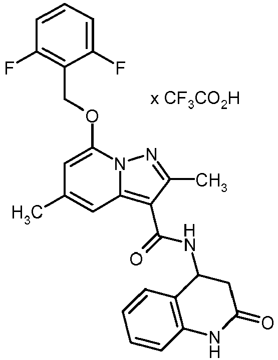 | |
| | (48% d. Th.) | |
| **77** | *rac*-7-[(2,6-Difluorbenzyl)oxy]-2,5-dimethyl-N-(1-methyl-2-oxo-1,2,3,4-tetrahydrochinolin-4-yl)pyrazolo[1,5-a]pyridin-3-carboxamid Trifluoracetat | LC-MS (Methode 2): Rₜ = 1.00 min MS (ESpos): m/z = 491 (M-TFA+H)⁺ |
| | 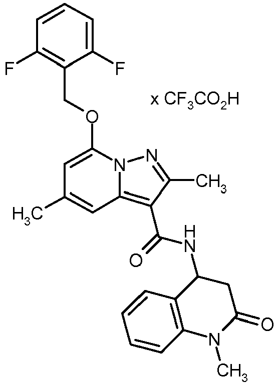 | |
| | (50% d. Th.) | |
| **78** | *rac*-N-[2-Amino-4-(benzyloxy)-2-methylbutyl]-7-[(2,6-difluorbenzyl)oxy]-2,5-dimethylpyrazolo[1,5-a]pyridin-3-carboxamid | LC-MS (Methode 2): Rₜ = 0.85 min MS (ESpos): m/z = 523 (M+H)⁺ |
| | 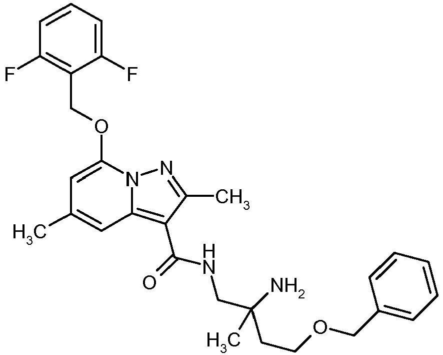 | |
| | (17% d. Th.) | |
| **79** | *rac*-N-[2-Amino-3-(3,4-difluorphenoxy)-2-methylpropyl]-7-[(2,6-difluorbenzyl)oxy]-2,5-dimethylpyrazolo[1,5-a]pyridin-3-carboxamid | LC-MS (Methode 2): Rₜ = 0.83 min MS (ESpos): m/z = 531 (M+H)⁺ |
| | 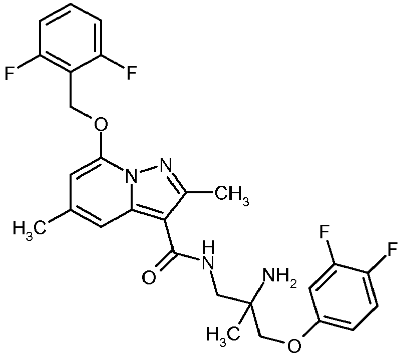 | |
| | (18% d. Th.; Reinheit 92%) | |
| **80** | 7-[(2,6-Difluorbenzyl)oxy]-N-(6-fluorchinolin-4-yl)-2,5-dimethylpyrazolo[1,5-a]pyridin-3-carboxamid Trifluoracetat | LC-MS (Methode 2): Rₜ = 0.93 min MS (ESpos): m/z = 477 (M-TFA+H)⁺ |
| | 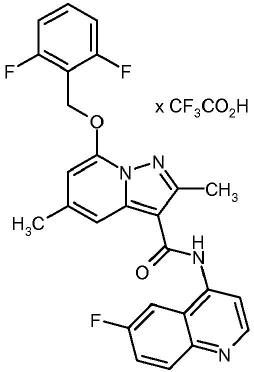 | |
| | (5% d. Th.; Reinheit 92%)²⁾ | |

1) Reaktionstemperatur: 60°C, Reaktionszeit: 1 h
2) Reaktionstemperatur: 60°C, Reaktionszeit: über Nacht

### Beispiel 81

### N-(3-Amino-2,2-difluorpropyl)-7-[(2,6-difluorbenzyl)oxy]-2,5-dimethylpyrazolo[1,5-a]pyridin-3-carboxamid

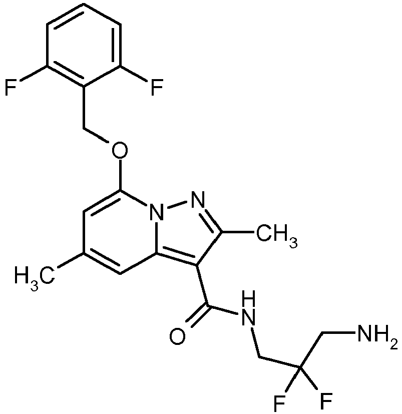

77 mg (0.12 mmol) tert-Butyl-{3-[({7-[(2,6-difluorbenzyl)oxy]-2,5-dimethylpyrazolo[1,5-a]pyridin-3-yl}carbonyl)amino]-2,2-difluorpropyl}carbamat Trifluoracetat aus Beispiel 112A wurden in 0.5 ml Diethylether suspendiert und mit 0.6 ml Chlorwasserstoff-Lösung (2 N in Diethylether) versetzt. Das Gemisch wurde über Nacht bei Raumtemperatur gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit wenig gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 47 mg der Zielverbindung (91% d. Th.) erhalten.
LC-MS (Methode 7): Rₜ = 2.13 min
MS (ESpos): m/z = 425 (M+H)⁺

In Analogie zu Beispiel 81 wurden die in Tabelle 4 gezeigten Beispielverbindungen hergestellt, indem die entsprechenden Ausgangsverbindungen [Boc-geschützte Amine] mit Chlorwasserstoff-Lösung (2 N in Diethylether, 5 - 20 Äquivalente), unter den beschriebenen Reaktionsbedingungen (Reaktionszeit: 2 - 24 h; Temperatur: RT) umgesetzt wurden.

Beispielhafte Aufarbeitung der Reaktionsmischung:
Das Reaktionsgemisch wurde mit Wasser, TFA oder Ameisensäure verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Dickschichtchromatographie oder Kieselgelchromatographie gereinigt (Laufmittel: Dichlormethan/Methanol oder Dichlormethan/2 M Ammoniak in Methanol). Die produkthaltigen Fraktionen wurden eingedampft.

Die produkthaltigen Fraktionen der präparativen HPLC wurden ggf. eingedampft, der Rückstand in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

**Tabelle 4:**

| **Beispiel** | **IUPAC**-**Name / Struktur (Ausbeute)** | **Analytische Daten** |
|---|---|---|
| **82** | *rac*-7-[(2,6-Difluorbenzyl)oxy]-2,5-dimethyl-N-(1,2,3,4-tetrahydrochinolin-2-ylmethyl)pyrazolo[1,5-a]pyridin-3-carboxamid | LC-MS (Methode 7): Rₜ = 2.70 min MS (ESpos): m/z = 477 (M+H)⁺ |
| | 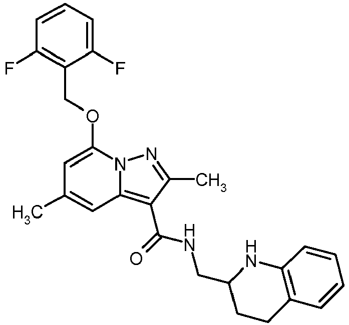 | |
| | (69% d. Th.) | |
| **83** | *rac*-Ethyl-6-{2-amino-1-[({7-[(2,6-difluorbenzyl)oxy]-2,5-dimethylpyrazolo[1,5-a]pyridin-3-yl}carbonyl)amino]ethyl}pyridin-2-carboxylat Trifluoracetat | LC-MS (Methode 2): Rₜ = 0.80 min MS (ESpos): m/z = 524 (M-TFA+H)⁺ |
| | 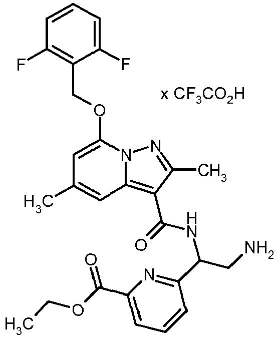 | |
| | (80% d. Th.; Reinheit 95%) | |
| **84** | *rac*-N-[2-Amino-1-(4-cyanphenyl)ethyl]-7-[(2,6-difluorbenzyl)oxy]-2,5-dimethylpyrazolo[1,5-a]pyridin-3-carboxamid | LC-MS (Methode 2): Rₜ = 0.77 min MS (ESpos): m/z = 476 (M+H)⁺ |
| | 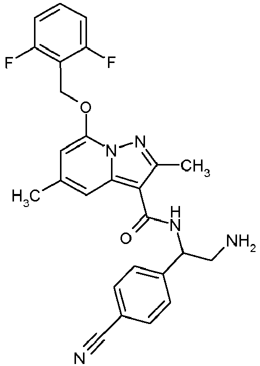 | |
| | (80% d. Th.) | |

### Beispiel 85

### rac-N-(2-Amino-1,2-dimethylcyclopropyl)-7-[(2,6-difluorbenzyl)oxy]-2,5-dimethylpyrazolo[1,5-a]pyridin-3-carboxamid

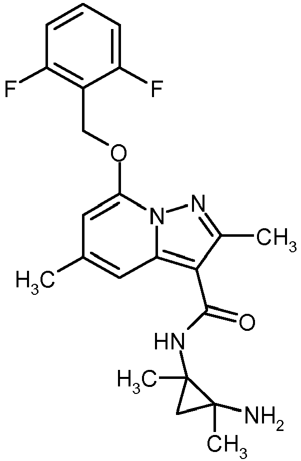

29 mg (0.11 mmol) *rac*-1,2-Dimethylcyclopropan-1,2-diamin-Dihydrobromid (beschrieben in: W. v. d. Saal et al. Liebigs Annalen der Chemie 1994, 569-580) wurden in einer 96er deep-well Multititerplatte vorgelegt. Eine Lösung von 33.2 mg (0.1 mmol) 7-[(2,6-Difluorbenzyl)oxy]-2,5-dimethylpyrazolo[1,5-a]pyridin-3-carbonsäure aus Beispiel 4A in 0.4 ml DMF/Dichlormethan (1:1, v/v) sowie eine Lösung von 49.4 mg (0.13 mmol) HATU in 0.4 ml DMF/Dichlormethan (1:1, v/v) wurden nacheinander hinzugegeben. Nach Zugabe von 20.2 mg (0.20 mmol) 4-Methylmorpholin wurde das Gemisch bei RT über Nacht geschüttelt. Anschließend wurde auf 60°C erwärmt und bei dieser Temperatur 7 h geschüttelt. Dann wurde das Lösemittel vollständig abgedampft. Der Rückstand wurde in 0.8 ml DMF gelöst, filtriert und aus dem Filtrat die Zielverbindung per präparativer LC-MS (Methode 9) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 2.3 mg (5% d. Th.) der Titelverbindung erhalten.
LC-MS (Methode 10): Rₜ = 0.66 min
MS (ESpos): m/z = 415 (M+H)⁺

In Analogie zu Beispiel 85 wurden die in Tabelle 5 gezeigten Beispielverbindungen hergestellt, indem 7-[(2,6-Difluorbenzyl)oxy]-2,5-dimethylpyrazolo[1,5-a]pyridin-3-carbonsäure aus Beispiel 4A mit den entsprechenden, kommerziell erhältlichen, literaturbekannten oder zuvor beschriebenen Aminen unter den beschriebenen Bedingungen umgesetzt wurden:

**Tabelle 5:**

| **Beispiel** | **IUPAC**-**Name**/**Struktur (Ausbeute)** | **Analytische Daten** |
|---|---|---|
| **86** | *ent*-7-[(2,6-Difluorbenzyl)oxy]-2,5-dimethyl-N-[2-methyl-6-(trifluormethyl)-1,2,3,4-tetrahydro-1,5-naphthyridin-4-yl]pyrazolo[1,5-a]pyridin-3-carboxamid | LC-MS (Methode 10): Rₜ = 1.27 min MS (ESpos): m/z = 546 (M+H)⁺ |
| | 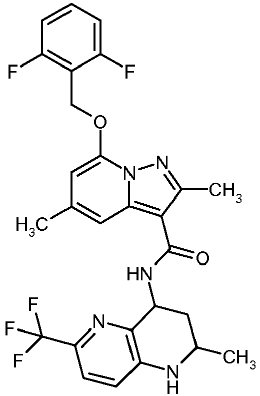 | |
| | (2% d. Th.; Reinheit 77%) | |
| **87** | *rac*-7-[(2,6-Difluorbenzyl)oxy]-N-(3,4-dihydro-2H-chromen-4-yl)-2,5-dimethylpyrazolo[1,5-a]pyridin-3-carboxamid | LC-MS (Methode 10): Rₜ = 1.18 min MS (ESpos): m/z = 464 (M+H)⁺ |
| | 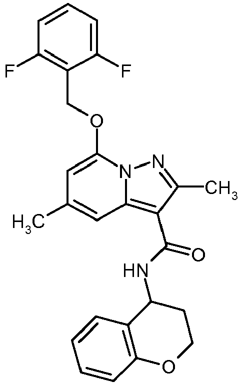 | |
| | (5% d. Th.; Reinheit 84%) | |
| **88** | *rac*-7-[(2,6-Difluorbenzyl)oxy]-2,5-dimethyl-N-(thiomorpholin-3-ylmethyl)pyrazolo[1,5-a]pyridin-3-carboxamid | LC-MS (Methode 10): Rₜ = 0.76 min MS (ESpos): m/z = 447 (M+H)⁺ |
| | 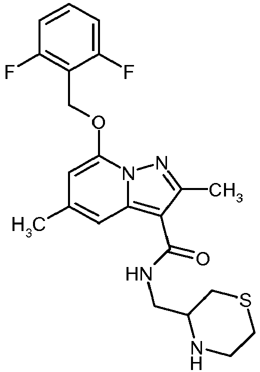 | |
| | (16% d. Th.) | |
| **89** | N-[2-(Cyclopropylamino)-2-oxoethyl]-7-[(2,6-difluorbenzyl)oxy]-2,5-dimethylpyrazolo[1,5-a]pyridin-3-carboxamid | LC-MS (Methode 10): Rₜ = 0.98 min MS (ESpos): m/z = 429 (M+H)⁺ |
| | 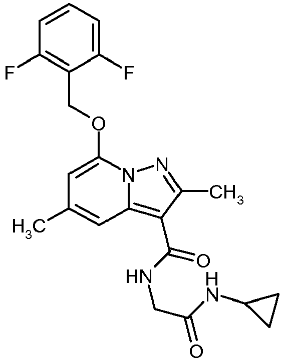 | |
| | (29% d. Th.; Reinheit 79%) | |
| **90** | 7-[(2,6-Difluorbenzyl)oxy]-N-[1-(3,4-difluorphenyl)cyclopropyl]-2,5-dimethylpyrazolo[1,5-a]pyridin-3-carboxamid | LC-MS (Methode 10): Rₜ = 1.20 min MS (ESpos): m/z = 484 (M+H)⁺ |
| | 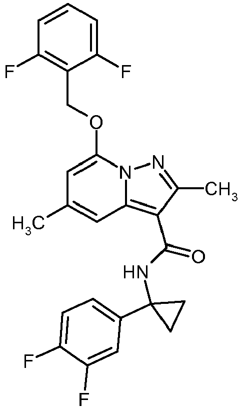 | |
| | (4% d. Th.; Reinheit 80%) | |
| **91** | *rac*-N-(2-Amino-2-methylcyclobutyl)-7-[(2,6-difluorbenzyl)oxy]-2,5-dimethylpyrazolo[1,5-a]pyridin-3-carboxamid¹⁾ | LC-MS (Methode 10): Rₜ = 0.75 min MS (ESpos): m/z = 415 (M+H)⁺ |
| | 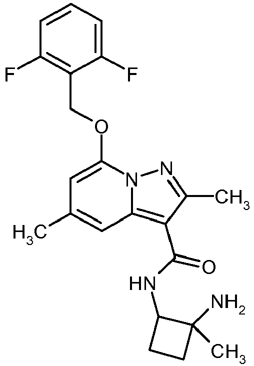 | |
| | (6% d. Th.; Reinheit 81%) | |
| **92** | *rac*-N-(2-Amino-2,4-dimethylpentyl)-7-[(2,6-difluorbenzyl)oxy]-2,5-dimethylpyrazolo[1,5-a]pyridin-3-carboxamid | LC-MS (Methode 10): Rₜ = 0.83 min MS (ESpos): m/z = 445 (M+H)⁺ |
| | 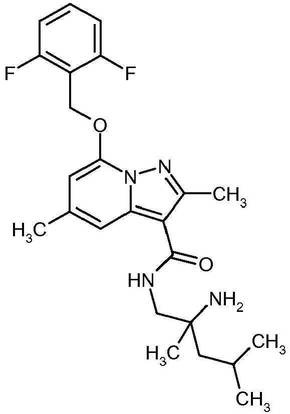 | |
| | (22% d. Th.) | |
| **93** | 7-[(2,6-Difluorbenzyl)oxy]-2,5-dimethyl-N-[2-(4-methylpiperazin-1-yl)ethyl]pyrazolo[1,5-a]pyridin-3-carboxamid | LC-MS (Methode 10): Rₜ = 0.71 min MS (ESpos): m/z = 458 (M+H)⁺ |
| | 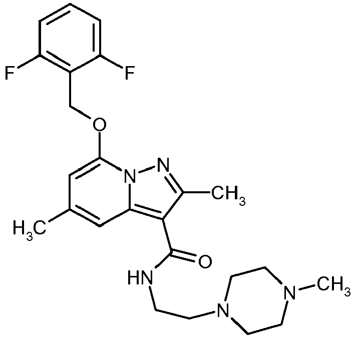 | |
| | (12% d. Th.) | |
| **94** | 7-[(2,6-Difluorbenzyl)oxy]-N-[(2R)-hexan-2-yl]-2,5-dimethylpyrazolo[1,5-a]pyridin-3-carboxamid | LC-MS (Methode 10): Rₜ = 1.22 min MS (ESpos): m/z = 416 (M+H)⁺ |
| | 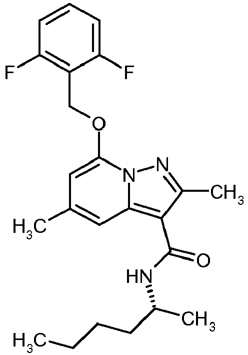 | |
| | (18% d. Th.) | |
| **95** | *rac*-7-[(2,6-Difluorbenzyl)oxy]-2,5-dimethyl-N-[(5-oxopyrrolidin-2-yl)methyl]pyrazolo[1,5-a]pyridin-3-carboxamid | LC-MS (Methode 10): Rₜ = 0.94 min MS (ESpos): m/z = 429 (M+H)⁺ |
| | 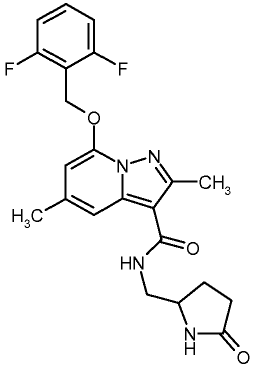 | |
| | (23% d. Th.) | |
| **96** | N-(2-Acetamidoethyl)-7-[(2,6-difluorbenzyl)oxy]-2,5-dimethylpyrazolo[1,5-a]pyridin-3-carboxamid | LC-MS (Methode 10): Rₜ = 0.94 min MS (ESpos): m/z = 417 (M+H)⁺ |
| | 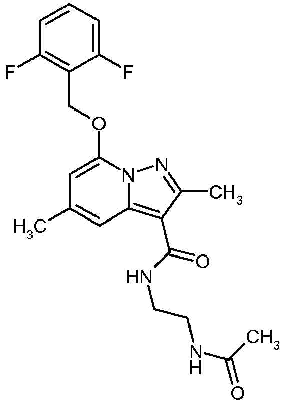 | |
| | (15% d. Th.) | |
| **97** | *rac*-N-(2-Amino-3-methoxy-2-methylpropyl)-7-[(2,6-difluorbenzyl)oxy]-2,5-dimethylpyrazolo[1,5-a]pyridin-3-carboxamid | LC-MS (Methode 10): Rₜ = 0.75 min MS (ESpos): m/z = 433 (M+H)⁺ |
| | 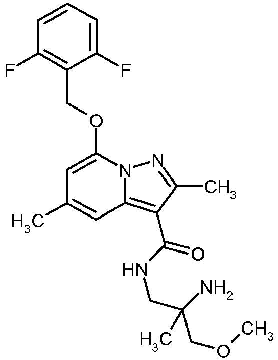 | |
| | (36% d. Th.) | |
| **98** | 7-[(2,6-Difluorbenzyl)oxy]-2,5-dimethyl-N-[(3R)-2-oxopyrrolidin-3-yl]pyrazolo[1,5-a]pyridin-3-carboxamid | LC-MS (Methode 10): Rₜ = 0.92 min MS (ESpos): m/z = 415 (M+H)⁺ |
| | 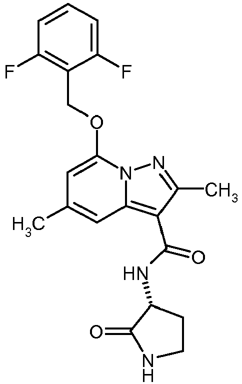 | |
| | (25% d. Th.) | |
| **99** | *rac*-N-[2-Amino-3-(4-methoxyphenyl)-2-methylpropyl]-7-[(2,6-difluorbenzyl)oxy]-2,5-dimethylpyrazolo[1,5-a]pyridin-3-carboxamid | LC-MS (Methode 10): Rₜ = 0.85 min MS (ESpos): m/z = 509 (M+H)⁺ |
| | 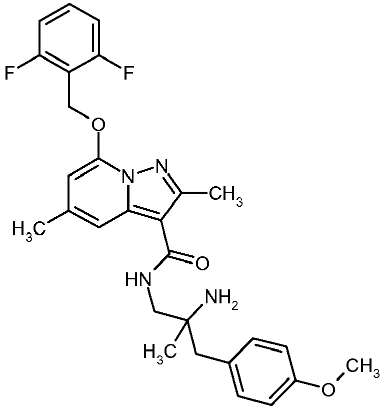 | |
| | (43% d. Th.) | |
| **100** | *rac*-7-[(2,6-Difluorbenzyl)oxy]-2,5-dimethyl-N-[(2-oxo-1,3-oxazolidin-5-yl)methyl]pyrazolo[1,5-a]pyridin-3-carboxamid | LC-MS (Methode 10): Rₜ = 0.92 min MS (ESpos): m/z = 431 (M+H)⁺ |
| | 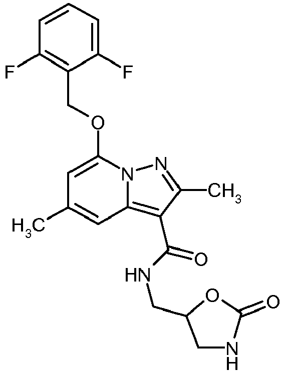 | |
| | (6% d. Th.) | |
| **101** | N-(2-Amino-3,3-dimethylcyclobutyl)-7-[(2,6-difluorbenzyl)oxy]-2,5-dimethylpyrazolo[1,5-a]pyridin-3-carboxamid (Isomerengemisch) ²⁾ | LC-MS (Methode 10): Rₜ = 0.79 min MS (ESpos): m/z = 429 (M+H)⁺ |
| | 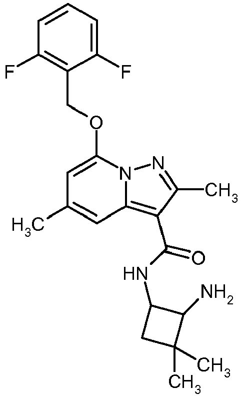 | |
| | (4% d. Th.; Reinheit 84%) | |
| **102** | *rac*-7-[(2,6-Difluorbenzyl)oxy]-N-(6-methoxy-1,2,3,4-tetrahydrochinolin-4-yl)-2,5-dimethylpyrazolo[1,5-a]pyridin-3-carboxamid | LC-MS (Methode 10): Rₜ = 0.95 min MS (ESpos): m/z = 493 (M+H)⁺ |
| | 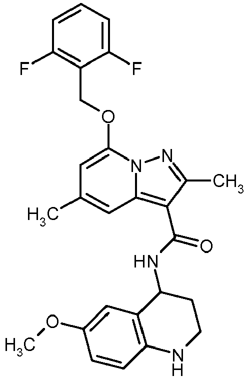 | |
| | (25% d. Th.) | |
| **103** | rac-N-[2-Amino-2-(chinolin-6-yl)ethyl]-7-[(2,6-difluorbenzyl)oxy]-2,5-dimethylpyrazolo[1,5-a]pyridin-3-carboxamid | LC-MS (Methode 10): Rₜ = 0.75 min MS (ESpos): m/z = 502 (M+H)⁺ |
| | 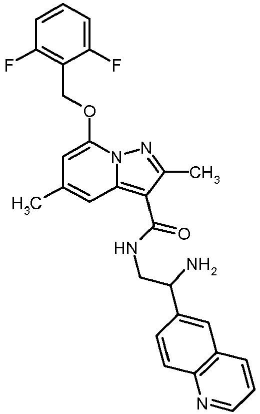 | |
| | (9% d. Th.; Reinheit 78%) | |
| **104** | *rac*-N-[2-Amino-2-(5-methyl-2-furyl)ethyl]-7-[(2,6-difluorbenzyl)oxy]-2,5-dimethylpyrazolo[1,5-a]pyridin-3-carboxamid | LC-MS (Methode 10): Rₜ = 0.79 min MS (ESpos): m/z = 455 (M+H)⁺ |
| | 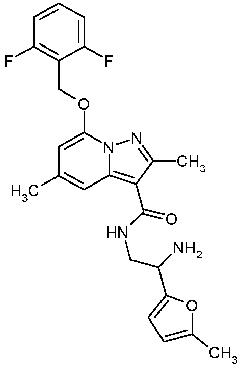 | |
| | (9% d. Th.) | |
| **105** | *rac*-N-{2-Amino-2-[3-(trifluormethyl)phenyl]ethyl}-7-[(2,6-difluorbenzyl)oxy]-2,5-dimethylpyrazolo[1,5-a]pyridin-3-carboxamid | LC-MS (Methode 10): Rₜ = 0.86 min MS (ESpos): m/z = 519 (M+H)⁺ |
| | 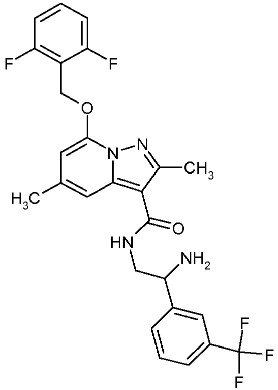 | |
| | (11% d. Th.) | |
| **106** | Ethyl-{2-[({7-[(2,6-difluorbenzyl)oxy]-2,5-dimethylpyrazolo[1,5-a]pyridin-3-yl}carbonyl)amino]ethyl}carbamat | LC-MS (Methode 10): Rₜ = 1.03 min MS (ESpos): m/z = 447 (M+H)⁺ |
| | 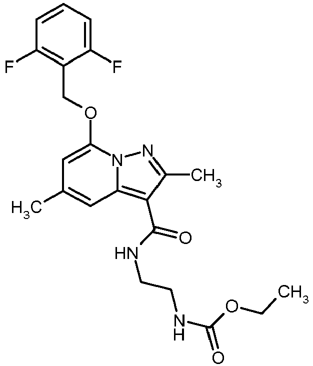 | |
| | (15% d. Th.) | |
| **107** | *rac*-N-(2-Amino-4,4,4-trifluorbutyl)-7-[(2,6-difluorbenzyl)oxy]-2,5-dimethylpyrazolo[1,5-a]pyridin-3-carboxamid | LC-MS (Methode 10): Rₜ = 0.79 min MS (ESpos): m/z = 457 (M+H)⁺ |
| | 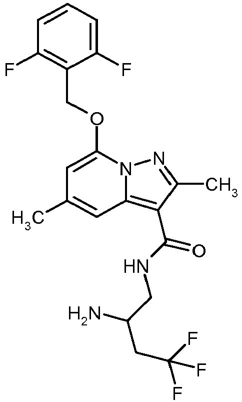 | |
| | (2% d. Th.) | |
| **108** | *rac*-7-[(2,6-Difluorbenzyl)oxy]-N-(2,3-dihydro-1-benzofur-3-yl)-2,5-dimethylpyrazolo[1,5-a]pyridin-3-carboxamid | LC-MS (Methode 10): Rₜ = 1.17 min MS (ESpos): m/z = 450 (M+H)⁺ |
| | 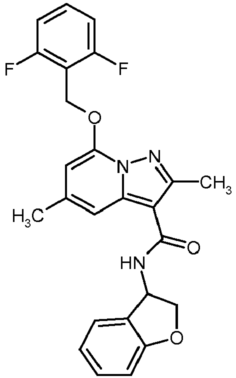 | |
| | (3% d. Th.) | |
| **109** | *rac*-N-(2-Amino-3,3,3-trifluor-2-methylpropyl)-7-[(2,6-difluorbenzyl)oxy]-2,5-dimethylpyrazolo[1,5-a]pyridin-3-carboxamid | LC-MS (Methode 10): Rₜ =0.92 min MS (ESpos): m/z = 457 (M+H)⁺ |
| | 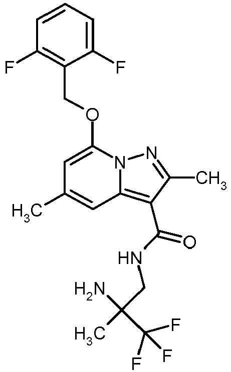 | |
| | (1% d. Th.) | |
| **110** | *rac*-N-[2-Amino-2-(2,3-dihydro-1,4-benzodioxin-6-yl)ethyl]-7-[(2,6-difluorbenzyl)oxy]-2,5-dimethylpyrazolo[1,5-a]pyridin-3-carboxamid | LC-MS (Methode 10): Rₜ = 0.80 min MS (ESpos): m/z = 509 (M+H)⁺ |
| | 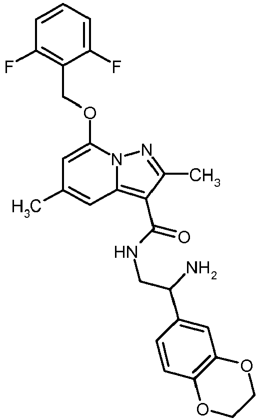 | |
| | (3% d. Th.) | |
| **111** | 7-[(2,6-Difluorbenzyl)oxy]-N-(7-fluor-2-methyl-2,3-dihydro-1-benzofur-3-yl)-2,5-dimethylpyrazolo[1,5-a]pyridin-3-carboxamid (Mischung von Stereoisomeren) | LC-MS (Methode 10): Rₜ = 1.22 min MS (ESpos): m/z = 482 (M+H)⁺ |
| | 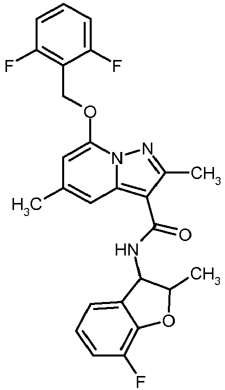 | |
| | (2% d. Th.) | |
| 112 | *rac*-N-[2-Amino-2-(1-benzothiophen-3-yl)ethyl]-7-[(2,6-difluorbenzyl)oxy]-2,5-dimethylpyrazolo[1,5-a]pyridin-3-carboxamid | LC-MS (Methode 10): Rₜ = 0.85 min MS (ESpos): m/z = 507 (M+H)⁺ |
| | 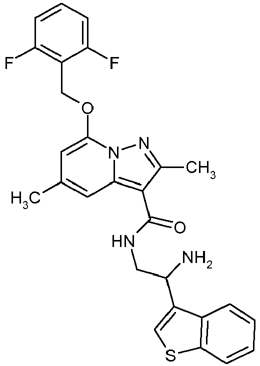 | |
| | (8% d. Th.) | |
| **113** | 7-[(2,6-Difluorbenzyl)oxy]-2,5-dimethyl-N-[(7S,8aS)-2-methyloctahydropyrrolo[1,2-a]pyrazin-7-yl]pyrazolo[1,5-a]pyridin-3-carboxamid | LC-MS (Methode 10): Rₜ = 0.73 min MS (ESpos): m/z = 470 (M+H)⁺ |
| | 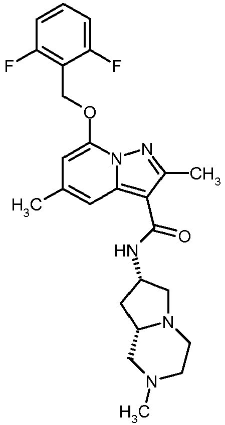 | |
| | (1% d. Th.) | |
| **114** | *rac*-Methyl-3-{1-amino-2-[({7-[(2,6-difluorbenzyl)oxy]-2,5-dimethylpyrazolo[1,5-a]pyridin-3-yl}carbonyl)amino]ethyl}benzoat | LC-MS (Methode 10): Rₜ = 0.81 min MS (ESpos): m/z = 509 (M+H)⁺ |
| | 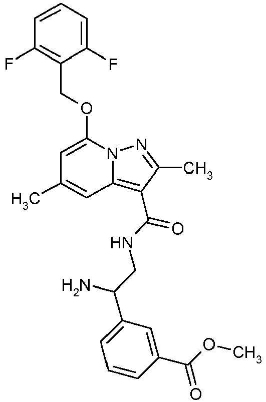 | |
| | (2% d. Th.) | |
| **115** | *rac*-N-[2-Aminohex-4-en-1-yl]-7-[(2,6-difluorbenzyl)oxy]-2,5-dimethylpyrazolo[1,5-a]pyridin-3-carboxamid (E/Z-Gemisch) | LC-MS (Methode 10): Rₜ = 0.81 min MS (ESpos): m/z = 429 (M+H)⁺ |
| | 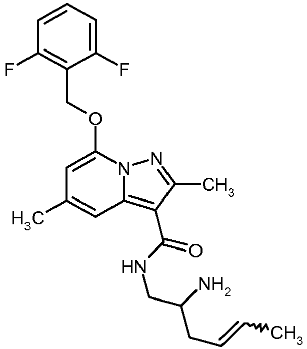 | |
| | (3% d. Th.; Reinheit 85%) | |
| **116** | rac-N-{2-Amino-2-[3-(difluormethoxy)phenyl]ethyl}-7-[(2,6-difluorbenzyl)oxy]-2,5-dimethylpyrazolo[1,5-a]pyridin-3-carboxamid | LC-MS (Methode 10): Rₜ = 0.84 min MS (ESpos): m/z = 517 (M+H)⁺ |
| | 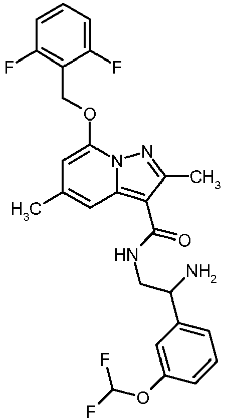 | |
| | (6% d. Th.) | |
| **117** | *rac*-N-[2-Amino-2-(3,4,5-trifluorphenyl)ethyl]-7-[(2,6-difluorbenzyl)oxy]-2,5-dimethylpyrazolo[1,5-a]pyridin-3-carboxamid | LC-MS (Methode 10): Rₜ = 0.84 min MS (ESpos): m/z = 505 (M+H)⁺ |
| | 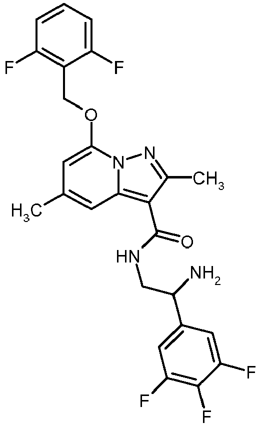 | |
| | (1% d. Th.) | |

| | | |
|---|---|---|
| ¹⁾ Das eingesetzte Amin *rac*-1-Methylcyclobutan-1,2-diamindihydrochlorid ist beschrieben in: K.-H. Scholz; J. Hinz; H.-G. Heine; W. Hartmann, Liebigs Annalen der Chemie, 1981, 248-255; Duschinsky; Dolan Journal of the American Chemical Society, 1945, 67, 2079, 2082. ²⁾ Das eingesetzte Amin 3,3-Dimethylcyclobutan-1,2-diamindihydrochlorid (Isomerengemisch) ist beschrieben in: K. Scholz et al. Liebigs Annalen der Chemie, 1981, (2), 248-55; D. Hossain et al. Synthetic Communications 2012 42, 1200-1210; M. Klapper et al. Angewandte Chemie 2003, 115, 4835-4690. | | |

### Beispiel 118

### N-[(1-Aminocyclopropyl)methyl]-7-[(2,6-difluorbenzyl)oxy]-2,5-dimethylpyrazolo[1,5-a]pyridin-3-carboxamid

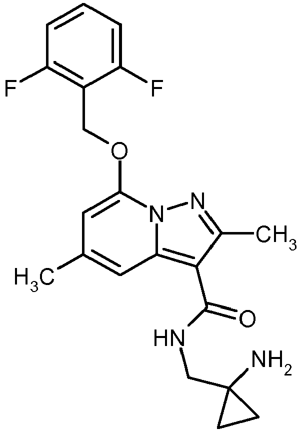

20.5 mg (0.11 mmol) tert-Butyl-[1-(aminomethyl)cyclopropyl]carbamat wurden in einer 96er deep well Multititerplatte vorgelegt. Eine Lösung von 33.2 mg (0.1 mmol) 7-[(2,6-Difluorbenzyl)oxy]-2,5-dimethylpyrazolo[1,5-a]pyridin-3-carbonsäure aus Beispiel 4A in 0.4 ml DMF/Dichlormethan (1:1, v/v) sowie eine Lösung von 49.4 mg (0.13 mol) HATU in 0.4 ml DMF/Dichlormethan (1:1, v/v) wurden nacheinander hinzugegeben. Nach Zugabe von 20.2 mg (0.20 mmol) 4-Methylmorpholin wurde das Gemisch bei RT über Nacht geschüttelt. Anschließend wurde auf 60°C erwärmt und bei dieser Temperatur 7 h geschüttelt. Dann wurde das Lösemittel vollständig abgedampft. Der Rückstand wurde mit 0.6 ml TFA versetzt und bei Raumtemperatur über Nacht geschüttelt. Dann wurde vollständig eingedampft, der Rückstand in 0.8 ml DMF gelöst, filtriert und aus dem Filtrat die Zielverbindung per präparativer LC-MS (Methode 9) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 0.5 mg (1.3% d. Th.) der Titelverbindung erhalten.
LC-MS (Methode 10): Rₜ = 0.74 min
MS (ESpos): m/z = 401 (M+H)⁺

In Analogie zu Beispiel 118 wurden die in Tabelle 6 gezeigten Beispielverbindungen hergestellt, indem 7-[(2,6-Difluorbenzyl)oxy]-2,5-dimethylpyrazolo[1,5-a]pyridin-3-carbonsäure aus Beispiel 4A mit den entsprechenden, kommerziell erhältlichen, literaturbekannten oder zuvor beschriebenen Mono-Boc-geschützten Diaminen unter den beschriebenen Bedingungen umgesetzt wurden:

**Tabelle 6:**

| **Beispiel** | **IUPAC**-**Name**/**Struktur (Ausbeute)** | **Analytische Daten** |
|---|---|---|
| **119** | *rac*-N-[2-Amino-1-(4-nitrophenyl)ethyl]-7-[(2,6-difluorbenzyl)oxy]-2,5-dimethylpyrazolo[1,5-a]pyridin-3-carboxamid | LC-MS (Methode 10): Rₜ = 0.75 min MS (ESpos): m/z = 496 (M+H)⁺ |
| | 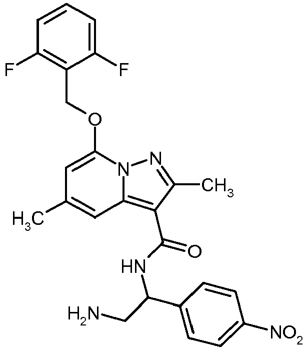 | |
| | (2% d. Th.) | |
| **120** | *rac*-N-[2-Amino-1-(1,3-benzodioxol-5-yl)ethyl]-7-[(2,6-difluorbenzyl)oxy]-2,5-dimethylpyrazolo[1,5-a]pyridin-3-carboxamid | LC-MS (Methode 10): Rₜ = 0.81 min MS (ESpos): m/z = 495 (M+H)⁺ |
| | 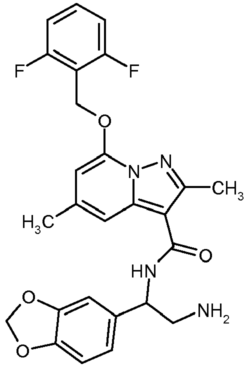 | |
| | (25% d. Th.) | |
| **121** | N-(3-Amino-3-methylbutyl)-7-[(2,6-difluorbenzyl)oxy]-2,5-dimethylpyrazolo[1,5-a]pyridin-3-carboxamid | LC-MS (Methode 10): Rₜ = 0.74 min MS (ESpos): m/z = 417 (M+H)⁺ |
| | 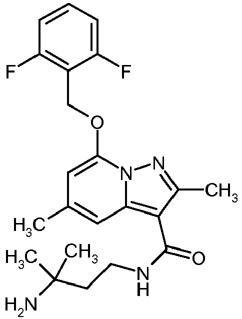 | |
| | (24% d. Th.) | |
| **122** | *rel*-7-[(2,6-Difluorbenzyl)oxy]-2,5-dimethyl-N-[(3aR,6aR)-octahydrocyclopenta[b]pyrrol-4-yl]pyrazolo[1,5-a]pyridin-3-carboxamid | LC-MS (Methode 10): Rₜ = 0.74 min MS (ESpos): m/z = 441 (M+H)⁺ |
| | 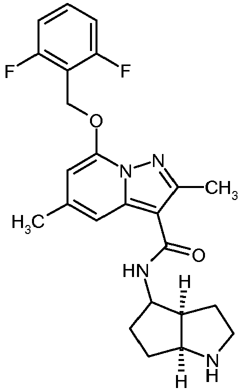 | |
| | (13% d. Th.) | |
| **123** | *rac*-N-[2-Amino-1-(3-methoxyphenyl)ethyl]-7-[(2,6-difluorbenzyl)oxy]-2,5-dimethylpyrazolo[1,5-a]pyridin-3-carboxamid | LC-MS (Methode 10): Rₜ = 0.82 min MS (ESpos): m/z = 481 (M+H)⁺ |
| | 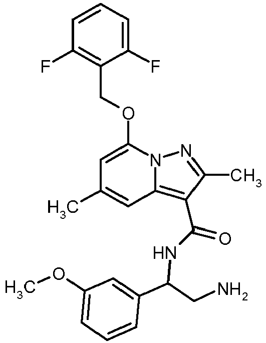 | |
| | (17% d. Th.) | |
| **124** | N-[(1-Aminocyclohexyl)methyl]-7-[(2,6-difluorbenzyl)oxy]-2,5-dimethylpyrazolo[1,5-a]pyridin-3-carboxamid | LC-MS (Methode 10): Rₜ = 0.81 min MS (ESpos): m/z = 443 (M+H)⁺ |
| | 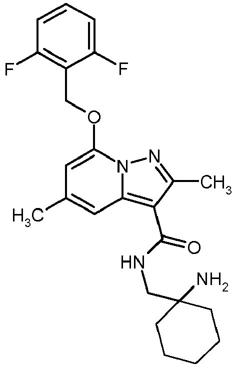 | |
| | (11% d. Th.) | |
| 125 | 7-[(2,6-Difluorbenzyl)oxy]-2,5-dimethyl-N-[4-(piperazin-1-yl)-3-(trifluormethyl)phenyl]pyrazolo[1,5-a]pyridin-3-carboxamid | LC-MS (Methode 10): Rₜ = 0.89 min MS (ESpos): m/z = 560 (M+H)⁺ |
| | 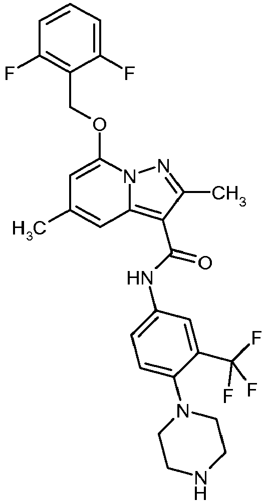 | |
| | (1% d. Th.; Reinheit 78%) | |
| **126** | *rac*-N-[2-Amino-1-(3-bromphenyl)ethyl]-7-[(2,6-difluorbenzyl)oxy]-2,5-dimethylpyrazolo[1,5-a]pyridin-3-carboxamid | LC-MS (Methode 10): Rₜ = 0.85min MS (ESpos): m/z = 529 (M+H)⁺ |
| | 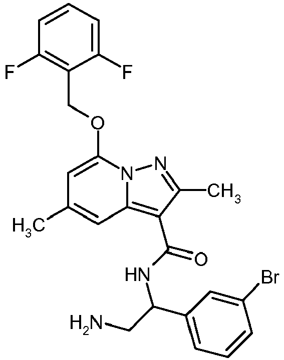 | |
| | (45% d. Th.) | |
| **127** | *rac*-N-(Azepan-3-yl)-7-[(2,6-difluorbenzyl)oxy]-2,5-dimethylpyrazolo[1,5-a]pyridin-3-carboxamid | LC-MS (Methode 10): Rₜ = 0.76 min MS (ESpos): m/z = 429 (M+H)⁺ |
| | 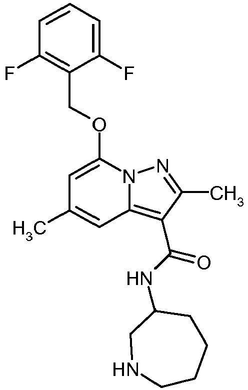 | |
| | (58% d. Th.) | |
| **128** | rel-7-[(2,6-Difluorbenzyl)oxy]-N-[(3R,4S)-4-fluorpyrrolidin-3-yl]-2,5-dimethylpyrazolo[1,5-a]pyridin-3-carboxamid | LC-MS (Methode 10): Rₜ = 0.72 min MS (ESpos): m/z = 419 (M+H)⁺ |
| | 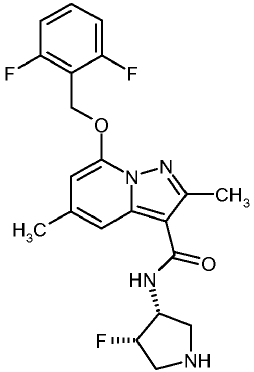 | |
| | (34% d. Th.) | |
| **129** | 7-[(2,6-Difluorbenzyl)oxy]-2,5-dimethyl-N-[(2S)-piperidin-2-ylmethyl]pyrazolo[1,5-a]pyridin-3-carboxamid | LC-MS (Methode 10): Rₜ = 0.76 min MS (ESpos): m/z = 429 (M+H)⁺ |
| | 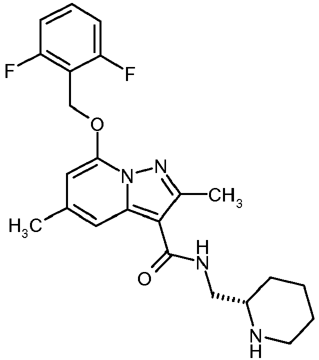 | |
| | (31% d. Th.) | |
| 130 | *rac*-N-(3-Aminocyclopentyl)-7-[(2,6-difluorbenzyl)oxy]-2,5-dimethylpyrazolo[1,5-a]pyridin-3-carboxamid | LC-MS (Methode 10): Rₜ = 0.73 min MS (ESpos): m/z = 415 (M+H)⁺ |
| | 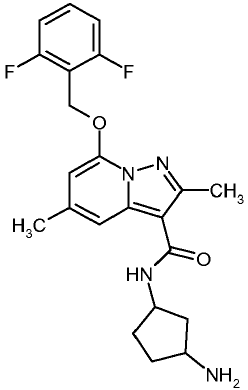 | |
| | (7% d. Th.) | |
| **131** | Methyl-N⁶-({7-[(2,6-difluorbenzyl)oxy]-2,5-dimethylpyrazolo[1,5-a]pyridin-3-yl}carbonyl)-L-lysinat | LC-MS (Methode 10): Rₜ = 0.78 min MS (ESpos): m/z = 475 (M+H)⁺ |
| | 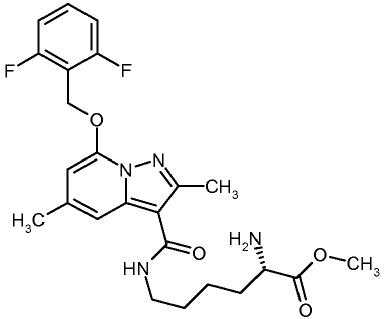 | |
| | (2% d. Th.) | |
| **132** | *rac*-N-{2-Amino-1-[3-(trifluormethoxy)phenyl]ethyl}-7-[(2,6-difluorbenzyl)oxy]-2,5-dimethylpyrazolo[1,5-a]pyridin-3-carboxamid | LC-MS (Methode 10): Rₜ = 0.88 min MS (ESpos): m/z = 535 (M+H)⁺ |
| | 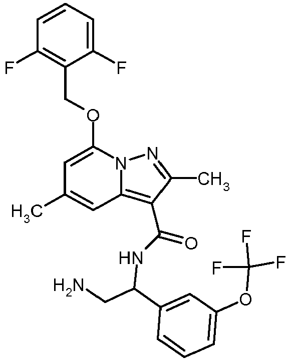 | |
| | (54% d. Th.) | |
| **133** | *ent*-N-[2-Amino-1-(3,4-difluorphenyl)ethyl]-7-[(2,6-difluorbenzyl)oxy]-2,5-dimethylpyrazolo[1,5-a]pyridin-3-carboxamid | LC-MS (Methode 10): Rₜ = 0.84 min MS (ESpos): m/z = 487 (M+H)⁺ |
| | 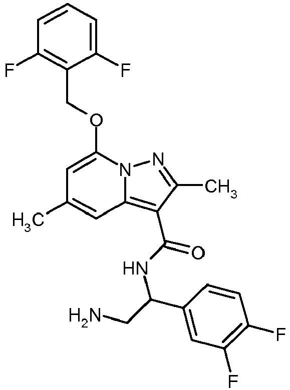 | |
| | (21% d. Th.) | |

### Beispiel 134

### rac-N-[(4-Chlorphenyl)(cyan)methyl]-7-[(2,6-difluorbenzyl)oxy]-2,5-dimethylpyrazolo[1,5-a]pyridin-3-carboxamid

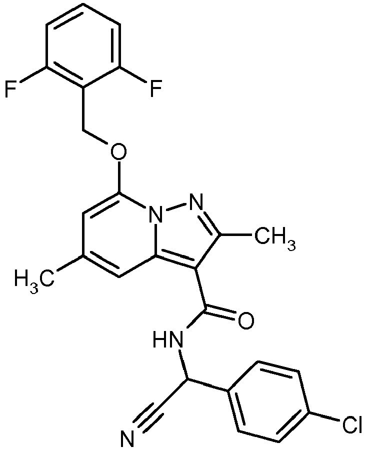

33 mg (0.10 mmol) 7-[(2,6-Difluorbenzyl)oxy]-2,5-dimethylpyrazolo[1,5-a]pyridin-3-carbonsäure aus Beispiel 4A wurden in einer 96er deep well Multititerplatte vorgelegt. Eine Lösung von 17 mg (0.10 mmol) *rac*-Amino(4-chlorphenyl)acetonitril in 0.4 ml DMF sowie eine Lösung von 45.6 mg (0.12 mol) HATU in 0.4 ml DMF wurden nacheinander hinzugegeben. Nach Zugabe von 20.2 mg (0.20 mmol) 4-Methylmorpholin wurde das Gemisch bei RT über Nacht geschüttelt. Dann wurde filtriert und aus dem Filtrat die Zielverbindung per präparativer LC-MS (Methode 9) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 0.7 mg (1.5% d. Th.) erhalten.
LC-MS (Methode 10): Rₜ = 1.22 min
MS (ESpos): m/z = 481 (M+H)⁺

### Beispiel 135

### 7-[(2,6-Difluorbenzyl)oxy]-2,5-dimethyl-N-(morpholin-4-yl)pyrazolo[1,5-a]pyridin-3-carboxamid

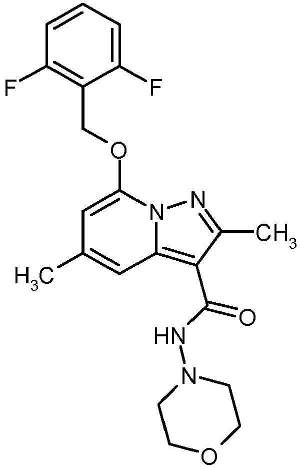

33 mg (0.10 mmol) 7-[(2,6-Difluorbenzyl)oxy]-2,5-dimethylpyrazolo[1,5-a]pyridin-3-carbonsäure aus Beispiel 4A wurden in einer 96er deep well Multititerplatte vorgelegt. Eine Lösung von 17 mg (0.10 mmol) Morpholin-4-amin in 0.4 ml DMF sowie eine Lösung von 45.6 mg (0.12 mol) HATU in 0.4 ml DMF wurden nacheinander hinzugegeben. Nach Zugabe von 20.2 mg (0.20 mmol) 4-Methylmorpholin wurde das Gemisch bei RT über Nacht geschüttelt. Dann wurde filtriert und aus dem Filtrat die Zielverbindung per präparativer LC-MS (Methode 9) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 0.5 mg (1% d. Th.) erhalten.
LC-MS (Methode 10): Rₜ = 0.96 min
MS (ESpos): m/z = 417 (M+H)⁺

### Beispiel 136

### rac-N-[2-Amino-2-(1-methyl-1H-pyrazol-4-yl)ethyl]-7-[(2,6-difluorbenzyl)oxy]-2,5-dimethylpyrazolo[1,5-a]pyridin-3-carboxamid

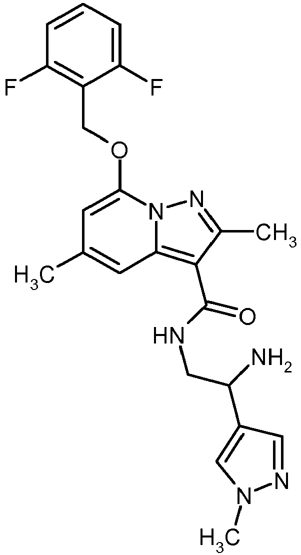

830 mg (2.50 mmol) 7-[(2,6-Difluorbenzyl)oxy]-2,5-dimethylpyrazolo[1,5-a]pyridin-3-carbonsäure aus Beispiel 4A wurden mit 5 ml (68.5 mmol) Thionylchlorid versetzt und bei RT 2 h gerührt. Dann wurde vollständig eingedampft, der Rückstand mit 10 ml Toluol versetzt und bis zur Trockne eingeengt.

14.0 mg (0.10 mmol) 1-(1-Methyl-1H-pyrazol-4-yl)ethan-1,2-diamin wurden in einer 96er deep well Multititerplatte vorgelegt. Eine Lösung von 35.1 mg (0.10 mmol) 7-[(2,6-Difluorbenzyl)oxy]-2,5-dimethylpyrazolo[1,5-a]pyridin-3-carbonsäurechlorid in 0.6 ml Dichlorethan sowie 0.032 ml (0.40 mmol) Pyridin wurden nacheinander hinzugegeben und das Gemisch bei RT über Nacht geschüttelt. Dann wurde das Lösemittel vollständig abgedampft, der Rückstand in 0.8 ml DMF gelöst, filtriert und aus dem Filtrat die Zielverbindung per präparativer LC-MS (Methode 9) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 18 mg (37% d. Th.; Reinheit 94%) der Titelverbindung erhalten.
LC-MS (Methode 10): Rₜ = 0.73 min
MS (ESpos): m/z = 455 (M+H)⁺

In Analogie zu Beispiel 136 wurden die in Tabelle 7 gezeigten Beispielverbindungen hergestellt, indem 2,5-Dimethyl-7-[(2,3,6-trifluorbenzyl)oxy]pyrazolo[1,5-a]pyridin-3-carbonsäurechlorid mit den entsprechenden, kommerziell erhältlichen oder zuvor beschriebenen Aminen unter den beschriebenen Bedingungen umgesetzt wurden:

**Tabelle 7:**

| **Beispiel** | **IUPAC**-**Name**/**Struktur (Ausbeute)** | **Analytische Daten** |
|---|---|---|
| **137** | *rac*-7-[(2,6-Difluorbenzyl)oxy]-2,5-dimethyl-N-(2,2,7-trimethyl-3,4-dihydro-2H-chromen-4-yl)pyrazolo[1,5-a]pyridin-3-carboxamid | LC-MS (Methode 10): Rₜ = 1.25 min MS (ESpos): m/z = 506 (M+H)⁺ |
| | 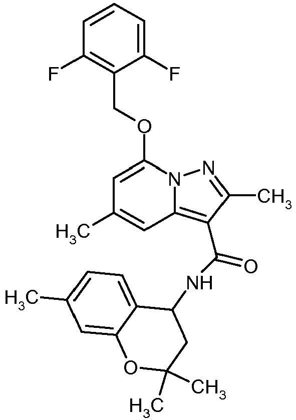 | |
| | (13% d. Th.; Reinheit 90%) | |
| **138** | *rac*-7-[(2,6-Difluorbenzyl)oxy]-2,5-dimethyl-N-(1,2,3,4-tetrahydronaphthalin-1-yl)pyrazolo[1,5-a]pyridin-3-carboxamid | LC-MS (Methode 10): Rₜ = 1.23 min MS (ESpos): m/z = 462 (M+H)⁺ |
| | 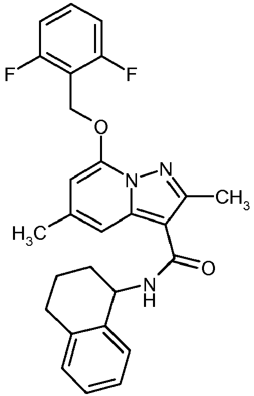 | |
| | (10% d. Th.) | |
| **139** | *rac*-N-(5-Amino-1,2,3,4-tetrahydronaphthalin-1-yl)-7-[(2,6-difluorbenzyl)oxy]-2,5-dimethylpyrazolo[1,5-a]pyridin-3-carboxamid | LC-MS (Methode 10): Rₜ = 1.06 min MS (ESpos): m/z = 477 (M+H)⁺ |
| | 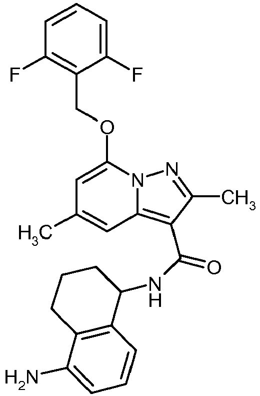 | |
| | (1% d. Th.; Reinheit 80%) | |
| **140** | *rac*-N-(8-Cyan-3,4-dihydro-2H-chromen-4-yl)-7-[(2,6-difluorbenzyl)oxy]-2,5-dimethylpyrazolo[1,5-a]pyridin-3-carboxamid | LC-MS (Methode 10): Rₜ = 1.17 min MS (ESpos): m/z = 489 (M+H)⁺ |
| | 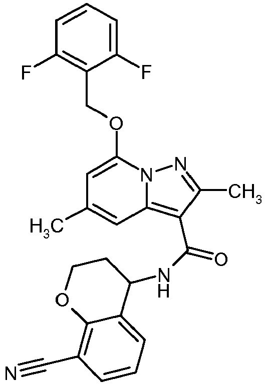 | |
| | (5% d. Th.) | |
| **141** | *rac*-N-[2-Amino-2-(3-vinylphenyl)ethyl]-7-[(2,6-difluorbenzyl)oxy]-2,5-dimethylpyrazolo[1,5-a]pyridin-3-carboxamid | LC-MS (Methode 10): Rₜ = 0.84 min MS (ESpos): m/z = 477 (M+H)⁺ |
| | 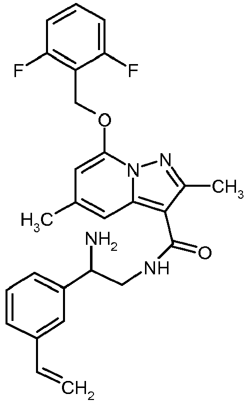 | |
| | (3% d. Th.) | |
| **142** | *rac*-7-[(2,6-Difluorbenzyl)oxy]-2,5-dimethyl-N-(morpholin-2-ylmethyl)pyrazolo[1,5-a]pyridin-3-carboxamid | LC-MS (Methode 10): Rₜ = 0.72 min MS (ESpos): m/z = 431 (M+H)⁺ |
| | 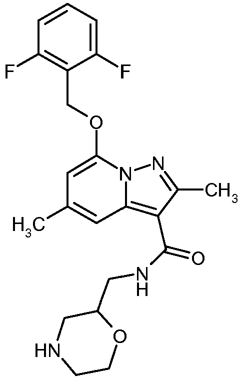 | |
| | (3% d. Th.; Reinheit 78%) | |
| **143** | *rel*-7-[(2,6-Difluorbenzyl)oxy]-N-[(1R,2R)-2-(hydroxymethyl)-2,3-dihydro-1H-inden-1-yl]-2,5-dimethylpyrazolo[1,5-a]pyridin-3-carboxamid | LC-MS (Methode 10): Rₜ = 1.14 min MS (ESpos): m/z = 478 (M+H)⁺ |
| | 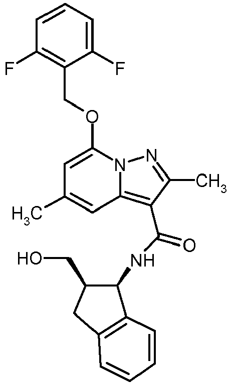 | |
| | (14% d. Th.) | |
| **144** | 7-[(2,6-Difluorbenzyl)oxy]-N-[(2R)-1-hydroxyhexan-2-yl]-2,5-dimethylpyrazolo[1,5-a]pyridin-3-carboxamid | LC-MS (Methode 10): Rₜ = 1.08 min MS (ESpos): m/z = 432 (M+H)⁺ |
| | 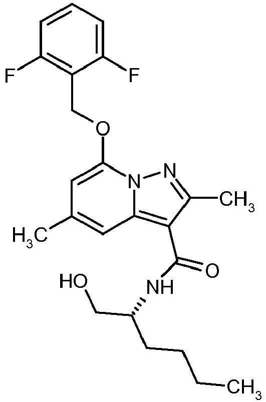 | |
| | (25% d. Th.) | |
| **145** | *rac*-7-[(2,6-Difluorbenzyl)oxy]-N-(4,4-dimethyl-1,2,3,4-tetrahydronaphthalin-1-yl)-2,5-dimethylpyrazolo[1,5-a]pyridin-3-carboxamid | LC-MS (Methode 10): Rₜ = 1.28 min MS (ESpos): m/z = 490 (M+H)⁺ |
| | 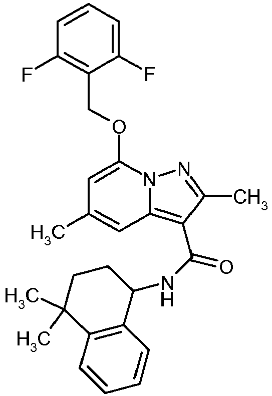 | |
| | (8% d. Th.; Reinheit 90%) | |
| **146** | *rac*-7-[(2,6-Difluorbenzyl)oxy]-N-(7-methoxy-1,2,3,4-tetrahydronaphthalin-1-yl)-2,5-dimethylpyrazolo[1,5-a]pyridin-3-carboxamid | LC-MS (Methode 10): Rₜ = 1.22 min MS (ESpos): m/z = 492 (M+H)⁺ |
| | 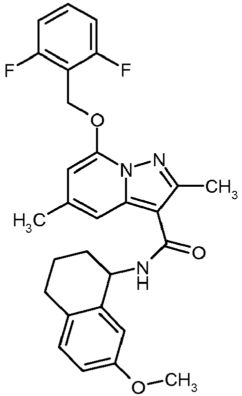 | |
| | (3% d. Th.; Reinheit 87%) | |
| **147** | *rac*-N-(8-Chlor-3,4-dihydro-2H-chromen-4-yl)-7-[(2,6-difluorbenzyl)oxy]-2,5-dimethylpyrazolo[1,5-a]pyridin-3-carboxamid | LC-MS (Methode 10): Rₜ = 1.21 min MS (ESpos): m/z = 498 (M+H)⁺ |
| | 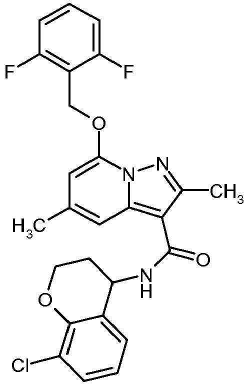 | |
| | (1% d. Th.) | |
| **148** | *rac*-7-[(2,6-Difluorbenzyl)oxy]-N-(6-fluor-8-methyl-3,4-dihydro-2H-chromen-4-yl)-2,5-dimethylpyrazolo[1,5-a]pyridin-3-carboxamid | LC-MS (Methode 10): Rₜ = 1.23 min MS (ESpos): m/z = 496 (M+H)⁺ |
| | 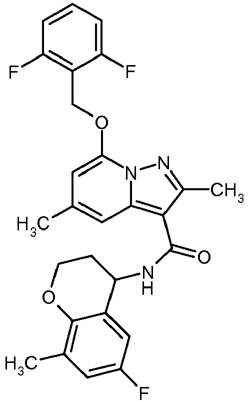 | |
| | (3% d. Th.; Reinheit 79%) | |
| **149** | 7-[(2,6-Difluorbenzyl)oxy]-2,5-dimethyl-N-[(3S)-2-oxopyrrolidin-3-yl]pyrazolo[1,5-a]pyridin-3-carboxamid | LC-MS (Methode 10): Rₜ = 0.92 min MS (ESpos): m/z = 415 (M+H)⁺ |
| | 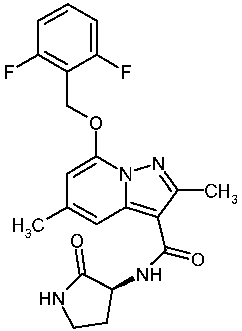 | |
| | (2% d. Th.; Reinheit 90%) | |
| **150** | *rac*-7-[(2,6-Difluorbenzyl)oxy]-N-(6-fluor-1,2,3,4-tetrahydronaphthalin-1-yl)-2,5-dimethylpyrazolo[1,5-a]pyridin-3-carboxamid | LC-MS (Methode 10): Rₜ = 1.23 min MS (ESpos): m/z = 480 (M+H)⁺ |
| | 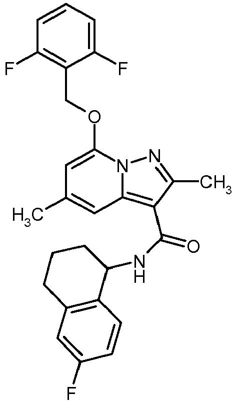 | |
| | (4% d. Th.; Reinheit 75%) | |
| **151** | N-[3-(Cyclopropylsulfamoyl)propyl]-7-[(2,6-difluorbenzyl)oxy]-2,5-dimethylpyrazolo[1,5-a]pyridin-3-carboxamid | LC-MS (Methode 10): Rₜ = 1.02 min MS (ESpos): m/z = 493 (M+H)⁺ |
| | 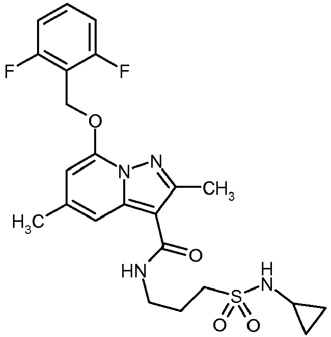 | |
| | (17% d. Th.) | |

### Beispiel 152

### Methyl-4-[({7-[(2,6-difluorbenzyl)oxy]-2,5-dimethylpyrazolo[1,5-a]pyridin-3-yl}carbonyl)amino]bicyclo[2.2.2]octan-1-carboxylat

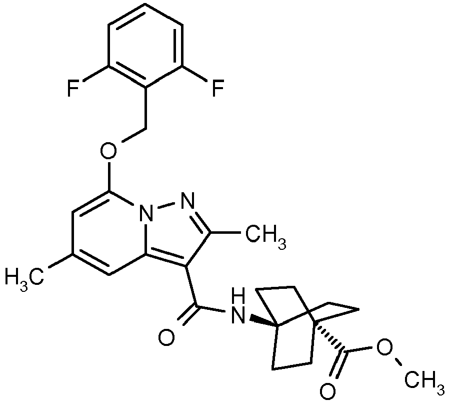

Eine Mischung von 80 mg 7-[(2,6-Difluorbenzyl)oxy]-2,5-dimethylpyrazolo[1,5-a]pyridin-3-carbonsäure (Reinheit 75%, 0.181 mmol) aus Beispiel 4A, 76 mg HATU (0.199 mmol) und 94 µl (0.542 mmol) N,N-Diisopropylethylamin in 0.54 ml DMF wurden 10 min bei RT gerührt. Anschließend wurden 40 mg (0.217 mmol) Methyl-4-aminobicyclo[2.2.2]octan-1-carboxylat zugegeben und das Gemisch wurde über Nacht bei RT gerührt. Das Reaktionsgemisch wurde mit Wasser und Acetonitril versetzt und mittels präparativer HPLC gereinigt (RP18 Säule, Laufmittel: Acetonitril/Wasser-Gradient unter Zusatz von 0.05% Ameisensäure). Es wurden 62 mg der Zielverbindung (70% d. Th.) erhalten.
LC-MS (Methode 2): Rₜ = 1.18 min
MS (ESpos): m/z = 498 (M+H)⁺
¹H NMR (500 MHz, DMSO-d₆) δ [ppm] = 7.57 - 7.68 (m, 1H), 7.21 - 7.30 (m, 3H), 6.86 (s, 1H), 6.51 (d, 1H), 5.43 (s, 2H), 3.58 (s, 3H), 2.43 (s, 3H), 2.39 (s, 3H), 1.94 - 2.01 (m, 6H), 1.78 - 1.85 (m, 6H).

### Beispiel 153

### 4-[({7-[(2,6-Difluorbenzyl)oxy]-2,5-dimethylpyrazolo[1,5-a]pyridin-3-yl}carbonyl)amino]bicyclo[2.2.2]octan-1-carbonsäure

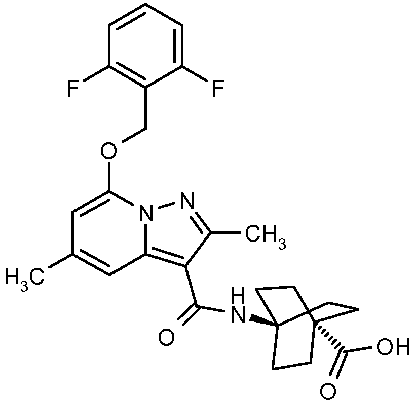

56 mg (0.113 mmol) Methyl-4-[({7-[(2,6-difluorbenzyl)oxy]-2,5-dimethylpyrazolo[1,5-a]pyridin-3-yl}carbonyl)amino]bicyclo[2.2.2]octan-1-carboxylat aus Beispiel 152 wurden in 1.1 ml THF/Methanol (5/1) gelöst und mit 8.1 mg (0.339 mmol) Lithiumhydroxid versetzt. Das Gemisch wurde 10 h bei 40°C gerührt. Nach Abkühlen wurde das Gemisch mit 20 µL Ameisensäure angesäuert und mittels präparativer HPLC gereinigt (RP18 Säule, Laufmittel: Acetonitril/Wasser-Gradient unter Zusatz von 0.05% Ameisensäure). Es wurden 41 mg der Zielverbindung (75% d. Th.) erhalten.
LC-MS (Methode 2): Rₜ = 0.98 min
MS (ESpos): m/z = 484 (M+H)⁺
¹H NMR (500 MHz, DMSO-d₆) δ [ppm] = 12.03 (br. s, 1H), 7.56 - 7.66 (m, 1H), 7.20 - 7.39 (m, 3H), 6.83 (s, 1H), 6.50 (s, 1H), 5.43 (s, 2H), 2.43 (s, 3H), 2.39 (s, 3H), 1.93 - 2.00 (m, 6H), 1.76 - 1.83 (m, 6H).

### Beispiel 154

### 7-[(2,6-Difluorbenzyl)oxy]-2,5-dimethyl-N-[2-(trifluormethyl)piperidin-4-yl]pyrazolo[1,5-a]pyridin-3-carboxamid

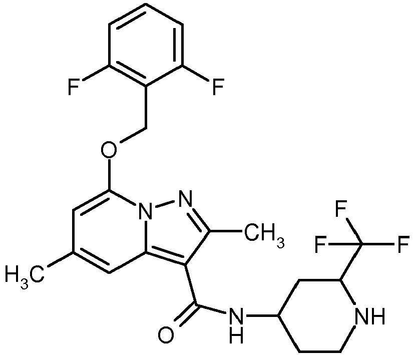

Eine Mischung von 57 mg 7-[(2,6-Difluorbenzyl)oxy]-2,5-dimethylpyrazolo[1,5-a]pyridin-3-carbonsäure (0.171 mmol) aus Beispiel 4A, 72 mg HATU (0.188 mmol) und 0.179 ml (1.025 mmol) N,N-Diisopropylethylamin in 1.1 ml DMF wurde 20 min bei RT gerührt. Anschließend wurden 42 mg (0.205 mmol) 2-(Trifluormethyl)piperidin-4-amin-Hydrochlorid (Beispiel 36A) zugegeben und das Gemisch wurde über Nacht bei RT gerührt. Das Reaktionsgemisch wurde mit Wasser und Acetonitril versetzt und mittels präparativer HPLC gereinigt (RP18 Säule, Laufmittel: Acetonitril/Wasser-Gradient unter Zusatz von 0.05% Ameisensäure). Es wurden 22 mg der Zielverbindung (27% d. Th.) erhalten.
LC-MS (Methode 2): Rₜ = 0.80 min
MS (ESpos): m/z = 483 (M+H)⁺
¹H NMR (500 MHz, DMSO-d₆) δ [ppm] = 7.58 - 7.66 (m, 1H), 7.44 (d, 1H), 7.22 - 7.31 (m, 3H), 6.53 (s, 1H), 5.44 (s, 2H), 4.19 - 4.27 (m, 1H), 3.53 - 3.62 (m, 1H), 2.78 - 2.92 (m, 2H), 2.49 (s, 3H), 2.45 - 2.47 (m, 1H), 2.41 (s, 3H), 1.82 - 1.89 (m, 1H), 1.58 - 1.79 (m, 3H).

### Beispiel 155

### Methyl-3-{[({7-[(2,6-difluorbenzyl)oxy]-2,5-dimethylpyrazolo[1,5-a]pyridin-3-yl}carbonyl)amino]methyl}adamantan-1-carboxylat

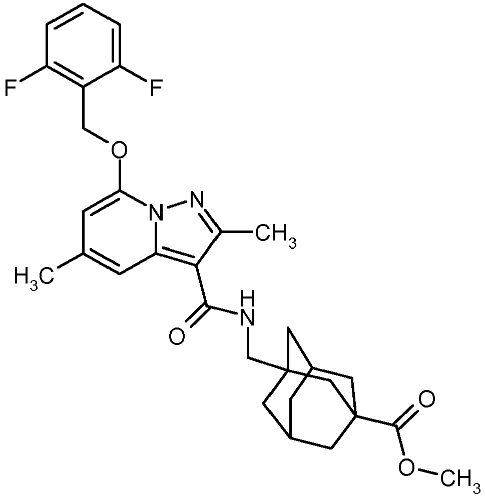

Eine Mischung von 52 mg 7-[(2,6-Difluorbenzyl)oxy]-2,5-dimethylpyrazolo[1,5-a]pyridin-3-carbonsäure (Reinheit 75%, 0.118 mmol) aus Beispiel 4A, 72 mg HATU (0.188 mmol) und 0.138 ml (0.792 mmol) N,N-Diisopropylethylamin in 0.7 ml DMF wurde 10 min bei RT gerührt. Anschließend wurden 53 mg (0.236 mmol) Methyl-3-(aminomethyl)adamantan-1-carboxylat aus Beispiel 37A in 0.5 ml DMF zugegeben und das Gemisch wurde über Nacht bei RT gerührt. Das Reaktionsgemisch wurde mit Wasser und Acetonitril versetzt und mittels präparativer HPLC gereinigt (RP18 Säule, Laufmittel: Acetonitril/Wasser-Gradient unter Zusatz von 0.05% Ameisensäure). Es wurden 35 mg der Zielverbindung (53% d. Th.) erhalten.
LC-MS (Methode 2): Rₜ = 1.21 min
MS (ESpos): m/z = 538 (M+H)⁺
¹H NMR (500 MHz, DMSO-d₆) δ [ppm] = 7.58 - 7.66 (m, 1H), 7.40 - 7.46 (m, 1H), 7.32 (s, 1H), 7.22 - 7.29 (m, 2H), 6.53 (d, 1H), 5.44 (s, 2H), 3.57 (s, 3H), 3.05 (d, 2H), 2.49 (s, 3H), 2.40 (s, 3H), 2.06 (br.s., 2H), 1.70 - 1.81 (m, 4H), 1.54 - 1.64 (m, 4H), 1.49 (s, 4H).

### Beispiel 156

### rac-N-[2-Amino-4-(methylsulfanyl)butyl]-7-[(2,6-difluorbenzyl)oxy]-2,5-dimethylpyrazolo[1,5-a]pyridin-3-carboxamid

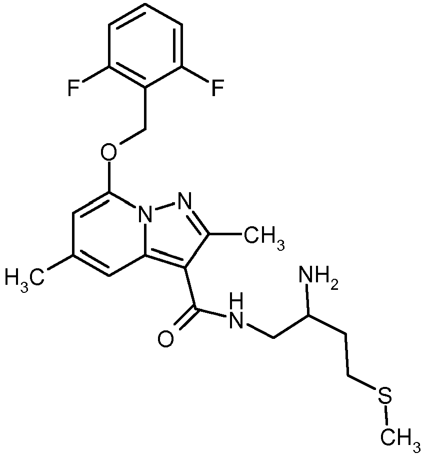

Eine Mischung von 70 mg 7-[(2,6-Difluorbenzyl)oxy]-2,5-dimethylpyrazolo[1,5-a]pyridin-3-carbonsäure (Reinheit 75%, 0.118 mmol) aus Beispiel 4A, 66 mg HATU (0.174 mmol) und 0.083 ml 0.474 mmol) N,N-Diisopropylethylamin in 0.7 ml DMF wurde 10 min bei RT gerührt. Diese Reaktionsmischung wurde zu einer Lösung von 53 mg (0.236 mmol) *rac*-4-(Methylsulfanyl)butan-1,2-diamin-Dihydrochlorid in 0.14 ml DMF bei 0°C zugegeben und 60 min gerührt. Das Reaktionsgemisch wurde mit Wasser und Acetonitril versetzt und mittels präparativer HPLC gereinigt (RP18 Säule, Laufmittel: Acetonitril/Wasser-Gradient unter Zusatz von 0.05% Ameisensäure). Es wurden 40 mg der Zielverbindung (54% d. Th.) erhalten.
LC-MS (Methode 2): Rₜ = 0.75 min
MS (ESpos): m/z = 449 (M+H)⁺
¹H NMR (500 MHz, DMSO-d₆) δ [ppm] = 7.56 - 7.67 (m, 2H), 7.42 (br.s, 1H), 7.23 - 7.29 (m, 2H), 6.59 (d, 1H), 5.45 (s, 2H), 3.35 - 3.54 (m, 3H), 2.59 - 2.66 (2H), 2.52 (s, 3H), 2.43 (s, 3H), 2.06 (s, 3H), 1.83 - 1.93 (m, 1H), 1.73 - 1.83 (m, 1H), 1.19 (br.s, 2H).

### Beispiel 157

### rac-N-[2-Amino-4-(methylsulfonyl)butyl]-7-[(2,6-difluorbenzyl)oxy]-2,5-dimethylpyrazolo[1,5-a]pyridin-3-carboxamid

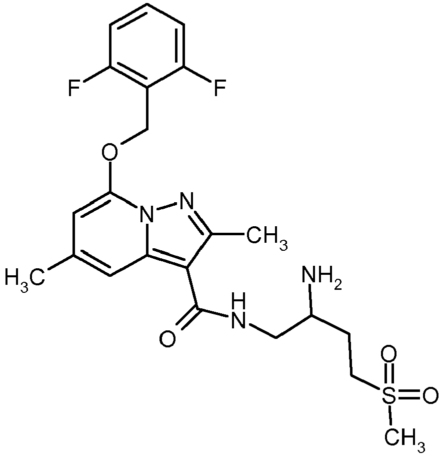

38 mg (0.085 mmol) *rac*-N-[2-Amino-4-(methylsulfanyl)butyl]-7-[(2,6-difluorbenzyl)oxy]-2,5-dimethylpyrazolo[1,5-a]pyridin-3-carboxamid aus Beispiel 156 wurden in 0.85 ml Dichlormethan gelöst und auf 0°C abgekühlt. Anschließend wurden 51 mg (0.213 mmol, Reinheit 72%) 3-Chlorbenzolcarboperoxosäure zugegeben und 1 h bei 0°C gerührt. Das Reaktionsgemisch wurde mit Dichlormethan verdünnt, zweimal mit 1 N wässrige Natriumhydroxid-Lösung und zweimal mit Wasser gewaschen und einrotiert. Das Rohprodukt wurde mittels präparativer HPLC gereinigt (RP18 Säule, Laufmittel: Acetonitril/Wasser-Gradient unter Zusatz von 0.05% Ameisensäure). Es wurden 11 mg der Zielverbindung (26% d. Th.) erhalten.
LC-MS (Methode 2): Rₜ = 0.68 min
MS (ESpos): m/z = 481 (M+H)⁺
¹H NMR (500 MHz, DMSO-d₆) δ [ppm] = 8.22 (s, 1H), 7.57 - 7.68 (m, 1H), 7.48 - 7.55 (m, 1H), 7.39 (s, 1H), 7.22 - 7.30 (m, 2H), 6.55 (d, 1H), 5.44 (s, 2H), 3.15 - 3.36 (m, z.T. überlagert mit dem Wasserpeak), 2.98 - 3.06 (m, 1H), 2.96 (s, 3H), 2.41 (s, 3H), 1.86 - 1.96 (m, 1H), 1.62 - 1.74 (m, 1H).

### Beispiel 158

### rac-N-(2-Amino-2-methylpentyl)-7-[(2,3-difluorbenzyl)oxy]-2,5-dimethylpyrazolo[1,5-a]pyridin-3-carbonsäureamid

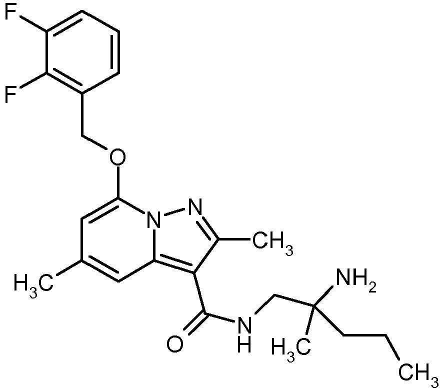

Eine Lösung von 35 mg (0.066 mmol) *rac*-{1-[({7-[(2,3-Difluorbenzyl)oxy]-2,5-dimethylpyrazolo[1,5-a]pyridin-3-yl}carbonyl)amino]-2-methylpentan-2-yl}carbamidsäure-tert.-butylester (Beispiel 118A) in 2 ml Dichlormethan wurde mit 0.079 ml (1.03 mmol) Trifluoressigsäure versetzt. Die erhaltene Lösung wurde über Nacht bei Raumtemperatur gerührt. Das Lösungsmittel wurde im Vakuum abgezogen und der Rückstand wurde durch präparative HPLC-Chromatographie (Methode 21) aufgereinigt, wodurch man 6 mg (27% d. Th.) der Zielverbindung erhielt.
LC-MS (Method 18): Rₜ = 8.91 min
MS (ESpos): m/z = 431.24 (M+H)⁺
¹H-NMR (600 MHz, DMSO-d₆): δ [ppm] = 0.86 (t, 3 H), 0.97 (s, 3 H), 1.25 - 1.39 (m, 2 H), 1.27 - 1.33 (m, 2 H), 2.38 (s, 3 H), 2.52 (s, 3 H), 3.13 (dd, 1 H), 3.15 - 3.20 (m, 1 H), 5.50 (s, 2 H), 6.52 (d, 1 H), 7.22 (t, 1 H), 7.28 - 7.34 (m, 1 H), 7.37 (s, 1 H), 7.48 (t, 1 H), 7.50 - 7.56 (m, 1 H).
¹³C-NMR (151 MHz, DMSO-d₆): δ [ppm] = 14.0, 14.8, 16.5, 21.4, 25.8, 43.3, 48.8, 52.1, 64.8, 93.9, 103.8, 108.7, 118.2, 125.2, 125.2, 126.2, 138.2, 141.8, 148.2, 148.4, 149.8, 150.7, 163.5.

### Beispiel 159

### rac-N-(2-Amino-2-methylpentyl)-7-(cyclohexylmethoxy)-2,5-dimethylpyrazolo[1,5-a]pyridin-3-carbonsäureamid

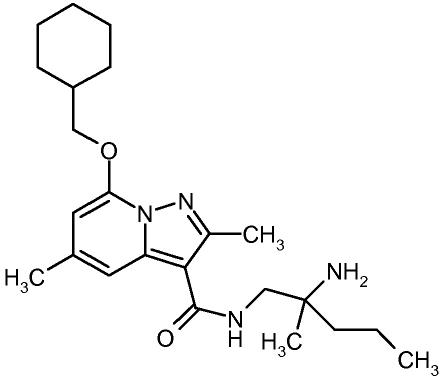

Eine Lösung von 40 mg (0.047 mmol, 59% rein) *rac*-[1-({[7-(Cyclohexylmethoxy)-2,5-dimethylpyrazolo[1,5-a]pyridin-3-yl]carbonyl}amino)-2-methylpentan-2-yl]carbamidsäure-tert.-butylester (Beispiel 121A) in 2 ml Dichlormethan wurde mit 0.072 ml (0.94 mmol) Trifluoressigsäure versetzt. Die erhaltene Lösung wurde über Nacht bei Raumtemperatur gerührt. Nach dem Ende der Umsetzung wurde das Lösungsmittel im Vakuum abgezogen und der Rückstand wurde durch präparative HPLC-Chromatographie (Methode 21) aufgereinigt, wodurch man 8 mg (43% d. Th.) der Zielverbindung erhielt.
LC-MS (Method 18): Rₜ = 10.32 min
MS (ESpos): m/z = 401.26 (M+H)⁺
¹H-NMR (500 MHz, DMSO-d₆): δ [ppm] = 0.87 (t, 3 H), 0.98 (s, 3 H), 1.03 - 1.14 (m, 2 H), 1.15-1.39 (m, 7H), 1.67 (d, 1H), 1.73 (d, 2H), 1.84-1.95 (m, 3H), 2.36 (s, 3 H), 2.54 (s, 3 H), 3.11 - 3.21 (m, 2 H), 4.10 (t, 2 H), 6.32 (s, 1 H), 7.20 (t, 1 H), 7.31 (s, 1 H).

### Beispiel 160

### rac-N-(2-Amino-2-methylpentyl)-2,5-dimethyl-7-(3-methylbutoxy)pyrazolo[1,5-a]pyridin-3-carbonsäureamid

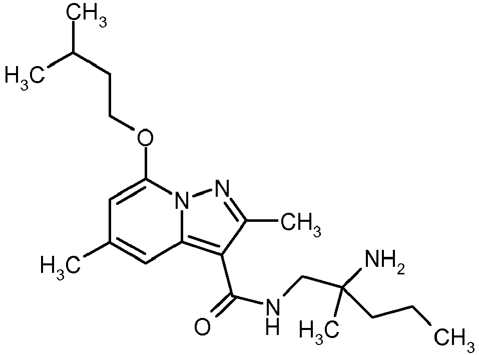

Eine Lösung von 80 mg (0.091 mmol, 54% rein) *rac*-[1-({[2,5-Dimethyl-7-(3-methylbutoxy)pyrazolo[1,5-a]pyridin-3-yl]carbonyl}amino)-2-methylpentan-2-yl]carbamidsäure-*tert*.-butylester (Beispiel 124A) in 4 ml Dichlormethan wurde mit 0.140 ml (1.82 mmol) Trifluoressigsäure versetzt. Die erhaltene Lösung wurde über Nacht bei Raumtemperatur gerührt. Nach dem Ende der Umsetzung wurde das Lösungsmittel im Vakuum abgezogen und der Rückstand wurde durch präparative HPLC-Chromatographie (Methode 21) aufgereinigt, wodurch man 17 mg (50% d. Th.) der Zielverbindung erhielt.
LC-MS (Method 18): Rₜ = 8.88 min
MS (ESpos): m/z = 375.25 (M+H)⁺
¹H-NMR (500 MHz, DMSO-d₆): δ [ppm] = 0.87 (t, 3 H), 0.96 (d, 6 H), 0.98 (s, 3 H), 1.21 - 1.38 (m, 4 H), 1.74 (q, 2 H), 1.79 - 1.90 (m, 1 H), 2.37 (s, 3 H), 2.54 (s, 3 H), 3.09 - 3.22 (m, 2 H), 4.31 (t, 2 H), 6.35 (s, 1 H), 7.19 (t, 1 H), 7.31 (s, 1 H).
¹³C-NMR (126 MHz, DMSO-d₆): δ [ppm] = 14.0, 14.8, 16.6, 21.5, 22.3, 24.4, 25.8, 36.9, 43.4, 48.8, 52.0, 68.1, 93.1, 104.3, 108.4, 138.4, 141.8, 149.1, 150.5, 163.6.

### Beispiel 161

### rac-N-(2-Amino-2-methylpentyl)-7-[(2,6-difluor-3-methoxybenzyl)oxy]-2,5-dimethylpyrazolo[1,5-a]pyridin-3-carbonsäureamid

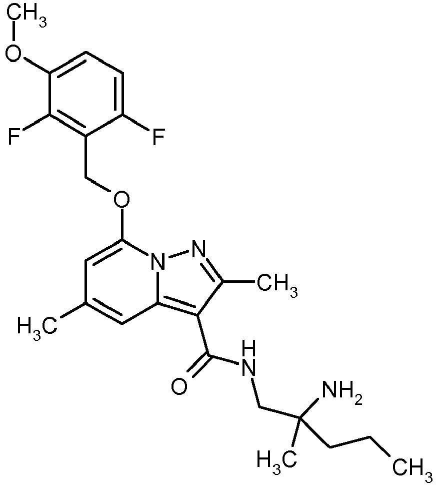

*rac-*{1-[({7-[(2,6-Difluor-3-methoxybenzyl)oxy]-2,5-dimethylpyrazolo[1,5-a]-pyridin-3-yl}carbonyl)amino]-2-methylpentan-2-yl}carbamidsäure-*tert*.-butylester (Beispiel 125A) in 4 ml Dichlormethan wurde mit 3 ml Trifluoressigsäure versetzt. Die erhaltene Lösung wurde 3 h bei Raumtemperatur gerührt. Nach dem Ende der Umsetzung wurde das Lösungsmittel im Vakuum abgezogen und der Rückstand wurde durch präparative HPLC-Chromatographie (Methode 21) aufgereinigt, wodurch man 10 mg (11% d. Th. über 2 Stufen) der Zielverbindung erhielt.
LC-MS (Method 18): Rₜ = 8.94 min
MS (ESpos): m/z = 461.31 (M+H)⁺
¹H-NMR (500 MHz, DMSO-d₆): δ [ppm] = 0.86 (t, 3 H), 0.97 (s, 3 H), 1.25 - 1.39 (m, 4 H), 2.40 (s, 3 H), 3.10 - 3.20 (m, 2 H), 3.86 (s, 3 H), 5.43 (s, 2 H), 6.64 (d, 1 H), 7.16 - 7.24 (m, 2 H), 7.35 (q, 1 H), 7.38 (s, 1 H).

### Beispiel 162

### rac-N-(2-Amino-2-methylpentyl)-7-[(3-cyano-2,6-difluorbenzyl)oxy]-2,5-dimethylpyrazolo[1,5-a]pyridin-3-carbonsäureamid

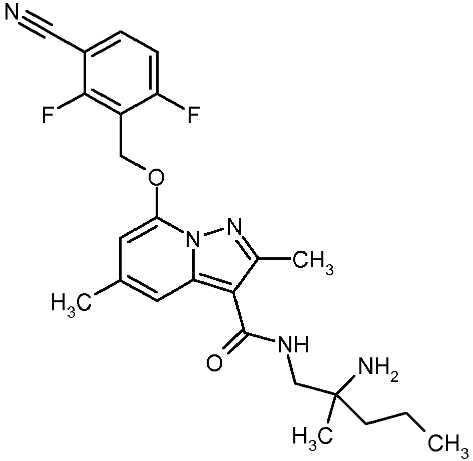

Rohes *rac*-{1-[({7-[(3-cyano-2,6-difluorobenzyl)oxy]-2,5-dimethylpyrazolo[1,5-a]pyridin-3-yl}carbonyl)amino]-2-methylpentan-2-yl}carbamidsäure-tert.-butylester (Beispiel 126A) in 4 ml Dichlormethan wurde mit 2 ml Trifluoressigsäure versetzt. Die erhaltene Lösung wurde 3 h bei Raumtemperatur gerührt. Das Lösungsmittel wurde im Vakuum abgezogen und der Rückstand wurde durch präparative HPLC-Chromatographie (Methode 21) aufgereinigt, wodurch man 4.5 mg (5% d. Th. über 2 Stufen) der Zielverbindung erhielt.
LC-MS (Method 18): Rₜ = 8.41 min
MS (ESpos): m/z = 456.29 (M+H)⁺
¹H-NMR (500 MHz, DMSO-d₆): δ [ppm] = 0.86 (t, 3 H), 0.99 (s, 3 H), 1.26 - 1.41 (m, 4 H), 2.40 (s, 3 H), 3.11 - 3.23 (m, 2 H), 5.50 (s, 2 H), 6.55 (d, 1 H), 7.25 (t, 1 H), 7.40 (s, 1 H), 7.53 (t, 1 H), 8.18 - 8.23 (m, 1 H).

### Beispiel 163

### rac-N-(2-Amino-2-methylpentyl)-7-[(2,6-difluorbenzyl)oxy]-2-methylpyrazolo[1,5-a]pyridin-3-carbonsäureamid

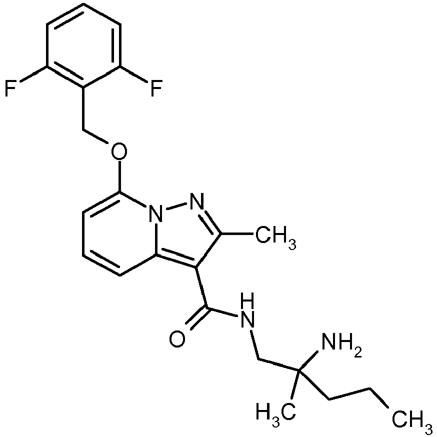

Eine Lösung von 45 mg (0.046 mmol, 53% rein) *rac*-{1-[({7-[(2,6-Difluorbenzyl)oxy]-2-methylpyrazolo[1,5-a]pyridin-3-yl}carbonyl)amino]-2-methylpentan-2-yl}carbamidsäure-tert.-butylester (Beispiel 131A) in 4 ml Dichlormethan wurde mit 0.5 ml (6.49 mmol) Trifluoressigsäure versetzt. Die erhaltene Lösung wurde 2 h bei Raumtemperatur gerührt. Das Lösungsmittel wurde im Vakuum abgezogen und der Rückstand wurde durch präparative HPLC-Chromatographie (Methode 21) aufgereinigt, wodurch man 6.5 mg (34% Ausbeute) der Zielverbindung als ein weißes Pulver erhielt.
LC-MS (Method 18): Rₜ = 8.53 min
MS (ESpos): m/z = 417.22 (M+H)⁺
¹H-NMR (500 MHz, DMSO-d₆): δ [ppm] = 0.86 (t, 3 H), 0.97 (s, 3 H), 1.25 - 1.39 (m, 4 H), 2.52 (s, 3 H), 3.12 - 3.23 (m, 2 H), 5.47 (s, 2 H), 6.65 (d, 1 H), 7.25 (t, 2 H), 7.32 (t, 1 H), 7.41 (t, 1 H), 7.57 (d, 1 H), 7.61 (d, 1 H).

### Beispiel 164

### rac-N-(2-Amino-2-methylpentyl)-7-[(2-chlor-6-fluorbenzyl)oxy]-2-methylpyrazolo[1,5-a]pyridin-3-carbonsäureamid

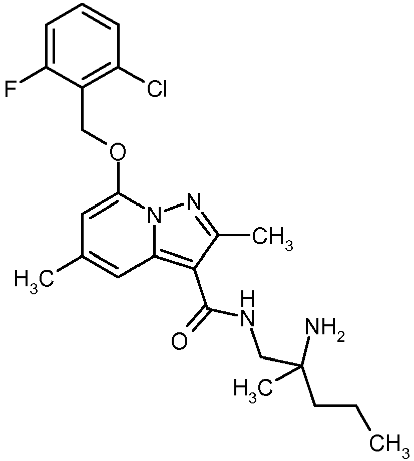

Eine Lösung von 40 mg (0.073 mmol) *rac*-{1-[({7-[(2-Difluorbenzyl)oxy]-2,5-dimethylpyrazolo[1,5-a]pyridin-3-yl}carbonyl)amino]-2-methylpentan-2-yl}carbamidsäure-tert.-butylester (Beispiel 138A) in 2 ml Dichlormethan wurde mit 0.113 ml Trifluoressigsäure versetzt. Die erhaltene Mischung wurde 18 Stunden lang bei Raumtemperatur gerührt und dann auf 40°C erhitzt. Weitere Trifluoressigsäure (0.169 ml) wurde portionsweise im Verlauf von 3 Stunden zugegeben. Die Reaktionsmischung wurde im Vakuum eingedampft und der verbliebene Rückstand wurde durch präparative HPLC-Chromatographie (Methode 21) aufgereinigt, wodurch man 11.0 mg (33% Ausbeute) der Zielverbindung erhielt.
LC-MS (Method 18): Rₜ = 9.08 min; m/z = 447.24 (M+H)⁺
¹H-NMR (500 MHz, DMSO-d₆): δ [ppm] = 0.83 - 0.90 (m, 3 H), 1.01 (s, 3 H), 1.28 - 1.39 (m, 4 H), 2.41 (s, 3 H), 2.49 (br. s, 3 H), 3.20 (qd, 2 H), 5.47 (s, 2 H), 6.58 (s, 1 H), 7.29 (t, 1 H), 7.37 - 7.43 (m, 2H), 7.50 (d, 1 H), 7.56 - 7.63 (m, 1H).
¹³C-NMR (126 MHz, DMSO-d₆): δ [ppm] = 13.8, 14.9, 16.4, 21.4, 25.1, 42.5, 48.2, 52.9, 62.5, 93.8, 104.5, 108.7, 115.1, 120.5, 125.8, 132.6, 135.8, 138.4, 141.8, 148.6, 150.8, 161.6, 163.7.

### Beispiel 165

### rac-N-(2-Amino-2-methylpentyl)-2,5-dimethyl-7-[4,4,4-trifluor-3-(trifluormethyl)butoxy]pyrazolo[1,5-a]pyridin-3-carbonsäureamid

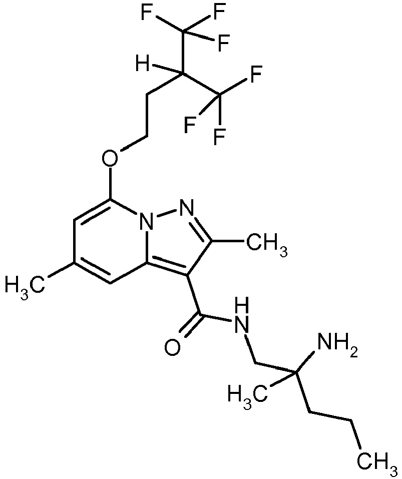

Eine Lösung von 30 mg (0.051 mmol) *rac*-{1-[({2,5-Dimethyl-7-[4,4,4-trifluor-3-(trifluormethyl)butoxy]pyrazolo[1,5-a]pyridin-3-yl}carbonyl)amino]-2-methylpentan-2-yl}carbamidsäure-*tert*.-butylester (Beispiel 142A) in 2 ml Dichlormethan wurde mit Trifluoressigsäure (0.117 ml) versetzt. Die Reaktionsmischung wurde 18 Stunden lang bei Raumtemperatur gerührt. Das Lösungsmittel wurde im Vakuum abgedampft und der Rückstand wurde zwischen Dichlormethan und Wasser verteilt. Die organische Phase wurde abgetrennt und im Vakuum eingedampft und der Rückstand wurde durch präparative HPLC-Chromatographie (Methode 21) aufgereinigt, wodurch man 1.68 mg (7% Ausbeute) der Zielverbindung erhielt.
LC-MS (Method 18): Rₜ = 9.47 min; m/z = 483.25 (M+H)⁺
¹H-NMR (600 MHz, DMSO-d₆): δ [ppm] = 0.86 (t, 3 H), 0.98 (s, 3 H), 1.24 - 1.40 (m, 4 H), 2.37 (s, 3 H), 2.39 (q, 2 H), 2.54 (s, 3H), 3.07 - 3.22 (m, 2 H), 4.19 - 4.33 (m, 1 H), 4.44 (t, 2 H), 6.38 (d, 1 H), 7.24 (t, 1 H), 7.36 (s, 1 H).
¹³C-NMR (151 MHz, DMSO-d₆): δ [ppm] = 14.2, 14.8, 16.5, 21.4, 22.8, 25.3, 43.1, 43.4, 48.6, 52.3, 66.4, 94.0, 104.5, 108.8, 123.1, 138.3, 141.8, 148.3, 150.7, 163.6.

### Beispiel 166

### rac-N-(2-Amino-2-methylpentyl)-7-[(3-fluorpyridin-2-yl)methoxy]-2,5-dimethylpyrazolo[1,5-a]pyridin-3-carbonsäureamid

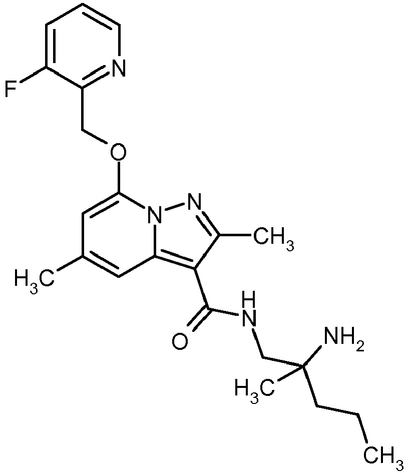

20 mg (0.039 mmol) *rac*-{1-[({7-[(3-fluoropyridin-2-yl)methoxy]-2,5-dimethylpyrazolo[1,5-a]pyridin-3-yl}carbonyl)amino]-2-methylpentan-2-yl}carbamidsäure-*tert*.-butylester (Beispiel 144A) wurden in 1 ml Trifluoressigsäure gelöst. Die Reaktionsmischung wurde 1 Stunde lang auf 50°C erhitzt. Wasser wurde zugefügt, und der pH-Wert wurde mit 2 N Natronlauge auf ∼ 9 eingestellt. Die wässrige Phase wurde mit Dichlormethan extrahiert, abgetrennt und im Vakuum eingedampft. Der Rückstand wurde durch präparative HPLC-Chromatographie (Methode 21) aufgereinigt, wodurch man 1.59 mg (10% Ausbeute) der Zielverbindung erhielt.
LC-MS (Method 18): Rₜ = 8.17 min; m/z = 414.28 (M+H)⁺
¹H-NMR (400 MHz, CDCl₃): δ [ppm] = 0.87 - 0.95 (m, 3 H), 1.12 (s, 3 H), 1.28 - 1.52 (m, 4 H), 2.36 (s, 3 H), 2.68 (s, 3 H), 3.14 - 3.33 (m, 1 H), 3.33 - 3.45 (m, 1 H), 5.56 (s, 2 H), 6.24 (s, 1 H), 6.43 (br. s., 1 H), 7.28 - 7.37 (m, 1 H), 7.39 - 7.48 (m, 1 H), 7.59 (s, 1 H), 8.44 (br. s., 1 H).
¹³C-NMR (101 MHz, CDCl₃): δ [ppm] = 15.1, 15.4, 17.4, 22.2, 26.1, 44.4, 48.8, 53.2, 67.7, 94.8, 104.0, 110.3, 124.1, 125.9, 139.5, 142.7, 144.0, 145.7, 148.9, 150.9, 158.5.

### Beispiel 167

### rac-N-(2-Amino-2-methylpentyl)-5-cyclopropyl-7-[(2,6-difluorbenzyl)oxy]-2-methylpyrazolo[1,5-a]pyridin-3-carbonsäureamid

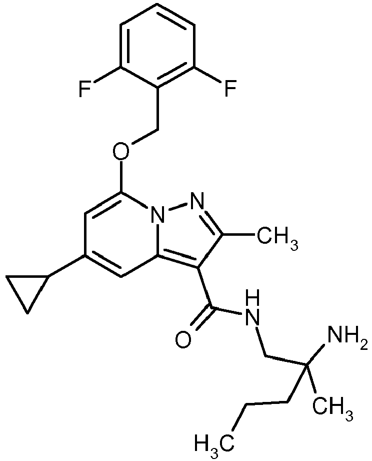

1 ml Trifluoressigsäure wurde zu einer Lösung von 125 mg (0.2 mmol) *rac*-{1-[({5-Cyclopropyl-7-[(2,6-difluorbenzyl)oxy]-2-methylpyrazolo[1,5-a]pyridin-3-yl}carbonyl)amino]-2-methylpentan-2-yl}carbamidsäure-*tert*.-butylester (Beispiel 150A) in 4 ml Dichlormethan gegeben. Die Reaktionsmischung wurde 1 h bei Raumtemperatur gerührt und im Vakuum eingeengt. Der Rückstand wurde durch Chromatographie an SCX-2-Kieselgel (mobile Phase: Methanol dann 20% (2 M Ammoniak in Methanol) in Dichlormethan) aufgereinigt. Hierdurch erhielt man 40 mg der Zielverbindung (39% d. Th.).
LC-MS (Method 20): Rₜ = 3.39 min; m/z = 457 (M+H)⁺
1H-NMR (400 MHz, DMSO-d₆): δ [ppm] = 0.82 - 0.87 (m, 5H), 0.95 (s, 3H), 1.03 (ddd, 2H), 1.22 - 1.35 (m, 4H), 1.39 (s, 1H), 1.95 - 2.04 (m, 1H), 3.06 - 3.18 (m, 3H), 5.44 (s, 2H), 6.29 (d, 1H), 7.17 - 7.29 (m, 4H), 7.54 - 7.63 (m, 1H).

### Beispiel 168

### ent-N-(2-Amino-5,5,5-trifluor-2-methylpentyl)-2,5-dimethyl-7-[(2,3,6-trifluorbenzyl)oxy]pyrazolo[1,5-a]pyridin-3-carboxamid (Enantiomer B)

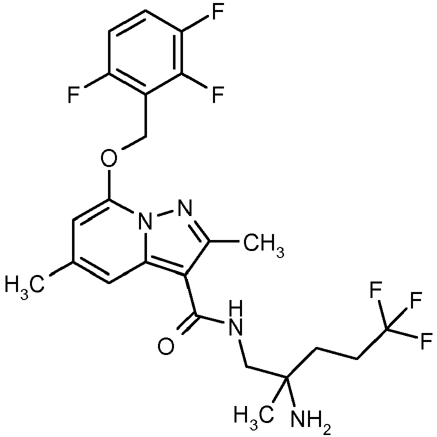

61 mg (0.06 mmol) *ent*-Benzyl-{1-[({2,5-dimethyl-7-[(2,3,6-trifluorbenzyl)oxy]pyrazolo[1,5-a]pyridin-3-yl}carbonyl)amino]-5,5,5-trifluor-2-methylpentan-2-yl}carbamat Trifluoroacetat (Enantiomer B) aus Beispiel 153A wurden in 6.5 ml Ethanol gelöst, mit 14 µl (0.18 mmol) Trifluoressigsäure und 2 mg 10%igem Palladium auf Aktivkohle versetzt und 1 Stunde bei Normaldruck hydriert. Die Reaktionsmischung wurde mittels Millipore-Filter filtriert und das Filtrat einrotiert. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 23 mg der Zielverbindung (74% d. Th.) erhalten.
LC-MS (Methode 2): Rₜ = 0.79 min
MS (ESpos): m/z = 503 (M+H)⁺
¹H-NMR (400 MHz, DMSO-d₆): δ [ppm] = 1.01 (s, 3H), 1.47 - 1.67 (m, 4H), 2.24 - 2.47 (m, 5H), 2.50 (s, 3H; überlagert vom Lösungsmittelpeak), 3.14 - 3.25 (m, 2H), 5.49 (s, 2H), 6.55 (s, 1H), 7.27 - 7.37 (m, 2H), 7.41 (t, 1H), 7.64 - 7.75 (m, 1H).

### Beispiel 169

### ent-N-(2-Amino-2-methylpentyl)-2-cyclopropyl-5-methyl-7-[(2,3,6-trifluorbenzyl)oxy]pyrazolo[1,5-a]pyridin-3-carboxamid (Enantiomer B)

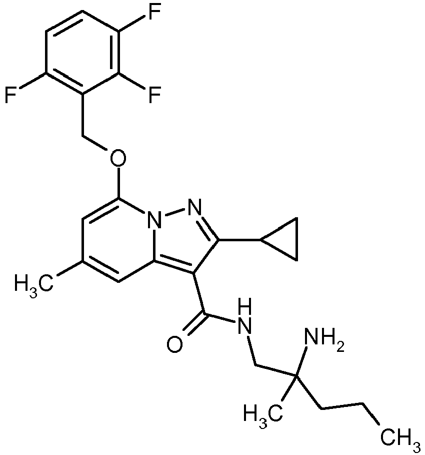

82 mg (0.11 mmol) *ent*-Benzyl-{1-[({2-cyclopropyl-5-methyl-7-[(2,3,6-trifluorbenzyl)oxy]pyrazolo[1,5-a]pyridin-3-yl}carbonyl)amino]-2-methylpentan-2-yl}carbamat Trifluoroacetat (Enantiomer B) aus Beispiel 158A wurden in 12 ml Ethanol gelöst, mit 26 µl (0.34 mmol) Trifluoressigsäure und 3.6 mg 10%igem Palladium auf Aktivkohle versetzt und 1.5 Stunden bei Normaldruck hydriert. Die Reaktionsmischung wurde mittels Millipore-Filter filtriert und das Filtrat einrotiert. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 53 mg der Zielverbindung (98% d. Th.) erhalten.
LC-MS (Methode 2): Rₜ = 0.78 min
MS (ESpos): m/z = 475 (M+H)⁺
¹H-NMR (400 MHz, DMSO-d₆): δ = 0.83 - 0.94 (m, 5H), 0.96 - 1.04 (m, 5H), 1.25 - 1.40 (m, 4H), 1.60 (br. s, 2H), 2.31 - 2.42 (m, 4H), 3.12 - 3.25 (m, 2H), 5.49 (s, 2H), 6.53 (s, 1H), 7.25 - 7.34 (m, 1H), 7.38 (t, 1H), 7.46 (s, 1H), 7.62 - 7.73 (m, 1H).

### Beispiel 170

### ent-N-(2-Amino-5,5,5-trifluor-2-methylpentyl)-2-cyclopropyl-5-methyl-7-[(2,3,6-trifluorbenzyl)oxy]pyrazolo[1,5-a]pyridin-3-carboxamid (Enantiomer B)

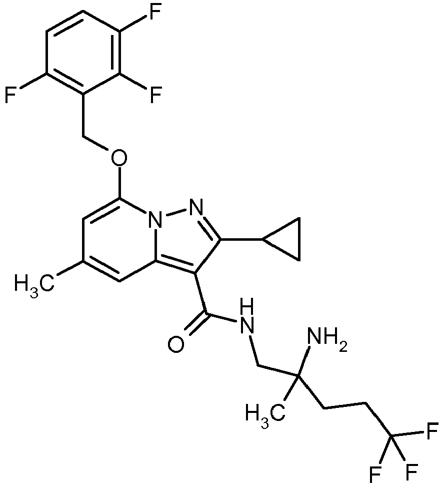

79 mg (0.10 mmol) *ent*-Benzyl-{1-[({2-cyclopropyl-5-methyl-7-[(2,3,6-trifluorbenzyl)oxy]pyrazolo[1,5-a]pyridin-3-yl}carbonyl)amino]-5,5,5-trifluor-2-methylpentan-2-yl}carbamat Trifluoroacetat (Enantiomer B) aus Beispiel 159A wurden in 11 ml Ethanol gelöst, mit 23 µl (0.30 mmol) Trifluoressigsäure und 3.2 mg 10%igem Palladium auf Aktivkohle versetzt und 2 Stunden bei Normaldruck hydriert. Es wurden nochmals 23 µl (0.30 mmol) Trifluoressigsäure und 3.2 mg 10%igem Palladium auf Aktivkohle hinzugegeben und das Gemisch wurde 45 min bei Normaldruck hydriert. Die Reaktionsmischung wurde mittels Millipore-Filter filtriert und das Filtrat einrotiert. Der Rückstand wurde erneut in 11 ml Ethanol gelöst, mit 77 µl (1 mmol) Trifluoressigsäure und 3.2 mg 10%igem Palladium auf Aktivkohle versetzt und 45 min bei Normaldruck hydriert. Die Reaktionsmischung wurde mittels Millipore-Filter filtriert und das Filtrat einrotiert. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingedampft. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 53 mg der Zielverbindung (97% d. Th.) erhalten.
LC-MS (Methode 2): Rₜ = 0.81 min
MS (ESpos): m/z = 529 (M+H)⁺
¹H-NMR (400 MHz, DMSO-d₆): δ = 0.86 - 0.92 (m, 2H), 0.93 - 1.00 (m, 2H), 1.02 (s, 3H), 1.49 - 1.58 (m, 2H), 1.64 (br. s, 2H), 2.26 - 2.44 (m, 6H), 3.17 - 3.28 (m, 2H), 5.49 (s, 2H), 6.53 (s, 1H), 7.25 - 7.33 (m, 1H), 7.40 (s, 1H), 7.51 (t, 1H), 7.62 - 7.73 (m, 1H).

### Beispiel 171

### rac-N-(2-Amino-2-methylpentyl)-7-[(2,6-difluorbenzyl)oxy]-5-methoxy-2-methylpyrazolo[1,5-a]pyridin-3-carbonsäureamid

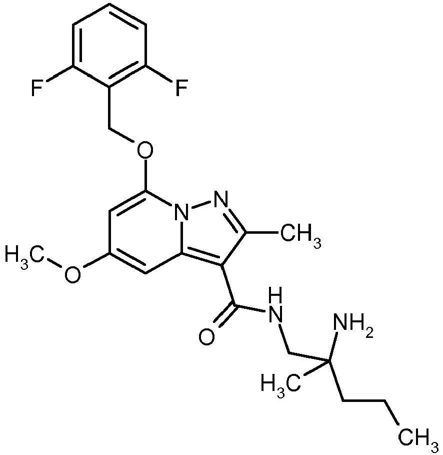

Eine Lösung von 118 mg (0.157 mmol, Reinheit 73%) *rac*-{1-[({7-[(2,6-Difluorbenzyl)oxy]-5-methoxy-2-methylpyrazolo[1,5-a]pyridin-3-yl}carbonyl)amino]-2-methylpentan-2-yl}carbamidsäure-tert.-butylester (Beispiel 164A) in 12 ml Dichlormethan wurde mit 1.5 ml Trifluoressigsäure versetzt. Die erhaltene Mischung wurde zwei Stunden lang bei Raumtemperatur gerührt. Die Reaktionsmischung wurde im Vakuum eingedampft und der verbliebene Rückstand wurde durch präparative HPLC-Chromatographie (Methode 21) aufgereinigt, wodurch man 37 mg (Ausbeute 53%) an Produkt erhielt.
LC-MS (Methode 18): Rₜ = 8.81 min; m/z = 447.17 (M+H)⁺
¹H-NMR (600 MHz, DMSO-d₆): δ [ppm] = 0.85 (t, J = 6.7 Hz, 3H), 0.97 (s, 3H), 1.21 - 1.40 (m, 4H), 2.47 (s, 3H), 3.06 - 3.20 (m, 2H), 3.85 (s, 3H), 5.43 (s, 2H), 6.37 (d, J = 2.4 Hz, 1H), 7.01 (d, J = 2.4 Hz, 1H), 7.15 (t, J = 5.8 Hz, 1H), 7.21 - 7.30 (m, 2H), 7.52 - 7.67 (m, 1H).
¹³C-NMR (151 MHz, DMSO-d₆): δ [ppm] = 14.0, 14.8, 16.5, 25.6, 43.3, 48.6, 52.0, 55.7, 59.5, 85.4, 88.7, 104.5, 110.6, 112.0, 132.8, 142.5, 149.5, 151.1, 159.3, 161.3, 163.6.

### Beispiel 172

### rac-N-(2-Amino-2-methylpentyl)-7-[(2,6-difluorbenzyl)oxy]-5-ethyl-2-methylpyrazolo[1,5-a]pyridin-3-carbonsäureamid

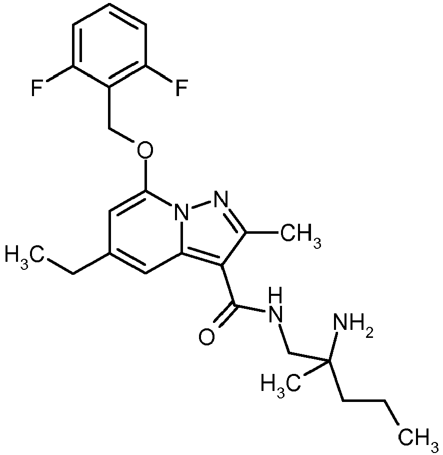

Eine Lösung von 19 mg (0.033 mmol, Reinheit 95%) *rac*-{1-[({7-[(2,6-Difluorbenzyl)oxy]-5-ethyl-2-methylpyrazolo[1,5-a]pyridin-3-yl}carbonyl)amino]-2-methylpentan-2-yl}carbamidsäure-tert.-butylester (Beispiel 169A) in 1 ml Dichlormethan wurde mit 0.15 ml Trifluoressigsäure versetzt. Die erhaltene Mischung wurde 2 Stunden lang bei Raumtemperatur gerührt. Die Reaktionsmischung wurde im Vakuum eingedampft und der verbliebene Rückstand wurde durch präparative HPLC-Chromatographie (Methode 21) aufgereinigt, wodurch man 4.5 mg (Ausbeute 15%) an Produkt erhielt.
LC-MS (Methode 18): Rₜ = 5.51 min; m/z = 445.31 (M+H)⁺
¹H-NMR (500 MHz, DMSO-d₆): δ [ppm] = 0.87 (t, J = 6.6 Hz, 3H), 0.98 (s, 3H), 1.26 (t, J = 7.5 Hz, 3H), 1.28 - 1.37 (m, 4H), 2.50 (s, 3H), 2.70 (q, J = 7.3 Hz, 2H), 3.05 - 3.23 (m, 2H), 5.46 (s, 2H), 6.57 (d, J = 1.2 Hz, 1H), 7.20 - 7.30 (m, 3H), 7.40 (s, 1H), 7.56 - 7.68 (m, 1H).
¹³C-NMR (126 MHz, DMSO-d₆): δ [ppm] = 13.9, 14.3, 14.8, 16.5, 25.8, 28.3, 43.9, 48.7, 53.2, 59.6, 95.1, 105.0, 107.4, 110.9, 111.9, 132.9, 142.0, 145.0, 149.0, 150.8, 160.5, 163.0.

### Beispiel 173

### 7-[(2,6-Difluorbenzyl)oxy]-2,5-dimethyl-N-(4-methylpiperazin-1-yl)pyrazolo[1,5-a]pyridin-3-carboxamid

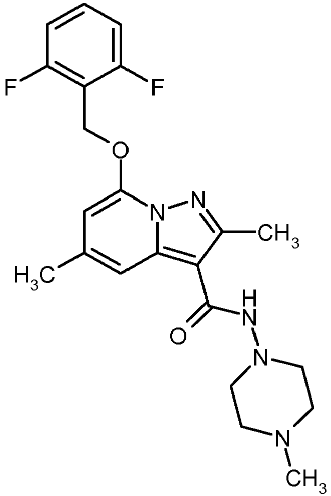

30 mg (0.09 mmol) 7-[(2,6-Difluorbenzyl)oxy]-2,5-dimethylpyrazolo[1,5-a]pyridin-3-carbonsäure aus Beispiel 4A wurden mit 45 mg (0.12 mmol) HATU und 0.08 ml (0.45 mmol) N,N-Diisopropylethylamin in 0.30 ml DMF vorgelegt und 10 min bei Raumtemperatur gerührt. Anschließend wurden 14 mg (0.12 mmol) 4-Methylpiperazin-1-amin zur Reaktionslösung gegeben und das Gemisch wurde über Nacht bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert. Es wurden 23 mg der Zielverbindung (58% d. Th.) erhalten.
LC-MS (Methode 2): Rₜ = 0.62 min
MS (ESpos): m/z = 430 (M+H)⁺
¹H-NMR (400 MHz, DMSO-d₆): δ = 2.29 (s, 3H), 2.31 - 2.54 (m, 11H; überlagert mit Lösungsmittelpeak), 2.88 (s, 3H), 5.43 (s, 2H), 6.53 (s, 1H), 7.20 - 7.29 (m, 3H), 7.57 - 7.67 (m, 1H), 8.50 (s, 1H).

In Analogie zu Beispiel 173 wurden die in Tabelle 8 gezeigten Beispielverbindungen hergestellt, indem 7-[(2,6-Difluorbenzyl)oxy]-2,5-dimethylpyrazolo[1,5-a]pyridin-3-carbonsäure aus Beispiel 4A mit den entsprechenden, kommerziell erhältlichen oder zuvor beschriebenen Aminen [Hydrazide] (1.1 - 5 Äquivalente), HATU (1.1 - 4.5 Äquivalente) und *N,N*-Diisopropylethylamin (3 - 12 Äquivalente) in DMF oder in DMF/Dichlormethan (1/1) unter den beschriebenen Reaktionsbedingungen (Reaktionszeit: 1 - 48 h; Temperatur: 0°C - RT, -20°C, RT oder 60°C) umgesetzt wurden.

Beispielhafte Aufarbeitung der Reaktionsmischung:
Die Reaktionslösung wurde mit Wasser versetzt und der entstandene Feststoff ca. 30 min bei Raumtemperatur verrührt. Anschließend wurde der Feststoff abfiltriert, gut mit Wasser gewaschen und im Hochvakuum getrocknet.

Alternativ dazu wurde das Reaktionsgemisch mit Wasser, TFA oder Ameisensäure verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Dickschichtchromatographie oder Kieselgelchromatographie gereinigt (Laufmittel: Dichlormethan/Methanol oder Dichlormethan/2 M Ammoniak in Methanol). Die produkthaltigen Fraktionen wurden eingedampft.

Die Produktfraktionen der Reinigungen wurden ggf. in Dichlormethan aufgenommen und zweimal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

**Tabelle 8:**

| **Beispiel** | **IUPAC-Name / Struktur (Ausbeute)** | **Analytische Daten** |
|---|---|---|
| **174** | 7-[(2,6-Difluorbenzyl)oxy]-2,5-dimethyl-N'-(piperidin-1-ylacetyl)pyrazolo[1,5-a]pyridin-3-carbohydrazid | ¹H-NMR (400 MHz, DMSO-d₆): δ = 1.35 - 1.46 (m, 2H), 1.49 - 1.61 (m, 4H), 2.41 (s, 3H), 3.01 (br. s, 2H), 5.44 (s, 2H), 6.58 (s, 1H), 7.20 - 7.29 (m, 2H), 7.39 (s, 1H), 7.58 - 7.68 (m, 1H), 9.38 (br. s, 1H), 9.58 (br. s, 1H), [weitere Signale unter Lösungsmittelpeaks verborgen]. LC-MS (Methode 7): Rₜ = 2.33 min MS (ESpos): m/z = 472 (M+H)⁺ |
| | 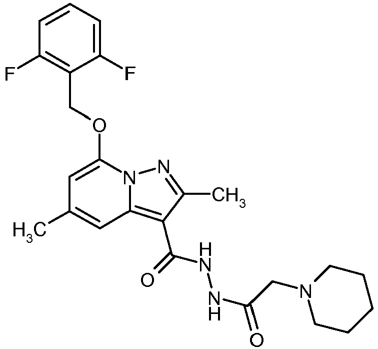 | |
| | (37% d. Th.) | |
| **175** | 7-[(2,6-Difluorbenzyl)oxy]-2,5-dimethyl-N'-[(2-oxopyrrolidin-1-yl)acetyl]pyrazolo[1,5-a]pyridin-3-carbohydrazid Trifluoracetat | LC-MS (Methode 2): Rₜ = 0.79 min MS (ESpos): m/z = 472 (M-TFA+H)⁺ |
| | 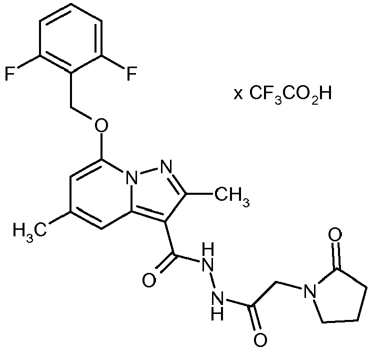 | |
| | (27% d. Th.) | |
| **176** | 7-[(2,6-Difluorbenzyl)oxy]-N-[(1*S*)-2-hydroxy-1-(2-thienyl)ethyl]-2,5-dimethylpyrazolo[1,5-a]pyridin-3-carboxamid | ¹H-NMR (400 MHz, DMSO-d₆): δ = 2.40 (s, 3H), 3.78 (t, 2H), 5.10 (t, 1H), 5.32 (q, 1H), 5.46 (s, 2H), 6.56 (s, 1H), 6.95 - 7.00 (m, 1H), 7.07 (d, 1H), 7.21 - 7.30 (m, 2H), 7.35 - 7.39 (m, 2H), 7.57 - 7.67 (m, 1H), 7.74 (d, 1H), [weiteres Signal unter Lösungsmittelpeak verborgen]. LC-MS (Methode 2): Rₜ = 0.95 min MS (ESpos): m/z = 458 (M+H)⁺ |
| | 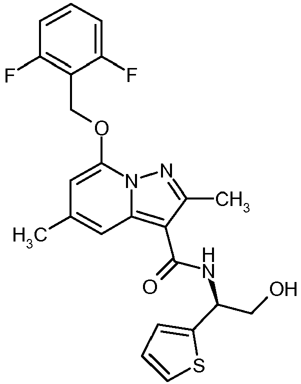 | |
| | (46% d. Th.) | |
| **177** | 7-[(2,6-Difluorbenzyl)oxy]-N-[(1*S*)-2-hydroxy-1-(5-methyl-2-furyl)ethyl]-2,5-dimethylpyrazolo[1,5-a]pyridin-3-carboxamid | ¹H-NMR (400 MHz, DMSO-d₆): δ = 2.23 (s, 3H), 2.40 (s, 3H), 3.68 - 3.79 (m, 2H), 4.97 (br. s, 1H), 5.12 (q, 1H), 5.44 (s, 2H), 5.98 (d, 1H), 6.16 (d, 1H), 6.55 (s, 1H), 7.20 - 7.30 (m, 2H), 7.32 (s, 1H), 7.54 (d, 1H), 7.58 - 7.67 (m, 1H), [weiteres Signal unter Lösungsmittelpeak verborgen]. LC-MS (Methode 2): Rₜ = 0.98 min MS (ESpos): m/z = 456 (M+H)⁺ |
| | 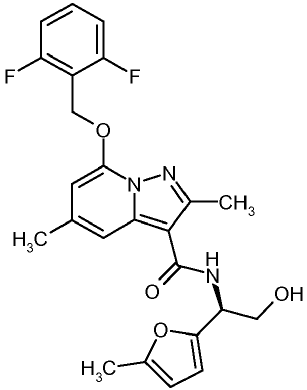 | |
| | (17% d. Th.; Reinheit 97%) | |
| **178** | 7-[(2,6-Difluorbenzyl)oxy]-N-[(1*R*)-2-hydroxy-1-(5-methyl-2-furyl)ethyl]-2,5-dimethylpyrazolo[1,5-a]pyridin-3-carboxamid | ¹H-NMR (400 MHz, DMSO-d₆): δ = 2.23 (s, 3H), 2.40 (s, 3H), 3.68 - 3.79 (m, 2H), 4.91 - 4.98 (m, 1H), 5.12 (q, 1H), 5.44 (s, 2H), 5.98 (d, 1H), 6.17 (d, 1H), 6.55 (s, 1H), 7.20 - 7.30 (m, 2H), 7.32 (s, 1H), 7.54 (d, 1H), 7.58 - 7.67 (m, 1H), [weiteres Signal unter Lösungsmittelpeak verborgen]. LC-MS (Methode 2): Rₜ = 0.98 min MS (ESpos): m/z = 456 (M+H)⁺ |
| | 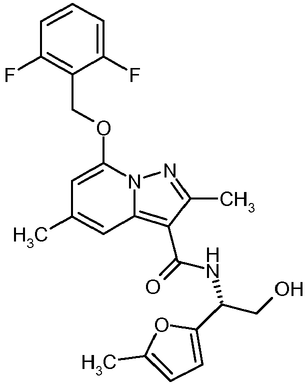 | |
| | (16% d. Th.) | |

### Beispiel 179

### N-[(1S)-1-{5-[(1S)-1-Amino-2-methylpropyl]-1,3,4-oxadiazol-2-yl}-2-hydroxyethyl]-7-[(2,6-difluorbenzyl)oxy]-2,5-dimethylpyrazolo[1,5-a]pyridin-3-carboxamid

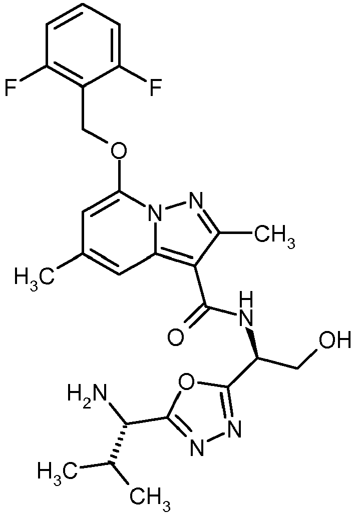

211 mg (0.27 mmol; Reinheit 94%) tert-Butyl-[(1*S*)-1-(5-{(1*S*)-1-[({7-[(2,6-difluorbenzyl)oxy]-2,5-dimethylpyrazolo[1,5-a]pyridin-3-yl}carbonyl)amino]-2-hydroxyethyl}-1,3,4-oxadiazol-2-yl)-2-methylpropyl]carbamat Trifluoracetat aus Beispiel 174A wurden in 1.5 ml Diethylether suspendiert und mit 1.36 ml (2.72 mmol) 2 M Chlorwasserstoff-Lösung in Diethylether versetzt. Das Reaktionsgemisch wurde 2 h bei RT gerührt. Das Gemisch wurde eingedampft, mit Acetonitril/Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert. Es wurden 45 mg der Zielverbindung (32% d. Th.) erhalten.
LC-MS (Methode 2): Rₜ = 0.68 min
MS (ESpos): m/z = 515 (M+H)⁺
¹H-NMR (400 MHz, DMSO-d₆): δ = 0.81 (d, 3H), 0.91 (d, 3H), 1.86 - 2.10 (m, 3H), 2.40 (s, 3H), 3.78 - 3.97 (m, 3H), 5.20 (t, 1H), 5.32 (q, 1H), 5.45 (s, 2H), 6.58 (s, 1H), 7.20 - 7.30 (m, 2H), 7.35 (s, 1H), 7.58 - 7.67 (m, 1H), 7.90 (d, 1H), [weiteres Signal unter Lösungsmittelpeak verborgen].

### B. Bewertung der pharmakologischen Wirksamkeit

Es werden die folgenden Abkürzungen verwendet:
- ATP: Adenosintriphosphat
- Brij35: Polyoxyethylen(23)laurylether

- BSA: Rinderserumalbumin
- DTT: Dithiothreitol

- TEA: Triethanolamin

Die pharmakologische Wirkung der erfindungsgemäßen Verbindungen kann in folgenden Assays gezeigt werden:

### B-1. Vermessung von sGC Enzymaktivität mittels PPi Nachweis

Lösliche Guanylylcyclase (sGC) setzt unter Stimulation GTP zu cGMP und Pyrophosphat (PPi) um. PPi wird mit Hilfe des in WO 2008/061626 beschriebenen Verfahrens nachgewiesen. Das im Test entstehende Signal nimmt mit fortschreitender Umsetzung zu und dient als Maß für die sGC-Enzymaktivität. Mit Hilfe einer PPi Referenzkurve kann das Enzym in bekannter Weise charakterisiert werden, z.B. hinsichtlich Umsatzrate, Stimulierbarkeit oder Michaelis Konstante.

### Durchführung des Tests

Zur Durchführung des Tests wurden 29 µL Enzymlösung (0-10 nM lösliche Guanylylcyclase (hergestellt nach Hönicka et al., Journal of Molecular Medicine 77(1999)14-23), in 50 mM TEA, 2 mM Magnesiumchlorid, 0.1% BSA (FraktionV), 0.005% Brij 35, pH 7.5) in die Mikroplatte vorgelegt und 1 µL der Stimulatorlösung (0-10 µM 3-Morpholinosydnonimine, SIN-1, Merck in DMSO) hinzugegeben. Es wurde 10 min bei RT inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT (Sigma) in 50 mM TEA, 2 mM Magnesiumchlorid, 0.1% BSA (Fraktion V), 0.005% Brij 35, pH 7,5) zugegeben. Die Enzymreaktion wurde durch Zugabe von 20 µl Substratlösung (1.25 mM Guanosin-5'-triphosphat (Sigma) in 50 mM TEA, 2 mM Magnesiumchlorid, 0.1% BSA (Fraktion V), 0.005% Brij 35, pH 7.5) gestartet und kontinuierlich luminometrisch vermessen.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative MEC-Werte (MEC = minimal effektive Konzentration) für die erfindungsgemäßen Verbindungen sind in der nachstehenden Tabelle wiedergegeben (zum Teil als Mittelwerte aus Einzelbestimmungen):

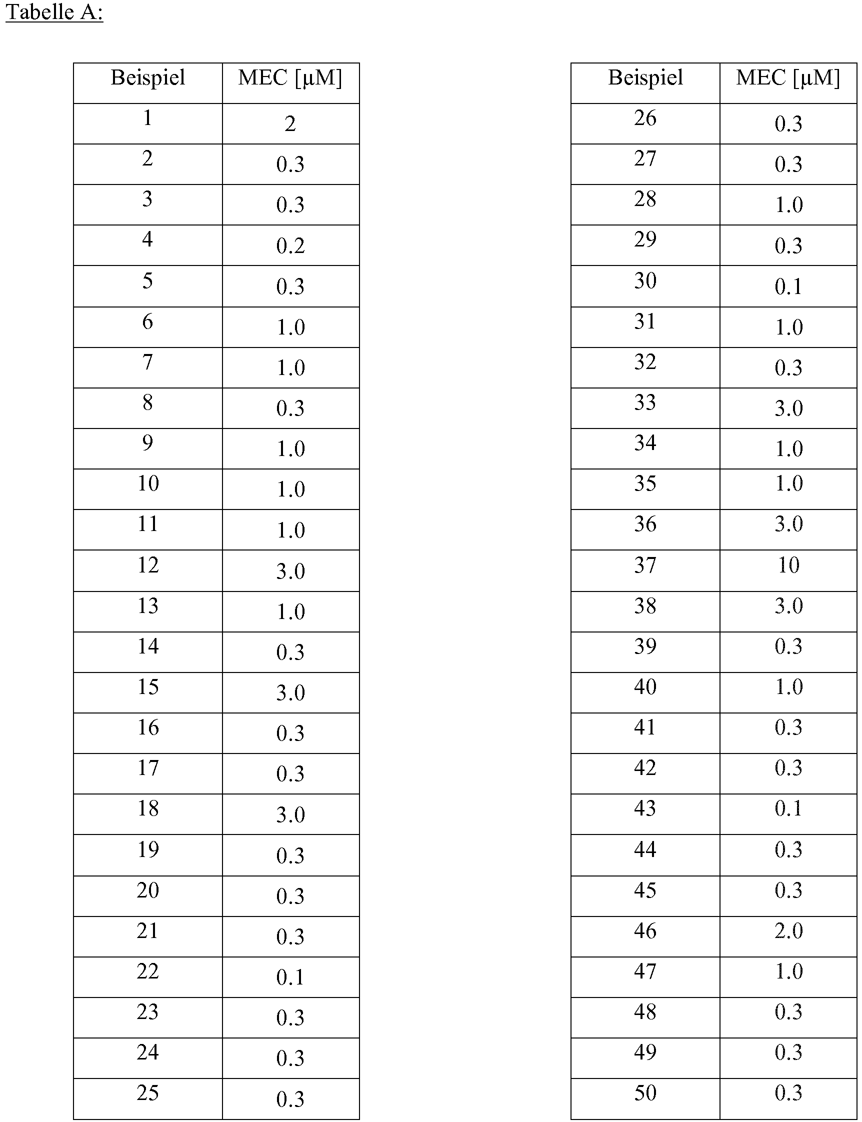

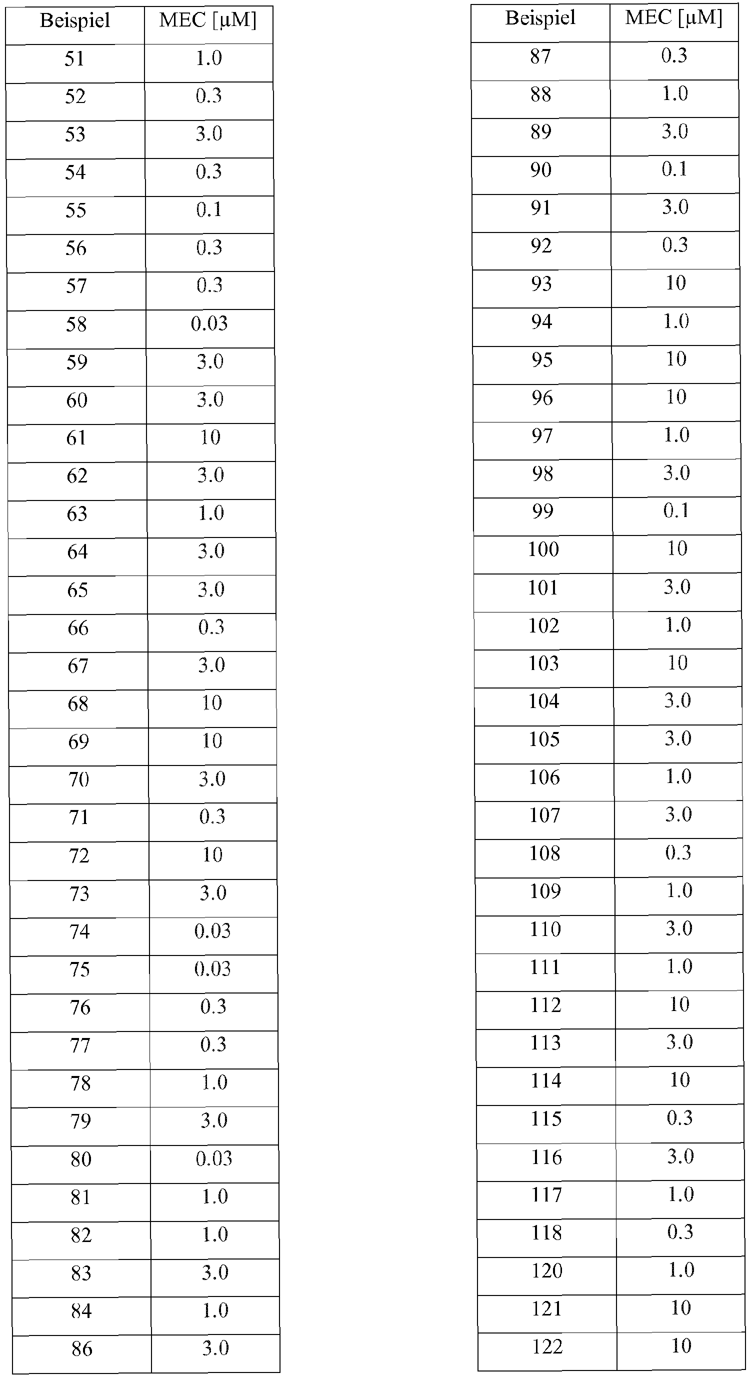

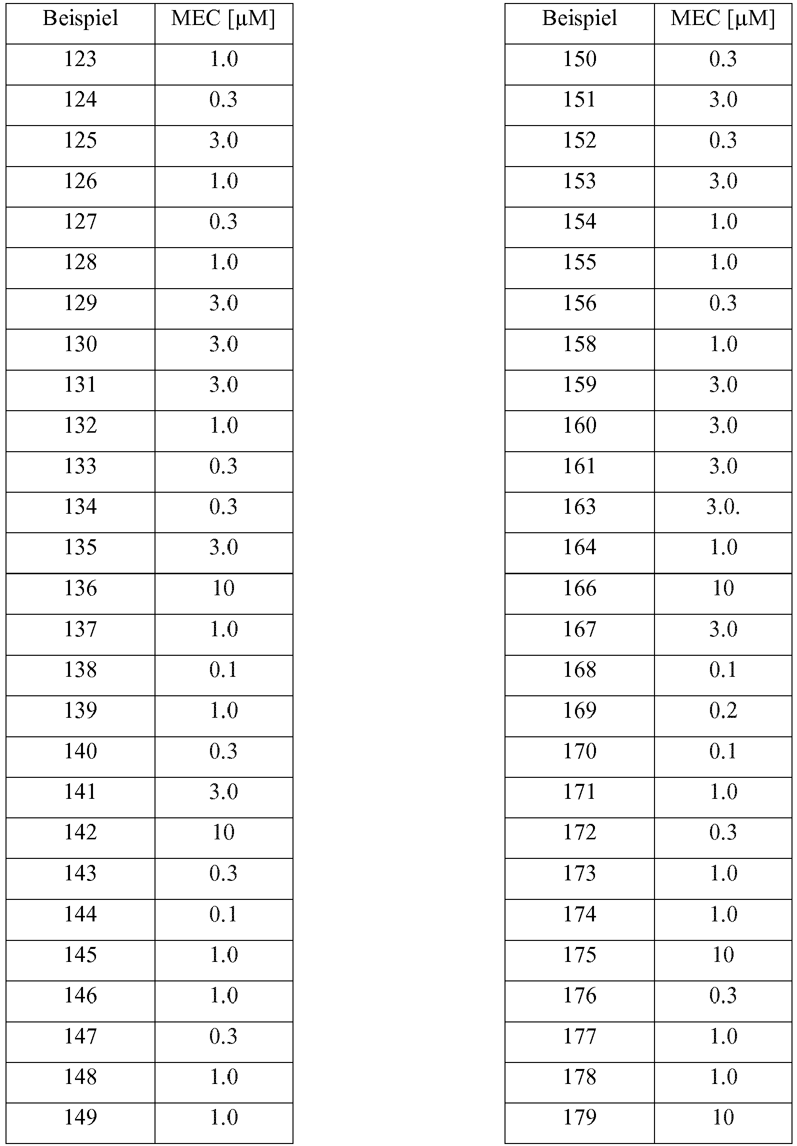

### B-3. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

### B-4. Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht (Stasch et al. Br. J. Pharmacol. 2002; 135: 344-355).

### B-5. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix) mit einem
Datenakquisitionscomputer verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5% iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.
Im Standardablauf werden über je 10 Sekunden Dauer gemessen
Systolischer Blutdruck (SBP)
Diastolischer Blutdruck (DBP)
Arterieller Mitteldruck (MAP)
Herzfrequenz (HR)
Aktivität (ACT)

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-6. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wistar-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

Tabelle B zeigt Daten repräsentativer Verbindungen der vorliegenden Erfindung nach intravenöser Gabe in Ratten:

**Tabelle B:**

| Beispiel | AUCₙₒᵣₘ [kg·h/L] | CL_{Blut} [L/h/kg] | t_{1/2} [h] | MRT [h] |
|---|---|---|---|---|
| 39 | 0.53 | 2.46 | 6.2 | 7.8 |
| 43 | 0.49 | 2.56 | 5.9 | 7.4 |
| 55 | 0.37 | 2.76 | 2.6 | 3.5 |
| 168 | 0.45 | 2.19 | 3.2 | 4.3 |
| 169 | 0.44 | 2.21 | 4.0 | 4.9 |
| 170 | 0.32 | 2.92 | 2.6 | 3.4 |

### B-7. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-8. Caco-2 Permeabilitäts-Test

Die Permeabilität einer Testsubstanz wurde mit Hilfe der Caco-2 Zelllinie, einem etablierten *in vitro* Modell für Permeabilitätsvorhersagen an der gastrointestinalen Barriere, bestimmt (Artursson, P. and Karlsson, J. (1991). Correlation between oral drug absorption in humans and apparent drug permeability coefficients in human intestinal epithelial (Caco-2) cells. Biochem. Biophys.175 (3), 880-885). Die Caco-2 Zellen (ACC No. 169, DSMZ, Deutsche Sammlung von Mikroorganismen und Zellkulturen, Braunschweig, Deutschland) wurden in 24-Well Platen mit Einsatz ausgesät und 14 bis 16 Tage kultiviert. Für die Permeabilitätsstudien wurde die Testsubstanz in DMSO gelöst und mit Transportpuffer (Hanks Buffered Salt Solution, Gibco/Invitrogen, mit 19.9 mM Glukose und 9.8 mM HEPES) auf die finale Testkonzentration verdünnt. Um die Permeabilität von apikal nach basolateral (PₐₚₚA-B) der Testsubstanz zu bestimmen, wurde die Lösung mit der Testsubstanz auf die apikale Seite des Caco-2 Zellmonolayers gegeben und Transportpuffer auf die basolaterale Seite. Um die Permeabilität von basolateral nach apikal (PₐₚₚB-A) der Testsubstanz zu bestimmen, wurde die Lösung mit der Testsubstanz auf die basolaterale Seite des Caco-2 Zellmonolayers gegeben und Transportpuffer auf die apikale Seite. Zu Beginn des Experiments wurden Proben aus dem jeweiligen Donor-Kompartiment genommen, um die Massenbilanz sicher zu stellen. Nach einer Inkubation von zwei Stunden bei 37° C wurden Proben aus beiden Kompartimenten genommen. Die Proben wurden mittels LC-MS/MS analysiert und die apparenten Permeabilitätskoeffizienten (Pₐₚₚ) berechnet. Die Permeabilität von Lucifer Yellow wurde für jeden Zellmonolayer bestimmt, um die Integrität der Zellschicht sicher zu stellen. Die Permeabilität von Atenolol (Marker für niedrige Permeabilität) und Sulfasalazin (Marker für aktive Exkretion) wurde in jedem Testlauf als Qualitätskontrolle mitbestimmt.

### B-9. hERG Kaliumstrom Assay.

Der sogenannte hERG (human ether-a-go-go related gene) Kaliumstrom trägt wesentlich zur Repolarisierung des humanen kardialen Aktionspotentials bei (Scheel et al., 2011). Eine Inhibition dieses Stroms durch Pharmaka kann in seltenen Fällen potentiell letale Herzrhythmusstörungen zur Folge haben, und wird deshalb frühzeitig während der Arzneimittelentwicklung untersucht.

Der hier verwendete funktionelle hERG Assay basiert auf einer recombinanten HEK293 ZellLinie, die das KCNH2(HERG)-Gen stabil exprimiert (Zhou et al., 1998). Diese Zellen werden mittels der "whole-cell voltage-clamp" Technik (Hamill et al., 1981) in einem automatisierten System (Patchliner™; Nanion, München, D) untersucht, welches die Membranspannung kontrolliert und den hERG Kalium-Strom bei Zimmertemperatur misst. Die PatchControlHT™ Software (Nanion) steuert Patchliner System, Datenerfassung und Datenanalyse. Die Spannungskontrolle erfolgt durch 2 EPC-10 quadro Verstärker unter Kontrolle der PatchMasterPro™ Software (beide: HEKA Elektronik, Lambrecht, D). NPC-16 Chips mit mittlerem Widerstand (∼2 MΩ; Nanion) dienen als planares Substrat für die Voltage-Clamp Experimente.

NPC-16 Chips werden mit intra- und extrazellulärer Lösung (vgl. Himmel, 2007) sowie mit Zellsuspension befüllt. Nach Bildung eines Giga-Ohm-Seals und Herstellen des Ganzzell-Modus (einschliesslich mehrerer automatisierter Qualitätskontrollschritte) wird die Zellmembran auf das Haltepotential -80 mV geklemmt. Das nachfolgende Spannungsklemm-Protokoll ändert die Kommandospannung auf +20 mV (Dauer 1000 ms), -120 mV (Dauer 500 ms), und zurück zum Haltepotential -80 mV; dies wird alle 12 s wiederholt. Nach einer initialen Stabilisierungsphase (ca 5-6 Minuten) wird Testsubstanzlösung in aufsteigenden Konzentrationen (z.B. 0.1, 1, und 10 µmol/L) zupipettiert (Exposition ca 5-6 Minuten pro Konzentration), gefolgt von mehreren Auswaschschritten.

Die Amplitude des einwärtsgerichteten "Tail"-Stroms, der durch eine Potentialänderung von +20 mV auf -120 mV erzeugt wird, dient zur Quantifizierung des hERG Kaliumstroms, und wird als Funktion der Zeit dargestellt (IgorPro™ Software). Die Stromamplitude am Ende verschiedener Zeitabschnitte (z.B. Stabilisierungsphase vor Testsubstanz, erste/zweite/dritte Konzentration Testsubstanz) dient zur Erstellung einer Konzentrations-Wirkungs-Kurve, aus der die halbmaximale Hemmkonzentration IC₅₀ der Testsubstanz errechnet wird.
Hamill OP, Marty A, Neher E, Sakmann B, Sigworth FJ. Improved patch-clamp techniques for high-resolution current recording from cells and cell-free membrane patches. Pfluegers Arch 1981; 391:85-100.
Himmel HM. Suitability of commonly used excipients for electrophysiological in-vitro safety pharmacology assessment of effects on hERG potassium current and on rabbit Purkinje fiber action potential. J Pharmacol Toxicol Methods 2007;56:145-158.
Scheel O, Himmel H, Rascher-Eggstein G, Knott T. Introduction of a modular automated voltage-clamp platform and its correlation with manual human ether-a-go-go related gene voltage-clamp data. Assay Drug Dev Technol 2011;9:600-607.
Zhou ZF, Gong Q, Ye B, Fan Z, Makielski JC, Robertson GA, January CT. Properties of hERG channels stably expressed in HEK293 cells studied at physiological temperature. Biophys J 1998;74:230-241.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Wässrige NatriumchloridLösung, Glucoselösung 5% und/oder PEG 400-Lösung 30%) gelöst. Die erhaltene Lösung wird steril filtriert und in sterile und pyrogenfreie Injektionsbehältnisse abgefüllt.

## Patentansprüche

1. Verbindung der Formel (I) 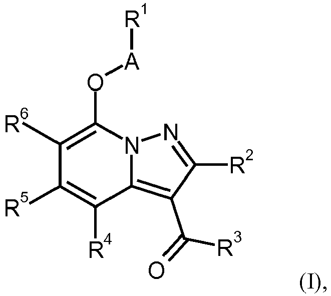 in welcher
A für CH₂, CD₂ oder CH(CH₃) steht,
R¹ für (C₄-C₆)-Alkyl, (C₃-C₇)-Cycloalkyl, Pyridyl oder Phenyl steht,
wobei (C₄-C₆)-Alkyl bis zu sechsfach mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 oder 2 Substituenten unabhängig voneinander ausgewählt aus der Gruppe Halogen, Cyano und (C₁-C₄)-Alkyl substituiert ist,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₂-C₃)-Alkinyl, (C₁-C₄)-Alkoxy, (C₃-C₅)-Cycloalkyl, Difluormethoxy und Trifluormethoxy substituiert sein kann oder an zwei benachbarten Kohlenstoffatomen des Phenyls mit einer Difluormethylendioxy-Brücke substituiert sein kann,
R² für Wasserstoff, (C₁-C₄)-Alkyl, (C₁-C₄)-Alkoxymethyl, Cyclopropyl, Cyclobutyl, Monofluormethyl, Difluormethyl oder Trifluormethyl steht,
R³ für eine Gruppe der Formel 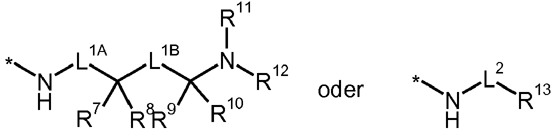 oder 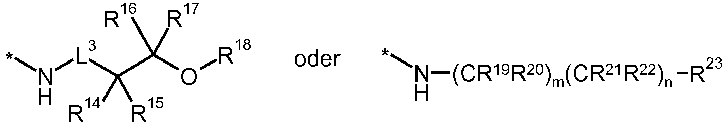 steht, wobei
* für die Anknüpfstelle an die Carbonylgruppe steht,
L^{1A} für eine Bindung, Methandiyl, 1,2-Ethandiyl oder 1,3-Propandiyl steht,
worin Methandiyl, 1,2-Ethandiyl oder 1,3-Propandiyl mit 1 oder 2 Substituenten unabhängig voneinander ausgewählt aus der Gruppe Fluor, Trifluormethyl, (C₁-C₄)-Alkyl, (C₃-C₅)-Cycloalkyl, Hydroxy und (C₁-C₄)-Alkoxy substituiert sein können,
L^{1B} für eine Bindung, Methandiyl oder 1,2-Ethandiyl steht,
L² für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₅)-Cycloalkyl, Hydroxy und (C₁-C₄)-Alkoxy substituiert sein können,
L³ für eine Bindung, Methandiyl oder 1,2-Ethandiyl steht,
worin Methandiyl oder 1,2-Ethandiyl mit 1 oder 2 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein können,
R⁷ für Wasserstoff, (C₁-C₆)-Alkyl, (C₃-C₇)-Cycloalkyl, (C₂-C₆)-Alkenyl, (C₂-C₆)-Alkinyl, Cyano, 5- bis 10-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl, sowie bis zu sechsfach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Halogen und (C₁-C₄)-Alkoxy substituiert sein können,
worin (C₃-C₇)-Cycloalkyl mit 1 oder 2 Substituenten Fluor, Trifluormethyl, (C₁-C₄)-Alkyl oder (C₁-C₄)-Alkoxy substituiert sein kann,
worin Phenyl und 5- bis 10-gliedriges Heteroaryl mit 1 bis 4 Substituenten unabhängig voneinander ausgewählt aus der Gruppe Halogen, Cyano, Nitro, Trifluormethyl, Difluormethyl, (C₁-C₄)-Alkyl, (C₂-C₄)-Alkenyl, (C₁-C₄)-Alkoxy, Difluormethoxy, Trifluormethoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
worin (C₁-C₄)-Alkyl mit Amino oder Hydroxy substitiuiert sein kann,
und
worin Phenyl an zwei benachbarten Kohlenstoffatomen des Phenyls mit einer Methylendioxy-Brücke oder Ethylendioxy-Brücke substituiert sein kann,
oder
für Fluor stehen kann, wenn L^{1A} nicht für eine Bindung steht,
R⁸ für Wasserstoff oder (C₁-C₆)-Alkyl steht,
oder
für Fluor stehen kann, wenn L^{1A} nicht für eine Bindung steht,
oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, (C₁-C₆)-Alkyl, (C₃-C₇)-Cycloalkyl, (C₂-C₆)-Alkenyl, (C₂-C₆)-Alkinyl, Cyano, 5- bis 10-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl sowie bis zu sechsfach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Halogen und (C₁-C₄)-Alkoxy substituiert sein können,
worin (C₃-C₇)-Cycloalkyl mit 1 oder 2 Substituenten Fluor, Trifluormethyl, (C₁-C₄)-Alkyl oder (C₁-C₄)-Alkoxy substituiert sein kann,
worin Phenyl und 5- bis 10-gliedriges Heteroaryl mit 1 bis 4 Substituenten unabhängig voneinander ausgewählt aus der Gruppe Halogen, Cyano, Nitro, Trifluormethyl, (C₁-C₄)-Alkyl, (C₂-C₄)-Alkenyl, (C₁-C₄)-Alkoxy, Difluormethoxy, Trifluormethoxy, (C₁-C₄)-Alkoxycarbonyl und (C₁-C₄)-Alkylsulfonyl substituiert sein können,
worin (C₁-C₄)-Alkoxy mit Hydroxy substituiert sein kann,
und
worin Phenyl an zwei benachbarten Kohlenstoffatomen des Phenyls mit einer Methylendioxy-Brücke oder Ethylendioxy-Brücke substituiert sein kann,
R¹⁰ für Wasserstoff oder (C₁-C₆)-Alkyl steht,
oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
R⁷ und R⁹ zusammen mit den Kohlenstoffatomen, an die sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus mit 1 oder 2 Substituenten (C₁-C₄)-Alkyl substituiert sein können,
und
worin der 3- bis 7-gliedrige Carbocyclus und der 4- bis 7-gliedrige Heterocyclus mit einem Phenyl-Ring oder einem Pyridyl-Ring anneliert sein können, die ihrerseits mit 1 oder 2 Substituenten ausgewählt aus Halogen, (C₁-C₄)-Alkyl und Trifluormethyl substituiert sein können,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
mit der Maßgabe, dass die Reste R⁷ und R⁹ nicht beide gleichzeitig für Phenyl oder 5- oder 6-gliedriges Heteroaryl stehen,
R¹¹ für Wasserstoff oder (C₁-C₄)-Alkyl steht,
worin (C₁-C₄)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
R¹² für Wasserstoff, (C₁-C₆)-Alkyl, (C₁-C₄)-Alkylcarbonyl, (C₁-C₄)-Alkoxycarbonyl, (C₃-C₇)-Cycloalkyl, Phenyl oder Benzyl steht,
worin (C₁-C₆)-Alkyl mit 1 bis 3 Substituenten unabhängig voneinander ausgewählt aus der Gruppe Fluor, Trifluormethyl, Hydroxy, (C₁-C₄)-Alkoxy und Phenoxy substituiert sein kann,
und
worin Phenyl und Benzyl mit 1 bis 3 Substituenten unabhängig voneinander ausgewählt aus der Gruppe Halogen und Trifluormethyl substituiert sein können,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit (C₁-C₄)-Alkyl substituiert sein kann,
R¹³ für 5- bis 10-gliedriges, über ein Ring-Kohlenstoffatom gebundenes Aza-Heterocyclyl oder über ein Ring-Stickstoffatom gebundenes 5- oder 6-gliedriges Heterocyclyl steht,
worin 5- bis 10-gliedriges, über ein Ring-Kohlenstoffatom gebundenes Aza-Heterocyclyl mit 1 bis 2 Substituenten Trifluormethyl, (C₃-C₇)-Cycloalkyl, Oxo und Benzyl sowie bis zu vierfach mit (C₁-C₄)-Alkyl und bis zu zweifach mit Fluor substituiert sein kann,
worin 5- bis 10-gliedriges Aza-Heterocyclyl mit einem Phenyl-Ring oder einem Pyridyl-Ring anneliert sein kann, die ihrerseits mit 1 oder 2 Substituenten ausgewählt aus Halogen, (C₁-C₄)-Alkyl, (C₁-C₄)-Alkoxy und Trifluormethyl substituiert sein können,
worin über ein Ring-Stickstoffatom gebundenes 5- oder 6-gliedriges Heterocyclyl mit (C₁-C₄)-Alkyl oder (C₁-C₄)-Cycloalkyl substituiert sein kann,
worin (C₁-C₄)-Alkyl mit Difluormethyl oder Trifluormethyl substituiert sein kann,
oder
für Amino stehen kann, wenn L² für eine Bindung steht,
worin Amino mit (C₁-C₁₀)-Alkyl, (C₁-C₄)-Alkylcarbonyl, (C₃-C₆)-Carbocyclyl, 4- bis 7-gliedriges Heterocyclyl, Phenyl oder 5- oder 6-gliedriges Heteroaryl substituiert sein kann,
worin (C₁-C₄)-Alkylcarbonyl mit Mono-alkylamino, Di-alkylamino oder über ein Ring-Stickstoffatom gebundenes 5- oder 6-gliedriges Heterocyclyl substituiert sein kann,
worin über ein Ring-Stickstoffatom gebundenes 5- oder 6-gliedriges Heterocyclyl mit Oxo substituiert sein kann,
worin (C₃-C₆)-Carbocyclyl und 4- bis 7-gliedriges Heterocyclyl mit Hydroxy substituiert sein können,
und
worin Phenyl und 5- oder 6-gliedriges Heteroaryl mit 1 bis 3 Substituenten unabhängig voneinander ausgewählt aus der Gruppe Halogen, (C₁-C₄)-Alkyl und Trifluormethyl substituiert sein können,
R¹⁴ für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, -(C=O)NR²⁴R²⁵, 5- oder 6-gliedriges Heteroaryl oder Phenyl steht,
worin (C₁-C₆)-Alkyl mit 1 oder 2 Substituenten unabhängig voneinander ausgewählt aus der Gruppe Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy, sowie bis zu sechsfach mit Fluor substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
worin R²⁴ für Wasserstoff, (C₁-C₄)-Alkyl, (C₃-C₇)-Cycloalkyl, Aryl oder Naphthyl steht,
worin R²⁵ für Wasserstoff oder Methyl steht,
und
worin Phenyl und 5- oder 6-gliedriges Heteroaryl mit 1 bis 3 Substituenten unabhängig voneinander ausgewählt aus der Gruppe Halogen, Cyano, Trifluormethyl, Difluormethyl, (C₁-C₄)-Alkyl, (C₁-C₄)-Alkoxy und (C₁-C₄)-Alkylsulfonyl substituiert sein können,
worin (C₁-C₄)-Alkyl mit Amino oder Hydroxy substitiuiert sein kann,
R¹⁵ für Wasserstoff oder (C₁-C₆)-Alkyl steht,
worin (C₁-C₄)-Alkyl mit Hydroxy substituiert sein kann,
oder
R¹⁴ und R¹⁵ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R¹⁶ für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 oder 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy, sowie bis zu sechsfach mit Fluor substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
R¹⁷ für Wasserstoff oder (C₁-C₆)-Alkyl steht,
worin (C₁-C₄)-Alkyl mit Hydroxy substituiert sein kann,
oder
R¹⁶ und R¹⁷ zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass die Reste R¹⁴ und R¹⁶ nicht beide gleichzeitig für Phenyl oder 5-oder 6-gliedriges Heteroaryl stehen,
oder
R¹⁴ und R¹⁶ zusammen mit den Kohlenstoffatomen, an die sie gebunden sind, einen 3- bis 7-gliedrigen Carbocyclus oder einen 4- bis 7-gliedrigen Heterocyclus bilden,
mit der Maßgabe, dass gleichzeitig nicht mehr als eines der Reste-Paare R¹⁴ und R¹⁵, R¹⁶ und R¹⁷ bzw. R¹⁴ und R¹⁶ einen Carbo- oder Heterocyclus bildet,
R¹⁸ für Wasserstoff oder (C₁-C₄)-Alkyl steht,
worin (C₁-C₄)-Alkyl mit 1 bis 5 Substituenten Fluor substituiert sein kann,
m für 0, 1 oder 2 steht,
n für 0 oder 1 steht,
R¹⁹ für Wasserstoff, Cyano oder (C₁-C₆)-Alkyl steht,
worin (C₁-C₆)-Alkyl mit 1 bis 5 Substituenten Fluor substituiert sein kann,
R²⁰ für Wasserstoff oder (C₁-C₄)-Alkyl steht,
worin (C₁-C₄)-Alkyl mit 1 bis 5 Substituenten Fluor substituiert sein kann,
R²¹ für Wasserstoff oder (C₁-C₆)-Alkyl steht,
worin (C₁-C₆)-Alkyl mit 1 bis 5 Substituenten Fluor substituiert sein kann,
R²² für Wasserstoff oder (C₁-C₄)-Alkyl steht,
worin (C₁-C₄)-Alkyl mit 1 bis 5 Substituenten Fluor substituiert sein kann,
oder
R¹⁹ und R²⁰ zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
R²¹ und R²² zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
R¹⁹ und R²¹ zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass gleichzeitig nicht mehr als eines der Reste-Paare R¹⁹ und R²⁰, R²¹ und R²² bzw. R¹⁹ und R²¹ einen Carbo- oder Heterocyclus bildet,
R²³ für (C₁-C₆)-Alkyl, (C₁-C₆)-Alkoxy, (C₁-C₄)-Alkoxycarbonyl, Hydroxycarbonyl, Aminocarbonyl, Aminosulfonyl, 5- bis 10-gliedriges, über ein Ring-Kohlenstoffatom gebundenes Heterocyclyl, 5- bis 10-gliedriges Carbocyclyl, Phenyl oder 5- bis 10-gliedriges Heteroaryl steht,
worin (C₁-C₆)-Alkyl mit Cyano, sowie bis zu sechsfach mit Fluor substituiert sein kann,
worin (C₁-C₆)-Alkoxy mit Hydroxy, Amino, Mono-alkylamino, Di-alkylamino, Cyclopropyl, Phenyl oder (C₂-C₄)-Alkenyl substituiert sein kann,
worin Aminocarbonyl mit (C₁-C₆)-Alkyl oder (C₃-C₆)-Cycloalkyl substituiert sein kann,
worin Aminosulfonyl mit (C₁-C₆)-Alkyl oder (C₃-C₆)-Cycloalkyl substituiert sein kann,
worin Phenyl mit 1 bis 3 Substituenten unabhängig voneinander ausgewählt aus der Gruppe Halogen, Cyano, Trifluormethyl, Difluormethyl, (C₁-C₆)-Alkyl, (C₁-C₄)-Alkylcarbonyl, (C₁-C₄)-Alkoxy-carbonyl, Hydroxycarbonyl, -(C=O)NR²⁶R²⁷, (C₁-C₄)-Alkylsulfonyl, (C₃-C₆)-Cycloalkylsulfonyl, (C₁-C₄)-Alkylthio, (C₁-C₄)-Alkoxy, Trifluormethoxy, Difluormethoxy, Phenoxy, Hydroxy, 5- bis 10-gliedriges Heteroaryl, 4- bis 7-gliedriges Heterocyclyl und (C₃-C₇)-Cycloalkyl substituiert sein kann,
worin (C₁-C₆)-Alkyl mit 1 oder 2 Substituenten unabhängig voneinander ausgewählt aus der Gruppe Fluor, Trifluormethoxy, (C₁-C₄)-Alkylcarbonyl, -(C=O)NR²⁶R²⁷, (C₁-C₄)-Alkoxy, (C₃-C₆)-Cycloalkyl, Morpholinyl, Piperidinyl, Pyrrolidinyl, Piperazinyl, Phenyl, Hydroxy und Amino substituiert sein kann,
worin Phenyl mit 1 bis 3 Substituenten Halogen substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl, (C₁-C₄)-Alkylsulfonyl und Methoxy-(C₁-C₄)-alkyl substituiert sein kann,
worin (C₃-C₆)-Cycloalkyl mit Amino oder Hydroxy substituiert sein kann, und worin
R²⁶ und R²⁷ jeweils unabhängig voneinander für Wasserstoff, (C₁-C₄)-Alkyl oder (C₃-C₇)-Cycloalkyl stehen,
worin 5- bis 10-gliedriges Heteroaryl mit 1 bis 3 Substituenten unabhängig voneinander ausgewählt aus der Gruppe Fluor, Chlor, Cyano, (C₁-C₆)-Alkyl, Trifluormethyl, (C₁-C₄)-Alkoxy, Amino, (C₁-C₄)-Alkoxycarbonyl, Hydroxycarbonyl, -(C=O)NR²⁵R²⁶, Phenyl, Pyridyl, Pyrimidyl, 1,3-Thiazol-5-yl und (C₃-C₇)-Cycloalkyl substituiert sein kann,
worin (C₁-C₆)-Alkyl mit 1 bis 3 Substituenten unabhängig voneinander ausgewählt aus der Gruppe Halogen, Cyano, Hydroxy, Amino, Trifluormethyl, Difluormethyl, (C₁-C₄)-Alkylsulfonyl, (C₁-C₄)-Alkylcarbonyl, (C₁-C₄)-Alkoxycarbonyl, Hydroxycarbonyl, (C₁-C₄)-Alkylthio, (C₁-C₄)-Alkoxy, Trifluormethoxy, Difluormethoxy, Phenoxy, Phenyl, Pyridyl, Pyrimidyl, 5-gliedriges Heteroaryl, Tetrahydrothiophenyl-1,1-dioxid, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
worin 5-gliedriges Heteroaryl mit 1 bis 3 Substituenten unabhängig voneinander ausgewählt aus der Gruppe Halogen, (C₁-C₄)-Alkyl und (C₁-C₄)-Alkoxy substituiert sein kann,
worin Piperidinyl mit 1 bis 4 Substituenten Fluor substituiert sein kann,
worin Phenyl mit 1 bis 3 Substituenten unabhängig voneinander ausgewählt aus der Gruppe Halogen, (C₁-C₄)-Alkyl und (C₁-C₄)-Alkoxy substituiert sein kann,
worin Azetidin mit Hydroxy substituiert sein kann,
worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
und worin
R²⁶ und R²⁷ jeweils unabhängig voneinander für Wasserstoff, (C₁-C₄)-Alkyl oder (C₃-C₇)-Cycloalkyl stehen,
worin 5- bis 10-gliedriges, über ein Ring-Kohlenstoffatom gebundenes, Heterocyclyl mit 1 bis 3 Substituenten unabhängig voneinander ausgewählt aus der Gruppe Oxo, Fluor,Trifluormethyl, Hydroxy und (C₁-C₄)-Alkyl substituiert sein kann,
worin 5- bis 10-gliedriges über ein Ring-Kohlenstoffatom gebundenes, Heterocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl und Trifluormethyl substituiert sein können,
und
worin 5- bis 10-gliedriges Carbocyclyl mit 1 bis 3 Substituenten unabhängig voneinander ausgewählt aus der Gruppe Trifluormethyl, Fluor, Cyano, Hydroxy, Hydroxycarbonyl, (C₁-C₄)-Alkoxycarbonyl, Amino und (C₁-C₄)-Alkyl substituiert sein kann,
worin (C₁-C₄)-Alkyl mit Hydroxy oder Hydroxycarbonyl substituiert sein kann,
worin 5- bis 10-gliedriges Carbocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
R⁴ für Wasserstoff steht,
R⁵ für Wasserstoff, Halogen, Cyano, Difluormethyl, Trifluormethyl, (C₁-C₄)-Alkyl, (C₃-C₇)-Cycloalkyl, (C₂-C₄)-Alkinyl, (C₁-C₄)-Alkylamino, Difluormethoxy, Trifluormethoxy, (C₁-C₄)-Alkoxy, Amino, 4- bis 7-gliedriges Heterocyclyl oder 5-oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

2. Verbindung der Formel (I) nach Anspruch 1, in welcher
A für CH₂ oder CD₂ steht,
R¹ für (C₃-C₆)-Cycloalkyl, Pyridyl oder Phenyl steht,
wobei (C₃-C₆)-Cycloalkyl mit 1 bis 2 Substituenten unabhängig voneinander ausgewählt aus der Gruppe Fluor, Trifluormethyl, Methyl und Ethyl substituiert sein kann,
wobei Pyridyl mit 1 oder 2 Substituenten Fluor substituiert ist,
und
wobei Phenyl mit 1 bis 4 Substituenten unabhängig voneinander ausgewählt aus der Gruppe Halogen, Cyano, Difluormethyl, Trifluormethyl, (C₁-C₄)-Alkyl, (C₁-C₄)-Alkoxy und (C₃-C₅)-Cycloalkyl substituiert sein kann,
R² für Wasserstoff, (C₁-C₄)-Alkyl, Cyclopropyl, Cyclobutyl, Difluormethyl oder Trifluormethyl steht,
R³ für eine Gruppe der Formel 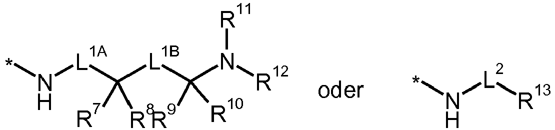 oder 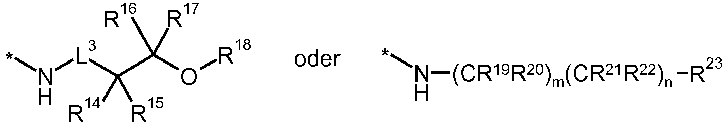 steht, wobei
* für die Anknüpfstelle an die Carbonylgruppe steht,
L^{1A} für eine Bindung, Methandiyl, 1,2-Ethandiyl oder 1,3-Propandiyl steht,
L^{1B} für eine Bindung, Methandiyl oder 1,2-Ethandiyl steht,
L² für eine Bindung, Methandiyl oder 1,2-Ethandiyl steht,
L³ für eine Bindung, Methandiyl oder 1,2-Ethandiyl steht,
R⁷ für Wasserstoff, (C₁-C₆)-Alkyl, (C₃-C₇)-Cycloalkyl, 5- oder 6-gliedriges Heteroaryl oder Phenyl steht,
worin (C₁-C₆)-Alkyl bis zu fünffach mit Fluor substituiert sein kann,
worin Phenyl und 5- oder 6-gliedriges Heteroaryl mit 1 bis 3 Substituenten unabhängig voneinander ausgewählt aus der Gruppe Fluor, Chlor, Brom, Cyano, Nitro, Trifluormethyl, Difluormethyl, Methyl, Ethyl, Methoxy, Ethoxy, Difluormethoxy, Trifluormethoxy, Methoxycarbonyl und Ethoxycarbonyl substituiert sein können,
und
worin Phenyl an zwei benachbarten Kohlenstoffatomen des Phenyls mit einer Methylendioxy-Brücke oder Ethylendioxy-Brücke substituiert sein kann,
oder
für Fluor stehen kann, wenn L^{1A} nicht für eine Bindung steht,
R⁸ für Wasserstoff oder (C₁-C₄)-Alkyl steht,
oder
für Fluor stehen kann, wenn L^{1A} nicht für eine Bindung steht,
oder
R⁷ und R⁸ zusammen mit dem Kohlenstoffatom, an das sie gebunden sind, einen 3- bis 7-gliedrigen Carbocyclus bilden,
R⁹ für Wasserstoff, (C₁-C₆)-Alkyl, (C₃-C₅)-Cycloalkyl, Cyano, 5- bis 10-gliedriges Heteroaryl oder Phenyl steht,
worin (C₁-C₆)-Alkyl mit (C₁-C₄)-Alkoxy, (C₁-C₄)-Alkoxycarbonyl, (C₁-C₄)-Alkylsulfonyl, (C₁-C₄)-Alkylthio, Benzyloxy, Phenoxy, 5- oder 6-gliedriges Heteroaryl und Phenyl, sowie bis zu fünffach mit Fluor substituiert sein kann,
worin Benzyloxy, Phenoxy, 5- oder 6-gliedriges Heteroaryl und Phenyl mit 1 bis 3 Substituenten unabhängig voneinander ausgewählt aus der Gruppe Fluor, Chlor, Brom, Methoxy und Ethoxy substituiert sein können,
worin (C₃-C₅)-Cycloalkyl mit 1 oder 2 Substituenten Fluor, Trifluormethyl, Methyl, Ethyl, Methoxy oder Ethoxy substituiert sein kann,
worin Phenyl und 5- bis 10-gliedriges Heteroaryl mit 1 bis 3 Substituenten unabhängig voneinander ausgewählt aus der Gruppe Fluor, Chlor, Brom, Cyano, Nitro, Trifluormethyl, Methyl, Ethyl, Ethenyl, Propenyl, Methoxy, Ethoxy, Difluormethoxy, Trifluormethoxy, Methoxycarbonyl und Ethoxycarbonyl substituiert sein können,
worin Methoxy und Ethoxy mit Hydroxy substituiert sein können,
und
worin Phenyl an zwei benachbarten Kohlenstoffatomen des Phenyls mit einer Methylendioxy-Brücke oder Ethylendioxy-Brücke substituiert sein kann,
R¹⁰ für Wasserstoff oder (C₁-C₄)-Alkyl steht,
oder
R⁹ und R¹⁰ zusammen mit dem Kohlenstoffatom, an das sie gebunden sind, einen 3- bis 7-gliedrigen Carbocyclus oder einen 4- bis 7-gliedrigen Heterocyclus bilden,
oder
R⁷ und R⁹ zusammen mit den Kohlenstoffatomen, an die sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus mit 1 oder 2 Substituenten (C₁-C₄)-Alkyl substituiert sein können,
und
worin der 3- bis 7-gliedrige Carbocyclus und der 4- bis 7-gliedrige Heterocyclus mit einem Phenyl-Ring oder einem Pyridyl-Ring anneliert sein können, die ihrerseits mit 1 oder 2 Substituenten ausgewählt aus Fluor, Chlor, Brom, Methyl, Ethyl und Trifluormethyl substituiert sein können,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
mit der Maßgabe, dass die Reste R⁷ und R⁹ nicht beide gleichzeitig für Phenyl oder 5- oder 6-gliedriges Heteroaryl stehen,
R¹¹ für Wasserstoff oder (C₁-C₄)-Alkyl steht,
worin (C₁-C₄)-Alkyl bis zu fünffach mit Fluor substituiert sein kann,
R¹² für Wasserstoff, (C₁-C₆)-Alkyl, (C₃-C₅)-Cycloalkyl oder (C₁-C₄)-Alkylcarbonyl steht,
worin (C₁-C₆)-Alkyl bis zu fünffach mit Fluor substituiert sein kann,
oder
R¹¹ und R¹² zusammen mit dem Sticktoffatom, an das sie gebunden sind, einen 4- bis 7-gliedrigen Aza-Heterocyclus bilden,
worin der 4- bis 7-gliedrige Aza-Heterocyclus mit Methyl oder Ethyl substituiert sein kann,
R¹³ für 5- bis 10-gliedriges, über ein Ring-Kohlenstoffatom gebundenes Aza-Heterocyclyl oder über ein Ring-Stickstoffatom gebundenes 5- oder 6-gliedriges Heterocyclyl steht,
worin 5- bis 10-gliedriges, über ein Ring-Kohlenstoffatom gebundenes Aza-Heterocyclyl mit 1 oder 2 Substituenten Trifluormethyl, (C₃-C₇)-Cycloalkyl, Oxo und Benzyl sowie bis zu vierfach mit (C₁-C₄)-Alkyl und bis zu zweifach mit Fluor substituiert sein kann,
worin 5- bis 10-gliedriges Aza-Heterocyclyl mit einem Phenyl-Ring oder einem Pyridyl-Ring anneliert sein kann, die ihrerseits mit 1 oder 2 Substituenten ausgewählt aus Fluor, Chlor, Methyl, (C₁-C₄)-Alkoxy und Trifluormethyl substituiert sein können,
worin über ein Ring-Stickstoffatom gebundenes 5- oder 6-gliedriges Heterocyclyl mit (C₁-C₄)-Alkyl oder (C₁-C₄)-Cycloalkyl substituiert sein kann,
oder
für Amino stehen kann, wenn L² für eine Bindung steht,
worin Amino mit (C₁-C₄)-Alkyl, (C₁-C₄)-Alkylcarbonyl, (C₃-C₆)-Carbocyclyl, 4- bis 7-gliedriges Heterocyclyl, Phenyl oder 5- oder 6-gliedriges Heteroaryl substituiert sein kann,
worin (C₁-C₄)-Alkylcarbonyl mit Mono-alkylamino, Di-alkylamino oder über ein Ring-Stickstoffatom gebundenes 5- oder 6-gliedriges Heterocyclyl substituiert sein kann, worin über ein 5-Ring-Stickstoffatom gebundenes oder 6-gliedriges Heterocyclyl mit Oxo substituiert sein kann,
worin (C₃-C₆)-Carbocyclyl und 4- bis 7-gliedriges Heterocyclyl mit Hydroxy substituiert sein können,
und
worin Phenyl und 5- oder 6-gliedriges Heteroaryl mit 1 bis 3 Substituenten unabhängig voneinander ausgewählt aus der Gruppe Fluor, Chlor, Methyl und Trifluormethyl substituiert sein können,
R¹⁴ für Wasserstoff, (C₁-C₆)-Alkyl, (C₃-C₇)-Cycloalkyl, -(C=O)NR²⁴R²⁵, 5-oder 6-gliedriges Heteroaryl oder Phenyl steht,
worin (C₁-C₆)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
worin R²⁴ für Wasserstoff, (C₁-C₄)-Alkyl, Aryl oder Naphthyl steht,
worin R²⁵ für Wasserstoff steht,
und
worin Phenyl und 5- oder 6-gliedriges Heteroaryl mit 1 bis 3 Substituenten unabhängig voneinander ausgewählt aus der Gruppe Fluor, Chlor, Brom, Trifluormethyl, Difluormethyl, Methyl, Ethyl und 1-Amino-2-methylpropyl substituiert sein können,
R¹⁵ für Wasserstoff oder (C₁-C₆)-Alkyl steht,
worin (C₁-C₄)-Alkyl mit Hydroxy substituiert sein kann,
oder
R¹⁴ und R¹⁵ zusammen mit dem Kohlenstoffatom, an das sie gebunden sind, einen 3- bis 7-gliedrigen Carbocyclus bilden,
R¹⁶ für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Hydroxy und (C₁-C₄)-Alkoxy, sowie bis zu sechsfach mit Fluor substituiert sein kann,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
R¹⁷ für Wasserstoff oder (C₁-C₆)-Alkyl steht,
oder
R¹⁶ und R¹⁷ zusammen mit dem Kohlenstoffatom, an das sie gebunden sind, einen 3- bis 6-gliedrigen Carbocyclus bilden,
worin der 3- bis 6-gliedrige Carbocyclus mit 1 oder 2 Substituenten unabhängig voneinander ausgewählt aus der Gruppe Fluor, Methyl und Ethyl substituiert sein kann,
oder
R¹⁴ und R¹⁶ zusammen mit den Kohlenstoffatomen, an die sie gebunden sind, einen 3- bis 7-gliedrigen Carbocyclus oder einen 4- bis 7-gliedrigen Heterocyclus bilden,
mit der Maßgabe, dass gleichzeitig nicht mehr als eines der Reste-Paare R¹⁴ und R¹⁵, R¹⁶ und R¹⁷ bzw. R¹⁴ und R¹⁶ einen Carbo- oder Heterocyclus bildet,
R¹⁸ für Wasserstoff oder (C₁-C₄)-Alkyl steht,
worin (C₁-C₄)-Alkyl mit 1 bis 5 Substituenten Fluor substituiert sein kann,
m für 0 oder 1 steht,
n für 0 oder 1 steht,
R¹⁹ für Wasserstoff, Cyano oder (C₁-C₄)-Alkyl steht,
worin (C₁-C₄)-Alkyl mit 1 bis 5 Substituenten Fluor substituiert sein kann,
R²⁰ für Wasserstoff oder (C₁-C₄)-Alkyl steht,
worin (C₁-C₄)-Alkyl mit 1 bis 5 Substituenten Fluor substituiert sein kann,
R²¹ für Wasserstoff oder (C₁-C₄)-Alkyl steht,
worin (C₁-C₄)-Alkyl mit 1 bis 5 Substituenten Fluor substituiert sein kann,
R²² für Wasserstoff oder (C₁-C₄)-Alkyl steht,
worin (C₁-C₄)-Alkyl mit 1 bis 5 Substituenten Fluor substituiert sein kann,
oder
R¹⁹ und R²⁰ zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
R²¹ und R²² zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
oder
R¹⁹ und R²¹ zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass gleichzeitig nicht mehr als eines der Reste-Paare R¹⁹ und R²⁰, R²¹ und R²² bzw. R¹⁹ und R²¹ einen Carbo- oder Heterocyclus bildet,
R²³ für (C₁-C₆)-Alkyl, (C₁-C₆)-Alkoxy, (C₁-C₄)-Alkoxycarbonyl, Hydroxycarbonyl, Aminocarbonyl, Aminosulfonyl, 5- bis 10-gliedriges, über ein Ring-Kohlenstoffatom gebundenes Heterocyclyl, 5- bis 10-gliedriges Carbocyclyl, Phenyl oder 5- bis 10-gliedriges Heteroaryl steht,
worin (C₁-C₆)-Alkyl mit Cyano sowie bis zu sechsfach mit Fluor substituiert sein kann,
worin (C₁-C₆)-Alkoxy mit Hydroxy oder (C₂-C₄)-Alkenyl substituiert sein kann,
worin Aminocarbonyl mit (C₁-C₆)-Alkyl oder (C₃-C₆)-Cycloalkyl substituiert sein kann,
worin Aminosulfonyl mit (C₁-C₆)-Alkyl oder (C₃-C₆)-Cycloalkyl substituiert sein kann,
worin Phenyl mit 1 bis 3 Substituenten unabhängig voneinander ausgewählt aus der Gruppe Halogen, Cyano, Trifluormethyl, Difluormethyl, (C₁-C₄)-Alkyl, (C₁-C₄)-Alkoxy, 5- bis 10-gliedriges Heteroaryl und 4- bis 7-gliedriges Heterocyclyl substituiert sein kann,
worin (C₁-C₆)-Alkyl mit 1 oder 2 Substituenten unabhängig voneinander ausgewählt aus der Gruppe Fluor, Trifluormethoxy, (C₁-C₄)-Alkoxy, (C₃-C₆)-Cycloalkyl, Hydroxy und Amino substituiert sein kann,
worin 5- bis 10-gliedriges Heteroaryl mit 1 bis 3 Substituenten unabhängig voneinander ausgewählt aus der Gruppe Fluor, Chlor, Cyano, (C₁-C₄)-Alkyl, Trifluormethyl, (C₁-C₄)-Alkoxy und Amino, substituiert sein kann,
worin (C₁-C₄)-Alkyl mit 1 bis 3 Substituenten unabhängig voneinander ausgewählt aus der Gruppe Halogen, Cyano, Hydroxy, Amino, Trifluormethyl, Difluormethyl, (C₁-C₄)-Alkoxy, Trifluormethoxy, Difluormethoxy und Phenyl substituiert sein kann,
worin Phenyl mit 1 bis 3 Substituenten Halogen substituiert sein kann,
worin 5- bis 10-gliedriges, über ein Ring-Kohlenstoffatom gebundenes, Heterocyclyl mit 1 oder 2 Substituenten unabhängig voneinander ausgewählt aus der Gruppe Oxo, Fluor,Trifluormethyl, Hydroxy und (C₁-C₄)-Alkyl substituiert sein kann,
worin 5- bis 10-gliedriges über ein Ring-Kohlenstoffatom gebundenes, Heterocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
und
worin 5- bis 10-gliedriges Carbocyclyl mit 1 oder 2 Substituenten unabhängig voneinander ausgewählt aus der Gruppe Trifluormethyl, Fluor, Cyano, Hydroxy, Hydroxycarbonyl, (C₁-C₄)-Alkoxycarbonyl, Amino und (C₁-C₄)-Alkyl substituiert sein kann,
worin 5- bis 10-gliedriges Carbocyclyl mit einem Phenyl-Ring oder einem Pyridyl-Ring anneliert sein kann, die ihrerseits mit 1 bis 3 Substituenten ausgewählt aus der Gruppe Halogen, (C₁-C₄)-Alkyl, (C₁-C₄)-Alkoxy und Trifluormethyl substituiert sein können,
R⁴ für Wasserstoff steht,
R⁵ für Wasserstoff, Fluor, Chlor, Brom, Cyano, Difluormethyl, Trifluormethyl, (C₁-C₄)-Alkyl, Methoxy, (C₃-C₅)-Cycloalkyl oder (C₂-C₄)-Alkinyl steht,
R⁶ für Wasserstoff oder Fluor steht,
sowie ihre *N*-Oxide, Salze, Solvate, Salze der *N*-Oxide und Solvate der *N*-Oxide und Salze.

3. Verbindung der Formel (I) nach Anspruch 1 oder 2, in welcher
A für CH₂ steht,
R¹ für Cyclohexyl, Pyridyl oder Phenyl steht,
wobei Pyridyl mit 1 oder 2 Substituenten F substituiert ist,
und
wobei Phenyl mit 1 bis 4 Substituenten unabhängig voneinander ausgewählt aus der Gruppe Fluor, Chlor, Cyano, Methyl, Ethyl, Methoxy, Ethoxy und Cyclopropyl substituiert sein kann,
R² für Methyl, Cyclopropyl, Difluormethyl oder Trifluormethyl steht,
R³ für eine Gruppe der Formel 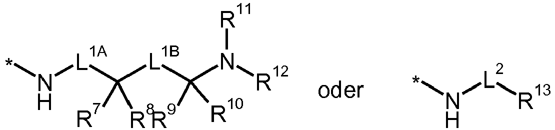 oder 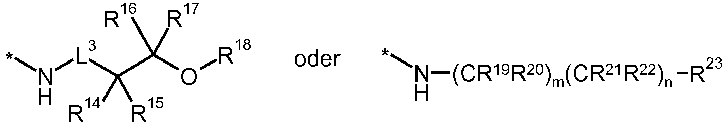 steht, wobei
* für die Anknüpfstelle an die Carbonylgruppe steht,
L^{1A} für eine Bindung oder Methandiyl steht,
L^{1B} für eine Bindung steht,
L² für eine Bindung steht,
L³ für eine Bindung steht,
R⁷ für Wasserstoff, (C₁-C₆)-Alkyl oder Phenyl steht,
worin (C₁-C₆)-Alkyl bis zu fünffach mit Fluor substituiert sein kann,
worin Phenyl mit 1 bis 3 Substituenten unabhängig voneinander ausgewählt aus der Gruppe Fluor, Chlor, Brom, Trifluormethyl, Methyl, Methoxy, Difluormethoxy und Trifluormethoxy substituiert sein können,
und
worin Phenyl an zwei benachbarten Kohlenstoffatomen des Phenyls mit einer Methylendioxy-Brücke oder Ethylendioxy-Brücke substituiert sein kann,
oder
für Fluor stehen kann, wenn L^{1A} nicht für eine Bindung steht,
R⁸ für Wasserstoff, Methyl oder Ethyl steht,
oder
für Fluor stehen kann, wenn L^{1A} nicht für eine Bindung steht,
R⁹ für Wasserstoff, (C₁-C₆)-Alkyl, Cyclopropyl oder Cyclobutyl steht,
worin (C₁-C₆)-Alkyl bis zu fünffach mit Fluor substituiert sein kann,
worin Cyclopropyl und Cyclobutyl mit Trifluormethyl substituiert sein können,
R¹⁰ für Wasserstoff oder (C₁-C₄)-Alkyl steht,
oder
R⁹ und R¹⁰ zusammen mit dem Kohlenstoffatom, an das sie gebunden sind, einen 3- bis 7-gliedrigen Carbocyclus bilden,
R¹¹ für Wasserstoff, Methyl oder Ethyl steht,
worin Ethyl bis zu dreifach mit Fluor substituiert sein kann,
R¹² für Wasserstoff, (C₁-C₄)-Alkyl, Cyclopropyl oder Cyclobutyl steht,
worin (C₁-C₆)-Alkyl bis zu fünffach mit Fluor substituiert sein kann,
oder
R¹¹ und R¹² zusammen mit dem Sticktoffatom, an das sie gebunden sind, einen Morpholinyl-Ring bilden,
R¹³ für 9-Azabicyclo[3.3.1]nonan-3-yl, Pyrrolidinyl, Piperidin-4-yl, Azepanyl oder 1,2,3,4-Tetrahydrochinolinyl steht,
worin Piperidin-4-yl mit 1 bis 5 Substituenten Methyl substituiert sein kann sowie bis zu zweifach mit Fluor substituiert sein kann,
worin1,2,3,4-Tetrahydrochinolinyl mit 1 bis 2 Substituenten unabhängig voneinander ausgewählt aus der Gruppe Fluor, Oxo, Methyl, Ethyl, Methoxy, Ethoxy und Trifluormethyl substituiert sein kann,
worin 9-Azabicyclo[3.3.1]nonan-3-yl mit Methyl substituiert sein kann,
worin Pyrrolidinyl mit 1 bis 2 Substituenten unabhängig voneinander ausgewählt aus der Gruppe Fluor, Oxo, Methyl und Ethyl substituiert sein kann,
R¹⁴ für Wasserstoff, (C₁-C₆)-Alkyl, -(C=O)NR²⁴R²⁵ oder Phenyl steht,
worin (C₁-C₆)-Alkyl mit Hydroxy oder Methoxy sowie bis zu fünffach mit Fluor substituiert sein kann,
worin R²⁴ für Aryl oder Naphthyl steht,
worin R²⁵ für Wasserstoff steht,
und
worin Phenyl mit 1 oder 2 Substituenten unabhängig voneinander ausgewählt aus der Gruppe Fluor, Chlor, Trifluormethyl und Methyl substituiert sein kann,
R¹⁵ für Wasserstoff oder (C₁-C₆)-Alkyl steht,
R¹⁶ für Wasserstoff, (C₁-C₆)-Alkyl, Cyclopropyl oder Cyclobutyl steht,
worin (C₁-C₆)-Alkyl bis zu sechsfach mit Fluor substituiert sein kann,
R¹⁷ für Wasserstoff oder (C₁-C₆)-Alkyl steht,
oder
R¹⁶ und R¹⁷ zusammen mit dem Kohlenstoffatom, an das sie gebunden sind, einen 3- bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann,
R¹⁸ für Wasserstoff oder (C₁-C₄)-Alkyl steht,
worin (C₁-C₄)-Alkyl mit 1 bis 5 Substituenten Fluor substituiert sein kann,
m für 0 oder 1 steht,
n für 0 oder 1 steht,
R¹⁹ für Wasserstoff, Cyano oder Methyl steht,
worin Methyl mit 1 bis 3 Substituenten Fluor substituiert sein kann,
R²⁰ für Wasserstoff oder Methyl steht,
worin Methyl mit 1 bis 3 Substituenten Fluor substituiert sein kann,
R²¹ für Wasserstoff oder Methyl steht,
worin Methyl mit 1 bis 3 Substituenten Fluor substituiert sein kann,
R²² für Wasserstoff oder Methyl steht,
worin Methyl mit 1 bis 3 Substituenten Fluor substituiert sein kann,
oder
R¹⁹ und R²⁰ zusammen mit dem Kohlenstoffatom, an das sie gebunden sind, einen 3- bis 5-gliedrigen Carbocyclus bilden,
oder
R¹⁹ und R²¹ zusammen mit dem Kohlenstoffatom, an das sie gebunden sind, einen Cyclopropyl-Ring bilden,
mit der Maßgabe, dass gleichzeitig nicht mehr als eines der Reste-Paare R¹⁹ und R²⁰ bzw. R¹⁹ und R²¹ einen Carbocyclus bildet,
R²³ für (C₁-C₆)-Alkyl, 2-Oxopyrrolidin-3-yl, 2-Oxotetrahydrofuran-3-yl, Cyclopentyl, Cyclohexyl, Indanyl, 1,2,4-Oxadiazol-5-yl, 1H-Imidazol-2-yl, 1H-Pyrazol-4-yl, Pyridin-3-yl, Pyrimidin-5-yl, Chinolin-4-yl, Pyrazolo[1,5-a]pyridin-3-yl, 3,4-Dihydro-2H-pyranyl, 1,2,3,4-Tetrahydronaphthalinyl, Bicyclo[2.2.2]octanyl, Chroman-4-yl, 2,3-Dihydro-1-benzofuran-3-yl, 2,3-Dihydro-1H-indenyl, 3,4-Dihydrochinolinyl, 3,4-Dihydro-2H-pyrano[2,3-b]pyridinyl oder Phenyl steht,
worin (C₁-C₆)-Alkyl mit Cyano sowie bis zu dreifach mit Fluor substituiert sein kann,
worin Phenyl mit 1 bis 3 Substituenten unabhängig voneinander ausgewählt aus der Gruppe Fluor, Chlor, Cyano, Trifluormethyl, Methyl, Ethyl, Methoxy, 1H-Imidazol-1-yl und Pyridyl substituiert sein kann,
worin 1,2,4-Oxadiazol-5-yl, 1H-Imidazol-2-yl, 1H-Pyrazol-4-yl, Pyridin-3-yl, Pyrimidin-5-yl, Chinolin-4-yl oder Pyrazolo[1,5-a]pyridin-3-yl mit 1 bis 3 Substituenten unabhängig voneinander ausgewählt aus der Gruppe Fluor, Chlor, Trifluormethyl, (C₁-C₃)-Alkyl, Amino und Hydroxy substituiert sein können,
worin (C₁-C₃)-Alkyl mit Fluor, Hydroxy, Amino oder Trifluormethyl, substituiert sein kann,
worin Cyclopentyl, Cyclohexyl und Bicyclo[2.2.2]octanyl mit Methoxy-carbonyl, Ethoxycarbonyl, Hydroxycarbonyl und Cyano substiuiert sein können,
worin Chroman-4-yl, 2,3-Dihydro-1-benzofuran-3-yl, 2,3-Dihydro-1H-indenyl, 3,4-Dihydrochinolinyl und 3,4-Dihydro-2H-pyrano[2,3-b]pyridinyl mit 1 oder 2 Substituenten ausgewählt aus der Gruppe Fluor, Chlor, Methyl, Ethyl, Methoxy, Ethoxy und Trifluormethyl substituiert sein können,
und
worin 2,3-Dihydro-1H-indenyl und Indanyl mit 1 oder 2 Substituenten ausgewählt aus der Gruppe Fluor, Chlor, Methyl, Ethyl, Methoxy, Ethoxy, Hydroxy und Trifluormethyl substituiert sein können,
worin Methyl und Ethyl mit Hydroxy substituiert sein können,
R⁴ für Wasserstoff steht,
R⁵ für Wasserstoff, Fluor, Chlor, Methyl oder Ethyl steht,
R⁶ für Wasserstoff steht,
sowie ihre *N*-Oxide, Salze, Solvate, Salze der *N*-Oxide und Solvate der *N*-Oxide und Salze.

4. Verbindung der Formel (I) nach Anspruch 1, 2 oder 3 in welcher
A für CH₂ steht,
R¹ für eine Phenyl-Gruppe der Formel 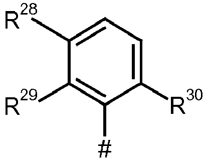 steht, wobei
# für die Anknüpfstelle an A steht,
und
R²⁸ für Wasserstoff oder Fluor steht,
R²⁹ für Fluor steht,
R³⁰ für Fluor steht,
R² für Methyl oder Cyclopropyl steht,
R³ für eine Gruppe der Formel 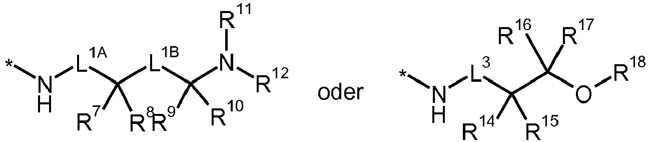 steht, wobei
* für die Anknüpfstelle an die Carbonylgruppe steht,
L^{1A} für eine Bindung steht,
L^{1B} für eine Bindung steht,
R⁷ für Wasserstoff, (C₁-C₆)-Alkyl oder Phenyl steht,
worin (C₁-C₆)-Alkyl bis zu fünffach mit Fluor substituiert sein kann,
worin Phenyl mit 1 bis 2 Substituenten Chlor oder Fluor substituiert sein kann,
R⁸ für Wasserstoff, Methyl oder Ethyl steht,
R⁹ für Wasserstoff, (C₁-C₆)-Alkyl oder Cyclopropyl steht,
worin (C₁-C₆)-Alkyl bis zu fünffach mit Fluor substituiert sein kann,
R¹⁰ für Wasserstoff, Methyl oder Ethyl steht,
R¹¹ für Wasserstoff steht,
R¹² für Wasserstoff steht,
R¹⁴ für Wasserstoff, (C₁-C₆)-Alkyl oder Phenyl steht,
worin (C₁-C₆)-Alkyl mit Hydroxy sowie bis zu fünffach mit Substituenten Fluor substituiert sein kann,
und
worin Phenyl mit 1 oder 2 Substituenten Fluor substituiert sein kann,
R¹⁵ für Wasserstoff, Methyl oder Ethyl steht,
R¹⁶ für Wasserstoff oder (C₁-C₆)-Alkyl steht,
R¹⁷ für Wasserstoff, Methyl oder Ethyl steht,
oder
R¹⁶ und R¹⁷ zusammen mit dem Kohlenstoffatom, an das sie gebunden sind, einen 3- bis 6-gliedrigen Carbocyclus bilden,
R¹⁸ für Wasserstoff steht,
R⁴ für Wasserstoff steht,
R⁵ für Wasserstoff oder Methyl steht,
R⁶ für Wasserstoff steht,
sowie ihre *N*-Oxide, Salze, Solvate, Salze der *N*-Oxide und Solvate der *N*-Oxide und Salze.

5. Verfahren zur Herstellung von Verbindungen der Formel (I), wie in den Ansprüchen 1 bis 4 definiert, **dadurch gekennzeichnet, dass** man
[A] eine Verbindung der Formel (II) 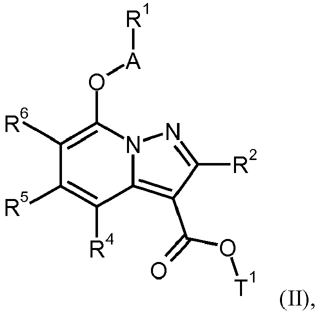 in welcher A, R¹, R², R⁴, R⁵ und R⁶ jeweils die oben angegebenen Bedeutungen haben und
T¹ für (C₁-C₄)-Alkyl oder Benzyl steht,
in einem inerten Lösungsmittel in Gegenwart einer geeigneten Base oder Säure zu einer Carbonsäure der Formel (III) 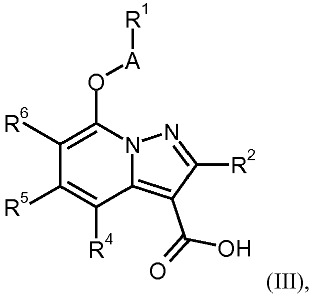 in welcher A, R¹, R², R⁴, R⁵ und R⁶ jeweils die oben angegebenen Bedeutungen haben, umsetzt und diese in der Folge in einem inerten Lösungsmittel unter Amidkupplungsbedingungen mit einem Amin der Formel (IV-A), (IV-B), (IV-C) oder (IV-D) 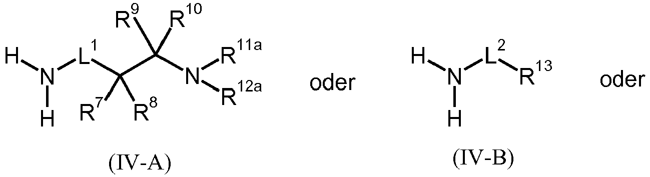 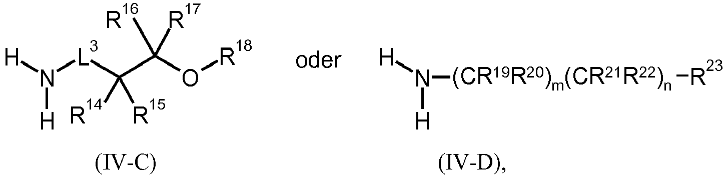 in welchem L¹ L², L³, R⁷, R⁸, R⁹, R¹⁰, R¹³, R¹⁴, R¹⁵, R¹⁶, R¹⁷, R¹⁸, R¹⁹, R²⁰, R²¹, R²² und R²³ jeweils die oben angegebenen Bedeutungen haben
und
R^{11A} und R^{12A} die oben für R¹¹ und R¹² angegebenen Bedeutungen haben oder für eine Amino-Schutzgruppe, wie beispielsweise tert.-Butoxycarbonyl, Benzyloxycarbonyl oder Benzyl stehen, umsetzt,
anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

6. Verbindung der Formel (I), wie in einem der Ansprüche 1 bis 4 definiert, zur Verwendung in der Behandlung und/oder Prophylaxe von Krankheiten.

7. Verwendung einer Verbindung der Formel (I), wie in einem der Ansprüche 1 bis 4 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

8. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 4 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

10. Arzneimittel nach Anspruch 8 oder 9 zur Verwendung in der Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

## Claims

1. Compound of the formula (I) 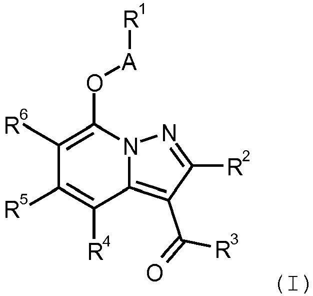 in which
A is CH₂, CD₂ or CH(CH₃),
R¹ is (C₄-C₆)-alkyl, (C₃-C₇)-cycloalkyl, pyridyl or phenyl,
where (C₄-C₆)-alkyl may be substituted up to six times by fluorine,
where (C₃-C₇)-cycloalkyl may be substituted by 1 to 4 substituents each independently selected from fluorine, trifluoromethyl and (C₁-C₄)-alkyl,
where pyridyl is substituted by 1 or 2 substituents each selected independently from the group of halogen, cyano and (C₁-C₄)-alkyl,
and
where phenyl may be substituted by 1 to 4 substituents each independently selected from the group of halogen, cyano, monofluoromethyl, difluoromethyl, trifluoromethyl, (C₁-C₄)-alkyl, (C₂-C₃)-alkynyl, (C₁-C₄)-alkoxy, (C₃-C₅)-cycloalkyl, difluoromethoxy and trifluoromethoxy, or may be substituted on two adjacent carbon atoms in the phenyl by a difluoromethylenedioxy bridge,
R² is hydrogen, (C₁-C₄)-alkyl, (C₁-C₄)-alkoxymethyl, cyclopropyl, cyclobutyl, monofluoromethyl, difluoromethyl or trifluoromethyl,
R³ is a group of the formula 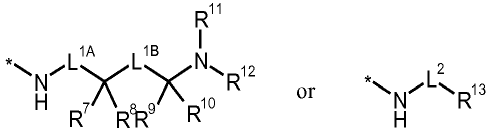 or 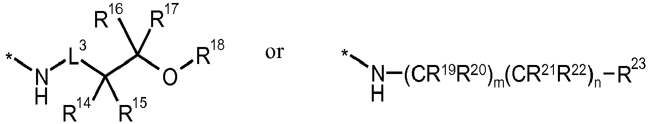 where
* is the attachment site to the carbonyl group,
L^{1A} is a bond, methanediyl, 1,2-ethanediyl or 1,3-propanediyl,
in which methanediyl, 1,2-ethanediyl or 1,3-propanediyl may be substituted by 1 or 2 substituents each independently selected from the group of fluorine, trifluoromethyl, (C₁-C₄)-alkyl, (C₃-C₅)-cycloalkyl, hydroxyl and (C₁-C₄)-alkoxy,
L^{1B} is a bond, methanediyl or 1,2-ethanediyl,
L² is a bond or (C₁-C₄) -alkanediyl,
in which (C₁-C₄)-alkanediyl may be substituted by 1 to 3 substituents each independently selected from the group of fluorine, trifluoromethyl, (C₁-C₄)-alkyl, (C₃-C₅)-cycloalkyl, hydroxyl and (C₁-C₄)-alkoxy,
L³ is a bond, methanediyl or 1,2-ethanediyl,
in which methanediyl or 1,2-ethanediyl may be substituted by 1 or 2 substituents each independently selected from the group of fluorine, trifluoromethyl, (C₁-C₄)-alkyl, (C₃-C₇)-cycloalkyl, hydroxyl and (C₁-C₄)-alkoxy,
R⁷ is hydrogen, (C₁-C₆)-alkyl, (C₃-C₇)-cycloalkyl, (C₂-C₆)-alkenyl, (C₂-C₆)-alkynyl, cyano, 5- to 10-membered heteroaryl or phenyl,
in which (C₁-C₆)-alkyl may be substituted by 1 or 2 substituents each independently selected from the group of difluoromethoxy, trifluoromethoxy, hydroxyl, (C₁-C₄)-alkoxy, (C₁-C₄)-alkoxycarbonyl, (C₁-C₄)-alkylsulphonyl, (C₁-C₄)-alkylthio, benzyloxy, phenoxy, 5- or 6-membered heteroaryl and phenyl, and may be substituted up to six times by fluorine,
in which benzyloxy, phenoxy, 5- or 6-membered heteroaryl and phenyl may be substituted by 1 to 3 substituents each independently selected from the group of halogen and (C₁-C₄)-alkoxy,
in which (C₃-C₇)-cycloalkyl may be substituted by 1 or 2 fluorine, trifluoromethyl, (C₁-C₄)-alkyl or (C₁-C₄)-alkoxy substituents,
in which phenyl and 5- to 10-membered heteroaryl may be substituted by 1 to 4 substituents each independently selected from the group of halogen, cyano, nitro, trifluoromethyl, difluoromethyl, (C₁-C₄)-alkyl, (C₂-C₄)-alkenyl, (C₁-C₄)-alkoxy, difluoromethoxy, trifluoromethoxy, (C₁-C₄-alkoxycarbonyl and (C₁-C₄)-alkylsulphonyl,
in which (C₁-C₄)-alkoxy may be substituted by hydroxyl,
in which (C₁-C₄)-alkyl may be substituted by amino or hydroxyl,
and
in which phenyl may be substituted on two adjacent carbon atoms in the phenyl by a methylenedioxy bridge or ethylenedioxy bridge,
or
may be fluorine when L^{1A} is not a bond,
R⁸ is hydrogen or (C₁-C₆)-alkyl,
or
may be fluorine when L^{1A} is not a bond,
or
R⁷ and R⁸ together with the carbon atom to which they are bonded form a 3- to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
R⁹ is hydrogen, (C₁-C₆)-alkyl, (C₃-C₇)-cycloalkyl, (C₂-C₆)-alkenyl, (C₂-C₆)-alkynyl, cyano, 5- to 10-membered heteroaryl or phenyl,
in which (C₁-C₆)-alkyl may be substituted by 1 or 2 substituents each independently selected from the group of difluoromethoxy, trifluoromethoxy, hydroxyl, (C₁-C₄)-alkoxy, (C₁-C₄)-alkoxycarbonyl, (C₁-C₄)-alkylsulphonyl, (C₁-C₄)-alkylthio, benzyloxy, phenoxy, 5- or 6-membered heteroaryl and phenyl, and may be substituted up to six times by fluorine,
in which benzyloxy, phenoxy, 5- or 6-membered heteroaryl and phenyl may be substituted by 1 to 3 substituents each independently selected from the group of halogen and (C₁-C₄)-alkoxy,
in which (C₃-C₇)-cycloalkyl may be substituted by 1 or 2 fluorine, trifluoromethyl, (C₁-C₄)-alkyl or (C₁-C₄)-alkoxy substituents,
in which phenyl and 5- to 10-membered heteroaryl may be substituted by 1 to 4 substituents each independently selected from the group of halogen, cyano, nitro, trifluoromethyl, (C₁-C₄)-alkyl, (C₂-C₄)-alkenyl, (C₁-C₄)-alkoxy, difluoromethoxy, trifluoromethoxy, (C₁-C₄)-alkoxycarbonyl and (C₁-C₄)-alkylsulphonyl,
in which (C₁-C₄)-alkoxy may be substituted by hydroxyl,
and
in which phenyl may be substituted on two adjacent carbon atoms in the phenyl by a methylenedioxy bridge or ethylenedioxy bridge,
R¹⁰ is hydrogen or (C₁-C₆)-alkyl,
or
R⁹ and R¹⁰ together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
or
R⁷ and R⁹ together with the carbon atoms to which they are bonded form a 3- to 7-membered carbocycle or a 4- to 7-membered heterocycle,
in which the 3- to 7-membered carbocycle and the 4- to 7-membered heterocycle may be substituted by 1 or 2 (C₁-C₄)-alkyl substituents,
and
in which the 3- to 7-membered carbocycle and the 4- to 7-membered heterocycle may be fused to a phenyl ring or a pyridyl ring, which may in turn be substituted by 1 or 2 substituents selected from halogen, (C₁-C₄)-alkyl and trifluoromethyl,
with the proviso that not more than one of the R⁷ and R⁸, R⁹ and R¹⁰, and R⁷ and R⁹ radical pairs at the same time forms a carbo- or heterocycle,
with the proviso that the R⁷ and R⁹ radicals are not at the same time both phenyl or 5- or 6-membered heteroaryl,
R¹¹ is hydrogen or (C₁-C₄)-alkyl,
in which (C₁-C₄)-alkyl may be substituted by 1 or 2 substituents each independently selected from the group of hydroxyl and (C₁-C₄)-alkoxy, and may be substituted up to six times by fluorine,
R¹² is hydrogen, (C₁-C₆)-alkyl, (C₁-C₄)-alkylcarbonyl, (C₁-C₄)-alkoxycarbonyl, (C₃-C₇)-cycloalkyl, phenyl or benzyl,
in which (C₁-C₆)-alkyl may be substituted by 1 to 3 substituents each independently selected from the group of fluorine, trifluoromethyl, hydroxyl, (C₁-C₄)-alkoxy and phenoxy,
and
in which phenyl and benzyl may be substituted by 1 to 3 substituents each independently selected from the group of halogen and trifluoromethyl,
or
R¹¹ and R¹² together with the nitrogen atom to which they are bonded form a 4-to 7-membered azaheterocycle,
in which the 4- to 7-membered azaheterocycle may be substituted by (C₁-C₄)-alkyl,
R¹³ is 5- to 10-membered azaheterocyclyl bonded via a ring carbon atom or 5- or 6-membered heterocyclyl bonded via a ring nitrogen atom,
in which 5- to 10-membered azaheterocyclyl bonded via a ring carbon atom may be substituted by 1 to 2 trifluoromethyl, (C₃-C₇)-cycloalkyl, oxo and benzyl substituents, and up to four times by (C₁-C₄)-alkyl and up to twice by fluorine,
in which 5- to 10-membered azaheterocyclyl may be fused to a phenyl ring or a pyridyl ring, which may in turn be substituted by 1 or 2 substituents selected from halogen, (C₁-C₄)-alkyl, (C₁-C₄)-alkoxy and trifluoromethyl,
in which 5- or 6-membered heterocyclyl bonded via a ring nitrogen atom may be substituted by (C₁-C₄)-alkyl or (C₁-C₄)-cycloalkyl,
in which (C₁-C₄)-alkyl may be substituted by difluoromethyl or trifluoromethyl,
or
may be amino when L² is a bond,
in which amino may be substituted by (C₁-C₁₀)-alkyl, (C₁-C₄) -alkylcarbonyl, (C₃-C₆)-carbocyclyl, 4- to 7-membered heterocyclyl, phenyl or 5- or 6-membered heteroaryl,
in which (C₁-C₄)-alkylcarbonyl may be substituted by monoalkylamino, dialkylamino or 5- or 6-membered heterocyclyl bonded via a ring nitrogen atom,
in which 5- or 6-membered heterocyclyl bonded via a ring nitrogen atom may be substituted by oxo,
in which (C₃-C₆) -carbocyclyl and 4-to 7-membered heterocyclyl may be substituted by hydroxyl,
and
in which phenyl and 5- or 6-membered heteroaryl may be substituted by 1 to 3 substituents each independently selected from the group of halogen, (C₁-C₄)-alkyl and trifluoromethyl,
R¹⁴ is hydrogen, (C₁-C₆)-alkyl, (C₂-C₆)-alkenyl, (C₂-C₆)-alkynyl, (C₃-C₇)-cycloalkyl, (C₁-C₄) -alkoxycarbonyl, - (C=O)NR²⁴R²⁵, 5- or 6-membered heteroaryl or phenyl,
in which (C₁-C₆) -alkyl may be substituted by 1 or 2 substituents each independently selected from the group of difluoromethoxy, trifluoromethoxy, hydroxyl, (C₁-C₄)-alkoxy, (C₁-C₄)-alkoxycarbonyl, (C₁-C₄) -alkylthio, (C₁-C₄)-alkylsulphonyl, phenyl, phenoxy and benzyloxy, and may be substituted up to six times by fluorine,
in which phenyl, phenoxy and benzyloxy may in turn be substituted by 1 to 3 halogen substituents,
in which (C₃-C₇)-cycloalkyl may be substituted by 1 or 2 substituents each independently selected from the group of fluorine, trifluoromethyl, (C₁-C₄)-alkyl and (C₁-C₄)-alkoxy,
in which R²⁴ is hydrogen, (C₁-C₄)-alkyl, (C₃-C₇)-cycloalkyl, aryl or naphthyl,
in which R²⁵ is hydrogen or methyl,
and
in which phenyl and 5- or 6-membered heteroaryl may be substituted by 1 to 3 substituents each independently selected from the group of halogen, cyano, trifluoromethyl, difluoromethyl, (C₁-C₄)-alkyl, (C₁-C₄)-alkoxy and (C₁-C₄)-alkylsulphonyl,
in which (C₁-C₄)-alkyl may be substituted by amino or hydroxyl,
R¹⁵ is hydrogen or (C₁-C₆)-alkyl,
in which (C₁-C₄)-alkyl may be substituted by hydroxyl,
or
R¹⁴ and R¹⁵ together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
R¹⁶ is hydrogen, (C₁-C₆)-alkyl, (C₂-C₆)-alkenyl, (C₂-C₆)-alkynyl, (C₃-C₇)-cycloalkyl, (C₁-C₄)-alkoxycarbonyl, 5- or 6-membered heteroaryl or phenyl, in which (C₁-C₆)-alkyl may be substituted by 1 or 2 substituents each independently selected from the group of fluorine, trifluoromethyl, difluoromethoxy, trifluoromethoxy, hydroxyl, (C₁-C₄)-alkoxy, (C₁-C₄)-alkoxycarbonyl, (C₁-C₄)-alkylthio, (C₁-C₄)-alkylsulphonyl, phenyl, phenoxy and benzyloxy, and may be substituted up to six times by fluorine,
in which phenyl, phenoxy and benzyloxy may in turn be substituted by 1 to 3 halogen substituents,
in which (C₃-C₇)-cycloalkyl may be substituted by 1 or 2 substituents each independently selected from the group of fluorine, trifluoromethyl, (C₁-C₄)-alkyl and (C₁-C₄)-alkoxy,
and
in which phenyl and 5- or 6-membered heteroaryl may be substituted by 1 to 3 substituents each independently selected from the group of halogen, cyano, trifluoromethyl, (C₁-C₄)-alkyl, (C₁-C₄)-alkoxy and (C₁-C₄)-alkylsulphonyl,
R¹⁷ is hydrogen or (C₁-C₆)-alkyl,
in which (C₁-C₄)-alkyl may be substituted by hydroxyl,
or
R¹⁶ and R¹⁷ together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl, with the proviso that the R¹⁴ and R¹⁶ radicals are not at the same time both phenyl or 5- or 6-membered heteroaryl,
or
R¹⁴ and R¹⁶ together with the carbon atoms
to which they are bonded form a 3-to 7-membered carbocycle or a 4- to 7-membered heterocycle,
with the proviso that not more than one of the R¹⁴ and R¹⁵, R¹⁶ and R¹⁷, and R¹⁴ and R¹⁶ radical pairs at the same time forms a carbo- or heterocycle,
R¹⁸ is hydrogen or (C₁-C₄)-alkyl,
in which (C₁-C₄)-alkyl may be substituted by 1 to 5 fluorine substituents,
m is 0, 1 or 2,
n is 0 or 1,
R¹⁹ is hydrogen, cyano or (C₁-C₆)-alkyl,
in which (C₁-C₆)-alkyl may be substituted by 1 to 5 fluorine substituents,
R²⁰ is hydrogen or (C₁-C₄)-alkyl,
in which (C₁-C₄)-alkyl may be substituted by 1 to 5 fluorine substituents,
R²¹ is hydrogen or (C₁-C₆)-alkyl,
in which (C₁-C₆)-alkyl may be substituted by 1 to 5 fluorine substituents,
R²² is hydrogen or (C₁-C₄)-alkyl,
in which (C₁-C₄)-alkyl may be substituted by 1 to 5 fluorine substituents,
or
R¹⁹ and R²⁰ together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
or
R²¹ and R²² together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
or
R¹⁹ and R²¹ together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
with the proviso that not more than one of the R¹⁹ and R²⁰, R²¹ and R²², and R¹⁹ and R²¹ radical pairs at the same time forms a carbo- or heterocycle,
R²³ is (C₁-C₆)-alkyl, (C₁-C₆)-alkoxy, (C₁-C₄)-alkoxycarbonyl, hydroxycarbonyl, aminocarbonyl, aminosulphonyl, 5- to 10-membered heterocyclyl bonded via a ring carbon atom, 5- to 10-membered carbocyclyl, phenyl or 5- to 10-membered heteroaryl,
in which (C₁-C₆)-alkyl may be substituted by cyano, and up to six times by fluorine,
in which (C₁-C₆)-alkoxy may be substituted by hydroxyl, amino, monoalkylamino, dialkylamino, cyclopropyl, phenyl or (C₂-C₄)-alkenyl,
in which aminocarbonyl may be substituted by (C₁-C₆)-alkyl or (C₃-C₆)-cycloalkyl,
in which aminosulphonyl may be substituted by (C₁-C₆)-alkyl or (C₃-C₆)-cycloalkyl,
in which phenyl may be substituted by 1 to 3 substituents each independently selected from the group of halogen, cyano, trifluoromethyl, difluoromethyl, (C₁-C₆)-alkyl, (C₁-C₄)-alkylcarbonyl, (C₁-C₄)-alkoxycarbonyl, hydroxycarbonyl,-(C=O)NR²⁶R²⁷, (C₁-C₄)-alkylsulphonyl, (C₃-C₆)-cycloalkylsulphonyl, (C₁-C₄)-alkylthio, (C₁-C₄)-alkoxy, trifluoromethoxy, difluoromethoxy, phenoxy, hydroxyl, 5- to 10-membered heteroaryl, 4- to 7-membered heterocyclyl and (C₃-C₇)-cycloalkyl,
in which (C₁-C₆)-alkyl may be substituted by 1 or 2 substituents each independently selected from the group of fluorine, trifluoromethoxy, (C₁-C₄)-alkylcarbonyl, -(C=O)NR²⁶R²⁷, (C₁-C₄)-alkoxy, (C₃-C₆)-cycloalkyl, morpholinyl, piperidinyl, pyrrolidinyl, piperazinyl, phenyl, hydroxyl and amino,
in which phenyl may be substituted by 1 to 3 halogen substituents,
in which amino may be substituted by 1 or 2 substituents each independently selected from (C₁-C₆)-alkyl, (C₁-C₄)-alkylcarbonyl, (C₃-C₆)-cycloalkylsulphonyl, (C₁-C₄)-alkylsulphonyl and methoxy-(C₁-C₄)-alkyl,
in which (C₃-C₆)-cycloalkyl may be substituted by amino or hydroxyl,
and in which
R²⁶ and R²⁷ are each independently hydrogen, (C₁-C₄)-alkyl or (C₃-C₇)-cycloalkyl,
in which 5- to 10-membered heteroaryl may be substituted by 1 to 3 substituents each independently selected from the group of fluorine, chlorine, cyano, (C₁-C₆)-alkyl, trifluoromethyl, (C₁-C₄)-alkoxy, amino, (C₁-C₄)-alkoxycarbonyl, hydroxycarbonyl, - (C=O)NR²⁵R²⁶, phenyl, pyridyl, pyrimidyl, 1,3-thiazol-5-yl and (C₃-C₇)-cycloalkyl,
in which (C₁-C₆)-alkyl may be substituted by 1 to 3 substituents each independently selected from the group of halogen, cyano, hydroxyl, amino, trifluoromethyl, difluoromethyl, (C₁-C₄)-alkylsulphonyl, (C₁-C₄)-alkylcarbonyl, (C₁-C₄)-alkoxycarbonyl, hydroxycarbonyl, (C₁-C₄)-alkylthio, (C₁-C₄)-alkoxy, trifluoromethoxy, difluoromethoxy, phenoxy, phenyl, pyridyl, pyrimidyl, 5-membered heteroaryl, tetrahydrothiophenyl-1,1-dioxide, (C₃-C₇)-cycloalkyl, morpholinyl, piperidinyl, pyrrolidinyl, 2-oxopyrrolidin-1-yl, piperazinyl, tetrahydrothiophenyl-1,1-dioxide, thiomorpholinyl-1,1-dioxide and azetidine,
in which 5-membered heteroaryl may be substituted by 1 to 3 substituents each independently selected from the group of halogen, (C₁-C₄)-alkyl and (C₁-C₄)-alkoxy,
in which piperidinyl may be substituted by 1 to 4 fluorine substituents,
in which phenyl may be substituted by 1 to 3 substituents each independently selected from the group of halogen, (C₁-C₄)-alkyl and (C₁-C₄)-alkoxy,
in which azetidine may be substituted by hydroxyl,
in which piperazinyl may be substituted by 1 to 3 substituents each independently selected from the group of (C₁-C₄)-alkyl, (C₃-C₇)-cycloalkyl and trifluoromethyl,
and in which
R²⁶ and R²⁷ are each independently hydrogen, (C₁-C₄)-alkyl or (C₃-C₇)-cycloalkyl,
in which 5- to 10-membered heterocyclyl bonded via a ring carbon atom may be substituted by 1 to 3 substituents each independently selected from the group of oxo, fluorine, trifluoromethyl, hydroxyl and (C₁-C₄)-alkyl,
in which 5- to 10-membered heterocyclyl bonded via a ring carbon atom may be fused to a phenyl ring or a pyridyl ring, which may in turn be substituted by 1 to 3 substituents selected from the group of halogen, (C₁-C₄)-alkyl and trifluoromethyl,
and
in which 5- to 10-membered carbocyclyl may be substituted by 1 to 3 substituents each independently selected from the group of trifluoromethyl, fluorine, cyano, hydroxyl, hydroxycarbonyl, (C₁-C₄)-alkoxycarbonyl, amino and (C₁-C₄)-alkyl,
in which (C₁-C₄)-alkyl may be substituted by hydroxyl or hydroxycarbonyl,
in which 5- to 10-membered carbocyclyl may be fused to a phenyl ring or a pyridyl ring, which may in turn be substituted by 1 to 3 substituents selected from the group of halogen, (C₁-C₄)-alkyl, (C₁-C₄)-alkoxy and trifluoromethyl,
R⁴ is hydrogen,
R⁵ is hydrogen, halogen, cyano, difluoromethyl, trifluoromethyl, (C₁-C₄)-alkyl, (C₃-C₇)-cycloalkyl, (C₂-C₄)-alkynyl, (C₁-C₄)-alkylamino, difluoromethoxy, trifluoromethoxy, (C₁-C₄)-alkoxy, amino, 4- to 7-membered heterocyclyl or 5- or 6-membered heteroaryl,
R⁶ is hydrogen, cyano or halogen,
and the *N*-oxides, salts, solvates, salts of the *N-*oxides and solvates of the *N*-oxides or salts thereof.

2. Compound of the formula (I) according to Claim 1 in which
A is CH₂ or CD₂,
R¹ is (C₃-C₆)-cycloalkyl, pyridyl or phenyl,
where (C₃-C₆)-cycloalkyl may be substituted by 1 to 2 substituents each independently selected from the group of fluorine, trifluoromethyl, methyl and ethyl,
where pyridyl is substituted by 1 or 2 fluorine substituents,
and
where phenyl may be substituted by 1 to 4 substituents each independently selected from the group of halogen, cyano, difluoromethyl, trifluoromethyl, (C₁-C₄)-alkyl, (C₁-C₄)-alkoxy and (C₃-C₅)-cycloalkyl,
R² is hydrogen, (C₁-C₄)-alkyl, cyclopropyl, cyclobutyl, difluoromethyl or trifluoromethyl,
R³ is a group of the formula 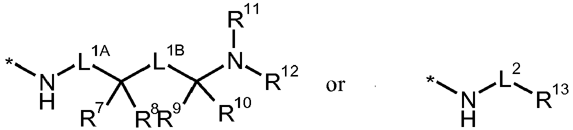 or 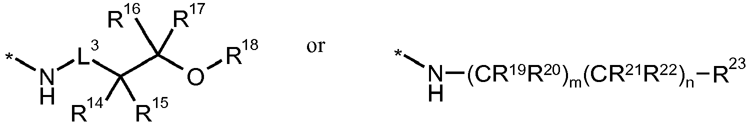 where
* is the attachment site to the carbonyl group,
L^{1A} is a bond, methanediyl, 1,2-ethanediyl or 1,3-propanediyl,
L^{1B} is a bond, methanediyl or 1,2-ethanediyl,
L² is a bond, methanediyl or 1,2-ethanediyl,
L³ is a bond, methanediyl or 1,2-ethanediyl,
R⁷ is hydrogen, (C₁-C₆)-alkyl, (C₃-C₇)-cycloalkyl, 5- or 6-membered heteroaryl or phenyl,
in which (C₁-C₆)-alkyl may be substituted up to five times by fluorine,
in which phenyl and 5- or 6-membered heteroaryl may be substituted by 1 to 3 substituents each independently selected from the group of fluorine, chlorine, bromine, cyano, nitro, trifluoromethyl, difluoromethyl, methyl, ethyl, methoxy, ethoxy, difluoromethoxy, trifluoromethoxy, methoxycarbonyl and ethoxycarbonyl,
and
in which phenyl may be substituted on two adjacent carbon atoms in the phenyl by a methylenedioxy bridge or ethylenedioxy bridge,
or
may be fluorine when L^{1A} is not a bond,
R⁸ is hydrogen or (C₁-C₄)-alkyl,
or
may be fluorine when L^{1A} is not a bond,
or
R⁷ and R⁸ together with the carbon atom to which they are bonded form a 3- to 7-membered carbocycle,
R⁹ is hydrogen, (C₁-C₆)-alkyl, (C₃-C₅)-cycloalkyl, cyano, 5- to 10-membered heteroaryl or phenyl,
in which (C₁-C₆)-alkyl may be substituted by (C₁-C₄)-alkoxy, (C₁-C₄)-alkoxycarbonyl, (C₁-C₄)-alkylsulphonyl, (C₁-C₄)-alkylthio, benzyloxy, phenoxy, 5- or 6-membered heteroaryl and phenyl, and may be substituted up to five times by fluorine,
in which benzyloxy, phenoxy, 5- or 6-membered heteroaryl and phenyl may be substituted by 1 to 3 substituents each independently selected from the group of fluorine, chlorine, bromine, methoxy and ethoxy,
in which (C₃-C₅)-cycloalkyl may be substituted by 1 or 2 fluorine, trifluoromethyl, methyl, ethyl, methoxy or ethoxy substituents,
in which phenyl and 5- to 10-membered heteroaryl may be substituted by 1 to 3 substituents each independently selected from the group of fluorine, chlorine, bromine, cyano, nitro, trifluoromethyl, methyl, ethyl, ethenyl, propenyl, methoxy, ethoxy, difluoromethoxy, trifluoromethoxy, methoxycarbonyl and ethoxycarbonyl,
in which methoxy and ethoxy may be substituted by hydroxyl,
and
in which phenyl may be substituted on two adjacent carbon atoms in the phenyl by a methylenedioxy bridge or ethylenedioxy bridge,
R¹⁰ is hydrogen or (C₁-C₄)-alkyl,
or
R⁹ and R¹⁰ together with the carbon atom to which they are bonded form a 3-to 7-membered carbocycle or a 4- to 7-membered heterocycle,
or
R⁷ and R⁹ together with the carbon atoms to which they are bonded form a 3- to 7-membered carbocycle or a 4- to 7-membered heterocycle,
in which the 3- to 7-membered carbocycle and the 4- to 7-membered heterocycle may be substituted by 1 or 2 (C₁-C₄)-alkyl substituents,
and
in which the 3- to 7-membered carbocycle and the 4- to 7-membered heterocycle may be fused to a phenyl ring or a pyridyl ring, which may in turn be substituted by 1 or 2 substituents selected from fluorine, chlorine, bromine, methyl, ethyl and trifluoromethyl,
with the proviso that not more than one of the R⁷ and R⁸, R⁹ and R¹⁰, and R⁷ and R⁹ radical pairs at the same time forms a carbo- or heterocycle,
with the proviso that the R⁷ and R⁹ radicals are not at the same time both phenyl or 5- or 6-membered heteroaryl,
R¹¹ is hydrogen or (C₁-C₄)-alkyl,
in which (C₁-C₄)-alkyl may be substituted up to five times by fluorine,
R¹² is hydrogen, (C₁-C₆)-alkyl, (C₃-C₅)-cycloalkyl or (C₁-C₄)-alkylcarbonyl,
in which (C₁-C₆)-alkyl may be substituted up to five times by fluorine,
or
R¹¹ and R¹² together with the nitrogen atom to which they are bonded form a 4- to 7-membered azaheterocycle,
in which the 4- to 7-membered azaheterocycle may be substituted by methyl or ethyl,
R¹³ is 5- to 10-membered azaheterocyclyl bonded via a ring carbon atom or 5- or 6-membered heterocyclyl bonded via a ring nitrogen atom,
in which 5- to 10-membered azaheterocyclyl bonded via a ring carbon atom may be substituted by 1 or 2 trifluoromethyl, (C₃-C₇)-cycloalkyl, oxo and benzyl substituents, and up to four times by (C₁-C₄)-alkyl and up to twice by fluorine,
in which 5- to 10-membered azaheterocyclyl may be fused to a phenyl ring or a pyridyl ring, which may in turn be substituted by 1 or 2 substituents selected from fluorine, chlorine, methyl, (C₁-C₄)-alkyl and trifluoromethyl,
in which 5- or 6-membered heterocyclyl bonded via a ring nitrogen atom may be substituted by (C₁-C₄)-alkyl or (C₁-C₄)-cycloalkyl,
or
may be amino when L² is a bond,
in which amino may be substituted by (C₁-C₄)-alkyl, (C₁-C₄)-alkylcarbonyl, (C₃-C₆)-carbocyclyl, 4- to 7-membered heterocyclyl, phenyl or 5- or 6-membered heteroaryl,
in which (C₁-C₄)-alkylcarbonyl may be substituted by monoalkylamino, dialkylamino or 5- or 6-membered heterocyclyl bonded via a ring nitrogen atom,
in which 5- or 6-membered heterocyclyl bonded via a ring nitrogen atom may be substituted by oxo,
in which (C₃-C₆)-carbocyclyl and 4-to 7-membered heterocyclyl may be substituted by hydroxyl,
and
in which phenyl and 5- or 6-membered heteroaryl may be substituted by 1 to 3 substituents each independently selected from the group of fluorine, chlorine, methyl and trifluoromethyl,
R¹⁴ is hydrogen, (C₁-C₆)-alkyl, (C₃-C₇)-cycloalkyl, -(C=O)NR²⁴R²⁵, 5- or 6-membered heteroaryl or phenyl,
in which (C₁-C₆)-alkyl may be substituted by 1 or 2 substituents each independently selected from the group of hydroxyl and (C₁-C₄)-alkoxy, and may be substituted up to six times by fluorine,
in which R²⁴ is hydrogen, (C₁-C₄)-alkyl, aryl or naphthyl,
in which R²⁵ is hydrogen,
and
in which phenyl and 5- or 6-membered heteroaryl may be substituted by 1 to 3 substituents each independently selected from the group of fluorine, chlorine, bromine, trifluoromethyl, difluoromethyl, methyl, ethyl and 1-amino-2-methylpropyl,
R¹⁵ is hydrogen or (C₁-C₆)-alkyl,
in which (C₁-C₄)-alkyl may be substituted by hydroxyl,
or
R¹⁴ and R¹⁵ together with the carbon atom to which they are bonded form a 3- to 7-membered carbocycle,
R¹⁶ is hydrogen, (C₁-C₆)-alkyl or (C₃-C₇)-cycloalkyl,
in which (C₁-C₆)-alkyl may be substituted by 1 or 2 substituents each independently selected from the group of hydroxyl and (C₁-C₄)-alkoxy, and may be substituted up to six times by fluorine,
in which (C₃-C₇)-cycloalkyl may be substituted by 1 or 2 substituents each independently selected from the group of fluorine, trifluoromethyl, (C₁-C₄)-alkyl and (C₁-C₄)-alkoxy,
R¹⁷ is hydrogen or (C₁-C₆)-alkyl,
or
R¹⁶ and R¹⁷ together with the carbon atom to which they are bonded form a 3- to 6-membered carbocycle,
in which the 3- to 6-membered carbocycle may be substituted by 1 or 2 substituents each independently selected from the group of fluorine, methyl and ethyl,
or
R¹⁴ and R¹⁶ together with the carbon atoms to which they are bonded form a 3-to 7-membered carbocycle or a 4- to 7-membered heterocycle,
with the proviso that not more than one of the R¹⁴ and R¹⁵, R¹⁶ and R¹⁷, and R¹⁴ and R¹⁶ radical pairs at the same time forms a carbo- or heterocycle,
R¹⁸ is hydrogen or (C₁-C₄)-alkyl,
in which (C₁-C₄)-alkyl may be substituted by 1 to 5 fluorine substituents,
m is 0 or 1,
n is 0 or 1,
R¹⁹ is hydrogen, cyano or (C₁-C₄)-alkyl,
in which (C₁-C₄)-alkyl may be substituted by 1 to 5 fluorine substituents,
R²⁰ is hydrogen or (C₁-C₄)-alkyl,
in which (C₁-C₄)-alkyl may be substituted by 1 to 5 fluorine substituents,
R²¹ is hydrogen or (C₁-C₄)-alkyl,
in which (C₁-C₄)-alkyl may be substituted by 1 to 5 fluorine substituents,
R²² is hydrogen or (C₁-C₄)-alkyl,
in which (C₁-C₄)-alkyl may be substituted by 1 to 5 fluorine substituents,
or
R¹⁹ and R²⁰ together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
or
R²¹ and R²² together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
or
R¹⁹ and R²¹ together with the carbon atom to which they are bonded form a 3-to 7-membered carbocycle or a 4- to 7-membered heterocycle,
in which the 3- to 7-membered carbocycle and the 4- to 7-membered heterocycle may in turn be substituted by 1 or 2 substituents each independently selected from the group of fluorine and (C₁-C₄)-alkyl,
with the proviso that not more than one of the R¹⁹ and R²⁰, R²¹ and R²², and R¹⁹ and R²¹ radical pairs at the same time forms a carbo- or heterocycle,
R²³ is (C₁-C₆)-alkyl, (C₁-C₆)-alkoxy, (C₁-C₄)-alkoxycarbonyl, hydroxycarbonyl, aminocarbonyl, aminosulphonyl, 5- to 10-membered heterocyclyl bonded via a ring carbon atom, 5- to 10-membered carbocyclyl, phenyl or 5- to 10-membered heteroaryl,
in which (C₁-C₆)-alkyl may be substituted by cyano and up to six times by fluorine,
in which (C₁-C₆)-alkoxy may be substituted by hydroxyl or (C₂-C₄)-alkenyl,
in which aminocarbonyl may be substituted by (C₁-C₆)-alkyl or (C₃-C₆)-cycloalkyl,
in which aminosulphonyl may be substituted by (C₁-C₆)-alkyl or (C₃-C₆)-cycloalkyl,
in which phenyl may be substituted by 1 to 3 substituents each independently selected from the group of halogen, cyano, trifluoromethyl, difluoromethyl, (C₁-C₄)-alkyl, (C₁-C₄)-alkoxy, 5- to 10-membered heteroaryl and 4- to 7-membered heterocyclyl,
in which (C₁-C₆)-alkyl may be substituted by 1 or 2 substituents each independently selected from the group of fluorine, trifluoromethoxy, (C₁-C₄)-alkoxy, (C₃-C₆)-cycloalkyl, hydroxyl and amino,
in which 5- to 10-membered heteroaryl may be substituted by 1 to 3 substituents each independently selected from the group of fluorine, chlorine, cyano, (C₁-C₄)-alkyl, trifluoromethyl, (C₁-C₄)-alkoxy and amino,
in which (C₁-C₄)-alkyl may be substituted by 1 to 3 substituents each independently selected from the group of halogen, cyano, hydroxyl, amino, trifluoromethyl, difluoromethyl, (C₁-C₄)-alkoxy, trifluoromethoxy, difluoromethoxy and phenyl,
in which phenyl may be substituted by 1 to 3 halogen substituents,
in which 5- to 10-membered heterocyclyl bonded via a ring carbon atom may be substituted by 1 or 2 substituents each independently selected from the group of oxo, fluorine, trifluoromethyl, hydroxyl and (C₁-C₄)-alkyl,
in which 5- to 10-membered heterocyclyl bonded via a ring carbon atom may be fused to a phenyl ring or a pyridyl ring, which may in turn be substituted by 1 to 3 substituents selected from the group of halogen, (C₁-C₄)-alkyl, (C₁-C₄)-alkoxy and trifluoromethyl,
and
in which 5- to 10-membered carbocyclyl may be substituted by 1 or 2 substituents each independently selected from the group of trifluoromethyl, fluorine, cyano, hydroxyl, hydroxycarbonyl, (C₁-C₄)-alkoxycarbonyl, amino and (C₁-C₄)-alkyl,
in which 5- to 10-membered carbocyclyl may be fused to a phenyl ring or a pyridyl ring, which may in turn be substituted by 1 to 3 substituents selected from the group of halogen, (C₁-C₄)-alkyl, (C₁-C₄)-alkoxy and trifluoromethyl,
R⁴ is hydrogen,
R⁵ is hydrogen, fluorine, chlorine, bromine, cyano, difluoromethyl, trifluoromethyl, (C₁-C₄)-alkyl, methoxy, (C₃-C₅)-cycloalkyl or (C₂-C₄)-alkynyl,
R⁶ is hydrogen or fluorine,
and the *N*-oxides, salts, solvates, salts of the *N-*oxides and solvates of the *N*-oxides or salts thereof.

3. Compound of the formula (I) according to Claim 1 or 2 in which
A is CH₂,
R¹ is cyclohexyl, pyridyl or phenyl,
where pyridyl is substituted by 1 or 2 F substituents,
and
where phenyl may be substituted by 1 to 4 substituents each independently selected from the group of fluorine, chlorine, cyano, methyl, ethyl, methoxy, ethoxy and cyclopropyl,
R² is methyl, cyclopropyl, difluoromethyl or trifluoromethyl,
R³ is a group of the formula 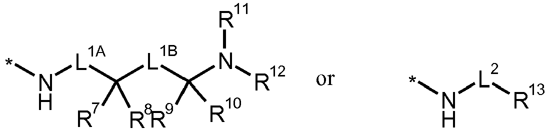 or 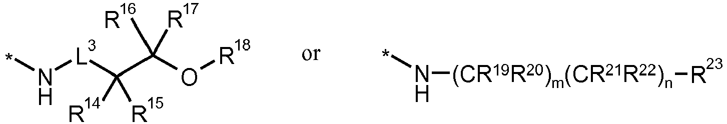 where
* is the attachment site to the carbonyl group,
L^{1A} is a bond or methanediyl,
L^{1B} is a bond,
L² is a bond,
L³ is a bond,
R⁷ is hydrogen, (C₁-C₆)-alkyl or phenyl,
in which (C₁-C₆)-alkyl may be substituted up to five times by fluorine,
in which phenyl may be substituted by 1 to 3 substituents each independently selected from the group of fluorine, chlorine, bromine, trifluoromethyl, methyl, methoxy, difluoromethoxy and trifluoromethoxy,
and
in which phenyl may be substituted on two adjacent carbon atoms in the phenyl by a methylenedioxy bridge or ethylenedioxy bridge,
or
may be fluorine when L^{1A} is not a bond,
R⁸ is hydrogen, methyl or ethyl,
or
may be fluorine when L^{1A} is not a bond,
R⁹ is hydrogen, (C₁-C₆)-alkyl, cyclopropyl or cyclobutyl,
in which (C₁-C₆)-alkyl may be substituted up to five times by fluorine,
in which cyclopropyl and cyclobutyl may be substituted by trifluoromethyl,
R¹⁰ is hydrogen or (C₁-C₄)-alkyl,
or
R⁹ and R¹⁰ together with the carbon atom to which they are bonded form a 3- to 7-membered carbocycle,
R¹¹ is hydrogen, methyl or ethyl,
in which ethyl may be substituted up to three times by fluorine,
R¹² is hydrogen, (C₁-C₄)-alkyl, cyclopropyl or cyclobutyl,
in which (C₁-C₆)-alkyl may be substituted up to five times by fluorine,
or
R¹¹ and R¹² together with the nitrogen atom to which they are bonded form a morpholinyl ring,
R¹³ is 9-azabicyclo[3.3.1]nonan-3-yl, pyrrolidinyl, piperidin-4-yl, azepanyl or 1,2,3,4-tetrahydroquinolinyl,
in which piperidin-4-yl may be substituted by 1 to 5 methyl substituents and may be substituted up to twice by fluorine,
in which 1,2,3,4-tetrahydroquinolinyl may be substituted by 1 to 2 substituents each independently selected from the group of fluorine, oxo, methyl, ethyl, methoxy, ethoxy and trifluoromethyl,
in which 9-azabicyclo[3.3.1]nonan-3-yl may be substituted by methyl,
in which pyrrolidinyl may be substituted by 1 to 2 substituents independently of one another selected from the group consisting of fluorine, oxo, methyl and ethyl,
R¹⁴ is hydrogen, (C₁-C₆)-alkyl, -(C=O)NR²⁴R²⁵ or phenyl,
in which (C₁-C₆)-alkyl may be substituted by hydroxyl or methoxy and up to five times by fluorine,
in which R²⁴ is aryl or naphthyl,
in which R²⁵ is hydrogen,
and
in which phenyl may be substituted by 1 or 2 substituents independently of one another selected from the group consisting of fluorine, chlorine, trifluoromethyl and methyl,
R¹⁵ is hydrogen or (C₁-C₆)-alkyl,
R¹⁶ is hydrogen, (C₁-C₆)-alkyl, cyclopropyl or cyclobutyl,
in which (C₁-C₆)-alkyl may be substituted up to six times by fluorine,
R¹⁷ is hydrogen or (C₁-C₆)-alkyl,
or
R¹⁶ and R¹⁷ together with the carbon atom to which they are bonded form a 3- to 6-membered carbocycle,
in which the 3- to 6-membered carbocycle may be substituted by 1 or 2 substituents each independently selected from the group of fluorine and methyl,
R¹⁸ is hydrogen or (C₁-C₄)-alkyl,
in which (C₁-C₄)-alkyl may be substituted by 1 to 5 fluorine substituents,
m is 0 or 1,
n is 0 or 1,
R¹⁹ is hydrogen, cyano or methyl,
in which methyl may be substituted by 1 to 3 fluorine substituents,
R²⁰ is hydrogen or methyl,
in which methyl may be substituted by 1 to 3 fluorine substituents,
R²¹ is hydrogen or methyl,
in which methyl may be substituted by 1 to 3 fluorine substituents,
R²² is hydrogen or methyl,
in which methyl may be substituted by 1 to 3 fluorine substituents,
or
R¹⁹ and R²⁰ together with the carbon atom to which they are bonded form a 3- to 5-membered carbocycle,
or
R¹⁹ and R²¹ together with the carbon atom to which they are bonded form a cyclopropyl ring,
with the proviso that not more than one of the R¹⁹ and R²⁰, and R¹⁹ and R²¹ radical pairs at the same time forms a carbocycle,
R²³ is (C₁-C₆)-alkyl, 2-oxopyrrolidin-3-yl, 2-oxotetrahydrofuran-3-yl, cyclopentyl, cyclohexyl, indanyl, 1,2,4-oxadiazol-5-yl, 1H-imidazol-2-yl, 1H-pyrazol-4-yl, pyridin-3-yl, pyrimidin-5-yl, quinolin-4-yl, pyrazolo[1,5-a]pyridin-3-yl, 3,4-dihydro-2H-pyranyl, 1,2,3,4-tetrahydronaphthalenyl, bicyclo[2.2.2]octanyl, chroman-4-yl, 2,3-dihydro-1-benzofuran-3-yl, 2,3-dihydro-1H-indenyl, 3,4-dihydroquinolinyl, 3,4-dihydro-2H-pyrano[2,3-b]pyridinyl or phenyl,
in which (C₁-C₆)-alkyl may be substituted by cyano and up to three times by fluorine,
in which phenyl may be substituted by 1 to 3 substituents each independently selected from the group of fluorine, chlorine, cyano, trifluoromethyl, methyl, ethyl, methoxy, 1H-imidazol-1-yl and pyridyl,
in which 1,2,4-oxadiazol-5-yl, 1H-imidazol-2-yl, 1H-pyrazol-4-yl, pyridin-3-yl, pyrimidin-5-yl, quinolin-4-yl or pyrazolo[1,5-a]pyridin-3-yl may be substituted by 1 to 3 substituents each independently selected from the group of fluorine, chlorine, trifluoromethyl, (C₁-C₃)-alkyl, amino and hydroxyl,
in which (C₁-C₃)-alkyl may be substituted by fluorine, hydroxyl, amino or trifluoromethyl,
in which cyclopentyl, cyclohexyl and bicyclo[2.2.2]octanyl may be substituted by methoxycarbonyl, ethoxycarbonyl, hydroxycarbonyl and cyano,
in which chroman-4-yl, 2,3-dihydro-1-benzofuran-3-yl, 2,3-dihydro-1H-indenyl, 3,4-dihydroquinolinyl and 3,4-dihydro-2H-pyrano[2,3-b]pyridinyl may be substituted by 1 or 2 substituents selected from the group of fluorine, chlorine, methyl, ethyl, methoxy, ethoxy and trifluoromethyl,
and
in which 2,3-dihydro-1H-indenyl and indanyl may be substituted by 1 or 2 substituents selected from the group of fluorine, chlorine, methyl, ethyl, methoxy, ethoxy, hydroxyl and trifluoromethyl,
in which methyl and ethyl may be substituted by hydroxyl,
R⁴ is hydrogen,
R⁵ is hydrogen, fluorine, chlorine, methyl or ethyl,
R⁶ is hydrogen,
and the N-oxides, salts, solvates, salts of the *N-*oxides and solvates of the N-oxides or salts thereof.

4. Compound of the formula (I) according to Claim 1, 2 or 3 in which
A is CH₂,
R¹ is a phenyl group of the formula 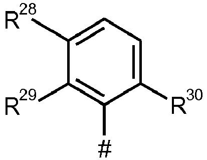 where
# is the attachment site to A, and
R²⁸ is hydrogen or fluorine,
R²⁹ is fluorine,
R³⁰ is fluorine,
R² is methyl or cyclopropyl,
R³ is a group of the formula 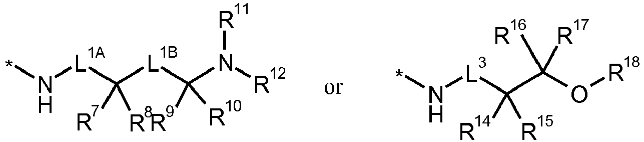 where
* is the attachment site to the carbonyl group,
L^{1A} is a bond,
L^{1B} is a bond,
R⁷ is hydrogen, (C₁-C₆)-alkyl or phenyl,
in which (C₁-C₆)-alkyl may be substituted up to five times by fluorine,
in which phenyl may be substituted by 1 to 2 chlorine or fluorine substituents,
R⁸ is hydrogen, methyl or ethyl,
R⁹ is hydrogen, (C₁-C₆)-alkyl or cyclopropyl,
in which (C₁-C₆)-alkyl may be substituted up to five times by fluorine,
R¹⁰ is hydrogen, methyl or ethyl,
R¹¹ is hydrogen,
R¹² is hydrogen,
R¹⁴ is hydrogen, (C₁-C₆)-alkyl or phenyl,
in which (C₁-C₆)-alkyl may be substituted by hydroxyl and up to five times by fluorine substituents,
and
in which phenyl may be substituted by 1 or 2 fluorine substituents,
R¹⁵ is hydrogen, methyl or ethyl,
R¹⁶ is hydrogen or (C₁-C₆)-alkyl,
R¹⁷ is hydrogen, methyl or ethyl,
or
R¹⁶ and R¹⁷ together with the carbon atom to which they are bonded form a 3- to 6-membered carbocycle,
R¹⁸ is hydrogen,
R⁴ is hydrogen,
R⁵ is hydrogen or methyl,
R⁶ is hydrogen,
and the N-oxides, salts, solvates, salts of the *N-*oxides and solvates of the N-oxides or salts thereof.

5. Process for preparing compounds of the formula (I) as defined in any of Claims 1 to 4, **characterized in that**
[A] a compound of the formula (II) 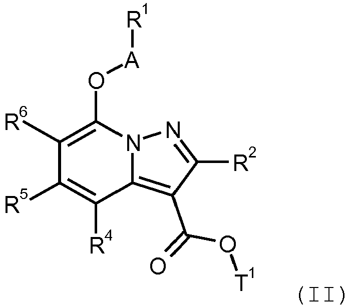 in which A, R¹, R², R⁴, R⁵ and R⁶ are each as defined above and
T¹ is (C₁-C₄)-alkyl or benzyl,
is converted in an inert solvent in the presence of a suitable base or acid to a carboxylic acid of the formula (III) 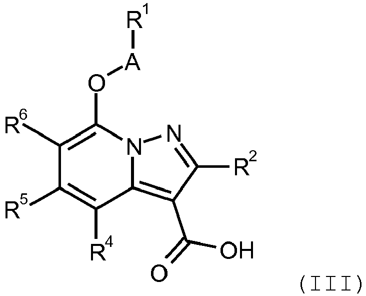 in which A, R¹, R², R⁴, R⁵ and R⁶ are each as defined above,
and the latter are subsequently reacted, in an inert solvent under amide coupling conditions, with an amine of the formula (IV-A), (IV-B), (IV-C) or (IV-D) 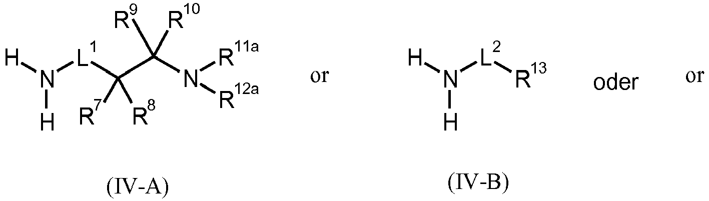 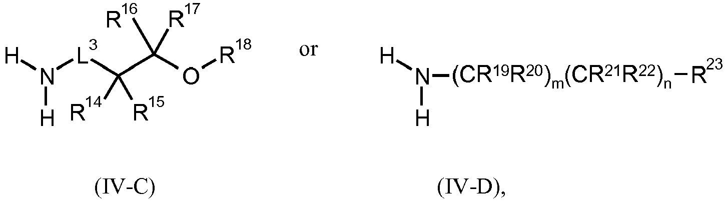 in which L¹, L², L³, R⁷, R⁸, R⁹, R¹⁰, R¹³, R¹⁴, R¹⁵, R¹⁶, R¹⁷, R¹⁸, R¹⁹, R²⁰, R²¹, R²² and R²³ are each as defined above
and
R^{11A} and R^{12A} are each as defined above for R¹¹ and R¹² or are an amino protecting group, for example tert-butoxycarbonyl, benzyloxycarbonyl or benzyl,
then any protecting groups present are detached, and the resulting compounds of the formula (I) are optionally converted with the appropriate (i) solvents and/or (ii) acids or bases to the solvates, salts and/or solvates of the salts thereof.

6. Compound of the formula (I) as defined in any of Claims 1 to 4 for use in the treatment and/or prophylaxis of diseases.

7. Use of a compound of the formula (I) as defined in any of Claims 1 to 4 for production of a medicament for treatment and/or prophylaxis of heart failure, angina pectoris, hypertension, pulmonary hypertension, ischaemias, vascular disorders, renal insufficiency, thromboembolic disorders and arteriosclerosis.

8. Medicament comprising a compound of the formula (I) as defined in any of Claims 1 to 4 in combination with an inert, non-toxic, pharmaceutically suitable excipient.

9. Medicament comprising a compound of the formula (I) as defined in any of Claims 1 to 4 in combination with a further active ingredient selected from the group consisting of organic nitrates, NO donors, cGMP-PDE inhibitors, antithrombotic agents, hypotensive agents and lipid metabolism modifiers.

10. Medicament according to Claim 8 or 9 for use in the treatment and/or prophylaxis of heart failure, angina pectoris, hypertension, pulmonary hypertension, ischaemias, vascular disorders, kidney failure, thromboembolic disorders and arteriosclerosis.

## Revendications

1. Composé de formule (I) 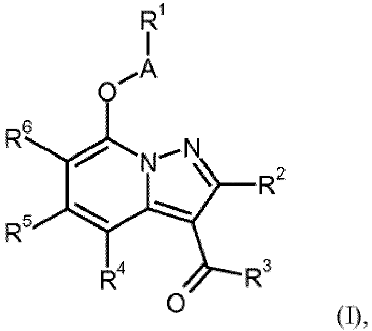 dans laquelle
A représente CH₂, CD₂ ou CH(CH₃),
R¹ représente alkyle en (C₄-C₆), cycloalkyle en (C₃-C₇), pyridyle ou phényle,
l'alkyle en (C₄-C₆) pouvant être substitué jusqu'à six fois avec fluor,
le cycloalkyle en (C₃-C₇) pouvant être substitué avec 1 à 4 substituants choisis indépendamment les uns des autres dans le groupe constitué par fluor, trifluorométhyle et alkyle en (C₁-C₄),
le pyridyle étant substitué avec 1 ou 2 substituants choisis indépendamment l'un de l'autre dans le groupe constitué par halogène, cyano et alkyle en (C₁-C₄),
et
le phényle pouvant être substitué avec 1 à 4 substituants choisis indépendamment les uns des autres dans le groupe constitué par halogène, cyano, monofluorométhyle, difluorométhyle, trifluorométhyle, alkyle en (C₁-C₄), alcynyle en (C₂-C₃), alcoxy en (C₁-C₄), cycloalkyle en (C₃-C₅), difluorométhoxy et trifluorométhoxy, ou pouvant être substitué sur deux atomes de carbone voisins du phényle avec un pont difluorométhylènedioxy,
R² représente hydrogène, alkyle en (C₁-C₄), alcoxyméthyle en (C₁-C₄), cyclopropyle, cyclobutyle, monofluorométhyle, difluorométhyle ou trifluorométhyle,
R³ représente un groupe de formule 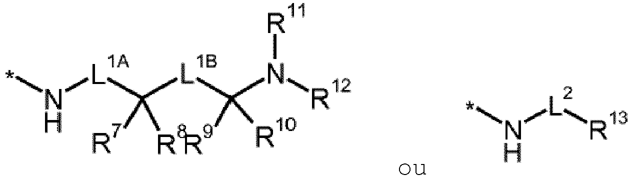 ou 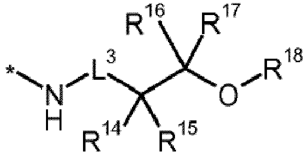 ou 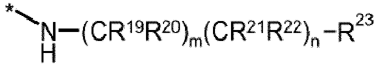 dans lesquelles
* représente l'emplacement de liaison au groupe carbonyle,
L^{1A} représente une liaison, méthanediyle, 1,2-éthanediyle ou 1,3-propanediyle,
le méthanediyle, le 1,2-éthanediyle ou le 1,3-propanediyle pouvant être substitué avec 1 ou 2 substituants choisis indépendamment l'un de l'autre dans le groupe constitué par fluor, trifluorométhyle, alkyle en (C₁-C₄), cycloalkyle en (C₃-C₅), hydroxy et alcoxy en (C₁-C₄),
L^{1B} représente une liaison, méthanediyle ou 1,2-éthanediyle,
L² représente une liaison ou alcanediyle en (C₁-C₄),
l'alcanediyle en (C₁-C₄) pouvant être substitué avec 1 à 3 substituants choisis indépendamment les uns des autres dans le groupe constitué par fluor, trifluorométhyle, alkyle en (C₁-C₄), cycloalkyle en (C₃-C₅), hydroxy et alcoxy en (C₁-C₄),
L³ représente une liaison, méthanediyle ou 1,2-éthanediyle,
le méthanediyle ou le 1,2-éthanediyle pouvant être substitué avec 1 ou 2 substituants choisis indépendamment l'un de l'autre dans le groupe constitué par fluor, trifluorométhyle, alkyle en (C₁-C₄), cycloalkyle en (C₃-C₇), hydroxy et alcoxy en (C₁-C₄),
R⁷ représente hydrogène, alkyle en (C₁-C₆), cycloalkyle en (C₃-C₇), alcényle en (C₂-C₆), alcynyle en (C₂-C₆), cyano, hétéroaryle de 5 à 10 éléments ou phényle,
l'alkyle en (C₁-C₆) pouvant être substitué avec 1 ou 2 substituants choisis indépendamment l'un de l'autre dans le groupe constitué par difluorométhoxy, trifluorométhoxy, hydroxy, alcoxy en (C₁-C₄), alcoxycarbonyle en (C₁-C₄), alkylsulfonyle en (C₁-C₄), alkylthio en (C₁-C₄), benzyloxy, phénoxy, hétéroaryle à 5 ou 6 éléments, et phényle, ainsi que jusqu'à six fois avec fluor,
le benzyloxy, le phénoxy, l'hétéroaryle à 5 ou 6 éléments et le phényle pouvant être substitués avec 1 à 3 substituants choisis indépendamment les uns des autres dans le groupe constitué par halogène et alcoxy en (C₁-C₄)
le cycloalkyle en (C₃-C₇) pouvant être substitué avec 1 ou 2 substituants fluor, trifluorométhyle, alkyle en (C₁-C₄) ou alcoxy en (C₁-C₄),
le phényle et l'hétéroaryle de 5 à 10 éléments pouvant être substitués avec 1 à 4 substituants choisis indépendamment les uns des autres dans le groupe constitué par halogène, cyano, nitro, trifluorométhyle, difluorométhyle, alkyle en (C₁-C₄), alcényle en (C₂-C₄), alcoxy en (C₁-C₄), difluorométhoxy, trifluorométhoxy, alcoxycarbonyle en (C₁-C₄) et alkylsulfonyle en (C₁-C₄),
l'alcoxy en (C₁-C₄) pouvant être substitué avec hydroxy,
l'alkyle en (C₁-C₄) pouvant être substitué avec amino ou hydroxy,
et
le phényle pouvant être substitué sur deux atomes de carbone voisins du phényle avec un pont méthylènedioxy ou un pont éthylènedioxy,
ou
peut représenter fluor lorsque L^{1A} ne représente pas une liaison,
R⁸ représente hydrogène ou alkyle en (C₁-C₆),
ou
peut représenter fluor lorsque L^{1A} ne représente pas une liaison,
ou
R⁷ et R⁸ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
R⁹ représente hydrogène, alkyle en (C₁-C₆), cycloalkyle en (C₃-C₇), alcényle en (C₂-C₆), alcynyle en (C₂-C₆), cyano, hétéroaryle de 5 à 10 éléments ou phényle,
l'alkyle en (C₁-C₆) pouvant être substitué avec 1 ou 2 substituants choisis indépendamment l'un de l'autre dans le groupe constitué par difluorométhoxy, trifluorométhoxy, hydroxy, alcoxy en (C₁-C₄), alcoxycarbonyle en (C₁-C₄) alkylsulfonyle en (C₁-C₄), alkylthio en (C₁-C₄), benzyloxy, phénoxy, hétéroaryle à 5 ou 6 éléments, et phényle, ainsi que jusqu'à six fois avec fluor,
le benzyloxy, le phénoxy, l'hétéroaryle à 5 ou 6 éléments et le phényle pouvant être substitués avec 1 à 3 substituants choisis indépendamment les uns des autres dans le groupe constitué par halogène et alcoxy en (C₁-C₄),
le cycloalkyle en (C₃-C₇) pouvant être substitué avec 1 ou 2 substituants fluor, trifluorométhyle, alkyle en (C₁-C₄) ou alcoxy en (C₁-C₄),
le phényle et l'hétéroaryle de 5 à 10 éléments pouvant être substitués avec 1 à 4 substituants choisis indépendamment les uns des autres dans le groupe constitué par halogène, cyano, nitro, trifluorométhyle, alkyle en (C₁-C₄), alcényle en (C₂-C₄), alcoxy en (C₁-C₄), difluorométhoxy, trifluorométhoxy, alcoxycarbonyle en (C₁-C₄) et alkylsulfonyle en (C₁-C₄),
l'alcoxy en (C₁-C₄) pouvant être substitué avec hydroxy,
et
le phényle pouvant être substitué sur deux atomes de carbone voisins du phényle avec un pont méthylènedioxy ou un pont éthylènedioxy,
R¹⁰ représente hydrogène ou alkyle en (C₁-C₆),
ou
R⁹ et R¹⁰ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
ou
R⁷ et R⁹ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant être substitués avec 1 ou 2 substituants alkyle en (C₁-C₄),
et
le carbocycle de 3 à 7 éléments et l'hétérocycle de 4 à 7 éléments pouvant être annelés avec un cycle phényle ou un cycle pyridyle, qui peuvent de leur côté être substitués avec 1 ou 2 substituants choisis parmi halogène, alkyle en (C₁-C₄) et trifluorométhyle,
à condition que pas plus d'une des paires de radicaux R⁷ et R⁸, R⁹ et R¹⁰, et R⁷ et R⁹ ne forment simultanément un carbo- ou hétérocycle,
à condition que les radicaux R⁷ et R⁹ ne représentent pas tous les deux simultanément phényle ou hétéroaryle à 5 ou 6 éléments,
R¹¹ représente hydrogène ou alkyle en (C₁-C₄),
l'alkyle en (C₁-C₄) pouvant être substitué avec 1 ou 2 substituants choisis indépendamment l'un de l'autre dans le groupe constitué par hydroxy et alcoxy en (C₁-C₄), ainsi que jusqu'à six fois avec fluor,
R¹² représente hydrogène, alkyle en (C₁-C₆), alkylcarbonyle en (C₁-C₄), alcoxycarbonyle en (C₁-C₄), cycloalkyle en (C₃-C₇), phényle ou benzyle,
l'alkyle en (C₁-C₆) pouvant être substitué avec 1 à 3 substituants choisis indépendamment les uns des autres dans le groupe constitué par fluor, trifluorométhyle, hydroxy, alcoxy en (C₁-C₄) et phénoxy,
et
le phényle et le benzyle pouvant être substitués avec 1 à 3 substituants choisis indépendamment les uns des autres dans le groupe constitué par halogène et trifluorométhyle,
ou
R¹¹ et R¹² forment ensemble avec l'atome d'azote auquel ils sont reliés un hétérocycle aza de 4 à 7 éléments,
l'hétérocycle aza de 4 à 7 éléments pouvant être substitué avec alkyle en (C₁-C₄),
R¹³ représente un hétérocyclyle aza de 5 à 10 éléments, relié par un atome de carbone de cycle, ou un hétérocyclyle à 5 ou 6 éléments, relié par un atome d'azote de cycle,
l'hétérocyclyle aza de 5 à 10 éléments, relié par un atome de carbone de cycle pouvant être substitué avec 1 à 2 substituants trifluorométhyle, cycloalkyle en (C₃-C₇), oxo et benzyle, ainsi que jusqu'à quatre fois avec alkyle en (C₁-C₄) et jusqu'à deux fois avec fluor,
l'hétérocyclyle aza de 5 à 10 éléments pouvant être annelé avec un cycle phényle ou un cycle pyridyle, qui peuvent de leur côté être substitués avec 1 ou 2 substituants choisis parmi halogène, alkyle en (C₁-C₄), alcoxy en (C₁-C₄) et trifluorométhyle,
l'hétérocyclyle à 5 ou 6 éléments relié par un atome d'azote de cycle pouvant être substitué avec alkyle en (C₁-C₄) ou cycloalkyle en (C₁-C₄),
l'alkyle en (C₁-C₄) pouvant être substitué avec difluorométhyle ou trifluorométhyle,
ou
peut représenter amino lorsque L² représente une liaison,
l'amino pouvant être substitué avec alkyle en (C₁-C₁₀), alkylcarbonyle en (C₁-C₄), carbocyclyle en (C₃-C₆), hétérocyclyle de 4 à 7 éléments, phényle ou hétéroaryle à 5 ou 6 éléments,
l'alkylcarbonyle en (C₁-C₄) pouvant être substitué avec monoalkylamino, dialkylamino ou un hétérocycle à 5 ou 6 éléments relié par un atome d'azote de cycle,
l'hétérocyclyle à 5 ou 6 éléments relié par un atome d'azote de cycle pouvant être substitué avec oxo,
le carbocyclyle en (C₃-C₆) et l'hétérocyclyle de 4 à 7 éléments pouvant être substitués avec hydroxy,
et
le phényle et l'hétéroaryle à 5 ou 6 éléments pouvant être substitués avec 1 à 3 substituants choisis indépendamment les uns des autres dans le groupe constitué par halogène, alkyle en (C₁-C₄) et trifluorométhyle,
R¹⁴ représente hydrogène, alkyle en (C₁-C₆), alcényle en (C₂-C₆), alcynyle en (C₂-C₆), cycloalkyle en (C₃-C₇), alcoxycarbonyle en (C₁-C₄), - (C=O)NR²⁴R²⁵, hétéroaryle à 5 ou 6 éléments ou phényle,
l'alkyle en (C₁-C₆) pouvant être substitué avec 1 ou 2 substituants choisis indépendamment l'un de l'autre dans le groupe constitué par difluorométhoxy, trifluorométhoxy, hydroxy, alcoxy en (C₁-C₄), alcoxycarbonyle en (C₁-C₄), alkylthio en (C₁-C₄), alkylsulfonyle en (C₁-C₄), phényle, phénoxy et benzyloxy, ainsi que jusqu'à six fois avec fluor,
le phényle, le phénoxy et le benzyloxy pouvant de leur côté être substitués avec 1 à 3 substituants halogène,
le cycloalkyle en (C₃-C₇) pouvant être substitué avec 1 ou 2 substituants choisis indépendamment l'un de l'autre dans le groupe constitué par fluor, trifluorométhyle, alkyle en (C₁-C₄) et alcoxy en (C₁-C₄),
R²⁴ représentant hydrogène, alkyle en (C₁-C₄), cycloalkyle en (C₃-C₇), aryle ou naphtyle,
R²⁵ représentant hydrogène ou méthyle,
et
le phényle et l'hétéroaryle à 5 ou 6 éléments pouvant être substitués avec 1 à 3 substituants choisis indépendamment les uns des autres dans le groupe constitué par halogène, cyano, trifluorométhyle, difluorométhyle, alkyle en (C₁-C₄), alcoxy en (C₁-C₄) et alkylsulfonyle en (C₁-C₄),
l'alkyle en (C₁-C₄) pouvant être substitué avec amino ou hydroxy,
R¹⁵ représente hydrogène ou alkyle en (C₁-C₆),
l'alkyle en (C₁-C₄) pouvant être substitué avec hydroxy,
ou
R¹⁴ et R¹⁵ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
R¹⁶ représente hydrogène, alkyle en (C₁-C₆), alcényle en (C₂-C₆), alcynyle en (C₂-C₆), cycloalkyle en (C₃-C₇), alcoxycarbonyle en (C₁-C₄), hétéroaryle à 5 ou 6 éléments ou phényle,
l'alkyle en (C₁-C₆) pouvant être substitué avec 1 ou 2 substituants choisis indépendamment l'un de l'autre dans le groupe constitué par fluor, trifluorométhyle, difluorométhoxy, trifluorométhoxy, hydroxy, alcoxy en (C₁-C₄), alcoxycarbonyle en (C₁-C₄), alkylthio en (C₁-C₄), alkylsulfonyle en (C₁-C₄), phényle, phénoxy et benzyloxy, ainsi que jusqu'à six fois avec fluor,
le phényle, le phénoxy et le benzyloxy pouvant de leur côté être substitués avec 1 à 3 substituants halogène,
le cycloalkyle en (C₃-C₇) pouvant être substitué avec 1 ou 2 substituants choisis indépendamment l'un de l'autre dans le groupe constitué par fluor, trifluorométhyle, alkyle en (C₁-C₄) et alcoxy en (C₁-C₄),
et
le phényle et l'hétéroaryle à 5 ou 6 éléments pouvant être substitués avec 1 à 3 substituants choisis indépendamment les uns des autres dans le groupe constitué par halogène, cyano, trifluorométhyle, alkyle en (C₁-C₄), alcoxy en (C₁-C₄) et alkylsulfonyle en (C₁-C₄),
R¹⁷ représente hydrogène ou alkyle en (C₁-C₆),
l'alkyle en (C₁-C₄) pouvant être substitué par hydroxy,
ou
R¹⁶ et R¹⁷ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
à condition que les radicaux R¹⁴ et R¹⁶ ne représentent pas tous les deux simultanément phényle ou hétéroaryle à 5 ou 6 éléments,
ou
R¹⁴ et R¹⁶ forment ensemble avec les atomes de carbone auxquels ils sont reliés un carbocycle de 3 à 7 éléments ou un hétérocycle de 4 à 7 éléments,
à condition que pas plus d'une des paires de radicaux R¹⁴ et R¹⁵, R¹⁶ et R¹⁷, et R¹⁴ et R¹⁶ ne forment simultanément un carbo- ou hétérocycle,
R¹⁸ représente hydrogène ou alkyle en (C₁-C₄),
l'alkyle en (C₁-C₄) pouvant être substitué avec 1 à 5 substituants fluor,
m représente 0, 1 ou 2,
n représente 0 ou 1,
R¹⁹ représente hydrogène, cyano ou alkyle en (C₁-C₆),
l'alkyle en (C₁-C₆) pouvant être substitué avec 1 à 5 substituants fluor,
R²⁰ représente hydrogène ou alkyle en (C₁-C₄),
l'alkyle en (C₁-C₄) pouvant être substitué avec 1 à 5 substituants fluor,
R²¹ représente hydrogène ou alkyle en (C₁-C₆),
l'alkyle en (C₁-C₆) pouvant être substitué avec 1 à 5 substituants fluor,
R²² représente hydrogène ou alkyle en (C₁-C₄),
l'alkyle en (C₁-C₄) pouvant être substitué avec 1 à 5 substituants fluor,
ou
R¹⁹ et R²⁰ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
ou
R²¹ et R²² forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
ou
R¹⁹ et R²¹ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
à condition que pas plus d'une des paires de radicaux R¹⁹ et R²⁰, R²¹ et R²², et R¹⁹ et R²¹ ne forment simultanément un carbo- ou hétérocycle,
R²³ représente alkyle en (C₁-C₆), alcoxy en (C₁-C₆), alcoxycarbonyle en (C₁-C₄), hydroxycarbonyle, aminocarbonyle, aminosulfonyle, hétérocyclyle de 5 à 10 éléments relié par un atome de carbone de cycle, carbocyclyle de 5 à 10 éléments, phényle ou hétéroaryle de 5 à 10 éléments,
l'alkyle en (C₁-C₆) pouvant être substitué avec cyano, ainsi que jusqu'à six fois avec fluor,
l'alcoxy en (C₁-C₆) pouvant être substitué avec hydroxy, amino, monoalkylamino, dialkylamino, cyclopropyle, phényle ou alcényle en (C₂-C₄),
l'aminocarbonyle pouvant être substitué avec alkyle en (C₁-C₆) ou cycloalkyle en (C₃-C₆),
l'aminosulfonyle pouvant être substitué avec alkyle en (C₁-C₆) ou cycloalkyle en (C₃-C₆),
le phényle pouvant être substitué avec 1 à 3 substituants choisis indépendamment les uns des autres dans le groupe constitué par halogène, cyano, trifluorométhyle, difluorométhyle, alkyle en (C₁-C₆), alkylcarbonyle en (C₁-C₄), alcoxycarbonyle en (C₁-C₄), hydroxycarbonyle, -(C=O)NR²⁶R²⁷, alkylsulfonyle en (C₁-C₄), cycloalkylsulfonyle en (C₃-C₆), alkylthio en (C₁-C₄), alcoxy en (C₁-C₄), trifluorométhoxy, difluorométhoxy, phénoxy, hydroxy, hétéroaryle de 5 à 10 éléments, hétérocyclyle de 4 à 7 éléments, et cycloalkyle en (C₃-C₇),
l'alkyle en (C₁-C₆) pouvant être substitué avec 1 ou 2 substituants choisis indépendamment l'un de l'autre dans le groupe constitué par fluor, trifluorométhoxy, alkylcarbonyle en (C₁-C₄), - (C=O)NR²⁶R²⁷, alcoxy en (C₁-C₄), cycloalkyle en (C₃-C₆), morpholinyle, pipéridinyle, pyrrolidinyle, pipérazinyle, phényle, hydroxy et amino,
le phényle pouvant être substitué avec 1 à 3 substituants halogène,
l'amino pouvant être substitué avec 1 ou 2 substituants choisis indépendamment l'un de l'autre parmi alkyle en (C₁-C₆), alkylcarbonyle en (C₁-C₄), cycloalkylsulfonyle en (C₃-C₆), alkylsulfonyle en (C₁-C₄) et méthoxy-alkyle en (C₁-C₄),
le cycloalkyle en (C₃-C₆) pouvant être substitué avec amino ou hydroxy,
et
R²⁶ et R²⁷ représentant chacun indépendamment l'un de l'autre hydrogène, alkyle en (C₁-C₄) ou cycloalkyle en (C₃-C₇),
l'hétéroaryle de 5 à 10 éléments pouvant être substitué avec 1 à 3 substituants choisis indépendamment les uns des autres dans le groupe constitué par fluor, chlore, cyano, alkyle en (C₁-C₆), trifluorométhyle, alcoxy en (C₁-C₄), amino, alcoxycarbonyle en (C₁-C₄), hydroxycarbonyle, - (C=O)NR²⁵R²⁶, phényle, pyridyle, pyrimidyle, 1,3-thiazol-5-yle et cycloalkyle en (C₃-C₇),
l'alkyle en (C₁-C₆) pouvant être substitué avec 1 à 3 substituants choisis indépendamment les uns des autres dans le groupe constitué par halogène, cyano, hydroxy, amino, trifluorométhyle, difluorométhyle, alkylsulfonyle en (C₁-C₄), alkylcarbonyle en (C₁-C₄), alcoxycarbonyle en (C₁-C₄), hydroxycarbonyle, alkylthio en (C₁-C₄), alcoxy en (C₁-C₄), trifluorométhoxy, difluorométhoxy, phénoxy, phényle, pyridyle, pyrimidyle, hétéroaryle à 5 éléments, tétrahydrothiophényl-1,1-dioxyde, cycloalkyle en (C₃-C₇), morpholinyle, pipéridinyle, pyrrolidinyle, 2-oxopyrrolidin-1-yle, pipérazinyle, tétrahydrothiophényl-1,1-dioxyde, thiomorpholinyl-1,1-dioxyde et azétidine,
l'hétéroaryle à 5 éléments pouvant être substitué avec 1 à 3 substituants choisis indépendamment les uns des autres dans le groupe constitué par halogène, alkyle en (C₁-C₄) et alcoxy en (C₁-C₄),
le pipéridinyle pouvant être substitué avec 1 à 4 substituants fluor,
le phényle pouvant être substitué avec 1 à 3 substituants choisis indépendamment les uns des autres dans le groupe constitué par halogène, alkyle en (C₁-C₄) et alcoxy en (C₁-C₄),
l'azétidine pouvant être substituée avec hydroxy,
le pipérazinyle pouvant être substitué avec 1 à 3 substituants choisis indépendamment les uns des autres dans le groupe constitué par alkyle en (C₁-C₄), cycloalkyle en (C₃-C₇) et trifluorométhyle,
et
R²⁶ et R²⁷ représentant chacun indépendamment l'un de l'autre hydrogène, alkyle en (C₁-C₄) ou cycloalkyle en (C₃-C₇),
l'hétérocyclyle de 5 à 10 éléments relié par un atome de carbone de cycle pouvant être substitué avec 1 à 3 substituants choisis indépendamment les uns des autres dans le groupe constitué par oxo, fluor, trifluorométhyle, hydroxy et alkyle en (C₁-C₄),
l'hétérocyclyle de 5 à 10 éléments relié par un atome de carbone de cycle pouvant être annelé avec un cycle phényle ou un cycle pyridyle, qui peuvent de leur côté être substitués avec 1 à 3 substituants, choisis dans le groupe constitué par halogène, alkyle en (C₁-C₄) et trifluorométhyle,
et
le carbocycle de 5 à 10 éléments pouvant être substitué avec 1 à 3 substituants choisis indépendamment les uns des autres dans le groupe constitué par trifluorométhyle, fluor, cyano, hydroxy, hydroxycarbonyle, alcoxycarbonyle en (C₁-C₄), amino et alkyle en (C₁-C₄),
l'alkyle en (C₁-C₄) pouvant être substitué avec hydroxy ou hydroxycarbonyle,
le carbocyclyle de 5 à 10 éléments pouvant être annelé avec un cycle phényle ou un cycle pyridyle, qui peuvent de leur côté être substitués avec 1 à 3 substituants choisis dans le groupe constitué par halogène, alkyle en (C₁-C₄), alcoxy en (C₁-C₄) et trifluorométhyle,
R⁴ représente hydrogène,
R⁵ représente hydrogène, halogène, cyano, difluorométhyle, trifluorométhyle, alkyle en (C₁-C₄), cycloalkyle en (C₃-C₇), alcynyle en (C₂-C₄), alkylamino en (C₁-C₄), difluorométhoxy, trifluorométhoxy, alcoxy en (C₁-C₄), amino, hétérocyclyle de 4 à 7 éléments, ou hétéroaryle à 5 ou 6 éléments,
R⁶ représente hydrogène, cyano ou halogène,
ainsi que leurs N-oxydes, sels, solvates, sels des N-oxydes et solvates des N-oxydes et des sels.

2. Composé de formule (I) selon la revendication 1, dans laquelle
A représente CH₂ ou CD₂,
R¹ représente cycloalkyle en (C₃-C₆), pyridyle ou phényle,
le cycloalkyle en (C₃-C₆) pouvant être substitué avec 1 à 2 substituants choisis indépendamment l'un de l'autre dans le groupe constitué par fluor, trifluorométhyle, méthyle et éthyle,
le pyridyle étant substitué avec 1 ou 2 substituants fluor,
et
le phényle pouvant être substitué avec 1 à 4 substituants choisis indépendamment les uns des autres dans le groupe constitué par halogène, cyano, difluorométhyle, trifluorométhyle, alkyle en (C₁-C₄), alcoxy en (C₁-C₄) et cycloalkyle en (C₃-C₅),
R² représente hydrogène, alkyle en (C₁-C₄), cyclopropyle, cyclobutyle, difluorométhyle ou trifluorométhyle,
R³ représente un groupe de formule 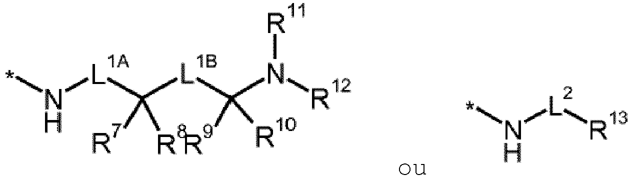 ou 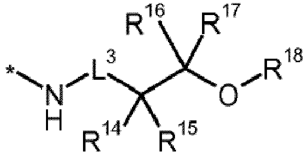 ou 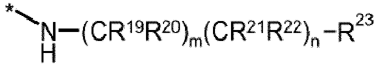 dans lesquelles
* représente l'emplacement de liaison au groupe carbonyle,
L^{1A} représente une liaison, méthanediyle, 1,2-éthanediyle ou 1,3-propanediyle,
L^{1B} représente une liaison, méthanediyle ou 1,2-éthanediyle,
L² représente une liaison, méthanediyle ou 1,2-éthanediyle,
L³ représente une liaison, méthanediyle ou 1,2-éthanediyle,
R⁷ représente hydrogène, alkyle en (C₁-C₆), cycloalkyle en (C₃-C₇), hétéroaryle à 5 ou 6 éléments ou phényle,
l'alkyle en (C₁-C₆) pouvant être substitué jusqu'à 5 fois avec fluor,
le phényle et l'hétéroaryle à 5 ou 6 éléments pouvant être substitués avec 1 à 3 substituants choisis indépendamment les uns des autres dans le groupe constitué par fluor, chlore, brome, cyano, nitro, trifluorométhyle, difluorométhyle, méthyle, éthyle, méthoxy, éthoxy, difluorométhoxy, trifluorométhoxy, méthoxycarbonyle et éthoxycarbonyle,
et
le phényle pouvant être substitué sur deux atomes de carbone voisins du phényle avec un pont méthylènedioxy ou un pont éthylènedioxy,
ou
peut représenter fluor lorsque L^{1A} ne représente pas une liaison,
R⁸ représente hydrogène ou alkyle en (C₁-C₄),
ou
peut représenter fluor lorsque L^{1A} ne représente pas une liaison,
ou
R⁷ et R⁸ forment ensemble avec l'atome de carbone auquel ils sont reliés un carbocycle de 3 à 7 éléments,
R⁹ représente hydrogène, alkyle en (C₁-C₆), cycloalkyle en (C₃-C₅), cyano, hétéroaryle de 5 à 10 éléments ou phényle,
l'alkyle en (C₁-C₆) pouvant être substitué avec alcoxy en (C₁-C₄), alcoxycarbonyle en (C₁-C₄) alkylsulfonyle en (C₁-C₄), alkylthio en (C₁-C₄), benzyloxy, phénoxy, hétéroaryle à 5 ou 6 éléments, et phényle, ainsi que jusqu'à cinq fois avec fluor,
le benzyloxy, le phénoxy, l'hétéroaryle à 5 ou 6 éléments et le phényle pouvant être substitués avec 1 à 3 substituants choisis indépendamment les uns des autres dans le groupe constitué par fluor, chlore, brome, méthoxy et éthoxy,
le cycloalkyle en (C₃-C₅) pouvant être substitué avec 1 ou 2 substituants fluor, trifluorométhyle, méthyle, éthyle, méthoxy ou éthoxy,
le phényle et l'hétéroaryle de 5 à 10 éléments pouvant être substitués avec 1 à 3 substituants choisis indépendamment les uns des autres dans le groupe constitué par fluor, chlore, brome, cyano, nitro, trifluorométhyle, méthyle, éthyle, éthényle, propényle, méthoxy, éthoxy, difluorométhoxy, trifluorométhoxy, méthoxycarbonyle et éthoxycarbonyle,
le méthoxy et l'éthoxy pouvant être substitués avec hydroxy,
et
le phényle pouvant être substitué sur deux atomes de carbone voisins du phényle avec un pont méthylènedioxy ou un pont éthylènedioxy,
R¹⁰ représente hydrogène ou alkyle en (C₁-C₄),
ou
R⁹ et R¹⁰ forment ensemble avec l'atome de carbone auquel ils sont reliés un carbocycle de 3 à 7 éléments ou un hétérocycle de 4 à 7 éléments,
ou
R⁷ et R⁹ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant être substitués avec 1 ou 2 substituants alkyle en (C₁-C₄),
et
le carbocycle de 3 à 7 éléments et l'hétérocycle de 4 à 7 éléments pouvant être annelés avec un cycle phényle ou un cycle pyridyle, qui peuvent de leur côté être substitués avec 1 ou 2 substituants choisis parmi fluor, chlore, brome, méthyle, éthyle et trifluorométhyle,
à condition que pas plus d'une des paires de radicaux R⁷ et R⁸, R⁹ et R¹⁰, et R⁷ et R⁹ ne forment simultanément un carbo- ou hétérocycle,
à condition que les radicaux R⁷ et R⁹ ne représentent pas tous les deux simultanément phényle ou hétéroaryle à 5 ou 6 éléments,
R¹¹ représente hydrogène ou alkyle en (C₁-C₄),
l'alkyle en (C₁-C₄) pouvant être substitué jusqu'à cinq fois avec fluor,
R¹² représente hydrogène, alkyle en (C₁-C₆), cycloalkyle en (C₃-C₇) ou alkylcarbonyle en (C₁-C₄),
l'alkyle en (C₁-C₆) pouvant être substitué jusqu'à cinq fois avec fluor,
ou
R¹¹ et R¹² forment ensemble avec l'atome d'azote auquel ils sont reliés un hétérocycle aza de 4 à 7 éléments,
l'hétérocycle aza de 4 à 7 éléments pouvant être substitué avec méthyle ou éthyle,
R¹³ représente un hétérocyclyle aza de 5 à 10 éléments, relié par un atome de carbone de cycle, ou un hétérocyclyle à 5 ou 6 éléments, relié par un atome d'azote de cycle,
l'hétérocyclyle aza de 5 à 10 éléments, relié par un atome de carbone de cycle pouvant être substitué avec 1 à 2 substituants trifluorométhyle, cycloalkyle en (C₃-C₇), oxo et benzyle, ainsi que jusqu'à quatre fois avec alkyle en (C₁-C₄) et jusqu'à deux fois avec fluor,
l'hétérocyclyle aza de 5 à 10 éléments pouvant être annelé avec un cycle phényle ou un cycle pyridyle, qui peuvent de leur côté être substitués avec 1 ou 2 substituants choisis parmi fluor, chlore, méthyle, alcoxy en (C₁-C₄) et trifluorométhyle,
l'hétérocyclyle à 5 ou 6 éléments relié par un atome d'azote de cycle pouvant être substitué avec alkyle en (C₁-C₄) ou cycloalkyle en (C₁-C₄),
ou
peut représenter amino lorsque L² représente une liaison,
l'amino pouvant être substitué avec alkyle en (C₁-C₁₀), alkylcarbonyle en (C₁-C₄), carbocyclyle en (C₃-C₆), hétérocyclyle de 4 à 7 éléments, phényle ou hétéroaryle à 5 ou 6 éléments,
l'alkylcarbonyle en (C₁-C₄) pouvant être substitué avec monoalkylamino, dialkylamino ou un hétérocycle à 5 ou 6 éléments relié par un atome d'azote de cycle,
l'hétérocyclyle à 5 ou 6 éléments relié par un atome d'azote de cycle pouvant être substitué avec oxo,
le carbocyclyle en (C₃-C₆) et l'hétérocyclyle de 4 à 7 éléments pouvant être substitués avec hydroxy,
et
le phényle et l'hétéroaryle à 5 ou 6 éléments pouvant être substitués avec 1 à 3 substituants choisis indépendamment les uns des autres dans le groupe constitué par fluor, chlore, méthyle et trifluorométhyle,
R¹⁴ représente hydrogène, alkyle en (C₁-C₆), cycloalkyle en (C₃-C₇), -(C=O)NR²⁴R²⁵, hétéroaryle à 5 ou 6 éléments ou phényle,
l'alkyle en (C₁-C₆) pouvant être substitué avec 1 ou 2 substituants choisis indépendamment l'un de l'autre dans le groupe constitué par hydroxy et alcoxy en (C₁-C₄), ainsi que jusqu'à six fois avec fluor,
R²⁴ représentant hydrogène, alkyle en (C₁-C₄), aryle ou naphtyle,
R²⁵ représentant hydrogène,
et
le phényle et l'hétéroaryle à 5 ou 6 éléments pouvant être substitués avec 1 à 3 substituants choisis indépendamment les uns des autres dans le groupe constitué par fluor, chlore, brome, trifluorométhyle, difluorométhyle, méthyle, éthyle et 1-amino-2-méthylpropyle,
R¹⁵ représente hydrogène ou alkyle en (C₁-C₆),
l'alkyle en (C₁-C₄) pouvant être substitué avec hydroxy,
ou
R¹⁴ et R¹⁵ forment ensemble avec l'atome de carbone auquel ils sont reliés un carbocycle de 3 à 7 éléments,
R¹⁶ représente hydrogène, alkyle en (C₁-C₆) ou cycloalkyle en (C₃-C₇),
l'alkyle en (C₁-C₆) pouvant être substitué avec 1 ou 2 substituants choisis indépendamment l'un de l'autre dans le groupe constitué par hydroxy et alcoxy en (C₁-C₄), ainsi que jusqu'à six fois avec fluor,
le cycloalkyle en (C₃-C₇) pouvant être substitué avec 1 ou 2 substituants choisis indépendamment l'un de l'autre dans le groupe constitué par fluor, trifluorométhyle, alkyle en (C₁-C₄) et alcoxy en (C₁-C₄),
R¹⁷ représente hydrogène ou alkyle en (C₁-C₆),
ou
R¹⁶ et R¹⁷ forment ensemble avec l'atome de carbone auquel ils sont reliés un carbocycle de 3 à 6 éléments,
le carbocycle de 3 à 6 éléments pouvant être substitué avec 1 ou 2 substituants choisis indépendamment l'un de l'autre dans le groupe constitué par fluor, méthyle et éthyle,
ou
R¹⁴ et R¹⁶ forment ensemble avec les atomes de carbone auxquels ils sont reliés un carbocycle de 3 à 7 éléments ou un hétérocycle de 4 à 7 éléments,
à condition que pas plus d'une des paires de radicaux R¹⁴ et R¹⁵, R¹⁶ et R¹⁷, et R¹⁴ et R¹⁶ ne forment simultanément un carbo- ou hétérocycle,
R¹⁸ représente hydrogène ou alkyle en (C₁-C₄),
l'alkyle en (C₁-C₄) pouvant être substitué avec 1 à 5 substituants fluor,
m représente 0 ou 1,
n représente 0 ou 1,
R¹⁹ représente hydrogène, cyano ou alkyle en (C₁-C₄),
l'alkyle en (C₁-C₄) pouvant être substitué avec 1 à 5 substituants fluor,
R²⁰ représente hydrogène ou alkyle en (C₁-C₄),
l'alkyle en (C₁-C₄) pouvant être substitué avec 1 à 5 substituants fluor,
R²¹ représente hydrogène ou alkyle en (C₁-C₄),
l'alkyle en (C₁-C₄) pouvant être substitué avec 1 à 5 substituants fluor,
R²² représente hydrogène ou alkyle en (C₁-C₄),
l'alkyle en (C₁-C₄) pouvant être substitué avec 1 à 5 substituants fluor,
ou
R¹⁹ et R²⁰ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
ou
R²¹ et R²² forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
ou
R¹⁹ et R²¹ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
à condition que pas plus d'une des paires de radicaux R¹⁹ et R²⁰, R²¹ et R²², et R¹⁹ et R²¹ ne forment simultanément un carbo- ou hétérocycle,
R²³ représente alkyle en (C₁-C₆), alcoxy en (C₁-C₆), alcoxycarbonyle en (C₁-C₄), hydroxycarbonyle, aminocarbonyle, aminosulfonyle, hétérocyclyle de 5 à 10 éléments relié par un atome de carbone de cycle, carbocyclyle de 5 à 10 éléments, phényle ou hétéroaryle de 5 à 10 éléments,
l'alkyle en (C₁-C₆) pouvant être substitué avec cyano, ainsi que jusqu'à six fois avec fluor,
l'alcoxy en (C₁-C₆) pouvant être substitué avec hydroxy ou alcényle en (C₂-C₄),
l'aminocarbonyle pouvant être substitué avec alkyle en (C₁-C₆) ou cycloalkyle en (C₃-C₆),
l'aminosulfonyle pouvant être substitué avec alkyle en (C₁-C₆) ou cycloalkyle en (C₃-C₆),
le phényle pouvant être substitué avec 1 à 3 substituants choisis indépendamment les uns des autres dans le groupe constitué par halogène, cyano, trifluorométhyle, difluorométhyle, alkyle en (C₁-C₄), alcoxy en (C₁-C₄), hétéroaryle de 5 à 10 éléments et hétérocyclyle de 4 à 7 éléments,
l'alkyle en (C₁-C₆) pouvant être substitué avec 1 ou 2 substituants choisis indépendamment l'un de l'autre dans le groupe constitué par fluor, trifluorométhoxy, alcoxy en (C₁-C₄), cycloalkyle en (C₃-C₆), hydroxy et amino,
l'hétéroaryle de 5 à 10 éléments pouvant être substitué avec 1 à 3 substituants choisis indépendamment les uns des autres dans le groupe constitué par fluor, chlore, cyano, alkyle en (C₁-C₄), trifluorométhyle, alcoxy en (C₁-C₄) et amino,
l'alkyle en (C₁-C₄) pouvant être substitué avec 1 à 3 substituants choisis indépendamment les uns des autres dans le groupe constitué par halogène, cyano, hydroxy, amino, trifluorométhyle, difluorométhyle, alcoxy en (C₁-C₄), trifluorométhoxy, difluorométhoxy et phényle,
le phényle pouvant être substitué avec 1 à 3 substituants halogène,
l'hétérocyclyle de 5 à 10 éléments relié par un atome de carbone de cycle pouvant être substitué avec 1 ou 2 substituants choisis indépendamment les uns des autres dans le groupe constitué par oxo, fluor, trifluorométhyle, hydroxy et alkyle en (C₁-C₄),
l'hétérocyclyle de 5 à 10 éléments relié par un atome de carbone de cycle pouvant être annelé avec un cycle phényle ou un cycle pyridyle, qui peuvent de leur côté être substitués avec 1 à 3 substituants, choisis dans le groupe constitué par halogène, alkyle en (C₁-C₄), alcoxy en (C₁-C₄) et trifluorométhyle,
et
le carbocycle de 5 à 10 éléments pouvant être substitué avec 1 ou 2 substituants choisis indépendamment l'un de l'autre dans le groupe constitué par trifluorométhyle, fluor, cyano, hydroxy, hydroxycarbonyle, alcoxycarbonyle en (C₁-C₄), amino et alkyle en (C₁-C₄),
le carbocyclyle de 5 à 10 éléments pouvant être annelé avec un cycle phényle ou un cycle pyridyle, qui peuvent de leur côté être substitués avec 1 à 3 substituants choisis dans le groupe constitué par halogène, alkyle en (C₁-C₄), alcoxy en (C₁-C₄) et trifluorométhyle,
R⁴ représente hydrogène,
R⁵ représente hydrogène, fluor, chlore, brome, cyano, difluorométhyle, trifluorométhyle, alkyle en (C₁-C₄), méthoxy, cycloalkyle en (C₃-C₅) ou alcynyle en (C₂-C₄),
R⁶ représente hydrogène ou fluor,
ainsi que leurs N-oxydes, sels, solvates, sels des N-oxydes et solvates des N-oxydes et des sels.

3. Composé de formule (I) selon la revendication 1 ou 2, dans lequel
A représente CH₂,
R¹ représente cyclohexyle, pyridyle ou phényle,
le pyridyle étant substitué avec 1 ou 2 substituants F,
et
le phényle pouvant être substitué avec 1 à 4 substituants choisis indépendamment les uns des autres dans le groupe constitué par fluor, chlore, cyano, méthyle, éthyle, méthoxy, éthoxy et cyclopropyle,
R² représente méthyle, cyclopropyle, difluorométhyle ou trifluorométhyle,
R³ représente un groupe de formule 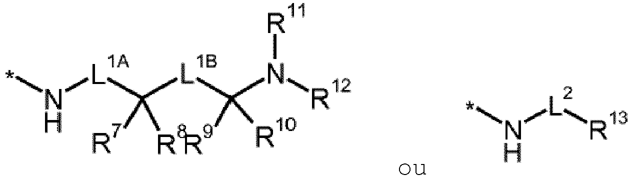 ou 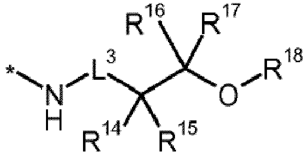 ou 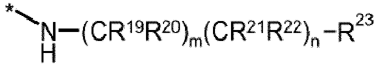 dans lesquelles
* représente l'emplacement de liaison au groupe carbonyle,
L^{1A} représente une liaison ou méthanediyle,
L^{1B} représente une liaison,
L² représente une liaison,
L³ représente une liaison,
R⁷ représente hydrogène, alkyle en (C₁-C₆) ou phényle,
l'alkyle en (C₁-C₆) pouvant être substitué jusqu'à cinq fois avec fluor,
le phényle pouvant être substitué avec 1 à 3 substituants choisis indépendamment les uns des autres dans le groupe constitué par fluor, chlore, brome, trifluorométhyle, méthyle, méthoxy, difluorométhoxy et trifluorométhoxy,
et
le phényle pouvant être substitué sur deux atomes de carbone voisins du phényle avec un pont méthylènedioxy ou un pont éthylènedioxy,
ou
peut représenter fluor lorsque L^{1A} ne représente pas une liaison,
R⁸ représente hydrogène, méthyle ou éthyle,
ou
peut représenter fluor lorsque L^{1A} ne représente pas une liaison,
R⁹ représente hydrogène, alkyle en (C₁-C₆), cyclopropyle ou cyclobutyle,
l'alkyle en (C₁-C₆) pouvant être substitué jusqu'à cinq fois avec fluor,
le cyclopropyle et le cyclobutyle pouvant être substitués avec trifluorométhyle,
R¹⁰ représente hydrogène ou alkyle en (C₁-C₄),
ou
R⁹ et R¹⁰ forment ensemble avec l'atome de carbone auquel ils sont reliés un carbocycle de 3 à 7 éléments,
R¹¹ représente hydrogène, méthyle ou éthyle,
l'éthyle pouvant être substitué jusqu'à trois fois avec fluor,
R¹² représente hydrogène, alkyle en (C₁-C₄), cyclopropyle ou cyclobutyle,
l'alkyle en (C₁-C₆) pouvant être substitué jusqu'à cinq fois avec fluor,
ou
R¹¹ et R¹² forment ensemble avec l'atome d'azote auquel ils sont reliés un cycle morpholinyle,
R¹³ représente 9-azabicyclo[3.3.1]nonan-3-yle, pyrrolidinyle, pipéridin-4-yle, azépanyle ou 1,2,3,4-tétrahydroquinolinyle,
le pipéridin-4-yle pouvant être substitué avec 1 à 5 substituants méthyle, ainsi que jusqu'à deux fois avec fluor,
le 1,2,3,4-tétrahydroquinolinyle pouvant être substitué avec 1 à 2 substituants choisis indépendamment l'un de l'autre dans le groupe constitué par fluor, oxo, méthyle, éthyle, méthoxy, éthoxy et trifluorométhyle,
le 9-azabicyclo[3.3.1]nonan-3-yle pouvant être substitué avec méthyle,
le pyrrolidinyle pouvant être substitué avec 1 à 2 substituants choisis indépendamment l'un de l'autre dans le groupe constitué par fluor, oxo, méthyle et éthyle,
R¹⁴ représente hydrogène, alkyle en (C₁-C₆), - (C=O)NR²⁴R²⁵ ou phényle,
l'alkyle en (C₁-C₆) pouvant être substitué avec hydroxy ou méthoxy, ainsi que jusqu'à cinq fois avec fluor,
R²⁴ représentant aryle ou naphtyle,
R²⁵ représentant hydrogène,
et
le phényle pouvant être substitué avec 1 ou 2 substituants choisis indépendamment l'un de l'autre dans le groupe constitué par fluor, chlore, trifluorométhyle et méthyle,
R¹⁵ représente hydrogène ou alkyle en (C₁-C₆),
R¹⁶ représente hydrogène, alkyle en (C₁-C₆), cyclopropyle ou cyclobutyle,
l'alkyle en (C₁-C₆) pouvant être substitué jusqu'à six fois avec fluor,
R¹⁷ représente hydrogène ou alkyle en (C₁-C₆),
ou
R¹⁶ et R¹⁷ forment ensemble avec l'atome de carbone auquel ils sont reliés un carbocycle de 3 à 6 éléments,
le carbocycle de 3 à 6 éléments pouvant être substitué avec 1 ou 2 substituants choisis indépendamment l'un de l'autre dans le groupe constitué par fluor et méthyle,
R¹⁸ représente hydrogène ou alkyle en (C₁-C₄),
l'alkyle en (C₁-C₄) pouvant être substitué avec 1 à 5 substituants fluor,
m représente 0 ou 1,
n représente 0 ou 1,
R¹⁹ représente hydrogène, cyano ou méthyle,
le méthyle pouvant être substitué avec 1 à 3 substituants fluor,
R²⁰ représente hydrogène ou méthyle,
le méthyle pouvant être substitué avec 1 à 3 substituants fluor,
R²¹ représente hydrogène ou méthyle,
le méthyle pouvant être substitué avec 1 à 3 substituants fluor,
R²² représente hydrogène ou méthyle,
le méthyle pouvant être substitué avec 1 à 3 substituants fluor,
ou
R¹⁹ et R²⁰ forment ensemble avec l'atome de carbone auquel ils sont reliés un carbocycle de 3 à 5 éléments,
ou
R¹⁹ et R²¹ forment ensemble avec l'atome de carbone auquel ils sont reliés un cycle cyclopropyle,
à condition que pas plus d'une des paires de radicaux R¹⁹ et R²⁰, et R¹⁹ et R²¹ ne forment simultanément un carbocycle,
R²³ représente alkyle en (C₁-C₆), 2-oxopyrrolidin-3-yle, 2-oxotétrahydrofuran-3-yle, cyclopentyle, cyclohexyle, indanyle, 1,2,4-oxadiazol-5-yle, 1H-imidazol-2-yle, 1H-pyrazol-4-yle, pyridin-3-yle, pyrimidin-5-yle, quinolin-4-yle, pyrazolo[1,5-a]pyridin-3-yle, 3,4-dihydro-2H-pyranyle, 1,2,3,4-tétrahydronaphtalinyle, bicyclo[2.2.2]octanyle, chroman-4-yle, 2,3-dihydro-1-benzofuran-3-yle, 2,3-dihydro-1H-indényle, 3,4-dihydroquinolinyle, 3,4-dihydro-2H-pyrano[2,3-b]pyridinyle ou phényle,
l'alkyle en (C₁-C₆) pouvant être substitué avec cyano, ainsi que jusqu'à trois fois avec fluor,
le phényle pouvant être substitué avec 1 à 3 substituants choisis indépendamment les uns des autres dans le groupe constitué par fluor, chlore, cyano, trifluorométhyle, méthyle, éthyle, méthoxy, 1H-imidazol-1-yle et pyridyle,
le 1,2,4-oxadiazol-5-yle, le 1H-imidazol-2-yle, le 1H-pyrazol-4-yle, le pyridin-3-yle, le pyrimidin-5-yle, le quinolin-4-yle ou le pyrazolo[1,5-a]pyridin-3-yle pouvant être substitué avec 1 à 3 substituants choisis indépendamment les uns des autres dans le groupe constitué par fluor, chlore, trifluorométhyle, alkyle en (C₁-C₃), amino et hydroxy,
l'alkyle en (C₁-C₃) pouvant être substitué avec fluor, hydroxy, amino ou trifluorométhyle,
le cyclopentyle, le cyclohexyle et le bicyclo[2.2.2]octanyle pouvant être substitués avec méthoxycarbonle, éthoxycarbonyle, hydroxycarbonyle et cyano,
le chroman-4-yle, le 2,3-dihydro-1-benzofuran-3-yle, le 2,3-dihydro-1H-indényle, le 3,4-dihydroquinolinyle et le 3,4-dihydro-2H-pyrano[2,3-b]pyridinyle pouvant être substitués avec 1 ou 2 substituants choisis dans le groupe constitué par fluor, chlore, méthyle, éthyle, méthoxy, éthoxy et trifluorométhyle,
et
le 2,3-dihydro-1H-indényle et l'indanyle pouvant être substitués avec 1 ou 2 substituants choisis dans le groupe constitué par fluor, chlore, méthyle, éthyle, méthoxy, éthoxy, hydroxy et trifluorométhyle,
le méthyle et l'éthyle pouvant être substitués avec hydroxy,
R⁴ représente hydrogène,
R⁵ représente hydrogène, fluor, chlore, méthyle ou éthyle,
R⁶ représente hydrogène,
ainsi que leurs N-oxydes, sels, solvates, sels des N-oxydes et solvates des N-oxydes et des sels.

4. Composé de formule (I) selon la revendication 1, 2 ou 3, dans lequel
A représente CH₂,
R¹ représente un groupe phényle de formule 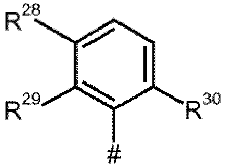 dans laquelle
# représente l'emplacement de liaison à A et
R²⁸ représente hydrogène ou fluor,
R²⁹ représente fluor,
R³⁰ représente fluor,
R² représente méthyle ou cyclopropyle,
R³ représente un groupe de formule 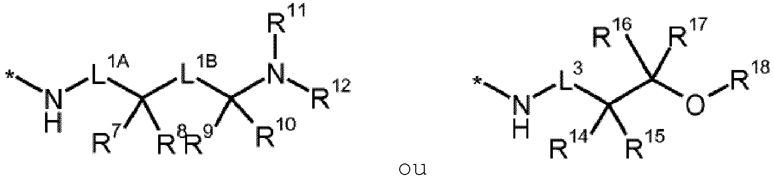 dans lesquelles
* représente l'emplacement de liaison au groupe carbonyle,
L^{1A} représente une liaison,
L^{1B} représente une liaison,
R⁷ représente hydrogène, alkyle en (C₁-C₆) ou phényle,
l'alkyle en (C₁-C₆) pouvant être substitué jusqu'à cinq fois avec fluor,
le phényle pouvant être substitué avec 1 à 2 substituants chlore ou fluor,
R⁸ représente hydrogène, méthyle ou éthyle,
R⁹ représente hydrogène, alkyle en (C₁-C₆) ou cyclopropyle,
l'alkyle en (C₁-C₆) pouvant être substitué jusqu'à cinq fois avec fluor,
R¹⁰ représente hydrogène, méthyle ou éthyle,
R¹¹ représente hydrogène,
R¹² représente hydrogène,
R¹⁴ représente hydrogène, alkyle en (C₁-C₆) ou phényle,
l'alkyle en (C₁-C₆) pouvant être substitué avec hydroxy, ainsi que jusqu'à cinq fois avec des substituants fluor,
et
le phényle pouvant être substitué avec 1 ou 2 substituants fluor,
R¹⁵ représente hydrogène, méthyle ou éthyle,
R¹⁶ représente hydrogène ou alkyle en (C₁-C₆),
R¹⁷ représente hydrogène, méthyle ou éthyle,
ou
R¹⁶ et R¹⁷ forment ensemble avec l'atome de carbone auquel ils sont reliés un carbocycle de 3 à 6 éléments,
R¹⁸ représente hydrogène,
R⁴ représente hydrogène,
R⁵ représente hydrogène ou méthyle,
R⁶ représente hydrogène,
ainsi que leurs N-oxydes, sels, solvates, sels des N-oxydes et solvates des N-oxydes et des sels.

5. Procédé de fabrication de composés de formule (I), tels que définis dans les revendications 1 à 4, **caractérisé en ce que**
[A] un composé de formule (II) 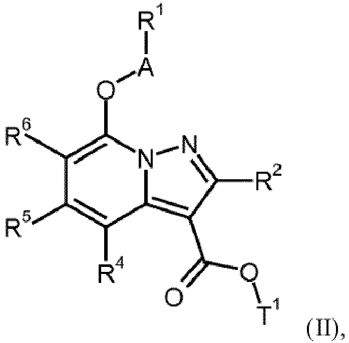 dans laquelle A, R¹, R², R⁴, R⁵ et R⁶ ont chacun les significations indiquées précédemment, et
T¹ représente alkyle en (C₁-C₄) ou benzyle,
est mis en réaction dans un solvant inerte en présence d'une base ou d'un acide approprié pour former un acide carboxylique de formule (III) 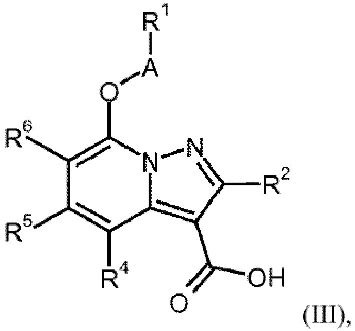 dans laquelle A, R¹, R², R⁴, R⁵ et R⁶ ont chacun les significations indiquées précédemment,
puis celui-ci est mis en réaction dans un solvant inerte dans des conditions de couplage d'amide avec une amine de formule (IV-A), (IV-B), (IV-C) ou (IV-D) 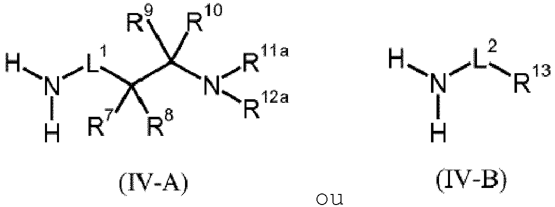 ou 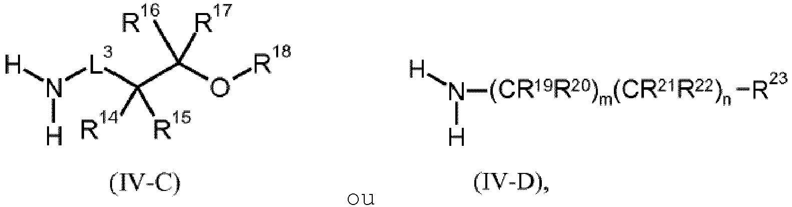 dans lesquelles L¹, L², L³, R⁷, R⁸, R⁹, R¹⁰, R¹³, R¹⁴, R¹⁵, R¹⁶, R¹⁷, R¹⁸, R¹⁹, R²⁰, R²¹, R²² et R²³ ont chacun les significations indiquées précédemment,
et
R^{11A} et R^{12A} ont les significations indiquées précédemment pour R¹¹ et R¹², ou représentent un groupe protecteur amino, tel que par exemple tert.- butoxycarbonyle, benzyloxycarbonyle ou benzyle,
puis des groupes protecteurs éventuellement présents sont clivés, et les composés de formule (I) résultants sont éventuellement transformés avec (i) les solvants et/ou (ii) les acides ou bases appropriés en leurs solvates, sels et/ou solvates des sels.

6. Composé de formule (I), tel que défini dans l'une quelconque des revendications 1 à 4, destiné à une utilisation dans le traitement et/ou la prophylaxie de maladies.

7. Utilisation d'un composé de formule (I), tel que défini dans l'une quelconque des revendications 1 à 4, pour la fabrication d'un médicament pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques et de l'artériosclérose.

8. Médicament contenant un composé de formule (I), tel que défini dans l'une quelconque des revendications 1 à 4, en combinaison avec un adjuvant inerte, non toxique, pharmaceutiquement approprié.

9. Médicament contenant un composé de formule (I), tel que défini dans l'une quelconque des revendications 1 à 4, en combinaison avec un autre agent actif choisi dans le groupe constitué par les nitrates organiques, les donneurs de NO, les inhibiteurs de cGMP-PDE, les agents antithrombotiques, les agents abaissant la tension artérielle, ainsi que les agents modifiant le métabolisme des lipides.

10. Médicament selon la revendication 8 ou 9, destiné à une utilisation dans le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques et de l'artériosclérose.
